# EUROPEAN PATENT APPLICATION

(11) **EP 2 476 433 A1**
(43) Date of publication of application: **18.07.2012**
(21) Application number: 12164165.8
(22) Date of filing: 29.03.2007
(51) Int. Cl.: A61K 39/085, A61K 31/70, A61P 31/04

(54) **Immunogenic composition**

(30) Priority: 30.03.2006 GB 0606416; 30.03.2006 US 787249 P; 30.03.2006 US 787587 P
(62) Divisional of application: 07727529.5
(71) Applicant: GlaxoSmithKline Biologicals S.A., 1330 Rixensart (BE)
(72) Inventor: Denoel, Philippe, 1330 Rixensart (BE); Poolman, Jan, 1330 Rixensart (BE)
(74) Representative: Johnston, Caroline Louise

(57) **Abstract**

The present application relates to immunogenic compositions comprising Type 5 and/or 8 capular polysaccharide or oligosaccharide from *S*. *aureus* having between 30-100% O-acetylation. Vaccines, methods of treatment using and processes to make an immunogenic composition comprising Type 5 and/or 8 capsular polysaccharides with 30-100% O-acetylation are also described.

## Description

### Technical Field

The present invention relates to the field of Staphylococcal immunogenic compositions and vaccines, their manufacture and the use of such compositions in medicine. More particularly, it relates to vaccine compositions comprising type 5 and/or 8 polysaccharides from *S*. *aureus* in which the degree of O-acetylation is between 30-100%. Methods for the treatment or prevention of staphylococcal infections using such vaccines are also provided.

### Background

The number of both community acquired and hospital acquired infections have increased over recent years with the increased use of intravascular devices. Hospital acquired (nosocomial) infections are a major cause of morbidity and mortality, more particularly in the US, where they affect more than 2 million patients annually. Following various studies, about 6 percent of the US patients will acquire an infection during their stay in hospital. The economic burden in the USA was estimated to be more than $4.5 billion in 1992 (Emori and Gaynes, 1993, Clin. Microbiol. Rev. 6; 428). The most frequent infections are urinary tract infections (UTI-33% of the infections), followed by pneumonia (15.5%), surgical site infections (14.8%) and primary bloodstream infections (13%) Emori and Gaynes, 1993, Clin. Microbiol. Rev. 6; 428).

*Staphylococcus aureus,* Coagulase-negative Staphylococci (mostly *Staphylococcus epidermidis*), *enterococcus* spp, *Esherichia coli* and *Pseudomonas aeruginosa* are the major nosocomial pathogens. Although those pathogens almost cause the same number of infections, the severity of the disorders they can produce combined with the frequency of antibiotic resistant isolates balance this ranking towards *S. aureus* and *S. epidermidis* as being the most significant nosocomial pathogens.

Staphylococcus aureus is the most common cause of nosocomial infections with a significant morbidity and mortality (Romero-Vivas et al 1995, Infect. Dis. 21; 1417). It is the cause of some cases of osteomyelitis, endocarditis, septic arthritis, pneumonia, abscesses and toxic shock syndrome.

*S. epidermidis* is a normal skin commensal which is also an important opportunistic pathogen responsible for infections of implanted medical devices and infections at sites of surgery. Medical devices infected by *S. epidermidis* include cardiac pacemakers, cerebrospinal fluid shunts, continuous ambulatory peritoneal dialysis catheters, orthopaedic devices and prosthetic heart valves.

*S. aureus* and *S. epidermidis* infections are treated with antibiotics, with penicillin being the drug of choice whereas vancomycin is used for methicillin resistant isolates. The percentage of staphylococcal strains exhibiting wide-spectrum resistance to antibiotics has become increasingly prevalent since the 1980's (Panlilo et al 1992, Infect.Control. Hosp. Epidemiol. 13; 582), posing a threat for effective antimicrobial therapy. In addition, the recent emergence of vancomycin resistant *S. aureus* strain has aroused fear that methicillin resistant *S. aureus* strains will emerge and spread for which no effective therapy is available.

An alternative approach of using antibodies against staphylococcal antigens in passive immunotherapy has been investigated. Therapy involving administration of polyclonal antisera are under development (WO 00/15238, WO 00/12132) as well as treatment with a monoclonal antibody against lipoteichoic acid (WO 98/57994).

An alternative approach would be use of active vaccination to generate an immune response against staphylococci. Several candidates for inclusion as vaccine components have been identified. These include Fibronectin binding protein (US5840846), MHC II analogue (US5648240), fibrinogen binding protein (US6008341), GehD (US 2002/0169288), collagen binding protein (US6288214), SdrF, SdrG and SdrH (WO 00/12689), mutant SEA and SEB exotoxins (WO 00/02523) and 52kDa vitronectin binding protein (WO 01/60852).

The *S. aureus* genome has been sequenced and many of the coding sequences have been identified (EP786519, WO02/094868). The same is true for *S. epidermidis* (WO 01/34809). As a refinement of this approach, others have identified proteins that are recognised by hyperimmune sera from patients who have suffered staphylococcal infection (WO01/98499, WO 02/059148).

The first generation of vaccines targeted against *S*. *aureus* or against the exoproteins it produces have met with limited success (Lee 1996 Trends Microbiol. 4; 162). There remains a need to develop effective vaccines against staphylococcal infections.

### Description of Figures

Figure 1 - Polypeptide sequences of proteins for inclusion in an immunogenic composition. Table 1 provides information on which protein is represented by each SEQ ID.
Figure 2 - Nucleotide sequences encoding proteins for inclusion in an immunogenic composition. Table 1 provides information on which protein is encoded by each SEQ ID.

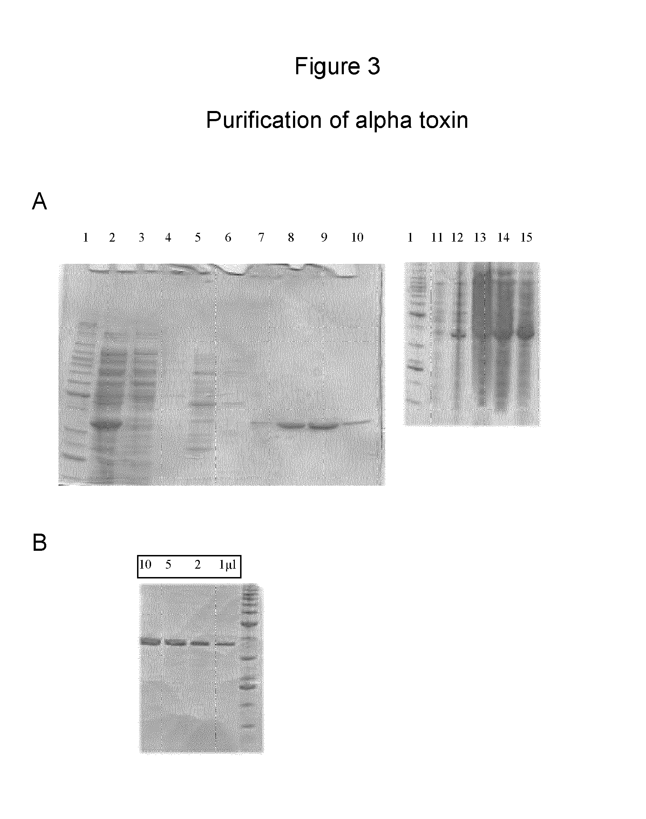

Figure 3 - Purification of alpha toxin under native conditions. Panel A shows a coommassie stained SDS-PAGE of samples prepared during the purification of alpha toxin. Lane 1 - molecular weight markers, lane 2 - soluble fraction containing over-expressed alpha toxin, lane 3 - flow through from the Ni-NTA column, lane 4 - fractions eluted with 10% buffer B, lane 5 - fractions eluted with 20% buffer B, lane 6 - fractions eluted with 30% buffer B, lane 7 - fractions eluted with 50% buffer B, lane 8 - fractions eluted with 75% buffer B, lane 9 and 10 fractions eluted with 100% buffer B, lane 11 bacteria at T=0 before induction, lane 12 - bacteria at T=4 hours after induction, lane 13 - cell lysate, lane 14 - soluble fraction, lane 15 - insoluble fraction. Panel B shows a coommassie stained SDS-PAGE of 10, 5, 2 and 1µl of the purified alpha toxin.
Figure 4 - Purification of SdrC underdenaturing conditions. Panel A shows a coommassie stained SDS-PAGE of samples prepared during the purification of alpha toxin. Lane M - molecular weight markers, lane Start - supernatant fromed from the insoluble fraction containing over-expressed SdrC, lane FT1 - flow through from the Ni-NTA column, lane C - fractions eluted with wash buffer C, lane D - fractions eluted with buffer D, lane E - fractions eluted with buffer E.
   Panel B shows a coommassie stained SDS-PAGE of 1, 2, 5 and 10µl of the purified SdrC.
Figure 5 - ELISA results for antisera against staphylococcal proteins in plates coated with purified proteins.
   Pool mice pre - result using pooled sera extracted from mice pre-innoculation. Pool mice Post III - result using pooled mouse sera extracted post-immunisation. Pool rabbit preresult using pooled sera extracted from rabbits pre-innoculation. Pool rabbit Post III - result using pooled rabbit sera extracted post-immunisation. Blc- negative contol.

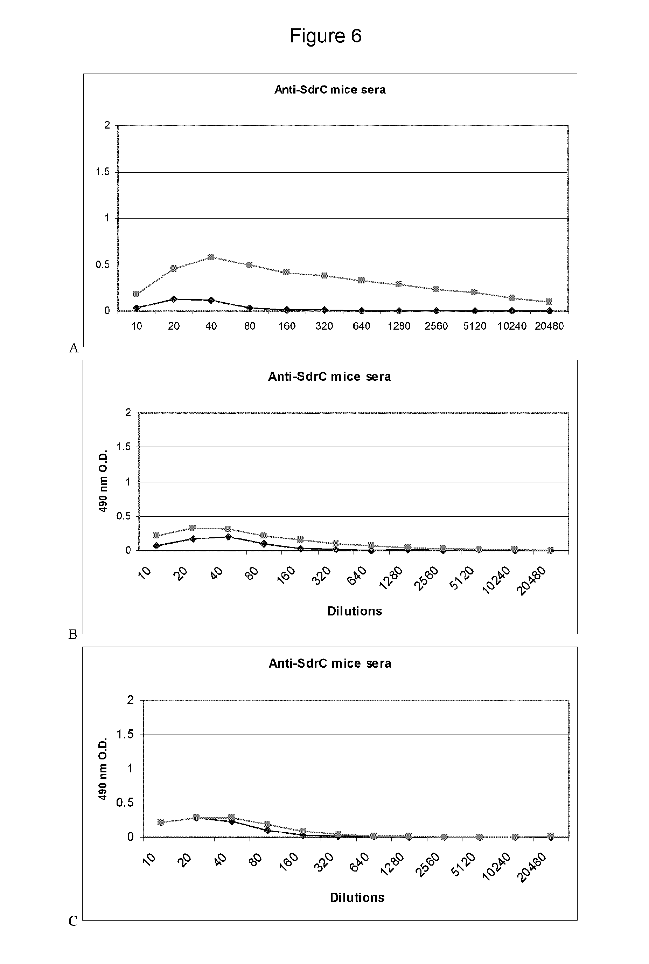

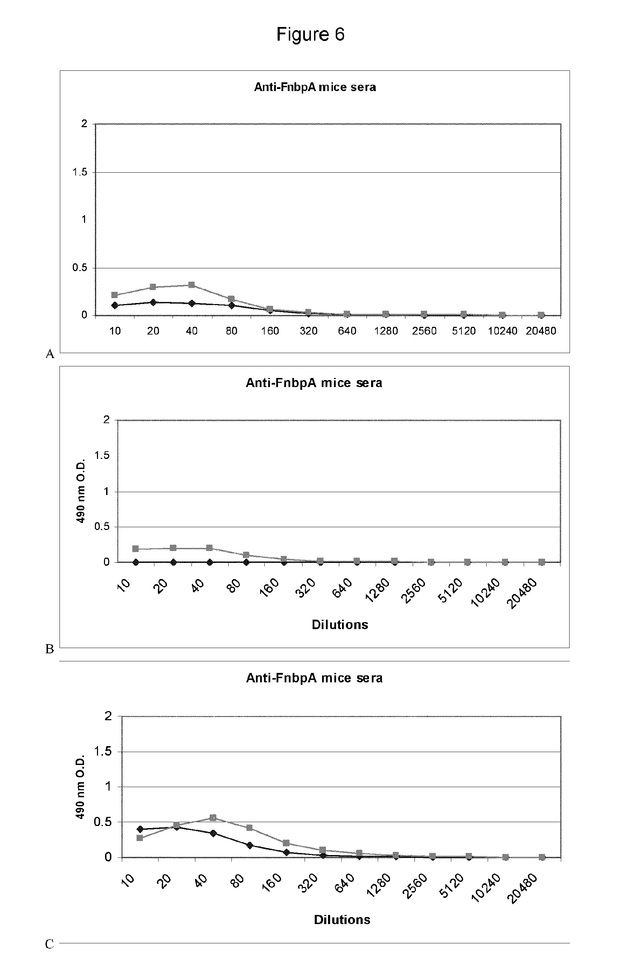

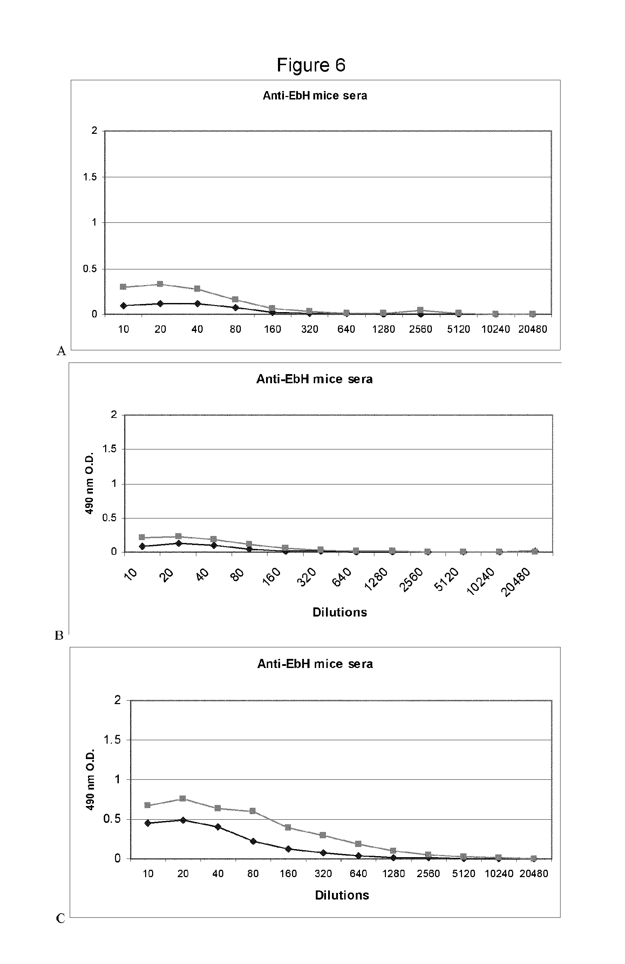

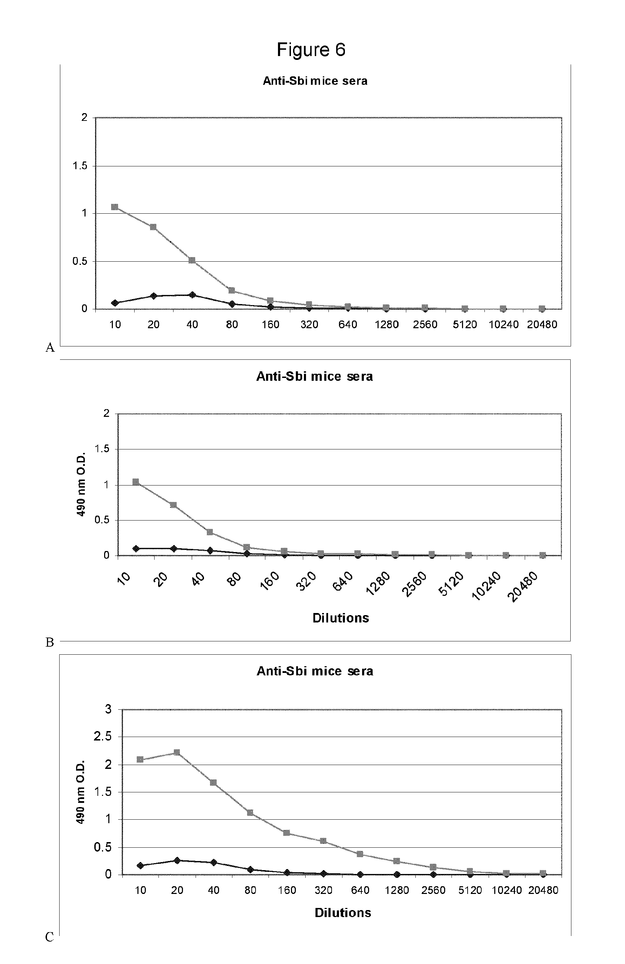

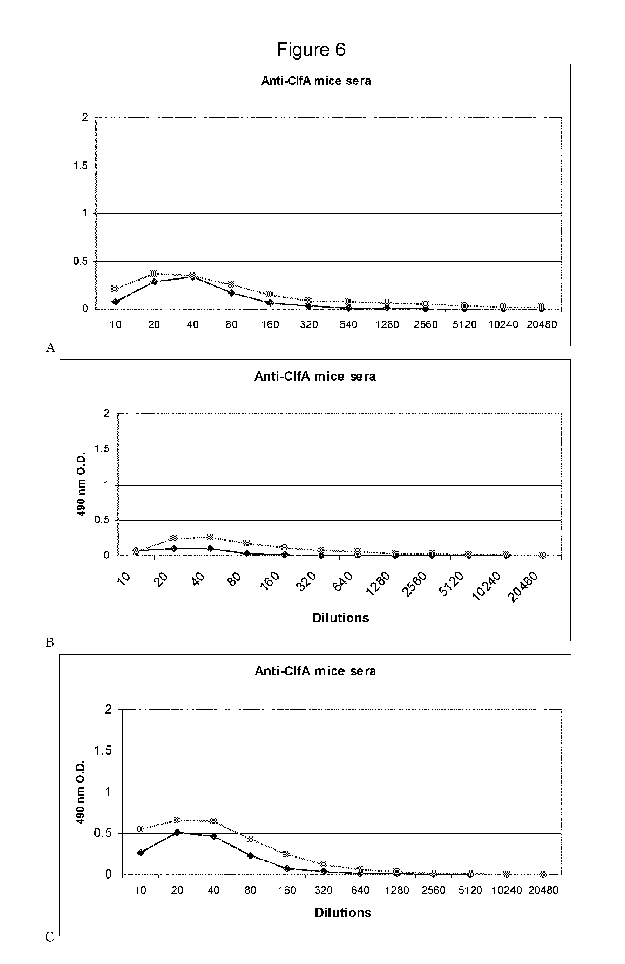

Figure 6 - ELISA results for mouse antisera raised against staphylococcal proteins in plates coated with killed staphylococci.
   Panel A uses plates coated with *S. aureus* serotype 5 killed whole cells. Panel B uses plates coated with *S. aureus* serotype 8 killed whole cells. Panel C uses plates coated with *S. epidermidis* killed whole cells.
   The line marked with square signs shows the ELISA result using antisera from mice immunised three times with the indicated staphylococcal protein. The line marked with diamond signs shows the ELISA result for pre-immune mouse sera.

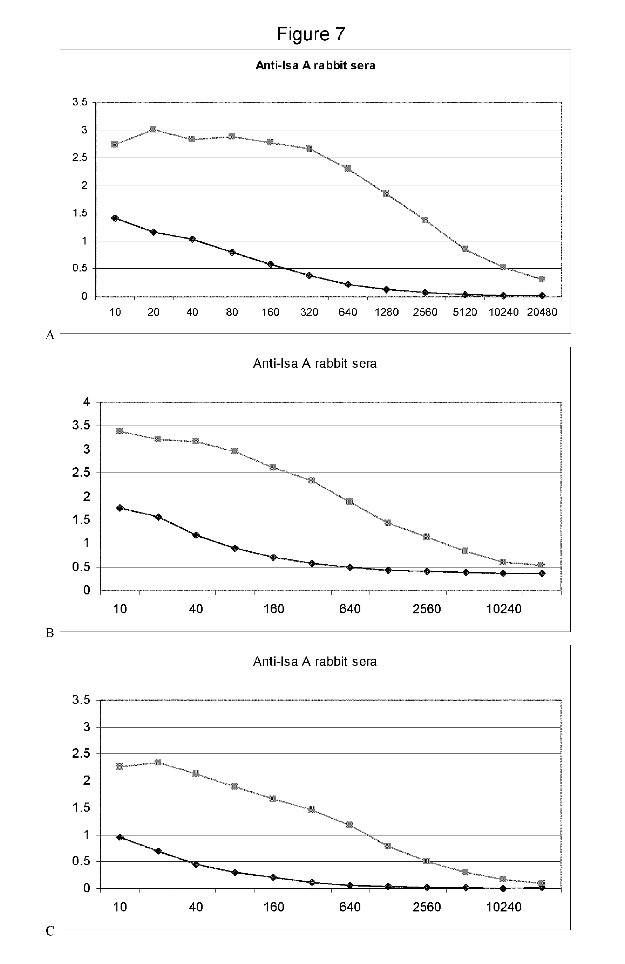

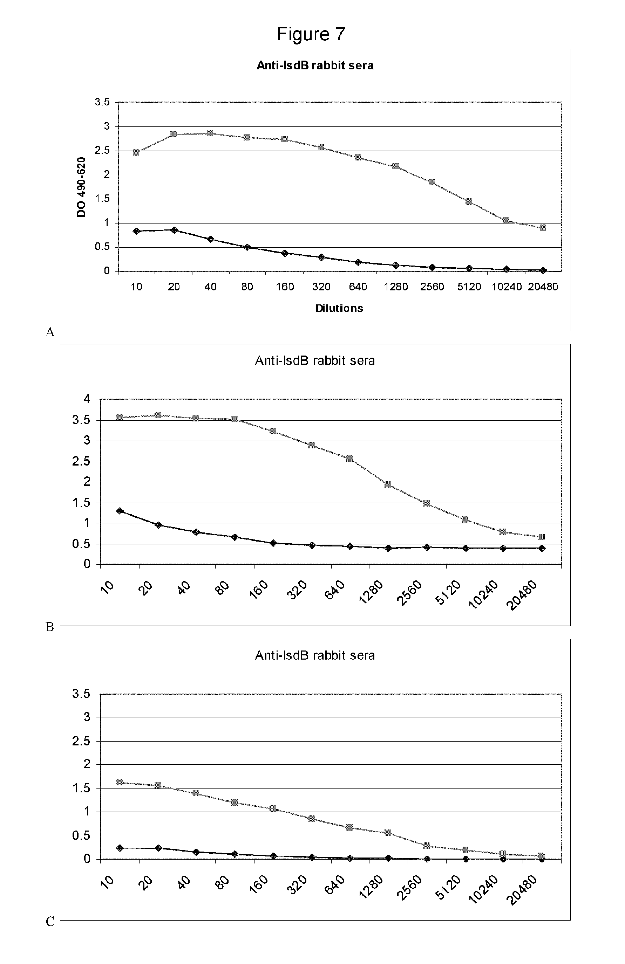

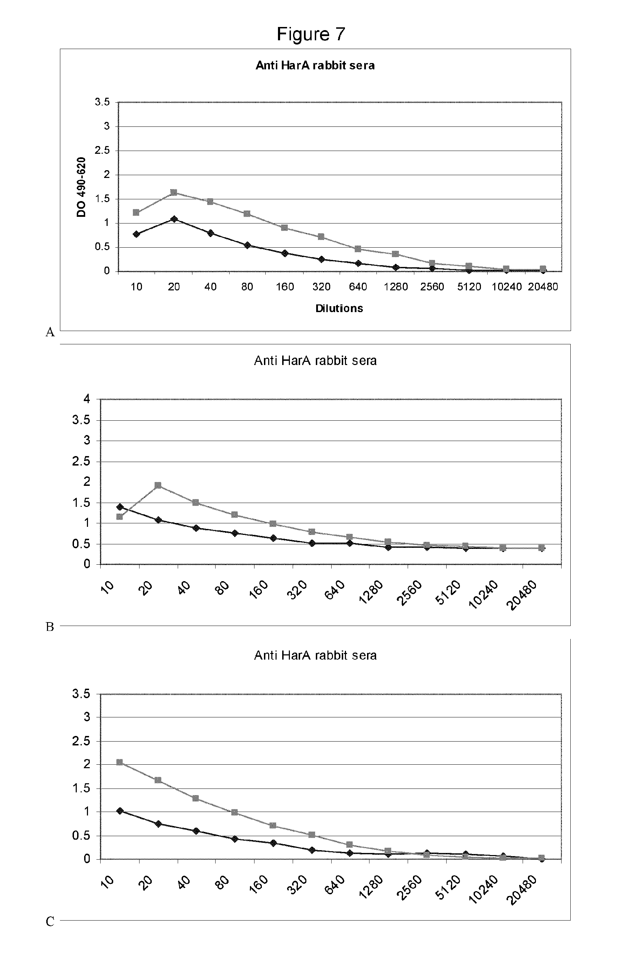

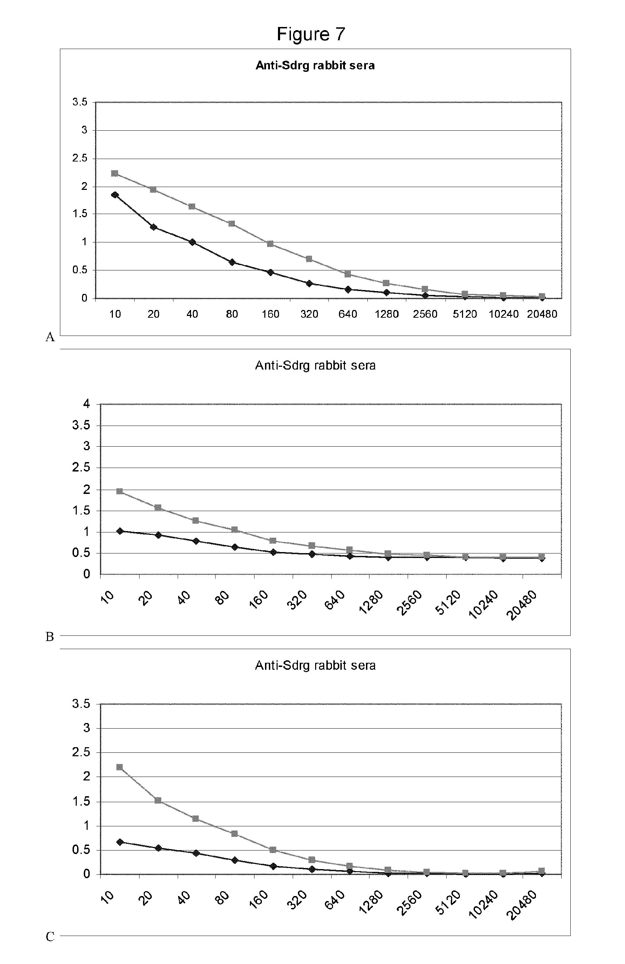

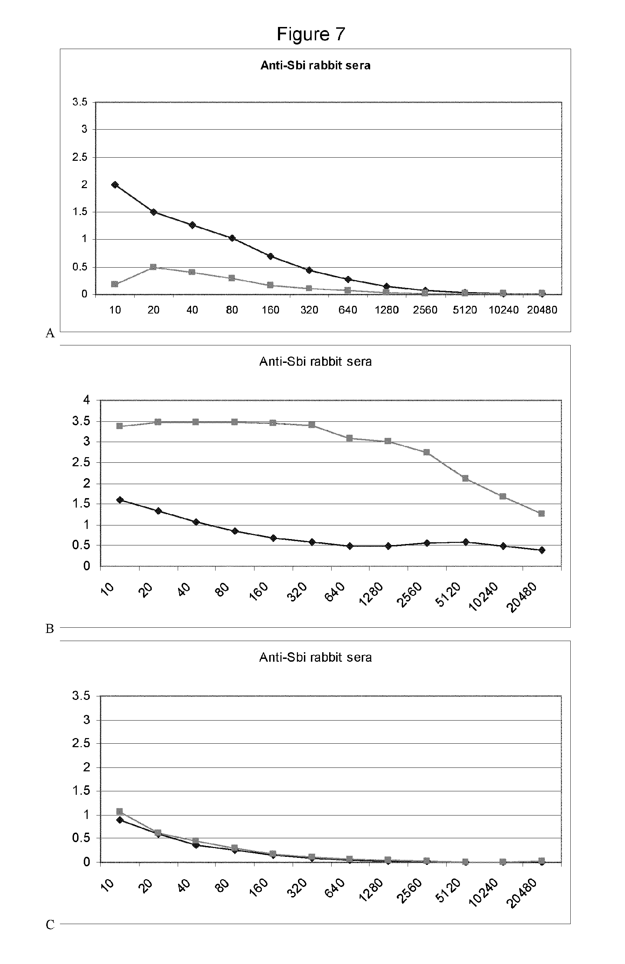

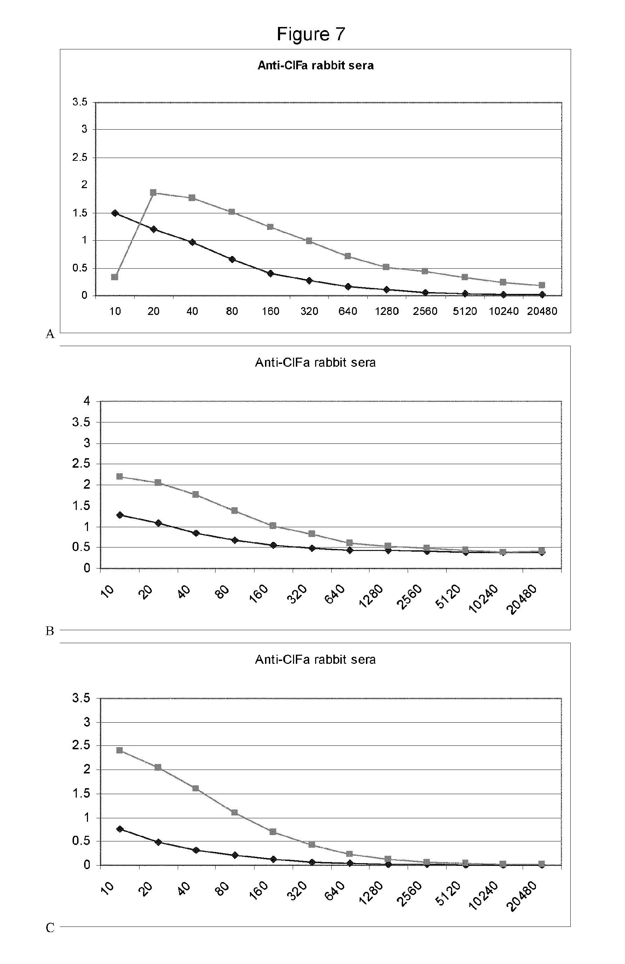

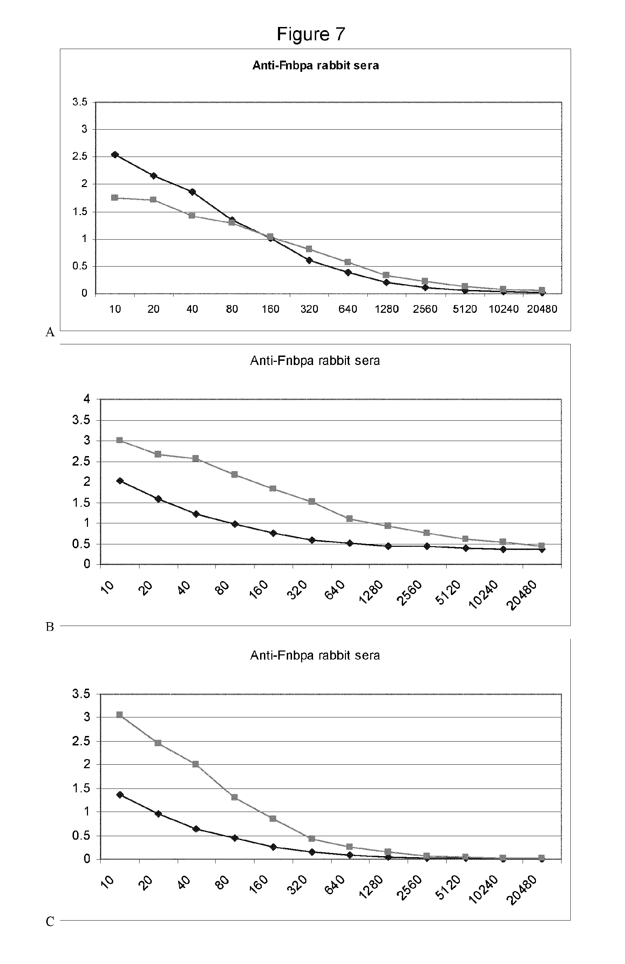

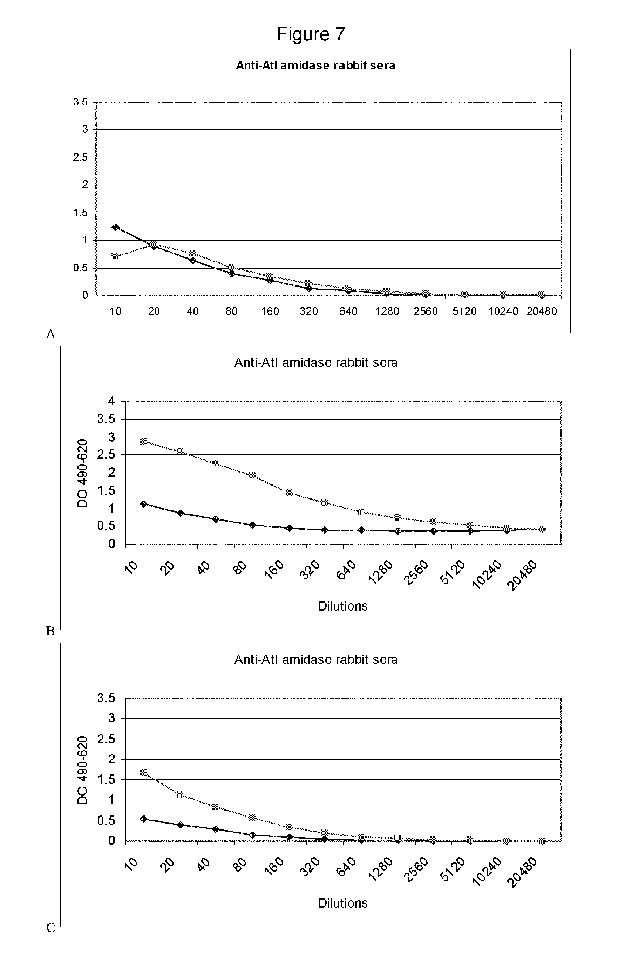

Figure 7 - ELISA results for rabbit antisera raised against staphylococcal proteins in plates coated with killed staphylococci.
   Panel A uses plates coated with *S. aureus* serotype 5 killed whole cells. Panel B uses plates coated with *S. aureus* serotype 8 killed whole cells. Panel C uses plates coated with *S. epidermidis* killed whole cells.
   The line marked with square signs shows the ELISA result using antisera from rabbits immunised three times with the indicated staphylococcal protein (except for HarA where only one immunisation was given). The line marked with diamond signs shows the ELISA result for pre-immune rabbit sera.

### Detailed description

The present invention discloses immunogenic compositions which comprise *S. aureus* polysaccharides Type 5 and/or 8 in which the Type 5 and/or Type 8 capsular polysaccharide or oligosaccharide is between 30% and 100% O-acetylated. In particular embodiments, the immunogenic composition of the invention additionally comprises PNAG or staphylococcal protein(s). PNAG is highly conserved among Gram positive bacteria and provides protection against a broad range of bacteria whereas Type 5 and 8 polysaccharides are potent immunogens that elicit an immune response against most strains of *S. aureus* which is the most common cause of nosocomial infection.

### Polysaccharides

The immunogenic compositions of the invention comprise PNAG and type 5 and 8 polysaccharides from *S. aureus* either or both of which are between 30% and 100% O-acetylated.

### Poly N-acetylated glucosamine (PNAG)

PNAG is a polysaccharide intercellular adhesin and is composed of a polymer of β-(1 →6)-linked glucosamine, optionally substituted with N-acetyl and/or O-succinyl constituents. This polysaccharide is present in both *S. aureus* and *S. epidermidis* and can be isolated from either source (Joyce et al 2003, Carbohydrate Research 338; 903; Maira-Litran et al 2002, Infect. Imun. 70; 4433). For example, PNAG may be isolated from *S*. *aureus* strain MN8m (WO 04/43407). The preparation of dPNAG is described in WO 04/43405.

The polysaccharide previously known as poly-N-succinyl-β-(1 →6)-glucosamine (PNSG) was recently shown not to have the expected structure since the identification of N-succinylation was incorrect (Maira-Litran et al 2002, Infect. Imun. 70; 4433). Therefore the polysaccharide formally known as PNSG and now found to be PNAG is also encompassed by the term PNAG.

PNAG may be of different sizes varying from over 400kDa to between 75 and 400kDa to between 10 and 75kDa to oligosaccharides composed of up to 30 repeat units (of β-(1 →6)-linked glucosamine, optionally substituted with N-acetyl and O-succinyl constituents). Any size of PNAG polysaccharide or oligosaccharide may be use in an immunogenic composition of the invention, for example a size of over 40kDa can be used. Sizing may be achieved by any method known in the art, for instance by microfluidisation, ultrasonic irradiation or by chemical cleavage (WO 03/53462, EP497524, EP497525).

Size ranges of PNAG are for example 40-400kDa, 50-350kDa, 40-300kDa, 60-300kDa, 50-250kDa and 60-200kDa.

PNAG can have different degree of acetylation due to substitution on the amino groups by acetate. PNAG produced in vitro is almost fully substituted on amino groups (95-100%). Alternatively, a deacetylated PNAG can be used having less than 50%, 40%, 30%, 20%, 10% or 5% N-acetylation. Use of a deacetylated PNAG allows opsonic killing of Gram positive bacteria, optionally *S. aureus* and/or *S. epidermidis* (WO 04/43405). In an embodiment, the PNAG has a size between 40kDa and 300kDa and is deacetylated so that less than 50%, 40%, 30%, 20%, 10% or 5% of amino groups are N acetylated.

In an embodiment, the PNAG is not O-succinylated or is O-succinylated on less than 25, 20, 15, 10, 5, 2 , 1 or 0.1% of residues.

The term deacetylated PNAG (dPNAG) refers to a PNAG polysaccharide or oligosaccharide in which less than 50%, 40%, 30%, 20%, 10% or 5% of the amino groups are acetylated.

As used herein, the term PNAG encompasses both acetylated and deacetylated forms of the saccharide.

In an embodiment, PNAG is deacetylated to form dPNAG, by chemically treating the native polysaccharide. For example, the native PNAG is treated with a basic solution such that the pH rises to above 10. For instance the PNAG is treated with 0.1-5M, 0.2-4M, 0.3-3M, 0.5-2M, 0.75-1.5M or 1M NaOH , KOH or NH4OH. Treatment is for at least 10 or 30 minutes, or 1, 2, 3, 4, 5, 10, 15 or 20 hours at a temperature of 20-100, 25-80, 30-60 or 30-50 or 35-45 °C. dPNAG may be prepared as described in WO 04/43405.

In an embodiment, the polysaccharide(s) included in the immunogenic composition of the invention are conjugated to a carrier protein as described below or alternatively unconjugated.

### Type 5 and Type 8 polysaccharides from S. aureus

Most strains of *S*. *aureus* that cause infection in man contain either Type 5 or Type 8 polysaccharides. Approximately 60% of human strains are Type 8 and approximately 30% are Type 5. The structures of Type 5 and Type 8 capsular polysaccharide antigens are described in Moreau et al Carbohydrate Res. 201; 285 (1990) and Fournier et al Infect. Immun. 45; 87 (1984). Both have FucNAcp in their repeat unit as well as ManNAcA which can be used to introduce a sulfhydryl group.

Recently (Jones Carbohydrate Research 340, 1097-1106 (2005)) NMR spectroscopy revised the structures of the capsular polysaccharides to:
Type 5
   → 4)-β-D-ManNAcA-(1 →4)-α-L-FucNAc(3OAc)-(1 →3)-β-D-FucNAc-(1 →
Type 8
   → 3)-β-D-ManNAcA(4OAc)-(1 →3)-α-L-FucNAc(1 →3)-α-D-FucNAc(1 →

Polysaccharides may be extracted from the appropriate strain of *S. aureus* using methods well known to the skilled man, for instance as described in US6294177 or Infection and Immunity (1990) 58(7); 2367. For example, ATCC 12902 is a Type 5 *S. aureus* strain and ATCC 12605 is a Type 8 *S. aureus* strain.

Polysaccharides are of native size or alternatively may be sized, for instance by microfluidisation, ultrasonic irradiation or by chemical treatment. The invention also covers oligosaccharides derived from the type 5 and 8 polysaccharides from *S. aureus.*

The type 5 and/or 8 capsular polysaccharide or oligosaccharides included in the immunogenic composition of the invention are O-acetylated. In an embodiment, the degree of O-acetylation of type 5 capsular polysaccharide or oligosaccharide is 10-100%, 20-100%, 30-100%, 40-100%, 50-100%. 60-100%, 70-100%, 80-100%, 90-100%, 50-90%, 60-90%, 70-90% or 80-90%. In an embodiment, the degree of O-acetylation of type 8 capsular polysaccharide or oligosaccharide is 10-100%, 20-100%, 30-100%, 40-100%, 50-100%. 60-100%, 70-100%, 80-100%, 90-100%, 50-90%, 60-90%, 70-90% or 80-90%. In an embodiment, the degree of O-acetylation of type 5 and type 8 capsular polysaccharides or oligosaccharides is 10-100%, 20-100%, 30-100%, 40-100%, 50-100%. 60-100%, 70-100%, 80-100%, 90-100%, 50-90%, 60-90%, 70-90% or 80-90%.

The degree of O-acetylation of the polysaccharide or oligosaccharide can be determined by any method known in the art, for example, by proton NMR ( Lemercinier and Jones 1996, Carbohydrate Resarch 296; 83-96, Jones and Lemercinier 2002, J Pharmaceutical and Biomedical analysis 30; 1233-1247, WO 05/033148 or WO 00/56357). A further commently used method is that described by Hestrin (1949) J. Biol. Chem. 180; 249-261. O-acetyl groups can be removed by hydrolysis, for example by treatment with a base such as anhydrous hydrazine (Konadu et al 1994; Infect. Immun. 62; 5048-5054) or treatment with 0.1N NaOH for 1-8 hours. In order to maintain high levels of O-acetylation on type 5 and/or 8 polysaccharide or oligosaccharide, treatments which would lead to hydrolysis of the O-acetyl groups are minimised. For example treatment at extremes of pH are minimised.

The type 5 and 8 polysaccharides included in the immunogenic composition of the invention are optionally conjugated to a carrier protein as described below or are alternatively unconjugated.

The immunogenic compositions of the invention alternatively contains either type 5 or type 8 polysaccharide.

### S. aureus 336 antigen

In an embodiment, the immunogenic composition of the invention comprises the *S*. *aureus* 336 antigen described in US6294177.

The 336 antigen comprises β-linked hexosamine, contains no O-acetyl groups and specifically binds to antibodies to *S*. *aureus* Type 336 deposited under ATCC 55804.

In an embodiment, the 336 antigen is a polysaccharide which is of native size or alternatively may be sized, for instance by microfluidisation, ultrasonic irradiation or by chemical treatment. The invention also covers oligosaccharides derived from the 336 antigen.

The 336 antigen, where included in the immunogenic composition of the invention is optionally conjugated to a carrier protein as described below or are alternatively unconjugated.

### Type I, II and III polysaccharides from S. epidermidis

Strains ATCC-31432, SE-360 and SE-10 of *S. epidermidis* are characteristic of three different capsular types, I, II and III respectively (Ichiman and Yoshida 1981, J. Appl. Bacteriol. 51; 229). Capsular polysaccharides extracted from each serotype of *S. epidermidis* constitute Type I, II and III polysaccharides. Polysaccharides may be extracted by serval methods including the method described in US4197290 or as described in Ichiman et al 1991, J. Appl. Bacteriol. 71; 176.

In one embodiment of the invention, the immunogenic composition comprises type I and/or II and/or III polysaccharides or oligosaccharides from *S. epidermidis.*

Polysaccharides are of native size or alternatively may be sized, for instance by microfluidisation, ultrasonic irradiation or chemical cleavage. The invention also covers oligosaccharides extracted from *S. epidermidis* strains.

These polysaccharides are unconjugated or are optionally conjugated as described below.

### Conjugation of polysaccharides

Amongst the problems associated with the use of polysaccharides in vaccination, is the fact that polysaccharides *per* se are poor immunogens. Strategies, which have been designed to overcome this lack of immunogenicity, include the linking of the polysaccharide to large protein carriers, which provide bystander T-cell help. In an embodiment, the polysaccharides utilised in the invention are linked to a protein carrier which provide bystander T -cell help. Examples of these carriers which may be used for coupling to polysaccharide or oligosaccharide immunogens include the Diphtheria and Tetanus toxoids (DT, DT Crm197 and TT), Keyhole Limpet Haemocyanin (KLH), *Pseudomonas aeruginosa* exoprotein A (rEPA) and the purified protein derivative of Tuberculin (PPD), protein D from *Haemophilus influenzae*, pneumolysin or fragments of any of the above. Fragments suitable for use include fragments encompassing T-helper epitopes. In particular protein D fragment will optionally contain the N-terminal 1/3 of the protein. Protein D is an IgD-binding protein from *Haemophilus influenzae* (EP 0 594 610 B1).

An alternative carrier protein to use in the immunogenic composition of the invention is a single staphylococcal protein or fragment thereof or a fusion protein comprising at least or exactly 1, 2, 3 or 4 or more of the staphylococcal proteins or fragments thereof listed in the section below.

A new carrier protein that would be particularly advantageous to use in the context of a staphylococcal vaccine is staphylococcal alpha toxoid. The native form may be conjugated to a polysaccharide since the process of conjugation reduces toxicity. Optionally a genetically detoxified alpha toxin such as the His35Leu or His 35 Arg variants are used as carriers since residual toxicity is lower. Alternatively the alpha toxin is chemically detoxified by treatment with a cross-linking reagent, formaldehyde or glutaraldehyde. A genetically detoxified alpha toxin is optionally chemically detoxified, optionally by treatment with a cross-linking reagent, formaldehyde or glutaraldehyde to further reduce toxicity.

The polysaccharides may be linked to the carrier protein(s) by any known method (for example, by Likhite, U.S. Patent 4,372,945 by Armor et al., U.S. Patent 4,474,757, WO and Jennings et al., U.S. Patent 4,356,170). Optionally, CDAP conjugation chemistry is carried out (see WO95/08348).

In CDAP, the cyanylating reagent 1-cyano-dimethylaminopyridinium tetrafluoroborate (CDAP) is optionally used for the synthesis of polysaccharide-protein conjugates. The cyanilation reaction can be performed under relatively mild conditions, which avoids hydrolysis of the alkaline sensitive polysaccharides. This synthesis allows direct coupling to a carrier protein.

The polysaccharide may be solubilized in water or a saline solution. CDAP may be dissolved in acetonitrile and added immediately to the polysaccharide solution. The CDAP reacts with the hydroxyl groups of the polysaccharide to form a cyanate ester. After the activation step, the carrier protein is added. Amino groups of lysine react with the activated polysaccharide to form an isourea covalent link. After the coupling reaction, a large excess of glycine is then added to quench residual activated functional groups. The product is then passed through a gel permeation column to remove unreacted carrier protein and residual reagents.

### Proteins

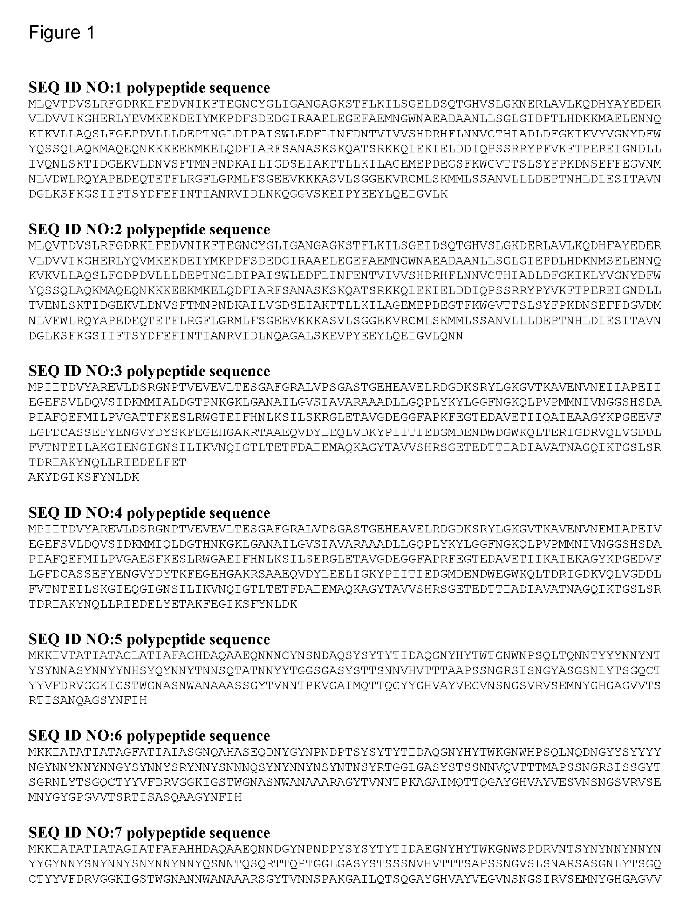

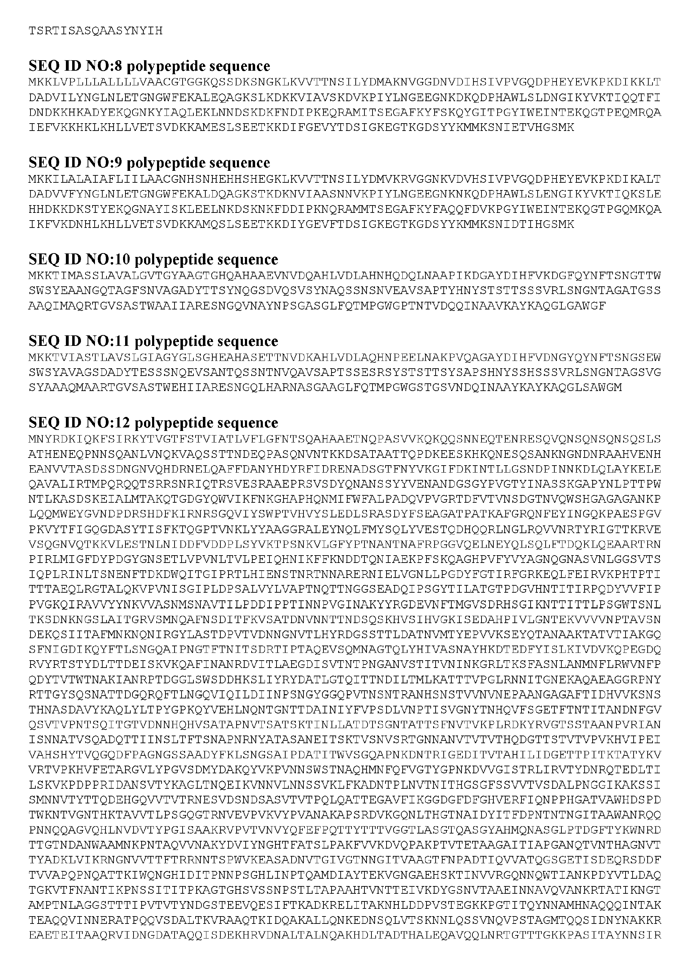

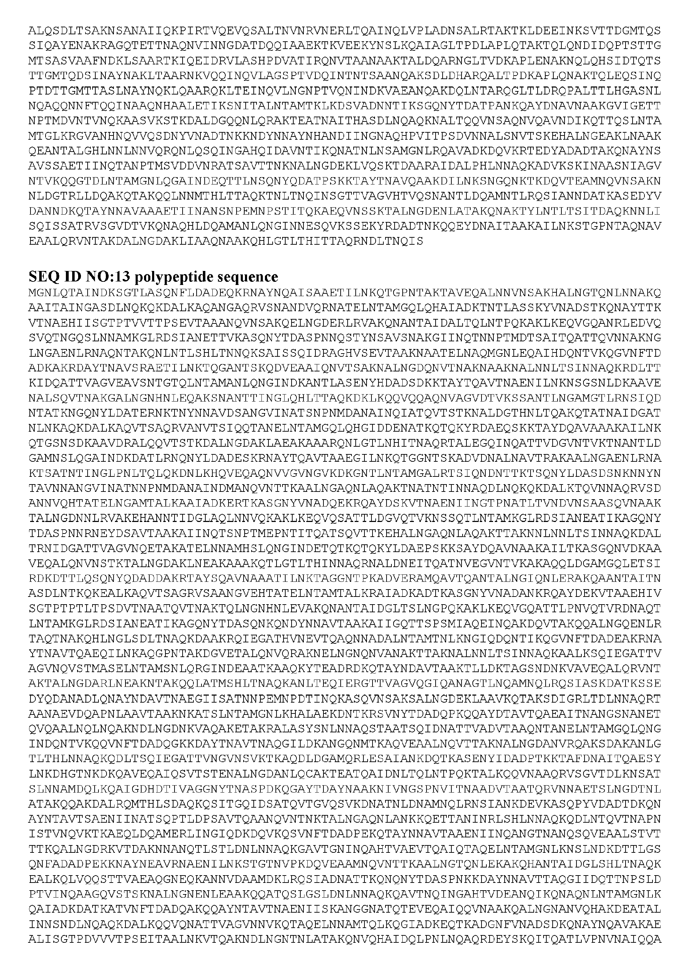

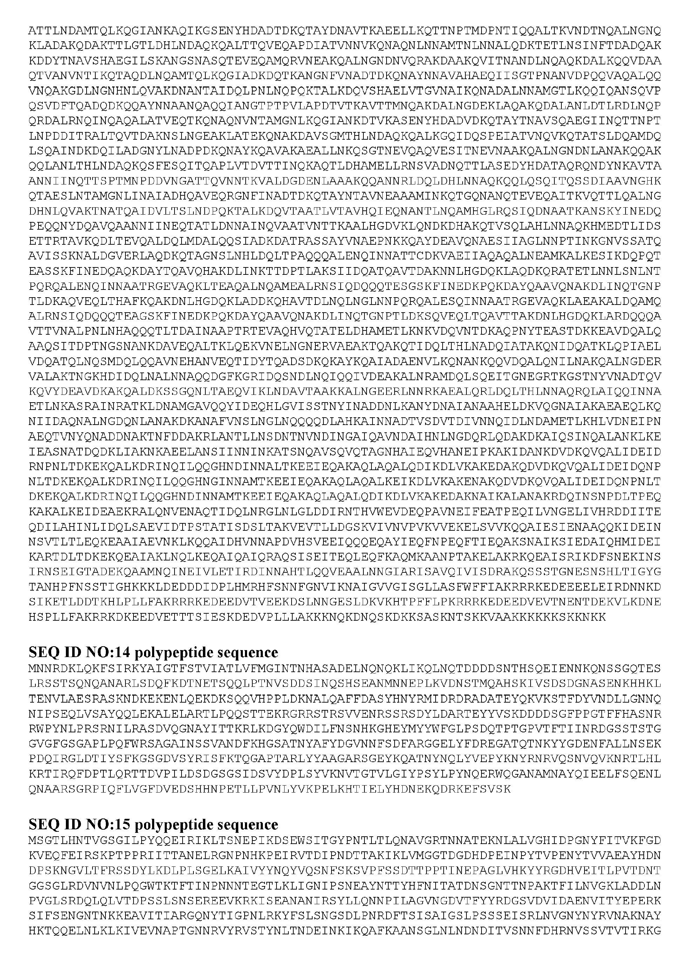

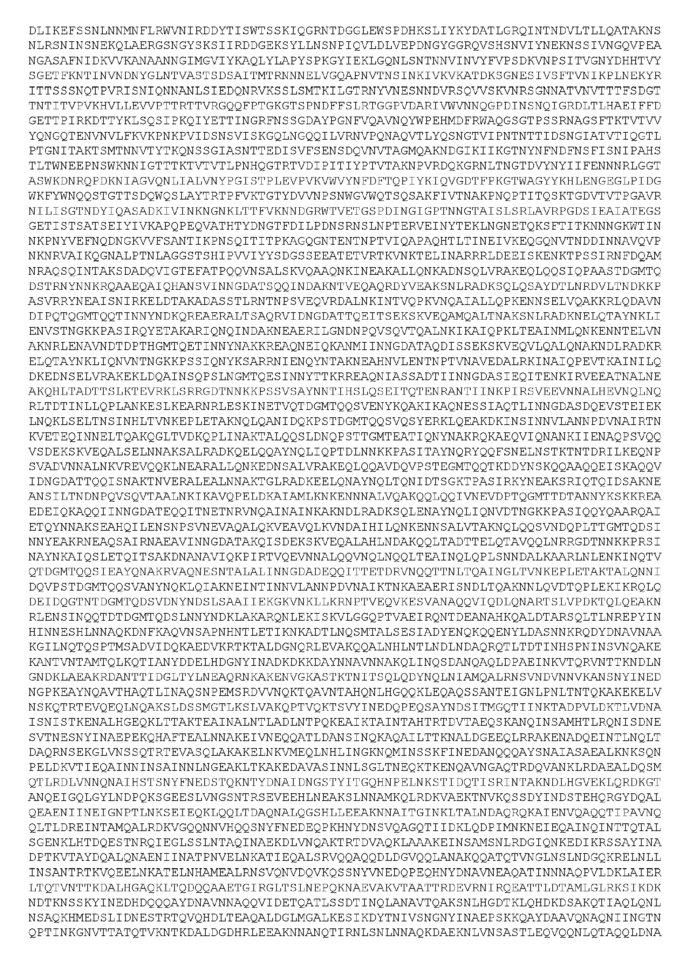

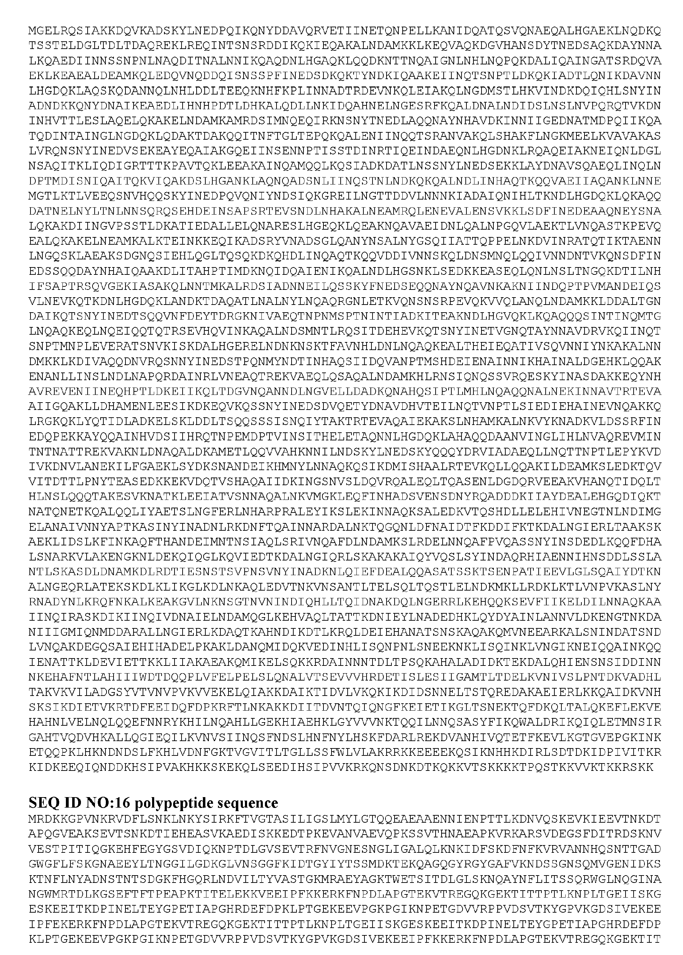

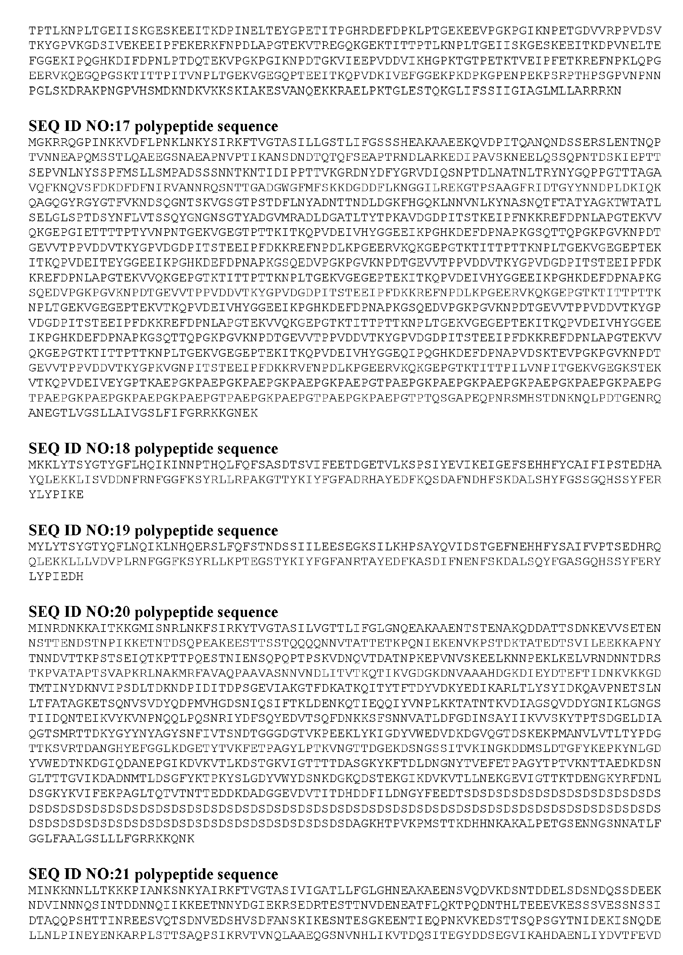

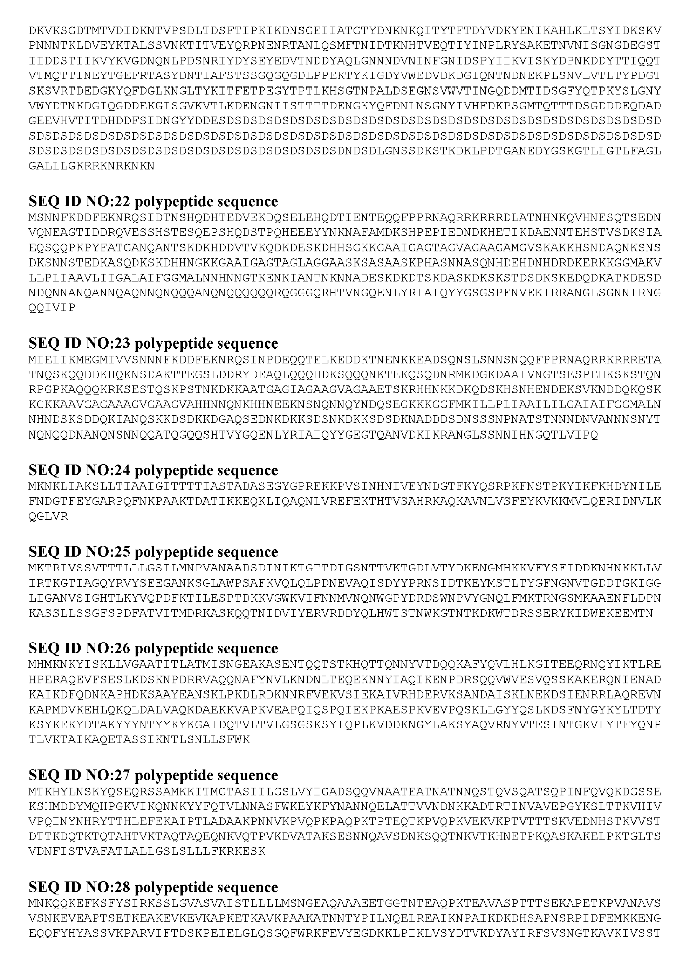

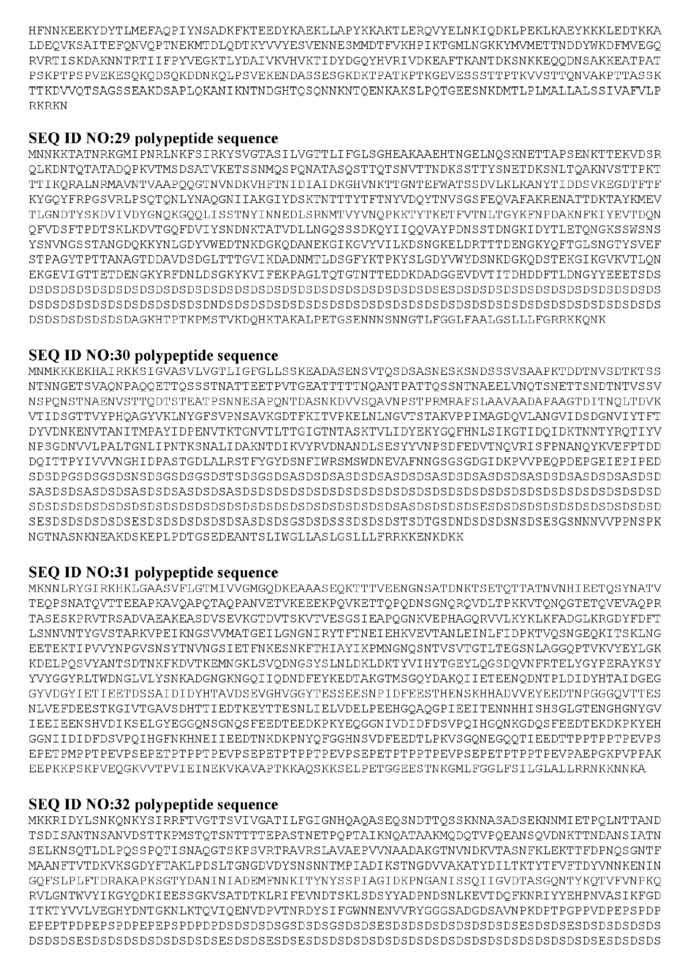

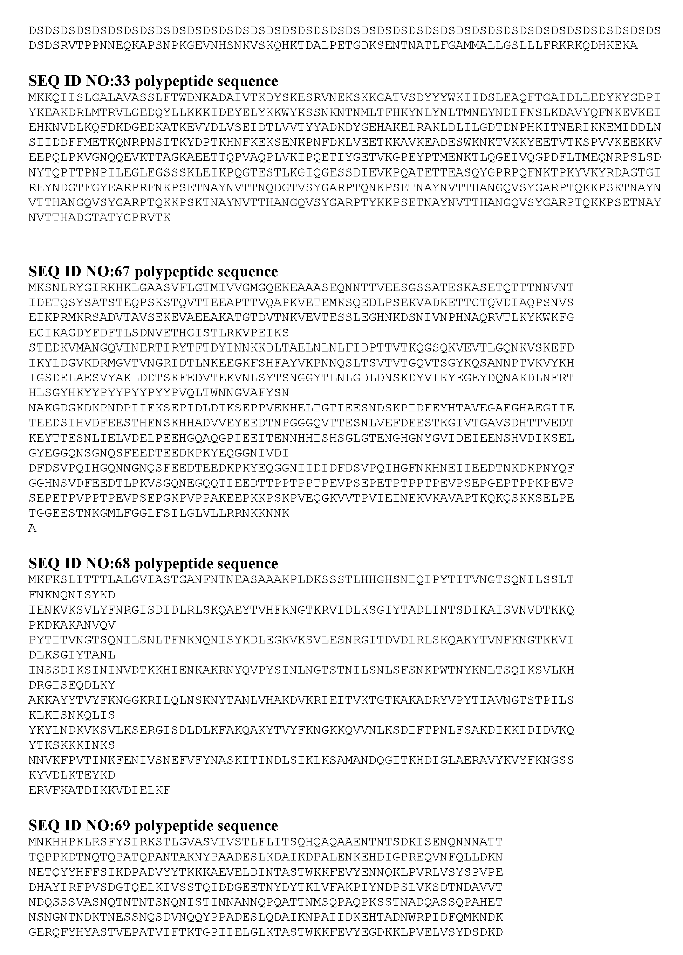

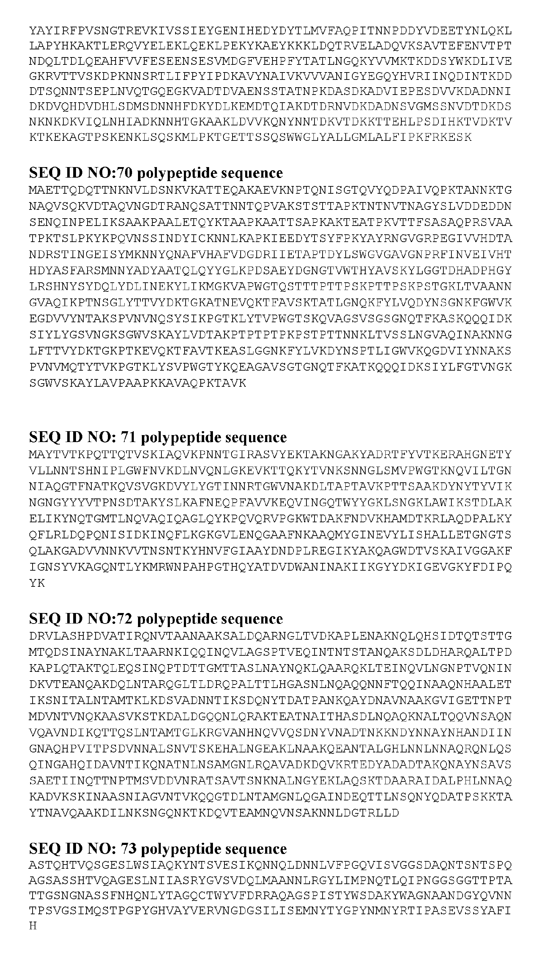

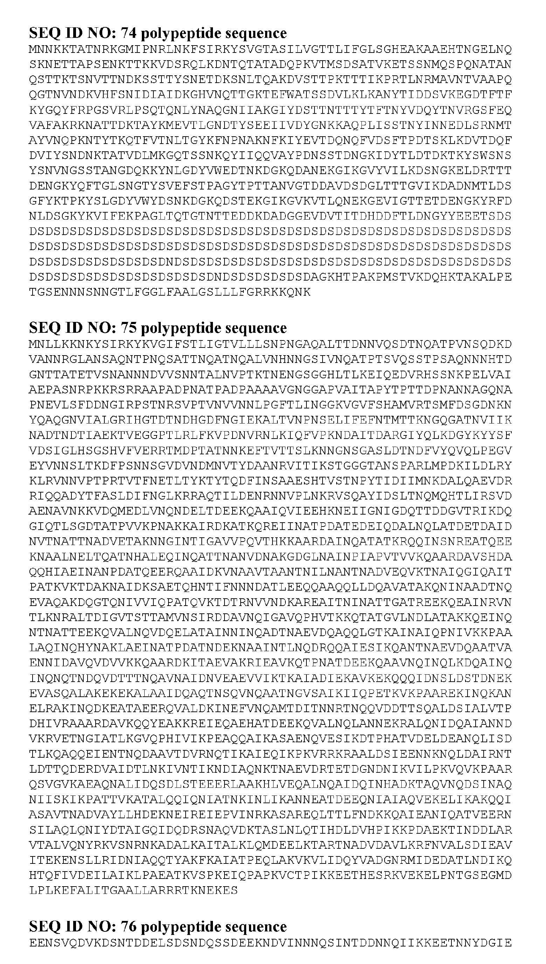

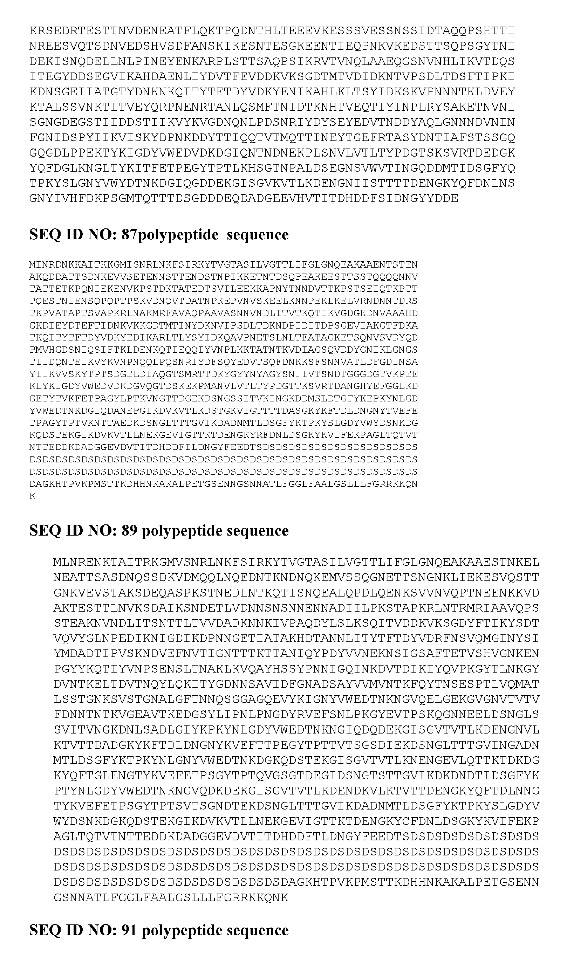

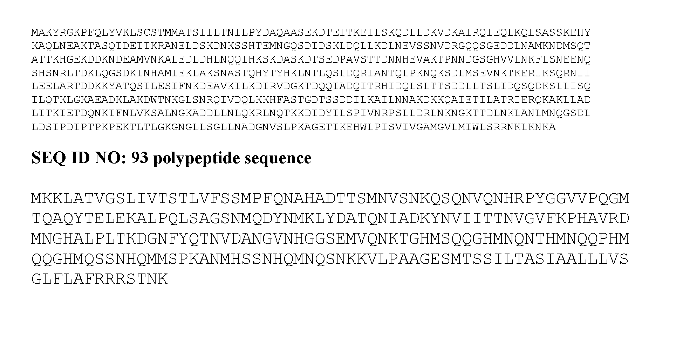

The immunogenic composition of the invention optionally further comprises a staphylococcal protein, for example a protein from *S. aureus* or *S. epidermidis.* Some embodiments of the invention contain proteins from both *S. aureus* and *S. epidermidis.* Immunogenic compositions of the invention comprise an isolated protein which comprises an amino acid sequence which has at least 85% identity, optionally at least 90% identity, at least 95% identity, at least 97-99% or exact identity, to that of any sequence of figure 1.

Where a protein is specifically mentioned herein, it is optionally a reference to a native or recombinant , full-length protein or optionally a mature protein in which any signal sequence has been removed. The protein may be isolated directly from the staphylococcal strain or produced by recombinant DNA techniques. Immunogenic fragments of the protein may be incorporated into the immunogenic composition of the invention. These are fragments comprising at least 10 amino acids, at least 20 amino acids, at least 30 amino acids, at least 40 amino acids, at least 50 amino acids or at least 100 amino acids, taken contiguously from the amino acid sequence of the protein. In addition, such immunogenic fragments are typically immunologically reactive with antibodies generated against the Staphylococcal proteins or with antibodies generated by infection of a mammalian host with Staphylococci or contain T cell epitopes. In an embodiment, immunogenic fragments also includes fragments that when administered at an effective dose, (either alone or as a hapten bound to a carrier), elicit a protective immune response against Staphylococcal infection, optionally it is protective against *S. aureus* and/or *S. epidermidis* infection. Such an immunogenic fragment may include, for example, the protein lacking an N-terminal leader sequence, and/or a transmembrane domain and/or a C-terminal anchor domain. In an embodiment, the immunogenic fragment according to the invention comprises substantially all of the extracellular domain of a protein which has at least 85%, 90%, 95%, 97% or 99% identity, to that a sequence selected from Figure 1 over the entire length of the fragment sequence.

In an embodiment, immunogenic compositions of the invention may contain fusion proteins of Staphylococcal proteins, or fragments of staphylococcal proteins. Such fusion proteins may be made recombinantly and may comprise one portion of at least 2, 3, 4, 5 or 6 staphylococcal proteins, for example the combinations of staphylococcal proteins listed below. Alternatively, a fusion protein may comprise multiple portions of at least 2, 3, 4 or 5 staphylococcal proteins. These may combine different Staphylococcal proteins or fragments thereof in the same protein. Alternatively, the invention also includes individual fusion proteins of Staphylococcal proteins or fragments thereof, as a fusion protein with heterologous sequences such as a provider of T-cell epitopes or purification tags, for example: β-galactosidase, glutathione-S-transferase, green fluorescent proteins (GFP), epitope tags such as FLAG, myc tag, poly histidine, or viral surface proteins such as influenza virus haemagglutinin, or bacterial proteins such as tetanus toxoid, diphtheria toxoid, CRM197. The fusion protein may be present in the immunogenic composition of the invention as a free protein or it may be a carrier protein linked to a saccharide.

### Proteins

In an embodiment, the immunogenic composition of the invention further comprises one or more of the proteins mentioned below or immunogenic fragments thereof. Many of the proteins fall into the categories of extracellular component binding proteins, transporter proteins or toxins and regulators of virulence. The immunogenic composition of the invention optionally further comprises a staphylococcal extracellular component binding protein or a staphylococcal transporter protein or a staphylococcal toxin or regulator of virulence. The immunogenic composition of the invention optionally comprises at least or exactly 1, 2, 3, 4, 5 or 6 staphylococcal proteins.

### Table 1

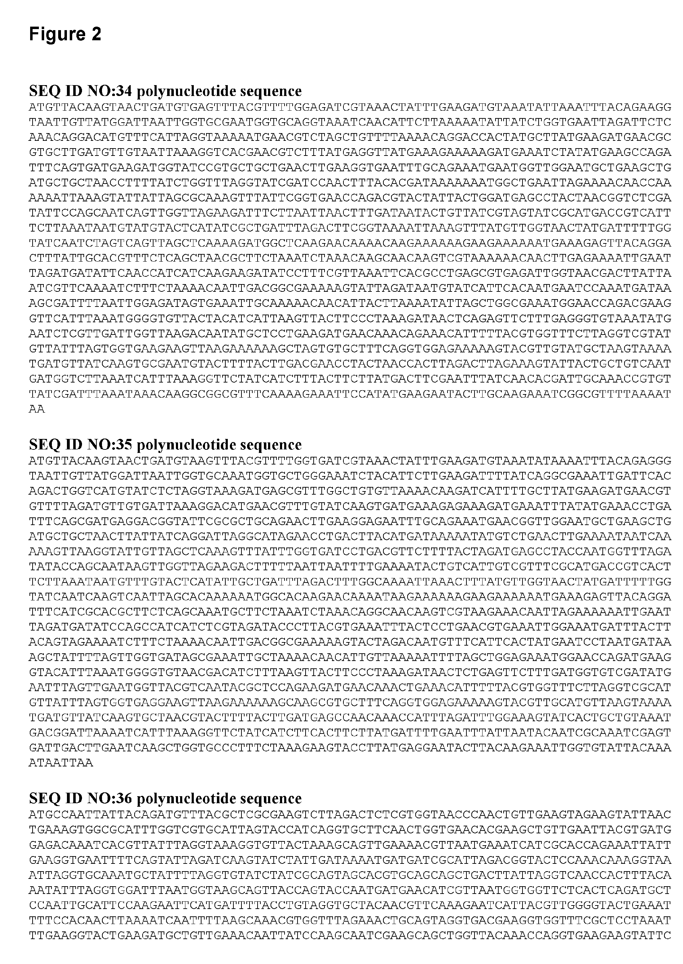

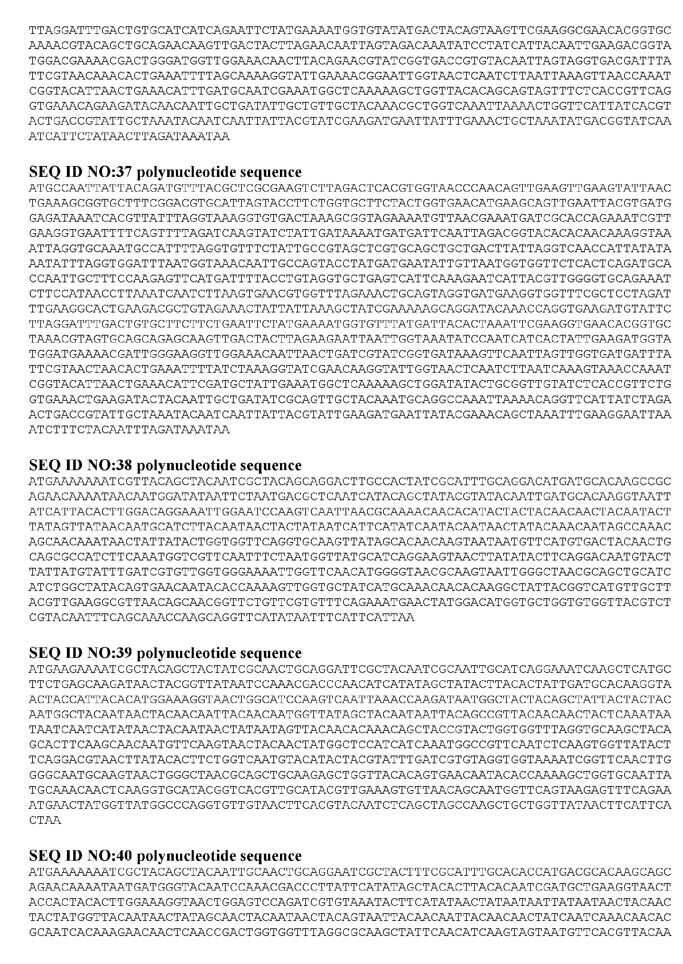

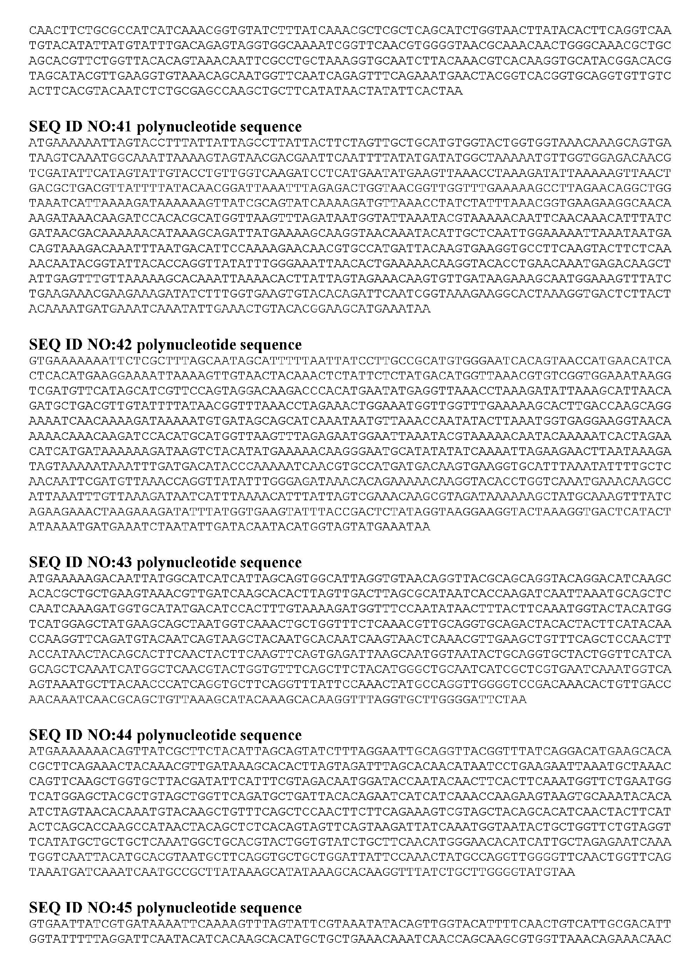

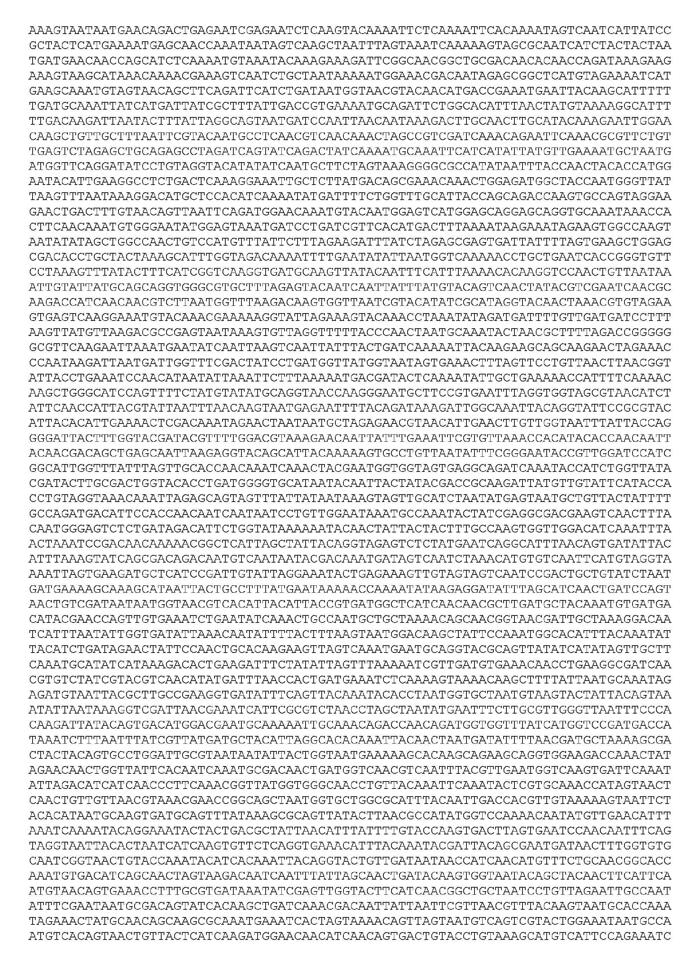

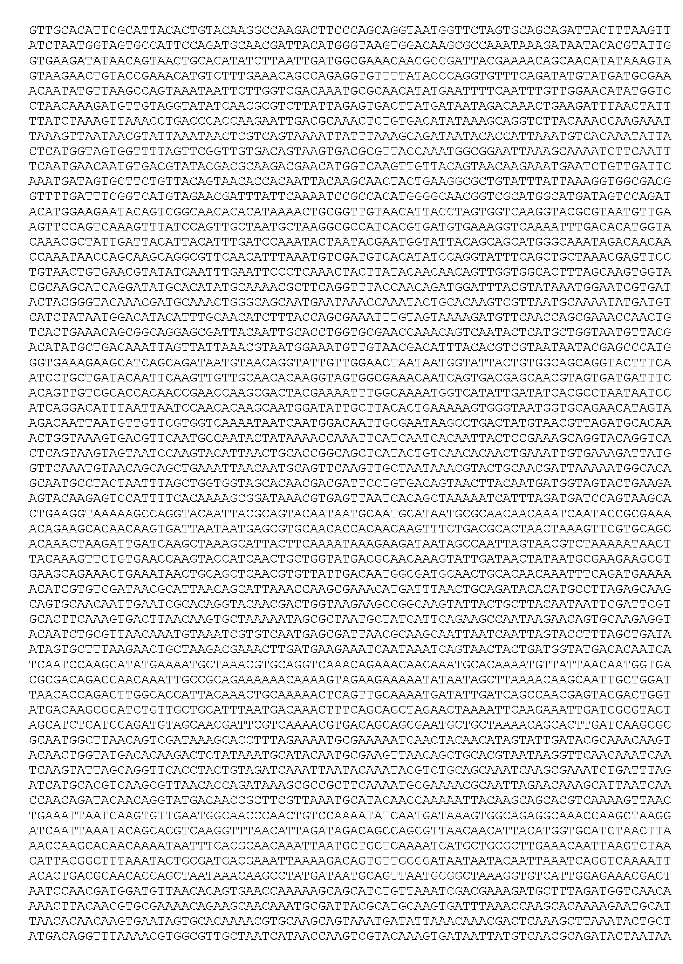

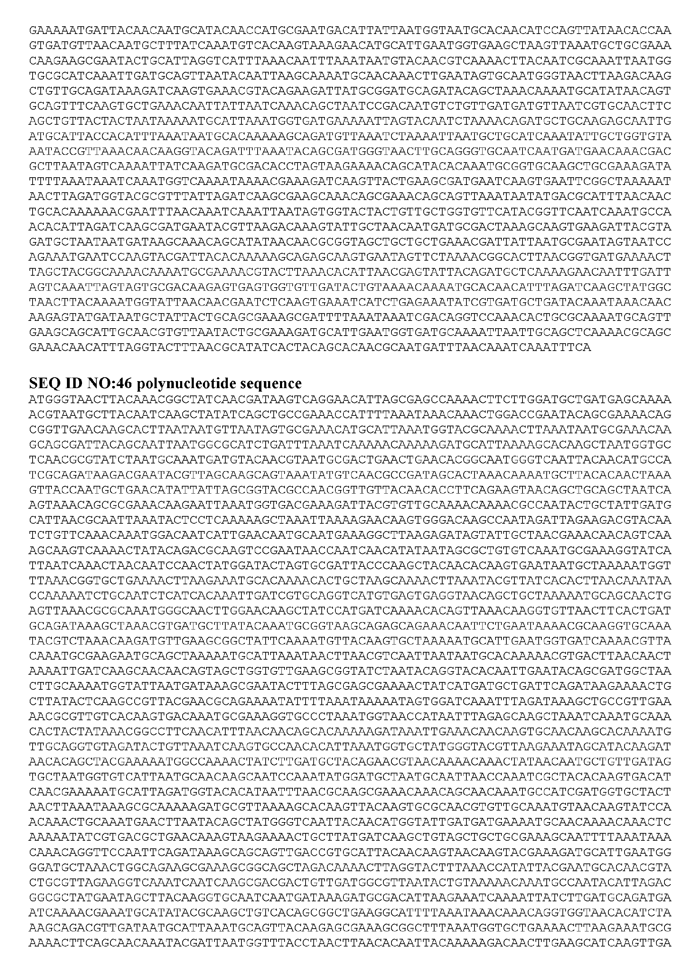

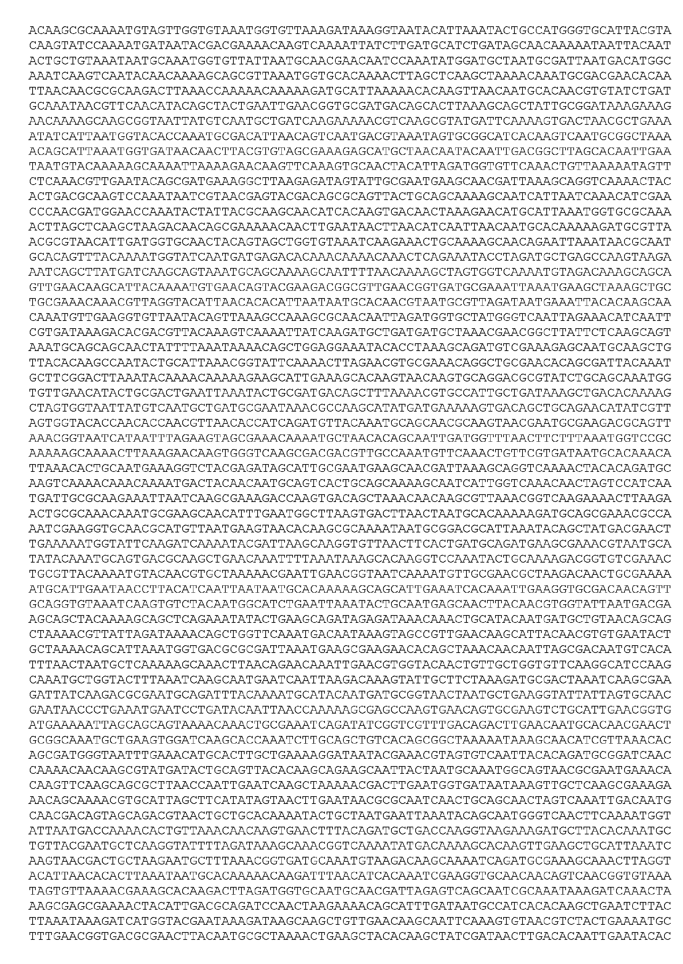

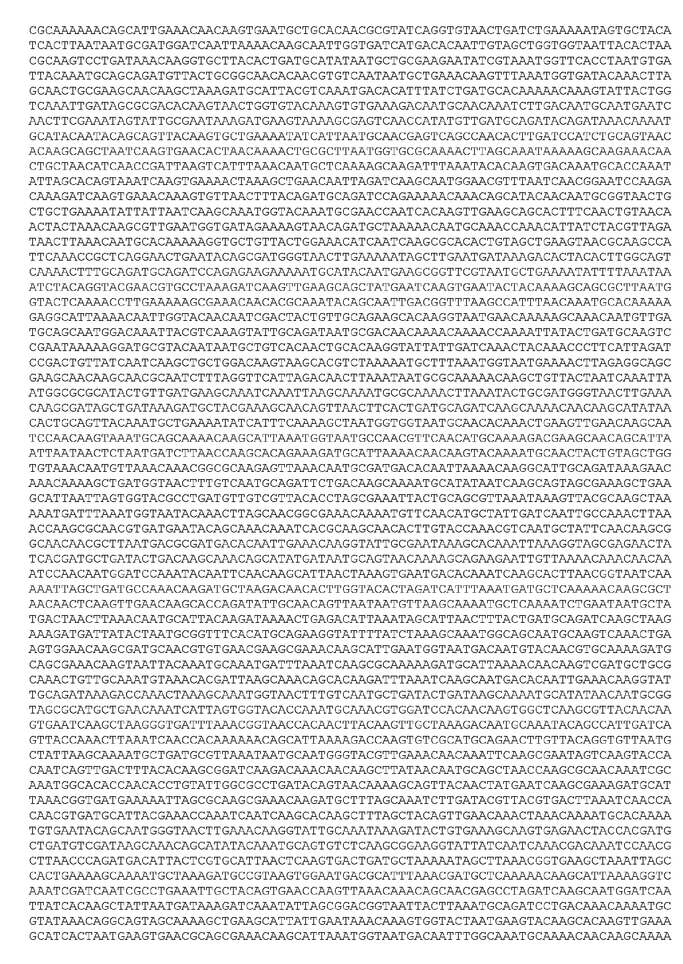

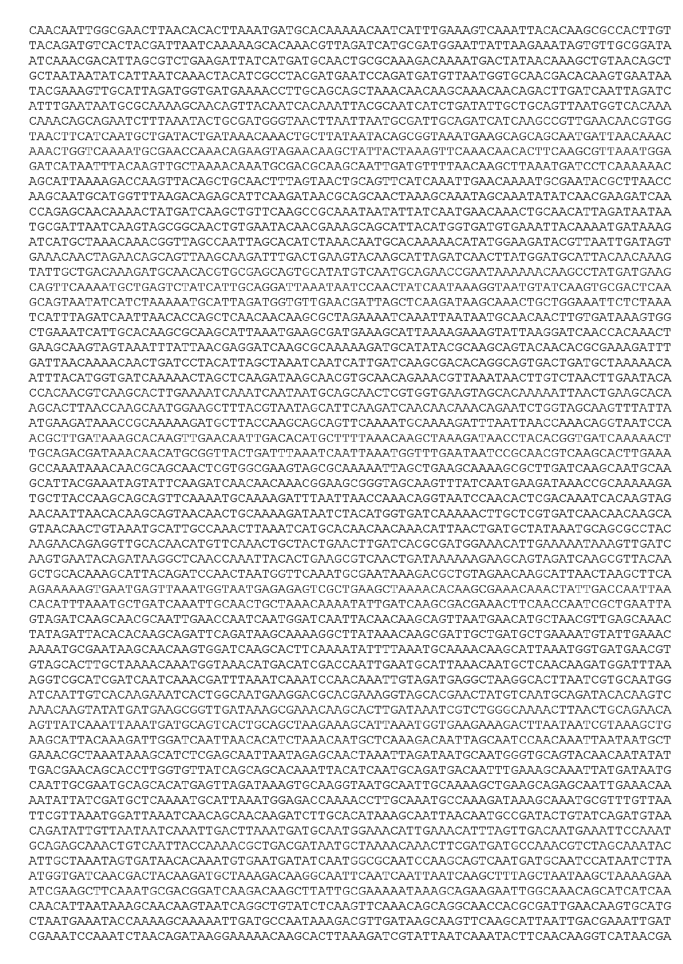

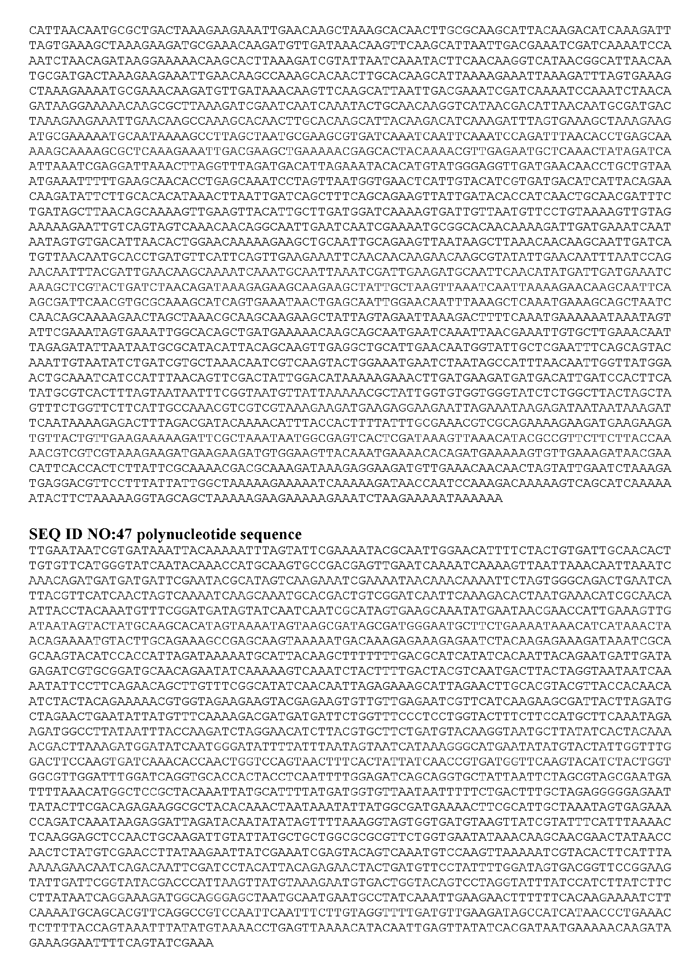

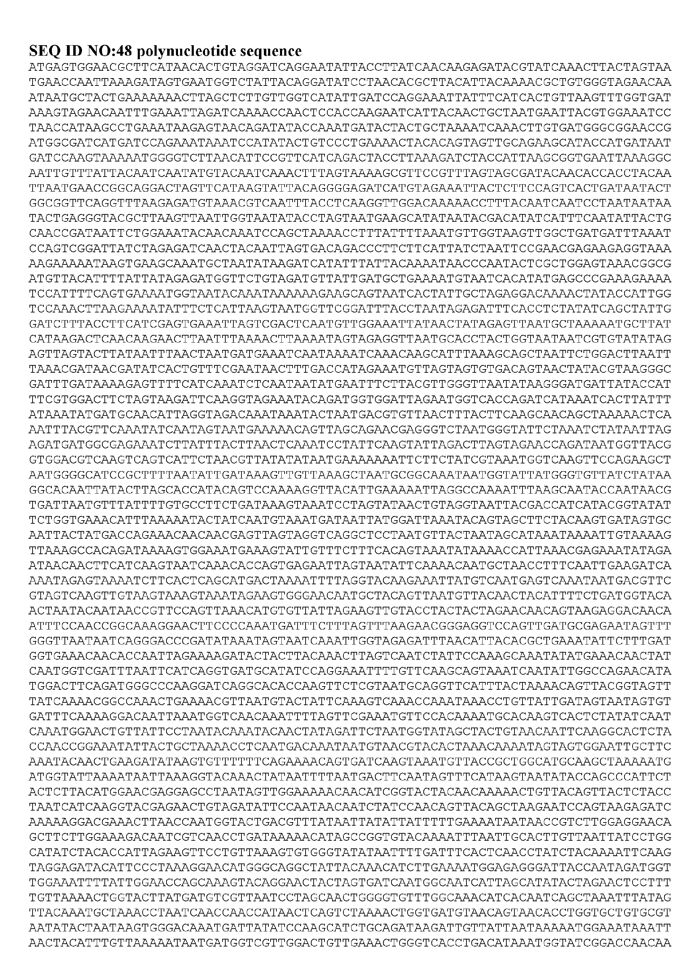

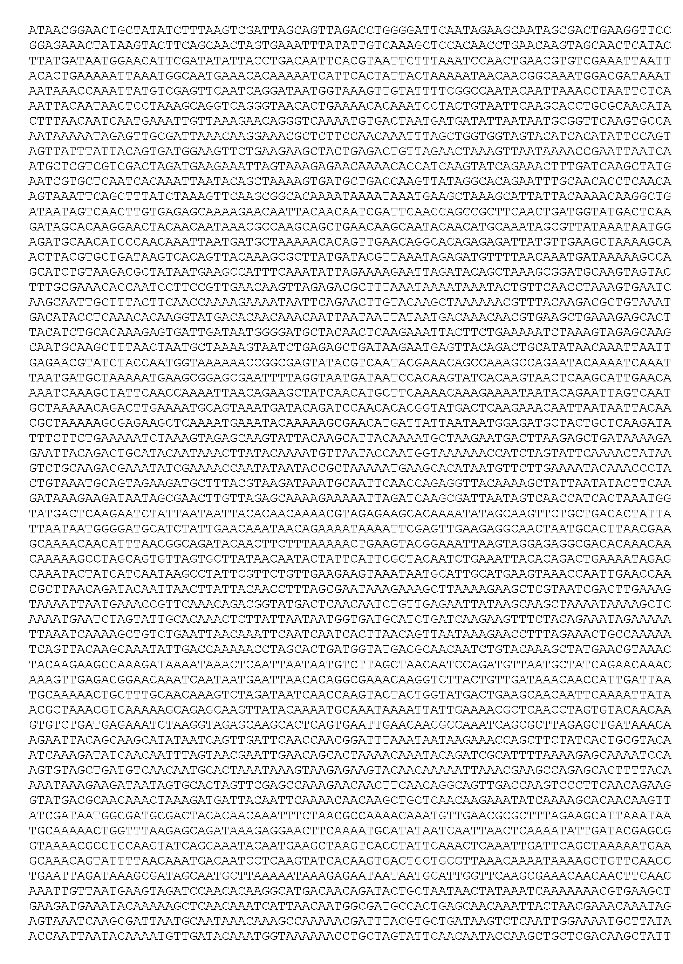

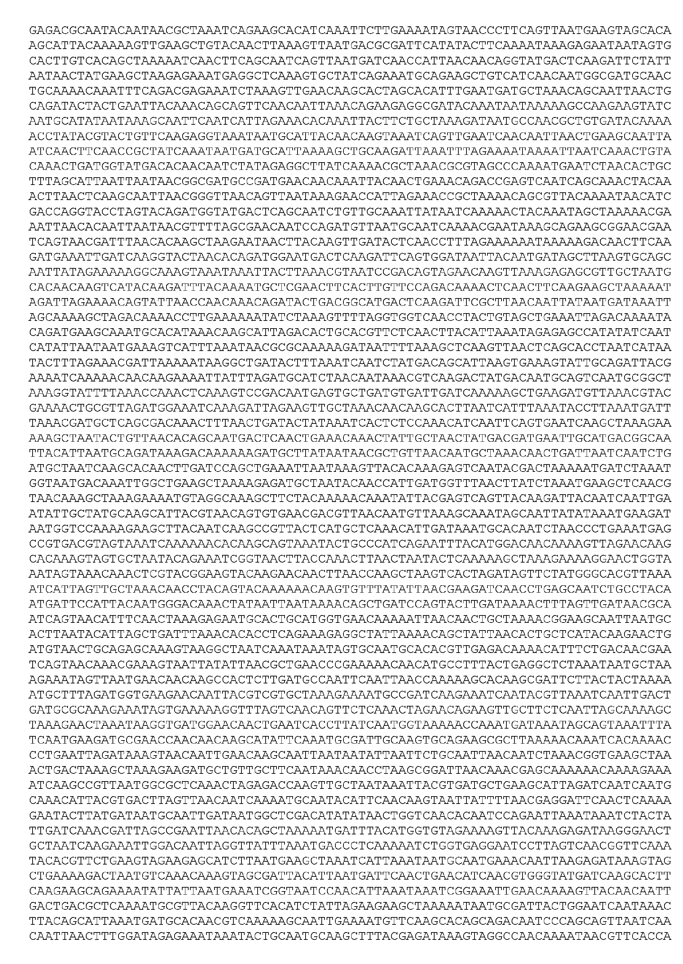

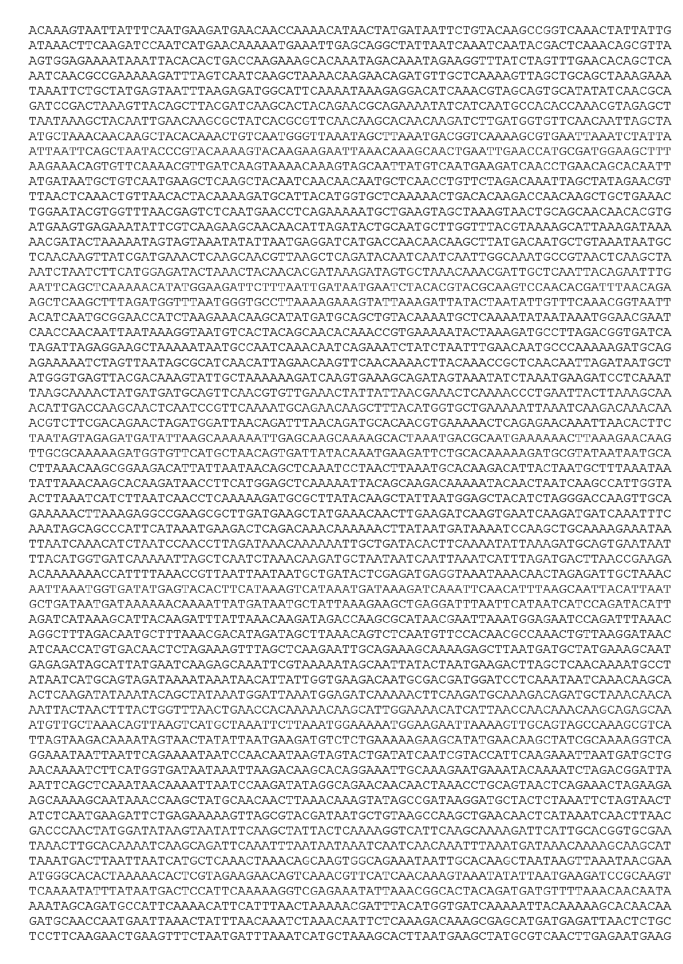

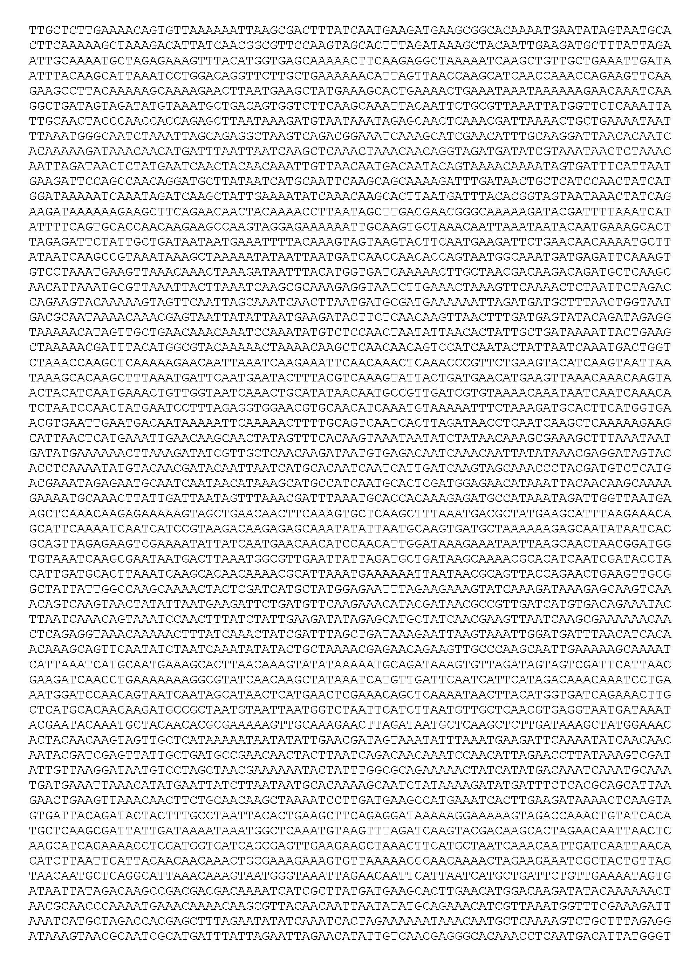

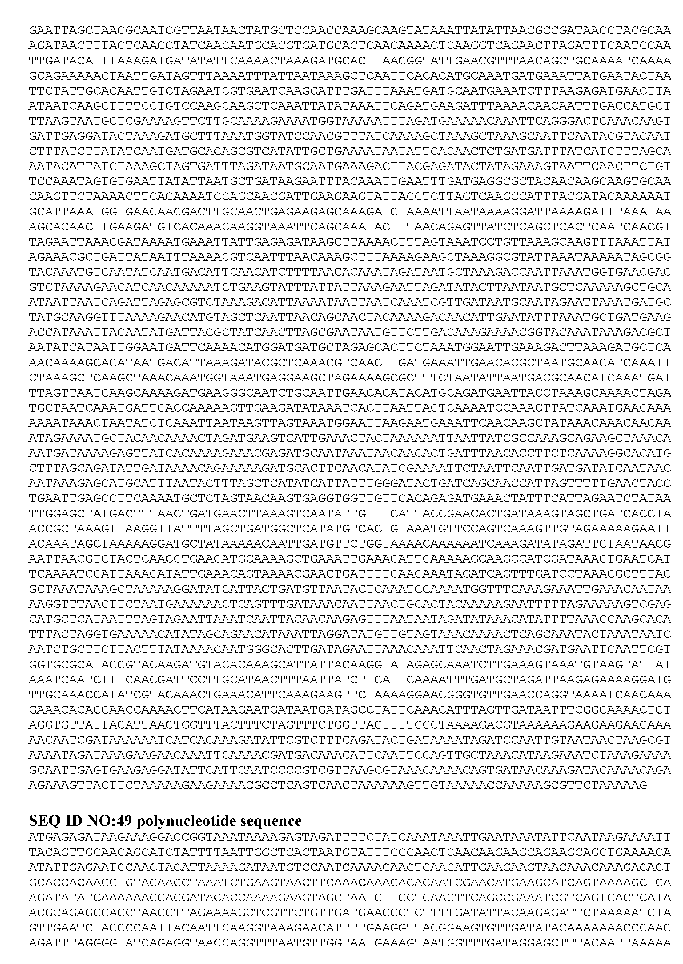

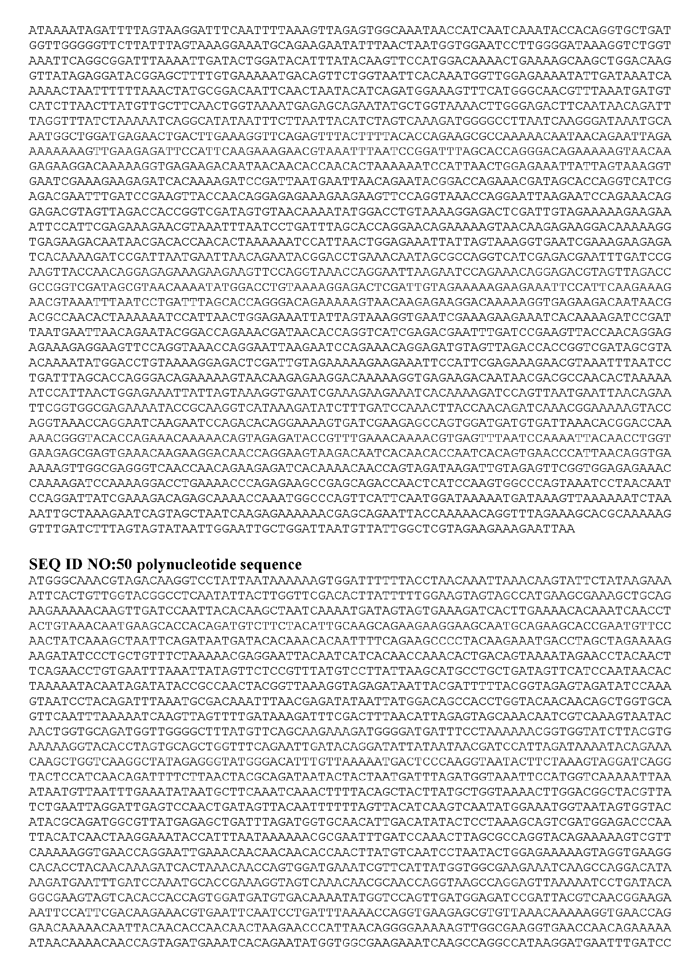

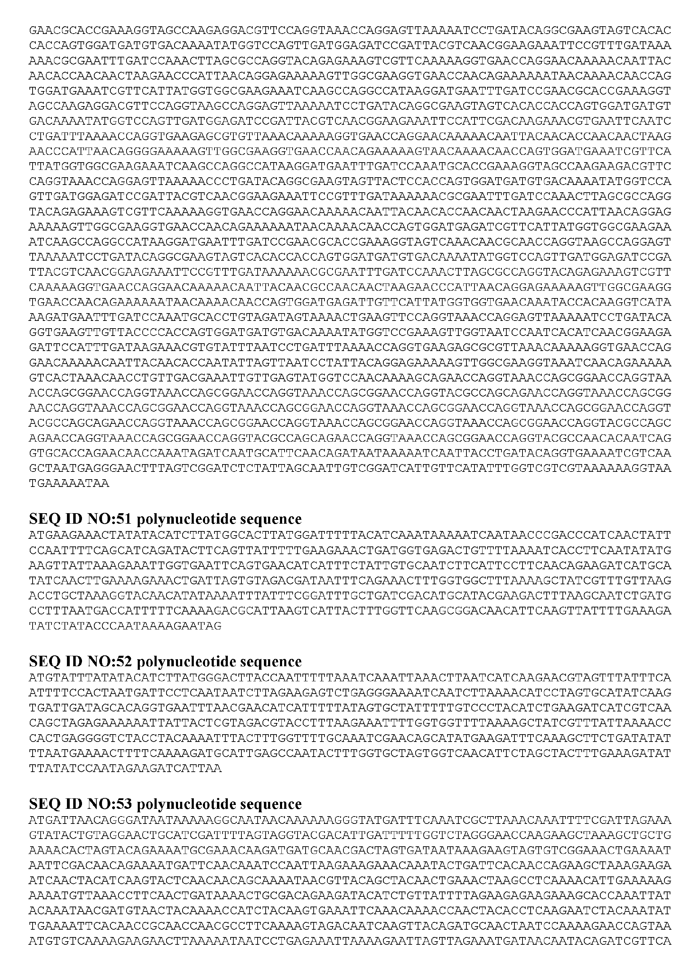

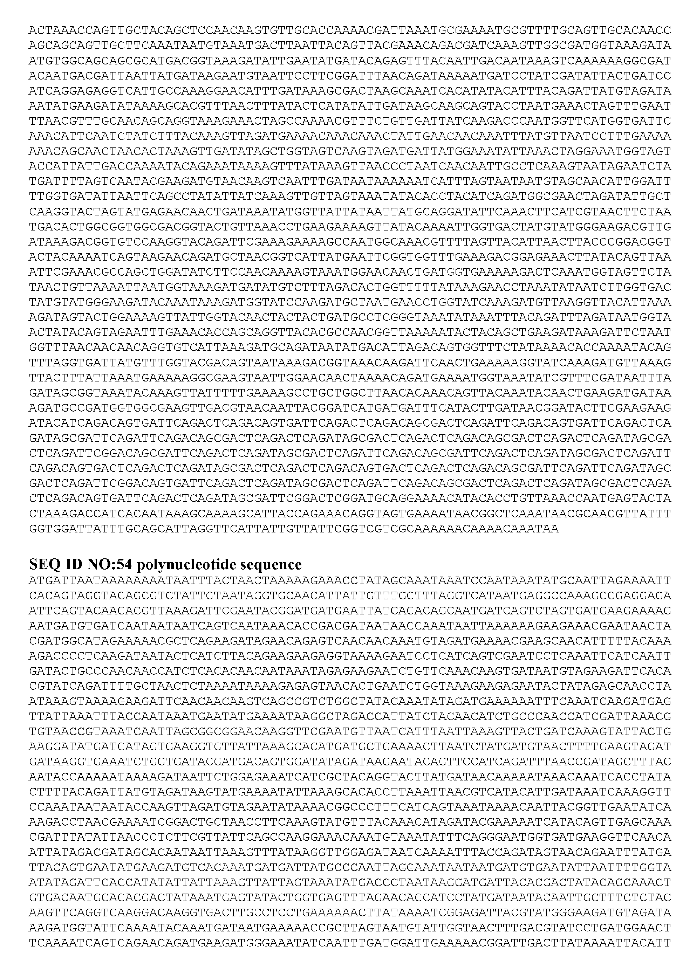

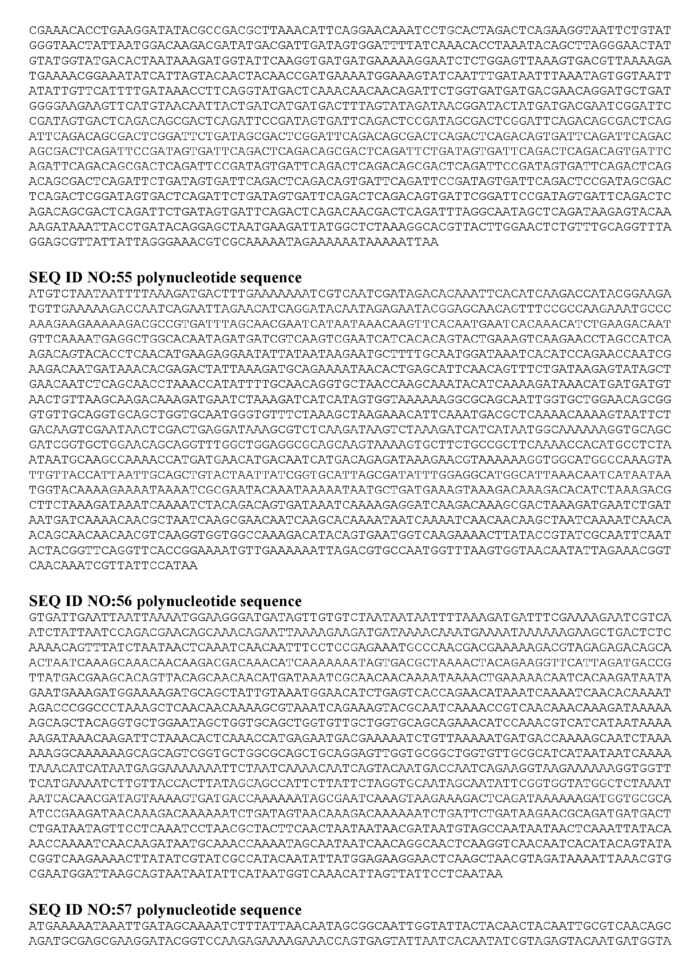

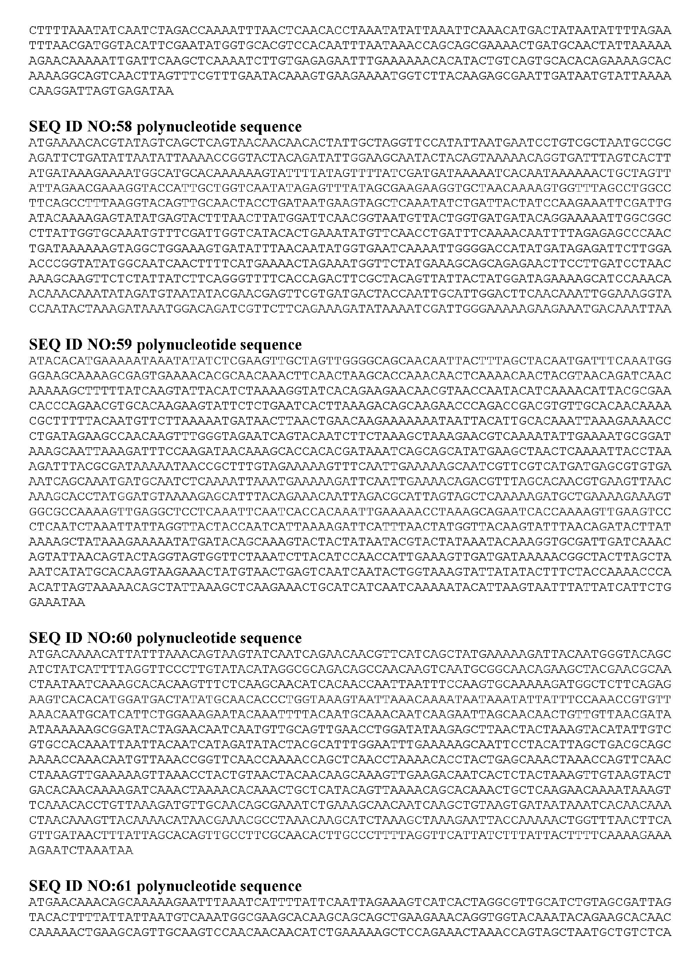

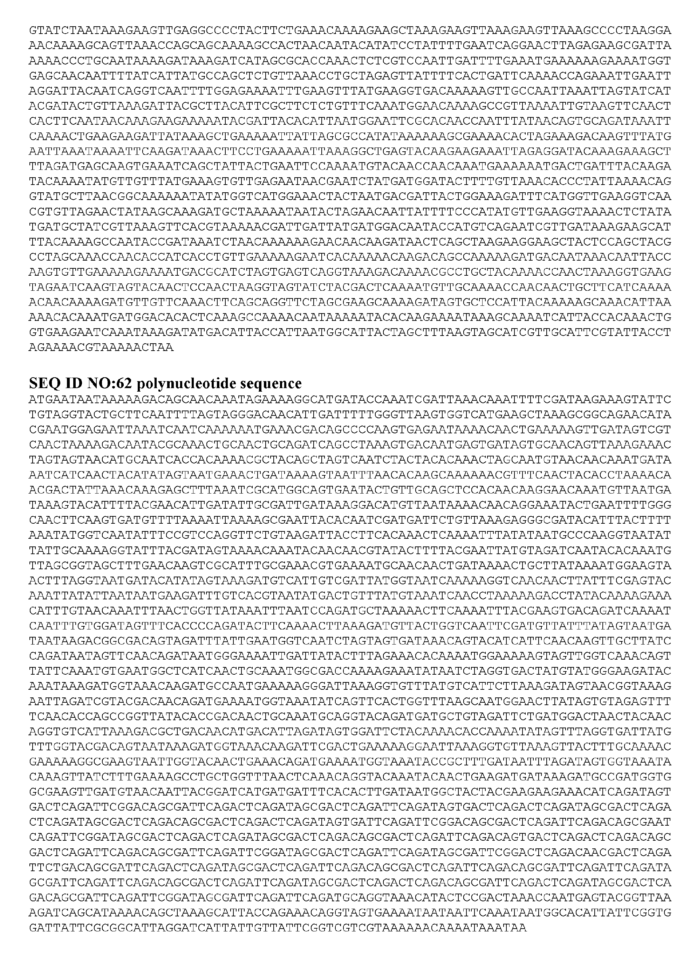

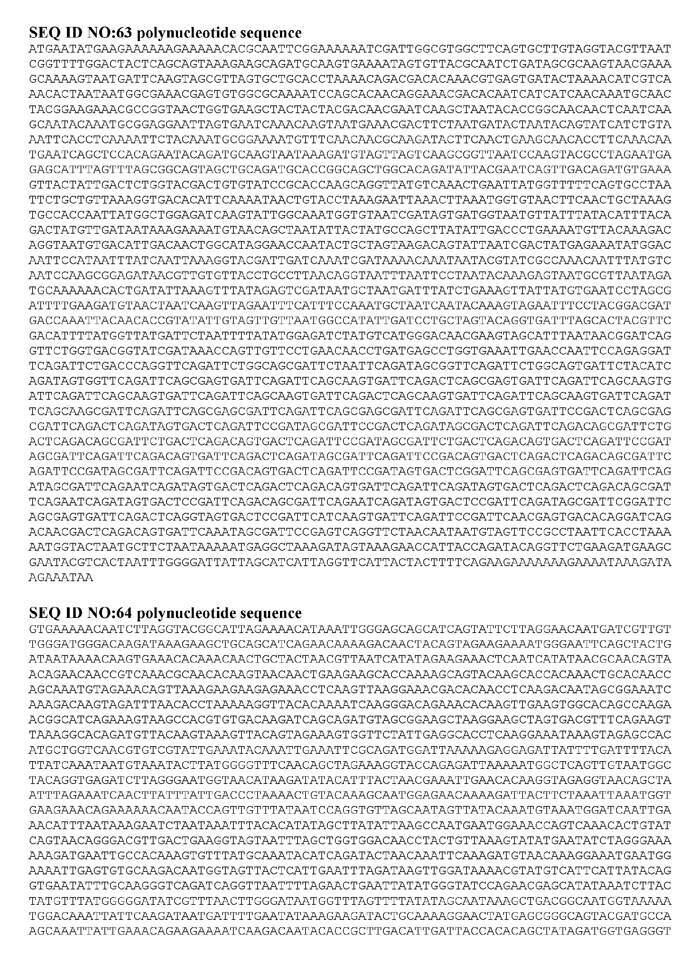

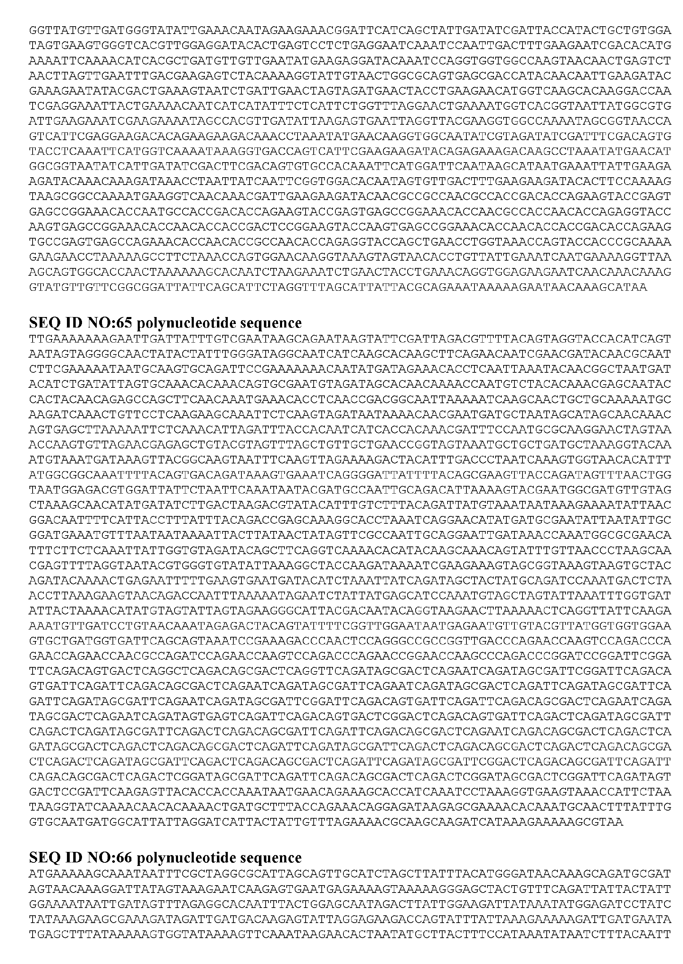

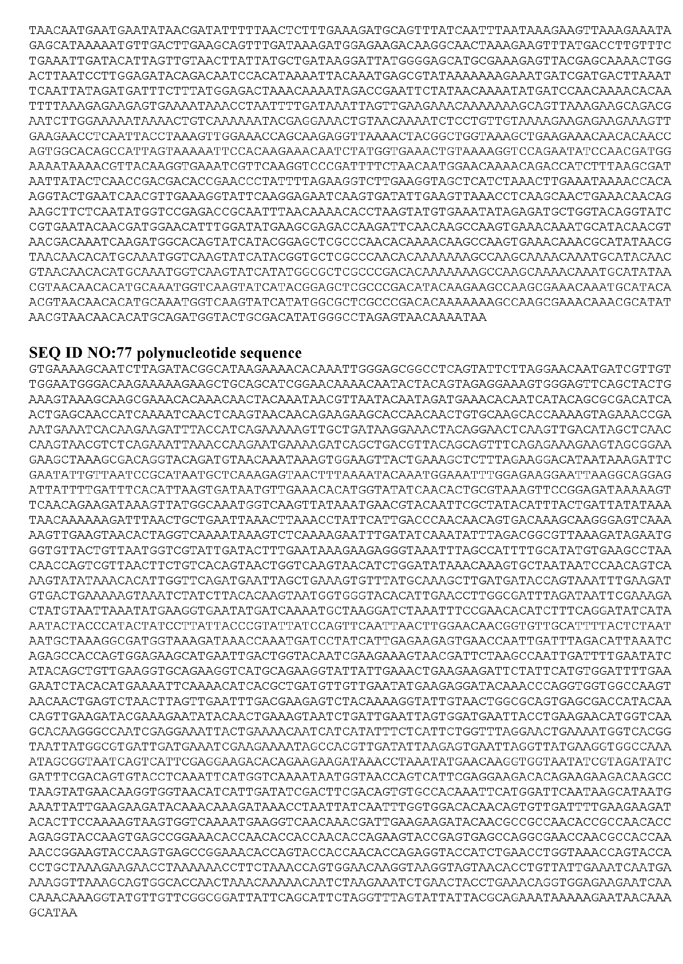

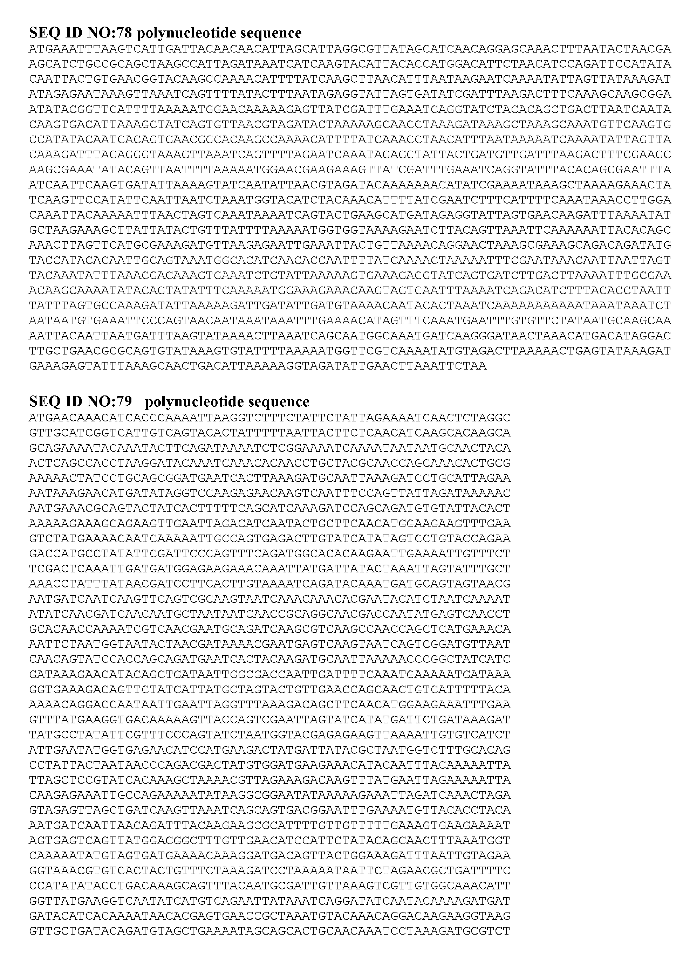

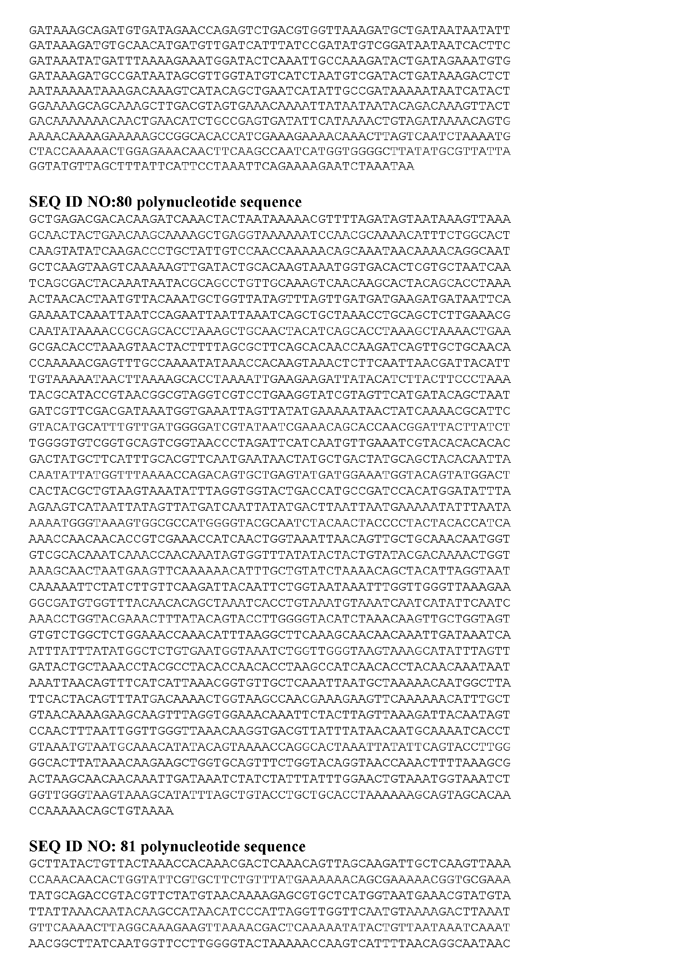

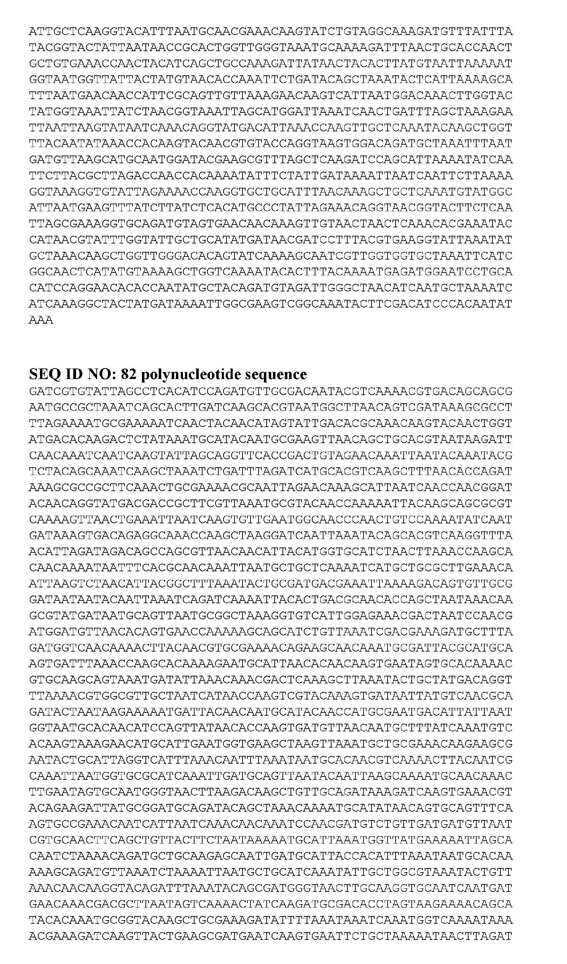

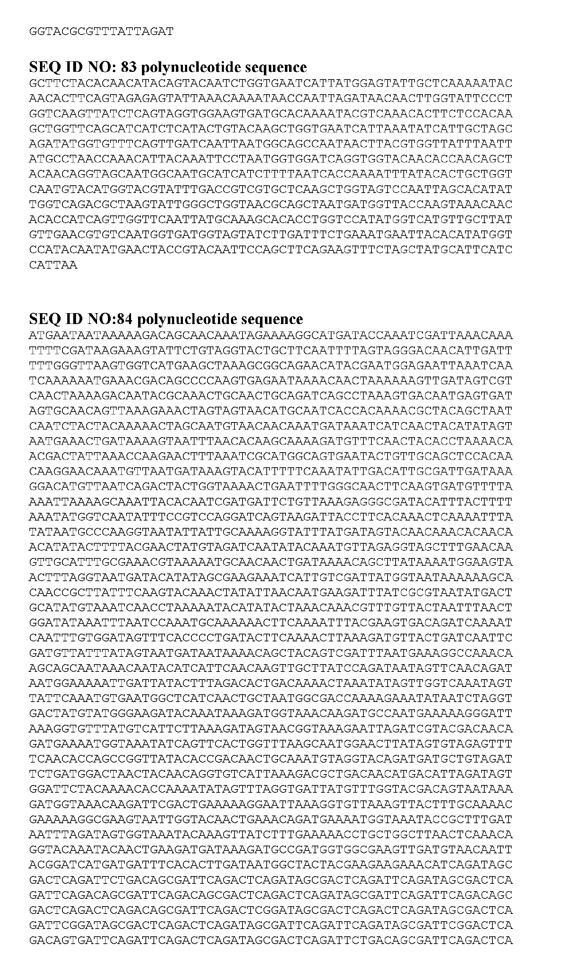

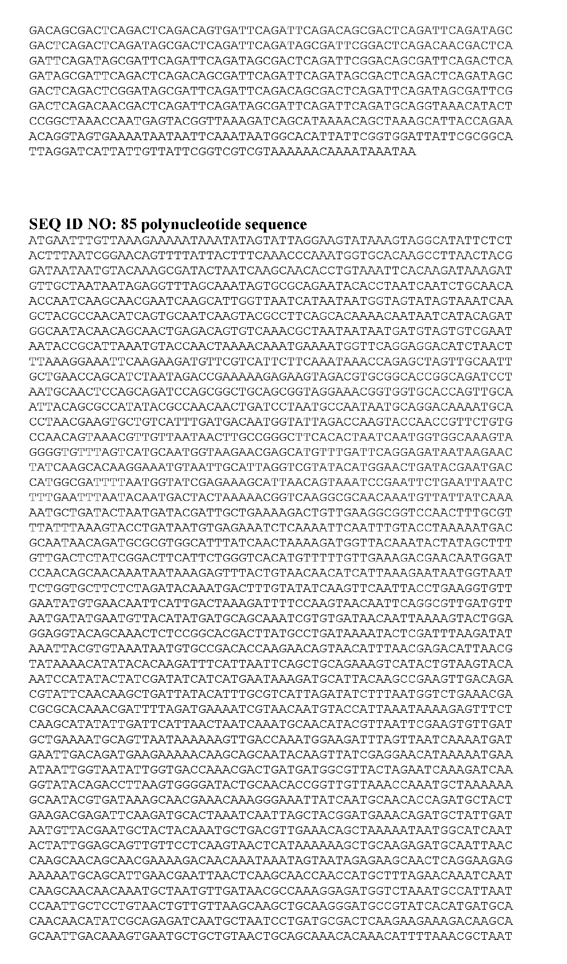

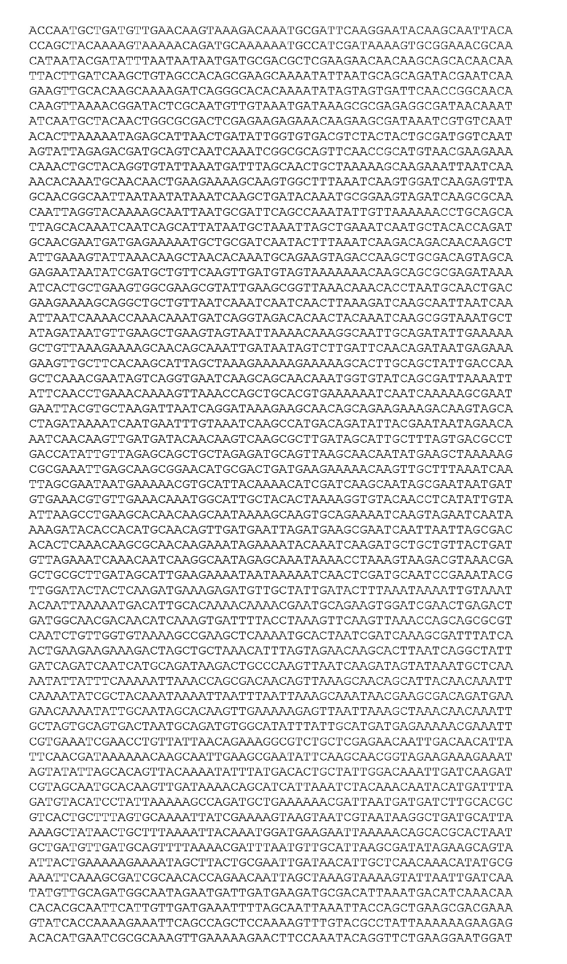

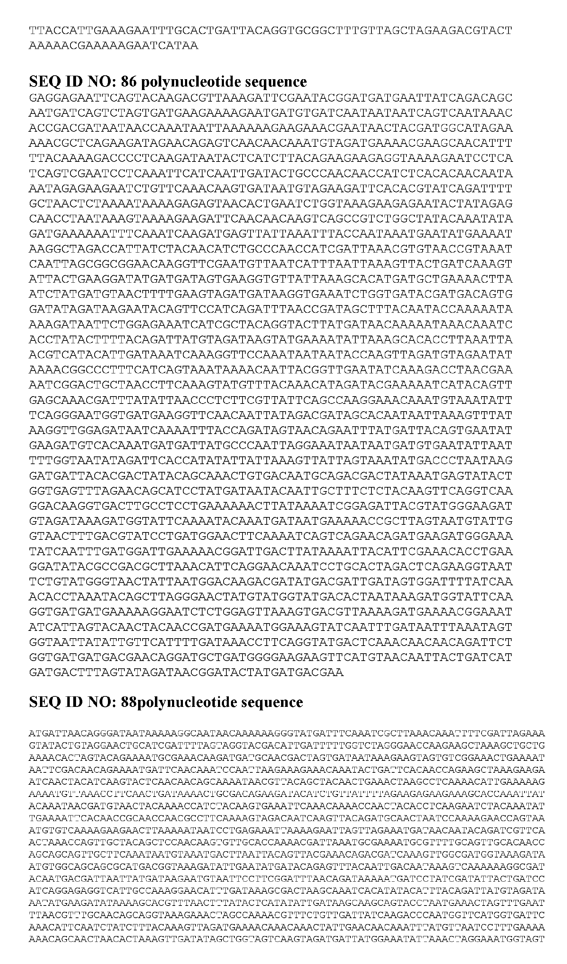

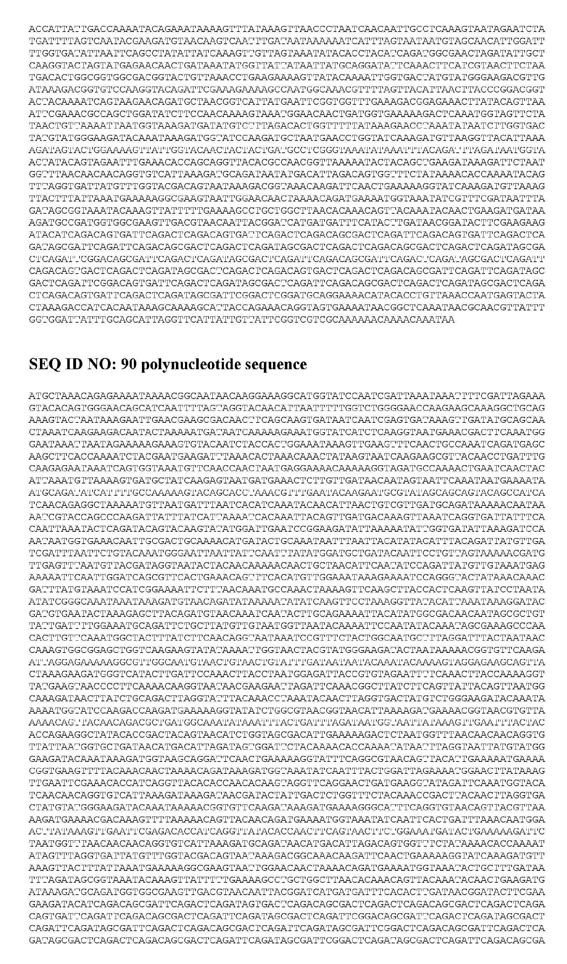

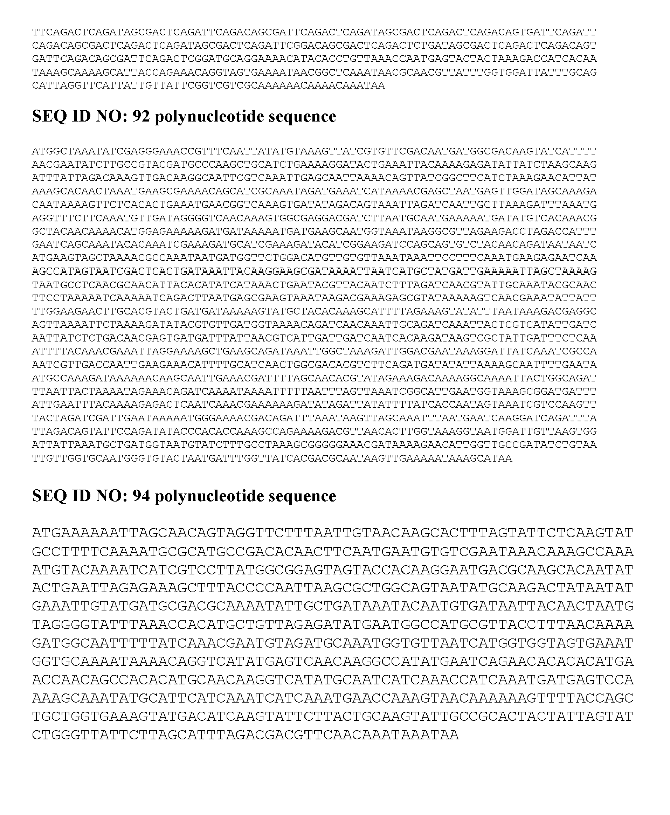

The following table sets out the SEQ ID numbers of preferred protein sequences and DNA sequences that are found in Figure 1 and Figure 2 respectively. SA indicates a sequence from *S. aureus* and SE indicates a sequence from *S. epidermidis.*

| **Name** | **Protein sequence** | **DNA sequence** |
|---|---|---|
| Immunodominant ABC transporter | | |
| **SA** | SEQ ID 1 | SEQ ID 34 |
| **SE** | SEQ ID 2 | SEQ ID 35 |
| Laminin receptor | | |
| **SA** | SEQ ID 3 | SEQ ID 36 |
| **SE** | SEQ ID 4 | SEQ ID 37 |
| Secretory Antigen A SsaA | | |
| **SA 1** | SEQ ID 5 | SEQ ID 38 |
| **SA 2** | SEQ ID 6 | SEQ ID 39 |
| **SE** | SEQ ID 7 | SEQ ID 40 |
| SitC | | |
| **SA** | SEQ ID 8 | SEQ ID 41 |
| **SE** | SEQ ID 9 | SEQ ID 42 |
| IsaA / PisA (IssA) | | |
| **SA** | SEQ ID 10 | SEQ ID 43 |
| **SE** | SEQ ID 11 | SEQ ID 44 |
| EbhA / B | | |
| **SA EbhA** | SEQ ID 12 | SEQ ID 45 |
| **SA EbhB** | SEQ ID 13 | SEQ ID 46 |
| **SE EbhA** | SEQ 10 14 | SEQ ID 47 |
| **SE EbhB** | SEQ 10 15 | SEQ ID 48 |
| Accumulation-assoc pro Aap | | |
| **SA** | SEQ ID 16 | SEQ ID 49 |
| **SE** | SEQ ID 17 | SEQ ID 50 |
| RNA III activating protein RAP | | |
| **SA** | SEQ ID 18 | SEQ ID 51 |
| **SE** | SEQ ID 19 | SEQ ID 52 |
| FIG / SdrG | | |
| **SA** | SEQ ID 20 | SEQ ID 53 |
| **SE** | SEQ ID 21 | SEQ ID 54 |
| Elastin binding protein EbpS | | |
| **SA** | SEQ ID 22 | SEQ ID 55 |
| **SE** | SEQ ID 23 | SEQ ID 56 |
| Extracellular protein EFB **SA** | SEQ ID 24 | SEQ ID 57 |
| alpha toxin **SA** | SEQ ID 25 | SEQ ID 58 |
| SBI **SA** | SEQ ID 26 | SEQ ID 59 |
| IsdA **SA** | SEQ ID 27 | SEQ ID 60 |
| IsdB **SA** | SEQ ID 28 | SEQ ID 61 |
| SdrC **SA** | SEQ ID 29 | SEQ ID 62 |
| CIfA **SA** | SEQ ID 30 | SEQ ID 63 |
| FnbA **SA** | SEQ ID 31 | SEQ ID 64 |
| ClfB **SA** | SEQ ID 32 | SEQ ID 65 |
| Coagulase **SA** | SEQ ID 33 | SEQ ID 66 |
| FnbB **SA** | SEQ ID 67 | SEQ ID 77 |
| MAP **SA** | SEQ ID 68 | SEQ ID 78 |
| HarA **SA** | SEQ ID 69 | SEQ ID 79 |
| Autolysin glucosaminidase **SA** | SEQ ID 70 | SEQ ID 80 |
| Autolysin amidase **SA** | SEQ ID 71 | SEQ ID 81 |
| Ebh fragment **SA** | SEQ ID 72 | SEQ ID 82 |
| Autolysin Ant **SA** | SEQ ID 73 | SEQ ID 83 |
| SdrC **SA** | SEQ ID 74 | SEQ ID 84 |
| MRPII **SA** | SEQ ID 75 | SEQ ID 85 |
| SdrG **SA** | SEQ ID 76 | SEQ ID 86 |
| SdrE **SA** | SEQ ID 87 | SEQ ID 88 |
| SdrD **SA** | SEQ ID 89 | SEQ ID 90 |
| SasF **SA** | SEQ ID 91 | SEQ ID 92 |

### Extracellular component binding proteins

Extracellular component binding proteins are proteins that bind to host extracellular components. The term includes, but is not limited to adhesins.

Examples of extracellular component binding proteins include laminin receptor (Naidu et al J. Med. Microbiol. 1992, 36; 177), SitC/MntC/saliva binding protein (US5801234, Wiltshire and Foster Infec. Immun. 2001, 69; 5198), EbhA (Williams et al Infect. Immun. 2002, 70; 6805), EbhB, Elastin binding protein (EbpS) (Park et al 1999, J. Biol. Chem. 274; 2845), EFB (FIB) (Wastfelt and Flock 1995, J. Clin. Microbiol. 33; 2347), SBI (Zhang et al FEMS Immun. Med. Microbiol. 2000, 28; 211), autolysin (Rupp et al 2001, J. Infect. Dis. 183; 1038), ClfA ( US6008341, McDevitt et al Mol. Microbiol. 1994, 11; 237), SdrC, SdrG (McCrea et al Microbiology 2000, 146; 1535), SdrH (McCrea et al Microbiology 2000, 146; 1535), Lipase GehD (US2002/0169288), SasA (Roche et al Microbiology 2003, 149 ; 643), SasC (Roche et al Microbiology 2003, 149 ; 643), SasK (Roche et al Microbiology 2003, 149; 643), FnbA (Flock et al Mol Microbiol. 1994, 12; 599, US6054572), FnbB (WO 97/14799, Booth et al 2001 Infec. Immun. 69; 345), collagen binding protein Cna (Visai et al 2000, J. Biol. Chem. 275; 39837), ClfB (WO 99/27109), SdrD (WO 99/27109), SdrE (WO 99/27109), FbpA (Phonimdaeng et al 1988 J. Gen Microbiol.134; 75), Npase (Flock 2001 J. Bacteriol. 183; 3999), IsaA/PisA (Lonenz et al FEMS Immuno. Med. Microbiol. 2000, 29; 145), SsaA (Lang et al FEMS Immunol. Med. Microbiol. 2000, 29; 213), EPB (Hussain and Hermann symposium on Staph Denmark 14-17^{th} 2000), SSP-1 (Veenstra et al 1996, J. Bacteriol. 178; 537), SSP-2 (Veenstra et al 1996, J. Bacteriol. 178; 537), 17 kDa heparin binding protein HBP (Fallgren et al 2001, J. Med. Microbiol. 50; 547), Vitronectin binding protein (Li et al 2001, Curr. Microbiol. 42; 361), fibrinogen binding protein, coagulase, Fig (WO 97/48727) and MAP (US5648240)

### SitC/MntC/saliva binding protein

This is an ABC transporter protein which is a homologue of adhesin PsaA in *S. pneumoniae.* It is a highly immunogenic 32kDa lipoprotein which is distributed through the bacterial cell wall (Cockayne et al Infect. Immun. 1998 66; 3767). It is expressed in *S. aureus* and *S. epidermidis* as a 32kDa lipoprotein and a 40kDa homologue is present in *S. hominis.* In *S. epidermidis,* it is a component of an iron-regulated operon. It shows considerable homology to both adhesins including FimA of Streptococcus parasanguis, and with lipoproteins of a family of ABC transporters with proven or putative metal iron transport functions. Therefore SitC is included as an extracellular biding protein and as a metal ion transporter.

The saliva binding protein disclosed in US5,801,234 is also a form of SitC and can be included in an immunogenic composition of the invention.

### ClfA and ClfB

Both these proteins have fibrinogen binding activity and trigger *S. aureus* to form clumps in the presence of plasma. They contain a LPXTG motif common to wall associated proteins.

ClfA is described in US6008341 and ClfB is described in WO 99/27109.

### Coagulase (FbpA)

This is a fibrinogen binding protein which triggers *S. aureus* to form clumps in the presence of plasma. It is described in references related to Coagulase : Phonimdaeng et al (J. Gen. Microbio. 1988, 134:75-83), Phonimdaeng et al. (Mol Microbiol 1990; 4:393-404), Cheung et al. (Infect Immun 1995; 63:1914-1920) and Shopsin et al. (J. CLin. Microbiol. 2000; 38:3453-3456). In an embodiment, fragments for inclusion in the immunogenic composition of the invention include the mature protein in which the signal peptide has been removed (amino acids 27 to the C-terminus).

Coagulase has three distinct domains. Amino acids 59-297 which is a coiled coil region, amino acids 326-505 which is a proline and glycine rich region and the C-terminal domain from amino acid 506 to 645 which has a beta sheet conformation. Each of these domains is a fragment which may be incorporated into the immunogenic composition of the invention.

### SdrG

This protein is described in WO 00/12689. SdrG is found in coagulase negative staphylococci and is a cell wall associated protein containing a LPXTG sequence.

SdrG contains a signal peptide (amino acids 1-51), a region containing fibrinogen binding sites and collagen binding sites (amino acids 51-825), two CnaB domains (amino acids 627-698 and 738-809), a SD repeat region (amino acids 825-1000) and an anchor domain (amino acids 1009-1056).

In an embodiment fragments of SdrG include polypeptides in which the signal peptide and/or the SD repeats and the anchor domain have been removed. These include polypeptides comprising or consisting of amino acids 50-825, amino acids 50-633, amino acids 50-597 (SEQ ID NO 2 of WO 03/76470), amino acids 273-597 (SEQ ID NO 4 of WO 03/76470), amino acids 273-577 (SEQ ID NO 6 of WO 03/76470) amino acids 1-549, amino acids 219-549, amino acids 225-549, amino acids 219-528, amino acids 225-528 of SEQ ID NO: 70 or 20 or 21.

Optionally, an SdrG polypeptide having a sequence at least 80%, 85%, 90%, 92%, 95%, 97%, 98%, 99% or 100% homologous to the sequence of SEQ ID NO: 70, 20 or 21 is incorporated into the immunogenic composition of the invention.

The compositions of the invention optionally comprise a fragment of the SdrG polypeptides described above.

In an embodiment fragments have the signal peptide and/or the SD repeat domain and/or the anchoring domain deleted. For example sequences corresponding to amino acids 1-713 , 1-549, 225-549, 225-529, 24-717, 1-707, 1-690, 1-680, 1-670, 1-660, 1-650, 1-640, 1-630, 1-620, 1-610, 1-600, 34-707, 44-697, 36-689 of SEQ ID 70 or sequences having 85%, 90%, 92%, 95%, 97%, 98%, 99% or 100% identity to SEQ ID 70 or 20 or 21.

In an embodiment, fragments with the signal peptide deleted have a methionine residue at the N-terminus of the fragment to ensure correct translation.

In an embodiment, the fragment has the following sequence:-

MEENSVQDVKDSNTDDELSDSNDQSSDEEKNDVINNNQSINTDDNNQIIKKEETNNYDGIEKRSEDRTESTTN
VDENEATFLQKTPQDNTHLTEEEVKESSSVESSNSSIDTAQQPSHTTINREESVQTSDNVEDSHVSDFANSKI
KESNTESGKEENTIEQPNKVKEDSTTSQPSGYTNIDEKISNQDE
LLNLPINEYENKARPLSTTSAQPSIKRVTVNQLAAEQGSNVNHLIKVTDQSITEGYDDSEGVIKAHDAENLIY
DVTFEVDDKVKSGDTMTVDIDKNTVPSDLTDSFTIPKIKDNSGEIIATGTYDNKNKQITYTFTDYVDKYENIK
AHLKLTSYIDKSKVPNNNTKLDVEYKTALSSVNKTITVEYQRPNENRTANLQSMFTNIDTKNHTVEQTIYINP
LRYSAKETNVNISGNGDEGST
IIDDSTIIKVYKVGDNQNLPDSNRIYDYSEYEDVTNDDYAQLGNNNDVNINFGNIDSPYIIKVISKYDPNKDD
YTTIQQTVTMQTTINEYTGEFRTASYDNTIAFSTSSGQGQGDLPPEKTYKIGDYVWEDVDKDGIQNTNDNEKP
LSNVLVTLTYPDGTSKSVRTDEDGKYQFDGLKNGLTYKITFETPEGYTPTLKHSGTNPALDSEGNSVWVTING
QDDMTIDSGFYQTPKYSLGNY
VWYDTNKDGIQGDDEKGISGVKVTLKDENGNIISTTTTDENGKYQFDNLNSGNYIVHFDKPSGMTQTTTDSGD
DDEQDADGEEVHVTITDHDDFSIDNGYYDDE

### EbhA and EbhB

EbhA and EbhB are proteins that are expressed in both *S*. *aureus* and *S. epidermidis* (Clarke and Foster Infect. Immun. 2002, 70; 6680 , Williams et al Infect. Immun. 2002, 20; 6805) and which bind to fibronectin. Since fibronectin is an important component of extracellular matrix, EbhA and EbhB have an important function in adhering staphylococci to host extracellular matrix.

The Ebh proteins are large, having a molecular weight of 1.1 megadaltons. It is advantageous to use a fragment of the Ebh protein rather than the complete sequence due to ease of production and formulation. The central region of the protein contains imperfect repeats which contain fibronectin binding sites. Fragments containing one or more of the repeat domains described below are some fragments suitable for incorporation into the immunogenic composition of the invention.

Ebh proteins contain imperfect repeats units of 127 amino acids in length which are characterised by containing the consensus sequence:-
L.G.{10}A.{13}Q.{26}L...M..L.{33}A
   or
.{19}L.G.{10}A.{13}Q.{26}L...M..L.{33}A.{12}
   or
.....I/V..A...I/V..AK.ALN/DG..NL..AK..A.{6}L..LN.AQK..L..QI/V..A..V.. V.{6}A..LN/D.AM..L...I/V.D/E...TK.S.NY/F.N/DAD..K..AY/F..AV..A..I/V.N /D.......

Where '.' means any amino acid and '.{10}' means any 10 amino acids and I/V indicates alternative choices of amino acid.

By reference to the sequence disclosed in Kuroda et al (2001) Lancet 357; 1225-1240, and Table 2, the repeat sequences within Ebh proteins are readily deduced.

In an embodiment, fragments to be included in the immunogenic composition of the invention include proteins containing of one, two, three, four, five, six, seven, eight, nine, ten or more than 10 of the 127 amino acid repeat units. Such fragments may consist of 1, 2, 3, 4, 5, 6, 7, 8, 9, 10 or more repeats of the 127 amino acid repeat region or may consist of 1, 2, 3, 4, 5, 6, 7, 8, 9, 10 or more repeats with additional amino acid residues present at either or both ends of the fragment. Optionally the fragment is the H2 polypeptide of about 44kDa spaning three repeats (amino acids 3202-3595) as described in Clarke et al Infection and Immunity 70, 6680-6687, 2002. Such fragments will optionally be able to bind fibronectin and/or to elicit antibodies that are reactive against the whole Ebh protein.

The Ebh proteins are capable of binding to fibronectin. In an embodiment, fragments of these polypeptides sequences retain the ability to bind to fibronectin. Binding to fibronectin can be assessed by ELISA as described by Clarke et al ( Infection and Immunity 70; 6680-6687 2002).

In an embodiment, the fragment is one which comprises a B-cell or T-helper epitope, for example those fragments/peptides described in Tables 3 and 4.

### TABLE 2 Repeat sequences in the full-length sequence of Ebh.

The full-length sequence of Ebh is disclosed in Kuroda et al (2001) Lancet 357; 1225-1240. The following table shows the amino acid residues at which the 127 amino acid repeats begin and end within the full length sequence.

| | Begin | End |
|---|---|---|
| 1 | 3204 | 3330 |
| 2 | 3331 | 3457 |
| 3 | 3457 | 3583 |
| 4 | 3583 | 3709 |
| 5 | 3709 | 3835 |
| 6 | 3835 | 3961 |
| 7 | 3961 | 4087 |
| 8 | 4200 | 4326 |
| 9 | 4326 | 4452 |
| 10 | 4452 | 4578 |
| 11 | 4578 | 4704 |
| 12 | 4704 | 4830 |
| 13 | 4830 | 4956 |
| 14 | 4956 | 5082 |
| 15 | 5082 | 5208 |
| 16 | 5208 | 5334 |
| 17 | 5334 | 5460 |
| 18 | 5460 | 5586 |
| 19 | 5585 | 5711 |
| 20 | 5711 | 5837 |
| 21 | 5837 | 5963 |
| 22 | 5963 | 6089 |
| 23 | 6089 | 6215 |
| 24 | 6215 | 6341 |
| 25 | 6341 | 6467 |
| 26 | 6467 | 6593 |
| 27 | 6593 | 6719 |
| 28 | 6719 | 6845 |
| 29 | 6845 | 6971 |
| 30 | 6971 | 7097 |
| 31 | 7097 | 7223 |
| 32 | 7223 | 7349 |
| 33 | 7349 | 7475 |
| 34 | 7475 | 7601 |
| 35 | 7601 | 7727 |
| 36 | 7727 | 7853 |
| 37 | 7852 | 7978 |
| 38 | 7978 | 8104 |
| 39 | 8104 | 8230 |
| 40 | 8230 | 8356 |
| 41 | 8356 | 8482 |
| 42 | 8482 | 8608 |
| 43 | 8604 | 8730 |
| 44 | 8858 | 8984 |

### Table 3 B-cell epitope prediction for a 127 amino acid repeat :

The full-length sequence is disclosed in Kuroda et al (2001) Lancet 357; 1225-1240. One of these repeats, encoded by amino acids 3204-3331 of the full-length sequence was chosen to carry out an epitope prediction:-

MDVNTVNQKAASVKSTKDALDGQQNLQRAKTEATNAITHASDLNQAQKNALTQQVN
SAQNVHAVNDIKQTTQSLNTAMTGLKRGVANHNQVVQSDNYVNADTNKKNDYNNAY
NHANDIINGNAQHPVI

| **Begin** | **End** | **Epitope sequence** | **Start** | **Stop** |
|---|---|---|---|---|
| 5 | 10 | TVNQKA | 3208 | 3213 |
| 14 | 19 | KSTKDA | 3217 | 3222 |
| 21 | 33 | DGQQNLQRAKTEA | 3224 | 3236 |
| 42 | 51 | DLNQAQKNAL | 3245 | 3254 |
| 66 | 74 | DIKQTTQSL | 3269 | 3277 |
| 100 | 112 | ADTNKKNDYNNAY | 3303 | 3315 |
| 117 | 123 | DIINGNA | 3320 | 3326 |

| | | | | |
|---|---|---|---|---|
| - The "Begin" and "End" columns present the position of the predicted B-cell epitopes in the 127 amino acid repeat - The "Start" and "Stop" columns present the position of the predicted B-cell epitopes in the Ebh full length sequence | | | | |

### Table 4 T-helper cell epitope prediction in Ebh :

The full-length sequence is disclosed in TrEMBL database, sequence reference Q8NWQ6. One of these repeats, encoded by amino acids 3204-3331 of the full-length sequence was chosen to carry out an epitope prediction:-

MDVNTVNQKAASVKSTKDALDGQQNLQRAKTEATNAITHASDLNQAQKNALTQQVN
SAQNVHAVNDIKQTTQSLNTAMTGLKRGVANHNQVVQSDNYVNADTNKKNDYNNAY
NHANDIINGNAQHPVI

| Position repeat | **Epitope sequence** | **Position sequence** |
|---|---|---|
| 1 | MDVNTVNQK | 3204 |
| 3 | VNTVNQKAA | 3206 |
| 6 | VNQKAASVK | 3209 |
| 26 | LQRAKTEAT | 3229 |
| 37 | ITHASDLNQ | 3240 |
| 43 | LNQAQKNAL | 3246 |
| 51 | LTQQVNSAQ | 3254 |
| 55 | VNSAQNVHA | 3258 |
| 61 | VHAVNDIKQ | 3264 |
| 64 | VNDIKQTTQ | 3267 |
| 67 | IKQTTQSLN | 3270 |
| 74 | LNTAMTGLK | 3277 |
| 78 | MTGLKRGVA | 3281 |
| 81 | LKRGVANHN | 3284 |
| 85 | VANHNQVVQ | 3288 |
| 91 | VVQSDNYVN | 3294 |
| 92 | VQSDNYVNA | 3295 |
| 97 | YVNADTNKK | 3301 |
| 98 | VNADTNKKN | 3302 |
| 108 | YNNAYNHAN | 3311 |
| 112 | YNHANDIIN | 3315 |
| 118 | IINGNAQHP | 3321 |
| 119 | INGNAQHPV | 3322 |

| | | |
|---|---|---|
| - The "Position repeat" column presents the position of the predicted T-cell epitopes in the repeat - The "Position sequence" column presents the position of the predicted T-cell epitopes in the Ebh full length sequence | | |

Fragments of the proteins of the invention may be employed for producing the corresponding full-length polypeptide by peptide synthesis; therefore, these fragments may be employed as intermediates for producing the full-length proteins of the invention.

In an embodiment, variants are used in which several, 5-10, 1-5, 1-3, 1-2 or 1 amino acids are substituted, deleted, or added in any combination.

### Elastin binding protein (EbpS)

EbpS is a protein containing 486 amino acids with a molecular weight of 83kDa. It is associated with the cytoplasmic membrane of *S. aureus* and has three hydrophobic regions which hold the protein in the membrane (Downer et al 2002, J. Biol. Chem. 277; 243; Park et al 1996, J. Biol. Chem. 271; 15803).

Two regions between amino acids 1-205 and 343-486 are surface exposed on the outer face of the cytoplasmic membrane. The ligand binding domain of EbpS is located between residues 14-34 at the N-terminus (Park et al 1999, J. Biol. Chem. 274; 2845).

In an embodiment, the fragment to be incorporated into the immunogenic composition of the invention is the surface exposed fragment containing the elastin binding region (amino acids 1-205). Optionally the fragments do not contain the entire exposed loop but should contain the elastin binding region (amino acids 14-34). An alternative fragment which could be used consists of amino acids forming the second surface exposed loop (amino acids 343-486). Alternative fragments containing up to 1, 2, 5, 10, 20, 50 amino acids less at one or both ends are also possible.

### Laminin receptors

The laminin receptor of *S*. *aureus* plays an important role in pathogenicity. A characteristic feature of infection is bloodstream invasion which allows widespread metastatic abscess formation. Bloodstream invasion requires the ability to extravasate across the vascular basement membrane. This is achieved through binding to laminin through the laminin receptor (Lopes et al Science 1985, 229; 275).

Laminin receptors are surface exposed and are present in many strains of staphylococci including *S. aureus* and *S*. *epidermidis.*

### SBI

Sbi is a second IgG binding protein in addition to protein A and it is expressed in most strains of *S*. *aureus* (Zhang et al 1998, Microbiology 144; 985).

The N-terminus of the sequence of Sbi has a typical signal sequence with a cleavage site after amino acid 29. Therefore a fragment of Sbi which could be used in an immunogenic composition of the invention starts at amino acid residue 30, 31, 32 or 33 and continues to the C-terminus of Sbi, for example of SEQ ID NO: 26.

The IgG binding domain of Sbi has been identified as a region towards the N-terminus of the protein from amino acids 41-92. This domain is homologous to the IgG binding domains of protein A.

The minimal IgG binding domain of Sbi contains the following sequence:-

QTTQNNYVTDQQKAFYQVLHLKGITEEQRNQYIKTLREHPERAQEVFSESLK
** *** * *** * * * * *

* - denotes amino acids which are similar between IgG binding domains

In an embodiment, a fragment of Sbi to be included in the immunogenic composition of the invention contains an IgG binding domain. This fragment contains the consensus sequence for an IgG binding domain as designated by * as shown in the above sequence. Optionally the fragment contains or consists of the complete sequence shown above. Optionally, the fragment contains or consists of amino acids 30-92, 33-92, 30-94, 33-94, 30-146, 33-146, 30-150, 33-150, 30-160, 33-160, 33-170, 33-180, 33-190, 33-200, 33-205 or 33-210 of Sbi, for example of SEQ ID NO:26.

A fragment may contain 1, 2, 3, 4, 5, 6, 7, 8, 9, 10 amino acid substitutions from the sequences indicated.

A fragments may contain multiple repeats (2, 3, 4, 5, 6, 7,8, 9 or 10) of the IgG binding domain.

### EFB - FIB

Fib is a 19kDa fibrinogen binding protein which is secreted into the extracellular medium by S. aureus. It is produced by all S aureus isolates tested (Wastfelt and Flock 1995, J. Clin. Microbiol. 33; 2347).

*S. aureus* clumps in the presence of fibrinogen and binds to fibrinogen coated surfaces. This ability facilitates staphylococcal colonisation of catheters and endothelial cells.

Fib contains a signal sequence at the N-terminus of the protein with a putative cleavage site at about amino acid 30. In an embodiment, the immunogenic composition of the invention comprises or consists of the sequence of the mature protein (from about amino acid 30 to the C-terminus of the protein).

### Fbe - EfB/FIG

Fbe is a fibrinogen binding protein that is found in many isolates of *S. epidermidis* and has a deduced molecular weight of 119 kDa (Nilsson et al 1998. Infect. Immun. 66; 2666). Its sequence is related to that of clumping factor from *S. aureus* (ClfA). Antibodies against Fbe can block the binding of *S. epidermidis* to fibrinogen coated plates and to catheters (Pei and Flock 2001, J. Infect. Dis. 184; 52).

Fbe has a putative signal sequence with a cleavage site between amino acids 51 and 52. Therefore a potential fragment of Fbe contains the mature form of Fbe extending from amino acid 52 to the C-terminus (amino acid 1,092).

The domain of Fbe from amino acid 52 to amino acid 825 is responsible for fibrinogen binding. In an embodiment, the fragment of Fbe consists of or contains amino acids 52-825.

The region between amino acid 373 and 516 of Fbe shows the most conservation between Fbe and ClfA. In an embodiment, the fragment contains amino acids 373-516 of Fbe.

Amino acids 825 - 1041 of Fbe contains a highly repetitive region composed of tandemly repeated aspartic acid and serine residues.

### IsaA/PisA

IsaA is a 29kDa protein, also known as PisA has been shown to be a immunodominant staphylococcal protein during sepsis in hospital patients (Lorenz et al 2000, FEMS Immunol. Med. Microb. 29; 145).

The first 29 amino acids of the IsaA sequence are thought to be a signal sequence. In an embodiment, the fragment of IsaA to be included in an immunogenic composition of the invention contains amino acid residues 30 onwards, to the end of the coded sequence.

### Fibronectin binding protein

Fibronectin binding protein A contains several domains that are involved in binding to fibronectin (WO 94/18327). These are called D1, D2, D3 and D4. In an embodiment fragments of fibronectin binding protein A or B comprise or consist of D1, D2, D3, D4, D1-D2, D2-D3, D3-D4, D1-D3, D2-D4 or D1-D4.

Fibronectin binding protein contains a 36 amino acid signal sequence. For example:
VKNNLRYGIRKHKLGAASVFLGTMIVVGMGQDKEAA

Optionally, the mature protein omitting this signal sequence is included in the immunogenic composition of the invention.

### Transporter proteins

The cell wall of Gram positive bacteria acts as a barrier preventing free diffusion of metabolites into the bacterium. A family of proteins orchestrates the passage of essential nutrients into the bacterium and are therefore essential for the viability of the bacterium. The term transporter protein covers proteins involved in the initial step of binding to metabolites such as iron as well as those involved in actually transporting the metabolite into the bacterium.

Molecular iron is an essential co-factor for bacterial growth. Siderophores are secreted that bind free iron and then are captured by bacterial surface receptors that deliver iron for transport across the cytoplasmic membrane. Iron acquisition is critical for the establishment of human infections so that the generation of an immune response against this class of proteins leads to a loss of staphylococcal viability.

Examples of transporter proteins include Immunodominant ABC transporter (Burnie et al 2000 Infect. Imun. 68; 3200), IsdA (Mazmanian et al 2002 PNAS 99; 2293), IsdB (Mazmanian et al 2002 PNAS 99; 2293), IsdC (WO 06/59247), Mg2+ transporter, SitC (Wiltshire and Foster 2001 Infect. Immun. 69; 5198) and Ni ABC transporter.

### Immunodominant ABC transporter

Immunodominant ABC transporter is a well conserved protein which may be capable of generating an immune response that is cross-protective against different staphylococcal strains (Mei et al 1997, Mol. Microbiol. 26; 399). Antibodies against this protein have been found in patients with septicaemia (Burnie et al 2000, Infect. Immun. 68; 3200).

Optional fragments of imunodominant ABC transporter will include the peptides DRHFLN, GNYD, RRYPF, KTTLLK, GVTTSLS, VDWLR, RGFL, KIKVYVGNYDFWYQS, TVIVVSHDRHFLYNNV and/or TETFLRGFLGRMLFS since these sequences contain epitopes that are recognised by the human immune system.

### IsdA-IsdB

The isd genes (iron-regulated surface determinant) of S. *aureus* encode proteins responsible for haemoglobin binding and passage of haem iron to the cytoplasm , where it acts as an essential nutrient. IsdA and IsdB are located in the cell wall of staphylococci. IsdA appear to be exposed on the surface of bacterium since it is susceptible to proteinase K digestion. IsdB was partially digested suggesting that it is partially exposed on the surface of the bacterium (Mazmanian et al 2003 Science 299; 906).

IsdA and IsdB are both 29kDa proteins which bind heme. Their expression is regulated by the availability of iron via the Fur repressor. Their expression will be high during infection in a host where the concentration of iron will be low.

They are also known as FrpA and FrpB (Morrissey et al 2002, Infect. Immun. 70; 2399). FrpA and FrpB are major surface proteins with a high charge. They have been shown to provide a major contribution to adhesion to plastic.

In an embodiment, the immunogenic composition of the invention comprises a fragment of IsdA and/or IsdB which is described in WO 01/98499 or WO 03/11899.

### Toxins and regulators of virulence

Members of this family of proteins include toxin such as alpha toxin, hemolysin, enterotoxin B and TSST-1 as well as proteins that regulate the production of toxins such as RAP.

### Alpha toxin (Hla)

Alpha toxin is an important virulence determinant produced by most strains of *S. aureus.* It is a pore forming toxin with haemolytic activity. Antibodies against alpha toxin have been shown to neutralise the detrimental and lethal effects of alpha toxin in animal models (Adlam et al 1977 Infect. Immun. 17; 250). Human platelets, endothelial cells and mononuclear cells are susceptible to the effects of alpha toxin.

The high toxicity of alpha toxin requires that it should be detoxified before being used as an immunogen. This can be achieved by chemical treatment, for instance by treating with formaldehyde, glutaraldehyde of other cross-linking reagents or by chemically conjugating it to bacterial polysaccharides as described below.

A further way of removing toxicity is to introduce point mutations that remove toxicity while retaining the antigenicity of the toxin. The introduction of a point mutation at amino acid 35 of alpha toxin where a histidine residue is replaced with a leucine residue results in the removal of toxicity whilst retaining immunogenicity (Menzies and Kernodle 1996; Infect. Immun. 64; 1839). Histidine 35 appears to be critical for the proper oligomerization required for pore formation and mutation of this residue leads to loss of toxicity.

When incorporated into immunogenic compositions of the invention, alpha toxin is optionally detoxified by mutation of His 35, for example by replacing His 35 with Leu or Arg. In an alternative embodiment, alpha toxin is detoxified by conjugation to other components of the immunogenic composition, for example capsular polysaccharides or PNAG, optionally to *S. aureus* type 5 polysaccharide and/or *S. aureus* Type 8 polysaccharide and/or PNAG.

### RNA III activating protein (RAP)

RAP is not itself a toxin, but is a regulator of the expression of virulence factors. RAP is produced and secreted by staphylococci. It activates the agr regulatory system of other staphylococci and activates the expression and subsequent release of virulence factors such as hemolysin, enterotoxin B and TSST-1.

### Other immunodominant proteins

### Accumulation-associated protein (Aap)

Aap is a 140kDa protein which is essential for the accumulation of *S. epidermidis* strains on surfaces (Hussain et al Infect. Immun. 1997, 65; 519). Strains expressing this protein produced significantly larger amounts of biofilm and Aap appear to be involved in biofilm formation. Antibodies against Aap are able to inhibit biofilm formation and inhibit the accumulation of *S. epidermidis.*

### Staphylococcal Secretory antigen SsaA

SsaA is a strongly immunogenic protein of 30kDa found in both S. aureus and S. epidermidis (Lang et al 2000 FEMS Immunol. Med. Microbiol. 29; 213). Its expression during endocarditis suggested a virulence role specific to the pathogenesis of the infectious disease.

SsaA contains an N-terminal leader sequence and a signal peptidase cleavage site. The leader peptide is followed by a hydrophilic region of approximately 100 amino acids from residue 30 to residue 130.

An optional fragment of SsaA to be incorporated into the immunogenic composition of the invention is made up of the mature protein (amino acids 27 to the C-terminus or amino acids 30 to the C-terminus).

A further optional fragments contains the hydrophilic area of SsaA from amino acid 30 to amino acid 130.

### Combinations

Staphylococcal infections progress through several different stages. For example, the staphylococcal life cycle involves commensal colonisation, initiation of infection by accessing adjoining tissues or the bloodstream, anaerobic multiplication in the blood, interplay between *S. aureus* virulence determinants and the host defence mechanisms and induction of complications including endocarditis, metastatic abscess formation and sepsis syndrome. Different molecules on the surface of the bacterium will be involved in different steps of the infection cycle. By targeting the immune response against a combination of particular antigens involved in different processes of Staphylococcal infection, multiple aspects of staphylococcal function are affected and this can result in good vaccine efficacy.

In particular, combinations of certain antigens from different classes, some of which are involved in adhesion to host cells, some of which are involved in iron acquisition or other transporter functions, some of which are toxins or regulators of virulence and immunodominant antigens can elicit an immune response which protects against multiple stages of infection.

Some combinations of antigens are particularly effective at inducing an immune response. This can be measured either in animal model assays as described in the examples and/or using an opsonophagocytic assay as described in the examples. Without wishing to be bound by theory, such effective combinations of antigens are thought to be enabled by a number of characteristics of the immune response to the antigen combination. The antigens themselves are usually exposed on the surface of Staphylococcal cells, they tend to be conserved but also tend not to be present in sufficient quantity on the surface cell for an optimal bactericidal response to take place using antibodies elicited against the single antigen. Combining the antigens of the invention can result in a formulation eliciting an advantageous combination of antibodies which interact with the Staphylococcal cell beyond a critical threshold. At this critical level, sufficient antibodies of sufficient quality bind to the surface of the bacterium to allow either efficient killing by complement or neutralisation of the bacterium. This can be measured in either an animal challenge model or an opsonisation assay as described in the examples.

Preferred immunogenic compositions of the invention comprise a plurality of proteins selected from at least two different categories of protein, having different functions within Staphylococci. Examples of such categories of proteins are extracellular binding proteins, transporter proteins such as Fe acquisition proteins, toxins or regulators of virulence and other immunodominant proteins.

In a preferred embodiment, immunogenic composition of the invention further comprises a number of proteins equal to or greater than 2, 3, 4, 5 or 6 selected from 2, 3 or 4 different groups selected from;
● Group a) extracelular component binding proteins;
● Group b) transporter proteins;
● Group c) toxins or regulators of virulence
● Group d) structural proteins.

In a preferred embodiment, immunogenic composition of the invention further comprises a number of proteins equal to or greater than 2, 3, 4, 5 or 6 selected from 2, 3 or 4 of the following groups:
● group a) - at least one staphylococcal extracellular component binding protein or fragment thereof selected from the group consisting of laminin receptor, SitC/MntC/saliva binding protein, EbhA, EbhB, Elastin binding protein (EbpS), EFB (FIB), SBI, ClfA, SdrC, SdrD, SdrE, SdrG, SdrH, SasF, lipase GehD, SasA, SasB, SasC, SasD, SasK, FnbA, FnbB, Cna, ClfB, FbpA, Npase, IsaA/PisA, SsaA, EPB, SSP-1, SSP-2, HBP, Vitronectin binding protein, fibrinogen binding protein, coagulase, Fig and MAP;
● group b) - at least one staphylococcal transporter protein or fragment thereof selected from the group consisting of Immunodominant ABC transporter, IsdA, IsdB, IsdC, Mg2+ transporter, HarA, SitC and Ni ABC transporter;
● group c) - at least one staphylococcal regulator of virulence, toxin or fragment thereof selected from the group consisting of alpha toxin (Hla), alpha toxin H35R mutant, RNA III activating protein (RAP);
● group d) - at least one staphylococcal structural protein or immunogenic fragment thereof selected from the group consisting of MRPII and autolysin.

In a preferred embodiment, the immunogenic composition of the invention comprises a number of proteins equal to or greater than 2, 3, 4, 5 or 6 selected from 2 or 3 of the following groups:
● group a) - at least one staphylococcal extracellular component binding protein or immunogenic fragment thereof selected from the group consisting of laminin receptor, SitC/MntC/saliva binding protein, EbhA, EbhB, Elastin binding protein (EbpS), EFB (FIB), SBI, autolysin, ClfA, SdrC, SdrD, SdrE, SdrG, SdrH, SasF, Lipase GehD, SasA, , SasB, SasC, SasD, SasK, FnbA, FnbB, Cna, ClfB, FbpA, Npase, IsaA/PisA, SsaA, EPB, SSP-1, SSP-2, HBP, Vitronectin binding protein, fibrinogen binding protein, coagulase, Fig and MAP;
● group b) - at least one staphylococcal transporter protein or immunogenic fragment thereof selected from the group consisting of Immunodominant ABC transporter, IsdA, IsdB, IsdC, HarA, Mg2+ transporter, SitC and Ni ABC transporter;
● group c) - at least one staphylococcal regulator of virulence, toxin or immunogenic fragment thereof selected from the group consisting of alpha toxin (Hla), alpha toxin H35R mutant, RNA III activating protein (RAP).

In a preferred embodiment, the immunogenic composition of the invention contains at least one protein selected from group a) and an additional protein selected from group b) and/or group c).

In a further embodiment, the immunogenic composition of the invention contains at least one antigen selected from group b) and an additional protein selected from group c) and/or group a).

In a further embodiment, the immunogenic composition of the invention contains at least one antigen selected from group c) and an additional protein selected from group a) and/or group b).

An optional combination of proteins in the immunogenic composition of the invention comprises laminin receptor and 1, 2, 3, 4 or 5 further antigens selected from the group consisting of immunodominant ABC transporter, IsdA, IsdB, IsdC, HarA, Mg2+ transporter, SitC, Ni ABC transporter, alpha toxin, alpha toxin H35L or H35R mutant, RAP, Aap and SsaA.

A further combination of proteins in the immunogenic composition of the invention comprises SitC and 1, 2, 3, 4 or 5 further antigens selected from the group consisting of immunodominant ABC transporter, IsdA, IsdB, IsdC, HarA, Mg2+ transporter, SitC, Ni ABC transporter, alpha toxin, alpha toxin H35L or H35R mutant, RAP, Aap and SsaA.

A further combination of proteins in the immunogenic composition of the invention comprises EbhA and 1, 2, 3, 4 or 5 further antigens selected from the group consisting of immunodominant ABC transporter, IsdA, IsdB, IsdC, HarA, Mg2+ transporter, SitC, Ni ABC transporter, alpha toxin, alpha toxin H35L or H35R mutant, RAP, Aap and SsaA.

A further combination of proteins in the immunogenic composition of the invention comprises EbhB and 1, 2, 3, 4 or 5 further antigens selected from the group consisting of immunodominant ABC transporter, IsdA, IsdB, IsdC, HarA, Mg2+ transporter, SitC, Ni ABC transporter, alpha toxin, alpha toxin H35L or H35R mutant, RAP, Aap and SsaA.

A further combination of proteins in the immunogenic composition of the invention comprises EbpS and 1, 2, 3, 4 or 5 further antigens selected from the group consisting of immunodominant ABC transporter, IsdA, IsdB, IsdA, HarA, Mg2+ transporter, SitC, Ni ABC transporter, alpha toxin, alpha toxin H35L or H35R mutant, RAP, Aap and SsaA.

A further combination of proteins in the immunogenic composition of the invention comprises EFB(FIB) and 1, 2, 3, 4 or 5 further antigens selected from the group consisting of immunodominant ABC transporter, IsdA, IsdB, IsdC, HarA, Mg2+ transporter, SitC, Ni ABC transporter, alpha toxin, alpha toxin H35L or H35R mutant, RAP, Aap and SsaA.

A further combination of proteins in the immunogenic composition of the invention comprises SBI and 1, 2, 3, 4 or 5 further antigens selected from the group consisting of immunodominant ABC transporter, IsdA, IsdB, IsdC, HarA, Mg2+ transporter, SitC, Ni ABC transporter, alpha toxin, alpha toxin H35L or H35R mutant, RAP, Aap and SsaA.

A further combination of proteins in the immunogenic composition of the invention comprises autolysin and 1, 2, 3, 4 or 5 further antigens selected from the group consisting of immunodominant ABC transporter, IsdA, IsdB, IsdC, HarA, Mg2+ transporter, SitC, Ni ABC transporter, alpha toxin, alpha toxin H35L or H35R mutant, RAP, Aap and SsaA.

A further combination of proteins in the immunogenic composition of the invention comprises ClfA and 1, 2, 3, 4 or 5 further antigens selected from the group consisting of immunodominant ABC transporter, IsdA, IsdB, IsdC, HarA, Mg2+ transporter, SitC, Ni ABC transporter, alpha toxin, alpha toxin H35L or H35R mutant, RAP, Aap and SsaA.

A further combination of proteins in the immunogenic composition of the invention comprises SdrC and 1, 2, 3, 4 or 5 further antigens selected from the group consisting of immunodominant ABC transporter, IsdA, IsdB, IsdC, HarA, Mg2+ transporter, SitC, Ni ABC transporter, alpha toxin, alpha toxin H35L or H35R mutant, RAP, Aap and SsaA.

A further combination of proteins in the immunogenic composition of the invention comprises SdrD and 1, 2, 3, 4 or 5 further antigens selected from the group consisting of immunodominant ABC transporter, IsdA, IsdB, IsdC, HarA, Mg2+ transporter, SitC, Ni ABC transporter, alpha toxin, alpha toxin H35L or H35R mutant, RAP, Aap and SsaA.

A further combination of proteins in the immunogenic composition of the invention comprises SdrE and 1, 2, 3, 4 or 5 further antigens selected from the group consisting of immunodominant ABC transporter, IsdA, IsdB, IsdC, HarA, Mg2+ transporter, SitC, Ni ABC transporter, alpha toxin, alpha toxin H35L or H35R mutant, RAP, Aap and SsaA.

A further combination of proteins in the immunogenic composition of the invention comprises SdrG and 1, 2, 3, 4 or 5 further antigens selected from the group consisting of immunodominant ABC transporter, IsdA, IsdB, IsdC. HarA, Mg2+ transporter, SitC, Ni ABC transporter, alpha toxin, alpha toxin H35L or H35R mutant and RAP.

A further combination of proteins in the immunogenic composition of the invention comprises SdrH and 1, 2, 3, 4 or 5 further antigens selected from the group consisting of immunodominant ABC transporter, IsdA, IsdB, IsdC, HarA, Mg2+ transporter, SitC, Ni ABC transporter, alpha toxin, alpha toxin H35L or H35R mutant, RAP, Aap and SsaA.

A further combination of proteins in the immunogenic composition of the invention comprises SasF and 1, 2, 3, 4 or 5 further antigens selected from the group consisting of immunodominant ABC transporter, IsdA, IsdB, IsdC, HarA, Mg2+ transporter, SitC, Ni ABC transporter, alpha toxin, alpha toxin H35L or H35R mutant, RAP, Aap and SsaA.

A further combination of proteins in the immunogenic composition of the invention comprises Lipase GehD and 1, 2, 3, 4 or 5 further antigens selected from the group consisting of immunodominant ABC transporter, IsdA, IsdB, IsdC, HarA, Mg2+ transporter, SitC, Ni ABC transporter, alpha toxin, alpha toxin H35L or H35R mutant, RAP, Aap and SsaA.

A further combination of proteins in the immunogenic composition of the invention comprises SasF and 1, 2, 3, 4 or 5 further antigens selected from the group consisting of immunodominant ABC transporter, IsdA, IsdB, IsdC, HarA, Mg2+ transporter, SitC, Ni ABC transporter, alpha toxin, alpha toxin H35L or H35R mutant, RAP, Aap and SsaA.

A further combination of proteins in the immunogenic composition of the invention comprises FnbA and 1, 2, 3, 4 or 5 further antigens selected from the group consisting of immunodominant ABC transporter, IsdA, IsdB, IsdC, HarA, Mg2+ transporter, SitC, Ni ABC transporter, alpha toxin, alpha toxin H35L or H35R mutant, RAP, Aap and SsaA.

A further combination of proteins in the immunogenic composition of the invention comprises FnbB and 1, 2, 3, 4 or 5 further antigens selected from the group consisting of immunodominant ABC transporter, IsdA, IsdB, IsdC, HarA, Mg2+ transporter, SitC, Ni ABC transporter, alpha toxin, alpha toxin H35L or H35R mutant, RAP, Aap and SsaA.

A further combination of proteins in the immunogenic composition of the invention comprises Cna and 1, 2, 3, 4 or 5 further antigens selected from the group consisting of immunodominant ABC transporter, IsdA, IsdB, IsdC, HarA, Mg2+ transporter, SitC, Ni ABC transporter, alpha toxin, alpha toxin H35L or H35R mutant, RAP, Aap and SsaA.

A further combination of proteins in the immunogenic composition of the invention comprises ClfB and 1, 2, 3, 4 or 5 further antigens selected from the group consisting of immunodominant ABC transporter, IsdA, IsdB, IsdC, HarA, Mg2+ transporter, SitC, Ni ABC transporter, alpha toxin, alpha toxin H35L or H35R mutant, RAP, Aap and SsaA.

A further combination of proteins in the immunogenic composition of the invention comprises FbpA and 1, 2, 3, 4 or 5 further antigens selected from the group consisting of immunodominant ABC transporter, IsdA, IsdB, IsdC, HarA, Mg2+ transporter, SitC, Ni ABC transporter, alpha toxin, alpha toxin H35L or H35R mutant, RAP, Aap and SsaA.

A further combination of proteins in the immunogenic composition of the invention comprises Npase and 1, 2, 3, 4 or 5 further antigens selected from the group consisting of immunodominant ABC transporter, IsdA, IsdB, IsdC, HarA, Mg2+ transporter, SitC, Ni ABC transporter, alpha toxin, alpha toxin H35L or H35R mutant, RAP, Aap and SsaA.

A further combination of proteins in the immunogenic composition of the invention comprises IsaA/PisA and 1, 2, 3, 4 or 5 further antigens selected from the group consisting of immunodominant ABC transporter, IsdA, IsdB, IsdC, HarA, Mg2+ transporter, SitC, Ni ABC transporter, alpha toxin, alpha toxin H35L or H35R mutant, RAP, Aap and SsaA.

A further combination of proteins in the immunogenic composition of the invention comprises SsaA and 1, 2, 3, 4 or 5 further antigens selected from the group consisting of immunodominant ABC transporter, IsdA, IsdB, IsdC, HarA, Mg2+ transporter, SitC, Ni ABC transporter, alpha toxin, alpha toxin H35L or H35R mutant, RAP, Aap and SsaA.

A further combination of proteins in the immunogenic composition of the invention comprises EPB and 1, 2, 3, 4 or 5 further antigens selected from the group consisting of immunodominant ABC transporter, IsdA, IsdB, IsdC, HarA, Mg2+ transporter, SitC, Ni ABC transporter, alpha toxin, alpha toxin H35L or H35R mutant, RAP, Aap and SsaA.

A further combination of proteins in the immunogenic composition of the invention comprises SSP-1 and 1, 2, 3, 4 or 5 further antigens selected from the group consisting of immunodominant ABC transporter, IsdA, IsdB, IsdC, HarA, Mg2+ transporter, SitC, Ni ABC transporter, alpha toxin, alpha toxin H35L or H35R mutant, RAP, Aap and SsaA.

A further combination of proteins in the immunogenic composition of the invention comprises SSP-2 and 1, 2, 3, 4 or 5 further antigens selected from the group consisting of immunodominant ABC transporter, IsdA, IsdB, IsdC, HarA, Mg2+ transporter, SitC, Ni ABC transporter, alpha toxin, alpha toxin H35L or H35R mutant, RAP, Aap and SsaA.

A further combination of proteins in the immunogenic composition of the invention comprises HPB and 1, 2, 3, 4 or 5 further antigens selected from the group consisting of immunodominant ABC transporter, IsdA, IsdB, IsdC, HarA, Mg2+ transporter, SitC, Ni ABC transporter, alpha toxin, alpha toxin H35L or H35R mutant, RAP, Aap and SsaA.

A further combination of proteins in the immunogenic composition of the invention comprises vitronectin binding protein and 1, 2, 3, 4 or 5 further antigens selected from the group consisting of immunodominant ABC transporter, IsdA, IsdB, IsdC, HarA, Mg2+ transporter, SitC, Ni ABC transporter, alpha toxin, alpha toxin H35L or H35R mutant, RAP, Aap and SsaA.

A further combination of proteins in the immunogenic composition of the invention comprises fibrinogen binding protein and 1, 2, 3, 4 or 5 further antigens selected from the group consisting of immunodominant ABC transporter, IsdA, IsdB, IsdC, HarA, Mg2+ transporter, SitC, Ni ABC transporter, alpha toxin, alpha toxin H35L or H35R mutant, RAP, Aap and SsaA.

A further combination of proteins in the immunogenic composition of the invention comprises coagulase and 1, 2, 3, 4 or 5 further antigens selected from the group consisting of immunodominant ABC transporter, IsdA, IsdB, IsdC, HarA, Mg2+ transporter, SitC, Ni ABC transporter, alpha toxin, alpha toxin H35L or H35R mutant, RAP, Aap and SsaA.

A further combination of proteins in the immunogenic composition of the invention comprises Fig and 1, 2, 3, 4 or 5 further antigens selected from the group consisting of immunodominant ABC transporter, IsdA, IsdB, IsdC, HarA, Mg2+ transporter, SitC, Ni ABC transporter, alpha toxin, alpha toxin H35L or H35R mutant, RAP, Aap and SsaA.

A further combination of proteins in the immunogenic composition of the invention comprises MAP and 1, 2, 3, 4 or 5 further antigens selected from the group consisting of immunodominant ABC transporter, IsdA, IsdB, IsdC, HarA, Mg2+ transporter, SitC, Ni ABC transporter, alpha toxin, alpha toxin H35L or H35R mutant, RAP, Aap and SsaA.

A further combination of protein in the immunogenic composition of the invention comprises immunodominant ABC tranporter and 1, 2, 3, 4 or 5 further antigens selected from the group consisting of laminin receptor, SitC/MntC/saliva binding protein, EbhA, EbhB, Elastin binding protein (EbpS), EFB (FIB), SBI, autolysin, ClfA, SdrC, SdrD, SdrE, SdrG, SdrH, Lipase GehD, SasA, FnbA, FnbB, Cna, ClfA, ClfB, FbpA, Npase, IsaA/PisA, SsaA, EPB, SSP-1, SSP-2, HBP, Vitronectin binding protein, fibrinogen binding protein, coagulase, Fig, MAP, alpha toxin, alpha toxin H35L or H35R mutant, RAP, Aap and SsaA.

A further combination of protein in the immunogenic composition of the invention comprises IsdA and 1, 2, 3, 4 or 5 further antigens selected from the group consisting of laminin receptor, SitC/MntC/saliva binding protein, EbhA, EbhB, Elastin binding protein (EbpS), EFB (FIB), SBI, autolysin, ClfA, SdrC, SdrC, SdrE, SdrG, SdrH, SasF, Lipase GehD, SasA, FnbA, FnbB, Cna, ClfA, ClfB, FbpA, Npase, IsaA/PisA, SsaA, EPB, SSP-1, SSP-2, HBP, Vitronectin binding protein, fibrinogen binding protein, coagulase, Fig, MAP, alpha toxin, alpha toxin H35L or H35R mutant, RAP, Aap and SsaA.

A further combination of protein in the immunogenic composition of the invention comprises IsdB and 1, 2, 3, 4 or 5 further antigens selected from the group consisting of laminin receptor, SitC/MntC/saliva binding protein, EbhA, EbhB, Elastin binding protein (EbpS), EFB (FIB), SBI, autolysin, ClfA, SdrC, SdrG, SdrH, SasF, Lipase GehD, SasA, FnbA, FnbB, Cna, ClfA, ClfB, FbpA, Npase, IsaA/PisA, SsaA, EPB, SSP-1, SSP-2, HBP, Vitronectin binding protein, fibrinogen binding protein, coagulase, Fig, MAP, alpha toxin, alpha toxin H35L or H35R mutant, RAP, Aap and SsaA.

A further combination of protein in the immunogenic composition of the invention comprises SitC and 1, 2, 3, 4 or 5 further antigens selected from the group consisting of laminin receptor, SitC/MntC/saliva binding protein, EbhA, EbhB, Elastin binding protein (EbpS), EFB (FIB), SBI, autolysin, ClfA, SdrC, SdrD, SdrE, SdrG, SdrH, SasF, Lipase GehD, SasA, FnbA, FnbB, Cna, ClfA, ClfB, FbpA, Npase, IsaA/PisA, SsaA, EPB, SSP-1, SSP-2, HBP, Vitronectin binding protein, fibrinogen binding protein, coagulase, Fig, MAP, alpha toxin, alpha toxin H35L or H35R mutant, RAP, Aap and SsaA.

A further combination of protein in the immunogenic composition of the invention comprises alpha toxin and 1, 2, 3, 4 or 5 further antigens selected from the group consisting of laminin receptor, SitC/MntC/saliva binding protein, EbhA, EbhB, Elastin binding protein (EbpS), EFB (FIB), SBI, autolysin, ClfA, SdrC, SdrD, SdrE, SdrG, SdrH, SasF, Lipase GehD, SasA, FnbA, FnbB, Cna, ClfB, FbpA, Npase, IsaA/PisA, SsaA, EPB, SSP-1, SSP-2, HBP, Vitronectin binding protein, fibrinogen binding protein, coagulase, Fig, MAP, immunodominant ABC transporter, IsdA, IsdB, IsdC, HarA, Mg2+ transporter, SitC, Ni ABC transporter, Aap and SsaA.

A further combination of protein in the immunogenic composition of the invention comprises alpha toxin H35L OR H35R variant and 1, 2, 3, 4 or 5 further antigens selected from the group consisting of laminin receptor, SitC/MntC/saliva binding protein, EbhA, EbhB, Elastin binding protein (EbpS), EFB (FIB), SBI, autolysin, ClfA, SdrC, SdrD, SdrE, SdrG, SdrH, SasF, Lipase GehD, SasA, FnbA, FnbB, Cna, ClfB, FbpA, Npase, IsaA/PisA, SsaA, EPB, SSP-1, SSP-2, HBP, Vitronectin binding protein, fibrinogen binding protein, coagulase, Fig, MAP, immunodominant ABC transporter, IsdA, IsdB, IsdC, HarA, Mg2+ transporter, SitC, Ni ABC transporter, Aap and SsaA.

A further combination of protein in the immunogenic composition of the invention comprises RAP and 1, 2, 3, 4 or 5 further antigens selected from the group consisting of laminin receptor, SitC/MntC/saliva binding protein, EbhA, EbhB, Elastin binding protein (EbpS), EFB (FIB), SBI, autolysin, ClfA, SdrC, SdrD, SdrE, SdrG, SdrH, SasF, Lipase GehD, SasA, FnbA, FnbB, Cna, ClfB, FbpA, Npase, IsaA/PisA, SsaA, EPB, SSP-1, SSP-2, HBP, Vitronectin binding protein, fibrinogen binding protein, coagulase, Fig, MAP, immunodominant ABC transporter, IsdA, IsdB, IsdC, HarA, Mg2+ transporter, SitC, Ni ABC transporter, Aap and SsaA.

A further combinations of protein in the immunogenic composition of the invention comprises IsdA and IsdB; IsdA and ClfA; IsdA and ClfB; IsdA and SdrC; IsdA and SdrD; IsdA and SdrE; IsdA and SdrG; IsdA and SasF;IsdB and ClfA; IsdB and ClfB; IsdB and SdrC; IsdB and SdrD; IsdB and SdrE; IsdB and SdrG; IsdB and SasF; ClfA and ClfB; ClfA and SdrC; ClfA and SdrD; ClfA and SdrE; ClfA and SasF; ClfB and SdrC; ClfB and SdrD; ClfB and SdrE; ClfB and SasF; SdrC and SdrD; SdrC and SdrE; SdrC and SasF; SdrD and SdrE; SdrD and SasF; SdrE and SasF.

In the above and below combinations, the specified proteins may optionally be present in the immunogenic composition of the invention as a fragment or fusion protein as described above.

### Combinations of three proteins

In an embodiment, the immunogenic composition of the invention further comprises three protein components in a combination of alpha-toxin, an extracellular component binding protein (for example an adhesin) and a transporter protein (for example an iron-binding protein).

In such a combination, the alpha toxin may be chemically detoxified or genetically detoxified by introduction of point mutation(s), for example the His35Leu point mutation. The alpha toxin is present as a free protein or alternatively is conjugated to a polysaccharide or PNAG component of the immunogenic composition.

### Examples of combinations include:-

An immunogenic composition comprising alpha toxin, IsdA and an extracellular component binding protein selected from the group consisting of laminin receptor, SitC/MntC/saliva binding protein, EbhA, EbhB, Elastin binding protein (EbpS), EFB (FIB), SBI, autolysin, ClfA, SdrC, SdrG, SdrH, Lipase GehD, SasA, FnbA, FnbB, Cna, ClfB, FbpA, Npase, IsaA/PisA, SsaA, EPB, SSP-1, SSP-2, HBP, Vitronectin binding protein, fibrinogen binding protein, coagulase, Fig and MAP.

An immunogenic composition comprising alpha toxin, IsdB and an extracellular component binding protein selected from the group consisting of laminin receptor, SitC/MntC/saliva binding protein, EbhA, EbhB, Elastin binding protein (EbpS), EFB (FIB), SBI, autolysin, ClfA, SdrC, SdrD, SdrE, SdrG, SdrH, Lipase GehD, SasA, FnbA, FnbB, Cna, ClfB, FbpA, Npase, IsaA/PisA, SsaA, EPB, SSP-1, SSP-2, HBP, Vitronectin binding protein, fibrinogen binding protein, coagulase, Fig and MAP.

An immunogenic composition comprising alpha toxin, IsdA and an adhesin selected from the group consisting of laminin receptor, EbhA, EbhB, Elastin binding protein (EbpS), EFB (FIB), ClfA, SdrC, SdrD, SdrE, SdrG, SdrH, autolysin, FnbA, FnbB, Cna, ClfB, FbpA, Npase, SSP-1, SSP-2, Vitronectin binding protein, fibrinogen binding protein, coagulase, Fig and MAP.

An immunogenic composition comprising alpha toxin, IsdB and an adhesin selected from the group consisting of laminin receptor, EbhA, EbhB, Elastin binding protein (EbpS), EFB (FIB), autolysin, ClfA, SdrC, SdrD, SdrE, SdrG, SdrH, FnbA, FnbB, Cna, ClfB, FbpA, Npase, SSP-1, SSP-2, Vitronectin binding protein, fibrinogen binding protein, coagulase, Fig and MAP.

An immunogenic composition comprising alpha toxin, IsdA and laminin receptor.

An immunogenic composition comprising alpha toxin, IsdA and EbhA.

An immunogenic composition comprising alpha toxin, IsdA and EbhB.

An immunogenic composition comprising alpha toxin, IsdA and EbpS.

An immunogenic composition comprising alpha toxin, IsdA and EFB (FIB).

An immunogenic composition comprising alpha toxin, IsdA and SdrG.

An immunogenic composition comprising alpha toxin, IsdA and ClfA.

An immunogenic composition comprising alpha toxin, IsdA and ClfB.

An immunogenic composition comprising alpha toxin, IsdA and FnbA.

An immunogenic composition comprising alpha toxin, IsdA and coagulase.

An immunogenic composition comprising alpha toxin, IsdA and Fig.

An immunogenic composition comprising alpha toxin, IsdA and SdrH.

An immunogenic composition comprising alpha toxin, IsdA and SdrC.

An immunogenic composition comprising alpha toxin, IsdA and SdrD.

An immunogenic composition comprising alpha toxin, IsdA and SdrE.

An immunogenic composition comprising alpha toxin, IsdA and MAP.

An immunogenic composition comprising IsaA and Sbi.

An immunogenic composition comprising IsaA and IsdB.

An immunogenic composition comprising IsaA and IsdA.

An immunogenic composition comprising IsaA and SdrC.

An immunogenic composition comprising IsaA and Ebh or fragment thereof as described above.

An immunogenic composition comprising Sbi and SdrC.

An immunogenic composition comprising Sbi and Ebh or fragment thereof as described above.

An immunogenic composition of the invention comprising IsaA, Sbi or SdrC

### Selection of antigens expressed in different clonal lineages

Analysis of the occurrence of virulence factors in relation with the population structure of *Staphylococcus aureus* showed variable presence of virulence genes in natural populations of *S*. *aureus.*

Among clinical isolates of *Staphylococcus aureus*, at least five clonal lineages were shown to be highly prevalent (Booth et al., 2001 Infect Immun. 69(1):345-52). Alpha-hemolysin (*hla*), fibronectin-binding protein A (*fnbA*) and clumping factor A (*clfA*) were shown to be present in most of the isolates, regardless of lineage identity, suggesting an important role of these proteins in the survival of *S. aureus* (Booth et al., 2001 Infect Immun. 69(1):345-52). Moreover, according to Peacock et al. 2002 the distributions of *fnbA*, *clfA*, coagulase, *spa*, *map*, *pvl* (Panton-Valentine leukocidin), *hlg* (gamma-toxin), alpha-toxin and *ica* appeared to be unrelated to the underlying clonal structure suggesting considerable horizontal transfer of these genes.

In contrary, other virulence genes such as fibronectin binding protein B (*fnbB*), betahemolysin (*hlb*), collagen binding protein (c*n*a), TSST-1 (*tst*) and methicillin resistance gene (*mecA*) are strongly associated with specific lineages (Booth et al., 2001 Infect Immun. 69(1):345-52). Similarly, Peacock et al. 2002 (Infect Immun. 70(9):4987-96) showed that the distributions of the enterotoxins, *tst*, the exfolatins (*eta* and *etb*), beta-and delta-toxins, the *sdr* genes (*sdr*D, *sdr*E and *bbp*), *cna*, *ebp*S and efb within the population are all highly significantly related to MLST-derived clonal complexes.

MLST data provide no evidence that strains responsible for nosocomial disease represent a distinct subpopulation from strains causing community-acquired disease or strains recovered from asymptomatic carriers (Feil et al., 2003 J Bacteriol. 185(11):3307-16).

In an embodiment, immunogenic compositions of the invention are effective against staphylococci from different clonal lineages.

In an embodiment, the immunogenic composition comprises 1, 2, 3, 4, or at least 1 protein that is expressed in most isolates of staphylococci. Examples of such proteins include alpha-hemolysin (*hla*), fibronectin-binding protein A (*fnbA*) and clumping factor A (*clfA*), coagulase, *spa*, *map*, *pvl* (Panton-Valentine leukocidin), *hlg* (gamma-toxin) , *ica*, immunodominant ABC transporter, RAP, autolysin (Rupp et al 2001, J. Infect. Dis. 183; 1038), laminin receptors, SitC, IsaA/PisA, SPOIIIE (), SsaA, EbpS, SasF (Roche et al 2003, Microbiology 149; 643), EFB(FIB), SBI, ClfB, IsdA, IsdB, FnbB, Npase, EBP, Bone sialo binding protein II, IsaB/PisB (Lorenz et al FEMS Immuno. Med. Microb. 2000, 29; 145), SasH (Roche et al 2003, Microbiology 149; 643), MRPI, SasD (Roche et al 2003, Microbiology 149; 643), SasH (Roche et al 2003, Microbiology 149; 643), aureolysin precursor (AUR)/Sepp1 and novel autolysin.

In an alternative embodiment, 2 or more proteins which are expressed in different sets of clonal strains are included in the immunogenic composition of the invention. Optionally the combination of antigens will allow an immune response to be generated that is effective against multiple clonal strains, or against all clonal stains. For example combinations include FnbB and betahemolysin, FnbB and Cna, FnbB and TSST-1, FnbB and mecA, FnbB and SdrD, FnbB and SdrF, FnbB and EbpS, FnbB and Efb, beta-haemolysin and Cna, beta-haemolysin and TSST-1, beta-haemolysin and mecA, beta-haemolysin and SdrD, beta-haemolysin and SdrF, beta-haemolysin and EbpS, beta-haemolysin and Efb, Cna and TSST-1, Cna and mecA, Cna and SdrD, Cna and SdrF, Cna and EbpS, Cna and Efb, TSST-1 and mecA, TSST-1 and SdrD, TSST-1 and SdrF, TSST-1 and EbpS, TssT-1 and Efb, MecA and SdrD, MecA and SdrF, MecA and EbpS, MecA and Efb, SdrD and SdrF, SdrD and EbpS, SdeD and Efb, SdrF and EbpS, SdrF and Efb, and, EbpS and Efb.

The combinations described above may be combined with additional components described above.

### Protection against S. aureus and S. epidermidis

In an embodiment of the invention the immunogenic composition provides an effective immune response against more than one strain of staphylococci, for example against strains from both *S. aureus* and *S. epidermidis.* For example, a protective immune response is generated against type 5 and 8 serotypes of *S. aureus.*

One use of the immunogenic composition of the invention is to prevent nosocomial infections, for instance in elective surgery patients, by inoculating prior to hospital treatment. At this stage, it is difficult to accurately predict which staphylococcal strains the patient will be exposed to. It is therefore advantageous to inoculate with a vaccine that is capable of generating an effective immune response against various strains of staphylococci.

An effective immune response is defined as an immune response that gives significant protection in a mouse challenge model or opsonophagocytosis assay as described in the examples. Significant protection in a mouse challenge model, for instance that of example 5, is defined as an increase in the LD50 in comparison with carrier inoculated mice of at least 10%, 20%, 50%, 100% or 200%. Significant protection in a cotton rat challenge model, for instance that of example 8, is defined as a decrease in the mean observed LogCFU/nose of at least 10%, 20%, 50%, 70% or 90%. The presence of opsonising antibodies is known to correlate with protection, therefore significant protection is indicated by a decrease in the bacterial count of at least 10%, 20%, 50%, 70% or 90% in an opsonophagocytosis assay, for instance that of example 7.

Several of the proteins including immunodominant ABC transporter, RNA III activating protein, Laminin receptors, SitC, IsaA/PisA, SsaA, EbhA/EbhB, EbpS and Aap are well conserved between *S. aureus* and *S. epidermidis* and example 8 shows that IsaA, ClfA, IsdB, SdrG, HarA, FnbpA and Sbi can generate a cross-reactive immune response (for example crossreactive between at least one *S. aureus* and at least one *S. epidermidis* strain). PIA is also well conserved between *S. aureus* and *S. epidermidis.*

Therefore in an embodiment, the immunogenic composition of the invention will comprise PNAG and type 5 and 8 polysaccharides and one, two, three or four of the above proteins.

### Vaccines

In an embodiment, the immunogenic composition of the invention is mixed with a pharmaceutically acceptable excipient, and optioanlly with an adjuvant to form a vaccine.

The vaccines of the present invention may be adjuvanted, particularly when intended for use in an elderly population but also for use in infant populations. Suitable adjuvants include an aluminum salt such as aluminum hydroxide gel or aluminum phosphate or alum, but may also be other metal salts such as those of calcium, magnesium, iron or zinc, or may be an insoluble suspension of acylated tyrosine, or acylated sugars, cationically or anionically derivatized saccharides, or polyphosphazenes.

It is preferred that the adjuvant be selected to be a preferential inducer of a TH1 type of response. Such high levels of Th1-type cytokines tend to favour the induction of cell mediated immune responses to a given antigen, whilst high levels of Th2-type cytokines tend to favour the induction of humoral immune responses to the antigen.

The distinction of Th1 and Th2-type immune response is not absolute. In reality an individual will support an immune response which is described as being predominantly Th1 or predominantly Th2. However, it is often convenient to consider the families of cytokines in terms of that described in murine CD4 +ve T cell clones by Mosmann and Coffman (Mosmann, T.R. and Coffman, R.L. (1989) TH1 and TH2 cells: different patterns of lymphokine secretion lead to different functional properties. (Annual Review of Immunology, 7, p145-173). Traditionally, Th1-type responses are associated with the production of the INF-γ and IL-2 cytokines by T-lymphocytes. Other cytokines often directly associated with the induction of Th1-type immune responses are not produced by T-cells, such as IL-12. In contrast, Th2-type responses are associated with the secretion of 11-4, IL-5, IL-6, IL-10. Suitable adjuvant systems which promote a predominantly Th1 response include: Monophosphoryl lipid A or a derivative thereof (or detoxified lipid A in general - see for instance WO2005107798), particularly 3-de-O-acylated monophosphoryl lipid A (3D-MPL) (for its preparation see GB 2220211 A); and a combination of monophosphoryl lipid A, preferably 3-de-O-acylated monophosphoryl lipid A, together with either an aluminum salt (for instance aluminum phosphate or aluminum hydroxide) or an oil-in-water emulsion. In such combinations, antigen and 3D-MPL are contained in the same particulate structures, allowing for more efficient delivery of antigenic and immunostimulatory signals. Studies have shown that 3D-MPL is able to further enhance the immunogenicity of an alum-adsorbed antigen [Thoelen et al. Vaccine (1998) 16:708-14; EP 689454-B1].

An enhanced system involves the combination of a monophosphoryl lipid A and a saponin derivative, particularly the combination of QS21 and 3D-MPL as disclosed in WO 94/00153, or a less reactogenic composition where the QS21 is quenched with cholesterol as disclosed in WO 96/33739. A particularly potent adjuvant formulation involving QS21, 3D-MPL and tocopherol in an oil in water emulsion is described in WO 95/17210. In one embodiment the immunogenic composition additionally comprises a saponin, which may be QS21. The formulation may also comprise an oil in water emulsion and tocopherol (WO 95/17210). Unmethylated CpG containing oligonucleotides (WO 96/02555) and other immunomodulatory oligonucleotides (WO0226757 and WO03507822) are also preferential inducers of a TH1 response and are suitable for use in the present invention.

Particular adjuvants are those selected from the group of metal Salts, oil in water emulsions, Toll like receptors agonist, (in particular Toll like receptor 2 agonist, Toll like receptor 3 agonist, Toll like receptor 4 agonist, Toll like receptor 7 agonist, Toll like receptor 8 agonist and Toll like receptor 9 agonist), saponins or combinations thereof.

An adjuvant that can be used with the vaccine compositions of the invention are bleb or outer membrane vesicle preparations from Gram negative bacterial strains such as those taught by WO02/09746 - particularly *N. meningitidis* blebs. Adjuvant properties of blebs can be improved by retaining LOS (lipooligosacccharide) on its surface (e.g. through extraction with low concentrations of detergent [for instanct 0-0.1 % deoxycholate]). LOS can be detoxified through the msbB(-) or htrB(-) mutations discussed in WO02/09746. Adjuvant properties can also be improved by retaining PorB (and optionally removing PorA) from meningococcal blebs. Adjuvant properties can also be improved by truncating the outer core saccharide structure of LOS on meningococcal blebs - for instance via the IgtB(-) mutation discussed in WO2004/014417. Alternatively, the aforementioned LOS (e.g. isolated from a msbB(-) and/or IgtB(-) strain) can be purified and used as an adjuvant in the compositions of the invention.

A further adjuvant which may be used with the compositions of the invention may be selected from the group: a saponin, lipid A or a derivative thereof, an immunostimulatory oligonucleotide, an alkyl glucosaminide phosphate, an oil in water emulsion or combinations thereof. A further preferred adjuvant is a metal salt in combination with another adjuvant. It is preferred that the adjuvant is a Toll like receptor agonist in particular an agonist of a Toll like receptor 2, 3, 4, 7, 8 or 9, or a saponin, in particular Qs21. It is further preferred that the adjuvant system comprises two or more adjuvants from the above list. In particular the combinations preferably contain a saponin (in particular Qs21) adjuvant and/or a Toll like receptor 9 agonist such as a CpG containing immunostimulatory oligonucleotide. Other preferred combinations comprise a saponin (in particular QS21) and a Toll like receptor 4 agonist such as monophosphoryl lipid A or its 3 deacylated derivative, 3 D - MPL, or a saponin (in particular QS21) and a Toll like receptor 4 ligand such as an alkyl glucosaminide phosphate.

Particularly preferred adjuvants are combinations of 3D-MPL and QS21 (EP 0 671 948 B1), oil in water emulsions comprising 3D-MPL and QS21 (WO 95/17210, WO 98/56414), or 3D-MPL formulated with other carriers (EP 0 689 454 B1). Other preferred adjuvant systems comprise a combination of 3 D MPL , QS21 and a CpG oligonucleotide as described in US6558670, US6544518.

In an embodiment the adjuvant is a Toll like receptor (TLR) 4 ligand, preferably an agonist such as a lipid A derivative particularly monophosphoryl lipid A or more particularly 3 Deacylated monophoshoryl lipid A (3 D - MPL).

3 D -MPL is available from GlaxoSmithKIine Biologicals North America and primarily promotes CD4+ T cell responses with an IFN-g (Th1) phenotype . It can be produced according to the methods disclosed in GB 2 220 211 A. Chemically it is a mixture of 3-deacylated monophosphoryl lipid A with 3, 4, 5 or 6 acylated chains. Preferably in the compositions of the present invention small particle 3 D- MPL is used. Small particle 3 D -MPL has a particle size such that it may be sterile-filtered through a 0.22µm filter. Such preparations are described in International Patent Application No. WO 94/21292. Synthetic derivatives of lipid A are known and thought to be TLR 4 agonists including, but not limited to:

OM174 (2-deoxy-6-o-[2-deoxy-2-[(R)-3-dodecanoyloxytetra-decanoylamino]-4-o-phosphono-β-D-glucopyranosyl]-2-[(R)-3-hydroxytetradecanoylamino]-α-D-glucopyranosyldihydrogenphosphate), (WO 95/14026)

OM 294 DP (3S, 9 R) -3--[(R)-dodecanoyloxytetradecanoylamino]-4-oxo-5-aza-9(R)-[(R)-3-hydroxytetradecanoylamino]decan-1,10-diol,1,10-bis(dihydrogenophosphate) (WO99 /64301 and WO 00/0462 )

OM 197 MP-Ac DP ( 3S-, 9R) -3-[(R) -dodecanoyloxytetradecanoylamino]-4-oxo-5-aza-9-[(R)-3-hydroxytetradecanoylamino]decan-1,10-diol,1 -dihydrogenophosphate 10-(6-aminohexanoate) (WO 01/46127)

Other TLR4 ligands which may be used are alkyl Glucosaminide phosphates (AGPs) such as those disclosed in WO9850399 or US6303347 (processes for preparation of AGPs are also disclosed), or pharmaceutically acceptable salts of AGPs as disclosed in US6764840. Some AGPs are TLR4 agonists, and some are TLR4 antagonists. Both are thought to be useful as adjuvants.

Another prefered immunostimulant for use in the present invention is Quil A and its derivatives. Quil A is a saponin preparation isolated from the South American tree Quilaja Saponaria Molina and was first described as having adjuvant activity by Dalsgaard et al. in 1974 ("Saponin adjuvants", Archiv. für die gesamte Virusforschung, Vol. 44, Springer Verlag, Berlin, p243-254). Purified fragments of Quil A have been isolated by HPLC which retain adjuvant activity without the toxicity associated with Quil A (EP 0 362 278), for example QS7 and QS21 (also known as QA7 and QA21). QS-21 is a natural saponin derived from the bark of Quillaja saponaria Molina which induces CD8+ cytotoxic T cells (CTLs), Th1 cells and a predominant IgG2a antibody response and is a preferred saponin in the context of the present invention.

Particular formulations of QS21 have been described which are particularly preferred, these formulations further comprise a sterol (WO96/33739). The saponins forming part of the present invention may be separate in the form of micelles, mixed micelles (preferentially, but not exclusively with bile salts) or may be in the form of ISCOM matrices (EP 0 109 942 B1), liposomes or related colloidal structures such as worm-like or ring-like multimeric complexes or lipidic/layered structures and lamellae when formulated with cholesterol and lipid, or in the form of an oil in water emulsion (for example as in WO 95/17210). The saponins may preferably be associated with a metallic salt, such as aluminium hydroxide or aluminium phosphate (WO 98/15287).

Preferably, the saponin is presented in the form of a liposome, ISCOM or an oil in water emulsion.

An enhanced system involves the combination of a monophosphoryl lipid A (or detoxified lipid A) and a saponin derivative, particularly the combination of QS21 and 3D-MPL as disclosed in WO 94/00153, or a less reactogenic composition where the QS21 is quenched with cholesterol as disclosed in WO 96/33739. A particularly potent adjuvant formulation involving tocopherol with or without QS21 and/or 3D-MPL in an oil in water emulsion is described in WO 95/17210. In one embodiment the immunogenic composition additionally comprises a saponin, which may be QS21.

Immunostimulatory oligonucleotides or any other Toll-like receptor (TLR) 9 agonist may also be used.The preferred oligonucleotides for use in adjuvants or vaccines of the present invention are CpG containing oligonucleotides, preferably containing two or more dinucleotide CpG motifs separated by at least three, more preferably at least six or more nucleotides. A CpG motif is a Cytosine nucleotide followed by a Guanine nucleotide. The CpG oligonucleotides of the present invention are typically deoxynucleotides. In a preferred embodiment the internucleotide in the oligonucleotide is phosphorodithioate, or more preferably a phosphorothioate bond, although phosphodiester and other internucleotide bonds are within the scope of the invention. Also included within the scope of the invention are oligonucleotides with mixed internucleotide linkages. Methods for producing phosphorothioate oligonucleotides or phosphorodithioate are described in US5,666,153, US5,278,302 and WO95/26204.

Examples of preferred oligonucleotides have the following sequences. The sequences preferably contain phosphorothioate modified internucleotide linkages.
OLIGO 1(SEQ ID NO:1): TCC ATG ACG TTC CTG ACG TT (CpG 1826)
OLIGO 2 (SEQ ID NO:2): TCT CCC AGC GTG CGC CAT (CpG 1758)
OLIGO 3(SEQ ID NO:3): ACC GAT GAC GTC GCC GGT GAC GGC ACC ACG
OLIGO 4 (SEQ ID NO:4): TCG TCG TTT TGT CGT TTT GTC GTT (CpG 2006)
OLIGO 5 (SEQ ID NO:5): TCC ATG ACG TTC CTG ATG CT (CpG 1668)
OLIGO 6 (SEQ ID NO:6): TCG ACG TTT TCG GCG CGC GCC G (CpG 5456)

Alternative CpG oligonucleotides may comprise the preferred sequences above in that they have inconsequential deletions or additions thereto.

The CpG oligonucleotides utilised in the present invention may be synthesized by any method known in the art (for example see EP 468520). Conveniently, such oligonucleotides may be synthesized utilising an automated synthesizer.

The adjuvant may be an oil in water emulsion or may comprise an oil in water emulsion in combination with other adjuvants. The oil phase of the emulsion system preferably comprises a metabolisable oil. The meaning of the term metabolisable oil is well known in the art. Metabolisable can be defined as "being capable of being transformed by metabolism" (Dorland's Illustrated Medical Dictionary, W.B. Sanders Company, 25th edition (1974)). The oil may be any vegetable oil, fish, oil, animal or synthetic oil, which is not toxic to the recipient and is capable of being transformed by metabolism. Nuts, seeds, and grains are common sources of vegetable oils. Synthetic oils are also part of this invention and can include commercially available oils such as NEOBEE® and others. Squalene (2,6,10,15,19, 23-Hexamethyl-2,6,10,14,18,22-tetracosahexaene) is an unsaturated oil which is found in large quantities in shark-liver oil, and in lower quantities in olive oil, wheat germ oil, rice bran oil, and yeast, and is a particularly preferred oil for use in this invention. Squalene is a metabolisable oil by virtue of the fact that it is an intermediate in the biosynthesis of cholesterol (Merck index, 10th Edition, entry no.8619).

Tocols (e.g. vitamin E) are also often used in oil emulsions adjuvants (EP 0 382 271 B1; US5667784; WO 95/17210). Tocols used in the oil emulsions (preferably oil in water emulsions) of the invention may be formulated as described in EP 0 382 271 B1, in that the tocols may be dispersions of tocol droplets, optionally comprising an emulsifier, of preferably less than 1 micron in diameter. Alternatively, the tocols may be used in combination with another oil, to form the oil phase of an oil emulsion. Examples of oil emulsions which may be used in combination with the tocol are described herein, such as the metabolisable oils described above.

Oil in water emulsion adjuvants per se have been suggested to be useful as adjuvant compositions (EP 0 399 843B), also combinations of oil in water emulsions and other active agents have been described as adjuvants for vaccines (WO 95/17210; WO 98/56414; WO 99/12565; WO 99/11241). Other oil emulsion adjuvants have been described, such as water in oil emulsions (US 5,422,109; EP 0 480 982 B2) and water in oil in water emulsions (US 5,424,067;EP 0 480 981 B). All of which form preferred oil emulsion systems (in particular when incorporating tocols) to form adjuvants and compositions of the present invention.

Most preferably the oil emulsion (for instance oil in water emulsions) further comprises an emulsifier such as TWEEN 80 and/or a sterol such as cholesterol.

A preferred oil emulsion (preferably oil-in-water emulsion) comprises a metabolisible, nontoxic oil, such as squalane, squalene or a tocopherol such as alpha tocopherol (and preferably both squalene and alpha tocopherol) and optionally an emulsifier (or surfactant) such as Tween 80. A sterol (preferably cholesterol) may also be included.

The method of producing oil in water emulsions is well known to the man skilled in the art. Commonly, the method comprises mixing the tocol-containing oil phase with a surfactant such as a PBS/TWEEN80™ solution, followed by homogenisation using a homogenizer, it would be clear to a man skilled in the art that a method comprising passing the mixture twice through a syringe needle would be suitable for homogenising small volumes of liquid. Equally, the emulsification process in microfluidiser (M110S Microfluidics machine, maximum of 50 passes, for a period of 2 minutes at maximum pressure input of 6 bar (output pressure of about 850 bar)) could be adapted by the man skilled in the art to produce smaller or larger volumes of emulsion. The adaptation could be achieved by routine experimentation comprising the measurement of the resultant emulsion until a preparation was achieved with oil droplets of the required diameter.

In an oil in water emulsion, the oil and emulsifier should be in an aqueous carrier. The aqueous carrier may be, for example, phosphate buffered saline.

The size of the oil droplets found within the stable oil in water emulsion are preferably less than 1 micron, may be in the range of substantially 30-600nm, preferably substantially around 30-500nm in diameter, and most preferably substantially 150-500nm in diameter, and in particular about 150 nm in diameter as measured by photon correlation spectroscopy. In this regard, 80% of the oil droplets by number should be within the preferred ranges, more preferably more than 90% and most preferably more than 95% of the oil droplets by number are within the defined size ranges. The amounts of the components present in the oil emulsions of the present invention are conventionally in the range of from 0.5-20% or 2 to 10% oil (of the total dose volume), such as squalene; and when present, from 2 to 10% alpha tocopherol; and from 0.3 to 3% surfactant, such as polyoxyethylene sorbitan monooleate. Preferably the ratio of oil (preferably squalene): tocol (preferably α-tocopherol) is equal or less than 1 as this provides a more stable emulsion. An emulsifier, such as Tween80 or Span 85 may also be present at a level of about 1%. In some cases it may be advantageous that the vaccines of the present invention will further contain a stabiliser.

Examples of preferred emulsion systems are described in WO 95/17210, WO 99/11241 and WO 99/12565 which disclose emulsion adjuvants based on squalene, α-tocopherol, and TWEEN 80, optionally formulated with the immunostimulants QS21 and/or 3D-MPL. Thus in a particularly, preferred embodiment of the present invention, the adjuvant of the invention may additionally comprise further immunostimulants, such as LPS or derivatives thereof, and/or saponins. Examples of further immunostimulants are described herein and in "Vaccine Design - The Subunit and Adjuvant Approach" 1995, Pharmaceutical Biotechnology, Volume 6, Eds. Powell, M.F., and Newman, M.J., Plenum Press, New York and London, ISBN 0-306-44867-X.

In a preferred aspect the adjuvant and immunogenic compositions according to the invention comprise a saponin (preferably QS21) and/or an LPS derivative (preferably 3D-MPL) in an oil emulsion described above, optionally with a sterol (preferably cholesterol). Additionally the oil emulsion (preferably oil in water emulsion) may contain span 85 and/or lecithin and/or tricaprylin. Adjuvants comprising an oil-in-water emulsion, a sterol and a saponin are described in WO 99/12565.

Typically for human administration the saponin (preferably QS21) and/or LPS derivative (preferably 3D-MPL) will be present in a human dose of immunogenic composition in the range of 1µg - 200µg, such as 10-100µg, preferably 10µg - 50µg per dose. Typically the oil emulsion (preferably oil in water emulsion) will comprise from 2 to 10% metabolisible oil. Preferably it will comprise from 2 to 10% squalene, from 2 to 10% alpha tocopherol and from 0.3 to 3% (preferably 0.4 - 2%) emulsifier (preferably tween 80 [polyoxyethylene sorbitan monooleate]). Where both squalene and alpha tocopherol are present, preferably the ratio of squalene: alpha tocopherol is equal to or less than 1 as this provides a more stable emulsion. Span 85 (Sorbitan trioleate) may also be present at a level of 0.5 to 1% in the emulsions used in the invention. In some cases it may be advantageous that the immunogenic compositions and vaccines of the present invention will further contain a stabiliser, for example other emulsifiers/surfactants, including caprylic acid (merck index 10th Edition, entry no. 1739), of which Tricaprylin is particularly preferred.

Where squalene and a saponin (preferably QS21) are included, it is of benefit to also include a sterol (preferably cholesterol) to the formulation as this allows a reduction in the total level of oil in the emulsion. This leads to a reduced cost of manufacture, improvement of the overall comfort of the vaccination, and also qualitative and quantitative improvements of the resultant immune responses, such as improved IFN-γ production. Accordingly, the adjuvant system of the present invention typically comprises a ratio of metabolisable oil:saponin (w/w) in the range of 200:1 to 300:1, also the present invention can be used in a "low oil" form the preferred range of which is 1:1 to 200:1, preferably 20:1 to 100:1, and most preferably substantially 48:1, this vaccine retains the beneficial adjuvant properties of all of the components, with a much reduced reactogenicity profile. Accordingly, the particularly preferred embodiments have a ratio of squalene:QS21 (w/w) in the range of 1:1 to 250:1, also a preferred range is 20:1 to 200:1, preferably 20:1 to 100:1, and most preferably substantially 48:1. Preferably a sterol (most preferably cholesterol) is also included present at a ratio of saponin:sterol as described herein.

The emulsion systems of the present invention preferably have a small oil droplet size in the sub-micron range. Most preferably the oil droplet sizes will be in the range 120 to 750 nm, and most preferably from 120-600nm in diameter.

A particularly potent adjuvant formulation (for ultimate combination with AlPO4 in the immunogenic compositions of the invention) involves a saponin (preferably QS21), an LPS derivative (preferably 3D-MPL) and an oil emulsion (preferably squalene and alpha tocopherol in an oil in water emulsion) as described in WO 95/17210 or in WO 99/12565 (in particular adjuvant formulation 11 in Example 2, Table 1).

Examples of a TLR 2 agonist include peptidoglycan or lipoprotein. Imidazoquinolines, such as Imiquimod and Resiquimod are known TLR7 agonists. Single stranded RNA is also a known TLR agonist (TLR8 in humans and TLR7 in mice), whereas double stranded RNA and poly IC (polyinosinic-polycytidylic acid - a commercial synthetic mimetic of viral RNA). are exemplary of TLR 3 agonists. 3D-MPL is an example of a TLR4 agonist whilst CPG is an example of a TLR9 agonist.

The immunogenic composition may comprise an antigen and an immunostimulant adsorbed onto a metal salt. Aluminium based vaccine formulations wherein the antigen and the immunostimulant 3-de-O-acylated monophosphoryl lipid A (3D-MPL), are adsorbed onto the same particle are described in EP 0 576 478 B1, EP 0 689 454 B1, and EP 0 633 784 B1. In these cases then antigen is first adsorbed onto the aluminium salt followed by the adsorption of the immunostimulant 3D-MPL onto the same aluminium salt particles. Such processes first involve the suspension of 3D-MPL by sonication in a water bath until the particles reach a size of between 80 and 500 nm. The antigen is typically adsorbed onto aluminium salt for one hour at room temperature under agitation. The 3D-MPL suspension is then added to the adsorbed antigen and the formulation is incubated at room temperature for 1 hour, and then kept at 4oC until use.

In another process, the immunostimulant and the antigen are on separate metal particles, as described in EP 1126876. The improved process comprises the adsorption of immunostimulant, onto a metallic salt particle, followed by the adsorption of the antigen onto another metallic salt particle, followed by the mixing of the discrete metallic particles to form a vaccine. The adjuvant for use in the present invention may be an adjuvant composition comprising an immunostimulant, adsorbed onto a metallic salt particle, characterised in that the metallic salt particle is substantially free of other antigen. Furthermore, vaccines are provided by the present invention and are characterised in that the immunostimulant is adsorbed onto particles of metallic salt which are substantially free from other antigen, and in that the particles of metallic salt which are adsorbed to the antigen are substantially free of other immunostimulant.

Accordingly, the present invention provides an adjuvant formulation comprising immunostimulant which has been adsorbed onto a particle of a metallic salt, characterised in the composition is substantially free of other antigen. Moreover, this adjuvant formulation can be an intermediate which, if such an adjuvant is used, is required for the manufacture of a vaccine. Accordingly there is provided a process for the manufacture of a vaccine comprising admixing an adjuvant composition which is one or more immunostimulants adsorbed onto a metal particle with an antigen. Preferably, the antigen has been pre-adsorbed onto a metallic salt. Said metallic salt may be identical or similar to the metallic salt which is adsorbed onto the immunostimulant. Preferably the metal salt is an aluminium salt, for example Aluminium phosphate or Aluminium hydroxide.

The present invention further provides for a vaccine composition comprising immunostimulant adsorbed onto a first particle of a metallic salt, and antigen adsorbed onto a metallic salt, characterised in that first and second particles of metallic salt are separate particles.

LPS or LOS derivatives or mutations or lipid A derivatives described herein are designed to be less toxic (e.g. 3D-MPL) than native lipopolysaccharides and are interchangeable equivalents with respect to any uses of these moieties described herein.

In one embodiment the adjuvant used for the compositions of the invention comprises a liposome carrier (made by known techniques from a phospholipids (such as dioleoyl phosphatidyl choline [DOPC]) and optionally a sterol [such as cholesterol]). Such liposome carriers may carry lipid A derivatives [such as 3D-MPL - see above] and/or saponins (such as QS21 - see above). In one embodiment the adjuvant comprises (per 0.5 mL dose) 0.1-10mg, 0.2-7, 0.3-5, 0.4-2, or 0.5-1 mg (e.g. 0.4-0.6, 0.9-1.1, 0.5 or 1 mg) phospholipid (for instance DOPC), 0.025-2.5, 0.05-1.5, 0.075-0.75, 0.1-0.3, or 0.125-0.25 mg (e.g. 0.2-0.3, 0.1-0.15, 0.25 or 0.125 mg) sterol (for instance cholesterol), 5-60, 10-50, or 20-30 µg (e.g. 5-15, 40-50, 10, 20, 30, 40 or 50 µg) lipid A derivative (for instance 3D-MPL), and 5-60, 10-50, or 20-30 µg (e.g. 5-15, 40-50, 10, 20, 30, 40 or 50 µg) saponin (for instance QS21).

In one embodiment the adjuvant used for the compositions of the invention comprises an oil in water emulsion made from a metabolisable oil (such as squalene), an emulsifier (such as Tween 80) and optionally a tocol (such as alpha tocopherol). In one embodiment the adjuvant comprises (per 0.5 mL dose) 0.5-15, 1-13, 2-11, 4-8, or 5-6mg (e.g. 2-3, 5-6, or 10-11 mg) metabolisable oil (such as squalene), 0.1-10, 0.3-8, 0.6-6, 0.9-5, 1-4, or 2-3 mg (e.g. 0.9-1.1, 2-3 or 4-5 mg) emulsifier (such as Tween 80) and optionally 0.5-20, 1-15, 2-12, 4-10, 5-7 mg (e.g. 11-13, 5-6, or 2-3 mg) tocol (such as alpha tocopherol).

This adjuvant may optionally further comprise 5-60, 10-50, or 20-30 µg (e.g. 5-15, 40-50, 10, 20, 30, 40 or 50 µg) lipid A derivative (for instance 3D-MPL).

This adjuvant may optionally contain 0.025-2.5, 0.05-1.5, 0.075-0.75, 0.1-0.3, or 0.125-0.25 mg (e.g. 0.2-0.3, 0.1-0.15, 0.25 or 0.125 mg) sterol (for instance cholesterol), 5-60, 10-50, or 20-30 µg (e.g. 5-15, 40-50, 10, 20, 30, 40 or 50 µg) lipid A derivative (for instance 3D-MPL), and 5-60, 10-50, or 20-30 µg (e.g. 5-15, 40-50, 10, 20, 30, 40 or 50 µg) saponin (for instance QS21).

In one embodiment the adjuvant used for the compositions of the invention comprises aluminium phosphate and a lipid A derivative (such as 3D-MPL). This adjuvant may comprise (per 0.5 mL dose) 100-750, 200-500, or 300-400 µg Al as aluminium phosphate, and 5-60, 10-50, or 20-30 µg (e.g. 5-15, 40-50, 10, 20, 30, 40 or 50 µg) lipid A derivative (for instance 3D-MPL).

The vaccine preparations of the present invention may be used to protect or treat a mammal susceptible to infection, by means of administering said vaccine via systemic or mucosal route. These administrations may include injection *via* the intramuscular, intraperitoneal, intradermal or subcutaneous routes; or *via* mucosal administration to the oral/alimentary, respiratory, genitourinary tracts. Intranasal administration of vaccines for the treatment of pneumonia or otitis media is preferred (as nasopharyngeal carriage of pneumococci can be more effectively prevented, thus attenuating infection at its earliest stage). Although the vaccine of the invention may be administered as a single dose, components thereof may also be co-administered together at the same time or at different times (for instance pneumococcal polysaccharides could be administered separately, at the same time or 1-2 weeks after the administration of any bacterial protein component of the vaccine for optimal coordination of the immune responses with respect to each other). For co-administration, the optional Th1 adjuvant may be present in any or all of the different administrations, for example, it may be present in combination with the bacterial protein component of the vaccine. In addition to a single route of administration, 2 different routes of administration may be used. For example, polysaccharides may be administered IM (or ID) and bacterial proteins may be administered IN (or ID). In addition, the vaccines of the invention may be administered IM for priming doses and IN for booster doses.

The amount of conjugate antigen in each vaccine dose is selected as an amount which induces an immunoprotective response without significant, adverse side effects in typical vaccines. Such amount will vary depending upon which specific immunogen is employed and how it is presented. Generally, it is expected that each dose will comprise 0.1-100 µg of polysaccharide, typically 0.1-50 µg , 0.1-10µg, 1-10µg or 1-5µg for polysaccharide conjugates.

The content of protein antigens in the vaccine will typically be in the range 1-100µg, 5-50µg or 5 - 25µg. Following an initial vaccination, subjects may receive one or several booster immunizations adequately spaced.

Vaccine preparation is generally described in Vaccine Design ("The subunit and adjuvant approach" (eds Powell M.F. & Newman M.J.) (1995) Plenum Press New York). Encapsulation within liposomes is described by Fullerton, US Patent 4,235,877.

The vaccines of the present invention may be stored in solution or lyophilized. Optionally the solution is lyophilized in the presence of a sugar such as sucrose, trehalose or lactose. It is typical that they are lyophilized and extemporaneously reconstituted prior to use. Lyophilizing may result in a more stable composition (vaccine).

### Methods

The invention also encompasses method of making the immunogenic compositions and vaccines of the invention.

In an embodiment, the process of the invention, is a method to make a vaccine comprising the steps of mixing antigens to make the immunogenic composition of the invention and adding a pharmaceutically acceptable excipient.

### Methods of treatment

The invention also encompasses method of treatment or staphylococcal infection, particularly hospital acquired nosocomial infections.

This immunogenic composition or vaccine of the invention is particularly advantageous to use in cases of elective surgery. Such patients will know the date of surgery in advance and could be inoculated in advance. Since it is not know whether the patient will be exposed to *S*. *aureus* or *S*. *epidermidis* infection, it is preferred to inoculate with a vaccine of the invention that protects against both, as described above. Typically adults over 16 awaiting elective surgery are treated with the immunogenic compositions and vaccines of the invention. Alternatively children aged 3-16 awaiting elective surgery are treated with the immunogenic compositions and vaccines of the invention.

It is also possible to inoculate health care workers with the vaccine of the invention.

The vaccine preparations of the present invention may be used to protect or treat a mammal susceptible to infection, by means of administering said vaccine via systemic or mucosal route. These administrations may include injection *via* the intramuscular, intraperitoneal, intradermal or subcutaneous routes; or via mucosal administration to the oral/alimentary, respiratory, genitourinary tracts.

The amount of antigen in each vaccine dose is selected as an amount which induces an immunoprotective response without significant, adverse side effects in typical vaccines. Such amount will vary depending upon which specific immunogen is employed and how it is presented. The protein content of the vaccine will typically be in the range 1 -100µg, 5-50µg, typically in the range 10 - 25µg. An optimal amount for a particular vaccine can be ascertained by standard studies involving observation of appropriate immune responses in subjects. Following an initial vaccination, subjects may receive one or several booster immunisations adequately spaced.

Although the vaccines of the present invention may be administered by any route, administration of the described vaccines into the skin (ID) forms one embodiment of the present invention. Human skin comprises an outer "horny" cuticle, called the stratum corneum, which overlays the epidermis. Underneath this epidermis is a layer called the dermis, which in turn overlays the subcutaneous tissue. Researchers have shown that injection of a vaccine into the skin, and in particular the dermis, stimulates an immune response, which may also be associated with a number of additional advantages. Intradermal vaccination with the vaccines described herein forms an optional feature of the present invention.

The conventional technique of intradermal injection, the "mantoux procedure", comprises steps of cleaning the skin, and then stretching with one hand, and with the bevel of a narrow gauge needle (26-31 gauge) facing upwards the needle is inserted at an angle of between 10-15°. Once the bevel of the needle is inserted, the barrel of the needle is lowered and further advanced whilst providing a slight pressure to elevate it under the skin. The liquid is then injected very slowly thereby forming a bleb or bump on the skin surface, followed by slow withdrawal of the needle.

More recently, devices that are specifically designed to administer liquid agents into or across the skin have been described, for example the devices described in WO 99/34850 and EP 1092444, also the jet injection devices described for example in WO 01/13977; US 5,480,381, US 5,599,302, US 5,334,144, US 5,993,412, US 5,649,912, US 5,569,189, US 5,704,911, US 5,383,851, US 5,893,397, US 5,466,220, US 5,339,163, US 5,312,335, US 5,503,627, US 5,064,413, US 5,520, 639, US 4,596,556, US 4,790,824, US 4,941,880, US 4,940,460, WO 97/37705 and WO 97/13537. Alternative methods of intradermal administration of the vaccine preparations may include conventional syringes and needles, or devices designed for ballistic delivery of solid vaccines (WO 99/27961), or transdermal patches (WO 97/48440; WO 98/28037); or applied to the surface of the skin (transdermal or transcutaneous delivery WO 98/20734 ; WO 98/28037).

When the vaccines of the present invention are to be administered to the skin, or more specifically into the dermis, the vaccine is in a low liquid volume, particularly a volume of between about 0.05 ml and 0.2 ml.

The content of antigens in the skin or intradermal vaccines of the present invention may be similar to conventional doses as found in intramuscular vaccines (see above). However, it is a feature of skin or intradermal vaccines that the formulations may be "low dose". Accordingly the protein antigens in "low dose" vaccines are optionally present in as little as 0.1 to 10µg, optionally 0.1 to 5 µg per dose; and the polysaccharide (optionally conjugated) antigens may be present in the range of 0.01-1µg, and optionally between 0.01 to 0.5 µg of polysaccharide per dose.

As used herein, the term "intradermal delivery" means delivery of the vaccine to the region of the dermis in the skin. However, the vaccine will not necessarily be located exclusively in the dermis. The dermis is the layer in the skin located between about 1.0 and about 2.0 mm from the surface in human skin, but there is a certain amount of variation between individuals and in different parts of the body. In general, it can be expected to reach the dermis by going 1.5 mm below the surface of the skin. The dermis is located between the stratum corneum and the epidermis at the surface and the subcutaneous layer below. Depending on the mode of delivery, the vaccine may ultimately be located solely or primarily within the dermis, or it may ultimately be distributed within the epidermis and the dermis.

An embodiment of the invention is a method of preventing or treating staphylococcal infection or disease comprising the step of administering the immunogenic composition or vaccine of the invention to a patient in need thereof.

A further embodiment of the invention is a use of the immunogenic composition of the invention in the manufacture of a vaccine for treatment or prevention of staphylococcal infection or disease, optionally post-surgery staphylococcal infection.

The term 'staphylococcal infection' encompasses infection caused by *S*. *aureus* and/or *S*. *epidermidis* and other staphylococcal strains capable of causing infection in a mammalina, optionally human host.

The terms "comprising", "comprise" and "comprises" herein are intended by the inventors to be optionally substitutable with the terms "consisting of", "consist of" and "consists of", respectively, in every instance.

All references or patent applications cited within this patent specification are incorporated by reference herein.

In order that this invention may be better understood, the following examples are set forth. These examples are for purposes of illustration only, and are not to be construed as limiting the scope of the invention in any manner.

### Examples

### Example 1 Construction of Plasmid to Express Recombinant proteins

### A: Cloning.

Appropriate restriction sites engineered into oligonucleotides specific for the staphylococcal gene permitted directional cloning of the PCR product into the *E.coli* expression plasmid pET24d or pQE-30 such that a protein could be expressed as a fusion protein containing a (His)6 affinity chromatography tag at the N- or C-terminus.

The primers used were:

Alpha toxin – 5'-CGCGGATCCGCAGATTCTGATATTAATATTAAAAC-3' and
5'CCCAAGCTTTTAATTTGTCATTTCTTCTTTTTC-3'
EbpS – 5'-CGCGGATCCGCTGGGTCTAATAATTTTAAAGATG-3' and
5'CCCAAGCTTTTATGGAATAACGATTTGTTG-3'
ClfA – 5'-CGCGGATCCAGTGAAAATAGTGTTACGCAATC-3' and
5'CCCAAGCTTTTACTCTGGAATTGGTTCAATTTC-3'
FnbpA – 5'-CGCGGATCCACACAAACAACTGCAACTAACG-3' and
5'CCCAAGCTTTTATGCTTTGTGATTCTTTTTCAAAC3'
Sbi – 5'-CGCGGATCCAACACGCAACAAACTTC-3' and
5'GGAACTGCAGTTATTTCCAGAATGATAATAAATTAC-3'
SdrC – 5'-CGCGGATCCGCAGAACATACGAATGGAG-3' and
5'CCCAAGCTTTTATGTTTCTTCTTCGTAGTAGC-3'
SdrG – 5'-CGCGGATCCGAGGAGAATTCAGTACAAG-3' and
5'CCCAAGCTTTTATTCGTCATCATAGTATCCG-3'
Ebh – 5'-AAAAGTACTCACCACCACCACCACC-3' and
5'AAAAGTACTCACTTGATTCATCGCTTCAG-3'
Aaa – 5'-GCGCGCCATGGCACAAGCTTCTACACAACATAC-3' and
5'GCGCGCTCGAGATGGATGAATGCATAGCTAGA-3'
IsaA – 5'-GCATCCATGGCACCATCACCATCACCACGAAGTAAACGTTGATCAAGC-3'
and 5'-AGCACTCGAGTTAGAATCCCCAAGCACCTAAACC-3'
HarA – 5'-GCACCCATGGCAGAAAATACAAATACTTC-3' and
5'TTTTCTCGAGCATTTTAGATTGACTAAGTTG-3'
Autolysin glucosaminidase – 5'-CAAGTCCCATGGCTGAGACGACACAAGATCAAC-3'
and 5'-CAGTCTCGAGTTTTACAGCTGTTTTTGGTTG-3'

Autolysin amidase – 5'-AGCTCATATGGCTTATACTGTTACTAAACC-3' and
5'GCGCCTCGAGTTTATATTGTGGGATGTCG-3'
IsdA – 5'-CAAGTCCCATGGCAACAGAAGCTACGAACGCAAC-3' and
5'ACCAGTCTCGAGTAATTCTTTAGCTTTAGAGCTTG-3'
IsdB – 5'-TATTCTCGAGGCTTTGAGTGTGTCCATCATTTG-3' and 5'
GAAGCCATGGCAGCAGCTGAAGAAACAGGTGG-3'
MRPII – 5'-GATTACACCATGGTTAAACCTCAAGCGAAA-3' and
5'AGGTGTCTCGAGTGCGATTGTAGCTTCATT-3'

The PCR products were first introduced into the pGEM-T cloning vector (Novagen) using Top10 bacterial cells, according to the manufacturer's instructions. This intermediate construct was made to facilitate further cloning into an expression vector. Transformants containing the DNA insert were selected by restriction enzyme analysis. Following digestion, a ∼20µl aliquot of the reaction was analyzed by agarose gel electrophoresis (0.8 % agarose in a Tris-acetate-EDTA (TAE) buffer). DNA fragments were visualized by UV illumination after gel electrophoresis and ethidium bromide staining. A DNA molecular size standard (1 Kb ladder, Life Technologies) was electrophoresed in parallel with the test samples and was used to estimate the size of the DNA fragments. Plasmid purified from selected transformants for each cloning was then sequentially digested to completion with appropriate restriction enzymes as recommended by the manufacturer (Life Technologies). The digested DNA fragment was then purified using silica gel-based spin columns prior to ligation with the pET24d or pQE-30 plasmid. Cloning of Ebh (H2 fragment), AaA, IsdA, IsdB, HarA, Atl-amidase, Atl-glucosamine, MRPII, IsaA was carried out using the pET24d plasmid and cloning of ClfA, SdrC, SdrE, FnbpA, SdrG/Fbe, alpha toxin and Sbi were carried out using the pQE-30 plasmid.

### B: Production of expression vector.

To prepare the expression plasmid pET24d or pQE-30 for ligation, it was similarly digested to completion with appropriate restriction enzymes. An approximately 5-fold molar excess of the digested fragments to the prepared vector was used to program the ligation reaction. A standard ∼20 µl ligation reaction (∼16°C, ∼16 hours), using methods well known in the art, was performed using T4 DNA ligase (∼2.0 units / reaction, Life Technologies). An aliquot of the ligation (∼5 µl) was used to transform M15(pREP4) or BT21::DE3 electro-competent cells according to methods well known in the art. Following a µ2-3 hour outgrowth period at 37°C in ∼1.0 ml of LB broth, transformed cells were plated on LB agar plates containing ampicillin (100 µg/ml) and/or kanamycin (30µg/ml). Antibiotics were included in the selection. Plates were incubated overnight at 37°C for ∼16 hours. Individual ApR/KanR colonies were picked with sterile toothpicks and used to "patch" inoculate fresh LB ApR/KanR plates as well as a ∼1.0 ml LB Ap/ Kan broth culture. Both the patch plates and the broth culture were incubated overnight at 37°C in either a standard incubator (plates) or a shaking water bath. A whole cell-based PCR analysis was employed to verify that transformants contained the DNA insert. Here, the ∼1.0 ml overnight LB Ap/Kan broth culture was transferred to a 1.5 ml polypropylene tube and the cells collected by centrifugation in a Beckmann microcentrifuge (∼3 min., room temperature, ∼12,000 X g). The cell pellet was suspended in ∼200µl of sterile water and a ∼10µl aliquot used to program a ∼50µl final volume PCR reaction containing both forward and reverse amplification primers. The initial 95°C denaturation step was increased to 3 minutes to ensure thermal disruption of the bacterial cells and liberation of plasmid DNA. An ABI Model 9700 thermal cycler and a 32 cycle, three-step thermal amplification profile, i.e. 95°C, 45sec; 55-58°C, 45sec, 72°C, 1min., were used to amplify the BASB203 fragment from the lysed transformant samples. Following thermal amplification, a ∼20µl aliquot of the reaction was analyzed by agarose gel electrophoresis (0.8 % agarose in a Tris-acetate-EDTA (TAE) buffer). DNA fragments were visualised by UV illumination after gel electrophoresis and ethidium bromide staining. A DNA molecular size standard (1 Kb ladder, Life Technologies) was electrophoresed in parallel with the test samples and was used to estimate the size of the PCR products. Transformants that produced the expected size PCR product were identified as strains containing a protein expression construct. Expression plasmid containing strains were then analyzed for the inducible expression of recombinant protein.

### C: Expression Analysis of PCR-Positive Transformants.

An aliquot of the overnight seed culture (∼1.0 ml) was inoculated into a 125 ml erlenmeyer flask containing ∼25 ml of LB Ap/Kan broth and was grown at 37 °C with shaking (∼250 rpm) until the culture turbidity reached O.D.600 of ∼0.5, i.e. mid-log phase (usually about 1.5 - 2.0 hours). At this time approximately half of the culture (∼12.5 ml) was transferred to a second 125 ml flask and expression of recombinant protein induced by the addition of IPTG (1.0 M stock prepared in sterile water, Sigma) to a final concentration of 1.0 mM. Incubation of both the IPTG-induced and non-induced cultures continued for an additional ∼4 hours at 37 °C with shaking. Samples (∼1.0 ml) of both induced and non-induced cultures were removed after the induction period and the cells collected by centrifugation in a microcentrifuge at room temperature for ∼3 minutes. Individual cell pellets were suspended in ∼50µl of sterile water, then mixed with an equal volume of 2X Laemelli SDS-PAGE sample buffer containing 2-mercaptoethanol, and placed in boiling water bath for ∼3 min to denature protein. Equal volumes (∼15µl) of both the crude IPTG-induced and the non-induced cell lysates were loaded onto duplicate 12% Tris/glycine polyacrylamide gel (1 mm thick Mini-gels, Novex). The induced and non-induced lysate samples were electrophoresed together with prestained molecular weight markers (SeeBlue, Novex) under conventional conditions using a standard SDS/Tris/glycine running buffer (BioRad). Following electrophoresis, one gel was stained with commassie brilliant blue R250 (BioRad) and then destained to visualize novel IPTG-inducible protein(s) .The second gel was electroblotted onto a PVDF membrane (0.45 micron pore size, Novex) for ∼2 hrs at 4 °C using a BioRad Mini-Protean II blotting apparatus and Towbin's methanol (20 %) transfer buffer. Blocking of the membrane and antibody incubations were performed according to methods well known in the art. A monoclonal anti-RGS (His)3 antibody, followed by a second rabbit anti-mouse antibody conjugated to HRP (QiaGen), were used to confirm the expression and identity of the recombinant protein. Visualization of the anti-His antibody reactive pattern was achieved using either an ABT insoluble substrate or using Hyperfilm with the Amersham ECL chemiluminescence system.

### Example 2: Production of Recombinant Protein

### Bacterial strain

A recombinant expression strain of *E. coli* M15(pREP4) containing a plasmid (pQE30) or BL21::DE3 containing plasmid pET24d encoding staphylococcal protein was used to produce cell mass for purification of recombinant protein.

### Media

The fermentation medium used for the production of recombinant protein consisted of 2X YT broth (Difco) containing 100µg/ml Ap and/or 30 µg/ml Km. Antifoam was added to medium for the fermentor at 0.25 ml/L (Antifoam 204, Sigma). To induce expression of the recombinant protein, IPTG (Isopropyl ß-D-Thiogalactopyranoside) was added to the fermentor (1 mM, final).

### Production of recombinant proteins

### Under native conditions

IPTG was added at a final concentration of 1mM and the culture was grown for 4 additional hours. The culture was then centrifuged at 6,000 rpm for 10 minutes and the pellet was resuspended in phosphate buffer (50mM K2HPO4, KH2PO4 pH 7) including a protease inhibior cocktail. This sample was subjected to French pressure lysis using 1500 bar pressure ( 2 runs). After centrifugation for 30 minutes at 15,000 rpm, the supernatant was reserved for further purification and NaCl was added to 0.5M. The sample was then loaded on a Ni-NTA resin (XK 16 column Pharmacia, Ni-NTA resin Qiagen) conditioned in 50mM K2HPO4, KH2PO4 pH 7. After loading the sample, the column was washed with Buffer A (0.2M NaH2PO4 pH7, 0.3M NaCl, 10% glycerol). To elute bound protein, a step gradient ws used where different proportions of buffer B (0.2M NaH2PO4 pH7, 0.3M NaCl, 10% glycerol and 200mM imidazole) were added to buffer A. The proportion of buffer B was gradually increased from 10% to 100%. After purification, eluted fraction containing the protein were pooled, concentrated and dialysed against 0.002M KH2PO4/K2HPO4 pH7, 0.15M NaCl.

This method was used to purify ClfA, SdrG, IsdA, IsaB, HarA, Atl-glucosamine and alpha toxin.

### Under denaturing conditions

IPTG was added at a final concentration of 1mM and the culture was grown for 4 additional hours. The culture was then centrifuged at 6,000 rpm for 10 mintes and the pellet was resuspended in phosphate buffer (50mM K2HPO4, KH2PO4 pH 7) including a protease inhibior cocktail. This sample was subjected to French pressure lysis using 1500 bar pressure ( 2 runs). After centrifugation for 30 minutes at 15,000 rpm, the pellet was washed with phosphate buffer including 1M urea. The sample was centrifuged for 30 mins at 15000rpm and the pellet was resuspended in 8M urea, 0.1 M NaH2PO4, 0.5M NaCl, 0.01 M Tris-Hcl pH8 and kept overnight at room temperature. The sample was centrifuged fro 20 minutes at 15000rpm and the supernatant was collected for further purification. The sample was then loaded on a Ni-NTA resin (XK 16 column Pharmacia, Ni-NTA resin Qiagen) conditioned in 8M urea, 0.1M NaH2PO4, 0.5M NaCl, 0.01 M Tris-Hcl pH8. After passsage of the flowthrough, the column was washed succesively with buffer A (8M Urea, 0.1MNaH2PO4, 0,5M NaCl, 0.01M Tris, pH 8.0), buffer C (8M Urea, 0.1MNaH2PO4, 0.5M NaCl, 0.01 M Tris, pH 6.3), buffer D (8M Urea, 0.1MNaH2PO4, 0.5M NaCl, 0.01 M Tris, pH 5.9) and buffer E (8M Urea, 0.1MNaH2PO4, 0.5M NaCl, 0.01M Tris, pH 4.5). The recombinant protein was eluted from the column during washes with buffer D and E. The denatured, recombinant protein could be solubilized in a solution devoid of urea. For this purpose, denatured protein contained in 8M urea was successively dialyzed against 4M urea, 0.1MNa2PO4, 0.01M Tris-HCl, pH7.1, 2M urea, 0.1 M NaH2PO4, 0.01M Tris-HCl, pH 7.1, 0.5M arginine and 0.002M KH2PO4/K2HPO4 pH7.1, 0.15M NaCl, 0.5M arginine. This method was used to purify Ebh (H2 fragment), AaA, SdrC, FnbpA, Sbi, Atl-amidase and IsaA.

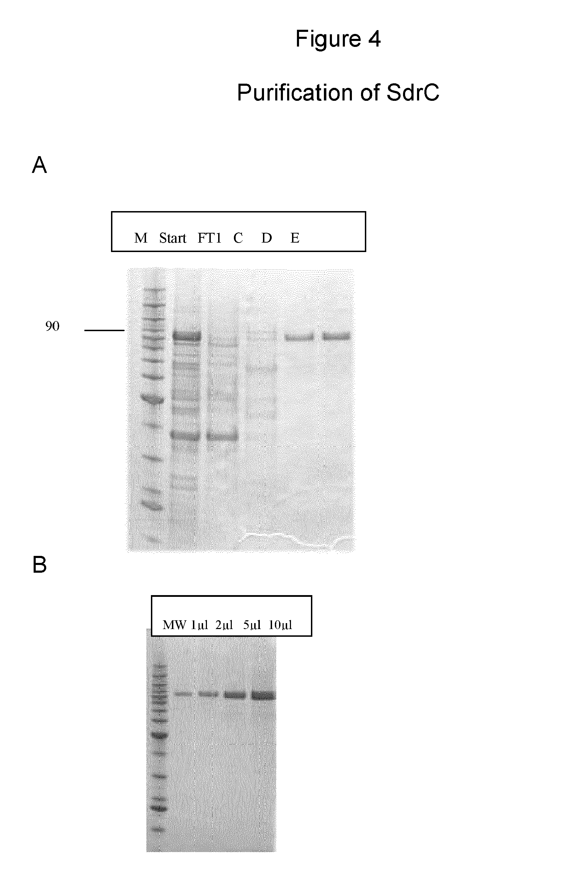

The purified proteins were analysed by SDS-PAGE. The results for one protein purified under native conditions (alpha toxin) and one protein purified under denaturing conditions (SdrC) are shown in Figures 3 and 4.

### Example 3 Preparation of S. aureus Capsular Polysaccharide Conjugates using CDAP

### Activation and coupling chemistry for native PS8 using CDAP:

### SA08-TT004

Activation and coupling were performed at room temperature under continuous stirring. 10 mg of native polysaccharide were dissolved to obtain a final PS concentration of 2.5 mg/ml in 0.2M NaCl. The solution was then adjusted to pH 6.0+/-0.2 before the activation step.

At time 0, 50 µl of a CDAP solution (100 mg/ml freshly prepared in acetonitrile/WFI, 50/50) were added manually to reach the appropriate CDAP/PS (0.5/1) ratio.

After 1.5 minutes the pH was raised to pH 9.00+/-0.05 by addition of 0.5M NaOH.

NaOH addition takes about 1 minutes and pH is stabilised at pH 9.00+/-0.05 up to carrier addition.

At time 4.5 minutes, 1.5 ml of TT (10 mg/ml in 0.2M NaCl) was added to reach the appropriate Protein/PS ratio (1.5/1); pH was immediately adjusted to coupling pH 9.00+/- 0.05. The solution is left for one hour under manual pH regulation.

After the coupling step, 0.5 ml of 2M glycine (ratio gly/PS (w/w): 7.5/1) were added; pH was immediately adjusted to 9.00+/-0.05. The solution was left for 30 minutes under manual pH regulation. Then the conjugate was clarified using a 5 µm Minisart filter and injected on Sephacryl S400HR (XK16/100). The flow-rate was fixed at 30 ml/h, using 150mM NaCl.

The elution fractions were analysed by resorcinol and by µBCA. Interesting fractions were pooled and filtered on 0.22 µm Sterivex.

The resulting conjugate had a final TT/PS ratio (w/w) of 1.05 as assessed by resorcinol and Lowry assays.

### Example 4 Preparation of S. aureus Capsular Polysaccharide Conjugates using CDAP on sized polysaccharides

### Activation and coupling chemistry for sized PS8 using CDAP

PS is weighted on the basis of 10% theoretical moisture content. 2 g of native, humid PS was dissolved overnight in WFI at an initial concentration of 10 mg/ml. Before the sizing, the solution of native PS was clarified on 5 µm cut-off filter.

A EMULSIFLEX C-50 homogenizer apparatus, in which the homogenizing cell was replaced with a Microfluidics F20Y-0.75µm interaction chamber, was used to reduce the molecular weight and the viscosity of the polysaccharide before the activation step

The size reduction was realized at 10000 psi during the 10 first cycles and then at 15000 psi for the following 60 cycles. The progress of the size reduction was followed *in-process* by measuring viscosity. The sizing was stopped after 70 cycles when the target of 2.74 ± 0.2 cp was reached.

Activation and coupling were performed at room temperature under continuous stirring. 50 mg of sized polysaccharide 8 were diluted to obtain a final PS concentration of 5 mg/ml in 0.2M NaCl.

At time 0, 375 µl of a CDAP solution (100 mg/ml freshly prepared in acetonitrile/WFI, 50/50) were added manually to reach the appropriate CDAP/PS (0.75/1) ratio. After 1 minute the pH was raised to pH 9.00+/-0.05 by addition of 0.5M NaOH.

At time 2.5 minutes, 10 ml of TT at 10 mg/ml in 0.2M NaCl were added to reach the appropriate Protein/PS ratio (2/1); pH was immediately adjusted to coupling pH 9.00+/- 0.05. The solution was left for 55 minutes under manual pH regulation.

After the coupling step, 2.5 ml of 2M glycine (ratio gly/PS (w/w): 7.5/1) were added; pH was immediately adjusted to 9.00+/-0.05 by the regulator. The solution was left for 30 minutes under manual pH regulation.

Then the conjugate was clarified using a 5µm Minisart filter and injected on Sephacryl S400HR (XK26/100). The flow-rate was fixed at 60 ml/h.

The elution fractions were analysed by resorcinol and by protein dosage. Interesting fractions were pooled and filtered on 0.22 µm Millipack20.

The resulting conjugate has a final TT/PS ratio of 1.94.

### Example 5 Preparation of S. aureus Capsular Polysaccharide Conjugates using EDAC

### Activation and coupling chemistry using EDAC:

### S. aureus capsular polysaccharide type 8-TT conjugate:

### PS derivatization

Activation and coupling were performed at room temperature under continuous stirring. 30 mg of native polysaccharide were diluted to obtain a final polysaccharide concentration of 5 mg/ml in water. The solution was adjusted to pH 4.5-5.0 with 0.5N HCl and then 66 µg of ADH were added (2.2 mg/mg PS). After complete dissolution, 60 mg of EDAC were added (2 mg/mg PS). After 70 min the pH was raised to pH 7.5 with 1 N NaOH to stop the reaction. Free ADH was removed by purification on Sephacryl S100HR (XK 16/40). The flow-rate was fixed at 60 ml/h using 0.2 M NaCl as elution buffer. A size reduction was done by sonication of 15 min allowing a sterile filtration on millex filter (0.22 µm).

### Coupling

Tetanus toxoid was added to 5 to 10 mg of derivatized polysaccharide in 0.2M NaCl and the pH was adjusted to pH 5.0 or pH 6.0 by addition of 0.5N HCl. EDAC was dissolved in 0.1M Tris buffer pH 7.5 and then added over a period of 10 min (1/5 vol each 2 min). According to the conditions used (see Table 6), the reaction was stopped after between 30 and 180 minutes by addition of 1M Tris-HCl pH 7.5. Prior to purification on Sephacryl S400HR, the conjugate was clarified using a 5µm Minisart filter. Alternatively, the conjugate was clarified by a 5 minute sonication step. The conjugate was then injected on Sephacryl S400HR (XK16/100). The flow-rate was fixed at 30 ml/h using 150 mM NaCl as elution buffer. The elution pool was selected on the basis of resorcinol and µBCA profiles (which measure polysaccharide and protein dosage respectively). The conjugate was filtered on a 0.22 µm sterilizing membrane (Millipack 20) at 10 ml/min.

**Table 5**

| **Conjugate** | **Coupling time** | **[PS (AH)] (mg/ml)** | **[TT (AH)] (mg/ml)** | **[reagent EDAC] (mg/mg PS)** |
|---|---|---|---|---|
| **SA08-TT011** | 40 min | 3.58 | 6.45 | 0.5/1 |
| **SA08-TT015*** | 180 min | 2 | 4.0 | 0.25/1 |
| **SA08-TT017** | 30 min | 3.75 | 7.5 | 0.25/1 |
| **SA08-TT018** | 50 min | 3.75 | 7.5 | 0.10/1 |

| | | | | |
|---|---|---|---|---|
| Table 5: * coupling done at pH 6.0 | | | | |

The resulting conjugates have the following characteristics shown in Table 6:

**Table 6**

| **Conjugate** | **In. TT/PS ratio(w/w)** | **F. TT/PS ratio (w/w)** | **Y. PS rec (%)** | **Filtr. Yield (%)** |
|---|---|---|---|---|
| **SA08-TT011** | 2/1 | 2.43/1 | 48 | 99 |
| **SA08-TT015** | 2/1 | 2.40/1 | 53 | 104 |
| **SA08-TT017** | 2/1 | 2.41/1 | 44 | 107 |
| **SA08-TT018** | 2/1 | 2.40/1 | 42 | 106 |

*S. aureus* polysaccharide type 8 was also treated by microfluidization before derivatization with ADH

### PS derivatization

Activation and coupling are performed at room temperature under continuous stirring. 200 mg of sized polysaccharide are diluted to obtain a final PS concentration of 10 mg/ml in water. Then 440 mg of ADH were added (2.2 mg/mg PS). The solution was adjusted to pH 4.7 with 1 N HCl before the addition of 400 mg of EDAC (2 mg/mg PS). After 60 min the pH was raised to pH 7.5 with 5M NaOH to stop the reaction. The mixture was concentrated on Amicon Ultra (cut-off 10.000 MWCO). Prior to purification on Sephacryl S200HR (XK16/100), the conjugate was clarified using a 5µm Minisart filter. The flow-rate was fixed at 30 ml/h using 0.150 M NaCl as elution buffer.

### Coupling

100 mg of TT was added to 50 mg of derivatized polysaccharide in 0.2M NaCl. The pH was adjusted to pH 5.0 ± 0.02 by addition of 0.3N HCl. EDAC was dissolvedd in 0.1M Tris buffer pH 7.5 and then added over a period of 10 min (1/10 vol each minute). According to the conditions used (see Table 8), the reaction was stopped after between 30 and 180 minutes by addition of 1M Tris-HCl pH 7.5. Prior to purification on Sephacryl S400HR, the conjugate was clarified using a 5µm Minisart filter. The conjugate was then injected on Sephacryl S400HR (XK50/100). The flow-rate was fixed at 60 ml/h using 150 mM NaCl as elution buffer. The elution pool was selected on the basis of resorcinol and µBCA profiles (which measure polysaccharide and protein dosage respectively). Then, the conjugate was filtered on a 0.22 µm sterilizing membrane (Millipack 20) at 10 ml/min.

**Table 7**

| **Conjugate** | **Coupling time** | **[PS-AH] (mg/ml)** | **[TT] (mg/ml)** | **[EDAC] (mg/mg PS)** |
|---|---|---|---|---|
| **SA08-TT045** | 65 min | 3.83 | 7.66 | 0.1 |
| **SA08-TT046** | 45 min | 3.75 | 7.5 | 0.2 |
| **SA08-TT047** | 30 min | 5.0 | 15.0 | 0.2 |
| **SA08-TT048** | 120 min | 5.0 | 10.0 | 0.05 |
| **SA08-TT049*** | 50 min | 5.0 | 10.0 | 0.1 |

| | | | | |
|---|---|---|---|---|
| * EDAC added in "one time" | | | | |

**Table 8**

| **Conjugate** | **In. TT/PS ratio(w/w)** | **F. TT/PS ratio (w/w)** | **Y. PS rec (%)** | **Filtr. Yield (%)** |
|---|---|---|---|---|
| **SA08-TT045** | 2/1 | 2.20/1 | 57 | 101 |
| **SA08-TT046** | 2/1 | 2.80/1 | | |
| **SA08-TT047** | 3/1 | Gel- Not purified | - | - |
| **SA08-TT048** | 2/1 | 3.35 | 30 | 101 |
| **SA08-TT049** | 2/1 | 3.5 | 24 | 106 |

### Example 6 Preparation of S. aureus Capsular Polysaccharide Conjugates using EDAC on de-O-acetylated S. aureus polysaccharide 8

### De-O-acetylation

0.1N NaOH was added to 16 ml of sized PS (10 mg/ml) to target a final PS concentration of 9 mg/ml and a final NaOH concentration of 0.1N. After a treatment of 1 or 2 h at 37°C, the PS had a level of O-acetylation of 35 and 12% (Hestrin dosage) respectively in comparison to the untreated PS. 0.1N NaOH was added to 19 ml of sized PS (10 mg/ml) to target a final PS concentration of 9.5 mg/ml and a final NaOH concentration of 0.05N. After a treatment of 1 or 2 h at 37°C, PS had a level of O-acetylation of 78 and 58% (Hestrin dosage) respectively in comparison to the untreated PS.

The derivatization step was done as shown previously for an untreated PS.

**Table 9**

| **Conjugate** | **O-acetyl level %** | **ADH/PS w/w* %** |
|---|---|---|
| **SA08-TT056** | 35 | 9.3 |
| **SA08-TT057** | 12 | 13.1 |
| **SA08-TT058** | 78 | 5.3 |
| **SA08-TT059** | 58 | 8.2 |

| | | |
|---|---|---|
| * TNBS assay | | |

Removal of the O-acetyl groups resulted in an increased availability of reactive carboxylic groups. Indeed, the derivatization level of a PS having only 12% of O-acetyl groups was ± 2.5-fold superior to the one having 78% of O-acetyl groups.

Coupling was done as shown previously for a untreated PS

**Table 10**

| **Conjugate** | **O-acetyl level %** | **Coupling time** | **[PS-AH] (mg/ml)** | **[TT] (mg/ml)** | **[EDAC] (mg/mg PS)** |
|---|---|---|---|---|---|
| **SA08-TT056** | 35 | 45 min | 2.87 | 5.74 | 0.5 |
| **SA08-TT057** | 12 | 30 min | 2.62 | 5.24 | 0.5 |
| **SA08-TT058** | 78 | 50 min | 3.16 | 6.32 | 0.5 |
| **SA08-TT059** | 58 | 40 min | 2.53 | 5 | 0.5 |

**Table 11**

| **Conjugate** | **In. TT/PS ratio(w/w)** | **F. TT/PS ratio (w/w)** | **Y. PS rec (%)** | **Filtr. Yield (%)** |
|---|---|---|---|---|
| **SA08-TT056** | 2/1 | 1.70/1 | 51.3 | 100 |
| **SA08-TT057** | 2/1 | 1.78/1 | 63.0 | 105.4 |
| **SA08-TT058** | 2/1 | 2.08/1 | 46.3 | 99.6 |
| **SA08-TT059** | 2/1 | 1.86/1 | 50.8 | 99.2 |

### Example 7 Conjugation of dPNAG

### Activation and coupling of dPNAG:

### dPNAG-TT conjugates

The following conjugates were produced using the approaches described herebelow:
dPNAG-TT010: dPNAG-S-GMBS + DTT treated TT-LC-SPDP
dPNAG-TT011: dPNAG-S-GMBS + DTT treated TT-LC-SPDP
dPNAG-TT012: dPNAG-S-GMBS + DTT treated TT-SPDP
dPNAG-TT014: dPNAG-SPDP + DTT treated TT-SPDP
dPNAG-TT017:DTT treated dPNAG-SPDP + TT-LC-SPDP
dPNAG-TT019: dPNAG-S-GMBS + DTT treated TT-SPDP
dPNAG-TT020: dPNAG-S-GMBS +DTT treated TT-SPDP

### dPNAG

1g of PNAG was dissolved in 5N HCl at a concentration of 20mg/ml and was incubated for 1 hour. It was then neutralized with 5N NaOH. The solution was clarified on a 5µm membrane and purified on Sephacryl S400HR. Interesting fractions, corresponding to the "medium molecular size" (see Infection and Immunity, 70: 4433-4440 (2002)), were pooled and concentrated prior to de-N-acetylation treatment.

The solution was adjusted at 1M NaOH and left 24 hours at 37°C. After neutralization, the product was subjected to dialysis and concentration.

### dPNAG Activation

S-GMBS (N-(γ-Maleimidobutyryloxy) sulfosuccinimide, Pierce) was added to dPNAG in 0.2M NaCl (ratio S-GMBS/PS (w/w):1/1) and incubated during 2h at room temperature at pH 7.0 (pH regulation using 1M NaOH). Excess GMBS and by-products were removed by purification on Toyopearl HW-40F using PBS, 10mM EDTA, 50 mM NaCl pH 7.2 as elution buffer with a flow-rate fixed at 60 ml/h. The elution pool was selected in function of the optical density (UV=206 nm) and then concentrated on Vivaspin tubes 3,000 MWCO or Amicon Ultra 10,000 MWCO.

### Coupling

GMBS-activated dPNAG and DTT reduced TT-SPDP were mixed and stirred at room temperature. According to the conditions used the reaction was quenched after 20-120 min by the addition of cysteine (4 mg/ml in Na phosphate buffer pH 8.0) for 30 minutes. The conjugate was clarified on 5 µm filter and injected on Sephacryl S300HR resin (XK16/100) for purification. Elution was realized in 200 mM NaCl with a flow-rate fixed at 30 ml/h. The elution fractions were analysed by hexosamine and by protein dosage. Interesting fractions were pooled and filtered on 0.22 µm Sterivex. The final conjugate was tested for polysaccharide (hexosamine dosage) and protein composition (Lowry dosage).

**Table 12**

| **Conjugate** | **N-acetylation level %** | **[dPNAG] mg/ml** | **[TT] mg/ml** | **PS scale (mg)** | **Coupl. time (min)** |
|---|---|---|---|---|---|
| **dPNAG-TT 010** | 10* | 15 | 15 | 30 | 120 |
| **dPNAG-TT 011** | 10* | 12 | 24 | 20 | 120 |
| **dPNAG-TT 012** | 10* | 17.5 | 35 | 22 | 80 |
| **dPNAG-TT 019** | 34 | 5 | 10 | 10 | 20 |
| **dPNAG-TT 020** | 34 | 2 | 2 | 10 | 20 |

| | | | | | |
|---|---|---|---|---|---|
| *Not done on the lot used in the conjugation but estimated on a previous lot by NMR using the same de-N-acetylation method. | | | | | |

**Table 13**

| **Conjugate** | **In.TT/PS ratio (w/w)** | **F.TT/PS ratio (w/w)** | **Yield PS rec (%)** | **Filtration yield (%)** |
|---|---|---|---|---|
| **dPNAG-TT010** | 1/1 | 1.86/1 | 43 | 99 |
| **dPNAG-TT011** | 2/1 | 2.86/1 | 56 | 99 |
| **dPNAG-TT012** | 2/1 | 2.29/1 | 61 | 108 |
| **dPNAG-TT019** | 2/1 | 1.45/1 | 81 | 97 |
| **dPNAG-TT020** | 1/1 | 0.89/1 | 82 | 109 |

### dPNAG-SPDP:

A 5-fold molar excess of SPDP (N-Succinimidyl-3-(2-Pyridyldithio) Propionate, MW: 312.4, Pierce) dissolved in DMSO (dimethylsulfoxid, Merck) was added to 100 mg of dPNAG at 5mg/ml in 100 mM Na phosphate, pH 7.2) and incubated 1 h at room temperature. Before purification on Sephacryl S100HR (XK16/40) the reaction mixture was concentrated to ± 6 ml on Amicon Ultra 10,000 MWCO (centrifugation at 3000 rpm during 28 min). Elution was realized in phosphate buffer pH 7.4with a flow-rate fixed at 60 ml/h. The interesting fractions (read at 206 nm) were pooled and concentrated to 1.1 ml on Amicon Ultra 10,000 MWCO (centrifugation at 3000 rpm during 30 min).

### TT-SPDP:

A 15-fold molar excess of SPDP (Pierce) dissolved in DMSO (dimethylsulfoxid, Merck) was added to 1 g of TT (50 mg/ml) in 100 mM Na phosphate, pH 7.2 and incubated 80 min at room temperature. Then the product was injected on Sephacryl S100HR (XK16/40) and eluted in 100 mM Na acetate pH 5.6, 100 mM NaCl, 1mM EDTA with a flow-rate fixed at 60 ml/h. The elution pool was selected in function of the optical density (UV=280nm) and then concentrated to 19.6 ml on Amicon Ultra 10,000 MWCO (centrifugation at 3000 rpm during 75 min).

TT-LC-SPDP was produced as TT-SPDP but using LC-SPDP (Succinimidyl 6-[3-(2-pyridyldithio)-propionamido]hexanoate, Pierce) and an incubation time of 60 min.

### TT-SH or TT-LC-SH

DTT was added to TT-SPDP or TT-LC-SPDP in a DTT/TT ratio (mg/mg) of 0.7/1. After 2h at room temperature, the release of pyridine-2-thione was followed by its characteristic absorbance at 343 nm. The thiolated protein was purified from excess DTT by gel filtration (PD-10, Amersham). After concentration on Amicon Ultra 10,000 MWCO, protein content was estimated by Lowry dosage.

### dPNAG-SPDP + TT-SH or TT-LC-SH (dPNAG-TT014 and 016)

Coupling was performed at room temperature under continuous stirring and with an initial TT/PS ratio (w/w) of 2/1.

dPNAG and TT-SH were mixed in order to obtain a final PS concentration of 20 mg/ml and a final protein concentration of 40 mg/ml. After 30 min, unreacted sulfhydryl groups were quenched by addition of 2-lodoacetamide (Merck).

dPNAG and TT-LC-SH was mixed in order to obtain a final PS concentration of 10 mg/ml and a final protein concentration of 20 mg/ml. After 75 min, unreacted sulfhydryl groups were quenched by addition of 2-lodoacetamide (Merck).

Then the conjugate is clarified using a 5 µm Minisart filter and injected on Sephacryl S300HR (XK16/100). Elution was realized in 200 mM NaCl with a flow-rate fixed at 30 ml/h.

The elution fractions were analysed by hexosamine and by protein dosage. Interesting fractions were pooled and filtered on 0.22 µm Sterivex.

The resulting conjugates have a final TT/PS ratio (w/w) of 2.18 (TT-SH) and 2.24 (TT-LC-SH).

### Thiolation of dPNAG

11.6 mg of DTT (1, 4-Dithiothreitol, Boerhinger Mannheim, MW: 154.24) were added to 16.5 mg of dPNAG-SPDP. After 2 h at room temperature, the release of pyridine-2-thione was followed by its characteristic absorbance at 343 nm. The thiolated PS was purified from excess DTT by gel filtration (Toyopearl HW40F) and then concentrated to 860 µl on Amicon Ultra 10,000 MWCO.

### dPNAG-SH + TT-SPDP (dPNAG-TT017)

Coupling was performed at room temperature under continuous stirring and with an initial TT/PS ratio (w/w) of 1.7/1.

dPNAG-SH and TT-SPDP were mixed in order to obtain a final PS concentration of 7.73 mg/ml and a final protein concentration of 13.3 mg/ml. After 90 min, unreacted sulfhydryl groups were quenched by addition of 2-lodoacetamide (Merck).

Then the conjugate was clarified using a 5 µm Minisart filter and injected on Sephacryl S300HR (XK16/100). Elution was realized in 200 mM NaCl with a flow-rate fixed at 30 ml/h.

The elution fractions are analysed by hexosamine and by protein dosage. Interesting fractions were pooled and filtered on 0.22 µm Sterivex.

The resulting conjugate has a final TT/PS ratio (w/w) of 2.74.

### Example 8 Formulation

### Adjuvant compositions

The conjugates were inoculated either unadjuvanted or adjuvanted with adjuvant A, having the following composition:

### Composition of Adjuvant A

### Qualitative Quantitative (per 0.5 mL dose)

Liposomes:
   - DOPC 1 mg
   - cholesterol 0.25 mg
   3DMPL 50 µg
   QS21 50 µg
   KH₂PO_{4 1} 3.124 mg Buffer
   Na₂HPO_{4 1} 0.290 mg Buffer
   Nacl 2.922 mg
   (100 mM)
   WFI q.s. ad 0.5 ml Solvent
   pH 6.1
   1. Total PO₄ concentration = 50 mM

### Example 9

### Animal experiments.

Female CD-1 mice, 8 to 10 weeks old, are obtained from Charles River Laboratories, Kingston, Mass. For lethality studies, five groups of 9 to 11 CD-1 mice are challenged intraperitoneally (i.p.) with serial dilutions of *S*. *aureus* grown on CSA plates. The inocular sizes range from ∼10¹⁰ to 10⁸ CFU/mouse. Mortality is assessed on a daily basis for 3 days. The 50% lethal doses (LD₅₀s) is estimated by using a probit model of the dose-response relationship. The null hypothesis of common LD₅₀s was tested by the likelihood ratio test. Sublethal bacteremia is initiated by challenging groups of 8 to 20 mice by the intravenous (i.v.) route with ∼ 2 x 10⁶ CFU/mouse or by the i.p. route with ∼ 2 x 10⁷ CFU/mouse. After inoculation separate groups of animals are bled from the tail at specified times, and the bacteremia levels are estimated by quantitative plate counts performed in duplicate on tryptic soy agar plates with 5% sheep blood (Becton Dickinson Microbiology Systems). Statistical significance is determined with the Welch modification of the unpaired Stutent's *t* test.

### Example 10

### Immunogenicity of S. aureus PS8-TT and dPNAG-TT conjugates

Groups of 30 mice were inoculated subcutaneously with *S*. *aureus* PS8-TT conjugate at a saccharide dose of 3µg, either unadjuvanted or combined with adjuvant A, on days 0, 14, 28 and 42. On day 0, the mice received a first saccharide dose including between 0.001 and 0.013µg. The further three immunisations were done wuth a dose of 0.3µg in saline. On day 55 serum was collected from the mice and each serum sample was tested by ELISA to assess the immune response against PS8. Groups of 10 mice were used in the control groups and these were inoculated with either saline or saline containing adjuvant A.

The purified PS8 was coated at 2 µg/ml in phosphate buffered saline (PBS) on high binding microtitre plates (Nunc Maxisorp) overnight at 4° C. The plates were blocked with PBS-BSA 1 % for 30 min at room temperature with agitation. The mice antisera were prediluted 1/100 , then further twofold dilutions were made in microplates which were incubated at 37°C for 1 hour. After washing, bound murine antibody was detected using Jackson ImmunoLaboratories Inc. peroxidase-conjugated affiniPure Goat Anti-Mouse IgG (H+L) (ref: 115-035-003) diluted 1:5000 in PBS-tween 0.05%. The detection antibodies were incubated for 30 minutes at room temperature with agitation. The color was developed using 4 mg OPD (Sigma) + 5 µl H2O2 per 10 ml pH 4.5 0.1 M citrate buffer for 15 minutes in the dark at room temperature. The reaction was stopped with 50 µl HCl, and the optical density was read at 490 nm relative to 650 nm.

The results were expressed in mid-point titers and the GMT was calculated for the 30 samples (10 for controls). The results are shown in Table 14 below.

**Table 14**

| **Conjugate** | **Anti-PS8 titre (GMT) nonadsorbed** | **Anti-PS8 titre (GMT) Adjuvant A** |
|---|---|---|
| **SA08-TT011** | 4714 | 2109 |
| **SA08-TT015** | 2806 | 5631 |
| **SA08-TT017** | 3770 | 4396 |
| **SA08-TT018** | 5349 | 4748 |
| **Control** | 50 | 50 |

Groups of 30 mice were inoculated subcutaneously with *S*. *aureus* dPNAG-TT conjugates (containing dPNAG which was between 10% and 30% N-acetylated) at a saccharide dose of 0.3µg in 200mM NaCl, either unadjuvanted or combined with adjuvant A. The mice received three inoculations on days 0, 14 and 28. On day 41 or 42 serum was collected from the mice and each serum sample was tested by ELISA to assess the immune response against PNAG. Groups of 10 mice were used in the control groups and these were inoculated with saline or with adjuvant alone.

### Anti-PNAG ELISA:

Purified PNAG (2.5 µg/ml) mixed with methylated HSA (2.5 µg/ml) diluted in phosphate buffered saline (PBS) was coated on high binding microtitre plates (Nunc Maxisorp) overnight at 4° C.

The plates were blocked with PBS-BSA 1%, 30 min at RT with agitation. The mice antisera were prediluted 1/100, then further twofold dilutions were made in microplates and incubated at room temperature with agitation for 1 hour. After washing, bound murine antibody was detected using Jackson ImmunoLaboratories Inc. peroxidase-conjugated affiniPure Goat Anti-Mouse IgG (H+L) (ref: 115-035-003) diluted 1:5000 in PBS-BSA 0.2%-tween 0.05%. The detection antibodies were incubated for 30 min. at room temperature with agitation. The color was developed using 4 mg OPD (Sigma) + 5 µl H2O2 per 10 ml pH 4.5 0.1M citrate buffer for 15 minutes in the dark at room temperature. The reaction was stopped with 50 µl HCl, and the optical density was read at 490 nm relative to 650 nm.

A GMT was calculated on the mid-point titers of the 30 samples (10 for the controls).

**Table 15**

| **Conjugate** | **Anti-PNAG GMT Non-adsorbed** | **Anti-PNAG GMT Adjuvant A** |
|---|---|---|
| **dPNAG-TT010** | 1371 | 28465 |
| **dPNAG-TT011** | 1133 | 40899 |
| **dPNAG-TT019** | 425 | 13429 |
| **dPNAG-TT020** | 656 | 10080 |
| **dPNAG-TT014** | 342 | 9806 |
| **dPNAG-TT017** | 203 | 8094 |
| **dPNAG-TT012** | 398 | 40509 |
| **dPNAG-TT016** | 719 | 7937 |
| **Control** | 50 | 50 |

### Example 11 Immunogenicity of PS*-TT conjugates made by the CDAP method

### Results

**Table 16**

| **Conjugate** | **Anti PS8 GMT post three inoculations in mice** | **Anti-PS8 GMT post two inoculations in mice** |
|---|---|---|
| **SAPSB-TT-04 Specol** | 207068 | 41326 |
| **SAPSB-TT-04 Adjuvant A** | 47405 | 15577 |
| **SAPS8-TT-04 AlPO4** | 7380 | 4510 |
| **Specol** | 50 | |
| **Adjuvant A** | 50 | |
| **AlPO4** | 50 | |

### Example 12

### Opsonophagocytosis assay.

The in vitro opsonophagocytosic killing of *S.aureus* by human polymorphonuclear leykocytes (PMNs) is performed as described in Xu et al 1992 Infect. Immun. 60; 1358. Human PMNs are prepared from heparinized blood by sedimentation in 3% dextran T-250. The opsonic reaction mixture (1 ml) contains ∼ 10⁶ PMNs in RPMI 1640 medium supplemented with 10% heat-inactivated fetal calf serum, ∼ 10⁸ CFU of S-aureus, and 0.1 ml of the test serum or IgG preparation. Hyperimmunized rabbit serum is used as a positive control, and 0.1 ml of nonimmune rabbit serum was used as a complete source for the IgG samples. The reaction mixtures are incubated at 37°C, and bacterial samples are transferred at 0, 60, and 120 min into water and subsequently diluted, spread on tryptic soy agar plates, and incubated at 37°C for bacterial count after overnight incubation.

### Example 13

### Immunogenicity of staphylococcal proteins in mice and rabbits

Animals were immunized with purified staphylococcal proteins in order to generate hyperimmune sera. Mice were immunized three times (days 0, 14 and 28) with 10 µg of each proteins adjuvanted in Specol. Rabbits were immunized three times (days 0, 21 and 42) with 20 µg of each proteins adjuvanted in Specol. Immune sera were collected and evaluated in anti-protein and anti-killed whole cells ELISA.

### Anti-Protein ELISA:

The purified protein was coated at 1 µg/ml in phosphate buffered saline (PBS) on high binding microtitre plates (Nunc Maxisorp) overnight at 4° C. The plates were blocked with PBS-BSA 1%, for 30 min at RT with agitation. The test samples were then diluted 1/1000 and incubated at room temperature for 1 hour with agitation. After washing, bound murine or rabbit antibody was detected using Jackson ImmunoLaboratories Inc. peroxidase-conjugated affiniPure Goat Anti-Mouse IgG (H+L) (ref: 115-035-003) or AffiniPure Goat Anti-Rabbit IgG (H+L) (ref: 11-035-003) diluted 1:5000 in PBS-tween 0.05%. The detection antibodies were incubated for 30 min. at room temperature with agitation. The color was developed using 4 mg OPD (Sigma) + 5 µl H2O2 per 10 ml pH 4.5 0.1M citrate buffer for 15 minutes in the dark at room temperature. The reaction was stopped with 50 µl HCl, and the optical density was read at 490 nm relative to 650 nm.

The O.D. for a 1/1000 dilution of Post III was compared to the O.D. obtained with the same dilution of Pre-immune sera.

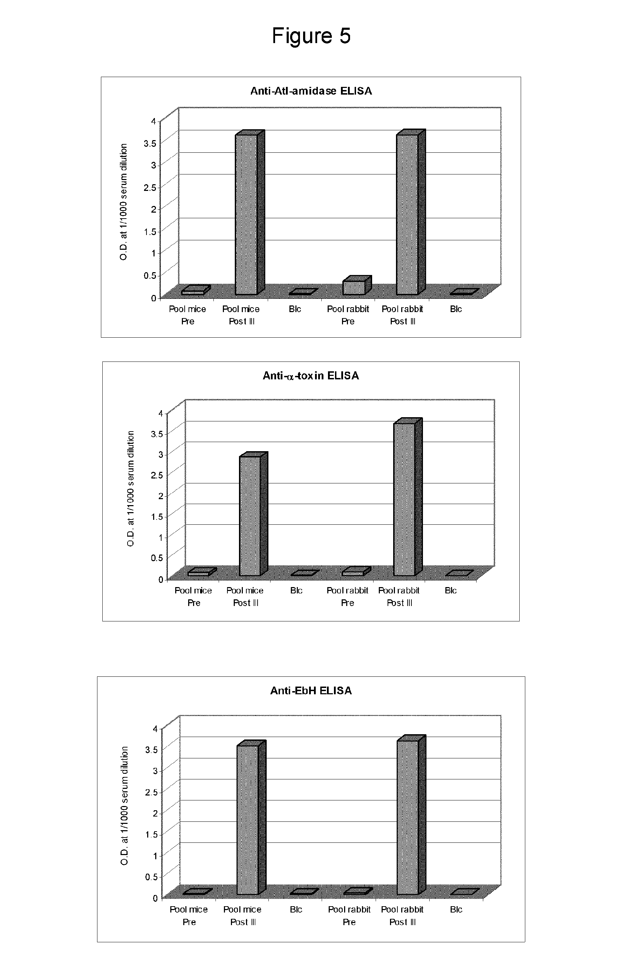

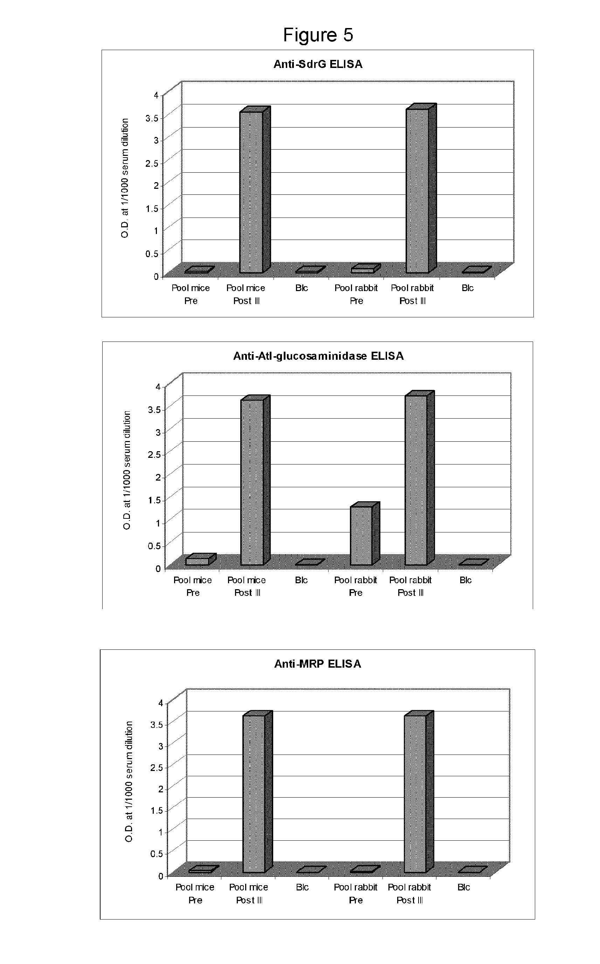

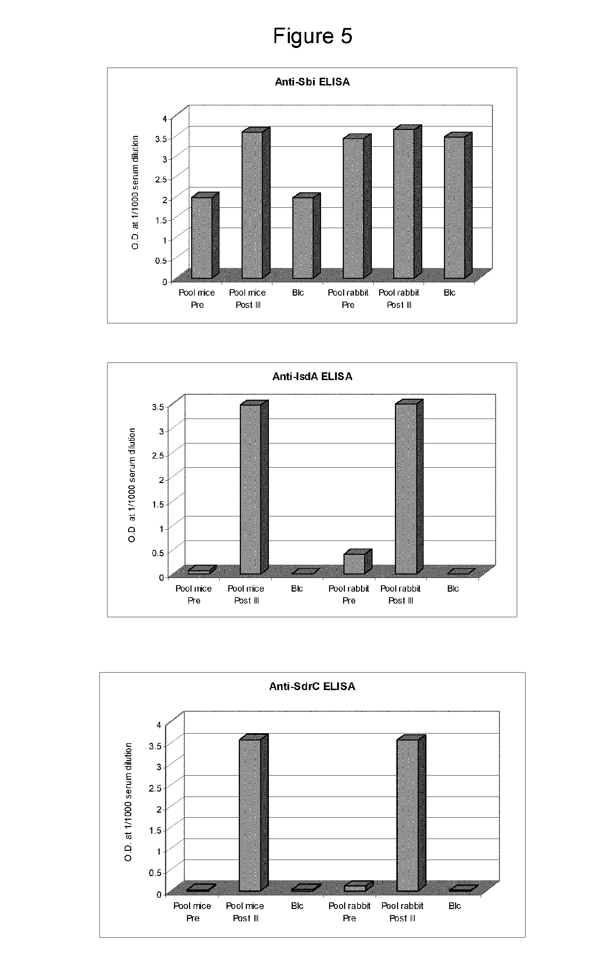

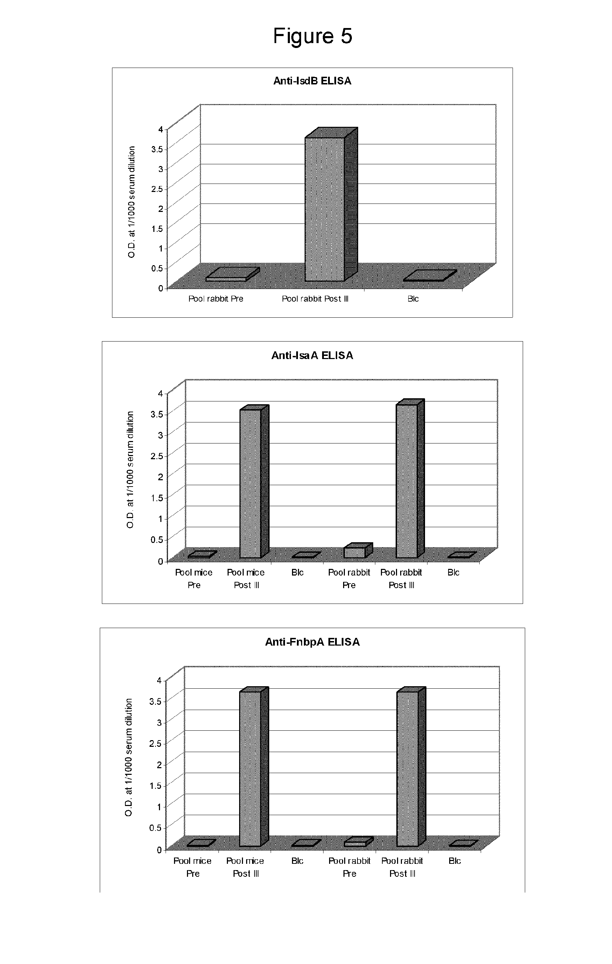

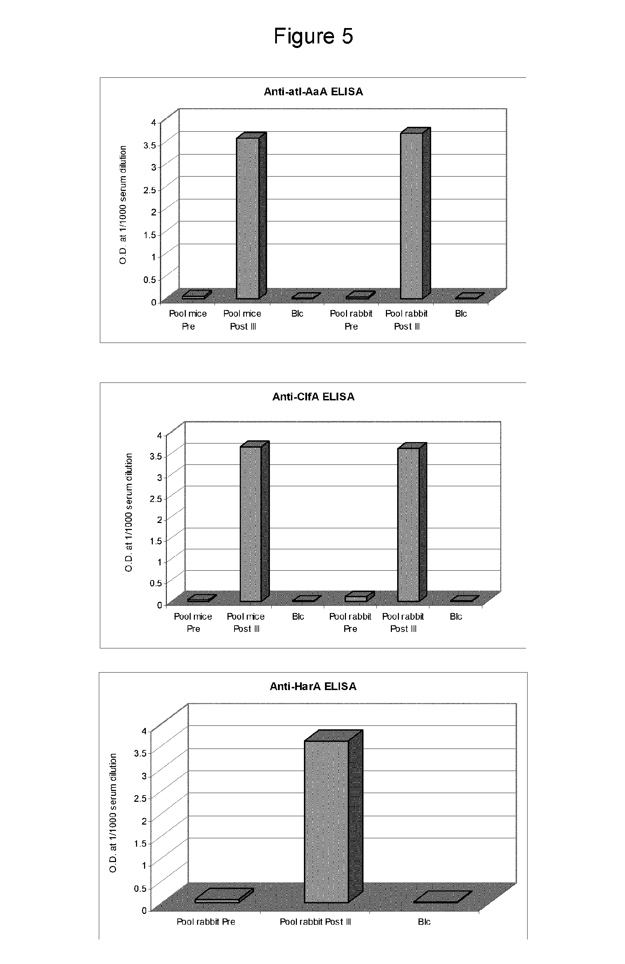

Results generated with mice and rabbit sera are presented in Figure 5. A good seroconversion against each antigen was observed. Evaluation of sera directed against SBI was impaired due to the Ig binding activity of this protein.

### Anti-killed whole cells ELISA:

Killed whole cells (heat or formaldehyde inactivated) from *S*. *aureus* type 5 and 8 or *S*. *epidermidis* strain Hay were coated at 20 µg/ml in phosphate buffered saline (PBS) on high binding microtitre plates (Nunc Maxisorp) overnight at 4° C with evaporation. The plates were blocked with PBS-BSA 1% 30 min at room temperature with agitation. Protein A was neutralised by addition of 10µg/ml of Affinity Purified Chickedn anti-ProteinA (ICL ref: CPA-65A-2) diluted in PBS-tween 0.05% followed by incubation for 1 hour at room temperature. The test samples were then diluted two-fold on the microplate in PBS-0.05% from a starting dilution at 1/10 and incubated 1 hour at room temperature with agitation. After washing, bound murine or rabbit antibody was detected using Jackson ImmunoLaboratories Inc. peroxidase-conjugated affiniPure Goat Anti-Mouse IgG (H+L) (ref: 115-035-003) or AffiniPure Goat Anti-Rabbit IgG (H+L) (ref: 11-035-003) diluted 1:5000 in PBS-tween 0.05%. This detection antibodies were incubated for 30 min. at room temperature with agitation. The color was developed using 4 mg OPD (Sigma) + 5 µl H2O2 per 10 ml pH 4.5 0.1M citrate buffer for 15 minutes in the dark, at room temperature. The reaction was stopped with 50 µl HCl, and the optical density was read at 490 nm relative to 650 nm.

It should be noted that expression levels of proteins in staphylococci will vary depending on culture conditions. Therefore a negative result may reflect the choice of incorrect culture conditions rather than a lack of immunogenicity.

The results using mice sera are shown in Table 17 and some of the graphs are shown in figure 6. A weak recognition of *S*. *aureus* strain 5 is observed with sera directed against SdrC, FnbpA, Ebh, Sbi and IsaA. Recognition of *S*. *aureus* strain 8 is only observed with the serum directed against Sbi. Weak recognition of *S*. *epidermidis* Hay is observed with sera directed against Atl amidase, MRPII, IsdA, IsaA, Ebh, Aaa and Sbi.

A selection of results generated using rabbit sera are shown in figure 7 and summarized in Table 18. Very good recognition of the three strains was observed with IsaA and IsdB. A weak recognition of the three stains was observed with HarA although animals only received one injection rather than the three injections used for the other proteins.

**Table 17**

| **Protein name** | **React on SA5** | **React on SA8** | **React on SE Hay** |
|---|---|---|---|
| IsaA | (+) | (+) | (+) |
| ClfA | - | (+) | (+) |
| Atl amidase | - | - | ++ |
| SdrG | - | - | - |
| Glucosamidase | - | - | - |
| IsdA | - | - | ++ |
| Alpha toxin | - | - | - |
| SrdC | ++ | (+) | - |
| Ebh | + | - | + |
| AaA | - | - | ++ |
| MRPII | - | - | ++ |
| Sbi | ++ | ++ | +++ |
| FnbpA | + | + | (+) |

**Table 18**

| **Protein name** | **React on SA5** | **React on SA8** | **React on SE Hay** |
|---|---|---|---|
| IsaA | +++ | +++ | +++ |
| ClfA | + | ++ | ++ |
| Atl amidase | - | ++ | + |
| IsdB | +++ | +++ | +++ |
| SdrG | + | + | + |
| Glucosamidase | - | - | - |
| HarA (1 inject.) | + | + | + |
| IsdA | - | - | - |
| Alpha toxin | - | - | + |
| SrdC | - | - | - |
| Ebh | - | + | - |
| AaA | - | - | - |
| MRPII | - | - | ++ |
| Sbi | - | +++ | - |
| FnbpA | - | ++ | ++ |

### Example 14

**Efficacy of combinations of staphylococcal proteins in a nasal colonization model.**

Fifteen groups of three cotton rats were inoculated with combinations of eight staphylococcal antigens and five cotton rats which acted as controls were treated with no antigen. These sixteen groups are as follows:
Group 1 - Atl-glucosamine, Atl-amidase, AAA, alpha toxin, SdrC, SdrG, Ebh, Sbi
Group 2 - Atl-glucosamine, Atl-amidase, IsdA, IsdB, ClfA, SdrC, Ebh, FnbpA
Group 3 - Atl-glucosamine, Atl-amidase, HarA, IsdA, MRPII, IsdB, AAA, alpha toxin
Group 4 - Atl-glucosamine, HarA, IsdA, AAA, ClfA, IsaA, Ebh, Sbi
Group 5 - HarA, MRPII, AAA, alpha toxin, ClfA, SdrC, Ebh, FnbpA
Group 6 - IsdA, IsdB, AAA, alpha toxin, ClfA, SdrG, Sbi, FnbpA
Group 7 - Atl-aminidase, IsdA, MRPII, AAA, IsaA, SdrG, Ebh, FnbpA
Group 8 - Control
Group 9 - Atl-glucosamine, IsdA, MRPII, alpha toxin, IsaA, SdrC, Sbi, FnbpA
Group 10 - Atl-glucosamine, MRPII, IsdB, AAA, ClfA, IsaA, SdrC, SdrG
Group 11- Atl-amindase, MRPII, IsdB, alpha toxin, ClfA, IsaA, Ebh, Sbi
Group 12 - Atl-glucosamine, HarA, IsdB, alpha toxin, IsaA, SdrG, Ebh, FnbpA
Group 13 - Atl-amidase, HarA, IsdB, AAA, IsaA, SdrC, Sbi, FnbpA
Group 14 - Atl-glucosamine, Atl-amidase, HarA, MRPII, ClfA, SdrG, Sbi, FnbpA
Group 15 - Atl-amidase, HarA, IsdA, alpha toxin, ClfA, IsaA, SdfC, SdrG
Group 16 - HarA, IsdA, MRPII, IsdB, SdrC, SdrG, Ebh, Sbi

Each mix of antigens contained 3µg of each antigen mixed with an adjuvant made of liposomes containing MPL and QS21. The cotton rats were inoculated three times on days 1, 14 and 28 of the experiment. Two weeks after inoculation, the efficacy of the immunisations were assessed using a nasal colonisation assay as described in Kokai-Kun et al (2003) Antimicrob.Agents.Chemother. 47; 1589-1597.

Classical multiple linear regression analysis was carried out on the data using "Design Expert 6" software. The presence of an antigen was coded as +1 and the absence of an antigen by -1. Using the equation of the model it was possible to determine which antigens were the key antigens which produced a large decrease in the number of colonies per nose.

### Results

The results of the nasal colonisation assay are shown in Table 19. The control group had a mean logCFU/nose of 3.51335 and a decrease in nasal colonisation could be see for all the groups of cotton rats inoculated with staphylococcal proteins. Groups 4, 9 and 13 showed the greatest decrease in nasal colonisation with a decrease of over 2 logs in CFU/nose. Groups 12 and 16 also gave good results, showing a decease of about 2 logs in CFU/nose.

**Table 19**

| Group | Mean observed LogCFU/nose | Predicted LogCFU/nose |
|---|---|---|
| 1 | 1.77527 | 2.03560 |
| 2 | 2.90435 | 2.52684 |
| 3 | 1.96556 | 2.23033 |
| 4 | 1.27748 | 1.21872 |
| 5 | 1.67304 | 1.93128 |
| 6 | 2.79745 | 2.98193 |
| 7 | 2.21481 | 2.30705 |
| 8 | 3.51355 | 3.47317 |
| 9 | 1.22480 | 1.44080 |
| 10 | 2.03085 | 1.93204 |
| 11 | 2.02522 | 1.81581 |
| 12 | 1.53402 | 1.70996 |
| 13 | 1.36063 | 1.49100 |
| 14 | 2.31201 | 1.73909 |
| 15 | 2.22979 | 1.98223 |
| 16 | 1.58109 | 1.44004 |

The contribution of specific antigens within the antigen mix was calculated using multiple regression analysis of the nasal colonisation data. The final mdel contains the seven best antigens. Results for these antigens are shown in Table 20. Within the context of the protein mix, the inclusion of HarA gave the greatest decrease in nasal colonisation, followed by IsaA, Sbi, SdrC, autolysin-glucosamine, MRPII and Ebh.

**Table 20 Effects in difference of logCFU/nose and ratio of CFU/nose for the seven best antigens in the model and corresponding p-values.**

| antigen | prob >F | Effect estimate | Reduction ratio | Cumulative effect | Cumulative ratio |
|---|---|---|---|---|---|
| HarA | 0.033 | -0.596 | 3.9 | -0.596 | 3.9 |
| IsaA | 0.046 | -0.558 | 3.6 | -1.154 | 14.3 |
| Sbi | 0.077 | -0.491 | 3.1 | -1.645 | 44.2 |
| SdrC | 0.22 | -0.337 | 2.2 | -1.982 | 96.0 |
| Atl-glucos | 0.238 | -0.324 | 2.1 | -2.306 | 202.2 |
| MRPII | 0.239 | -0.323 | 2.1 | -2.629 | 425.3 |
| Ebh | 0.297 | -0.286 | 1.9 | -2.914 | 821.0 |

## Claims

1. An immunogenic composition comprising Type 5 and/or 8 capsular polysaccharide or oligosaccharide from *S*. *aureus* wherein the Type 5 capsular polysaccharide or oligosaccharide is between 30% and 100% O-acetylated.

2. An immunogenic composition comprising Type 5 and/or 8 capsular polysaccharide or oligosaccharide from *S*. *aureus* wherein the Type 8 capsular polysaccharide or oligosaccharide is between 30% and 100% O-acetylated.

3. The immunogenic composition of claim 2 wherein the Type 5 capsular polysaccharide or oligosaccharide is between 30% and 100% O-acetylated.

4. The immunogenic composition of any one of claims 1-3 comprising staphylococcal PNAG.

5. The immunogenic composition of claim 4 wherein the PNAG is less than 40% N acetylated.

6. The immunogenic composition of any one of claims 1-5 further comprising Type I, and/or Type II and/or Type III capsular polysaccharide or oligosaccharide from *S. epidermidis.*

7. The immunogenic composition of any one fo claims 1-6 further comprising a *S*. *aureus* 336 antigen.

8. The immunogenic composition of any one of claims 1-7 further comprising a staphylococcal protein or fragment thereof.

9. The immunogenic composition of claim 8 wherein the staphylococcal protein or fragment thereof is an extracellular component binding protein selected form the group consisting of laminin receptor, SitC/MntC/saliva binding protein, EbhA, EbhB, Elastin binding protein (EbpS), EFB (FIB), SBI, autolysin, ClfA, SdrC, SdrD, SdrE, SdrG, SdrH, Lipase GehD, SasA, FnbA, FnbB, Cna, ClfB, FbpA, Npase, IsaA/PisA, SsaA, EPB, SSP-1, SSP-2, HBP, Vitronectin binding protein, fibrinogen binding protein, coagulase, Fig and MAP.

10. The immunogenic composition of claim 8 wherein the staphylococcal protein or fragment thereof is a transporter protein selected from the group consisting of Immunodominant ABC transporter, IsdA, IsdB, IsdC, HarA, Mg2+ transporter, SitC and Ni ABC transporter.

11. The immunogenic composition of claim 8 wherein the staphylococcal protein or fragment thereof is a toxin or regulator of virulence selected from the group consisting of alpha toxin (Hla), alpha toxin H35R mutant, RNA III activating protein (RAP).

12. The immunogenic composition of any one of claims 8-11 comprising 2 or more staphylococcal proteins selected from at least 2 different groups selected from;
a) at least one staphylococcal extracellular component binding protein or fragment thereof selected from the group consisting of laminin receptor, SitC/MntC/saliva binding protein, EbhA, EbhB, Elastin binding protein (EbpS), EFB (FIB), SBI, autolysin, ClfA, SdrC, SdrD, SdrE, SdrG, SdrH, Lipase GehD, SasA, FnbA, FnbB, Cna, ClfB, FbpA, Npase, IsaA/PisA, SsaA, EPB, SSP-1, SSP-2, Vitronectin binding protein, fibrinogen binding protein, coagulase, Fig and MAP;
b) at least one staphylococcal transporter protein or fragment thereof selected from the group consisting of Immunodominant ABC transporter, IsdA, IsdB, IsdC, HarA, Mg2+ transporter, SitC and Ni ABC transporter;
c) at least one staphylococcal regulator of virulence, toxin or fragment thereof selected from the group consisting of alpha toxin (Hla), alpha toxin H35R mutant, RNA III activating protein (RAP).

13. The immunogenic composition of any one of claims 1-12 wherein a staphylococcal polysaccharide is conjugated to a protein carrier.

14. The immunogenic composition of claim 13 wherein the carrier protein comprises a staphylococcal protein or fragment thereof selected from the group consisting of laminin receptor, SitC/MntC/saliva binding protein, EbhA, EbhB, Elastin binding protein (EbpS), EFB (FIB), SBI, autolysin, ClfA, SdrC, SdrD, SdrE, SdrG, SdrH, Lipase GehD, SasA, FnbA, FnbB, Cna, ClfB, FbpA, Npase, IsaA/PisA, SsaA, EPB, SSP-1, SSP-2, HBP, Vitronectin binding protein, fibrinogen binding protein, coagulase, Fig, MAP, Immunodominant ABC transporter, IsdA, IsdB, IsdC, Mg2+ transporter, SitC and Ni ABC transporter, alpha toxin (Hla), alpha toxin H35R mutant and RNA III activating protein (RAP).

15. The immunogenic composition of claim 13 wherein the carrier protein is selected from the group consisting of tetanus toxoid, diphtheria toxoid, CRM197, *Haemophilus influenzae* protein D, *Pseudomonas aeruginosa* exoprotein A, pneumococcal pneumolysin and alpha toxoid.

16. The immunogenic composition of claims 1-15 wherein an effective immune response is generated against both *S*. *aureus* and *S*. *epidermidis.*

17. A vaccine comprising the immunogenic composition of claims 1-16 and a pharmaceutically acceptable excipient.

18. A method of making a vaccine comprising the steps of mixing antigens to make the immunogenic composition of claims 1-16 and adding a pharmaceutically acceptable excipient.

19. A use of the immunogenic composition of claims 1-16 in the manufacture of a vaccine for treatment or prevention of staphylococcal infection.

20. A process for conjugating Type 5 or 8 capsular polysaccharide or oligosaccharide from *S. aureus* comprising the steps of:
a) dissolving the Type 5 or 8 polysaccharide or oligosaccharide in water or a saline solution;
b) adding a cyanylating agent (for example CDAP) to form an activated polysaccharide or oligosaccharide;
c) adding carrier protein so that amino groups react with the activated polysaccharide to form an isourea covalent link;
wherein the Type 5 capsular polysaccharide or oligosaccharide is between 30% and 100% O-acetylated.


SEQUENCE LISTING

<110> GlaxoSmithKline Biologicals s.a.

<120> Immunogenic composition

<130> VB61915

<160> 170

<170> FastSEQ for Windows Version 4.0

<210> 1
<211> 533
<212> PRT
<213> Staphylococcus aureus

<400> 1
Met Leu Gln Val Thr Asp Val Ser Leu Arg Phe Gly Asp Arg Lys Leu
 1               5                  10                  15
Phe Glu Asp Val Asn Ile Lys Phe Thr Glu Gly Asn Cys Tyr Gly Leu
            20                  25                  30
Ile Gly Ala Asn Gly Ala Gly Lys Ser Thr Phe Leu Lys Ile Leu Ser
        35                  40                  45
Gly Glu Leu Asp Ser Gln Thr Gly His Val Ser Leu Gly Lys Asn Glu
    50                  55                  60
Arg Leu Ala Val Leu Lys Gln Asp His Tyr Ala Tyr Glu Asp Glu Arg
65                  70                  75                  80
Val Leu Asp Val Val Ile Lys Gly His Glu Arg Leu Tyr Glu Val Met
                85                  90                  95
Lys Glu Lys Asp Glu Ile Tyr Met Lys Pro Asp Phe Ser Asp Glu Asp
            100                 105                 110
Gly Ile Arg Ala Ala Glu Leu Glu Gly Glu Phe Ala Glu Met Asn Gly
        115                 120                 125
Trp Asn Ala Glu Ala Asp Ala Ala Asn Leu Leu Ser Gly Leu Gly Ile
    130                 135                 140
Asp Pro Thr Leu His Asp Lys Lys Met Ala Glu Leu Glu Asn Asn Gln
145                 150                 155                 160
Lys Ile Lys Val Leu Leu Ala Gln Ser Leu Phe Gly Glu Pro Asp Val
                165                 170                 175
Leu Leu Leu Asp Glu Pro Thr Asn Gly Leu Asp Ile Pro Ala Ile Ser
            180                 185                 190
Trp Leu Glu Asp Phe Leu Ile Asn Phe Asp Asn Thr Val Ile Val Val
        195                 200                 205
Ser His Asp Arg His Phe Leu Asn Asn Val Cys Thr His Ile Ala Asp
    210                 215                 220
Leu Asp Phe Gly Lys Ile Lys Val Tyr Val Gly Asn Tyr Asp Phe Trp
225                 230                 235                 240
Tyr Gln Ser Ser Gln Leu Ala Gln Lys Met Ala Gln Glu Gln Asn Lys
                245                 250                 255
Lys Lys Glu Glu Lys Met Lys Glu Leu Gln Asp Phe Ile Ala Arg Phe
            260                 265                 270
Ser Ala Asn Ala Ser Lys Ser Lys Gln Ala Thr Ser Arg Lys Lys Gln
        275                 280                 285
Leu Glu Lys Ile Glu Leu Asp Asp Ile Gln Pro Ser Ser Arg Arg Tyr
    290                 295                 300
Pro Phe Val Lys Phe Thr Pro Glu Arg Glu Ile Gly Asn Asp Leu Leu
305                 310                 315                 320
Ile Val Gln Asn Leu Ser Lys Thr Ile Asp Gly Glu Lys Val Leu Asp
                325                 330                 335
Asn Val Ser Phe Thr Met Asn Pro Asn Asp Lys Ala Ile Leu Ile Gly
            340                 345                 350

Asp Ser Glu Ile Ala Lys Thr Thr Leu Leu Lys Ile Leu Ala Gly Glu
        355                 360                 365
Met Glu Pro Asp Glu Gly Ser Phe Lys Trp Gly Val Thr Thr Ser Leu
    370                 375                 380
Ser Tyr Phe Pro Lys Asp Asn Ser Glu Phe Phe Glu Gly Val Asn Met
385                 390                 395                 400
Asn Leu Val Asp Trp Leu Arg Gln Tyr Ala Pro Glu Asp Glu Gln Thr
                405                 410                 415
Glu Thr Phe Leu Arg Gly Phe Leu Gly Arg Met Leu Phe Ser Gly Glu
            420                 425                 430
Glu Val Lys Lys Lys Ala Ser Val Leu Ser Gly Gly Glu Lys Val Arg
        435                 440                 445
Cys Met Leu Ser Lys Met Met Leu Ser Ser Ala Asn Val Leu Leu Leu
    450                 455                 460
Asp Glu Pro Thr Asn His Leu Asp Leu Glu Ser Ile Thr Ala Val Asn
465                 470                 475                 480
Asp Gly Leu Lys Ser Phe Lys Gly Ser Ile Ile Phe Thr Ser Tyr Asp
                485                 490                 495
Phe Glu Phe Ile Asn Thr Ile Ala Asn Arg Val Ile Asp Leu Asn Lys
            500                 505                 510
Gln Gly Gly Val Ser Lys Glu Ile Pro Tyr Glu Glu Tyr Leu Gln Glu
        515                 520                 525
Ile Gly Val Leu Lys
    530

<210> 2
<211> 535
<212> PRT
<213> Staphylococcus aureus

<400> 2
Met Leu Gln Val Thr Asp Val Ser Leu Arg Phe Gly Asp Arg Lys Leu
1               5                   10                  15
Phe Glu Asp Val Asn Ile Lys Phe Thr Glu Gly Asn Cys Tyr Gly Leu
            20                  25                  30
Ile Gly Ala Asn Gly Ala Gly Lys Ser Thr Phe Leu Lys Ile Leu Ser
        35                  40                  45
Gly Glu Ile Asp Ser Gln Thr Gly His Val Ser Leu Gly Lys Asp Glu
    50                  55                  60
Arg Leu Ala Val Leu Lys Gln Asp His Phe Ala Tyr Glu Asp Glu Arg
65                  70                  75                  80
Val Leu Asp Val Val Ile Lys Gly His Glu Arg Leu Tyr Gln Val Met
                85                  90                  95
Lys Glu Lys Asp Glu Ile Tyr Met Lys Pro Asp Phe Ser Asp Glu Asp
            100                 105                 110
Gly Ile Arg Ala Ala Glu Leu Glu Gly Glu Phe Ala Glu Met Asn Gly
        115                 120                 125
Trp Asn Ala Glu Ala Asp Ala Ala Asn Leu Leu Ser Gly Leu Gly Ile
    130                 135                 140
Glu Pro Asp Leu His Asp Lys Asn Met Ser Glu Leu Glu Asn Asn Gln
145                 150                 155                 160
Lys Val Lys Val Leu Leu Ala Gln Ser Leu Phe Gly Asp Pro Asp Val
                165                 170                 175
Leu Leu Leu Asp Glu Pro Thr Asn Gly Leu Asp Ile Pro Ala Ile Ser
            180                 185                 190
Trp Leu Glu Asp Phe Leu Ile Asn Phe Glu Asn Thr Val Ile Val Val
        195                 200                 205
Ser His Asp Arg His Phe Leu Asn Asn Val Cys Thr His Ile Ala Asp
    210                 215                 220
Leu Asp Phe Gly Lys Ile Lys Leu Tyr Val Gly Asn Tyr Asp Phe Trp
225                 230                 235                 240
Tyr Gln Ser Ser Gln Leu Ala Gln Lys Met Ala Gln Glu Gln Asn Lys
                245                 250                 255
Lys Lys Glu Glu Lys Met Lys Glu Leu Gln Asp Phe Ile Ala Arg Phe

                260                 265                 270
Ser Ala Asn Ala Ser Lys Ser Lys Gln Ala Thr Ser Arg Lys Lys Gln
        275                 280                 285
Leu Glu Lys Ile Glu Leu Asp Asp Ile Gln Pro Ser Ser Arg Arg Tyr
    290                 295                 300
Pro Tyr Val Lys Phe Thr Pro Glu Arg Glu Ile Gly Asn Asp Leu Leu
305                 310                 315                 320
Thr Val Glu Asn Leu Ser Lys Thr Ile Asp Gly Glu Lys Val Leu Asp
                325                 330                 335
Asn Val Ser Phe Thr Met Asn Pro Asn Asp Lys Ala Ile Leu Val Gly
            340                 345                 350
Asp Ser Glu Ile Ala Lys Thr Thr Leu Leu Lys Ile Leu Ala Gly Glu
        355                 360                 365
Met Glu Pro Asp Glu Gly Thr Phe Lys Trp Gly Val Thr Thr Ser Leu
    370                 375                 380
Ser Tyr Phe Pro Lys Asp Asn Ser Glu Phe Phe Asp Gly Val Asp Met
385                 390                 395                 400
Asn Leu Val Glu Trp Leu Arg Gln Tyr Ala Pro Glu Asp Glu Gln Thr
                405                 410                 415
Glu Thr Phe Leu Arg Gly Phe Leu Gly Arg Met Leu Phe Ser Gly Glu
            420                 425                 430
Glu Val Lys Lys Lys Ala Ser Val Leu Ser Gly Gly Glu Lys Val Arg
        435                 440                 445
Cys Met Leu Ser Lys Met Met Leu Ser Ser Ala Asn Val Leu Leu Leu
    450                 455                 460
Asp Glu Pro Thr Asn His Leu Asp Leu Glu Ser Ile Thr Ala Val Asn
465                 470                 475                 480
Asp Gly Leu Lys Ser Phe Lys Gly Ser Ile Ile Phe Thr Ser Tyr Asp
                485                 490                 495
Phe Glu Phe Ile Asn Thr Ile Ala Asn Arg Val Ile Asp Leu Asn Gln
            500                 505                 510
Ala Gly Ala Leu Ser Lys Glu Val Pro Tyr Glu Glu Tyr Leu Gln Glu
        515                 520                 525
Ile Gly Val Leu Gln Asn Asn
    530                 535

<210> 3
<211> 434
<212> PRT
<213> Staphylococcus aureus

<400> 3

<210> 4
<211> 434
<212> PRT
<213> Staphylococcus aureus

<400> 4
Met Pro Ile Ile Thr Asp Val Tyr Ala Arg Glu Val Leu Asp Ser Arg
1               5                   10                  15
Gly Asn Pro Thr Val Glu Val Glu Val Leu Thr Glu Ser Gly Ala Phe
            20                  25                  30
Gly Arg Ala Leu Val Pro Ser Gly Ala Ser Thr Gly Glu His Glu Ala
        35                  40                  45
Val Glu Leu Arg Asp Gly Asp Lys Ser Arg Tyr Leu Gly Lys Gly Val
    50                  55                  60
Thr Lys Ala Val Glu Asn Val Asn Glu Met Ile Ala Pro Glu Ile Val
65                  70                  75                  80
Glu Gly Glu Phe Ser Val Leu Asp Gln Val Ser Ile Asp Lys Met Met
                85                  90                  95
Ile Gln Leu Asp Gly Thr His Asn Lys Gly Lys Leu Gly Ala Asn Ala
            100                 105                 110
Ile Leu Gly Val Ser Ile Ala Val Ala Arg Ala Ala Ala Asp Leu Leu
        115                 120                 125
Gly Gln Pro Leu Tyr Lys Tyr Leu Gly Gly Phe Asn Gly Lys Gln Leu
    130                 135                 140
Pro Val Pro Met Met Asn Ile Val Asn Gly Gly Ser His Ser Asp Ala
145                 150                 155                 160
Pro Ile Ala Phe Gln Glu Phe Met Ile Leu Pro Val Gly Ala Glu Ser
                165                 170                 175

Phe Lys Glu Ser Leu Arg Trp Gly Ala Glu Ile Phe His Asn Leu Lys
            180                 185                 190
Ser Ile Leu Ser Glu Arg Gly Leu Glu Thr Ala Val Gly Asp Glu Gly
        195                 200                 205
Gly Phe Ala Pro Arg Phe Glu Gly Thr Glu Asp Ala Val Glu Thr Ile
    210                 215                 220
Ile Lys Ala Ile Glu Lys Ala Gly Tyr Lys Pro Gly Glu Asp Val Phe
225                 230                 235                 240
Leu Gly Phe Asp Cys Ala Ser Ser Glu Phe Tyr Glu Asn Gly Val Tyr
                245                 250                 255
Asp Tyr Thr Lys Phe Glu Gly Glu His Gly Ala Lys Arg Ser Ala Ala
            260                 265                 270
Glu Gln Val Asp Tyr Leu Glu Glu Leu Ile Gly Lys Tyr Pro Ile Ile
        275                 280                 285
Thr Ile Glu Asp Gly Met Asp Glu Asn Asp Trp Glu Gly Trp Lys Gln
    290                 295                 300
Leu Thr Asp Arg Ile Gly Asp Lys Val Gln Leu Val Gly Asp Asp Leu
305                 310                 315                 320
Phe Val Thr Asn Thr Glu Ile Leu Ser Lys Gly Ile Glu Gln Gly Ile
                325                 330                 335
Gly Asn Ser Ile Leu Ile Lys Val Asn Gln Ile Gly Thr Leu Thr Glu
            340                 345                 350
Thr Phe Asp Ala Ile Glu Met Ala Gln Lys Ala Gly Tyr Thr Ala Val
        355                 360                 365
Val Ser His Arg Ser Gly Glu Thr Glu Asp Thr Thr Ile Ala Asp Ile
    370                 375                 380
Ala Val Ala Thr Asn Ala Gly Gln Ile Lys Thr Gly Ser Leu Ser Arg
385                 390                 395                 400
Thr Asp Arg Ile Ala Lys Tyr Asn Gln Leu Leu Arg Ile Glu Asp Glu
                405                 410                 415
Leu Tyr Glu Thr Ala Lys Phe Glu Gly Ile Lys Ser Phe Tyr Asn Leu
            420                 425                 430
Asp Lys

<210> 5
<211> 255
<212> PRT
<213> Staphylococcus aureus

<400> 5
Met Lys Lys Ile Val Thr Ala Thr Ile Ala Thr Ala Gly Leu Ala Thr
1               5                   10                  15
Ile Ala Phe Ala Gly His Asp Ala Gln Ala Ala Glu Gln Asn Asn Asn
            20                  25                  30
Gly Tyr Asn Ser Asn Asp Ala Gln Ser Tyr Ser Tyr Thr Tyr Thr Ile
        35                  40                  45
Asp Ala Gln Gly Asn Tyr His Tyr Thr Trp Thr Gly Asn Trp Asn Pro
    50                  55                  60
Ser Gln Leu Thr Gln Asn Asn Thr Tyr Tyr Tyr Asn Asn Tyr Asn Thr
65                  70                  75                  80
Tyr Ser Tyr Asn Asn Ala Ser Tyr Asn Asn Tyr Tyr Asn His Ser Tyr
                85                  90                  95
Gln Tyr Asn Asn Tyr Thr Asn Asn Ser Gln Thr Ala Thr Asn Asn Tyr
            100                 105                 110
Tyr Thr Gly Gly Ser Gly Ala Ser Tyr Ser Thr Thr Ser Asn Asn Val
        115                 120                 125
His Val Thr Thr Thr Ala Ala Pro Ser Ser Asn Gly Arg Ser Ile Ser
    130                 135                 140
Asn Gly Tyr Ala Ser Gly Ser Asn Leu Tyr Thr Ser Gly Gln Cys Thr
145                 150                 155                 160
Tyr Tyr Val Phe Asp Arg Val Gly Gly Lys Ile Gly Ser Thr Trp Gly
                165                 170                 175
Asn Ala Ser Asn Trp Ala Asn Ala Ala Ala Ser Ser Gly Tyr Thr Val

180                 185                 190
Asn Asn Thr Pro Lys Val Gly Ala Ile Met Gln Thr Thr Gln Gly Tyr
        195                 200                 205
Tyr Gly His Val Ala Tyr Val Glu Gly Val Asn Ser Asn Gly Ser Val
    210                 215                 220
Arg Val Ser Glu Met Asn Tyr Gly His Gly Ala Gly Val Val Thr Ser
225                 230                 235                 240
Arg Thr Ile Ser Ala Asn Gln Ala Gly Ser Tyr Asn Phe Ile His
                245                 250                 255

<210> 6
<211> 267
<212> PRT
<213> Staphylococcus aureus

<400> 6
Met Lys Lys Ile Ala Thr Ala Thr Ile Ala Thr Ala Gly Phe Ala Thr
 1               5                  10                  15
Ile Ala Ile Ala Ser Gly Asn Gln Ala His Ala Ser Glu Gln Asp Asn
            20                  25                  30
Tyr Gly Tyr Asn Pro Asn Asp Pro Thr Ser Tyr Ser Tyr Thr Tyr Thr
        35                  40                  45
Ile Asp Ala Gln Gly Asn Tyr His Tyr Thr Trp Lys Gly Asn Trp His
    50                  55                  60
Pro Ser Gln Leu Asn Gln Asp Asn Gly Tyr Tyr Ser Tyr Tyr Tyr Tyr
65                  70                  75                  80
Asn Gly Tyr Asn Asn Tyr Asn Asn Tyr Asn Asn Gly Tyr Ser Tyr Asn
                85                  90                  95
Asn Tyr Ser Arg Tyr Asn Asn Tyr Ser Asn Asn Asn Gln Ser Tyr Asn
            100                 105                 110
Tyr Asn Asn Tyr Asn Ser Tyr Asn Thr Asn Ser Tyr Arg Thr Gly Gly
        115                 120                 125
Leu Gly Ala Ser Tyr Ser Thr Ser Ser Asn Asn Val Gln Val Thr Thr
    130                 135                 140
Thr Met Ala Pro Ser Ser Asn Gly Arg Ser Ile Ser Ser Gly Tyr Thr
145                 150                 155                 160
Ser Gly Arg Asn Leu Tyr Thr Ser Gly Gln Cys Thr Tyr Tyr Val Phe
                165                 170                 175
Asp Arg Val Gly Gly Lys Ile Gly Ser Thr Trp Gly Asn Ala Ser Asn
            180                 185                 190
Trp Ala Asn Ala Ala Ala Arg Ala Gly Tyr Thr Val Asn Asn Thr Pro
        195                 200                 205
Lys Ala Gly Ala Ile Met Gln Thr Thr Gln Gly Ala Tyr Gly His Val
    210                 215                 220
Ala Tyr Val Glu Ser Val Asn Ser Asn Gly Ser Val Arg Val Ser Glu
225                 230                 235                 240
Met Asn Tyr Gly Tyr Gly Pro Gly Val Val Thr Ser Arg Thr Ile Ser
                245                 250                 255
Ala Ser Gln Ala Ala Gly Tyr Asn Phe Ile His
            260                 265

<210> 7
<211> 257
<212> PRT
<213> Staphylococcus aureus

<400> 7
Met Lys Lys Ile Ala Thr Ala Thr Ile Ala Thr Ala Gly Ile Ala Thr
 1               5                  10                  15
Phe Ala Phe Ala His His Asp Ala Gln Ala Ala Glu Gln Asn Asn Asp
            20                  25                  30
Gly Tyr Asn Pro Asn Asp Pro Tyr Ser Tyr Ser Tyr Thr Tyr Thr Ile
        35                  40                  45

Asp Ala Glu Gly Asn Tyr His Tyr Thr Trp Lys Gly Asn Trp Ser Pro
    50                  55                  60
Asp Arg Val Asn Thr Ser Tyr Asn Tyr Asn Asn Tyr Asn Asn Tyr Asn
65                  70                  75                  80
Tyr Tyr Gly Tyr Asn Asn Tyr Ser Asn Tyr Asn Asn Tyr Ser Asn Tyr
                85                  90                  95
Asn Asn Tyr Asn Asn Tyr Gln Ser Asn Asn Thr Gln Ser Gln Arg Thr
            100                 105                 110
Thr Gln Pro Thr Gly Gly Leu Gly Ala Ser Tyr Ser Thr Ser Ser Ser
        115                 120                 125
Asn Val His Val Thr Thr Thr Ser Ala Pro Ser Ser Asn Gly Val Ser
    130                 135                 140
Leu Ser Asn Ala Arg Ser Ala Ser Gly Asn Leu Tyr Thr Ser Gly Gln
145                 150                 155                 160
Cys Thr Tyr Tyr Val Phe Asp Arg Val Gly Gly Lys Ile Gly Ser Thr
                165                 170                 175
Trp Gly Asn Ala Asn Asn Trp Ala Asn Ala Ala Ala Arg Ser Gly Tyr
            180                 185                 190
Thr Val Asn Asn Ser Pro Ala Lys Gly Ala Ile Leu Gln Thr Ser Gln
        195                 200                 205
Gly Ala Tyr Gly His Val Ala Tyr Val Glu Gly Val Asn Ser Asn Gly
    210                 215                 220
Ser Ile Arg Val Ser Glu Met Asn Tyr Gly His Gly Ala Gly Val Val
225                 230                 235                 240
Thr Ser Arg Thr Ile Ser Ala Ser Gln Ala Ala Ser Tyr Asn Tyr Ile
                245                 250                 255
His

<210> 8
<211> 309
<212> PRT
<213> Staphylococcus aureus

<400> 8
Met Lys Lys Leu Val Pro Leu Leu Leu Ala Leu Leu Leu Leu Val Ala
1               5                   10                  15
Ala Cys Gly Thr Gly Gly Lys Gln Ser Ser Asp Lys Ser Asn Gly Lys
            20                  25                  30
Leu Lys Val Val Thr Thr Asn Ser Ile Leu Tyr Asp Met Ala Lys Asn
        35                  40                  45
Val Gly Gly Asp Asn Val Asp Ile His Ser Ile Val Pro Val Gly Gln
    50                  55                  60
Asp Pro His Glu Tyr Glu Val Lys Pro Lys Asp Ile Lys Lys Leu Thr
65                  70                  75                  80
Asp Ala Asp Val Ile Leu Tyr Asn Gly Leu Asn Leu Glu Thr Gly Asn
                85                  90                  95
Gly Trp Phe Glu Lys Ala Leu Glu Gln Ala Gly Lys Ser Leu Lys Asp
            100                 105                 110
Lys Lys Val Ile Ala Val Ser Lys Asp Val Lys Pro Ile Tyr Leu Asn
        115                 120                 125
Gly Glu Glu Gly Asn Lys Asp Lys Gln Asp Pro His Ala Trp Leu Ser
    130                 135                 140
Leu Asp Asn Gly Ile Lys Tyr Val Lys Thr Ile Gln Gln Thr Phe Ile
145                 150                 155                 160
Asp Asn Asp Lys Lys His Lys Ala Asp Tyr Glu Lys Gln Gly Asn Lys
                165                 170                 175
Tyr Ile Ala Gln Leu Glu Lys Leu Asn Asn Asp Ser Lys Asp Lys Phe
            180                 185                 190
Asn Asp Ile Pro Lys Glu Gln Arg Ala Met Ile Thr Ser Glu Gly Ala
        195                 200                 205
Phe Lys Tyr Phe Ser Lys Gln Tyr Gly Ile Thr Pro Gly Tyr Ile Trp
    210                 215                 220
Glu Ile Asn Thr Glu Lys Gln Gly Thr Pro Glu Gln Met Arg Gln Ala

225                 230                 235                 240
Ile Glu Phe Val Lys Lys His Lys Leu Lys His Leu Leu Val Glu Thr
                245                 250                 255
Ser Val Asp Lys Lys Ala Met Glu Ser Leu Ser Glu Glu Thr Lys Lys
            260                 265                 270
Asp Ile Phe Gly Glu Val Tyr Thr Asp Ser Ile Gly Lys Glu Gly Thr
        275                 280                 285
Lys Gly Asp Ser Tyr Tyr Lys Met Met Lys Ser Asn Ile Glu Thr Val
    290                 295                 300
His Gly Ser Met Lys
305

<210> 9
<211> 309
<212> PRT
<213> Staphylococcus aureus

<400> 9
Met Lys Lys Ile Leu Ala Leu Ala Ile Ala Phe Leu Ile Ile Leu Ala
 1               5                  10                  15
Ala Cys Gly Asn His Ser Asn His Glu His His Ser His Glu Gly Lys
            20                  25                  30
Leu Lys Val Val Thr Thr Asn Ser Ile Leu Tyr Asp Met Val Lys Arg
        35                  40                  45
Val Gly Gly Asn Lys Val Asp Val His Ser Ile Val Pro Val Gly Gln
    50                  55                  60
Asp Pro His Glu Tyr Glu Val Lys Pro Lys Asp Ile Lys Ala Leu Thr
65                  70                  75                  80
Asp Ala Asp Val Val Phe Tyr Asn Gly Leu Asn Leu Glu Thr Gly Asn
                85                  90                  95
Gly Trp Phe Glu Lys Ala Leu Asp Gln Ala Gly Lys Ser Thr Lys Asp
            100                 105                 110
Lys Asn Val Ile Ala Ala Ser Asn Asn Val Lys Pro Ile Tyr Leu Asn
        115                 120                 125
Gly Glu Glu Gly Asn Lys Asn Lys Gln Asp Pro His Ala Trp Leu Ser
    130                 135                 140
Leu Glu Asn Gly Ile Lys Tyr Val Lys Thr Ile Gln Lys Ser Leu Glu
145                 150                 155                 160
His His Asp Lys Lys Asp Lys Ser Thr Tyr Glu Lys Gln Gly Asn Ala
                165                 170                 175
Tyr Ile Ser Lys Leu Glu Glu Leu Asn Lys Asp Ser Lys Asn Lys Phe
            180                 185                 190
Asp Asp Ile Pro Lys Asn Gln Arg Ala Met Met Thr Ser Glu Gly Ala
        195                 200                 205
Phe Lys Tyr Phe Ala Gln Gln Phe Asp Val Lys Pro Gly Tyr Ile Trp
    210                 215                 220
Glu Ile Asn Thr Glu Lys Gln Gly Thr Pro Gly Gln Met Lys Gln Ala
225                 230                 235                 240
Ile Lys Phe Val Lys Asp Asn His Leu Lys His Leu Leu Val Glu Thr
                245                 250                 255
Ser Val Asp Lys Lys Ala Met Gln Ser Leu Ser Glu Glu Thr Lys Lys
            260                 265                 270
Asp Ile Tyr Gly Glu Val Phe Thr Asp Ser Ile Gly Lys Glu Gly Thr
        275                 280                 285
Lys Gly Asp Ser Tyr Tyr Lys Met Met Lys Ser Asn Ile Asp Thr Ile
    290                 295                 300
His Gly Ser Met Lys
305

<210> 10
<211> 233
<212> PRT
<213> Staphylococcus aureus

<400> 10
Met Lys Lys Thr Ile Met Ala Ser Ser Leu Ala Val Ala Leu Gly Val
1               5                   10                  15
Thr Gly Tyr Ala Ala Gly Thr Gly His Gln Ala His Ala Ala Glu Val
            20                  25                  30
Asn Val Asp Gln Ala His Leu Val Asp Leu Ala His Asn His Gln Asp
        35                  40                  45
Gln Leu Asn Ala Ala Pro Ile Lys Asp Gly Ala Tyr Asp Ile His Phe
    50                  55                  60
Val Lys Asp Gly Phe Gln Tyr Asn Phe Thr Ser Asn Gly Thr Thr Trp
65                  70                  75                  80
Ser Trp Ser Tyr Glu Ala Ala Asn Gly Gln Thr Ala Gly Phe Ser Asn
                85                  90                  95
Val Ala Gly Ala Asp Tyr Thr Thr Ser Tyr Asn Gln Gly Ser Asp Val
            100                 105                 110
Gln Ser Val Ser Tyr Asn Ala Gln Ser Ser Asn Ser Asn Val Glu Ala
        115                 120                 125
Val Ser Ala Pro Thr Tyr His Asn Tyr Ser Thr Ser Thr Thr Ser Ser
    130                 135                 140
Ser Val Arg Leu Ser Asn Gly Asn Thr Ala Gly Ala Thr Gly Ser Ser
145                 150                 155                 160
Ala Ala Gln Ile Met Ala Gln Arg Thr Gly Val Ser Ala Ser Thr Trp
                165                 170                 175
Ala Ala Ile Ile Ala Arg Glu Ser Asn Gly Gln Val Asn Ala Tyr Asn
            180                 185                 190
Pro Ser Gly Ala Ser Gly Leu Phe Gln Thr Met Pro Gly Trp Gly Pro
        195                 200                 205
Thr Asn Thr Val Asp Gln Gln Ile Asn Ala Ala Val Lys Ala Tyr Lys
    210                 215                 220
Ala Gln Gly Leu Gly Ala Trp Gly Phe
225                 230

<210> 11
<211> 235
<212> PRT
<213> Staphylococcus aureus

<400> 11
Met Lys Lys Thr Val Ile Ala Ser Thr Leu Ala Val Ser Leu Gly Ile
1               5                   10                  15
Ala Gly Tyr Gly Leu Ser Gly His Glu Ala His Ala Ser Glu Thr Thr
            20                  25                  30
Asn Val Asp Lys Ala His Leu Val Asp Leu Ala Gln His Asn Pro Glu
        35                  40                  45
Glu Leu Asn Ala Lys Pro Val Gln Ala Gly Ala Tyr Asp Ile His Phe
    50                  55                  60
Val Asp Asn Gly Tyr Gln Tyr Asn Phe Thr Ser Asn Gly Ser Glu Trp
65                  70                  75                  80
Ser Trp Ser Tyr Ala Val Ala Gly Ser Asp Ala Asp Tyr Thr Glu Ser
                85                  90                  95
Ser Ser Asn Gln Glu Val Ser Ala Asn Thr Gln Ser Ser Asn Thr Asn
            100                 105                 110
Val Gln Ala Val Ser Ala Pro Thr Ser Ser Glu Ser Arg Ser Tyr Ser
        115                 120                 125
Thr Ser Thr Thr Ser Tyr Ser Ala Pro Ser His Asn Tyr Ser Ser His
    130                 135                 140
Ser Ser Ser Val Arg Leu Ser Asn Gly Asn Thr Ala Gly Ser Val Gly
145                 150                 155                 160
Ser Tyr Ala Ala Ala Gln Met Ala Ala Arg Thr Gly Val Ser Ala Ser
                165                 170                 175
Thr Trp Glu His Ile Ile Ala Arg Glu Ser Asn Gly Gln Leu His Ala
            180                 185                 190
Arg Asn Ala Ser Gly Ala Ala Gly Leu Phe Gln Thr Met Pro Gly Trp

195                 200                 205
Gly Ser Thr Gly Ser Val Asn Asp Gln Ile Asn Ala Ala Tyr Lys Ala
    210                 215                 220

Tyr Lys Ala Gln Gly Leu Ser Ala Trp Gly Met
225                 230                 235


<210> 12
<211> 3890
<212> PRT
<213> Staphylococcus aureus

<400> 12
Met Asn Tyr Arg Asp Lys Ile Gln Lys Phe Ser Ile Arg Lys Tyr Thr
 1               5                  10                  15
Val Gly Thr Phe Ser Thr Val Ile Ala Thr Leu Val Phe Leu Gly Phe
            20                  25                  30
Asn Thr Ser Gln Ala His Ala Ala Glu Thr Asn Gln Pro Ala Ser Val
        35                  40                  45
Val Lys Gln Lys Gln Gln Ser Asn Asn Glu Gln Thr Glu Asn Arg Glu
    50                  55                  60
Ser Gln Val Gln Asn Ser Gln Asn Ser Gln Asn Ser Gln Ser Leu Ser
65                  70                  75                  80
Ala Thr His Glu Asn Glu Gln Pro Asn Asn Ser Gln Ala Asn Leu Val
                85                  90                  95
Asn Gln Lys Val Ala Gln Ser Ser Thr Thr Asn Asp Glu Gln Pro Ala
            100                 105                 110
Ser Gln Asn Val Asn Thr Lys Lys Asp Ser Ala Thr Ala Ala Thr Thr
        115                 120                 125
Gln Pro Asp Lys Glu Glu Ser Lys His Lys Gln Asn Glu Ser Gln Ser
    130                 135                 140
Ala Asn Lys Asn Gly Asn Asp Asn Arg Ala Ala His Val Glu Asn His
145                 150                 155                 160
Glu Ala Asn Val Val Thr Ala Ser Asp Ser Ser Asp Asn Gly Asn Val
                165                 170                 175
Gln His Asp Arg Asn Glu Leu Gln Ala Phe Phe Asp Ala Asn Tyr His
            180                 185                 190
Asp Tyr Arg Phe Ile Asp Arg Glu Asn Ala Asp Ser Gly Thr Phe Asn
        195                 200                 205
Tyr Val Lys Gly Ile Phe Asp Lys Ile Asn Thr Leu Leu Gly Ser Asn
    210                 215                 220
Asp Pro Ile Asn Asn Lys Asp Leu Gln Leu Ala Tyr Lys Glu Leu Glu
225                 230                 235                 240
Gln Ala Val Ala Leu Ile Arg Thr Met Pro Gln Arg Gln Gln Thr Ser
                245                 250                 255
Arg Arg Ser Asn Arg Ile Gln Thr Arg Ser Val Glu Ser Arg Ala Ala
            260                 265                 270
Glu Pro Arg Ser Val Ser Asp Tyr Gln Asn Ala Asn Ser Ser Tyr Tyr
        275                 280                 285
Val Glu Asn Ala Asn Asp Gly Ser Gly Tyr Pro Val Gly Thr Tyr Ile
    290                 295                 300
Asn Ala Ser Ser Lys Gly Ala Pro Tyr Asn Leu Pro Thr Thr Pro Trp
305                 310                 315                 320
Asn Thr Leu Lys Ala Ser Asp Ser Lys Glu Ile Ala Leu Met Thr Ala
                325                 330                 335
Lys Gln Thr Gly Asp Gly Tyr Gln Trp Val Ile Lys Phe Asn Lys Gly
            340                 345                 350
His Ala Pro His Gln Asn Met Ile Phe Trp Phe Ala Leu Pro Ala Asp
        355                 360                 365
Gln Val Pro Val Gly Arg Thr Asp Phe Val Thr Val Asn Ser Asp Gly
    370                 375                 380
Thr Asn Val Gln Trp Ser His Gly Ala Gly Ala Gly Ala Asn Lys Pro
385                 390                 395                 400
Leu Gln Gln Met Trp Glu Tyr Gly Val Asn Asp Pro Asp Arg Ser His

405                 410                 415
Asp Phe Lys Ile Arg Asn Arg Ser Gly Gln Val Ile Tyr Ser Trp Pro
            420                 425                 430
Thr Val His Val Tyr Ser Leu Glu Asp Leu Ser Arg Ala Ser Asp Tyr
        435                 440                 445
Phe Ser Glu Ala Gly Ala Thr Pro Ala Thr Lys Ala Phe Gly Arg Gln
    450                 455                 460
Asn Phe Glu Tyr Ile Asn Gly Gln Lys Pro Ala Glu Ser Pro Gly Val
465                 470                 475                 480
Pro Lys Val Tyr Thr Phe Ile Gly Gln Gly Asp Ala Ser Tyr Thr Ile
                485                 490                 495
Ser Phe Lys Thr Gln Gly Pro Thr Val Asn Lys Leu Tyr Tyr Ala Ala
            500                 505                 510
Gly Gly Arg Ala Leu Glu Tyr Asn Gln Leu Phe Met Tyr Ser Gln Leu
        515                 520                 525
Tyr Val Glu Ser Thr Gln Asp His Gln Gln Arg Leu Asn Gly Leu Arg
    530                 535                 540
Gln Val Val Asn Arg Thr Tyr Arg Ile Gly Thr Thr Lys Arg Val Glu
545                 550                 555                 560
Val Ser Gln Gly Asn Val Gln Thr Lys Lys Val Leu Glu Ser Thr Asn
                565                 570                 575
Leu Asn Ile Asp Asp Phe Val Asp Asp Pro Leu Ser Tyr Val Lys Thr
            580                 585                 590
Pro Ser Asn Lys Val Leu Gly Phe Tyr Pro Thr Asn Ala Asn Thr Asn
        595                 600                 605
Ala Phe Arg Pro Gly Gly Val Gln Glu Leu Asn Glu Tyr Gln Leu Ser
    610                 615                 620
Gln Leu Phe Thr Asp Gln Lys Leu Gln Glu Ala Ala Arg Thr Arg Asn
625                 630                 635                 640
Pro Ile Arg Leu Met Ile Gly Phe Asp Tyr Pro Asp Gly Tyr Gly Asn
                645                 650                 655
Ser Glu Thr Leu Val Pro Val Asn Leu Thr Val Leu Pro Glu Ile Gln
            660                 665                 670
His Asn Ile Lys Phe Phe Lys Asn Asp Asp Thr Gln Asn Ile Ala Glu
        675                 680                 685
Lys Pro Phe Ser Lys Gln Ala Gly His Pro Val Phe Tyr Val Tyr Ala
    690                 695                 700
Gly Asn Gln Gly Asn Ala Ser Val Asn Leu Gly Gly Ser Val Thr Ser
705                 710                 715                 720
Ile Gln Pro Leu Arg Ile Asn Leu Thr Ser Asn Glu Asn Phe Thr Asp
                725                 730                 735
Lys Asp Trp Gln Ile Thr Gly Ile Pro Arg Thr Leu His Ile Glu Asn
            740                 745                 750
Ser Thr Asn Arg Thr Asn Asn Ala Arg Glu Arg Asn Ile Glu Leu Val
        755                 760                 765
Gly Asn Leu Leu Pro Gly Asp Tyr Phe Gly Thr Ile Arg Phe Gly Arg
    770                 775                 780
Lys Glu Gln Leu Phe Glu Ile Arg Val Lys Pro His Thr Pro Thr Ile
785                 790                 795                 800
Thr Thr Thr Ala Glu Gln Leu Arg Gly Thr Ala Leu Gln Lys Val Pro
                805                 810                 815
Val Asn Ile Ser Gly Ile Pro Leu Asp Pro Ser Ala Leu Val Tyr Leu
            820                 825                 830
Val Ala Pro Thr Asn Gln Thr Thr Asn Gly Gly Ser Glu Ala Asp Gln
        835                 840                 845
Ile Pro Ser Gly Tyr Thr Ile Leu Ala Thr Gly Thr Pro Asp Gly Val
    850                 855                 860
His Asn Thr Ile Thr Ile Arg Pro Gln Asp Tyr Val Val Phe Ile Pro
865                 870                 875                 880
Pro Val Gly Lys Gln Ile Arg Ala Val Val Tyr Tyr Asn Lys Val Val
                885                 890                 895
Ala Ser Asn Met Ser Asn Ala Val Thr Ile Leu Pro Asp Asp Ile Pro
            900                 905                 910
Pro Thr Ile Asn Asn Pro Val Gly Ile Asn Ala Lys Tyr Tyr Arg Gly
        915                 920                 925

Asp Glu Val Asn Phe Thr Met Gly Val Ser Asp Arg His Ser Gly Ile
    930                 935                 940
Lys Asn Thr Thr Ile Thr Thr Leu Pro Ser Gly Trp Thr Ser Asn Leu
945                 950                 955                 960
Thr Lys Ser Asp Asn Lys Asn Gly Ser Leu Ala Ile Thr Gly Arg Val
                965                 970                 975
Ser Met Asn Gln Ala Phe Asn Ser Asp Ile Thr Phe Lys Val Ser Ala
            980                 985                 990
Thr Asp Asn Val Asn Asn Thr Thr Asn Asp Ser Gln Ser Lys His Val
        995                 1000                1005
Ser Ile His Val Gly Lys Ile Ser Glu Asp Ala His Pro Ile Val Leu
    1010                1015                1020
Gly Asn Thr Glu Lys Val Val Val Val Asn Pro Thr Ala Val Ser Asn
1025                1030                1035                1040
Asp Glu Lys Gln Ser Ile Ile Thr Ala Phe Met Asn Lys Asn Gln Asn
                1045                1050                1055
Ile Arg Gly Tyr Leu Ala Ser Thr Asp Pro Val Thr Val Asp Asn Asn
            1060                1065                1070
Gly Asn Val Thr Leu His Tyr Arg Asp Gly Ser Ser Thr Thr Leu Asp
        1075                1080                1085
Ala Thr Asn Val Met Thr Tyr Glu Pro Val Val Lys Ser Glu Tyr Gln
    1090                1095                1100
Thr Ala Asn Ala Ala Lys Thr Ala Thr Val Thr Ile Ala Lys Gly Gln
1105                1110                1115                1120
Ser Phe Asn Ile Gly Asp Ile Lys Gln Tyr Phe Thr Leu Ser Asn Gly
                1125                1130                1135
Gln Ala Ile Pro Asn Gly Thr Phe Thr Asn Ile Thr Ser Asp Arg Thr
            1140                1145                1150
Ile Pro Thr Ala Gln Glu Val Ser Gln Met Asn Ala Gly Thr Gln Leu
        1155                1160                1165
Tyr His Ile Val Ala Ser Asn Ala Tyr His Lys Asp Thr Glu Asp Phe
    1170                1175                1180
Tyr Ile Ser Leu Lys Ile Val Asp Val Lys Gln Pro Glu Gly Asp Gln
1185                1190                1195                1200
Arg Val Tyr Arg Thr Ser Thr Tyr Asp Leu Thr Thr Asp Glu Ile Ser
                1205                1210                1215
Lys Val Lys Gln Ala Phe Ile Asn Ala Asn Arg Asp Val Ile Thr Leu
            1220                1225                1230
Ala Glu Gly Asp Ile Ser Val Thr Asn Thr Pro Asn Gly Ala Asn Val
        1235                1240                1245
Ser Thr Ile Thr Val Asn Ile Asn Lys Gly Arg Leu Thr Lys Ser Phe
    1250                1255                1260
Ala Ser Asn Leu Ala Asn Met Asn Phe Leu Arg Trp Val Asn Phe Pro
1265                1270                1275                1280
Gln Asp Tyr Thr Val Thr Trp Thr Asn Ala Lys Ile Ala Asn Arg Pro
                1285                1290                1295
Thr Asp Gly Gly Leu Ser Trp Ser Asp Asp His Lys Ser Leu Ile Tyr
            1300                1305                1310
Arg Tyr Asp Ala Thr Leu Gly Thr Gln Ile Thr Thr Asn Asp Ile Leu
        1315                1320                1325
Thr Met Leu Lys Ala Thr Thr Thr Val Pro Gly Leu Arg Asn Asn Ile
    1330                1335                1340
Thr Gly Asn Glu Lys Ala Gln Ala Glu Ala Gly Gly Arg Pro Asn Tyr
1345                1350                1355                1360
Arg Thr Thr Gly Tyr Ser Gln Ser Asn Ala Thr Thr Asp Gly Gln Arg
                1365                1370                1375
Gln Phe Thr Leu Asn Gly Gln Val Ile Gln Ile Leu Asp Ile Ile Asn
            1380                1385                1390
Pro Ser Asn Gly Tyr Gly Gly Gln Pro Val Thr Asn Ser Asn Thr Arg
        1395                1400                1405
Ala Asn His Ser Asn Ser Thr Val Val Asn Val Asn Glu Pro Ala Ala
    1410                1415                1420
Asn Gly Ala Gly Ala Phe Thr Ile Asp His Val Val Lys Ser Asn Ser
1425                1430                1435                1440
Thr His Asn Ala Ser Asp Ala Val Tyr Lys Ala Gln Leu Tyr Leu Thr

                    1445                    1450                    1455
Pro Tyr Gly Pro Lys Gln Tyr Val Glu His Leu Asn Gln Asn Thr Gly
                1460                    1465                    1470
Asn Thr Thr Asp Ala Ile Asn Ile Tyr Phe Val Pro Ser Asp Leu Val
            1475                    1480                    1485
Asn Pro Thr Ile Ser Val Gly Asn Tyr Thr Asn His Gln Val Phe Ser
        1490                    1495                    1500
Gly Glu Thr Phe Thr Asn Thr Ile Thr Ala Asn Asp Asn Phe Gly Val
1505                    1510                    1515                    1520
Gln Ser Val Thr Val Pro Asn Thr Ser Gln Ile Thr Gly Thr Val Asp
                    1525                    1530                    1535
Asn Asn His Gln His Val Ser Ala Thr Ala Pro Asn Val Thr Ser Ala
                1540                    1545                    1550
Thr Ser Lys Thr Ile Asn Leu Leu Ala Thr Asp Thr Ser Gly Asn Thr
            1555                    1560                    1565
Ala Thr Thr Ser Phe Asn Val Thr Val Lys Pro Leu Arg Asp Lys Tyr
        1570                    1575                    1580
Arg Val Gly Thr Ser Ser Thr Ala Ala Asn Pro Val Arg Ile Ala Asn
1585                    1590                    1595                    1600
Ile Ser Asn Asn Ala Thr Val Ser Gln Ala Asp Gln Thr Thr Ile Ile
                    1605                    1610                    1615
Asn Ser Leu Thr Phe Thr Ser Asn Ala Pro Asn Arg Asn Tyr Ala Thr
                1620                    1625                    1630
Ala Ser Ala Asn Glu Ile Thr Ser Lys Thr Val Ser Asn Val Ser Arg
            1635                    1640                    1645
Thr Gly Asn Asn Ala Asn Val Thr Val Thr Val Thr His Gln Asp Gly
        1650                    1655                    1660
Thr Thr Ser Thr Val Thr Val Pro Val Lys His Val Ile Pro Glu Ile
1665                    1670                    1675                    1680
Val Ala His Ser His Tyr Thr Val Gln Gly Gln Asp Phe Pro Ala Gly
                    1685                    1690                    1695
Asn Gly Ser Ser Ala Ala Asp Tyr Phe Lys Leu Ser Asn Gly Ser Ala
                1700                    1705                    1710
Ile Pro Asp Ala Thr Ile Thr Trp Val Ser Gly Gln Ala Pro Asn Lys
            1715                    1720                    1725
Asp Asn Thr Arg Ile Gly Glu Asp Ile Thr Val Thr Ala His Ile Leu
        1730                    1735                    1740
Ile Asp Gly Glu Thr Thr Pro Ile Thr Lys Thr Ala Thr Tyr Lys Val
1745                    1750                    1755                    1760
Val Arg Thr Val Pro Lys His Val Phe Glu Thr Ala Arg Gly Val Leu
                    1765                    1770                    1775
Tyr Pro Gly Val Ser Asp Met Tyr Asp Ala Lys Gln Tyr Val Lys Pro
                1780                    1785                    1790
Val Asn Asn Ser Trp Ser Thr Asn Ala Gln His Met Asn Phe Gln Phe
            1795                    1800                    1805
Val Gly Thr Tyr Gly Pro Asn Lys Asp Val Val Gly Ile Ser Thr Arg
        1810                    1815                    1820
Leu Ile Arg Val Thr Tyr Asp Asn Arg Gln Thr Glu Asp Leu Thr Ile
1825                    1830                    1835                    1840
Leu Ser Lys Val Lys Pro Asp Pro Pro Arg Ile Asp Ala Asn Ser Val
                    1845                    1850                    1855
Thr Tyr Lys Ala Gly Leu Thr Asn Gln Glu Ile Lys Val Asn Asn Val
                1860                    1865                    1870
Leu Asn Asn Ser Ser Val Lys Leu Phe Lys Ala Asp Asn Thr Pro Leu
            1875                    1880                    1885
Asn Val Thr Asn Ile Thr His Gly Ser Gly Phe Ser Ser Val Val Thr
        1890                    1895                    1900
Val Ser Asp Ala Leu Pro Asn Gly Gly Ile Lys Ala Lys Ser Ser Ile
1905                    1910                    1915                    1920
Ser Met Asn Asn Val Thr Tyr Thr Thr Gln Asp Glu His Gly Gln Val
                    1925                    1930                    1935
Val Thr Val Thr Arg Asn Glu Ser Val Asp Ser Asn Asp Ser Ala Ser
                1940                    1945                    1950
Val Thr Val Thr Pro Gln Leu Gln Ala Thr Thr Glu Gly Ala Val Phe
            1955                    1960                    1965

Ile Lys Gly Gly Asp Gly Phe Asp Phe Gly His Val Glu Arg Phe Ile
    1970                1975                1980
Gln Asn Pro Pro His Gly Ala Thr Val Ala Trp His Asp Ser Pro Asp
1985                1990                1995                2000
Thr Trp Lys Asn Thr Val Gly Asn Thr His Lys Thr Ala Val Val Thr
                2005                2010                2015
Leu Pro Ser Gly Gln Gly Thr Arg Asn Val Glu Val Pro Val Lys Val
            2020                2025                2030
Tyr Pro Val Ala Asn Ala Lys Ala Pro Ser Arg Asp Val Lys Gly Gln
        2035                2040                2045
Asn Leu Thr His Gly Thr Asn Ala Ile Asp Tyr Ile Thr Phe Asp Pro
    2050                2055                2060
Asn Thr Asn Thr Asn Gly Ile Thr Ala Ala Trp Ala Asn Arg Gln Gln
2065                2070                2075                2080
Pro Asn Asn Gln Gln Ala Gly Val Gln His Leu Asn Val Asp Val Thr
                2085                2090                2095
Tyr Pro Gly Ile Ser Ala Ala Lys Arg Val Pro Val Thr Val Asn Val
            2100                2105                2110
Tyr Gln Phe Glu Phe Pro Gln Thr Thr Tyr Thr Thr Thr Val Gly Gly
        2115                2120                2125
Thr Leu Ala Ser Gly Thr Gln Ala Ser Gly Tyr Ala His Met Gln Asn
    2130                2135                2140
Ala Ser Gly Leu Pro Thr Asp Gly Phe Thr Tyr Lys Trp Asn Arg Asp
2145                2150                2155                2160
Thr Thr Gly Thr Asn Asp Ala Asn Trp Ala Ala Met Asn Lys Pro Asn
                2165                2170                2175
Thr Ala Gln Val Val Asn Ala Lys Tyr Asp Val Ile Tyr Asn Gly His
            2180                2185                2190
Thr Phe Ala Thr Ser Leu Pro Ala Lys Phe Val Val Lys Asp Val Gln
        2195                2200                2205
Pro Ala Lys Pro Thr Val Thr Glu Thr Ala Ala Gly Ala Ile Thr Ile
    2210                2215                2220
Ala Pro Gly Ala Asn Gln Thr Val Asn Thr His Ala Gly Asn Val Thr
2225                2230                2235                2240
Thr Tyr Ala Asp Lys Leu Val Ile Lys Arg Asn Gly Asn Val Val Thr
                2245                2250                2255
Thr Phe Thr Arg Arg Asn Asn Thr Ser Pro Trp Val Lys Glu Ala Ser
            2260                2265                2270
Ala Asp Asn Val Thr Gly Ile Val Gly Thr Asn Asn Gly Ile Thr Val
        2275                2280                2285
Ala Ala Gly Thr Phe Asn Pro Ala Asp Thr Ile Gln Val Val Ala Thr
    2290                2295                2300
Gln Gly Ser Gly Glu Thr Ile Ser Asp Glu Gln Arg Ser Asp Asp Phe
2305                2310                2315                2320
Thr Val Val Ala Pro Gln Pro Asn Gln Ala Thr Thr Lys Ile Trp Gln
                2325                2330                2335
Asn Gly His Ile Asp Ile Thr Pro Asn Asn Pro Ser Gly His Leu Ile
            2340                2345                2350
Asn Pro Thr Gln Ala Met Asp Ile Ala Tyr Thr Glu Lys Val Gly Asn
        2355                2360                2365
Gly Ala Glu His Ser Lys Thr Ile Asn Val Val Arg Gly Gln Asn Asn
    2370                2375                2380
Gln Trp Thr Ile Ala Asn Lys Pro Asp Tyr Val Thr Leu Asp Ala Gln
2385                2390                2395                2400
Thr Gly Lys Val Thr Phe Asn Ala Asn Thr Ile Lys Pro Asn Ser Ser
                2405                2410                2415
Ile Thr Ile Thr Pro Lys Ala Gly Thr Gly His Ser Val Ser Ser Asn
            2420                2425                2430
Pro Ser Thr Leu Thr Ala Pro Ala Ala His Thr Val Asn Thr Thr Glu
        2435                2440                2445
Ile Val Lys Asp Tyr Gly Ser Asn Val Thr Ala Ala Glu Ile Asn Asn
    2450                2455                2460
Ala Val Gln Val Ala Asn Lys Arg Thr Ala Thr Ile Lys Asn Gly Thr
2465                2470                2475                2480
Ala Met Pro Thr Asn Leu Ala Gly Gly Ser Thr Thr Thr Ile Pro Val

2485                    2490                    2495
Thr Val Thr Tyr Asn Asp Gly Ser Thr Glu Glu Val Gln Glu Ser Ile
            2500                    2505                    2510
Phe Thr Lys Ala Asp Lys Arg Glu Leu Ile Thr Ala Lys Asn His Leu
        2515                    2520                    2525
Asp Asp Pro Val Ser Thr Glu Gly Lys Lys Pro Gly Thr Ile Thr Gln
    2530                    2535                    2540
Tyr Asn Asn Ala Met His Asn Ala Gln Gln Gln Ile Asn Thr Ala Lys
2545                    2550                    2555                    2560
Thr Glu Ala Gln Gln Val Ile Asn Asn Glu Arg Ala Thr Pro Gln Gln
                2565                    2570                    2575
Val Ser Asp Ala Leu Thr Lys Val Arg Ala Ala Gln Thr Lys Ile Asp
            2580                    2585                    2590
Gln Ala Lys Ala Leu Leu Gln Asn Lys Glu Asp Asn Ser Gln Leu Val
        2595                    2600                    2605
Thr Ser Lys Asn Asn Leu Gln Ser Ser Val Asn Gln Val Pro Ser Thr
    2610                    2615                    2620
Ala Gly Met Thr Gln Gln Ser Ile Asp Asn Tyr Asn Ala Lys Lys Arg
2625                    2630                    2635                    2640
Glu Ala Glu Thr Glu Ile Thr Ala Ala Gln Arg Val Ile Asp Asn Gly
                2645                    2650                    2655
Asp Ala Thr Ala Gln Gln Ile Ser Asp Glu Lys His Arg Val Asp Asn
            2660                    2665                    2670
Ala Leu Thr Ala Leu Asn Gln Ala Lys His Asp Leu Thr Ala Asp Thr
        2675                    2680                    2685
His Ala Leu Glu Gln Ala Val Gln Gln Leu Asn Arg Thr Gly Thr Thr
    2690                    2695                    2700
Thr Gly Lys Lys Pro Ala Ser Ile Thr Ala Tyr Asn Asn Ser Ile Arg
2705                    2710                    2715                    2720
Ala Leu Gln Ser Asp Leu Thr Ser Ala Lys Asn Ser Ala Asn Ala Ile
                2725                    2730                    2735
Ile Gln Lys Pro Ile Arg Thr Val Gln Glu Val Gln Ser Ala Leu Thr
            2740                    2745                    2750
Asn Val Asn Arg Val Asn Glu Arg Leu Thr Gln Ala Ile Asn Gln Leu
        2755                    2760                    2765
Val Pro Leu Ala Asp Asn Ser Ala Leu Arg Thr Ala Lys Thr Lys Leu
    2770                    2775                    2780
Asp Glu Glu Ile Asn Lys Ser Val Thr Thr Asp Gly Met Thr Gln Ser
2785                    2790                    2795                    2800
Ser Ile Gln Ala Tyr Glu Asn Ala Lys Arg Ala Gly Gln Thr Glu Thr
                2805                    2810                    2815
Thr Asn Ala Gln Asn Val Ile Asn Asn Gly Asp Ala Thr Asp Gln Gln
            2820                    2825                    2830
Ile Ala Ala Glu Lys Thr Lys Val Glu Glu Lys Tyr Asn Ser Leu Lys
        2835                    2840                    2845
Gln Ala Ile Ala Gly Leu Thr Pro Asp Leu Ala Pro Leu Gln Thr Ala
    2850                    2855                    2860
Lys Thr Gln Leu Gln Asn Asp Ile Asp Gln Pro Thr Ser Thr Thr Gly
2865                    2870                    2875                    2880
Met Thr Ser Ala Ser Val Ala Ala Phe Asn Asp Lys Leu Ser Ala Ala
                2885                    2890                    2895
Arg Thr Lys Ile Gln Glu Ile Asp Arg Val Leu Ala Ser His Pro Asp
            2900                    2905                    2910
Val Ala Thr Ile Arg Gln Asn Val Thr Ala Ala Asn Ala Ala Lys Thr
        2915                    2920                    2925
Ala Leu Asp Gln Ala Arg Asn Gly Leu Thr Val Asp Lys Ala Pro Leu
    2930                    2935                    2940
Glu Asn Ala Lys Asn Gln Leu Gln His Ser Ile Asp Thr Gln Thr Ser
2945                    2950                    2955                    2960
Thr Thr Gly Met Thr Gln Asp Ser Ile Asn Ala Tyr Asn Ala Lys Leu
                2965                    2970                    2975
Thr Ala Ala Arg Asn Lys Val Gln Gln Ile Asn Gln Val Leu Ala Gly
            2980                    2985                    2990
Ser Pro Thr Val Asp Gln Ile Asn Thr Asn Thr Ser Ala Ala Asn Gln
        2995                    3000                    3005

Ala Lys Ser Asp Leu Asp His Ala Arg Gln Ala Leu Thr Pro Asp Lys
    3010                3015                3020
Ala Pro Leu Gln Asn Ala Lys Thr Gln Leu Glu Gln Ser Ile Asn Gln
3025                3030                3035                3040
Pro Thr Asp Thr Thr Gly Met Thr Thr Ala Ser Leu Asn Ala Tyr Asn
                3045                3050                3055
Gln Lys Leu Gln Ala Ala Arg Gln Lys Leu Thr Glu Ile Asn Gln Val
            3060                3065                3070
Leu Asn Gly Asn Pro Thr Val Gln Asn Ile Asn Asp Lys Val Ala Glu
        3075                3080                3085
Ala Asn Gln Ala Lys Asp Gln Leu Asn Thr Ala Arg Gln Gly Leu Thr
    3090                3095                3100
Leu Asp Arg Gln Pro Ala Leu Thr Thr Leu His Gly Ala Ser Asn Leu
3105                3110                3115                3120
Asn Gln Ala Gln Gln Asn Asn Phe Thr Gln Gln Ile Asn Ala Ala Gln
                3125                3130                3135
Asn His Ala Ala Leu Glu Thr Ile Lys Ser Asn Ile Thr Ala Leu Asn
            3140                3145                3150
Thr Ala Met Thr Lys Leu Lys Asp Ser Val Ala Asp Asn Asn Thr Ile
        3155                3160                3165
Lys Ser Gly Gln Asn Tyr Thr Asp Ala Thr Pro Ala Asn Lys Gln Ala
    3170                3175                3180
Tyr Asp Asn Ala Val Asn Ala Ala Lys Gly Val Ile Gly Glu Thr Thr
3185                3190                3195                3200
Asn Pro Thr Met Asp Val Asn Thr Val Asn Gln Lys Ala Ala Ser Val
                3205                3210                3215
Lys Ser Thr Lys Asp Ala Leu Asp Gly Gln Gln Asn Leu Gln Arg Ala
            3220                3225                3230

Lys Thr Glu Ala Thr Asn Ala Ile Thr His Ala Ser Asp Leu Asn Gln
        3235                3240                3245
Ala Gln Lys Asn Ala Leu Thr Gln Gln Val Asn Ser Ala Gln Asn Val
    3250                3255                3260
Gln Ala Val Asn Asp Ile Lys Gln Thr Thr Gln Ser Leu Asn Thr Ala
3265                3270                3275                3280
Met Thr Gly Leu Lys Arg Gly Val Ala Asn His Asn Gln Val Val Gln
                3285                3290                3295
Ser Asp Asn Tyr Val Asn Ala Asp Thr Asn Lys Lys Asn Asp Tyr Asn
            3300                3305                3310
Asn Ala Tyr Asn His Ala Asn Asp Ile Ile Asn Gly Asn Ala Gln His
        3315                3320                3325
Pro Val Ile Thr Pro Ser Asp Val Asn Asn Ala Leu Ser Asn Val Thr
    3330                3335                3340
Ser Lys Glu His Ala Leu Asn Gly Glu Ala Lys Leu Asn Ala Ala Lys
3345                3350                3355                3360
Gln Glu Ala Asn Thr Ala Leu Gly His Leu Asn Asn Leu Asn Asn Val
                3365                3370                3375
Gln Arg Gln Asn Leu Gln Ser Gln Ile Asn Gly Ala His Gln Ile Asp
            3380                3385                3390
Ala Val Asn Thr Ile Lys Gln Asn Ala Thr Asn Leu Asn Ser Ala Met
        3395                3400                3405
Gly Asn Leu Arg Gln Ala Val Ala Asp Lys Asp Gln Val Lys Arg Thr
    3410                3415                3420
Glu Asp Tyr Ala Asp Ala Asp Thr Ala Lys Gln Asn Ala Tyr Asn Ser
3425                3430                3435                3440
Ala Val Ser Ser Ala Glu Thr Ile Ile Asn Gln Thr Ala Asn Pro Thr
                3445                3450                3455
Met Ser Val Asp Asp Val Asn Arg Ala Thr Ser Ala Val Thr Thr Asn
            3460                3465                3470
Lys Asn Ala Leu Asn Gly Asp Glu Lys Leu Val Gln Ser Lys Thr Asp
        3475                3480                3485
Ala Ala Arg Ala Ile Asp Ala Leu Pro His Leu Asn Asn Ala Gln Lys
    3490                3495                3500
Ala Asp Val Lys Ser Lys Ile Asn Ala Ala Ser Asn Ile Ala Gly Val
3505                3510                3515                3520

Asn Thr Val Lys Gln Gln Gly Thr Asp Leu Asn Thr Ala Met Gly Asn
                3525                3530                3535
Leu Gln Gly Ala Ile Asn Asp Glu Gln Thr Thr Leu Asn Ser Gln Asn
            3540                3545                3550
Tyr Gln Asp Ala Thr Pro Ser Lys Lys Thr Ala Tyr Thr Asn Ala Val
        3555                3560                3565
Gln Ala Ala Lys Asp Ile Leu Asn Lys Ser Asn Gly Gln Asn Lys Thr

    3570                3575                3580
Lys Asp Gln Val Thr Glu Ala Met Asn Gln Val Asn Ser Ala Lys Asn
3585                3590                3595                3600
Asn Leu Asp Gly Thr Arg Leu Leu Asp Gln Ala Lys Gln Thr Ala Lys
                3605                3610                3615
Gln Gln Leu Asn Asn Met Thr His Leu Thr Thr Ala Gln Lys Thr Asn
            3620                3625                3630
Leu Thr Asn Gln Ile Asn Ser Gly Thr Thr Val Ala Gly Val His Thr
        3635                3640                3645
Val Gln Ser Asn Ala Asn Thr Leu Asp Gln Ala Met Asn Thr Leu Arg
    3650                3655                3660
Gln Ser Ile Ala Asn Asn Asp Ala Thr Lys Ala Ser Glu Asp Tyr Val
3665                3670                3675                3680
Asp Ala Asn Asn Asp Lys Gln Thr Ala Tyr Asn Asn Ala Val Ala Ala
                3685                3690                3695
Ala Glu Thr Ile Ile Asn Ala Asn Ser Asn Pro Glu Met Asn Pro Ser
            3700                3705                3710
Thr Ile Thr Gln Lys Ala Glu Gln Val Asn Ser Ser Lys Thr Ala Leu
        3715                3720                3725
Asn Gly Asp Glu Asn Leu Ala Thr Ala Lys Gln Asn Ala Lys Thr Tyr
    3730                3735                3740
Leu Asn Thr Leu Thr Ser Ile Thr Asp Ala Gln Lys Asn Asn Leu Ile
3745                3750                3755                3760
Ser Gln Ile Ser Ser Ala Thr Arg Val Ser Gly Val Asp Thr Val Lys
                3765                3770                3775
Gln Asn Ala Gln His Leu Asp Gln Ala Met Ala Asn Leu Gln Asn Gly
            3780                3785                3790
Ile Asn Asn Glu Ser Gln Val Lys Ser Ser Glu Lys Tyr Arg Asp Ala
        3795                3800                3805
Asp Thr Asn Lys Gln Gln Glu Tyr Asp Asn Ala Ile Thr Ala Ala Lys
    3810                3815                3820
Ala Ile Leu Asn Lys Ser Thr Gly Pro Asn Thr Ala Gln Asn Ala Val
3825                3830                3835                3840
Glu Ala Ala Leu Gln Arg Val Asn Thr Ala Lys Asp Ala Leu Asn Gly
                3845                3850                3855
Asp Ala Lys Leu Ile Ala Ala Gln Asn Ala Ala Lys Gln His Leu Gly
            3860                3865                3870
Thr Leu Thr His Ile Thr Thr Ala Gln Arg Asn Asp Leu Thr Asn Gln
        3875                3880                3885
Ile Ser
    3890



<210> 13
<211> 6713
<212> PRT
<213> Staphylococcus aureus

<400> 13
Met Gly Asn Leu Gln Thr Ala Ile Asn Asp Lys Ser Gly Thr Leu Ala
 1               5                  10                  15
Ser Gln Asn Phe Leu Asp Ala Asp Glu Gln Lys Arg Asn Ala Tyr Asn
            20                  25                  30
Gln Ala Ile Ser Ala Ala Glu Thr Ile Leu Asn Lys Gln Thr Gly Pro
        35                  40                  45
Asn Thr Ala Lys Thr Ala Val Glu Gln Ala Leu Asn Asn Val Asn Ser
    50                  55                  60

Ala Lys His Ala Leu Asn Gly Thr Gln Asn Leu Asn Asn Ala Lys Gln
65                  70                  75                  80
Ala Ala Ile Thr Ala Ile Asn Gly Ala Ser Asp Leu Asn Gln Lys Gln
                85                  90                  95
Lys Asp Ala Leu Lys Ala Gln Ala Asn Gly Ala Gln Arg Val Ser Asn
            100                 105                 110
Ala Asn Asp Val Gln Arg Asn Ala Thr Glu Leu Asn Thr Ala Met Gly
        115                 120                 125
Gln Leu Gln His Ala Ile Ala Asp Lys Thr Asn Thr Leu Ala Ser Ser
    130                 135                 140
Lys Tyr Val Asn Ala Asp Ser Thr Lys Gln Asn Ala Tyr Thr Thr Lys
145                 150                 155                 160
Val Thr Asn Ala Glu His Ile Ile Ser Gly Thr Pro Thr Val Val Thr
                165                 170                 175
Thr Pro Ser Glu Val Thr Ala Ala Ala Asn Gln Val Asn Ser Ala Lys
            180                 185                 190
Gln Glu Leu Asn Gly Asp Glu Arg Leu Arg Val Ala Lys Gln Asn Ala
        195                 200                 205
Asn Thr Ala Ile Asp Ala Leu Thr Gln Leu Asn Thr Pro Gln Lys Ala
    210                 215                 220
Lys Leu Lys Glu Gln Val Gly Gln Ala Asn Arg Leu Glu Asp Val Gln
225                 230                 235                 240
Ser Val Gln Thr Asn Gly Gln Ser Leu Asn Asn Ala Met Lys Gly Leu
                245                 250                 255
Arg Asp Ser Ile Ala Asn Glu Thr Thr Val Lys Ala Ser Gln Asn Tyr
            260                 265                 270
Thr Asp Ala Ser Pro Asn Asn Gln Ser Thr Tyr Asn Ser Ala Val Ser
        275                 280                 285
Asn Ala Lys Gly Ile Ile Asn Gln Thr Asn Asn Pro Thr Met Asp Thr
    290                 295                 300
Ser Ala Ile Thr Gln Ala Thr Thr Gln Val Asn Asn Ala Lys Asn Gly
305                 310                 315                 320
Leu Asn Gly Ala Glu Asn Leu Arg Asn Ala Gln Asn Thr Ala Lys Gln
                325                 330                 335
Asn Leu Asn Thr Leu Ser His Leu Thr Asn Asn Gln Lys Ser Ala Ile
            340                 345                 350
Ser Ser Gln Ile Asp Arg Ala Gly His Val Ser Glu Val Thr Ala Ala
        355                 360                 365
Lys Asn Ala Ala Thr Glu Leu Asn Ala Gln Met Gly Asn Leu Glu Gln
    370                 375                 380
Ala Ile His Asp Gln Asn Thr Val Lys Gln Gly Val Asn Phe Thr Asp
385                 390                 395                 400
Ala Asp Lys Ala Lys Arg Asp Ala Tyr Thr Asn Ala Val Ser Arg Ala
                405                 410                 415
Glu Thr Ile Leu Asn Lys Thr Gln Gly Ala Asn Thr Ser Lys Gln Asp
            420                 425                 430
Val Glu Ala Ala Ile Gln Asn Val Thr Ser Ala Lys Asn Ala Leu Asn
        435                 440                 445
Gly Asp Gln Asn Val Thr Asn Ala Lys Asn Ala Ala Lys Asn Ala Leu
    450                 455                 460
Asn Asn Leu Thr Ser Ile Asn Asn Ala Gln Lys Arg Asp Leu Thr Thr
465                 470                 475                 480
Lys Ile Asp Gln Ala Thr Thr Val Ala Gly Val Glu Ala Val Ser Asn
                485                 490                 495
Thr Gly Thr Gln Leu Asn Thr Ala Met Ala Asn Leu Gln Asn Gly Ile
            500                 505                 510
Asn Asp Lys Ala Asn Thr Leu Ala Ser Glu Asn Tyr His Asp Ala Asp
        515                 520                 525
Ser Asp Lys Lys Thr Ala Tyr Thr Gln Ala Val Thr Asn Ala Glu Asn
    530                 535                 540
Ile Leu Asn Lys Asn Ser Gly Ser Asn Leu Asp Lys Ala Ala Val Glu
545                 550                 555                 560
Asn Ala Leu Ser Gln Val Thr Asn Ala Lys Gly Ala Leu Asn Gly Asn
                565                 570                 575
His Asn Leu Glu Gln Ala Lys Ser Asn Ala Asn Thr Thr Ile Asn Gly

            580                 585                 590
Leu Gln His Leu Thr Thr Ala Gln Lys Asp Lys Leu Lys Gln Gln Val
        595                 600                 605
Gln Gln Ala Gln Asn Val Ala Gly Val Asp Thr Val Lys Ser Ser Ala
    610                 615                 620
Asn Thr Leu Asn Gly Ala Met Gly Thr Leu Arg Asn Ser Ile Gln Asp
625                 630                 635                 640
Asn Thr Ala Thr Lys Asn Gly Gln Asn Tyr Leu Asp Ala Thr Glu Arg
                645                 650                 655
Asn Lys Thr Asn Tyr Asn Asn Ala Val Asp Ser Ala Asn Gly Val Ile
            660                 665                 670
Asn Ala Thr Ser Asn Pro Asn Met Asp Ala Asn Ala Ile Asn Gln Ile
        675                 680                 685
Ala Thr Gln Val Thr Ser Thr Lys Asn Ala Leu Asp Gly Thr His Asn
    690                 695                 700
Leu Thr Gln Ala Lys Gln Thr Ala Thr Asn Ala Ile Asp Gly Ala Thr
705                 710                 715                 720
Asn Leu Asn Lys Ala Gln Lys Asp Ala Leu Lys Ala Gln Val Thr Ser
                725                 730                 735
Ala Gln Arg Val Ala Asn Val Thr Ser Ile Gln Gln Thr Ala Asn Glu
            740                 745                 750
Leu Asn Thr Ala Met Gly Gln Leu Gln His Gly Ile Asp Asp Glu Asn
        755                 760                 765
Ala Thr Lys Gln Thr Gln Lys Tyr Arg Asp Ala Glu Gln Ser Lys Lys
    770                 775                 780
Thr Ala Tyr Asp Gln Ala Val Ala Ala Ala Lys Ala Ile Leu Asn Lys
785                 790                 795                 800
Gln Thr Gly Ser Asn Ser Asp Lys Ala Ala Val Asp Arg Ala Leu Gln
                805                 810                 815
Gln Val Thr Ser Thr Lys Asp Ala Leu Asn Gly Asp Ala Lys Leu Ala
            820                 825                 830
Glu Ala Lys Ala Ala Ala Arg Gln Asn Leu Gly Thr Leu Asn His Ile
        835                 840                 845
Thr Asn Ala Gln Arg Thr Ala Leu Glu Gly Gln Ile Asn Gln Ala Thr
    850                 855                 860
Thr Val Asp Gly Val Asn Thr Val Lys Thr Asn Ala Asn Thr Leu Asp
865                 870                 875                 880
Gly Ala Met Asn Ser Leu Gln Gly Ala Ile Asn Asp Lys Asp Ala Thr
                885                 890                 895
Leu Arg Asn Gln Asn Tyr Leu Asp Ala Asp Glu Ser Lys Arg Asn Ala
            900                 905                 910
Tyr Thr Gln Ala Val Thr Ala Ala Glu Gly Ile Leu Asn Lys Gln Thr
        915                 920                 925
Gly Gly Asn Thr Ser Lys Ala Asp Val Asp Asn Ala Leu Asn Ala Val
    930                 935                 940
Thr Arg Ala Lys Ala Ala Leu Asn Gly Ala Glu Asn Leu Arg Asn Ala
945                 950                 955                 960
Lys Thr Ser Ala Thr Asn Thr Ile Asn Gly Leu Pro Asn Leu Thr Gln
                965                 970                 975
Leu Gln Lys Asp Asn Leu Lys His Gln Val Glu Gln Ala Gln Asn Val
            980                 985                 990
Val Gly Val Asn Gly Val Lys Asp Lys Gly Asn Thr Leu Asn Thr Ala
        995                 1000                1005
Met Gly Ala Leu Arg Thr Ser Ile Gln Asn Asp Asn Thr Thr Lys Thr
    1010                1015                1020
Ser Gln Asn Tyr Leu Asp Ala Ser Asp Ser Asn Lys Asn Asn Tyr Asn
1025                1030                1035                1040
Thr Ala Val Asn Asn Ala Asn Gly Val Ile Asn Ala Thr Asn Asn Pro
                1045                1050                1055
Asn Met Asp Ala Asn Ala Ile Asn Asp Met Ala Asn Gln Val Asn Thr
            1060                1065                1070
Thr Lys Ala Ala Leu Asn Gly Ala Gln Asn Leu Ala Gln Ala Lys Thr
        1075                1080                1085
Asn Ala Thr Asn Thr Ile Asn Asn Ala Gln Asp Leu Asn Gln Lys Gln
    1090                1095                1100

Lys Asp Ala Leu Lys Thr Gln Val Asn Asn Ala Gln Arg Val Ser Asp
1105                1110                1115                1120
Ala Asn Asn Val Gln His Thr Ala Thr Glu Leu Asn Gly Ala Met Thr
                1125                1130                1135
Ala Leu Lys Ala Ala Ile Ala Asp Lys Glu Arg Thr Lys Ala Ser Gly
            1140                1145                1150
Asn Tyr Val Asn Ala Asp Gln Glu Lys Arg Gln Ala Tyr Asp Ser Lys
        1155                1160                1165
Val Thr Asn Ala Glu Asn Ile Ile Asn Gly Thr Pro Asn Ala Thr Leu
    1170                1175                1180
Thr Val Asn Asp Val Asn Ser Ala Ala Ser Gln Val Asn Ala Ala Lys
1185                1190                1195                1200
Thr Ala Leu Asn Gly Asp Asn Asn Leu Arg Val Ala Lys Glu His Ala
                1205                1210                1215
Asn Asn Thr Ile Asp Gly Leu Ala Gln Leu Asn Asn Val Gln Lys Ala
            1220                1225                1230
Lys Leu Lys Glu Gln Val Gln Ser Ala Thr Thr Leu Asp Gly Val Gln
        1235                1240                1245
Thr Val Lys Asn Ser Ser Gln Thr Leu Asn Thr Ala Met Lys Gly Leu
    1250                1255                1260
Arg Asp Ser Ile Ala Asn Glu Ala Thr Ile Lys Ala Gly Gln Asn Tyr
1265                1270                1275                1280
Thr Asp Ala Ser Pro Asn Asn Arg Asn Glu Tyr Asp Ser Ala Val Thr
                1285                1290                1295
Ala Ala Lys Ala Ile Ile Asn Gln Thr Ser Asn Pro Thr Met Glu Pro
            1300                1305                1310
Asn Thr Ile Thr Gln Ala Thr Ser Gln Val Thr Thr Lys Glu His Ala
        1315                1320                1325
Leu Asn Gly Ala Gln Asn Leu Ala Gln Ala Lys Thr Thr Ala Lys Asn
    1330                1335                1340
Asn Leu Asn Asn Leu Thr Ser Ile Asn Asn Ala Gln Lys Asp Ala Leu
1345                1350                1355                1360
Thr Arg Asn Ile Asp Gly Ala Thr Thr Val Ala Gly Val Asn Gln Glu
                1365                1370                1375
Thr Ala Lys Ala Thr Glu Leu Asn Asn Ala Met His Ser Leu Gln Asn
            1380                1385                1390
Gly Ile Asn Asp Glu Thr Gln Thr Lys Gln Thr Gln Lys Tyr Leu Asp
        1395                1400                1405
Ala Glu Pro Ser Lys Lys Ser Ala Tyr Asp Gln Ala Val Asn Ala Ala
    1410                1415                1420
Lys Ala Ile Leu Thr Lys Ala Ser Gly Gln Asn Val Asp Lys Ala Ala
1425                1430                1435                1440
Val Glu Gln Ala Leu Gln Asn Val Asn Ser Thr Lys Thr Ala Leu Asn
                1445                1450                1455
Gly Asp Ala Lys Leu Asn Glu Ala Lys Ala Ala Ala Lys Gln Thr Leu
            1460                1465                1470
Gly Thr Leu Thr His Ile Asn Asn Ala Gln Arg Asn Ala Leu Asp Asn
        1475                1480                1485
Glu Ile Thr Gln Ala Thr Asn Val Glu Gly Val Asn Thr Val Lys Ala
    1490                1495                1500
Lys Ala Gln Gln Leu Asp Gly Ala Met Gly Gln Leu Glu Thr Ser Ile
1505                1510                1515                1520
Arg Asp Lys Asp Thr Thr Leu Gln Ser Gln Asn Tyr Gln Asp Ala Asp
                1525                1530                1535
Asp Ala Lys Arg Thr Ala Tyr Ser Gln Ala Val Asn Ala Ala Ala Thr
            1540                1545                1550
Ile Leu Asn Lys Thr Ala Gly Gly Asn Thr Pro Lys Ala Asp Val Glu
        1555                1560                1565
Arg Ala Met Gln Ala Val Thr Gln Ala Asn Thr Ala Leu Asn Gly Ile
    1570                1575                1580
Gln Asn Leu Glu Arg Ala Lys Gln Ala Ala Asn Thr Ala Ile Thr Asn
1585                1590                1595                1600
Ala Ser Asp Leu Asn Thr Lys Gln Lys Glu Ala Leu Lys Ala Gln Val
                1605                1610                1615
Thr Ser Ala Gly Arg Val Ser Ala Ala Asn Gly Val Glu His Thr Ala

1620                    1625                    1630
Thr Glu Leu Asn Thr Ala Met Thr Ala Leu Lys Arg Ala Ile Ala Asp
        1635                    1640                    1645
Lys Ala Asp Thr Lys Ala Ser Gly Asn Tyr Val Asn Ala Asp Ala Asn
    1650                    1655                    1660
Lys Arg Gln Ala Tyr Asp Glu Lys Val Thr Ala Ala Glu His Ile Val
1665                    1670                    1675                    1680
Ser Gly Thr Pro Thr Pro Thr Leu Thr Pro Ser Asp Val Thr Asn Ala
                1685                    1690                    1695
Ala Thr Gln Val Thr Asn Ala Lys Thr Gln Leu Asn Gly Asn His Asn
            1700                    1705                    1710
Leu Glu Val Ala Lys Gln Asn Ala Asn Thr Ala Ile Asp Gly Leu Thr
        1715                    1720                    1725
Ser Leu Asn Gly Pro Gln Lys Ala Lys Leu Lys Glu Gln Val Gly Gln
    1730                    1735                    1740
Ala Thr Thr Leu Pro Asn Val Gln Thr Val Arg Asp Asn Ala Gln Thr
1745                    1750                    1755                    1760
Leu Asn Thr Ala Met Lys Gly Leu Arg Asp Ser Ile Ala Asn Glu Ala
                1765                    1770                    1775
Thr Ile Lys Ala Gly Gln Asn Tyr Thr Asp Ala Ser Gln Asn Lys Gln
            1780                    1785                    1790
Asn Asp Tyr Asn Asn Ala Val Thr Ala Ala Lys Ala Ile Ile Gly Gln
        1795                    1800                    1805
Thr Thr Ser Pro Ser Met Ile Ala Gln Glu Ile Asn Gln Ala Lys Asp
    1810                    1815                    1820
Gln Val Thr Ala Lys Gln Gln Ala Leu Asn Gly Gln Glu Asn Leu Arg
1825                    1830                    1835                    1840
Thr Ala Gln Thr Asn Ala Lys Gln His Leu Asn Gly Leu Ser Asp Leu
                1845                    1850                    1855
Thr Asn Ala Gln Lys Asp Ala Ala Lys Arg Gln Ile Glu Gly Ala Thr
            1860                    1865                    1870
His Val Asn Glu Val Thr Gln Ala Gln Asn Asn Ala Asp Ala Leu Asn
        1875                    1880                    1885
Thr Ala Met Thr Asn Leu Lys Asn Gly Ile Gln Asp Gln Asn Thr Ile
    1890                    1895                    1900
Lys Gln Gly Val Asn Phe Thr Asp Ala Asp Glu Ala Lys Arg Asn Ala
1905                    1910                    1915                    1920
Tyr Thr Asn Ala Val Thr Gln Ala Glu Gln Ile Leu Asn Lys Ala Gln
                1925                    1930                    1935
Gly Pro Asn Thr Ala Lys Asp Gly Val Glu Thr Ala Leu Gln Asn Val
            1940                    1945                    1950
Gln Arg Ala Lys Asn Glu Leu Asn Gly Asn Gln Asn Val Ala Asn Ala
        1955                    1960                    1965
Lys Thr Thr Ala Lys Asn Ala Leu Asn Asn Leu Thr Ser Ile Asn Asn
    1970                    1975                    1980
Ala Gln Lys Ala Ala Leu Lys Ser Gln Ile Glu Gly Ala Thr Thr Val
1985                    1990                    1995                    2000
Ala Gly Val Asn Gln Val Ser Thr Met Ala Ser Glu Leu Asn Thr Ala
                2005                    2010                    2015
Met Ser Asn Leu Gln Arg Gly Ile Asn Asp Glu Ala Ala Thr Lys Ala
            2020                    2025                    2030
Ala Gln Lys Tyr Thr Glu Ala Asp Arg Asp Lys Gln Thr Ala Tyr Asn
        2035                    2040                    2045
Asp Ala Val Thr Ala Ala Lys Thr Leu Leu Asp Lys Thr Ala Gly Ser
    2050                    2055                    2060
Asn Asp Asn Lys Val Ala Val Glu Gln Ala Leu Gln Arg Val Asn Thr
2065                    2070                    2075                    2080
Ala Lys Thr Ala Leu Asn Gly Asp Ala Arg Leu Asn Glu Ala Lys Asn
                2085                    2090                    2095
Thr Ala Lys Gln Gln Leu Ala Thr Met Ser His Leu Thr Asn Ala Gln
            2100                    2105                    2110
Lys Ala Asn Leu Thr Glu Gln Ile Glu Arg Gly Thr Thr Val Ala Gly
        2115                    2120                    2125
Val Gln Gly Ile Gln Ala Asn Ala Gly Thr Leu Asn Gln Ala Met Asn
    2130                    2135                    2140

Gln Leu Arg Gln Ser Ile Ala Ser Lys Asp Ala Thr Lys Ser Ser Glu
2145                2150                2155                2160
Asp Tyr Gln Asp Ala Asn Ala Asp Leu Gln Asn Ala Tyr Asn Asp Ala
                2165                2170                2175
Val Thr Asn Ala Glu Gly Ile Ile Ser Ala Thr Asn Asn Pro Glu Met
            2180                2185                2190
Asn Pro Asp Thr Ile Asn Gln Lys Ala Ser Gln Val Asn Ser Ala Lys
        2195                2200                2205
Ser Ala Leu Asn Gly Asp Glu Lys Leu Ala Ala Val Lys Gln Thr Ala
    2210                2215                2220
Lys Ser Asp Ile Gly Arg Leu Thr Asp Leu Asn Asn Ala Gln Arg Thr
2225                2230                2235                2240
Ala Ala Asn Ala Glu Val Asp Gln Ala Pro Asn Leu Ala Ala Val Thr
                2245                2250                2255
Ala Ala Lys Asn Lys Ala Thr Ser Leu Asn Thr Ala Met Gly Asn Leu
            2260                2265                2270
Lys His Ala Leu Ala Glu Lys Asp Asn Thr Lys Arg Ser Val Asn Tyr
        2275                2280                2285
Thr Asp Ala Asp Gln Pro Lys Gln Gln Ala Tyr Asp Thr Ala Val Thr
    2290                2295                2300
Gln Ala Glu Ala Ile Thr Asn Ala Asn Gly Ser Asn Ala Asn Glu Thr
2305                2310                2315                2320
Gln Val Gln Ala Ala Leu Asn Gln Leu Asn Gln Ala Lys Asn Asp Leu
                2325                2330                2335
Asn Gly Asp Asn Lys Val Ala Gln Ala Lys Glu Thr Ala Lys Arg Ala
            2340                2345                2350
Leu Ala Ser Tyr Ser Asn Leu Asn Asn Ala Gln Ser Thr Ala Ala Thr
        2355                2360                2365
Ser Gln Ile Asp Asn Ala Thr Thr Val Ala Asp Val Thr Ala Ala Gln
    2370                2375                2380
Asn Thr Ala Asn Glu Leu Asn Thr Ala Met Gly Gln Leu Gln Asn Gly
2385                2390                2395                2400
Ile Asn Asp Gln Asn Thr Val Lys Gln Gln Val Asn Phe Thr Asp Ala
                2405                2410                2415
Asp Gln Gly Lys Lys Asp Ala Tyr Thr Asn Ala Val Thr Asn Ala Gln
            2420                2425                2430
Gly Ile Leu Asp Lys Ala Asn Gly Gln Asn Met Thr Lys Ala Gln Val
        2435                2440                2445
Glu Ala Ala Leu Asn Gln Val Thr Thr Ala Lys Asn Ala Leu Asn Gly
    2450                2455                2460
Asp Ala Asn Val Arg Gln Ala Lys Ser Asp Ala Lys Ala Asn Leu Gly
2465                2470                2475                2480
Thr Leu Thr His Leu Asn Asn Ala Gln Lys Gln Asp Leu Thr Ser Gln
                2485                2490                2495
Ile Glu Gly Ala Thr Thr Val Asn Gly Val Asn Ser Val Lys Thr Lys
            2500                2505                2510
Ala Gln Asp Leu Asp Gly Ala Met Gln Arg Leu Glu Ser Ala Ile Ala
        2515                2520                2525
Asn Lys Asp Gln Thr Lys Ala Ser Glu Asn Tyr Ile Asp Ala Asp Pro
    2530                2535                2540
Thr Lys Lys Thr Ala Phe Asp Asn Ala Ile Thr Gln Ala Glu Ser Tyr
2545                2550                2555                2560
Leu Asn Lys Asp His Gly Thr Asn Lys Asp Lys Gln Ala Val Glu Gln
                2565                2570                2575
Ala Ile Gln Ser Val Thr Ser Thr Glu Asn Ala Leu Asn Gly Asp Ala
            2580                2585                2590
Asn Leu Gln Cys Ala Lys Thr Glu Ala Thr Gln Ala Ile Asp Asn Leu
        2595                2600                2605
Thr Gln Leu Asn Thr Pro Gln Lys Thr Ala Leu Lys Gln Gln Val Asn
    2610                2615                2620
Ala Ala Gln Arg Val Ser Gly Val Thr Asp Leu Lys Asn Ser Ala Thr
2625                2630                2635                2640
Ser Leu Asn Asn Ala Met Asp Gln Leu Lys Gln Ala Ile Gly Asp His
                2645                2650                2655
Asp Thr Ile Val Ala Gly Gly Asn Tyr Thr Asn Ala Ser Pro Asp Lys

2660                 2665                 2670
Gln Gly Ala Tyr Thr Asp Ala Tyr Asn Ala Ala Lys Asn Ile Val Asn
        2675                 2680                 2685
Gly Ser Pro Asn Val Ile Thr Asn Ala Ala Asp Val Thr Ala Ala Thr
    2690                 2695                 2700
Gln Arg Val Asn Asn Ala Glu Thr Ser Leu Asn Gly Asp Thr Asn Leu
2705                 2710                 2715                 2720
Ala Thr Ala Lys Gln Gln Ala Lys Asp Ala Leu Arg Gln Met Thr His
                2725                 2730                 2735
Leu Ser Asp Ala Gln Lys Gln Ser Ile Thr Gly Gln Ile Asp Ser Ala
            2740                 2745                 2750
Thr Gln Val Thr Gly Val Gln Ser Val Lys Asp Asn Ala Thr Asn Leu
        2755                 2760                 2765
Asp Asn Ala Met Asn Gln Leu Arg Asn Ser Ile Ala Asn Lys Asp Glu
    2770                 2775                 2780
Val Lys Ala Ser Gln Pro Tyr Val Asp Ala Asp Thr Asp Lys Gln Asn
2785                 2790                 2795                 2800
Ala Tyr Asn Thr Ala Val Thr Ser Ala Glu Asn Ile Ile Asn Ala Thr
                2805                 2810                 2815
Ser Gln Pro Thr Leu Asp Pro Ser Ala Val Thr Gln Ala Ala Asn Gln
            2820                 2825                 2830
Val Asn Thr Asn Lys Thr Ala Leu Asn Gly Ala Gln Asn Leu Ala Asn
        2835                 2840                 2845
Lys Lys Gln Glu Thr Thr Ala Asn Ile Asn Arg Leu Ser His Leu Asn
    2850                 2855                 2860
Asn Ala Gln Lys Gln Asp Leu Asn Thr Gln Val Thr Asn Ala Pro Asn
2865                 2870                 2875                 2880
Ile Ser Thr Val Asn Gln Val Lys Thr Lys Ala Glu Gln Leu Asp Gln
                2885                 2890                 2895
Ala Met Glu Arg Leu Ile Asn Gly Ile Gln Asp Lys Asp Gln Val Lys
            2900                 2905                 2910
Gln Ser Val Asn Phe Thr Asp Ala Asp Pro Glu Lys Gln Thr Ala Tyr
        2915                 2920                 2925
Asn Asn Ala Val Thr Ala Ala Glu Asn Ile Ile Asn Gln Ala Asn Gly
    2930                 2935                 2940
Thr Asn Ala Asn Gln Ser Gln Val Glu Ala Ala Leu Ser Thr Val Thr
2945                 2950                 2955                 2960

Thr Thr Lys Gln Ala Leu Asn Gly Asp Arg Lys Val Thr Asp Ala Lys
                2965                 2970                 2975
Asn Asn Ala Asn Gln Thr Leu Ser Thr Leu Asp Asn Leu Asn Asn Ala
            2980                 2985                 2990
Gln Lys Gly Ala Val Thr Gly Asn Ile Asn Gln Ala His Thr Val Ala
        2995                 3000                 3005
Glu Val Thr Gln Ala Ile Gln Thr Ala Gln Glu Leu Asn Thr Ala Met
    3010                 3015                 3020
Gly Asn Leu Lys Asn Ser Leu Asn Asp Lys Asp Thr Thr Leu Gly Ser
3025                 3030                 3035                 3040
Gln Asn Phe Ala Asp Ala Asp Pro Glu Lys Lys Asn Ala Tyr Asn Glu
                3045                 3050                 3055
Ala Val Arg Asn Ala Glu Asn Ile Leu Asn Lys Ser Thr Gly Thr Asn
            3060                 3065                 3070
Val Pro Lys Asp Gln Val Glu Ala Ala Met Asn Gln Val Asn Thr Thr
        3075                 3080                 3085
Lys Ala Ala Leu Asn Gly Thr Gln Asn Leu Glu Lys Ala Lys Gln His
    3090                 3095                 3100
Ala Asn Thr Ala Ile Asp Gly Leu Ser His Leu Thr Asn Ala Gln Lys
3105                 3110                 3115                 3120
Glu Ala Leu Lys Gln Leu Val Gln Gln Ser Thr Thr Val Ala Glu Ala
                3125                 3130                 3135
Gln Gly Asn Glu Gln Lys Ala Asn Asn Val Asp Ala Ala Met Asp Lys
            3140                 3145                 3150
Leu Arg Gln Ser Ile Ala Asp Asn Ala Thr Thr Lys Gln Asn Gln Asn
        3155                 3160                 3165
Tyr Thr Asp Ala Ser Pro Asn Lys Lys Asp Ala Tyr Asn Asn Ala Val

3170                3175                3180
Thr Thr Ala Gln Gly Ile Ile Asp Gln Thr Thr Asn Pro Ser Leu Asp
3185                3190                3195                3200
Pro Thr Val Ile Asn Gln Ala Ala Gly Gln Val Ser Thr Ser Lys Asn
                3205                3210                3215
Ala Leu Asn Gly Asn Glu Asn Leu Glu Ala Ala Lys Gln Gln Ala Thr
            3220                3225                3230
Gln Ser Leu Gly Ser Leu Asp Asn Leu Asn Asn Ala Gln Lys Gln Ala
        3235                3240                3245
Val Thr Asn Gln Ile Asn Gly Ala His Thr Val Asp Glu Ala Asn Gln
    3250                3255                3260
Ile Lys Gln Asn Ala Gln Asn Leu Asn Thr Ala Met Gly Asn Leu Lys
3265                3270                3275                3280
Gln Ala Ile Ala Asp Lys Asp Ala Thr Lys Ala Thr Val Asn Phe Thr
                3285                3290                3295
Asp Ala Asp Gln Ala Lys Gln Gln Ala Tyr Asn Thr Ala Val Thr Asn

            3300                3305                3310
Ala Glu Asn Ile Ile Ser Lys Ala Asn Gly Gly Asn Ala Thr Gln Thr
        3315                3320                3325
Glu Val Glu Gln Ala Ile Gln Gln Val Asn Ala Ala Lys Gln Ala Leu
    3330                3335                3340
Asn Gly Asn Ala Asn Val Gln His Ala Lys Asp Glu Ala Thr Ala Leu
3345                3350                3355                3360
Ile Asn Asn Ser Asn Asp Leu Asn Gln Ala Gln Lys Asp Ala Leu Lys
                3365                3370                3375
Gln Gln Val Gln Asn Ala Thr Thr Val Ala Gly Val Asn Asn Val Lys
            3380                3385                3390
Gln Thr Ala Gln Glu Leu Asn Asn Ala Met Thr Gln Leu Lys Gln Gly
        3395                3400                3405
Ile Ala Asp Lys Glu Gln Thr Lys Ala Asp Gly Asn Phe Val Asn Ala
    3410                3415                3420
Asp Ser Asp Lys Gln Asn Ala Tyr Asn Gln Ala Val Ala Lys Ala Glu
3425                3430                3435                3440
Ala Leu Ile Ser Gly Thr Pro Asp Val Val Val Thr Pro Ser Glu Ile
                3445                3450                3455
Thr Ala Ala Leu Asn Lys Val Thr Gln Ala Lys Asn Asp Leu Asn Gly
            3460                3465                3470
Asn Thr Asn Leu Ala Thr Ala Lys Gln Asn Val Gln His Ala Ile Asp
        3475                3480                3485
Gln Leu Pro Asn Leu Asn Gln Ala Gln Arg Asp Glu Tyr Ser Lys Gln
    3490                3495                3500
Ile Thr Gln Ala Thr Leu Val Pro Asn Val Asn Ala Ile Gln Gln Ala
3505                3510                3515                3520
Ala Thr Thr Leu Asn Asp Ala Met Thr Gln Leu Lys Gln Gly Ile Ala
                3525                3530                3535
Asn Lys Ala Gln Ile Lys Gly Ser Glu Asn Tyr His Asp Ala Asp Thr
            3540                3545                3550
Asp Lys Gln Thr Ala Tyr Asp Asn Ala Val Thr Lys Ala Glu Glu Leu
        3555                3560                3565
Leu Lys Gln Thr Thr Asn Pro Thr Met Asp Pro Asn Thr Ile Gln Gln
    3570                3575                3580
Ala Leu Thr Lys Val Asn Asp Thr Asn Gln Ala Leu Asn Gly Asn Gln
3585                3590                3595                3600
Lys Leu Ala Asp Ala Lys Gln Asp Ala Lys Thr Thr Leu Gly Thr Leu
                3605                3610                3615
Asp His Leu Asn Asp Ala Gln Lys Gln Ala Leu Thr Thr Gln Val Glu
            3620                3625                3630
Gln Ala Pro Asp Ile Ala Thr Val Asn Asn Val Lys Gln Asn Ala Gln
        3635                3640                3645
Asn Leu Asn Asn Ala Met Thr Asn Leu Asn Asn Ala Leu Gln Asp Lys
    3650                3655                3660
Thr Glu Thr Leu Asn Ser Ile Asn Phe Thr Asp Ala Asp Gln Ala Lys
3665                3670                3675                3680
Lys Asp Asp Tyr Thr Asn Ala Val Ser His Ala Glu Gly Ile Leu Ser

3685                    3690                    3695
Lys Ala Asn Gly Ser Asn Ala Ser Gln Thr Glu Val Glu Gln Ala Met
            3700                    3705                    3710
Gln Arg Val Asn Glu Ala Lys Gln Ala Leu Asn Gly Asn Asp Asn Val
        3715                    3720                    3725
Gln Arg Ala Lys Asp Ala Ala Lys Gln Val Ile Thr Asn Ala Asn Asp
    3730                    3735                    3740
Leu Asn Gln Ala Gln Lys Asp Ala Leu Lys Gln Gln Val Asp Ala Ala
3745                    3750                    3755                    3760
Gln Thr Val Ala Asn Val Asn Thr Ile Lys Gln Thr Ala Gln Asp Leu
                3765                    3770                    3775
Asn Gln Ala Met Thr Gln Leu Lys Gln Gly Ile Ala Asp Lys Asp Gln
            3780                    3785                    3790
Thr Lys Ala Asn Gly Asn Phe Val Asn Ala Asp Thr Asp Lys Gln Asn
        3795                    3800                    3805
Ala Tyr Asn Asn Ala Val Ala His Ala Glu Gln Ile Ile Ser Gly Thr
    3810                    3815                    3820
Pro Asn Ala Asn Val Asp Pro Gln Gln Val Ala Gln Ala Leu Gln Gln
3825                    3830                    3835                    3840
Val Asn Gln Ala Lys Gly Asp Leu Asn Gly Asn His Asn Leu Gln Val
                3845                    3850                    3855
Ala Lys Asp Asn Ala Asn Thr Ala Ile Asp Gln Leu Pro Asn Leu Asn
            3860                    3865                    3870
Gln Pro Gln Lys Thr Ala Leu Lys Asp Gln Val Ser His Ala Glu Leu
        3875                    3880                    3885
Val Thr Gly Val Asn Ala Ile Lys Gln Asn Ala Asp Ala Leu Asn Asn
    3890                    3895                    3900
Ala Met Gly Thr Leu Lys Gln Gln Ile Gln Ala Asn Ser Gln Val Pro
3905                    3910                    3915                    3920
Gln Ser Val Asp Phe Thr Gln Ala Asp Gln Asp Lys Gln Gln Ala Tyr
                3925                    3930                    3935
Asn Asn Ala Ala Asn Gln Ala Gln Gln Ile Ala Asn Gly Thr Pro Thr
            3940                    3945                    3950
Pro Val Leu Ala Pro Asp Thr Val Thr Lys Ala Val Thr Thr Met Asn
        3955                    3960                    3965
Gln Ala Lys Asp Ala Leu Asn Gly Asp Glu Lys Leu Ala Gln Ala Lys
    3970                    3975                    3980
Gln Asp Ala Leu Ala Asn Leu Asp Thr Leu Arg Asp Leu Asn Gln Pro
3985                    3990                    3995                    4000
Gln Arg Asp Ala Leu Arg Asn Gln Ile Asn Gln Ala Gln Ala Leu Ala
                4005                    4010                    4015
Thr Val Glu Gln Thr Lys Gln Asn Ala Gln Asn Val Asn Thr Ala Met
            4020                    4025                    4030
Gly Asn Leu Lys Gln Gly Ile Ala Asn Lys Asp Thr Val Lys Ala Ser
        4035                    4040                    4045
Glu Asn Tyr His Asp Ala Asp Val Asp Lys Gln Thr Ala Tyr Thr Asn
    4050                    4055                    4060
Ala Val Ser Gln Ala Glu Gly Ile Ile Asn Gln Thr Thr Asn Pro Thr
4065                    4070                    4075                    4080
Leu Asn Pro Asp Asp Ile Thr Arg Ala Leu Thr Gln Val Thr Asp Ala
                4085                    4090                    4095
Lys Asn Ser Leu Asn Gly Glu Ala Lys Leu Ala Thr Glu Lys Gln Asn
            4100                    4105                    4110
Ala Lys Asp Ala Val Ser Gly Met Thr His Leu Asn Asp Ala Gln Lys
        4115                    4120                    4125
Gln Ala Leu Lys Gly Gln Ile Asp Gln Ser Pro Glu Ile Ala Thr Val
    4130                    4135                    4140
Asn Gln Val Lys Gln Thr Ala Thr Ser Leu Asp Gln Ala Met Asp Gln
4145                    4150                    4155                    4160
Leu Ser Gln Ala Ile Asn Asp Lys Asp Gln Ile Leu Ala Asp Gly Asn
                4165                    4170                    4175
Tyr Leu Asn Ala Asp Pro Asp Lys Gln Asn Ala Tyr Lys Gln Ala Val
            4180                    4185                    4190
Ala Lys Ala Glu Ala Leu Leu Asn Lys Gln Ser Gly Thr Asn Glu Val
        4195                    4200                    4205

Gln Ala Gln Val Glu Ser Ile Thr Asn Glu Val Asn Ala Ala Lys Gln
    4210                4215                4220
Ala Leu Asn Gly Asn Asp Asn Leu Ala Asn Ala Lys Gln Gln Ala Lys
4225                4230                4235                4240
Gln Gln Leu Ala Asn Leu Thr His Leu Asn Asp Ala Gln Lys Gln Ser
                4245                4250                4255
Phe Glu Ser Gln Ile Thr Gln Ala Pro Leu Val Thr Asp Val Thr Thr
            4260                4265                4270
Ile Asn Gln Lys Ala Gln Thr Leu Asp His Ala Met Glu Leu Leu Arg
        4275                4280                4285
Asn Ser Val Ala Asp Asn Gln Thr Thr Leu Ala Ser Glu Asp Tyr His
    4290                4295                4300
Asp Ala Thr Ala Gln Arg Gln Asn Asp Tyr Asn Lys Ala Val Thr Ala
4305                4310                4315                4320
Ala Asn Asn Ile Ile Asn Gln Thr Thr Ser Pro Thr Met Asn Pro Asp
                4325                4330                4335
Asp Val Asn Gly Ala Thr Thr Gln Val Asn Asn Thr Lys Val Ala Leu
            4340                4345                4350
Asp Gly Asp Glu Asn Leu Ala Ala Ala Lys Gln Gln Ala Asn Asn Arg
        4355                4360                4365
Leu Asp Gln Leu Asp His Leu Asn Asn Ala Gln Lys Gln Gln Leu Gln
    4370                4375                4380
Ser Gln Ile Thr Gln Ser Ser Asp Ile Ala Ala Val Asn Gly His Lys
4385                4390                4395                4400
Gln Thr Ala Glu Ser Leu Asn Thr Ala Met Gly Asn Leu Ile Asn Ala
                4405                4410                4415
Ile Ala Asp His Gln Ala Val Glu Gln Arg Gly Asn Phe Ile Asn Ala
            4420                4425                4430
Asp Thr Asp Lys Gln Thr Ala Tyr Asn Thr Ala Val Asn Glu Ala Ala
        4435                4440                4445
Ala Met Ile Asn Lys Gln Thr Gly Gln Asn Ala Asn Gln Thr Glu Val
    4450                4455                4460
Glu Gln Ala Ile Thr Lys Val Gln Thr Thr Leu Gln Ala Leu Asn Gly
4465                4470                4475                4480
Asp His Asn Leu Gln Val Ala Lys Thr Asn Ala Thr Gln Ala Ile Asp
                4485                4490                4495
Val Leu Thr Ser Leu Asn Asp Pro Gln Lys Thr Ala Leu Lys Asp Gln
            4500                4505                4510
Val Thr Ala Ala Thr Leu Val Thr Ala Val His Gln Ile Glu Gln Asn
        4515                4520                4525
Ala Asn Thr Leu Asn Gln Ala Met His Gly Leu Arg Gln Ser Ile Gln
    4530                4535                4540
Asp Asn Ala Ala Thr Lys Ala Asn Ser Lys Tyr Ile Asn Glu Asp Gln
4545                4550                4555                4560
Pro Glu Gln Gln Asn Tyr Asp Gln Ala Val Gln Ala Ala Asn Asn Ile
                4565                4570                4575
Ile Asn Glu Gln Thr Ala Thr Leu Asp Asn Asn Ala Ile Asn Gln Val
            4580                4585                4590
Ala Ala Thr Val Asn Thr Thr Lys Ala Ala Leu His Gly Asp Val Lys
        4595                4600                4605
Leu Gln Asn Asp Lys Asp His Ala Lys Gln Thr Val Ser Gln Leu Ala
    4610                4615                4620
His Leu Asn Asn Ala Gln Lys His Met Glu Asp Thr Leu Ile Asp Ser
4625                4630                4635                4640
Glu Thr Thr Arg Thr Ala Val Lys Gln Asp Leu Thr Glu Val Gln Ala
                4645                4650                4655
Leu Asp Gln Leu Met Asp Ala Leu Gln Gln Ser Ile Ala Asp Lys Asp
            4660                4665                4670
Ala Thr Arg Ala Ser Ser Ala Tyr Val Asn Ala Glu Pro Asn Lys Lys
        4675                4680                4685
Gln Ala Tyr Asp Glu Ala Val Gln Asn Ala Glu Ser Ile Ile Ala Gly
    4690                4695                4700
Leu Asn Asn Pro Thr Ile Asn Lys Gly Asn Val Ser Ser Ala Thr Gln
4705                4710                4715                4720
Ala Val Ile Ser Ser Lys Asn Ala Leu Asp Gly Val Glu Arg Leu Ala

4725                4730                4735
Gln Asp Lys Gln Thr Ala Gly Asn Ser Leu Asn His Leu Asp Gln Leu
            4740                4745                4750
Thr Pro Ala Gln Gln Gln Ala Leu Glu Asn Gln Ile Asn Asn Ala Thr
        4755                4760                4765
Thr Cys Asp Lys Val Ala Glu Ile Ile Ala Gln Ala Gln Ala Leu Asn
    4770                4775                4780
Glu Ala Met Lys Ala Leu Lys Glu Ser Ile Lys Asp Gln Pro Gln Thr
4785                4790                4795                4800
Glu Ala Ser Ser Lys Phe Ile Asn Glu Asp Gln Ala Gln Lys Asp Ala
                4805                4810                4815
Tyr Thr Gln Ala Val Gln His Ala Lys Asp Leu Ile Asn Lys Thr Thr
            4820                4825                4830
Asp Pro Thr Leu Ala Lys Ser Ile Ile Asp Gln Ala Thr Gln Ala Val
        4835                4840                4845
Thr Asp Ala Lys Asn Asn Leu His Gly Asp Gln Lys Leu Ala Gln Asp
    4850                4855                4860
Lys Gln Arg Ala Thr Glu Thr Leu Asn Asn Leu Ser Asn Leu Asn Thr
4865                4870                4875                4880
Pro Gln Arg Gln Ala Leu Glu Asn Gln Ile Asn Asn Ala Ala Thr Arg
                4885                4890                4895
Gly Glu Val Ala Gln Lys Leu Thr Glu Ala Gln Ala Leu Asn Gln Ala
            4900                4905                4910
Met Glu Ala Leu Arg Asn Ser Ile Gln Asp Gln Gln Gln Thr Glu Ser
        4915                4920                4925
Gly Ser Lys Phe Ile Asn Glu Asp Lys Pro Gln Lys Asp Ala Tyr Gln
    4930                4935                4940
Ala Ala Val Gln Asn Ala Lys Asp Leu Ile Asn Gln Thr Gly Asn Pro
4945                4950                4955                4960
Thr Leu Asp Lys Ala Gln Val Glu Gln Leu Thr His Ala Phe Lys Gln
                4965                4970                4975
Ala Lys Asp Asn Leu His Gly Asp Gln Lys Leu Ala Asp Asp Lys Gln
            4980                4985                4990
His Ala Val Thr Asp Leu Asn Gln Leu Asn Gly Leu Asn Asn Pro Gln
        4995                5000                5005
Arg Gln Ala Leu Glu Ser Gln Ile Asn Asn Ala Ala Thr Arg Gly Glu
    5010                5015                5020
Val Ala Gln Lys Leu Ala Glu Ala Lys Ala Leu Asp Gln Ala Met Gln
5025                5030                5035                5040
Ala Leu Arg Asn Ser Ile Gln Asp Gln Gln Gln Thr Glu Ala Gly Ser
                5045                5050                5055
Lys Phe Ile Asn Glu Asp Lys Pro Gln Lys Asp Ala Tyr Gln Ala Ala
            5060                5065                5070
Val Gln Asn Ala Lys Asp Leu Ile Asn Gln Thr Gly Asn Pro Thr Leu
        5075                5080                5085
Asp Lys Ser Gln Val Glu Gln Leu Thr Gln Ala Val Thr Thr Ala Lys
    5090                5095                5100
Asp Asn Leu His Gly Asp Gln Lys Leu Ala Arg Asp Gln Gln Gln Ala
5105                5110                5115                5120
Val Thr Thr Val Asn Ala Leu Pro Asn Leu Asn His Ala Gln Gln Gln
                5125                5130                5135
Thr Leu Thr Asp Ala Ile Asn Ala Ala Pro Thr Arg Thr Glu Val Ala
            5140                5145                5150
Gln His Val Gln Thr Ala Thr Glu Leu Asp His Ala Met Glu Thr Leu
        5155                5160                5165
Lys Asn Lys Val Asp Gln Val Asn Thr Asp Lys Ala Gln Pro Asn Tyr
    5170                5175                5180
Thr Glu Ala Ser Thr Asp Lys Lys Glu Ala Val Asp Gln Ala Leu Gln
5185                5190                5195                5200
Ala Ala Gln Ser Ile Thr Asp Pro Thr Asn Gly Ser Asn Ala Asn Lys
                5205                5210                5215
Asp Ala Val Glu Gln Ala Leu Thr Lys Leu Gln Glu Lys Val Asn Glu
            5220                5225                5230
Leu Asn Gly Asn Glu Arg Val Ala Glu Ala Lys Thr Gln Ala Lys Gln
        5235                5240                5245

Thr Ile Asp Gln Leu Thr His Leu Asn Ala Asp Gln Ile Ala Thr Ala
    5250                5255                5260
Lys Gln Asn Ile Asp Gln Ala Thr Lys Leu Gln Pro Ile Ala Glu Leu
5265                5270                5275                5280
Val Asp Gln Ala Thr Gln Leu Asn Gln Ser Met Asp Gln Leu Gln Gln
                5285                5290                5295
Ala Val Asn Glu His Ala Asn Val Glu Gln Thr Ile Asp Tyr Thr Gln
            5300                5305                5310
Ala Asp Ser Asp Lys Gln Lys Ala Tyr Lys Gln Ala Ile Ala Asp Ala
        5315                5320                5325
Glu Asn Val Leu Lys Gln Asn Ala Asn Lys Gln Gln Val Asp Gln Ala
    5330                5335                5340
Leu Gln Asn Ile Leu Asn Ala Lys Gln Ala Leu Asn Gly Asp Glu Arg
5345                5350                5355                5360
Val Ala Leu Ala Lys Thr Asn Gly Lys His Asp Ile Asp Gln Leu Asn
                5365                5370                5375
Ala Leu Asn Asn Ala Gln Gln Asp Gly Phe Lys Gly Arg Ile Asp Gln
            5380                5385                5390
Ser Asn Asp Leu Asn Gln Ile Gln Gln Ile Val Asp Glu Ala Lys Ala
        5395                5400                5405
Leu Asn Arg Ala Met Asp Gln Leu Ser Gln Glu Ile Thr Gly Asn Glu
    5410                5415                5420
Gly Arg Thr Lys Gly Ser Thr Asn Tyr Val Asn Ala Asp Thr Gln Val
5425                5430                5435                5440
Lys Gln Val Tyr Asp Glu Ala Val Asp Lys Ala Lys Gln Ala Leu Asp
                5445                5450                5455
Lys Ser Ser Gly Gln Asn Leu Thr Ala Glu Gln Val Ile Lys Leu Asn
            5460                5465                5470
Asp Ala Val Thr Ala Ala Lys Lys Ala Leu Asn Gly Glu Glu Arg Leu
        5475                5480                5485
Asn Asn Arg Lys Ala Glu Ala Leu Gln Arg Leu Asp Gln Leu Thr His
    5490                5495                5500
Leu Asn Asn Ala Gln Arg Gln Leu Ala Ile Gln Gln Ile Asn Asn Ala
5505                5510                5515                5520
Glu Thr Leu Asn Lys Ala Ser Arg Ala Ile Asn Arg Ala Thr Lys Leu
                5525                5530                5535
Asp Asn Ala Met Gly Ala Val Gln Gln Tyr Ile Asp Glu Gln His Leu
            5540                5545                5550
Gly Val Ile Ser Ser Thr Asn Tyr Ile Asn Ala Asp Asp Asn Leu Lys
        5555                5560                5565
Ala Asn Tyr Asp Asn Ala Ile Ala Asn Ala Ala His Glu Leu Asp Lys
    5570                5575                5580
Val Gln Gly Asn Ala Ile Ala Lys Ala Glu Ala Glu Gln Leu Lys Gln
5585                5590                5595                5600
Asn Ile Ile Asp Ala Gln Asn Ala Leu Asn Gly Asp Gln Asn Leu Ala
                5605                5610                5615
Asn Ala Lys Asp Lys Ala Asn Ala Phe Val Asn Ser Leu Asn Gly Leu
            5620                5625                5630
Asn Gln Gln Gln Gln Asp Leu Ala His Lys Ala Ile Asn Asn Ala Asp
        5635                5640                5645
Thr Val Ser Asp Val Thr Asp Ile Val Asn Asn Gln Ile Asp Leu Asn
    5650                5655                5660
Asp Ala Met Glu Thr Leu Lys His Leu Val Asp Asn Glu Ile Pro Asn
5665                5670                5675                5680
Ala Glu Gln Thr Val Asn Tyr Gln Asn Ala Asp Asp Asn Ala Lys Thr
                5685                5690                5695
Asn Phe Asp Asp Ala Lys Arg Leu Ala Asn Thr Leu Leu Asn Ser Asp
            5700                5705                5710
Asn Thr Asn Val Asn Asp Ile Asn Gly Ala Ile Gln Ala Val Asn Asp
        5715                5720                5725
Ala Ile His Asn Leu Asn Gly Asp Gln Arg Leu Gln Asp Ala Lys Asp
    5730                5735                5740
Lys Ala Ile Gln Ser Ile Asn Gln Ala Leu Ala Asn Lys Leu Lys Glu
5745                5750                5755                5760
Ile Glu Ala Ser Asn Ala Thr Asp Gln Asp Lys Leu Ile Ala Lys Asn

5765                5770                5775
Lys Ala Glu Glu Leu Ala Asn Ser Ile Ile Asn Asn Ile Asn Lys Ala
            5780                5785                5790
Thr Ser Asn Gln Ala Val Ser Gln Val Gln Thr Ala Gly Asn His Ala
        5795                5800                5805
Ile Glu Gln Val His Ala Asn Glu Ile Pro Lys Ala Lys Ile Asp Ala
    5810                5815                5820
Asn Lys Asp Val Asp Lys Gln Val Gln Ala Leu Ile Asp Glu Ile Asp
5825                5830                5835                5840
Arg Asn Pro Asn Leu Thr Asp Lys Glu Lys Gln Ala Leu Lys Asp Arg
                5845                5850                5855
Ile Asn Gln Ile Leu Gln Gln Gly His Asn Asp Ile Asn Asn Ala Leu
            5860                5865                5870
Thr Lys Glu Glu Ile Glu Gln Ala Lys Ala Gln Leu Ala Gln Ala Leu
        5875                5880                5885
Gln Asp Ile Lys Asp Leu Val Lys Ala Lys Glu Asp Ala Lys Gln Asp
    5890                5895                5900
Val Asp Lys Gln Val Gln Ala Leu Ile Asp Glu Ile Asp Gln Asn Pro
5905                5910                5915                5920
Asn Leu Thr Asp Lys Glu Lys Gln Ala Leu Lys Asp Arg Ile Asn Gln
                5925                5930                5935
Ile Leu Gln Gln Gly His Asn Gly Ile Asn Asn Ala Met Thr Lys Glu
            5940                5945                5950
Glu Ile Glu Gln Ala Lys Ala Gln Leu Ala Gln Ala Leu Lys Glu Ile
        5955                5960                5965
Lys Asp Leu Val Lys Ala Lys Glu Asn Ala Lys Gln Asp Val Asp Lys
    5970                5975                5980
Gln Val Gln Ala Leu Ile Asp Glu Ile Asp Gln Asn Pro Asn Leu Thr
5985                5990                5995                6000
Asp Lys Glu Lys Gln Ala Leu Lys Asp Arg Ile Asn Gln Ile Leu Gln
                6005                6010                6015
Gln Gly His Asn Asp Ile Asn Asn Ala Met Thr Lys Glu Glu Ile Glu
            6020                6025                6030
Gln Ala Lys Ala Gln Leu Ala Gln Ala Leu Gln Asp Ile Lys Asp Leu
        6035                6040                6045
Val Lys Ala Lys Glu Asp Ala Lys Asn Ala Ile Lys Ala Leu Ala Asn
    6050                6055                6060
Ala Lys Arg Asp Gln Ile Asn Ser Asn Pro Asp Leu Thr Pro Glu Gln
6065                6070                6075                6080
Lys Ala Lys Ala Leu Lys Glu Ile Asp Glu Ala Glu Lys Arg Ala Leu
                6085                6090                6095
Gln Asn Val Glu Asn Ala Gln Thr Ile Asp Gln Leu Asn Arg Gly Leu
            6100                6105                6110
Asn Leu Gly Leu Asp Asp Ile Arg Asn Thr His Val Trp Glu Val Asp
        6115                6120                6125
Glu Gln Pro Ala Val Asn Glu Ile Phe Glu Ala Thr Pro Glu Gln Ile
    6130                6135                6140
Leu Val Asn Gly Glu Leu Ile Val His Arg Asp Asp Ile Ile Thr Glu
6145                6150                6155                6160
Gln Asp Ile Leu Ala His Ile Asn Leu Ile Asp Gln Leu Ser Ala Glu
                6165                6170                6175
Val Ile Asp Thr Pro Ser Thr Ala Thr Ile Ser Asp Ser Leu Thr Ala
            6180                6185                6190
Lys Val Glu Val Thr Leu Leu Asp Gly Ser Lys Val Ile Val Asn Val
        6195                6200                6205
Pro Val Lys Val Val Glu Lys Glu Leu Ser Val Val Lys Gln Gln Ala
    6210                6215                6220
Ile Glu Ser Ile Glu Asn Ala Ala Gln Gln Lys Ile Asp Glu Ile Asn
6225                6230                6235                6240
Asn Ser Val Thr Leu Thr Leu Glu Gln Lys Glu Ala Ala Ile Ala Glu
                6245                6250                6255
Val Asn Lys Leu Lys Gln Gln Ala Ile Asp His Val Asn Asn Ala Pro
            6260                6265                6270
Asp Val His Ser Val Glu Glu Ile Gln Gln Gln Glu Gln Ala Tyr Ile
        6275                6280                6285

Glu Gln Phe Asn Pro Glu Gln Phe Thr Ile Glu Gln Ala Lys Ser Asn
    6290                6295                6300
Ala Ile Lys Ser Ile Glu Asp Ala Ile Gln His Met Ile Asp Glu Ile
6305                6310                6315                6320
Lys Ala Arg Thr Asp Leu Thr Asp Lys Glu Lys Gln Glu Ala Ile Ala
                6325                6330                6335
Lys Leu Asn Gln Leu Lys Glu Gln Ala Ile Gln Ala Ile Gln Arg Ala
            6340                6345                6350
Gln Ser Ile Ser Glu Ile Thr Glu Gln Leu Glu Gln Phe Lys Ala Gln
        6355                6360                6365
Met Lys Ala Ala Asn Pro Thr Ala Lys Glu Leu Ala Lys Arg Lys Gln
    6370                6375                6380
Glu Ala Ile Ser Arg Ile Lys Asp Phe Ser Asn Glu Lys Ile Asn Ser
6385                6390                6395                6400
Ile Arg Asn Ser Glu Ile Gly Thr Ala Asp Glu Lys Gln Ala Ala Met
                6405                6410                6415
Asn Gln Ile Asn Glu Ile Val Leu Glu Thr Ile Arg Asp Ile Asn Asn
            6420                6425                6430
Ala His Thr Leu Gln Gln Val Glu Ala Ala Leu Asn Asn Gly Ile Ala
        6435                6440                6445
Arg Ile Ser Ala Val Gln Ile Val Ile Ser Asp Arg Ala Lys Gln Ser
    6450                6455                6460
Ser Ser Thr Gly Asn Glu Ser Asn Ser His Leu Thr Ile Gly Tyr Gly
6465                6470                6475                6480
Thr Ala Asn His Pro Phe Asn Ser Ser Thr Ile Gly His Lys Lys Lys
                6485                6490                6495
Leu Asp Glu Asp Asp Asp Ile Asp Pro Leu His Met Arg His Phe Ser
            6500                6505                6510
Asn Asn Phe Gly Asn Val Ile Lys Asn Ala Ile Gly Val Val Gly Ile
        6515                6520                6525
Ser Gly Leu Leu Ala Ser Phe Trp Phe Phe Ile Ala Lys Arg Arg Arg
    6530                6535                6540
Lys Glu Asp Glu Glu Glu Glu Leu Glu Ile Arg Asp Asn Asn Lys Asp
6545                6550                6555                6560
Ser Ile Lys Glu Thr Leu Asp Asp Thr Lys His Leu Pro Leu Leu Phe
                6565                6570                6575
Ala Lys Arg Arg Arg Lys Glu Asp Glu Glu Asp Val Thr Val Glu Glu
            6580                6585                6590
Lys Asp Ser Leu Asn Asn Gly Glu Ser Leu Asp Lys Val Lys His Thr
        6595                6600                6605
Pro Phe Phe Leu Pro Lys Arg Arg Arg Lys Glu Asp Glu Glu Asp Val
    6610                6615                6620
Glu Val Thr Asn Glu Asn Thr Asp Glu Lys Val Leu Lys Asp Asn Glu
6625                6630                6635                6640
His Ser Pro Leu Leu Phe Ala Lys Arg Arg Lys Asp Lys Glu Glu Asp
                6645                6650                6655
Val Glu Thr Thr Thr Ser Ile Glu Ser Lys Asp Glu Asp Val Pro Leu
            6660                6665                6670
Leu Leu Ala Lys Lys Lys Asn Gln Lys Asp Asn Gln Ser Lys Asp Lys
        6675                6680                6685
Lys Ser Ala Ser Lys Asn Thr Ser Lys Lys Val Ala Ala Lys Lys Lys
    6690                6695                6700
Lys Lys Lys Ser Lys Lys Asn Lys Lys
6705                6710

<210> 14
<211> 701
<212> PRT
<213> Staphylococcus aureus

<400> 14
   Met Asn Asn Arg Asp Lys Leu Gln Lys Phe Ser Ile Arg Lys Tyr Ala
   1                   5                   10                  15
   Ile Gly Thr Phe Ser Thr Val Ile Ala Thr Leu Val Phe Met Gly Ile
               20                  25                  30
   Asn Thr Asn His Ala Ser Ala Asp Glu Leu Asn Gln Asn Gln Lys Leu
           35                  40                  45
   Ile Lys Gln Leu Asn Gln Thr Asp Asp Asp Asp Ser Asn Thr His Ser
       50                  55                  60
   Gln Glu Ile Glu Asn Asn Lys Gln Asn Ser Ser Gly Gln Thr Glu Ser
   65                  70                  75                  80
   Leu Arg Ser Ser Thr Ser Gln Asn Gln Ala Asn Ala Arg Leu Ser Asp
                   85                  90                  95
   Gln Phe Lys Asp Thr Asn Glu Thr Ser Gln Gln Leu Pro Thr Asn Val
               100                 105                 110
   Ser Asp Asp Ser Ile Asn Gln Ser His Ser Glu Ala Asn Met Asn Asn
           115                 120                 125
   Glu Pro Leu Lys Val Asp Asn Ser Thr Met Gln Ala His Ser Lys Ile
       130                 135                 140
   Val Ser Asp Ser Asp Gly Asn Ala Ser Glu Asn Lys His His Lys Leu
   145                 150                 155                 160
   Thr Glu Asn Val Leu Ala Glu Ser Arg Ala Ser Lys Asn Asp Lys Glu
                   165                 170                 175
   Lys Glu Asn Leu Gln Glu Lys Asp Lys Ser Gln Gln Val His Pro Pro
               180                 185                 190
   Leu Asp Lys Asn Ala Leu Gln Ala Phe Phe Asp Ala Ser Tyr His Asn
           195                 200                 205
   Tyr Arg Met Ile Asp Arg Asp Arg Ala Asp Ala Thr Glu Tyr Gln Lys
       210                 215                 220
   Val Lys Ser Thr Phe Asp Tyr Val Asn Asp Leu Leu Gly Asn Asn Gln
   225                 230                 235                 240
   Asn Ile Pro Ser Glu Gln Leu Val Ser Ala Tyr Gln Gln Leu Glu Lys
                   245                 250                 255
   Ala Leu Glu Leu Ala Arg Thr Leu Pro Gln Gln Ser Thr Thr Glu Lys
               260                 265                 270
   Arg Gly Arg Arg Ser Thr Arg Ser Val Val Glu Asn Arg Ser Ser Arg
           275                 280                 285
   Ser Asp Tyr Leu Asp Ala Arg Thr Glu Tyr Tyr Val Ser Lys Asp Asp
       290                 295                 300
   Asp Asp Ser Gly Phe Pro Pro Gly Thr Phe Phe His Ala Ser Asn Arg
   305                 310                 315                 320
   Arg Trp Pro Tyr Asn Leu Pro Arg Ser Arg Asn Ile Leu Arg Ala Ser
                   325                 330                 335
   Asp Val Gln Gly Asn Ala Tyr Ile Thr Thr Lys Arg Leu Lys Asp Gly
               340                 345                 350
   Tyr Gln Trp Asp Ile Leu Phe Asn Ser Asn His Lys Gly His Glu Tyr
           355                 360                 365
   Met Tyr Tyr Trp Phe Gly Leu Pro Ser Asp Gln Thr Pro Thr Gly Pro
       370                 375                 380
   Val Thr Phe Thr Ile Ile Asn Arg Asp Gly Ser Ser Thr Ser Thr Gly
   385                 390                 395                 400
   Gly Val Gly Phe Gly Ser Gly Ala Pro Leu Pro Gln Phe Trp Arg Ser
                   405                 410                 415
   Ala Gly Ala Ile Asn Ser Ser Val Ala Asn Asp Phe Lys His Gly Ser
               420                 425                 430
   Ala Thr Asn Tyr Ala Phe Tyr Asp Gly Val Asn Asn Phe Ser Asp Phe
           435                 440                 445
   Ala Arg Gly Gly Glu Leu Tyr Phe Asp Arg Glu Gly Ala Thr Gln Thr
       450                 455                 460
   Asn Lys Tyr Tyr Gly Asp Glu Asn Phe Ala Leu Leu Asn Ser Glu Lys
   465                 470                 475                 480
   Pro Asp Gln Ile Arg Gly Leu Asp Thr Ile Tyr Ser Phe Lys Gly Ser
                   485                 490                 495
   Gly Asp Val Ser Tyr Arg Ile Ser Phe Lys Thr Gln Gly Ala Pro Thr
               500                 505                 510
   Ala Arg Leu Tyr Tyr Ala Ala Gly Ala Arg Ser Gly Glu Tyr Lys Gln
           515                 520                 525
   Ala Thr Asn Tyr Asn Gln Leu Tyr Val Glu Pro Tyr Lys Asn Tyr Arg
       530                 535                 540
   Asn Arg Val Gln Ser Asn Val Gln Val Lys Asn Arg Thr Leu His Leu
   545                 550                 555                 560
   Lys Arg Thr Ile Arg Gln Phe Asp Pro Thr Leu Gln Arg Thr Thr Asp
                   565                 570                 575
   Val Pro Ile Leu Asp Ser Asp Gly Ser Gly Ser Ile Asp Ser Val Tyr
               580                 585                 590
   Asp Pro Leu Ser Tyr Val Lys Asn Val Thr Gly Thr Val Leu Gly Ile
           595                 600                 605
   Tyr Pro Ser Tyr Leu Pro Tyr Asn Gln Glu Arg Trp Gln Gly Ala Asn
       610                 615                 620
   Ala Met Asn Ala Tyr Gln Ile Glu Glu Leu Phe Ser Gln Glu Asn Leu
   625                 630                 635                 640
   Gln Asn Ala Ala Arg Ser Gly Arg Pro Ile Gln Phe Leu Val Gly Phe
                   645                 650                 655
   Asp Val Glu Asp Ser His His Asn Pro Glu Thr Leu Leu Pro Val Asn
               660                 665                 670
   Leu Tyr Val Lys Pro Glu Leu Lys His Thr Ile Glu Leu Tyr His Asp
           675                 680                 685
   Asn Glu Lys Gln Asp Arg Lys Glu Phe Ser Val Ser Lys
       690                 695                 700

<210> 15
<211> 9439
<212> PRT
<213> Staphylococcus aureus

<400> 15
Met Ser Gly Thr Leu His Asn Thr Val Gly Ser Gly Ile Leu Pro Tyr
1               5                   10                  15
Gln Gln Glu Ile Arg Ile Lys Leu Thr Ser Asn Glu Pro Ile Lys Asp
            20                  25                  30
Ser Glu Trp Ser Ile Thr Gly Tyr Pro Asn Thr Leu Thr Leu Gln Asn
        35                  40                  45
Ala Val Gly Arg Thr Asn Asn Ala Thr Glu Lys Asn Leu Ala Leu Val
    50                  55                  60
Gly His Ile Asp Pro Gly Asn Tyr Phe Ile Thr Val Lys Phe Gly Asp
65                  70                  75                  80
Lys Val Glu Gln Phe Glu Ile Arg Ser Lys Pro Thr Pro Pro Arg Ile
                85                  90                  95
Ile Thr Thr Ala Asn Glu Leu Arg Gly Asn Pro Asn His Lys Pro Glu
            100                 105                 110
Ile Arg Val Thr Asp Ile Pro Asn Asp Thr Thr Ala Lys Ile Lys Leu
        115                 120                 125
Val Met Gly Gly Thr Asp Gly Asp His Asp Pro Glu Ile Asn Pro Tyr
    130                 135                 140
Thr Val Pro Glu Asn Tyr Thr Val Val Ala Glu Ala Tyr His Asp Asn
145                 150                 155                 160
Asp Pro Ser Lys Asn Gly Val Leu Thr Phe Arg Ser Ser Asp Tyr Leu
                165                 170                 175
Lys Asp Leu Pro Leu Ser Gly Glu Leu Lys Ala Ile Val Tyr Tyr Asn
            180                 185                 190
Gln Tyr Val Gln Ser Asn Phe Ser Lys Ser Val Pro Phe Ser Ser Asp
        195                 200                 205
Thr Thr Pro Pro Thr Ile Asn Glu Pro Ala Gly Leu Val His Lys Tyr
    210                 215                 220
Tyr Arg Gly Asp His Val Glu Ile Thr Leu Pro Val Thr Asp Asn Thr
225                 230                 235                 240
Gly Gly Ser Gly Leu Arg Asp Val Asn Val Asn Leu Pro Gln Gly Trp
                245                 250                 255
Thr Lys Thr Phe Thr Ile Asn Pro Asn Asn Asn Thr Glu Gly Thr Leu
            260                 265                 270
Lys Leu Ile Gly Asn Ile Pro Ser Asn Glu Ala Tyr Asn Thr Thr Tyr

275                 280                 285
His Phe Asn Ile Thr Ala Thr Asp Asn Ser Gly Asn Thr Thr Asn Pro
    290                 295                 300
Ala Lys Thr Phe Ile Leu Asn Val Gly Lys Leu Ala Asp Asp Leu Asn
305                 310                 315                 320
Pro Val Gly Leu Ser Arg Asp Gln Leu Gln Leu Val Thr Asp Pro Ser
                325                 330                 335
Ser Leu Ser Asn Ser Glu Arg Glu Glu Val Lys Arg Lys Ile Ser Glu
            340                 345                 350
Ala Asn Ala Asn Ile Arg Ser Tyr Leu Leu Gln Asn Asn Pro Ile Leu
        355                 360                 365
Ala Gly Val Asn Gly Asp Val Thr Phe Tyr Tyr Arg Asp Gly Ser Val
    370                 375                 380
Asp Val Ile Asp Ala Glu Asn Val Ile Thr Tyr Glu Pro Glu Arg Lys
385                 390                 395                 400
Ser Ile Phe Ser Glu Asn Gly Asn Thr Asn Lys Lys Glu Ala Val Ile
                405                 410                 415
Thr Ile Ala Arg Gly Gln Asn Tyr Thr Ile Gly Pro Asn Leu Arg Lys
            420                 425                 430
Tyr Phe Ser Leu Ser Asn Gly Ser Asp Leu Pro Asn Arg Asp Phe Thr
        435                 440                 445
Ser Ile Ser Ala Ile Gly Ser Leu Pro Ser Ser Ser Glu Ile Ser Arg
    450                 455                 460
Leu Asn Val Gly Asn Tyr Asn Tyr Arg Val Asn Ala Lys Asn Ala Tyr
465                 470                 475                 480
His Lys Thr Gln Gln Glu Leu Asn Leu Lys Leu Lys Ile Val Glu Val
                485                 490                 495
Asn Ala Pro Thr Gly Asn Asn Arg Val Tyr Arg Val Ser Thr Tyr Asn
            500                 505                 510
Leu Thr Asn Asp Glu Ile Asn Lys Ile Lys Gln Ala Phe Lys Ala Ala
        515                 520                 525
Asn Ser Gly Leu Asn Leu Asn Asp Asn Asp Ile Thr Val Ser Asn Asn
    530                 535                 540
Phe Asp His Arg Asn Val Ser Ser Val Thr Val Thr Ile Arg Lys Gly
545                 550                 555                 560
Asp Leu Ile Lys Glu Phe Ser Ser Asn Leu Asn Asn Met Asn Phe Leu
                565                 570                 575
Arg Trp Val Asn Ile Arg Asp Asp Tyr Thr Ile Ser Trp Thr Ser Ser
            580                 585                 590
Lys Ile Gln Gly Arg Asn Thr Asp Gly Gly Leu Glu Trp Ser Pro Asp
        595                 600                 605
His Lys Ser Leu Ile Tyr Lys Tyr Asp Ala Thr Leu Gly Arg Gln Ile
    610                 615                 620
Asn Thr Asn Asp Val Leu Thr Leu Leu Gln Ala Thr Ala Lys Asn Ser
625                 630                 635                 640
Asn Leu Arg Ser Asn Ile Asn Ser Asn Glu Lys Gln Leu Ala Glu Arg
                645                 650                 655
Gly Ser Asn Gly Tyr Ser Lys Ser Ile Ile Arg Asp Asp Gly Glu Lys
            660                 665                 670
Ser Tyr Leu Leu Asn Ser Asn Pro Ile Gln Val Leu Asp Leu Val Glu
        675                 680                 685
Pro Asp Asn Gly Tyr Gly Gly Arg Gln Val Ser His Ser Asn Val Ile
    690                 695                 700
Tyr Asn Glu Lys Asn Ser Ser Ile Val Asn Gly Gln Val Pro Glu Ala
705                 710                 715                 720
Asn Gly Ala Ser Ala Phe Asn Ile Asp Lys Val Val Lys Ala Asn Ala
                725                 730                 735
Ala Asn Asn Gly Ile Met Gly Val Ile Tyr Lys Ala Gln Leu Tyr Leu
            740                 745                 750
Ala Pro Tyr Ser Pro Lys Gly Tyr Ile Glu Lys Leu Gly Gln Asn Leu
        755                 760                 765
Ser Asn Thr Asn Asn Val Ile Asn Val Tyr Phe Val Pro Ser Asp Lys
    770                 775                 780
Val Asn Pro Ser Ile Thr Val Gly Asn Tyr Asp His His Thr Val Tyr
785                 790                 795                 800

Ser Gly Glu Thr Phe Lys Asn Thr Ile Asn Val Asn Asp Asn Tyr Gly
                805                 810                 815
Leu Asn Thr Val Ala Ser Thr Ser Asp Ser Ala Ile Thr Met Thr Arg
            820                 825                 830
Asn Asn Asn Glu Leu Val Gly Gln Ala Pro Asn Val Thr Asn Ser Ile
        835                 840                 845
Asn Lys Ile Val Lys Val Lys Ala Thr Asp Lys Ser Gly Asn Glu Ser
    850                 855                 860
Ile Val Ser Phe Thr Val Asn Ile Lys Pro Leu Asn Glu Lys Tyr Arg
865                 870                 875                 880
Ile Thr Thr Ser Ser Ser Asn Gln Thr Pro Val Arg Ile Ser Asn Ile
                885                 890                 895
Gln Asn Asn Ala Asn Leu Ser Ile Glu Asp Gln Asn Arg Val Lys Ser
            900                 905                 910
Ser Leu Ser Met Thr Lys Ile Leu Gly Thr Arg Asn Tyr Val Asn Glu
        915                 920                 925
Ser Asn Asn Asp Val Arg Ser Gln Val Val Ser Lys Val Asn Arg Ser
    930                 935                 940
Gly Asn Asn Ala Thr Val Asn Val Thr Thr Thr Phe Ser Asp Gly Thr
945                 950                 955                 960
Thr Asn Thr Ile Thr Val Pro Val Lys His Val Leu Leu Glu Val Val
                965                 970                 975
Pro Thr Thr Arg Thr Thr Val Arg Gly Gln Gln Phe Pro Thr Gly Lys
            980                 985                 990
Gly Thr Ser Pro Asn Asp Phe Phe Ser Leu Arg Thr Gly Gly Pro Val
        995                 1000                1005
Asp Ala Arg Ile Val Trp Val Asn Asn Gln Gly Pro Asp Ile Asn Ser
    1010                1015                1020
Asn Gln Ile Gly Arg Asp Leu Thr Leu His Ala Glu Ile Phe Phe Asp
1025                1030                1035                1040
Gly Glu Thr Thr Pro Ile Arg Lys Asp Thr Thr Tyr Lys Leu Ser Gln
                1045                1050                1055
Ser Ile Pro Lys Gln Ile Tyr Glu Thr Thr Ile Asn Gly Arg Phe Asn
            1060                1065                1070
Ser Ser Gly Asp Ala Tyr Pro Gly Asn Phe Val Gln Ala Val Asn Gln
        1075                1080                1085
Tyr Trp Pro Glu His Met Asp Phe Arg Trp Ala Gln Gly Ser Gly Thr
    1090                1095                1100
Pro Ser Ser Arg Asn Ala Gly Ser Phe Thr Lys Thr Val Thr Val Val
1105                1110                1115                1120
Tyr Gln Asn Gly Gln Thr Glu Asn Val Asn Val Leu Phe Lys Val Lys
                1125                1130                1135
Pro Asn Lys Pro Val Ile Asp Ser Asn Ser Val Ile Ser Lys Gly Gln
            1140                1145                1150
Leu Asn Gly Gln Gln Ile Leu Val Arg Asn Val Pro Gln Asn Ala Gln
        1155                1160                1165
Val Thr Leu Tyr Gln Ser Asn Gly Thr Val Ile Pro Asn Thr Asn Thr
    1170                1175                1180
Thr Ile Asp Ser Asn Gly Ile Ala Thr Val Thr Ile Gln Gly Thr Leu
1185                1190                1195                1200
Pro Thr Gly Asn Ile Thr Ala Lys Thr Ser Met Thr Asn Asn Val Thr
                1205                1210                1215
Tyr Thr Lys Gln Asn Ser Ser Gly Ile Ala Ser Asn Thr Thr Glu Asp
            1220                1225                1230
Ile Ser Val Phe Ser Glu Asn Ser Asp Gln Val Asn Val Thr Ala Gly
        1235                1240                1245
Met Gln Ala Lys Asn Asp Gly Ile Lys Ile Ile Lys Gly Thr Asn Tyr
    1250                1255                1260
Asn Phe Asn Asp Phe Asn Ser Phe Ile Ser Asn Ile Pro Ala His Ser
1265                1270                1275                1280
Thr Leu Thr Trp Asn Glu Glu Pro Asn Ser Trp Lys Asn Asn Ile Gly
                1285                1290                1295
Thr Thr Thr Lys Thr Val Thr Val Thr Leu Pro Asn His Gln Gly Thr
            1300                1305                1310
Arg Thr Val Asp Ile Pro Ile Thr Ile Tyr Pro Thr Val Thr Ala Lys

1315                1320                1325
Asn Pro Val Arg Asp Gln Lys Gly Arg Asn Leu Thr Asn Gly Thr Asp
    1330                1335                1340

Val Tyr Asn Tyr Ile Ile Phe Glu Asn Asn Asn Arg Leu Gly Gly Thr
1345                1350                1355                1360
Ala Ser Trp Lys Asp Asn Arg Gln Pro Asp Lys Asn Ile Ala Gly Val
                1365                1370                1375
Gln Asn Leu Ile Ala Leu Val Asn Tyr Pro Gly Ile Ser Thr Pro Leu
            1380                1385                1390
Glu Val Pro Val Lys Val Trp Val Tyr Asn Phe Asp Phe Thr Gln Pro
        1395                1400                1405
Ile Tyr Lys Ile Gln Val Gly Asp Thr Phe Pro Lys Gly Thr Trp Ala
    1410                1415                1420
Gly Tyr Tyr Lys His Leu Glu Asn Gly Glu Gly Leu Pro Ile Asp Gly
1425                1430                1435                1440
Trp Lys Phe Tyr Trp Asn Gln Gln Ser Thr Gly Thr Thr Ser Asp Gln
                1445                1450                1455
Trp Gln Ser Leu Ala Tyr Thr Arg Thr Pro Phe Val Lys Thr Gly Thr
            1460                1465                1470
Tyr Asp Val Val Asn Pro Ser Asn Trp Gly Val Trp Gln Thr Ser Gln
        1475                1480                1485
Ser Ala Lys Phe Ile Val Thr Asn Ala Lys Pro Asn Gln Pro Thr Ile
    1490                1495                1500
Thr Gln Ser Lys Thr Gly Asp Val Thr Val Thr Pro Gly Ala Val Arg
1505                1510                1515                1520
Asn Ile Leu Ile Ser Gly Thr Asn Asp Tyr Ile Gln Ala Ser Ala Asp
                1525                1530                1535
Lys Ile Val Ile Asn Lys Asn Gly Asn Lys Leu Thr Thr Phe Val Lys
            1540                1545                1550
Asn Asn Asp Gly Arg Trp Thr Val Glu Thr Gly Ser Pro Asp Ile Asn
        1555                1560                1565
Gly Ile Gly Pro Thr Asn Asn Gly Thr Ala Ile Ser Leu Ser Arg Leu
    1570                1575                1580
Ala Val Arg Pro Gly Asp Ser Ile Glu Ala Ile Ala Thr Glu Gly Ser
1585                1590                1595                1600
Gly Glu Thr Ile Ser Thr Ser Ala Thr Ser Glu Ile Tyr Ile Val Lys
                1605                1610                1615
Ala Pro Gln Pro Glu Gln Val Ala Thr His Thr Tyr Asp Asn Gly Thr
            1620                1625                1630
Phe Asp Ile Leu Pro Asp Asn Ser Arg Asn Ser Leu Asn Pro Thr Glu
        1635                1640                1645
Arg Val Glu Ile Asn Tyr Thr Glu Lys Leu Asn Gly Asn Glu Thr Gln
    1650                1655                1660
Lys Ser Phe Thr Ile Thr Lys Asn Asn Asn Gly Lys Trp Thr Ile Asn
1665                1670                1675                1680
Asn Lys Pro Asn Tyr Val Glu Phe Asn Gln Asp Asn Gly Lys Val Val

                1685                1690                1695
Phe Ser Ala Asn Thr Ile Lys Pro Asn Ser Gln Ile Thr Ile Thr Pro
            1700                1705                1710
Lys Ala Gly Gln Gly Asn Thr Glu Asn Thr Asn Pro Thr Val Ile Gln
        1715                1720                1725
Ala Pro Ala Gln His Thr Leu Thr Ile Asn Glu Ile Val Lys Glu Gln
    1730                1735                1740
Gly Gln Asn Val Thr Asn Asp Asp Ile Asn Asn Ala Val Gln Val Pro
1745                1750                1755                1760
Asn Lys Asn Arg Val Ala Ile Lys Gln Gly Asn Ala Leu Pro Thr Asn
                1765                1770                1775
Leu Ala Gly Gly Ser Thr Ser His Ile Pro Val Val Ile Tyr Tyr Ser
            1780                1785                1790
Asp Gly Ser Ser Glu Glu Ala Thr Glu Thr Val Arg Thr Lys Val Asn
        1795                1800                1805
Lys Thr Glu Leu Ile Asn Ala Arg Arg Arg Leu Asp Glu Glu Ile Ser
    1810                1815                1820

Lys Glu Asn Lys Thr Pro Ser Ser Ile Arg Asn Phe Asp Gln Ala Met
1825                1830                1835                1840
Asn Arg Ala Gln Ser Gln Ile Asn Thr Ala Lys Ser Asp Ala Asp Gln
                1845                1850                1855
Val Ile Gly Thr Glu Phe Ala Thr Pro Gln Gln Val Asn Ser Ala Leu
            1860                1865                1870
Ser Lys Val Gln Ala Ala Gln Asn Lys Ile Asn Glu Ala Lys Ala Leu
        1875                1880                1885
Leu Gln Asn Lys Ala Asp Asn Ser Gln Leu Val Arg Ala Lys Glu Gln
    1890                1895                1900
Leu Gln Gln Ser Ile Gln Pro Ala Ala Ser Thr Asp Gly Met Thr Gln
1905                1910                1915                1920
Asp Ser Thr Arg Asn Tyr Asn Asn Lys Arg Gln Ala Ala Glu Gln Ala
                1925                1930                1935
Ile Gln His Ala Asn Ser Val Ile Asn Asn Gly Asp Ala Thr Ser Gln
            1940                1945                1950
Gln Ile Asn Asp Ala Lys Asn Thr Val Glu Gln Ala Gln Arg Asp Tyr
        1955                1960                1965
Val Glu Ala Lys Ser Asn Leu Arg Ala Asp Lys Ser Gln Leu Gln Ser
    1970                1975                1980
Ala Tyr Asp Thr Leu Asn Arg Asp Val Leu Thr Asn Asp Lys Lys Pro
1985                1990                1995                2000
Ala Ser Val Arg Arg Tyr Asn Glu Ala Ile Ser Asn Ile Arg Lys Glu
                2005                2010                2015
Leu Asp Thr Ala Lys Ala Asp Ala Ser Ser Thr Leu Arg Asn Thr Asn
            2020                2025                2030
Pro Ser Val Glu Gln Val Arg Asp Ala Leu Asn Lys Ile Asn Thr Val
        2035                2040                2045
Gln Pro Lys Val Asn Gln Ala Ile Ala Leu Leu Gln Pro Lys Glu Asn
    2050                2055                2060
Asn Ser Glu Leu Val Gln Ala Lys Lys Arg Leu Gln Asp Ala Val Asn
2065                2070                2075                2080
Asp Ile Pro Gln Thr Gln Gly Met Thr Gln Gln Thr Ile Asn Asn Tyr
                2085                2090                2095
Asn Asp Lys Gln Arg Glu Ala Glu Arg Ala Leu Thr Ser Ala Gln Arg
            2100                2105                2110
Val Ile Asp Asn Gly Asp Ala Thr Thr Gln Glu Ile Thr Ser Glu Lys
        2115                2120                2125
Ser Lys Val Glu Gln Ala Met Gln Ala Leu Thr Asn Ala Lys Ser Asn
    2130                2135                2140
Leu Arg Ala Asp Lys Asn Glu Leu Gln Thr Ala Tyr Asn Lys Leu Ile
2145                2150                2155                2160
Glu Asn Val Ser Thr Asn Gly Lys Lys Pro Ala Ser Ile Arg Gln Tyr
                2165                2170                2175
Glu Thr Ala Lys Ala Arg Ile Gln Asn Gln Ile Asn Asp Ala Lys Asn
            2180                2185                2190
Glu Ala Glu Arg Ile Leu Gly Asn Asp Asn Pro Gln Val Ser Gln Val
        2195                2200                2205
Thr Gln Ala Leu Asn Lys Ile Lys Ala Ile Gln Pro Lys Leu Thr Glu
    2210                2215                2220
Ala Ile Asn Met Leu Gln Asn Lys Glu Asn Asn Thr Glu Leu Val Asn
2225                2230                2235                2240
Ala Lys Asn Arg Leu Glu Asn Ala Val Asn Asp Thr Asp Pro Thr His
                2245                2250                2255
Gly Met Thr Gln Glu Thr Ile Asn Asn Tyr Asn Ala Lys Lys Arg Glu
            2260                2265                2270
Ala Gln Asn Glu Ile Gln Lys Ala Asn Met Ile Ile Asn Asn Gly Asp
        2275                2280                2285
Ala Thr Ala Gln Asp Ile Ser Ser Glu Lys Ser Lys Val Glu Gln Val
    2290                2295                2300
Leu Gln Ala Leu Gln Asn Ala Lys Asn Asp Leu Arg Ala Asp Lys Arg
2305                2310                2315                2320
Glu Leu Gln Thr Ala Tyr Asn Lys Leu Ile Gln Asn Val Asn Thr Asn
                2325                2330                2335
Gly Lys Lys Pro Ser Ser Ile Gln Asn Tyr Lys Ser Ala Arg Arg Asn

2340                2345                2350
Ile Glu Asn Gln Tyr Asn Thr Ala Lys Asn Glu Ala His Asn Val Leu
        2355                2360                2365
Glu Asn Thr Asn Pro Thr Val Asn Ala Val Glu Asp Ala Leu Arg Lys
    2370                2375                2380
Ile Asn Ala Ile Gln Pro Glu Val Thr Lys Ala Ile Asn Ile Leu Gln
2385                2390                2395                2400
Asp Lys Glu Asp Asn Ser Glu Leu Val Arg Ala Lys Glu Lys Leu Asp
                2405                2410                2415
Gln Ala Ile Asn Ser Gln Pro Ser Leu Asn Gly Met Thr Gln Glu Ser
            2420                2425                2430
Ile Asn Asn Tyr Thr Thr Lys Arg Arg Glu Ala Gln Asn Ile Ala Ser
        2435                2440                2445
Ser Ala Asp Thr Ile Ile Asn Asn Gly Asp Ala Ser Ile Glu Gln Ile
    2450                2455                2460
Thr Glu Asn Lys Ile Arg Val Glu Glu Ala Thr Asn Ala Leu Asn Glu
2465                2470                2475                2480
Ala Lys Gln His Leu Thr Ala Asp Thr Thr Ser Leu Lys Thr Glu Val
                2485                2490                2495
Arg Lys Leu Ser Arg Arg Gly Asp Thr Asn Asn Lys Lys Pro Ser Ser
            2500                2505                2510
Val Ser Ala Tyr Asn Asn Thr Ile His Ser Leu Gln Ser Glu Ile Thr
        2515                2520                2525
Gln Thr Glu Asn Arg Ala Asn Thr Ile Ile Asn Lys Pro Ile Arg Ser
    2530                2535                2540
Val Glu Glu Val Asn Asn Ala Leu His Glu Val Asn Gln Leu Asn Gln
2545                2550                2555                2560
Arg Leu Thr Asp Thr Ile Asn Leu Leu Gln Pro Leu Ala Asn Lys Glu
                2565                2570                2575
Ser Leu Lys Glu Ala Arg Asn Arg Leu Glu Ser Lys Ile Asn Glu Thr
            2580                2585                2590
Val Gln Thr Asp Gly Met Thr Gln Gln Ser Val Glu Asn Tyr Lys Gln
        2595                2600                2605
Ala Lys Ile Lys Ala Gln Asn Glu Ser Ser Ile Ala Gln Thr Leu Ile
    2610                2615                2620
Asn Asn Gly Asp Ala Ser Asp Gln Glu Val Ser Thr Glu Ile Glu Lys
2625                2630                2635                2640
Leu Asn Gln Lys Leu Ser Glu Leu Thr Asn Ser Ile Asn His Leu Thr
                2645                2650                2655
Val Asn Lys Glu Pro Leu Glu Thr Ala Lys Asn Gln Leu Gln Ala Asn
            2660                2665                2670
Ile Asp Gln Lys Pro Ser Thr Asp Gly Met Thr Gln Gln Ser Val Gln
        2675                2680                2685
Ser Tyr Glu Arg Lys Leu Gln Glu Ala Lys Asp Lys Ile Asn Ser Ile
    2690                2695                2700
Asn Asn Val Leu Ala Asn Asn Pro Asp Val Asn Ala Ile Arg Thr Asn
2705                2710                2715                2720
Lys Val Glu Thr Glu Gln Ile Asn Asn Glu Leu Thr Gln Ala Lys Gln
                2725                2730                2735
Gly Leu Thr Val Asp Lys Gln Pro Leu Ile Asn Ala Lys Thr Ala Leu
            2740                2745                2750
Gln Gln Ser Leu Asp Asn Gln Pro Ser Thr Thr Gly Met Thr Glu Ala
        2755                2760                2765
Thr Ile Gln Asn Tyr Asn Ala Lys Arg Gln Lys Ala Glu Gln Val Ile
    2770                2775                2780
Gln Asn Ala Asn Lys Ile Ile Glu Asn Ala Gln Pro Ser Val Gln Gln
2785                2790                2795                2800
Val Ser Asp Glu Lys Ser Lys Val Glu Gln Ala Leu Ser Glu Leu Asn
                2805                2810                2815
Asn Ala Lys Ser Ala Leu Arg Ala Asp Lys Gln Glu Leu Gln Gln Ala
            2820                2825                2830
Tyr Asn Gln Leu Ile Gln Pro Thr Asp Leu Asn Asn Lys Lys Pro Ala
        2835                2840                2845
Ser Ile Thr Ala Tyr Asn Gln Arg Tyr Gln Gln Phe Ser Asn Glu Leu
    2850                2855                2860

Asn Ser Thr Lys Thr Asn Thr Asp Arg Ile Leu Lys Glu Gln Asn Pro
2865                2870                2875                2880
Ser Val Ala Asp Val Asn Asn Ala Leu Asn Lys Val Arg Glu Val Gln
                2885                2890                2895
Gln Lys Leu Asn Glu Ala Arg Ala Leu Leu Gln Asn Lys Glu Asp Asn
            2900                2905                2910
Ser Ala Leu Val Arg Ala Lys Glu Gln Leu Gln Gln Ala Val Asp Gln
        2915                2920                2925
Val Pro Ser Thr Glu Gly Met Thr Gln Gln Thr Lys Asp Asp Tyr Asn
    2930                2935                2940
Ser Lys Gln Gln Ala Ala Gln Gln Glu Ile Ser Lys Ala Gln Gln Val
2945                2950                2955                2960
Ile Asp Asn Gly Asp Ala Thr Thr Gln Gln Ile Ser Asn Ala Lys Thr
                2965                2970                2975
Asn Val Glu Arg Ala Leu Glu Ala Leu Asn Asn Ala Lys Thr Gly Leu
            2980                2985                2990
Arg Ala Asp Lys Glu Glu Leu Gln Asn Ala Tyr Asn Gln Leu Thr Gln
        2995                3000                3005
Asn Ile Asp Thr Ser Gly Lys Thr Pro Ala Ser Ile Arg Lys Tyr Asn
    3010                3015                3020
Glu Ala Lys Ser Arg Ile Gln Thr Gln Ile Asp Ser Ala Lys Asn Glu
3025                3030                3035                3040
Ala Asn Ser Ile Leu Thr Asn Asp Asn Pro Gln Val Ser Gln Val Thr
                3045                3050                3055
Ala Ala Leu Asn Lys Ile Lys Ala Val Gln Pro Glu Leu Asp Lys Ala
            3060                3065                3070
Ile Ala Met Leu Lys Asn Lys Glu Asn Asn Asn Ala Leu Val Gln Ala
        3075                3080                3085
Lys Gln Gln Leu Gln Gln Ile Val Asn Glu Val Asp Pro Thr Gln Gly
    3090                3095                3100
Met Thr Thr Asp Thr Ala Asn Asn Tyr Lys Ser Lys Lys Arg Glu Ala
3105                3110                3115                3120
Glu Asp Glu Ile Gln Lys Ala Gln Gln Ile Ile Asn Asn Gly Asp Ala
                3125                3130                3135
Thr Glu Gln Gln Ile Thr Asn Glu Thr Asn Arg Val Asn Gln Ala Ile
            3140                3145                3150
Asn Ala Ile Asn Lys Ala Lys Asn Asp Leu Arg Ala Asp Lys Ser Gln
        3155                3160                3165
Leu Glu Asn Ala Tyr Asn Gln Leu Ile Gln Asn Val Asp Thr Asn Gly
    3170                3175                3180
Lys Lys Pro Ala Ser Ile Gln Gln Tyr Gln Ala Ala Arg Gln Ala Ile
3185                3190                3195                3200
Glu Thr Gln Tyr Asn Asn Ala Lys Ser Glu Ala His Gln Ile Leu Glu
                3205                3210                3215
Asn Ser Asn Pro Ser Val Asn Glu Val Ala Gln Ala Leu Gln Lys Val
            3220                3225                3230
Glu Ala Val Gln Leu Lys Val Asn Asp Ala Ile His Ile Leu Gln Asn
        3235                3240                3245
Lys Glu Asn Asn Ser Ala Leu Val Thr Ala Lys Asn Gln Leu Gln Gln
    3250                3255                3260
Ser Val Asn Asp Gln Pro Leu Thr Thr Gly Met Thr Gln Asp Ser Ile
3265                3270                3275                3280
Asn Asn Tyr Glu Ala Lys Arg Asn Glu Ala Gln Ser Ala Ile Arg Asn
                3285                3290                3295
Ala Glu Ala Val Ile Asn Asn Gly Asp Ala Thr Ala Lys Gln Ile Ser
            3300                3305                3310
Asp Glu Lys Ser Lys Val Glu Gln Ala Leu Ala His Leu Asn Asp Ala
        3315                3320                3325
Lys Gln Gln Leu Thr Ala Asp Thr Thr Glu Leu Gln Thr Ala Val Gln
    3330                3335                3340
Gln Leu Asn Arg Arg Gly Asp Thr Asn Asn Lys Lys Pro Arg Ser Ile
3345                3350                3355                3360
Asn Ala Tyr Asn Lys Ala Ile Gln Ser Leu Glu Thr Gln Ile Thr Ser
                3365                3370                3375
Ala Lys Asp Asn Ala Asn Ala Val Ile Gln Lys Pro Ile Arg Thr Val

                3380                3385                3390
Gln Glu Val Asn Asn Ala Leu Gln Gln Val Asn Gln Leu Asn Gln Gln
        3395                3400                3405
Leu Thr Glu Ala Ile Asn Gln Leu Gln Pro Leu Ser Asn Asn Asp Ala
    3410                3415                3420
Leu Lys Ala Ala Arg Leu Asn Leu Glu Asn Lys Ile Asn Gln Thr Val
3425                3430                3435                3440
Gln Thr Asp Gly Met Thr Gln Gln Ser Ile Glu Ala Tyr Gln Asn Ala
                3445                3450                3455
Lys Arg Val Ala Gln Asn Glu Ser Asn Thr Ala Leu Ala Leu Ile Asn
            3460                3465                3470
Asn Gly Asp Ala Asp Glu Gln Gln Ile Thr Thr Glu Thr Asp Arg Val
        3475                3480                3485
Asn Gln Gln Thr Thr Asn Leu Thr Gln Ala Ile Asn Gly Leu Thr Val
    3490                3495                3500
Asn Lys Glu Pro Leu Glu Thr Ala Lys Thr Ala Leu Gln Asn Asn Ile
3505                3510                3515                3520
Asp Gln Val Pro Ser Thr Asp Gly Met Thr Gln Gln Ser Val Ala Asn
                3525                3530                3535
Tyr Asn Gln Lys Leu Gln Ile Ala Lys Asn Glu Ile Asn Thr Ile Asn
            3540                3545                3550
Asn Val Leu Ala Asn Asn Pro Asp Val Asn Ala Ile Lys Thr Asn Lys
        3555                3560                3565
Ala Glu Ala Glu Arg Ile Ser Asn Asp Leu Thr Gln Ala Lys Asn Asn
    3570                3575                3580
Leu Gln Val Asp Thr Gln Pro Leu Glu Lys Ile Lys Arg Gln Leu Gln
3585                3590                3595                3600
Asp Glu Ile Asp Gln Gly Thr Asn Thr Asp Gly Met Thr Gln Asp Ser
                3605                3610                3615
Val Asp Asn Tyr Asn Asp Ser Leu Ser Ala Ala Ile Ile Glu Lys Gly
            3620                3625                3630
Lys Val Asn Lys Leu Leu Lys Arg Asn Pro Thr Val Glu Gln Val Lys
        3635                3640                3645
Glu Ser Val Ala Asn Ala Gln Gln Val Ile Gln Asp Leu Gln Asn Ala
    3650                3655                3660
Arg Thr Ser Leu Val Pro Asp Lys Thr Gln Leu Gln Glu Ala Lys Asn
3665                3670                3675                3680
Arg Leu Glu Asn Ser Ile Asn Gln Gln Thr Asp Thr Asp Gly Met Thr
                3685                3690                3695
Gln Asp Ser Leu Asn Asn Tyr Asn Asp Lys Leu Ala Lys Ala Arg Gln
            3700                3705                3710
Asn Leu Glu Lys Ile Ser Lys Val Leu Gly Gly Gln Pro Thr Val Ala
        3715                3720                3725
Glu Ile Arg Gln Asn Thr Asp Glu Ala Asn Ala His Lys Gln Ala Leu
    3730                3735                3740
Asp Thr Ala Arg Ser Gln Leu Thr Leu Asn Arg Glu Pro Tyr Ile Asn
3745                3750                3755                3760
His Ile Asn Asn Glu Ser His Leu Asn Asn Ala Gln Lys Asp Asn Phe
                3765                3770                3775
Lys Ala Gln Val Asn Ser Ala Pro Asn His Asn Thr Leu Glu Thr Ile
            3780                3785                3790
Lys Asn Lys Ala Asp Thr Leu Asn Gln Ser Met Thr Ala Leu Ser Glu
        3795                3800                3805
Ser Ile Ala Asp Tyr Glu Asn Gln Lys Gln Gln Glu Asn Tyr Leu Asp
    3810                3815                3820
Ala Ser Asn Asn Lys Arg Gln Asp Tyr Asp Asn Ala Val Asn Ala Ala
3825                3830                3835                3840
Lys Gly Ile Leu Asn Gln Thr Gln Ser Pro Thr Met Ser Ala Asp Val
                3845                3850                3855
Ile Asp Gln Lys Ala Glu Asp Val Lys Arg Thr Lys Thr Ala Leu Asp
            3860                3865                3870
Gly Asn Gln Arg Leu Glu Val Ala Lys Gln Gln Ala Leu Asn His Leu
        3875                3880                3885
Asn Thr Leu Asn Asp Leu Asn Asp Ala Gln Arg Gln Thr Leu Thr Asp
    3890                3895                3900

Thr Ile Asn His Ser Pro Asn Ile Asn Ser Val Asn Gln Ala Lys Glu
3905                3910                3915                3920
Lys Ala Asn Thr Val Asn Thr Ala Met Thr Gln Leu Lys Gln Thr Ile
                3925                3930                3935
Ala Asn Tyr Asp Asp Glu Leu His Asp Gly Asn Tyr Ile Asn Ala Asp
            3940                3945                3950
Lys Asp Lys Lys Asp Ala Tyr Asn Asn Ala Val Asn Asn Ala Lys Gln
        3955                3960                3965
Leu Ile Asn Gln Ser Asp Ala Asn Gln Ala Gln Leu Asp Pro Ala Glu
    3970                3975                3980
Ile Asn Lys Val Thr Gln Arg Val Asn Thr Thr Lys Asn Asp Leu Asn
3985                3990                3995                4000
Gly Asn Asp Lys Leu Ala Glu Ala Lys Arg Asp Ala Asn Thr Thr Ile
                4005                4010                4015
Asp Gly Leu Thr Tyr Leu Asn Glu Ala Gln Arg Asn Lys Ala Lys Glu
            4020                4025                4030
Asn Val Gly Lys Ala Ser Thr Lys Thr Asn Ile Thr Ser Gln Leu Gln
        4035                4040                4045
Asp Tyr Asn Gln Leu Asn Ile Ala Met Gln Ala Leu Arg Asn Ser Val
    4050                4055                4060
Asn Asp Val Asn Asn Val Lys Ala Asn Ser Asn Tyr Ile Asn Glu Asp
4065                4070                4075                4080
Asn Gly Pro Lys Glu Ala Tyr Asn Gln Ala Val Thr His Ala Gln Thr
                4085                4090                4095
Leu Ile Asn Ala Gln Ser Asn Pro Glu Met Ser Arg Asp Val Val Asn
            4100                4105                4110
Gln Lys Thr Gln Ala Val Asn Thr Ala His Gln Asn Leu His Gly Gln
        4115                4120                4125
Gln Lys Leu Glu Gln Ala Gln Ser Ser Ala Asn Thr Glu Ile Gly Asn
    4130                4135                4140
Leu Pro Asn Leu Thr Asn Thr Gln Lys Ala Lys Glu Lys Glu Leu Val
4145                4150                4155                4160
Asn Ser Lys Gln Thr Arg Thr Glu Val Gln Glu Gln Leu Asn Gln Ala
                4165                4170                4175
Lys Ser Leu Asp Ser Ser Met Gly Thr Leu Lys Ser Leu Val Ala Lys
            4180                4185                4190
Gln Pro Thr Val Gln Lys Thr Ser Val Tyr Ile Asn Glu Asp Gln Pro
        4195                4200                4205
Glu Gln Ser Ala Tyr Asn Asp Ser Ile Thr Met Gly Gln Thr Ile Ile
    4210                4215                4220
Asn Lys Thr Ala Asp Pro Val Leu Asp Lys Thr Leu Val Asp Asn Ala
4225                4230                4235                4240
Ile Ser Asn Ile Ser Thr Lys Glu Asn Ala Leu His Gly Glu Gln Lys
                4245                4250                4255
Leu Thr Thr Ala Lys Thr Glu Ala Ile Asn Ala Leu Asn Thr Leu Ala
            4260                4265                4270
Asp Leu Asn Thr Pro Gln Lys Glu Ala Ile Lys Thr Ala Ile Asn Thr
        4275                4280                4285
Ala His Thr Arg Thr Asp Val Thr Ala Glu Gln Ser Lys Ala Asn Gln
    4290                4295                4300
Ile Asn Ser Ala Met His Thr Leu Arg Gln Asn Ile Ser Asp Asn Glu
4305                4310                4315                4320
Ser Val Thr Asn Glu Ser Asn Tyr Ile Asn Ala Glu Pro Glu Lys Gln
                4325                4330                4335
His Ala Phe Thr Glu Ala Leu Asn Asn Ala Lys Glu Ile Val Asn Glu
            4340                4345                4350
Gln Gln Ala Thr Leu Asp Ala Asn Ser Ile Asn Gln Lys Ala Gln Ala
        4355                4360                4365
Ile Leu Thr Thr Lys Asn Ala Leu Asp Gly Glu Glu Gln Leu Arg Arg
    4370                4375                4380
Ala Lys Glu Asn Ala Asp Gln Glu Ile Asn Thr Leu Asn Gln Leu Thr
4385                4390                4395                4400
Asp Ala Gln Arg Asn Ser Glu Lys Gly Leu Val Asn Ser Ser Gln Thr
                4405                4410                4415
Arg Thr Glu Val Ala Ser Gln Leu Ala Lys Ala Lys Glu Leu Asn Lys

4420                    4425                    4430
Val Met Glu Gln Leu Asn His Leu Ile Asn Gly Lys Asn Gln Met Ile
        4435                    4440                    4445
Asn Ser Ser Lys Phe Ile Asn Glu Asp Ala Asn Gln Gln Gln Ala Tyr
    4450                    4455                    4460
Ser Asn Ala Ile Ala Ser Ala Glu Ala Leu Lys Asn Lys Ser Gln Asn
4465                    4470                    4475                    4480
Pro Glu Leu Asp Lys Val Thr Ile Glu Gln Ala Ile Asn Asn Ile Asn
                4485                    4490                    4495
Ser Ala Ile Asn Asn Leu Asn Gly Glu Ala Lys Leu Thr Lys Ala Lys
            4500                    4505                    4510
Glu Asp Ala Val Ala Ser Ile Asn Asn Leu Ser Gly Leu Thr Asn Glu
        4515                    4520                    4525
Gln Lys Thr Lys Glu Asn Gln Ala Val Asn Gly Ala Gln Thr Arg Asp
    4530                    4535                    4540
Gln Val Ala Asn Lys Leu Arg Asp Ala Glu Ala Leu Asp Gln Ser Met
4545                    4550                    4555                    4560
Gln Thr Leu Arg Asp Leu Val Asn Asn Gln Asn Ala Ile His Ser Thr
                4565                    4570                    4575
Ser Asn Tyr Phe Asn Glu Asp Ser Thr Gln Lys Asn Thr Tyr Asp Asn
            4580                    4585                    4590
Ala Ile Asp Asn Gly Ser Thr Tyr Ile Thr Gly Gln His Asn Pro Glu
        4595                    4600                    4605
Leu Asn Lys Ser Thr Ile Asp Gln Thr Ile Ser Arg Ile Asn Thr Ala
    4610                    4615                    4620
Lys Asn Asp Leu His Gly Val Glu Lys Leu Gln Arg Asp Lys Gly Thr
4625                    4630                    4635                    4640
Ala Asn Gln Glu Ile Gly Gln Leu Gly Tyr Leu Asn Asp Pro Gln Lys
                4645                    4650                    4655
Ser Gly Glu Glu Ser Leu Val Asn Gly Ser Asn Thr Arg Ser Glu Val
            4660                    4665                    4670
Glu Glu His Leu Asn Glu Ala Lys Ser Leu Asn Asn Ala Met Lys Gln
        4675                    4680                    4685
Leu Arg Asp Lys Val Ala Glu Lys Thr Asn Val Lys Gln Ser Ser Asp
    4690                    4695                    4700
Tyr Ile Asn Asp Ser Thr Glu His Gln Arg Gly Tyr Asp Gln Ala Leu
4705                    4710                    4715                    4720
Gln Glu Ala Glu Asn Ile Ile Asn Glu Ile Gly Asn Pro Thr Leu Asn
                4725                    4730                    4735
Lys Ser Glu Ile Glu Gln Lys Leu Gln Gln Leu Thr Asp Ala Gln Asn
            4740                    4745                    4750
Ala Leu Gln Gly Ser His Leu Leu Glu Glu Ala Lys Asn Asn Ala Ile
        4755                    4760                    4765
Thr Gly Ile Asn Lys Leu Thr Ala Leu Asn Asp Ala Gln Arg Gln Lys
    4770                    4775                    4780
Ala Ile Glu Asn Val Gln Ala Gln Gln Thr Ile Pro Ala Val Asn Gln
4785                    4790                    4795                    4800
Gln Leu Thr Leu Asp Arg Glu Ile Asn Thr Ala Met Gln Ala Leu Arg
                4805                    4810                    4815
Asp Lys Val Gly Gln Gln Asn Asn Val His Gln Gln Ser Asn Tyr Phe
            4820                    4825                    4830
Asn Glu Asp Glu Gln Pro Lys His Asn Tyr Asp Asn Ser Val Gln Ala
        4835                    4840                    4845
Gly Gln Thr Ile Ile Asp Lys Leu Gln Asp Pro Ile Met Asn Lys Asn
    4850                    4855                    4860
Glu Ile Glu Gln Ala Ile Asn Gln Ile Asn Thr Thr Gln Thr Ala Leu
4865                    4870                    4875                    4880
Ser Gly Glu Asn Lys Leu His Thr Asp Gln Glu Ser Thr Asn Arg Gln
                4885                    4890                    4895
Ile Glu Gly Leu Ser Ser Leu Asn Thr Ala Gln Ile Asn Ala Glu Lys
            4900                    4905                    4910
Asp Leu Val Asn Gln Ala Lys Thr Arg Thr Asp Val Ala Gln Lys Leu
        4915                    4920                    4925
Ala Ala Ala Lys Glu Ile Asn Ser Ala Met Ser Asn Leu Arg Asp Gly
    4930                    4935                    4940

Ile Gln Asn Lys Glu Asp Ile Lys Arg Ser Ser Ala Tyr Ile Asn Ala
4945                4950                4955                4960
Asp Pro Thr Lys Val Thr Ala Tyr Asp Gln Ala Leu Gln Asn Ala Glu
                4965                4970                4975
Asn Ile Ile Asn Ala Thr Pro Asn Val Glu Leu Asn Lys Ala Thr Ile
            4980                4985                4990
Glu Gln Ala Leu Ser Arg Val Gln Gln Ala Gln Gln Asp Leu Asp Gly
        4995                5000                5005
Val Gln Gln Leu Ala Asn Ala Lys Gln Gln Ala Thr Gln Thr Val Asn
    5010                5015                5020
Gly Leu Asn Ser Leu Asn Asp Gly Gln Lys Arg Glu Leu Asn Leu Leu
5025                5030                5035                5040
Ile Asn Ser Ala Asn Thr Arg Thr Lys Val Gln Glu Glu Leu Asn Lys
                5045                5050                5055
Ala Thr Glu Leu Asn His Ala Met Glu Ala Leu Arg Asn Ser Val Gln
            5060                5065                5070
Asn Val Asp Gln Val Lys Gln Ser Ser Asn Tyr Val Asn Glu Asp Gln
        5075                5080                5085
Pro Glu Gln His Asn Tyr Asp Asn Ala Val Asn Glu Ala Gln Ala Thr
    5090                5095                5100
Ile Asn Asn Asn Ala Gln Pro Val Leu Asp Lys Leu Ala Ile Glu Arg
5105                5110                5115                5120
Leu Thr Gln Thr Val Asn Thr Thr Lys Asp Ala Leu His Gly Ala Gln
                5125                5130                5135
Lys Leu Thr Gln Asp Gln Gln Ala Ala Glu Thr Gly Ile Arg Gly Leu
            5140                5145                5150
Thr Ser Leu Asn Glu Pro Gln Lys Asn Ala Glu Val Ala Lys Val Thr
        5155                5160                5165
Ala Ala Thr Thr Arg Asp Glu Val Arg Asn Ile Arg Gln Glu Ala Thr
    5170                5175                5180
Thr Leu Asp Thr Ala Met Leu Gly Leu Arg Lys Ser Ile Lys Asp Lys
5185                5190                5195                5200
Asn Asp Thr Lys Asn Ser Ser Lys Tyr Ile Asn Glu Asp His Asp Gln
                5205                5210                5215
Gln Gln Ala Tyr Asp Asn Ala Val Asn Asn Ala Gln Gln Val Ile Asp
            5220                5225                5230
Glu Thr Gln Ala Thr Leu Ser Ser Asp Thr Ile Asn Gln Leu Ala Asn
        5235                5240                5245
Ala Val Thr Gln Ala Lys Ser Asn Leu His Gly Asp Thr Lys Leu Gln
    5250                5255                5260
His Asp Lys Asp Ser Ala Lys Gln Thr Ile Ala Gln Leu Gln Asn Leu
5265                5270                5275                5280
Asn Ser Ala Gln Lys His Met Glu Asp Ser Leu Ile Asp Asn Glu Ser
                5285                5290                5295
Thr Arg Thr Gln Val Gln His Asp Leu Thr Glu Ala Gln Ala Leu Asp
            5300                5305                5310
Gly Leu Met Gly Ala Leu Lys Glu Ser Ile Lys Asp Tyr Thr Asn Ile
        5315                5320                5325
Val Ser Asn Gly Asn Tyr Ile Asn Ala Glu Pro Ser Lys Lys Gln Ala
    5330                5335                5340
Tyr Asp Ala Ala Val Gln Asn Ala Gln Asn Ile Ile Asn Gly Thr Asn
5345                5350                5355                5360
Gln Pro Thr Ile Asn Lys Gly Asn Val Thr Thr Ala Thr Gln Thr Val
                5365                5370                5375
Lys Asn Thr Lys Asp Ala Leu Asp Gly Asp His Arg Leu Glu Glu Ala
            5380                5385                5390
Lys Asn Asn Ala Asn Gln Thr Ile Arg Asn Leu Ser Asn Leu Asn Asn
        5395                5400                5405
Ala Gln Lys Asp Ala Glu Lys Asn Leu Val Asn Ser Ala Ser Thr Leu
    5410                5415                5420
Glu Gln Val Gln Gln Asn Leu Gln Thr Ala Gln Gln Leu Asp Asn Ala
5425                5430                5435                5440

Met Gly Glu Leu Arg Gln Ser Ile Ala Lys Lys Asp Gln Val Lys Ala
                5445                5450                5455

Asp Ser Lys Tyr Leu Asn Glu Asp Pro Gln Ile Lys Gln Asn Tyr Asp
            5460                5465                5470
Asp Ala Val Gln Arg Val Glu Thr Ile Ile Asn Glu Thr Gln Asn Pro
        5475                5480                5485
Glu Leu Leu Lys Ala Asn Ile Asp Gln Ala Thr Gln Ser Val Gln Asn
    5490                5495                5500
Ala Glu Gln Ala Leu His Gly Ala Glu Lys Leu Asn Gln Asp Lys Gln
5505                5510                5515                5520
Thr Ser Ser Thr Glu Leu Asp Gly Leu Thr Asp Leu Thr Asp Ala Gln
                5525                5530                5535
Arg Glu Lys Leu Arg Glu Gln Ile Asn Thr Ser Asn Ser Arg Asp Asp
            5540                5545                5550
Ile Lys Gln Lys Ile Glu Gln Ala Lys Ala Leu Asn Asp Ala Met Lys
        5555                5560                5565
Lys Leu Lys Glu Gln Val Ala Gln Lys Asp Gly Val His Ala Asn Ser
    5570                5575                5580
Asp Tyr Thr Asn Glu Asp Ser Ala Gln Lys Asp Ala Tyr Asn Asn Ala
5585                5590                5595                5600
Leu Lys Gln Ala Glu Asp Ile Ile Asn Asn Ser Ser Asn Pro Asn Leu
                5605                5610                5615
Asn Ala Gln Asp Ile Thr Asn Ala Leu Asn Asn Ile Lys Gln Ala Gln
            5620                5625                5630
Asp Asn Leu His Gly Ala Gln Lys Leu Gln Gln Asp Lys Asn Thr Thr
        5635                5640                5645
Asn Gln Ala Ile Gly Asn Leu Asn His Leu Asn Gln Pro Gln Lys Asp
    5650                5655                5660
Ala Leu Ile Gln Ala Ile Asn Gly Ala Thr Ser Arg Asp Gln Val Ala
5665                5670                5675                5680
Glu Lys Leu Lys Glu Ala Glu Ala Leu Asp Glu Ala Met Lys Gln Leu
                5685                5690                5695
Glu Asp Gln Val Asn Gln Asp Asp Gln Ile Ser Asn Ser Ser Pro Phe
            5700                5705                5710
Ile Asn Glu Asp Ser Asp Lys Gln Lys Thr Tyr Asn Asp Lys Ile Gln
        5715                5720                5725
Ala Ala Lys Glu Ile Ile Asn Gln Thr Ser Asn Pro Thr Leu Asp Lys
    5730                5735                5740
Gln Lys Ile Ala Asp Thr Leu Gln Asn Ile Lys Asp Ala Val Asn Asn
5745                5750                5755                5760
Leu His Gly Asp Gln Lys Leu Ala Gln Ser Lys Gln Asp Ala Asn Asn
                5765                5770                5775
Gln Leu Asn His Leu Asp Asp Leu Thr Glu Glu Gln Lys Asn His Phe

            5780                5785                5790
Lys Pro Leu Ile Asn Asn Ala Asp Thr Arg Asp Glu Val Asn Lys Gln
        5795                5800                5805
Leu Glu Ile Ala Lys Gln Leu Asn Gly Asp Met Ser Thr Leu His Lys
    5810                5815                5820
Val Ile Asn Asp Lys Asp Gln Ile Gln His Leu Ser Asn Tyr Ile Asn
5825                5830                5835                5840
Ala Asp Asn Asp Lys Lys Gln Asn Tyr Asp Asn Ala Ile Lys Glu Ala
                5845                5850                5855
Glu Asp Leu Ile His Asn His Pro Asp Thr Leu Asp His Lys Ala Leu
            5860                5865                5870
Gln Asp Leu Leu Asn Lys Ile Asp Gln Ala His Asn Glu Leu Asn Gly
        5875                5880                5885
Glu Ser Arg Phe Lys Gln Ala Leu Asp Asn Ala Leu Asn Asp Ile Asp
    5890                5895                5900
Ser Leu Asn Ser Leu Asn Val Pro Gln Arg Gln Thr Val Lys Asp Asn
5905                5910                5915                5920
Ile Asn His Val Thr Thr Leu Glu Ser Leu Ala Gln Glu Leu Gln Lys
                5925                5930                5935
Ala Lys Glu Leu Asn Asp Ala Met Lys Ala Met Arg Asp Ser Ile Met
            5940                5945                5950
Asn Gln Glu Gln Ile Arg Lys Asn Ser Asn Tyr Thr Asn Glu Asp Leu
        5955                5960                5965

Ala Gln Gln Asn Ala Tyr Asn His Ala Val Asp Lys Ile Asn Asn Ile
    5970                5975                5980
Ile Gly Glu Asp Asn Ala Thr Met Asp Pro Gln Ile Ile Lys Gln Ala
5985                5990                5995                6000
Thr Gln Asp Ile Asn Thr Ala Ile Asn Gly Leu Asn Gly Asp Gln Lys
                6005                6010                6015
Leu Gln Asp Ala Lys Thr Asp Ala Lys Gln Gln Ile Thr Asn Phe Thr
            6020                6025                6030
Gly Leu Thr Glu Pro Gln Lys Gln Ala Leu Glu Asn Ile Ile Asn Gln
        6035                6040                6045
Gln Thr Ser Arg Ala Asn Val Ala Lys Gln Leu Ser His Ala Lys Phe
    6050                6055                6060
Leu Asn Gly Lys Met Glu Glu Leu Lys Val Ala Val Ala Lys Ala Ser
6065                6070                6075                6080
Leu Val Arg Gln Asn Ser Asn Tyr Ile Asn Glu Asp Val Ser Glu Lys
                6085                6090                6095
Glu Ala Tyr Glu Gln Ala Ile Ala Lys Gly Gln Glu Ile Ile Asn Ser
            6100                6105                6110
Glu Asn Asn Pro Thr Ile Ser Ser Thr Asp Ile Asn Arg Thr Ile Gln
        6115                6120                6125
Glu Ile Asn Asp Ala Glu Gln Asn Leu His Gly Asp Asn Lys Leu Arg
    6130                6135                6140
Gln Ala Gln Glu Ile Ala Lys Asn Glu Ile Gln Asn Leu Asp Gly Leu
6145                6150                6155                6160
Asn Ser Ala Gln Ile Thr Lys Leu Ile Gln Asp Ile Gly Arg Thr Thr
                6165                6170                6175
Thr Lys Pro Ala Val Thr Gln Lys Leu Glu Glu Ala Lys Ala Ile Asn
            6180                6185                6190
Gln Ala Met Gln Gln Leu Lys Gln Ser Ile Ala Asp Lys Asp Ala Thr
        6195                6200                6205
Leu Asn Ser Ser Asn Tyr Leu Asn Glu Asp Ser Glu Lys Lys Leu Ala
    6210                6215                6220
Tyr Asp Asn Ala Val Ser Gln Ala Glu Gln Leu Ile Asn Gln Leu Asn
6225                6230                6235                6240
Asp Pro Thr Met Asp Ile Ser Asn Ile Gln Ala Ile Thr Gln Lys Val
                6245                6250                6255
Ile Gln Ala Lys Asp Ser Leu His Gly Ala Asn Lys Leu Ala Gln Asn
            6260                6265                6270
Gln Ala Asp Ser Asn Leu Ile Ile Asn Gln Ser Thr Asn Leu Asn Asp
        6275                6280                6285
Lys Gln Lys Gln Ala Leu Asn Asp Leu Ile Asn His Ala Gln Thr Lys
    6290                6295                6300
Gln Gln Val Ala Glu Ile Ile Ala Gln Ala Asn Lys Leu Asn Asn Glu
6305                6310                6315                6320
Met Gly Thr Leu Lys Thr Leu Val Glu Glu Gln Ser Asn Val His Gln
                6325                6330                6335
Gln Ser Lys Tyr Ile Asn Glu Asp Pro Gln Val Gln Asn Ile Tyr Asn
            6340                6345                6350
Asp Ser Ile Gln Lys Gly Arg Glu Ile Leu Asn Gly Thr Thr Asp Asp
        6355                6360                6365
Val Leu Asn Asn Asn Lys Ile Ala Asp Ala Ile Gln Asn Ile His Leu
    6370                6375                6380
Thr Lys Asn Asp Leu His Gly Asp Gln Lys Leu Gln Lys Ala Gln Gln
6385                6390                6395                6400
Asp Ala Thr Asn Glu Leu Asn Tyr Leu Thr Asn Leu Asn Asn Ser Gln
                6405                6410                6415
Arg Gln Ser Glu His Asp Glu Ile Asn Ser Ala Pro Ser Arg Thr Glu
            6420                6425                6430
Val Ser Asn Asp Leu Asn His Ala Lys Ala Leu Asn Glu Ala Met Arg
        6435                6440                6445
Gln Leu Glu Asn Glu Val Ala Leu Glu Asn Ser Val Lys Lys Leu Ser
    6450                6455                6460
Asp Phe Ile Asn Glu Asp Glu Ala Ala Gln Asn Glu Tyr Ser Asn Ala
6465                6470                6475                6480
Leu Gln Lys Ala Lys Asp Ile Ile Asn Gly Val Pro Ser Ser Thr Leu

6485                6490                6495
Asp Lys Ala Thr Ile Glu Asp Ala Leu Leu Glu Leu Gln Asn Ala Arg
        6500                6505                6510
Glu Ser Leu His Gly Glu Gln Lys Leu Gln Glu Ala Lys Asn Gln Ala
    6515                6520                6525
Val Ala Glu Ile Asp Asn Leu Gln Ala Leu Asn Pro Gly Gln Val Leu
    6530                6535                6540
Ala Glu Lys Thr Leu Val Asn Gln Ala Ser Thr Lys Pro Glu Val Gln
6545                6550                6555                6560
Glu Ala Leu Gln Lys Ala Lys Glu Leu Asn Glu Ala Met Lys Ala Leu
                6565                6570                6575
Lys Thr Glu Ile Asn Lys Lys Glu Gln Ile Lys Ala Asp Ser Arg Tyr
            6580                6585                6590
Val Asn Ala Asp Ser Gly Leu Gln Ala Asn Tyr Asn Ser Ala Leu Asn
        6595                6600                6605
Tyr Gly Ser Gln Ile Ile Ala Thr Thr Gln Pro Pro Glu Leu Asn Lys
    6610                6615                6620
Asp Val Ile Asn Arg Ala Thr Gln Thr Ile Lys Thr Ala Glu Asn Asn
6625                6630                6635                6640
Leu Asn Gly Gln Ser Lys Leu Ala Glu Ala Lys Ser Asp Gly Asn Gln
                6645                6650                6655
Ser Ile Glu His Leu Gln Gly Leu Thr Gln Ser Gln Lys Asp Lys Gln
            6660                6665                6670
His Asp Leu Ile Asn Gln Ala Gln Thr Lys Gln Gln Val Asp Asp Ile
        6675                6680                6685
Val Asn Asn Ser Lys Gln Leu Asp Asn Ser Met Asn Gln Leu Gln Gln
    6690                6695                6700
Ile Val Asn Asn Asp Asn Thr Val Lys Gln Asn Ser Asp Phe Ile Asn
6705                6710                6715                6720
Glu Asp Ser Ser Gln Gln Asp Ala Tyr Asn His Ala Ile Gln Ala Ala
                6725                6730                6735
Lys Asp Leu Ile Thr Ala His Pro Thr Ile Met Asp Lys Asn Gln Ile
            6740                6745                6750
Asp Gln Ala Ile Glu Asn Ile Lys Gln Ala Leu Asn Asp Leu His Gly
        6755                6760                6765
Ser Asn Lys Leu Ser Glu Asp Lys Lys Glu Ala Ser Glu Gln Leu Gln
    6770                6775                6780
Asn Leu Asn Ser Leu Thr Asn Gly Gln Lys Asp Thr Ile Leu Asn His
6785                6790                6795                6800
Ile Phe Ser Ala Pro Thr Arg Ser Gln Val Gly Glu Lys Ile Ala Ser
                6805                6810                6815
Ala Lys Gln Leu Asn Asn Thr Met Lys Ala Leu Arg Asp Ser Ile Ala
            6820                6825                6830
Asp Asn Asn Glu Ile Leu Gln Ser Ser Lys Tyr Phe Asn Glu Asp Ser
        6835                6840                6845
Glu Gln Gln Asn Ala Tyr Asn Gln Ala Val Asn Lys Ala Lys Asn Ile
    6850                6855                6860
Ile Asn Asp Gln Pro Thr Pro Val Met Ala Asn Asp Glu Ile Gln Ser
6865                6870                6875                6880
Val Leu Asn Glu Val Lys Gln Thr Lys Asp Asn Leu His Gly Asp Gln
                6885                6890                6895
Lys Leu Ala Asn Asp Lys Thr Asp Ala Gln Ala Thr Leu Asn Ala Leu
            6900                6905                6910
Asn Tyr Leu Asn Gln Ala Gln Arg Gly Asn Leu Glu Thr Lys Val Gln
        6915                6920                6925
Asn Ser Asn Ser Arg Pro Glu Val Gln Lys Val Val Gln Leu Ala Asn
    6930                6935                6940
Gln Leu Asn Asp Ala Met Lys Lys Leu Asp Asp Ala Leu Thr Gly Asn
6945                6950                6955                6960
Asp Ala Ile Lys Gln Thr Ser Asn Tyr Ile Asn Glu Asp Thr Ser Gln
                6965                6970                6975
Gln Val Asn Phe Asp Glu Tyr Thr Asp Arg Gly Lys Asn Ile Val Ala
            6980                6985                6990
Glu Gln Thr Asn Pro Asn Met Ser Pro Thr Asn Ile Asn Thr Ile Ala
        6995                7000                7005

Asp Lys Ile Thr Glu Ala Lys Asn Asp Leu His Gly Val Gln Lys Leu
    7010                7015                7020
Lys Gln Ala Gln Gln Gln Ser Ile Asn Thr Ile Asn Gln Met Thr Gly
7025                7030                7035                7040
Leu Asn Gln Ala Gln Lys Glu Gln Leu Asn Gln Glu Ile Gln Gln Thr
                7045                7050                7055
Gln Thr Arg Ser Glu Val His Gln Val Ile Asn Lys Ala Gln Ala Leu
            7060                7065                7070
Asn Asp Ser Met Asn Thr Leu Arg Gln Ser Ile Thr Asp Glu His Glu
        7075                7080                7085
Val Lys Gln Thr Ser Asn Tyr Ile Asn Glu Thr Val Gly Asn Gln Thr
    7090                7095                7100
Ala Tyr Asn Asn Ala Val Asp Arg Val Lys Gln Ile Ile Asn Gln Thr
7105                7110                7115                7120
Ser Asn Pro Thr Met Asn Pro Leu Glu Val Glu Arg Ala Thr Ser Asn
                7125                7130                7135
Val Lys Ile Ser Lys Asp Ala Leu His Gly Glu Arg Glu Leu Asn Asp
            7140                7145                7150
Asn Lys Asn Ser Lys Thr Phe Ala Val Asn His Leu Asp Asn Leu Asn
        7155                7160                7165
Gln Ala Gln Lys Glu Ala Leu Thr His Glu Ile Glu Gln Ala Thr Ile
    7170                7175                7180
Val Ser Gln Val Asn Asn Ile Tyr Asn Lys Ala Lys Ala Leu Asn Asn
7185                7190                7195                7200
Asp Met Lys Lys Leu Lys Asp Ile Val Ala Gln Gln Asp Asn Val Arg
                7205                7210                7215
Gln Ser Asn Asn Tyr Ile Asn Glu Asp Ser Thr Pro Gln Asn Met Tyr
            7220                7225                7230
Asn Asp Thr Ile Asn His Ala Gln Ser Ile Ile Asp Gln Val Ala Asn
        7235                7240                7245
Pro Thr Met Ser His Asp Glu Ile Glu Asn Ala Ile Asn Asn Ile Lys
    7250                7255                7260
His Ala Ile Asn Ala Leu Asp Gly Glu His Lys Leu Gln Gln Ala Lys
7265                7270                7275                7280
Glu Asn Ala Asn Leu Leu Ile Asn Ser Leu Asn Asp Leu Asn Ala Pro
                7285                7290                7295
Gln Arg Asp Ala Ile Asn Arg Leu Val Asn Glu Ala Gln Thr Arg Glu
            7300                7305                7310
Lys Val Ala Glu Gln Leu Gln Ser Ala Gln Ala Leu Asn Asp Ala Met
        7315                7320                7325
Lys His Leu Arg Asn Ser Ile Gln Asn Gln Ser Ser Val Arg Gln Glu
    7330                7335                7340
Ser Lys Tyr Ile Asn Ala Ser Asp Ala Lys Lys Glu Gln Tyr Asn His
7345                7350                7355                7360
Ala Val Arg Glu Val Glu Asn Ile Ile Asn Glu Gln His Pro Thr Leu
                7365                7370                7375
Asp Lys Glu Ile Ile Lys Gln Leu Thr Asp Gly Val Asn Gln Ala Asn
            7380                7385                7390
Asn Asp Leu Asn Gly Val Glu Leu Leu Asp Ala Asp Lys Gln Asn Ala
        7395                7400                7405
His Gln Ser Ile Pro Thr Leu Met His Leu Asn Gln Ala Gln Gln Asn
    7410                7415                7420
Ala Leu Asn Glu Lys Ile Asn Asn Ala Val Thr Arg Thr Glu Val Ala
7425                7430                7435                7440
Ala Ile Ile Gly Gln Ala Lys Leu Leu Asp His Ala Met Glu Asn Leu
                7445                7450                7455
Glu Glu Ser Ile Lys Asp Lys Glu Gln Val Lys Gln Ser Ser Asn Tyr
            7460                7465                7470
Ile Asn Glu Asp Ser Asp Val Gln Glu Thr Tyr Asp Asn Ala Val Asp
        7475                7480                7485
His Val Thr Glu Ile Leu Asn Gln Thr Val Asn Pro Thr Leu Ser Ile
    7490                7495                7500
Glu Asp Ile Glu His Ala Ile Asn Glu Val Asn Gln Ala Lys Lys Gln
7505                7510                7515                7520
Leu Arg Gly Lys Gln Lys Leu Tyr Gln Thr Ile Asp Leu Ala Asp Lys

7525                7530                7535
Glu Leu Ser Lys Leu Asp Asp Leu Thr Ser Gln Gln Ser Ser Ser Ile
            7540                7545                7550
Ser Asn Gln Ile Tyr Thr Ala Lys Thr Arg Thr Glu Val Ala Gln Ala
        7555                7560                7565
Ile Glu Lys Ala Lys Ser Leu Asn His Ala Met Lys Ala Leu Asn Lys
    7570                7575                7580
Val Tyr Lys Asn Ala Asp Lys Val Leu Asp Ser Ser Arg Phe Ile Asn
7585                7590                7595                7600
Glu Asp Gln Pro Glu Lys Lys Ala Tyr Gln Gln Ala Ile Asn His Val
                7605                7610                7615
Asp Ser Ile Ile His Arg Gln Thr Asn Pro Glu Met Asp Pro Thr Val
            7620                7625                7630
Ile Asn Ser Ile Thr His Glu Leu Glu Thr Ala Gln Asn Asn Leu His
        7635                7640                7645
Gly Asp Gln Lys Leu Ala His Ala Gln Gln Asp Ala Ala Asn Val Ile
    7650                7655                7660
Asn Gly Leu Ile His Leu Asn Val Ala Gln Arg Glu Val Met Ile Asn
7665                7670                7675                7680
Thr Asn Thr Asn Ala Thr Thr Arg Glu Lys Val Ala Lys Asn Leu Asp
                7685                7690                7695
Asn Ala Gln Ala Leu Asp Lys Ala Met Glu Thr Leu Gln Gln Val Val
            7700                7705                7710
Ala His Lys Asn Asn Ile Leu Asn Asp Ser Lys Tyr Leu Asn Glu Asp
        7715                7720                7725
Ser Lys Tyr Gln Gln Gln Tyr Asp Arg Val Ile Ala Asp Ala Glu Gln
    7730                7735                7740
Leu Leu Asn Gln Thr Thr Asn Pro Thr Leu Glu Pro Tyr Lys Val Asp
7745                7750                7755                7760
Ile Val Lys Asp Asn Val Leu Ala Asn Glu Lys Ile Leu Phe Gly Ala
                7765                7770                7775
Glu Lys Leu Ser Tyr Asp Lys Ser Asn Ala Asn Asp Glu Ile Lys His
            7780                7785                7790
Met Asn Tyr Leu Asn Asn Ala Gln Lys Gln Ser Ile Lys Asp Met Ile
        7795                7800                7805
Ser His Ala Ala Leu Arg Thr Glu Val Lys Gln Leu Leu Gln Gln Ala
    7810                7815                7820
Lys Ile Leu Asp Glu Ala Met Lys Ser Leu Glu Asp Lys Thr Gln Val
7825                7830                7835                7840
Val Ile Thr Asp Thr Thr Leu Pro Asn Tyr Thr Glu Ala Ser Glu Asp
                7845                7850                7855
Lys Lys Glu Lys Val Asp Gln Thr Val Ser His Ala Gln Ala Ile Ile
            7860                7865                7870
Asp Lys Ile Asn Gly Ser Asn Val Ser Leu Asp Gln Val Arg Gln Ala
        7875                7880                7885
Leu Glu Gln Leu Thr Gln Ala Ser Glu Asn Leu Asp Gly Asp Gln Arg
    7890                7895                7900
Val Glu Glu Ala Lys Val His Ala Asn Gln Thr Ile Asp Gln Leu Thr
7905                7910                7915                7920
His Leu Asn Ser Leu Gln Gln Gln Thr Ala Lys Glu Ser Val Lys Asn
                7925                7930                7935
Ala Thr Lys Leu Glu Glu Ile Ala Thr Val Ser Asn Asn Ala Gln Ala
            7940                7945                7950
Leu Asn Lys Val Met Gly Lys Leu Glu Gln Phe Ile Asn His Ala Asp
        7955                7960                7965
Ser Val Glu Asn Ser Asp Asn Tyr Arg Gln Ala Asp Asp Asp Lys Ile
    7970                7975                7980
Ile Ala Tyr Asp Glu Ala Leu Glu His Gly Gln Asp Ile Gln Lys Thr
7985                7990                7995                8000
Asn Ala Thr Gln Asn Glu Thr Lys Gln Ala Leu Gln Gln Leu Ile Tyr
                8005                8010                8015
Ala Glu Thr Ser Leu Asn Gly Phe Glu Arg Leu Asn His Ala Arg Pro
            8020                8025                8030
Arg Ala Leu Glu Tyr Ile Lys Ser Leu Glu Lys Ile Asn Asn Ala Gln
        8035                8040                8045

Lys Ser Ala Leu Glu Asp Lys Val Thr Gln Ser His Asp Leu Leu Glu
    8050                8055                8060
Leu Glu His Ile Val Asn Glu Gly Thr Asn Leu Asn Asp Ile Met Gly
8065                8070                8075                8080
Glu Leu Ala Asn Ala Ile Val Asn Asn Tyr Ala Pro Thr Lys Ala Ser
                8085                8090                8095
Ile Asn Tyr Ile Asn Ala Asp Asn Leu Arg Lys Asp Asn Phe Thr Gln
            8100                8105                8110
Ala Ile Asn Asn Ala Arg Asp Ala Leu Asn Lys Thr Gln Gly Gln Asn
        8115                8120                8125
Leu Asp Phe Asn Ala Ile Asp Thr Phe Lys Asp Asp Ile Phe Lys Thr
    8130                8135                8140
Lys Asp Ala Leu Asn Gly Ile Glu Arg Leu Thr Ala Ala Lys Ser Lys
8145                8150                8155                8160
Ala Glu Lys Leu Ile Asp Ser Leu Lys Phe Ile Asn Lys Ala Gln Phe
                8165                8170                8175
Thr His Ala Asn Asp Glu Ile Met Asn Thr Asn Ser Ile Ala Gln Leu
            8180                8185                8190
Ser Arg Ile Val Asn Gln Ala Phe Asp Leu Asn Asp Ala Met Lys Ser
        8195                8200                8205
Leu Arg Asp Glu Leu Asn Asn Gln Ala Phe Pro Val Gln Ala Ser Ser
    8210                8215                8220
Asn Tyr Ile Asn Ser Asp Glu Asp Leu Lys Gln Gln Phe Asp His Ala
8225                8230                8235                8240
Leu Ser Asn Ala Arg Lys Val Leu Ala Lys Glu Asn Gly Lys Asn Leu
                8245                8250                8255
Asp Glu Lys Gln Ile Gln Gly Leu Lys Gln Val Ile Glu Asp Thr Lys
            8260                8265                8270
Asp Ala Leu Asn Gly Ile Gln Arg Leu Ser Lys Ala Lys Ala Lys Ala
        8275                8280                8285
Ile Gln Tyr Val Gln Ser Leu Ser Tyr Ile Asn Asp Ala Gln Arg His
    8290                8295                8300
Ile Ala Glu Asn Asn Ile His Asn Ser Asp Asp Leu Ser Ser Leu Ala
8305                8310                8315                8320
Asn Thr Leu Ser Lys Ala Ser Asp Leu Asp Asn Ala Met Lys Asp Leu
                8325                8330                8335
Arg Asp Thr Ile Glu Ser Asn Ser Thr Ser Val Pro Asn Ser Val Asn
            8340                8345                8350
Tyr Ile Asn Ala Asp Lys Asn Leu Gln Ile Glu Phe Asp Glu Ala Leu
        8355                8360                8365
Gln Gln Ala Ser Ala Thr Ser Ser Lys Thr Ser Glu Asn Pro Ala Thr
    8370                8375                8380
Ile Glu Glu Val Leu Gly Leu Ser Gln Ala Ile Tyr Asp Thr Lys Asn
8385                8390                8395                8400
Ala Leu Asn Gly Glu Gln Arg Leu Ala Thr Glu Lys Ser Lys Asp Leu
                8405                8410                8415
Lys Leu Ile Lys Gly Leu Lys Asp Leu Asn Lys Ala Gln Leu Glu Asp
            8420                8425                8430
Val Thr Asn Lys Val Asn Ser Ala Asn Thr Leu Thr Glu Leu Ser Gln
        8435                8440                8445
Leu Thr Gln Ser Thr Leu Glu Leu Asn Asp Lys Met Lys Leu Leu Arg
    8450                8455                8460
Asp Lys Leu Lys Thr Leu Val Asn Pro Val Lys Ala Ser Leu Asn Tyr
8465                8470                8475                8480
Arg Asn Ala Asp Tyr Asn Leu Lys Arg Gln Phe Asn Lys Ala Leu Lys
                8485                8490                8495
Glu Ala Lys Gly Val Leu Asn Lys Asn Ser Gly Thr Asn Val Asn Ile
            8500                8505                8510
Asn Asp Ile Gln His Leu Leu Thr Gln Ile Asp Asn Ala Lys Asp Gln
        8515                8520                8525
Leu Asn Gly Glu Arg Arg Leu Lys Glu His Gln Gln Lys Ser Glu Val
    8530                8535                8540
Phe Ile Ile Lys Glu Leu Asp Ile Leu Asn Asn Ala Gln Lys Ala Ala
8545                8550                8555                8560
Ile Ile Asn Gln Ile Arg Ala Ser Lys Asp Ile Lys Ile Ile Asn Gln

8565                    8570                    8575
Ile Val Asp Asn Ala Ile Glu Leu Asn Asp Ala Met Gln Gly Leu Lys
            8580                    8585                    8590
Glu His Val Ala Gln Leu Thr Ala Thr Thr Lys Asp Asn Ile Glu Tyr
        8595                    8600                    8605
Leu Asn Ala Asp Glu Asp His Lys Leu Gln Tyr Asp Tyr Ala Ile Asn
    8610                    8615                    8620
Leu Ala Asn Asn Val Leu Asp Lys Glu Asn Gly Thr Asn Lys Asp Ala
8625                    8630                    8635                    8640
Asn Ile Ile Ile Gly Met Ile Gln Asn Met Asp Asp Ala Arg Ala Leu
                8645                    8650                    8655
Leu Asn Gly Ile Glu Arg Leu Lys Asp Ala Gln Thr Lys Ala His Asn
            8660                    8665                    8670
Asp Ile Lys Asp Thr Leu Lys Arg Gln Leu Asp Glu Ile Glu His Ala
        8675                    8680                    8685
Asn Ala Thr Ser Asn Ser Lys Ala Gln Ala Lys Gln Met Val Asn Glu
    8690                    8695                    8700
Glu Ala Arg Lys Ala Leu Ser Asn Ile Asn Asp Ala Thr Ser Asn Asp
8705                    8710                    8715                    8720
Leu Val Asn Gln Ala Lys Asp Glu Gly Gln Ser Ala Ile Glu His Ile
                8725                    8730                    8735
His Ala Asp Glu Leu Pro Lys Ala Lys Leu Asp Ala Asn Gln Met Ile
            8740                    8745                    8750
Asp Gln Lys Val Glu Asp Ile Asn His Leu Ile Ser Gln Asn Pro Asn
        8755                    8760                    8765
Leu Ser Asn Glu Glu Lys Asn Lys Leu Ile Ser Gln Ile Asn Lys Leu
    8770                    8775                    8780
Val Asn Gly Ile Lys Asn Glu Ile Gln Gln Ala Ile Asn Lys Gln Gln
8785                    8790                    8795                    8800
Ile Glu Asn Ala Thr Thr Lys Leu Asp Glu Val Ile Glu Thr Thr Lys
                8805                    8810                    8815
Lys Leu Ile Ile Ala Lys Ala Glu Ala Lys Gln Met Ile Lys Glu Leu
            8820                    8825                    8830
Ser Gln Lys Lys Arg Asp Ala Ile Asn Asn Asn Thr Asp Leu Thr Pro
        8835                    8840                    8845
Ser Gln Lys Ala His Ala Leu Ala Asp Ile Asp Lys Thr Glu Lys Asp
    8850                    8855                    8860
Ala Leu Gln His Ile Glu Asn Ser Asn Ser Ile Asp Asp Ile Asn Asn
8865                    8870                    8875                    8880
Asn Lys Glu His Ala Phe Asn Thr Leu Ala His Ile Ile Ile Trp Asp
                8885                    8890                    8895
Thr Asp Gln Gln Pro Leu Val Phe Glu Leu Pro Glu Leu Ser Leu Gln
            8900                    8905                    8910
Asn Ala Leu Val Thr Ser Glu Val Val Val His Arg Asp Glu Thr Ile
        8915                    8920                    8925
Ser Leu Glu Ser Ile Ile Gly Ala Met Thr Leu Thr Asp Glu Leu Lys
    8930                    8935                    8940
Val Asn Ile Val Ser Leu Pro Asn Thr Asp Lys Val Ala Asp His Leu
8945                    8950                    8955                    8960
Thr Ala Lys Val Lys Val Ile Leu Ala Asp Gly Ser Tyr Val Thr Val
                8965                    8970                    8975
Asn Val Pro Val Lys Val Val Glu Lys Glu Leu Gln Ile Ala Lys Lys
            8980                    8985                    8990
Asp Ala Ile Lys Thr Ile Asp Val Leu Val Lys Gln Lys Ile Lys Asp
        8995                    9000                    9005
Ile Asp Ser Asn Asn Glu Leu Thr Ser Thr Gln Arg Glu Asp Ala Lys
    9010                    9015                    9020
Ala Glu Ile Glu Arg Leu Lys Lys Gln Ala Ile Asp Lys Val Asn His
9025                    9030                    9035                    9040
Ser Lys Ser Ile Lys Asp Ile Glu Thr Val Lys Arg Thr Asp Phe Glu
                9045                    9050                    9055
Glu Ile Asp Gln Phe Asp Pro Lys Arg Phe Thr Leu Asn Lys Ala Lys
            9060                    9065                    9070
Lys Asp Ile Ile Thr Asp Val Asn Thr Gln Ile Gln Asn Gly Phe Lys
        9075                    9080                    9085

Glu Ile Glu Thr Ile Lys Gly Leu Thr Ser Asn Glu Lys Thr Gln Phe
    9090                9095                9100
Asp Lys Gln Leu Thr Ala Leu Gln Lys Glu Phe Leu Glu Lys Val Glu
9105                9110                9115                9120
His Ala His Asn Leu Val Glu Leu Asn Gln Leu Gln Gln Glu Phe Asn
                9125                9130                9135
Asn Arg Tyr Lys His Ile Leu Asn Gln Ala His Leu Leu Gly Glu Lys
            9140                9145                9150
His Ile Ala Glu His Lys Leu Gly Tyr Val Val Val Asn Lys Thr Gln
        9155                9160                9165
Gln Ile Leu Asn Asn Gln Ser Ala Ser Tyr Phe Ile Lys Gln Trp Ala
    9170                9175                9180
Leu Asp Arg Ile Lys Gln Ile Gln Leu Glu Thr Met Asn Ser Ile Arg
9185                9190                9195                9200
Gly Ala His Thr Val Gln Asp Val His Lys Ala Leu Leu Gln Gly Ile
                9205                9210                9215
Glu Gln Ile Leu Lys Val Asn Val Ser Ile Ile Asn Gln Ser Phe Asn
            9220                9225                9230
Asp Ser Leu His Asn Phe Asn Tyr Leu His Ser Lys Phe Asp Ala Arg
        9235                9240                9245
Leu Arg Glu Lys Asp Val Ala Asn His Ile Val Gln Thr Glu Thr Phe
    9250                9255                9260
Lys Glu Val Leu Lys Gly Thr Gly Val Glu Pro Gly Lys Ile Asn Lys
9265                9270                9275                9280
Glu Thr Gln Gln Pro Lys Leu His Lys Asn Asp Asn Asp Ser Leu Phe
                9285                9290                9295
Lys His Leu Val Asp Asn Phe Gly Lys Thr Val Gly Val Ile Thr Leu
            9300                9305                9310
Thr Gly Leu Leu Ser Ser Phe Trp Leu Val Leu Ala Lys Arg Arg Lys
        9315                9320                9325
Lys Glu Glu Glu Glu Lys Gln Ser Ile Lys Asn His His Lys Asp Ile
    9330                9335                9340
Arg Leu Ser Asp Thr Asp Lys Ile Asp Pro Ile Val Ile Thr Lys Arg
9345                9350                9355                9360
Lys Ile Asp Lys Glu Glu Gln Ile Gln Asn Asp Asp Lys His Ser Ile
                9365                9370                9375
Pro Val Ala Lys His Lys Lys Ser Lys Glu Lys Gln Leu Ser Glu Glu
            9380                9385                9390
Asp Ile His Ser Ile Pro Val Val Lys Arg Lys Gln Asn Ser Asp Asn
        9395                9400                9405
Lys Asp Thr Lys Gln Lys Lys Val Thr Ser Lys Lys Lys Lys Thr Pro
    9410                9415                9420
Gln Ser Thr Lys Lys Val Val Lys Thr Lys Lys Arg Ser Lys Lys
9425                9430                9435

<210> 16
<211> 1115
<212> PRT
<213> Staphylococcus aureus

<400> 16
Met Arg Asp Lys Lys Gly Pro Val Asn Lys Arg Val Asp Phe Leu Ser
 1               5                  10                  15
Asn Lys Leu Asn Lys Tyr Ser Ile Arg Lys Phe Thr Val Gly Thr Ala
            20                  25                  30
Ser Ile Leu Ile Gly Ser Leu Met Tyr Leu Gly Thr Gln Gln Glu Ala
        35                  40                  45
Glu Ala Ala Glu Asn Asn Ile Glu Asn Pro Thr Thr Leu Lys Asp Asn
    50                  55                  60
Val Gln Ser Lys Glu Val Lys Ile Glu Glu Val Thr Asn Lys Asp Thr
65                  70                  75                  80
Ala Pro Gln Gly Val Glu Ala Lys Ser Glu Val Thr Ser Asn Lys Asp

                85                  90                  95
Thr Ile Glu His Glu Ala Ser Val Lys Ala Glu Asp Ile Ser Lys Lys
            100                 105                 110
Glu Asp Thr Pro Lys Glu Val Ala Asn Val Ala Glu Val Gln Pro Lys
        115                 120                 125
Ser Ser Val Thr His Asn Ala Glu Ala Pro Lys Val Arg Lys Ala Arg
    130                 135                 140
Ser Val Asp Glu Gly Ser Phe Asp Ile Thr Arg Asp Ser Lys Asn Val
145                 150                 155                 160
Val Glu Ser Thr Pro Ile Thr Ile Gln Gly Lys Glu His Phe Glu Gly
                165                 170                 175
Tyr Gly Ser Val Asp Ile Gln Lys Asn Pro Thr Asp Leu Gly Val Ser
            180                 185                 190
Glu Val Thr Arg Phe Asn Val Gly Asn Glu Ser Asn Gly Leu Ile Gly
        195                 200                 205
Ala Leu Gln Leu Lys Asn Lys Ile Asp Phe Ser Lys Asp Phe Asn Phe
    210                 215                 220
Lys Val Arg Val Ala Asn Asn His Gln Ser Asn Thr Thr Gly Ala Asp
225                 230                 235                 240
Gly Trp Gly Phe Leu Phe Ser Lys Gly Asn Ala Glu Glu Tyr Leu Thr
                245                 250                 255
Asn Gly Gly Ile Leu Gly Asp Lys Gly Leu Val Asn Ser Gly Gly Phe
            260                 265                 270
Lys Ile Asp Thr Gly Tyr Ile Tyr Thr Ser Ser Met Asp Lys Thr Glu
        275                 280                 285
Lys Gln Ala Gly Gln Gly Tyr Arg Gly Tyr Gly Ala Phe Val Lys Asn
    290                 295                 300
Asp Ser Ser Gly Asn Ser Gln Met Val Gly Glu Asn Ile Asp Lys Ser
305                 310                 315                 320
Lys Thr Asn Phe Leu Asn Tyr Ala Asp Asn Ser Thr Asn Thr Ser Asp
                325                 330                 335
Gly Lys Phe His Gly Gln Arg Leu Asn Asp Val Ile Leu Thr Tyr Val
            340                 345                 350
Ala Ser Thr Gly Lys Met Arg Ala Glu Tyr Ala Gly Lys Thr Trp Glu
        355                 360                 365
Thr Ser Ile Thr Asp Leu Gly Leu Ser Lys Asn Gln Ala Tyr Asn Phe
    370                 375                 380
Leu Ile Thr Ser Ser Gln Arg Trp Gly Leu Asn Gln Gly Ile Asn Ala
385                 390                 395                 400
Asn Gly Trp Met Arg Thr Asp Leu Lys Gly Ser Glu Phe Thr Phe Thr
                405                 410                 415
Pro Glu Ala Pro Lys Thr Ile Thr Glu Leu Glu Lys Lys Val Glu Glu
            420                 425                 430

Ile Pro Phe Lys Lys Glu Arg Lys Phe Asn Pro Asp Leu Ala Pro Gly
        435                 440                 445
Thr Glu Lys Val Thr Arg Glu Gly Gln Lys Gly Glu Lys Thr Ile Thr
    450                 455                 460
Thr Pro Thr Leu Lys Asn Pro Leu Thr Gly Glu Ile Ile Ser Lys Gly
465                 470                 475                 480
Glu Ser Lys Glu Glu Ile Thr Lys Asp Pro Ile Asn Glu Leu Thr Glu
                485                 490                 495
Tyr Gly Pro Glu Thr Ile Ala Pro Gly His Arg Asp Glu Phe Asp Pro
            500                 505                 510
Lys Leu Pro Thr Gly Glu Lys Glu Glu Val Pro Gly Lys Pro Gly Ile
        515                 520                 525
Lys Asn Pro Glu Thr Gly Asp Val Val Arg Pro Pro Val Asp Ser Val
    530                 535                 540
Thr Lys Tyr Gly Pro Val Lys Gly Asp Ser Ile Val Glu Lys Glu Glu
545                 550                 555                 560
Ile Pro Phe Glu Lys Glu Arg Lys Phe Asn Pro Asp Leu Ala Pro Gly
                565                 570                 575
Thr Glu Lys Val Thr Arg Glu Gly Gln Lys Gly Glu Lys Thr Ile Thr
            580                 585                 590
Thr Pro Thr Leu Lys Asn Pro Leu Thr Gly Glu Ile Ile Ser Lys Gly

        595                 600                 605
Glu Ser Lys Glu Glu Ile Thr Lys Asp Pro Ile Asn Glu Leu Thr Glu
    610                 615                 620
Tyr Gly Pro Glu Thr Ile Ala Pro Gly His Arg Asp Glu Phe Asp Pro
625                 630                 635                 640
Lys Leu Pro Thr Gly Glu Lys Glu Glu Val Pro Gly Lys Pro Gly Ile
                645                 650                 655
Lys Asn Pro Glu Thr Gly Asp Val Val Arg Pro Pro Val Asp Ser Val
            660                 665                 670
Thr Lys Tyr Gly Pro Val Lys Gly Asp Ser Ile Val Glu Lys Glu Glu
        675                 680                 685
Ile Pro Phe Lys Lys Glu Arg Lys Phe Asn Pro Asp Leu Ala Pro Gly
    690                 695                 700
Thr Glu Lys Val Thr Arg Glu Gly Gln Lys Gly Glu Lys Thr Ile Thr
705                 710                 715                 720
Thr Pro Thr Leu Lys Asn Pro Leu Thr Gly Glu Ile Ile Ser Lys Gly
                725                 730                 735
Glu Ser Lys Glu Glu Ile Thr Lys Asp Pro Ile Asn Glu Leu Thr Glu
            740                 745                 750
Tyr Gly Pro Glu Thr Ile Thr Pro Gly His Arg Asp Glu Phe Asp Pro
        755                 760                 765
Lys Leu Pro Thr Gly Glu Lys Glu Glu Val Pro Gly Lys Pro Gly Ile

    770                 775                 780
Lys Asn Pro Glu Thr Gly Asp Val Val Arg Pro Pro Val Asp Ser Val
785                 790                 795                 800
Thr Lys Tyr Gly Pro Val Lys Gly Asp Ser Ile Val Glu Lys Glu Glu
                805                 810                 815
Ile Pro Phe Glu Lys Glu Arg Lys Phe Asn Pro Asp Leu Ala Pro Gly
            820                 825                 830
Thr Glu Lys Val Thr Arg Glu Gly Gln Lys Gly Glu Lys Thr Ile Thr
        835                 840                 845
Thr Pro Thr Leu Lys Asn Pro Leu Thr Gly Glu Ile Ile Ser Lys Gly
    850                 855                 860
Glu Ser Lys Glu Glu Ile Thr Lys Asp Pro Val Asn Glu Leu Thr Glu
865                 870                 875                 880
Phe Gly Gly Glu Lys Ile Pro Gln Gly His Lys Asp Ile Phe Asp Pro
                885                 890                 895
Asn Leu Pro Thr Asp Gln Thr Glu Lys Val Pro Gly Lys Pro Gly Ile
            900                 905                 910
Lys Asn Pro Asp Thr Gly Lys Val Ile Glu Glu Pro Val Asp Asp Val
        915                 920                 925
Ile Lys His Gly Pro Lys Thr Gly Thr Pro Glu Thr Lys Thr Val Glu
    930                 935                 940
Ile Pro Phe Glu Thr Lys Arg Glu Phe Asn Pro Lys Leu Gln Pro Gly
945                 950                 955                 960
Glu Glu Arg Val Lys Gln Glu Gly Gln Pro Gly Ser Lys Thr Ile Thr
                965                 970                 975
Thr Pro Ile Thr Val Asn Pro Leu Thr Gly Glu Lys Val Gly Glu Gly
            980                 985                 990
Gln Pro Thr Glu Glu Ile Thr Lys Gln Pro Val Asp Lys Ile Val Glu
        995                 1000                1005
Phe Gly Gly Glu Lys Pro Lys Asp Pro Lys Gly Pro Glu Asn Pro Glu
    1010                1015                1020
Lys Pro Ser Arg Pro Thr His Pro Ser Gly Pro Val Asn Pro Asn Asn
1025                1030                1035                1040
Pro Gly Leu Ser Lys Asp Arg Ala Lys Pro Asn Gly Pro Val His Ser
                1045                1050                1055
Met Asp Lys Asn Asp Lys Val Lys Lys Ser Lys Ile Ala Lys Glu Ser
            1060                1065                1070
Val Ala Asn Gln Glu Lys Lys Arg Ala Glu Leu Pro Lys Thr Gly Leu
        1075                1080                1085
Glu Ser Thr Gln Lys Gly Leu Ile Phe Ser Ser Ile Ile Gly Ile Ala
    1090                1095                1100
Gly Leu Met Leu Leu Ala Arg Arg Arg Lys Asn

1105                1110                1115

<210> 17
<211> 1469
<212> PRT
<213> Staphylococcus aureus

<400> 17
Met Gly Lys Arg Arg Gln Gly Pro Ile Asn Lys Lys Val Asp Phe Leu
1               5                   10                  15
Pro Asn Lys Leu Asn Lys Tyr Ser Ile Arg Lys Phe Thr Val Gly Thr
            20                  25                  30
Ala Ser Ile Leu Leu Gly Ser Thr Leu Ile Phe Gly Ser Ser Ser His
        35                  40                  45
Glu Ala Lys Ala Ala Glu Glu Lys Gln Val Asp Pro Ile Thr Gln Ala
    50                  55                  60
Asn Gln Asn Asp Ser Ser Glu Arg Ser Leu Glu Asn Thr Asn Gln Pro
65                  70                  75                  80
Thr Val Asn Asn Glu Ala Pro Gln Met Ser Ser Thr Leu Gln Ala Glu
                85                  90                  95
Glu Gly Ser Asn Ala Glu Ala Pro Asn Val Pro Thr Ile Lys Ala Asn
            100                 105                 110
Ser Asp Asn Asp Thr Gln Thr Gln Phe Ser Glu Ala Pro Thr Arg Asn
        115                 120                 125
Asp Leu Ala Arg Lys Glu Asp Ile Pro Ala Val Ser Lys Asn Glu Glu
    130                 135                 140
Leu Gln Ser Ser Gln Pro Asn Thr Asp Ser Lys Ile Glu Pro Thr Thr
145                 150                 155                 160
Ser Glu Pro Val Asn Leu Asn Tyr Ser Ser Pro Phe Met Ser Leu Leu
                165                 170                 175
Ser Met Pro Ala Asp Ser Ser Ser Asn Asn Thr Lys Asn Thr Ile Asp
            180                 185                 190
Ile Pro Pro Thr Thr Val Lys Gly Arg Asp Asn Tyr Asp Phe Tyr Gly
        195                 200                 205
Arg Val Asp Ile Gln Ser Asn Pro Thr Asp Leu Asn Ala Thr Asn Leu
    210                 215                 220
Thr Arg Tyr Asn Tyr Gly Gln Pro Pro Gly Thr Thr Thr Ala Gly Ala
225                 230                 235                 240
Val Gln Phe Lys Asn Gln Val Ser Phe Asp Lys Asp Phe Asp Phe Asn
                245                 250                 255
Ile Arg Val Ala Asn Asn Arg Gln Ser Asn Thr Thr Gly Ala Asp Gly
            260                 265                 270
Trp Gly Phe Met Phe Ser Lys Lys Asp Gly Asp Asp Phe Leu Lys Asn
        275                 280                 285
Gly Gly Ile Leu Arg Glu Lys Gly Thr Pro Ser Ala Ala Gly Phe Arg
    290                 295                 300
Ile Asp Thr Gly Tyr Tyr Asn Asn Asp Pro Leu Asp Lys Ile Gln Lys
305                 310                 315                 320
Gln Ala Gly Gln Gly Tyr Arg Gly Tyr Gly Thr Phe Val Lys Asn Asp
                325                 330                 335
Ser Gln Gly Asn Thr Ser Lys Val Gly Ser Gly Thr Pro Ser Thr Asp
            340                 345                 350
Phe Leu Asn Tyr Ala Asp Asn Thr Thr Asn Asp Leu Asp Gly Lys Phe
        355                 360                 365
His Gly Gln Lys Leu Asn Asn Val Asn Leu Lys Tyr Asn Ala Ser Asn
    370                 375                 380
Gln Thr Phe Thr Ala Thr Tyr Ala Gly Lys Thr Trp Thr Ala Thr Leu
385                 390                 395                 400
Ser Glu Leu Gly Leu Ser Pro Thr Asp Ser Tyr Asn Phe Leu Val Thr
                405                 410                 415
Ser Ser Gln Tyr Gly Asn Gly Asn Ser Gly Thr Tyr Ala Asp Gly Val
            420                 425                 430
Met Arg Ala Asp Leu Asp Gly Ala Thr Leu Thr Tyr Thr Pro Lys Ala
        435                 440                 445

Val Asp Gly Asp Pro Ile Thr Ser Thr Lys Glu Ile Pro Phe Asn Lys
    450                 455                 460
Lys Arg Glu Phe Asp Pro Asn Leu Ala Pro Gly Thr Glu Lys Val Val
465                 470                 475                 480
Gln Lys Gly Glu Pro Gly Ile Glu Thr Thr Thr Thr Pro Thr Tyr Val
                485                 490                 495
Asn Pro Asn Thr Gly Glu Lys Val Gly Glu Gly Thr Pro Thr Thr Lys
            500                 505                 510
Ile Thr Lys Gln Pro Val Asp Glu Ile Val His Tyr Gly Gly Glu Glu
        515                 520                 525
Ile Lys Pro Gly His Lys Asp Glu Phe Asp Pro Asn Ala Pro Lys Gly
    530                 535                 540
Ser Gln Thr Thr Gln Pro Gly Lys Pro Gly Val Lys Asn Pro Asp Thr
545                 550                 555                 560
Gly Glu Val Val Thr Pro Pro Val Asp Asp Val Thr Lys Tyr Gly Pro
                565                 570                 575
Val Asp Gly Asp Pro Ile Thr Ser Thr Glu Glu Ile Pro Phe Asp Lys
            580                 585                 590
Lys Arg Glu Phe Asn Pro Asp Leu Lys Pro Gly Glu Glu Arg Val Lys
        595                 600                 605
Gln Lys Gly Glu Pro Gly Thr Lys Thr Ile Thr Thr Pro Thr Thr Lys
    610                 615                 620
Asn Pro Leu Thr Gly Glu Lys Val Gly Glu Gly Glu Pro Thr Glu Lys
625                 630                 635                 640
Ile Thr Lys Gln Pro Val Asp Glu Ile Thr Glu Tyr Gly Gly Glu Glu
                645                 650                 655
Ile Lys Pro Gly His Lys Asp Glu Phe Asp Pro Asn Ala Pro Lys Gly
            660                 665                 670
Ser Gln Glu Asp Val Pro Gly Lys Pro Gly Val Lys Asn Pro Asp Thr
        675                 680                 685
Gly Glu Val Val Thr Pro Pro Val Asp Asp Val Thr Lys Tyr Gly Pro
    690                 695                 700
Val Asp Gly Asp Pro Ile Thr Ser Thr Glu Glu Ile Pro Phe Asp Lys
705                 710                 715                 720
Lys Arg Glu Phe Asp Pro Asn Leu Ala Pro Gly Thr Glu Lys Val Val
                725                 730                 735
Gln Lys Gly Glu Pro Gly Thr Lys Thr Ile Thr Thr Pro Thr Thr Lys
            740                 745                 750
Asn Pro Leu Thr Gly Glu Lys Val Gly Glu Gly Glu Pro Thr Glu Lys
        755                 760                 765
Ile Thr Lys Gln Pro Val Asp Glu Ile Val His Tyr Gly Gly Glu Glu
    770                 775                 780
Ile Lys Pro Gly His Lys Asp Glu Phe Asp Pro Asn Ala Pro Lys Gly
785                 790                 795                 800
Ser Gln Glu Asp Val Pro Gly Lys Pro Gly Val Lys Asn Pro Asp Thr
                805                 810                 815
Gly Glu Val Val Thr Pro Pro Val Asp Asp Val Thr Lys Tyr Gly Pro
            820                 825                 830
Val Asp Gly Asp Pro Ile Thr Ser Thr Glu Glu Ile Pro Phe Asp Lys
        835                 840                 845
Lys Arg Glu Phe Asn Pro Asp Leu Lys Pro Gly Glu Glu Arg Val Lys
    850                 855                 860
Gln Lys Gly Glu Pro Gly Thr Lys Thr Ile Thr Thr Pro Thr Thr Lys
865                 870                 875                 880
Asn Pro Leu Thr Gly Glu Lys Val Gly Glu Gly Glu Pro Thr Glu Lys
                885                 890                 895
Val Thr Lys Gln Pro Val Asp Glu Ile Val His Tyr Gly Gly Glu Glu
            900                 905                 910
Ile Lys Pro Gly His Lys Asp Glu Phe Asp Pro Asn Ala Pro Lys Gly
        915                 920                 925
Ser Gln Glu Asp Val Pro Gly Lys Pro Gly Val Lys Asn Pro Asp Thr
    930                 935                 940
Gly Glu Val Val Thr Pro Pro Val Asp Asp Val Thr Lys Tyr Gly Pro
945                 950                 955                 960
Val Asp Gly Asp Pro Ile Thr Ser Thr Glu Glu Ile Pro Phe Asp Lys

965                 970                 975
Lys Arg Glu Phe Asp Pro Asn Leu Ala Pro Gly Thr Glu Lys Val Val
            980                 985                 990
Gln Lys Gly Glu Pro Gly Thr Lys Thr Ile Thr Thr Pro Thr Thr Lys
        995                 1000                1005
Asn Pro Leu Thr Gly Glu Lys Val Gly Glu Gly Glu Pro Thr Glu Lys
    1010                1015                1020
Ile Thr Lys Gln Pro Val Asp Glu Ile Val His Tyr Gly Gly Glu Glu
1025                1030                1035                1040
Ile Lys Pro Gly His Lys Asp Glu Phe Asp Pro Asn Ala Pro Lys Gly
                1045                1050                1055
Ser Gln Thr Thr Gln Pro Gly Lys Pro Gly Val Lys Asn Pro Asp Thr
            1060                1065                1070
Gly Glu Val Val Thr Pro Pro Val Asp Asp Val Thr Lys Tyr Gly Pro
        1075                1080                1085
Val Asp Gly Asp Pro Ile Thr Ser Thr Glu Glu Ile Pro Phe Asp Lys
    1090                1095                1100
Lys Arg Glu Phe Asp Pro Asn Leu Ala Pro Gly Thr Glu Lys Val Val
1105                1110                1115                1120
Gln Lys Gly Glu Pro Gly Thr Lys Thr Ile Thr Thr Pro Thr Thr Lys
                1125                1130                1135
Asn Pro Leu Thr Gly Glu Lys Val Gly Glu Gly Glu Pro Thr Glu Lys
            1140                1145                1150
Ile Thr Lys Gln Pro Val Asp Glu Ile Val His Tyr Gly Gly Glu Gln
        1155                1160                1165
Ile Pro Gln Gly His Lys Asp Glu Phe Asp Pro Asn Ala Pro Val Asp
    1170                1175                1180
Ser Lys Thr Glu Val Pro Gly Lys Pro Gly Val Lys Asn Pro Asp Thr
1185                1190                1195                1200
Gly Glu Val Val Thr Pro Pro Val Asp Asp Val Thr Lys Tyr Gly Pro
                1205                1210                1215
Lys Val Gly Asn Pro Ile Thr Ser Thr Glu Glu Ile Pro Phe Asp Lys
            1220                1225                1230
Lys Arg Val Phe Asn Pro Asp Leu Lys Pro Gly Glu Glu Arg Val Lys
        1235                1240                1245
Gln Lys Gly Glu Pro Gly Thr Lys Thr Ile Thr Thr Pro Ile Leu Val
    1250                1255                1260
Asn Pro Ile Thr Gly Glu Lys Val Gly Glu Gly Lys Ser Thr Glu Lys
1265                1270                1275                1280
Val Thr Lys Gln Pro Val Asp Glu Ile Val Glu Tyr Gly Pro Thr Lys
                1285                1290                1295
Ala Glu Pro Gly Lys Pro Ala Glu Pro Gly Lys Pro Ala Glu Pro Gly
            1300                1305                1310
Lys Pro Ala Glu Pro Gly Lys Pro Ala Glu Pro Gly Thr Pro Ala Glu
        1315                1320                1325
Pro Gly Lys Pro Ala Glu Pro Gly Lys Pro Ala Glu Pro Gly Lys Pro
    1330                1335                1340
Ala Glu Pro Gly Lys Pro Ala Glu Pro Gly Lys Pro Ala Glu Pro Gly
1345                1350                1355                1360
Thr Pro Ala Glu Pro Gly Lys Pro Ala Glu Pro Gly Lys Pro Ala Glu
                1365                1370                1375
Pro Gly Lys Pro Ala Glu Pro Gly Thr Pro Ala Glu Pro Gly Lys Pro
            1380                1385                1390
Ala Glu Pro Gly Thr Pro Ala Glu Pro Gly Lys Pro Ala Glu Pro Gly
        1395                1400                1405
Thr Pro Thr Gln Ser Gly Ala Pro Glu Gln Pro Asn Arg Ser Met His
    1410                1415                1420
Ser Thr Asp Asn Lys Asn Gln Leu Pro Asp Thr Gly Glu Asn Arg Gln
1425                1430                1435                1440
Ala Asn Glu Gly Thr Leu Val Gly Ser Leu Leu Ala Ile Val Gly Ser
                1445                1450                1455
Leu Phe Ile Phe Gly Arg Arg Lys Lys Gly Asn Glu Lys
            1460                1465

<210> 18
<211> 167
<212> PRT
<213> Staphylococcus aureus

<400> 18
Met Lys Lys Leu Tyr Thr Ser Tyr Gly Thr Tyr Gly Phe Leu His Gln
1               5                   10                  15
Ile Lys Ile Asn Asn Pro Thr His Gln Leu Phe Gln Phe Ser Ala Ser
            20                  25                  30
Asp Thr Ser Val Ile Phe Glu Glu Thr Asp Gly Glu Thr Val Leu Lys
        35                  40                  45
Ser Pro Ser Ile Tyr Glu Val Ile Lys Glu Ile Gly Glu Phe Ser Glu
    50                  55                  60
His His Phe Tyr Cys Ala Ile Phe Ile Pro Ser Thr Glu Asp His Ala
65                  70                  75                  80
Tyr Gln Leu Glu Lys Lys Leu Ile Ser Val Asp Asp Asn Phe Arg Asn
                85                  90                  95
Phe Gly Gly Phe Lys Ser Tyr Arg Leu Leu Arg Pro Ala Lys Gly Thr
            100                 105                 110
Thr Tyr Lys Ile Tyr Phe Gly Phe Ala Asp Arg His Ala Tyr Glu Asp
        115                 120                 125
Phe Lys Gln Ser Asp Ala Phe Asn Asp His Phe Ser Lys Asp Ala Leu
    130                 135                 140
Ser His Tyr Phe Gly Ser Ser Gly Gln His Ser Ser Tyr Phe Glu Arg
145                 150                 155                 160
Tyr Leu Tyr Pro Ile Lys Glu
                165

<210> 19
<211> 167
<212> PRT
<213> Staphylococcus aureus

<400> 19
Met Tyr Leu Tyr Thr Ser Tyr Gly Thr Tyr Gln Phe Leu Asn Gln Ile
1               5                   10                  15
Lys Leu Asn His Gln Glu Arg Ser Leu Phe Gln Phe Ser Thr Asn Asp
            20                  25                  30
Ser Ser Ile Ile Leu Glu Glu Ser Glu Gly Lys Ser Ile Leu Lys His
        35                  40                  45
Pro Ser Ala Tyr Gln Val Ile Asp Ser Thr Gly Glu Phe Asn Glu His
    50                  55                  60
His Phe Tyr Ser Ala Ile Phe Val Pro Thr Ser Glu Asp His Arg Gln
65                  70                  75                  80
Gln Leu Glu Lys Lys Leu Leu Leu Val Asp Val Pro Leu Arg Asn Phe
                85                  90                  95
Gly Gly Phe Lys Ser Tyr Arg Leu Leu Lys Pro Thr Glu Gly Ser Thr
            100                 105                 110
Tyr Lys Ile Tyr Phe Gly Phe Ala Asn Arg Thr Ala Tyr Glu Asp Phe
        115                 120                 125
Lys Ala Ser Asp Ile Phe Asn Glu Asn Phe Ser Lys Asp Ala Leu Ser
    130                 135                 140
Gln Tyr Phe Gly Ala Ser Gly Gln His Ser Ser Tyr Phe Glu Arg Tyr
145                 150                 155                 160
Leu Tyr Pro Ile Glu Asp His
                165

<210> 20
<211> 1141
<212> PRT
<213> Staphylococcus aureus

<400> 20
Met Ile Asn Arg Asp Asn Lys Lys Ala Ile Thr Lys Lys Gly Met Ile
1               5                   10                  15
Ser Asn Arg Leu Asn Lys Phe Ser Ile Arg Lys Tyr Thr Val Gly Thr
            20                  25                  30
Ala Ser Ile Leu Val Gly Thr Thr Leu Ile Phe Gly Leu Gly Asn Gln
        35                  40                  45
Glu Ala Lys Ala Ala Glu Asn Thr Ser Thr Glu Asn Ala Lys Gln Asp
    50                  55                  60
Asp Ala Thr Thr Ser Asp Asn Lys Glu Val Val Ser Glu Thr Glu Asn
65                  70                  75                  80
Asn Ser Thr Thr Glu Asn Asp Ser Thr Asn Pro Ile Lys Lys Glu Thr
                85                  90                  95
Asn Thr Asp Ser Gln Pro Glu Ala Lys Glu Glu Ser Thr Thr Ser Ser
            100                 105                 110
Thr Gln Gln Gln Gln Asn Asn Val Thr Ala Thr Thr Glu Thr Lys Pro
        115                 120                 125
Gln Asn Ile Glu Lys Glu Asn Val Lys Pro Ser Thr Asp Lys Thr Ala
    130                 135                 140
Thr Glu Asp Thr Ser Val Ile Leu Glu Glu Lys Lys Ala Pro Asn Tyr
145                 150                 155                 160
Thr Asn Asn Asp Val Thr Thr Lys Pro Ser Thr Ser Glu Ile Gln Thr

                165                 170                 175
Lys Pro Thr Thr Pro Gln Glu Ser Thr Asn Ile Glu Asn Ser Gln Pro
            180                 185                 190
Gln Pro Thr Pro Ser Lys Val Asp Asn Gln Val Thr Asp Ala Thr Asn
        195                 200                 205
Pro Lys Glu Pro Val Asn Val Ser Lys Glu Glu Leu Lys Asn Asn Pro
    210                 215                 220
Glu Lys Leu Lys Glu Leu Val Arg Asn Asp Asn Asn Thr Asp Arg Ser
225                 230                 235                 240
Thr Lys Pro Val Ala Thr Ala Pro Thr Ser Val Ala Pro Lys Arg Leu
                245                 250                 255
Asn Ala Lys Met Arg Phe Ala Val Ala Gln Pro Ala Ala Val Ala Ser
            260                 265                 270
Asn Asn Val Asn Asp Leu Ile Thr Val Thr Lys Gln Thr Ile Lys Val
        275                 280                 285
Gly Asp Gly Lys Asp Asn Val Ala Ala Ala His Asp Gly Lys Asp Ile
    290                 295                 300
Glu Tyr Asp Thr Glu Phe Thr Ile Asp Asn Lys Val Lys Lys Gly Asp
305                 310                 315                 320
Thr Met Thr Ile Asn Tyr Asp Lys Asn Val Ile Pro Ser Asp Leu Thr
                325                 330                 335
Asp Lys Asn Asp Pro Ile Asp Ile Thr Asp Pro Ser Gly Glu Val Ile
            340                 345                 350
Ala Lys Gly Thr Phe Asp Lys Ala Thr Lys Gln Ile Thr Tyr Thr Phe
        355                 360                 365
Thr Asp Tyr Val Asp Lys Tyr Glu Asp Ile Lys Ala Arg Leu Thr Leu
    370                 375                 380
Tyr Ser Tyr Ile Asp Lys Gln Ala Val Pro Asn Glu Thr Ser Leu Asn
385                 390                 395                 400
Leu Thr Phe Ala Thr Ala Gly Lys Glu Thr Ser Gln Asn Val Ser Val
                405                 410                 415
Asp Tyr Gln Asp Pro Met Val His Gly Asp Ser Asn Ile Gln Ser Ile
            420                 425                 430
Phe Thr Lys Leu Asp Glu Asn Lys Gln Thr Ile Glu Gln Gln Ile Tyr
        435                 440                 445
Val Asn Pro Leu Lys Lys Thr Ala Thr Asn Thr Lys Val Asp Ile Ala
    450                 455                 460
Gly Ser Gln Val Asp Asp Tyr Gly Asn Ile Lys Leu Gly Asn Gly Ser
465                 470                 475                 480
Thr Ile Ile Asp Gln Asn Thr Glu Ile Lys Val Tyr Lys Val Asn Pro
                485                 490                 495
Asn Gln Gln Leu Pro Gln Ser Asn Arg Ile Tyr Asp Phe Ser Gln Tyr

                    500                 505                 510
Glu Asp Val Thr Ser Gln Phe Asp Asn Lys Lys Ser Phe Ser Asn Asn
        515                 520                 525
Val Ala Thr Leu Asp Phe Gly Asp Ile Asn Ser Ala Tyr Ile Ile Lys
    530                 535                 540
Val Val Ser Lys Tyr Thr Pro Thr Ser Asp Gly Glu Leu Asp Ile Ala
545                 550                 555                 560
Gln Gly Thr Ser Met Arg Thr Thr Asp Lys Tyr Gly Tyr Tyr Asn Tyr
                565                 570                 575
Ala Gly Tyr Ser Asn Phe Ile Val Thr Ser Asn Asp Thr Gly Gly Gly
            580                 585                 590
Asp Gly Thr Val Lys Pro Glu Glu Lys Leu Tyr Lys Ile Gly Asp Tyr
        595                 600                 605
Val Trp Glu Asp Val Asp Lys Asp Gly Val Gln Gly Thr Asp Ser Lys
    610                 615                 620
Glu Lys Pro Met Ala Asn Val Leu Val Thr Leu Thr Tyr Pro Asp Gly
625                 630                 635                 640
Thr Thr Lys Ser Val Arg Thr Asp Ala Asn Gly His Tyr Glu Phe Gly
                645                 650                 655
Gly Leu Lys Asp Gly Glu Thr Tyr Thr Val Lys Phe Glu Thr Pro Ala
            660                 665                 670
Gly Tyr Leu Pro Thr Lys Val Asn Gly Thr Thr Asp Gly Glu Lys Asp
        675                 680                 685
Ser Asn Gly Ser Ser Ile Thr Val Lys Ile Asn Gly Lys Asp Asp Met
    690                 695                 700
Ser Leu Asp Thr Gly Phe Tyr Lys Glu Pro Lys Tyr Asn Leu Gly Asp
705                 710                 715                 720
Tyr Val Trp Glu Asp Thr Asn Lys Asp Gly Ile Gln Asp Ala Asn Glu
                725                 730                 735
Pro Gly Ile Lys Asp Val Lys Val Thr Leu Lys Asp Ser Thr Gly Lys
            740                 745                 750
Val Ile Gly Thr Thr Thr Thr Asp Ala Ser Gly Lys Tyr Lys Phe Thr
        755                 760                 765
Asp Leu Asp Asn Gly Asn Tyr Thr Val Glu Phe Glu Thr Pro Ala Gly
    770                 775                 780
Tyr Thr Pro Thr Val Lys Asn Thr Thr Ala Glu Asp Lys Asp Ser Asn
785                 790                 795                 800
Gly Leu Thr Thr Thr Gly Val Ile Lys Asp Ala Asp Asn Met Thr Leu
                805                 810                 815
Asp Ser Gly Phe Tyr Lys Thr Pro Lys Tyr Ser Leu Gly Asp Tyr Val
            820                 825                 830
Trp Tyr Asp Ser Asn Lys Asp Gly Lys Gln Asp Ser Thr Glu Lys Gly
        835                 840                 845
Ile Lys Asp Val Lys Val Thr Leu Leu Asn Glu Lys Gly Glu Val Ile
    850                 855                 860
Gly Thr Thr Lys Thr Asp Glu Asn Gly Lys Tyr Arg Phe Asp Asn Leu
865                 870                 875                 880
Asp Ser Gly Lys Tyr Lys Val Ile Phe Glu Lys Pro Ala Gly Leu Thr
                885                 890                 895
Gln Thr Val Thr Asn Thr Thr Glu Asp Asp Lys Asp Ala Asp Gly Gly
            900                 905                 910
Glu Val Asp Val Thr Ile Thr Asp His Asp Asp Phe Ile Leu Asp Asn
        915                 920                 925
Gly Tyr Phe Glu Glu Asp Thr Ser Asp Ser Asp Ser Asp Ser Asp Ser
    930                 935                 940
Asp Ser Asp Ser Asp Ser Asp Ser Asp Ser Asp Ser Asp Ser Asp Ser
945                 950                 955                 960
Asp Ser Asp Ser Asp Ser Asp Ser Asp Ser Asp Ser Asp Ser Asp Ser
                965                 970                 975
Asp Ser Asp Ser Asp Ser Asp Ser Asp Ser Asp Ser Asp Ser Asp Ser
            980                 985                 990
Asp Ser Asp Ser Asp Ser Asp Ser Asp Ser Asp Ser Asp Ser Asp Ser
        995                 1000                1005
Asp Ser Asp Ser Asp Ser Asp Ser Asp Ser Asp Ser Asp Ser Asp Ser
    1010                1015                1020

Asp Ser Asp Ser Asp Ser Asp Ser Asp Ser Asp Ser Asp Ser Asp Ser
1025                1030                1035                1040
Asp Ser Asp Ser Asp Ser Asp Ser Asp Ser Asp Ser Asp Ser Asp Ser
                1045                1050                1055
Asp Ser Asp Ser Asp Ser Asp Ser Asp Ser Asp Ser Asp Ser Asp Ser
            1060                1065                1070
Asp Ser Asp Ser Asp Ser Asp Ser Asp Ala Gly Lys His Thr Pro Val
        1075                1080                1085
Lys Pro Met Ser Thr Thr Lys Asp His His Asn Lys Ala Lys Ala Leu
    1090                1095                1100
Pro Glu Thr Gly Ser Glu Asn Asn Gly Ser Asn Asn Ala Thr Leu Phe
1105                1110                1115                1120
Gly Gly Leu Phe Ala Ala Leu Gly Ser Leu Leu Leu Phe Gly Arg Arg
                1125                1130                1135
Lys Lys Gln Asn Lys
            1140

<210> 21
<211> 1056
<212> PRT
<213> Staphylococcus aureus

<400> 21
Met Ile Asn Lys Lys Asn Asn Leu Leu Thr Lys Lys Lys Pro Ile Ala
1               5                   10                  15
Asn Lys Ser Asn Lys Tyr Ala Ile Arg Lys Phe Thr Val Gly Thr Ala
            20                  25                  30
Ser Ile Val Ile Gly Ala Thr Leu Leu Phe Gly Leu Gly His Asn Glu
        35                  40                  45
Ala Lys Ala Glu Glu Asn Ser Val Gln Asp Val Lys Asp Ser Asn Thr
    50                  55                  60
Asp Asp Glu Leu Ser Asp Ser Asn Asp Gln Ser Ser Asp Glu Glu Lys
65                  70                  75                  80
Asn Asp Val Ile Asn Asn Asn Gln Ser Ile Asn Thr Asp Asp Asn Asn
                85                  90                  95
Gln Ile Ile Lys Lys Glu Glu Thr Asn Asn Tyr Asp Gly Ile Glu Lys
            100                 105                 110
Arg Ser Glu Asp Arg Thr Glu Ser Thr Thr Asn Val Asp Glu Asn Glu
        115                 120                 125
Ala Thr Phe Leu Gln Lys Thr Pro Gln Asp Asn Thr His Leu Thr Glu
    130                 135                 140
Glu Glu Val Lys Glu Ser Ser Ser Val Glu Ser Ser Asn Ser Ser Ile
145                 150                 155                 160
Asp Thr Ala Gln Gln Pro Ser His Thr Thr Ile Asn Arg Glu Glu Ser
                165                 170                 175
Val Gln Thr Ser Asp Asn Val Glu Asp Ser His Val Ser Asp Phe Ala
            180                 185                 190
Asn Ser Lys Ile Lys Glu Ser Asn Thr Glu Ser Gly Lys Glu Glu Asn
        195                 200                 205
Thr Ile Glu Gln Pro Asn Lys Val Lys Glu Asp Ser Thr Thr Ser Gln
    210                 215                 220
Pro Ser Gly Tyr Thr Asn Ile Asp Glu Lys Ile Ser Asn Gln Asp Glu
225                 230                 235                 240
Leu Leu Asn Leu Pro Ile Asn Glu Tyr Glu Asn Lys Ala Arg Pro Leu
                245                 250                 255
Ser Thr Thr Ser Ala Gln Pro Ser Ile Lys Arg Val Thr Val Asn Gln
            260                 265                 270
Leu Ala Ala Glu Gln Gly Ser Asn Val Asn His Leu Ile Lys Val Thr
        275                 280                 285
Asp Gln Ser Ile Thr Glu Gly Tyr Asp Asp Ser Glu Gly Val Ile Lys
    290                 295                 300
Ala His Asp Ala Glu Asn Leu Ile Tyr Asp Val Thr Phe Glu Val Asp
305                 310                 315                 320
Asp Lys Val Lys Ser Gly Asp Thr Met Thr Val Asp Ile Asp Lys Asn

                325                 330                 335
Thr Val Pro Ser Asp Leu Thr Asp Ser Phe Thr Ile Pro Lys Ile Lys
            340                 345                 350
Asp Asn Ser Gly Glu Ile Ile Ala Thr Gly Thr Tyr Asp Asn Lys Asn
        355                 360                 365
Lys Gln Ile Thr Tyr Thr Phe Thr Asp Tyr Val Asp Lys Tyr Glu Asn
    370                 375                 380
Ile Lys Ala His Leu Lys Leu Thr Ser Tyr Ile Asp Lys Ser Lys Val
385                 390                 395                 400
Pro Asn Asn Asn Thr Lys Leu Asp Val Glu Tyr Lys Thr Ala Leu Ser
                405                 410                 415
Ser Val Asn Lys Thr Ile Thr Val Glu Tyr Gln Arg Pro Asn Glu Asn
            420                 425                 430
Arg Thr Ala Asn Leu Gln Ser Met Phe Thr Asn Ile Asp Thr Lys Asn
        435                 440                 445
His Thr Val Glu Gln Thr Ile Tyr Ile Asn Pro Leu Arg Tyr Ser Ala
    450                 455                 460
Lys Glu Thr Asn Val Asn Ile Ser Gly Asn Gly Asp Glu Gly Ser Thr
465                 470                 475                 480
Ile Ile Asp Asp Ser Thr Ile Ile Lys Val Tyr Lys Val Gly Asp Asn
                485                 490                 495
Gln Asn Leu Pro Asp Ser Asn Arg Ile Tyr Asp Tyr Ser Glu Tyr Glu
            500                 505                 510
Asp Val Thr Asn Asp Asp Tyr Ala Gln Leu Gly Asn Asn Asn Asp Val
        515                 520                 525
Asn Ile Asn Phe Gly Asn Ile Asp Ser Pro Tyr Ile Ile Lys Val Ile
    530                 535                 540
Ser Lys Tyr Asp Pro Asn Lys Asp Asp Tyr Thr Thr Ile Gln Gln Thr
545                 550                 555                 560
Val Thr Met Gln Thr Thr Ile Asn Glu Tyr Thr Gly Glu Phe Arg Thr
                565                 570                 575
Ala Ser Tyr Asp Asn Thr Ile Ala Phe Ser Thr Ser Ser Gly Gln Gly
            580                 585                 590
Gln Gly Asp Leu Pro Pro Glu Lys Thr Tyr Lys Ile Gly Asp Tyr Val
        595                 600                 605
Trp Glu Asp Val Asp Lys Asp Gly Ile Gln Asn Thr Asn Asp Asn Glu
    610                 615                 620
Lys Pro Leu Ser Asn Val Leu Val Thr Leu Thr Tyr Pro Asp Gly Thr
625                 630                 635                 640
Ser Lys Ser Val Arg Thr Asp Glu Asp Gly Lys Tyr Gln Phe Asp Gly
                645                 650                 655
Leu Lys Asn Gly Leu Thr Tyr Lys Ile Thr Phe Glu Thr Pro Glu Gly
            660                 665                 670
Tyr Thr Pro Thr Leu Lys His Ser Gly Thr Asn Pro Ala Leu Asp Ser
        675                 680                 685
Glu Gly Asn Ser Val Trp Val Thr Ile Asn Gly Gln Asp Asp Met Thr
    690                 695                 700
Ile Asp Ser Gly Phe Tyr Gln Thr Pro Lys Tyr Ser Leu Gly Asn Tyr
705                 710                 715                 720
Val Trp Tyr Asp Thr Asn Lys Asp Gly Ile Gln Gly Asp Asp Glu Lys
                725                 730                 735
Gly Ile Ser Gly Val Lys Val Thr Leu Lys Asp Glu Asn Gly Asn Ile
            740                 745                 750
Ile Ser Thr Thr Thr Thr Asp Glu Asn Gly Lys Tyr Gln Phe Asp Asn
        755                 760                 765
Leu Asn Ser Gly Asn Tyr Ile Val His Phe Asp Lys Pro Ser Gly Met
    770                 775                 780
Thr Gln Thr Thr Thr Asp Ser Gly Asp Asp Asp Glu Gln Asp Ala Asp
785                 790                 795                 800
Gly Glu Glu Val His Val Thr Ile Thr Asp His Asp Asp Phe Ser Ile
                805                 810                 815
Asp Asn Gly Tyr Tyr Asp Asp Glu Ser Asp Ser Asp Ser Asp Ser Asp
            820                 825                 830
Ser Asp Ser Asp Ser Asp Ser Asp Ser Asp Ser Asp Ser Asp Ser Asp
        835                 840                 845

Ser Asp Ser Asp Ser Asp Ser Asp Ser Asp Ser Asp Ser Asp Ser Asp
    850                 855                 860
Ser Asp Ser Asp Ser Asp Ser Asp Ser Asp Ser Asp Ser Asp Ser Asp
865                 870                 875                 880
Ser Asp Ser Asp Ser Asp Ser Asp Ser Asp Ser Asp Ser Asp Ser Asp
                885                 890                 895
Ser Asp Ser Asp Ser Asp Ser Asp Ser Asp Ser Asp Ser Asp Ser Asp
            900                 905                 910
Ser Asp Ser Asp Ser Asp Ser Asp Ser Asp Ser Asp Ser Asp Ser Asp
        915                 920                 925
Ser Asp Ser Asp Ser Asp Ser Asp Ser Asp Ser Asp Ser Asp Ser Asp
    930                 935                 940
Ser Asp Ser Asp Ser Asp Ser Asp Ser Asp Ser Asp Ser Asp Ser Asp
945                 950                 955                 960
Ser Asp Ser Asp Ser Asp Ser Asp Ser Asp Ser Asp Ser Asp Ser Asp
                965                 970                 975
Ser Asp Ser Asp Ser Asp Ser Asp Ser Asp Ser Asp Ser Asp Ser Asp
            980                 985                 990
Ser Asp Ser Asp Ser Asp Ser Asp Asn Asp Ser Asp Leu Gly Asn Ser
        995                 1000                1005
Ser Asp Lys Ser Thr Lys Asp Lys Leu Pro Asp Thr Gly Ala Asn Glu
    1010                1015                1020
Asp Tyr Gly Ser Lys Gly Thr Leu Leu Gly Thr Leu Phe Ala Gly Leu
1025                1030                1035                1040
Gly Ala Leu Leu Leu Gly Lys Arg Arg Lys Asn Arg Lys Asn Lys Asn
                1045                1050                1055


<210> 22
<211> 486
<212> PRT
<213> Staphylococcus aureus

<400> 22
Met Ser Asn Asn Phe Lys Asp Asp Phe Glu Lys Asn Arg Gln Ser Ile
 1               5                  10                  15
Asp Thr Asn Ser His Gln Asp His Thr Glu Asp Val Glu Lys Asp Gln
            20                  25                  30
Ser Glu Leu Glu His Gln Asp Thr Ile Glu Asn Thr Glu Gln Gln Phe
        35                  40                  45
Pro Pro Arg Asn Ala Gln Arg Arg Lys Arg Arg Arg Asp Leu Ala Thr
    50                  55                  60
Asn His Asn Lys Gln Val His Asn Glu Ser Gln Thr Ser Glu Asp Asn
65                  70                  75                  80
Val Gln Asn Glu Ala Gly Thr Ile Asp Asp Arg Gln Val Glu Ser Ser
                85                  90                  95
His Ser Thr Glu Ser Gln Glu Pro Ser His Gln Asp Ser Thr Pro Gln
            100                 105                 110
His Glu Glu Glu Tyr Tyr Asn Lys Asn Ala Phe Ala Met Asp Lys Ser
        115                 120                 125
His Pro Glu Pro Ile Glu Asp Asn Asp Lys His Glu Thr Ile Lys Asp
    130                 135                 140
Ala Glu Asn Asn Thr Glu His Ser Thr Val Ser Asp Lys Ser Ile Ala
145                 150                 155                 160
Glu Gln Ser Gln Gln Pro Lys Pro Tyr Phe Ala Thr Gly Ala Asn Gln
                165                 170                 175
Ala Asn Thr Ser Lys Asp Lys His Asp Asp Val Thr Val Lys Gln Asp
            180                 185                 190
Lys Asp Glu Ser Lys Asp His His Ser Gly Lys Lys Gly Ala Ala Ile
        195                 200                 205
Gly Ala Gly Thr Ala Gly Val Ala Gly Ala Ala Gly Ala Met Gly Val
    210                 215                 220
Ser Lys Ala Lys Lys His Ser Asn Asp Ala Gln Asn Lys Ser Asn Ser
225                 230                 235                 240
Asp Lys Ser Asn Asn Ser Thr Glu Asp Lys Ala Ser Gln Asp Lys Ser

245                 250                 255
Lys Asp His His Asn Gly Lys Lys Gly Ala Ala Ile Gly Ala Gly Thr
            260                 265                 270
Ala Gly Leu Ala Gly Gly Ala Ala Ser Lys Ser Ala Ser Ala Ala Ser
        275                 280                 285
Lys Pro His Ala Ser Asn Asn Ala Ser Gln Asn His Asp Glu His Asp
    290                 295                 300
Asn His Asp Arg Asp Lys Glu Arg Lys Lys Gly Gly Met Ala Lys Val
305                 310                 315                 320
Leu Leu Pro Leu Ile Ala Ala Val Leu Ile Ile Gly Ala Leu Ala Ile
                325                 330                 335
Phe Gly Gly Met Ala Leu Asn Asn His Asn Asn Gly Thr Lys Glu Asn
            340                 345                 350
Lys Ile Ala Asn Thr Asn Lys Asn Asn Ala Asp Glu Ser Lys Asp Lys
        355                 360                 365
Asp Thr Ser Lys Asp Ala Ser Lys Asp Lys Ser Lys Ser Thr Asp Ser
    370                 375                 380
Asp Lys Ser Lys Glu Asp Gln Asp Lys Ala Thr Lys Asp Glu Ser Asp
385                 390                 395                 400
Asn Asp Gln Asn Asn Ala Asn Gln Ala Asn Asn Gln Ala Gln Asn Asn
                405                 410                 415
Gln Asn Gln Gln Gln Ala Asn Gln Asn Gln Gln Gln Gln Gln Gln Arg
            420                 425                 430
Gln Gly Gly Gly Gln Arg His Thr Val Asn Gly Gln Glu Asn Leu Tyr
        435                 440                 445
Arg Ile Ala Ile Gln Tyr Tyr Gly Ser Gly Ser Pro Glu Asn Val Glu
    450                 455                 460
Lys Ile Arg Arg Ala Asn Gly Leu Ser Gly Asn Asn Ile Arg Asn Gly
465                 470                 475                 480
Gln Gln Ile Val Ile Pro
                485

<210> 23
<211> 472
<212> PRT
<213> Staphylococcus aureus

<400> 23
Met Ile Glu Leu Ile Lys Met Glu Gly Met Ile Val Val Ser Asn Asn
1               5                   10                  15
Asn Phe Lys Asp Asp Phe Glu Lys Asn Arg Gln Ser Ile Asn Pro Asp
            20                  25                  30
Glu Gln Gln Thr Glu Leu Lys Glu Asp Asp Lys Thr Asn Glu Asn Lys
        35                  40                  45
Lys Glu Ala Asp Ser Gln Asn Ser Leu Ser Asn Asn Ser Asn Gln Gln
    50                  55                  60
Phe Pro Pro Arg Asn Ala Gln Arg Arg Lys Arg Arg Arg Glu Thr Ala
65                  70                  75                  80
Thr Asn Gln Ser Lys Gln Gln Asp Asp Lys His Gln Lys Asn Ser Asp
                85                  90                  95
Ala Lys Thr Thr Glu Gly Ser Leu Asp Asp Arg Tyr Asp Glu Ala Gln
            100                 105                 110
Leu Gln Gln Gln His Asp Lys Ser Gln Gln Gln Asn Lys Thr Glu Lys
        115                 120                 125
Gln Ser Gln Asp Asn Arg Met Lys Asp Gly Lys Asp Ala Ala Ile Val
    130                 135                 140
Asn Gly Thr Ser Glu Ser Pro Glu His Lys Ser Lys Ser Thr Gln Asn
145                 150                 155                 160
Arg Pro Gly Pro Lys Ala Gln Gln Gln Lys Arg Lys Ser Glu Ser Thr
                165                 170                 175
Gln Ser Lys Pro Ser Thr Asn Lys Asp Lys Lys Ala Ala Thr Gly Ala
            180                 185                 190
Gly Ile Ala Gly Ala Ala Gly Val Ala Gly Ala Ala Glu Thr Ser Lys
        195                 200                 205

Arg His His Asn Lys Lys Asp Lys Gln Asp Ser Lys His Ser Asn His
    210                 215                 220
Glu Asn Asp Glu Lys Ser Val Lys Asn Asp Asp Gln Lys Gln Ser Lys
225                 230                 235                 240
Lys Gly Lys Lys Ala Ala Val Gly Ala Gly Ala Ala Ala Gly Val Gly
                245                 250                 255
Ala Ala Gly Val Ala His His Asn Asn Gln Asn Lys His His Asn Glu
            260                 265                 270
Glu Lys Asn Ser Asn Gln Asn Asn Gln Tyr Asn Asp Gln Ser Glu Gly
        275                 280                 285
Lys Lys Lys Gly Gly Phe Met Lys Ile Leu Leu Pro Leu Ile Ala Ala
    290                 295                 300

Ile Leu Ile Leu Gly Ala Ile Ala Ile Phe Gly Gly Met Ala Leu Asn
305                 310                 315                 320
Asn His Asn Asp Ser Lys Ser Asp Asp Gln Lys Ile Ala Asn Gln Ser
                325                 330                 335
Lys Lys Asp Ser Asp Lys Lys Asp Gly Ala Gln Ser Glu Asp Asn Lys
            340                 345                 350
Asp Lys Lys Ser Asp Ser Asn Lys Asp Lys Lys Ser Asp Ser Asp Lys
        355                 360                 365
Asn Ala Asp Asp Asp Ser Asp Asn Ser Ser Ser Asn Pro Asn Ala Thr
    370                 375                 380
Ser Thr Asn Asn Asn Asp Asn Val Ala Asn Asn Asn Ser Asn Tyr Thr
385                 390                 395                 400
Asn Gln Asn Gln Gln Asp Asn Ala Asn Gln Asn Ser Asn Asn Gln Gln
                405                 410                 415
Ala Thr Gln Gly Gln Gln Ser His Thr Val Tyr Gly Gln Glu Asn Leu
            420                 425                 430
Tyr Arg Ile Ala Ile Gln Tyr Tyr Gly Glu Gly Thr Gln Ala Asn Val
        435                 440                 445
Asp Lys Ile Lys Arg Ala Asn Gly Leu Ser Ser Asn Asn Ile His Asn
    450                 455                 460
Gly Gln Thr Leu Val Ile Pro Gln
465                 470


<210> 24
<211> 165
<212> PRT
<213> Staphylococcus aureus

<400> 24
Met Lys Asn Lys Leu Ile Ala Lys Ser Leu Leu Thr Ile Ala Ala Ile
 1               5                  10                  15
Gly Ile Thr Thr Thr Thr Ile Ala Ser Thr Ala Asp Ala Ser Glu Gly
            20                  25                  30
Tyr Gly Pro Arg Glu Lys Lys Pro Val Ser Ile Asn His Asn Ile Val
        35                  40                  45
Glu Tyr Asn Asp Gly Thr Phe Lys Tyr Gln Ser Arg Pro Lys Phe Asn
    50                  55                  60
Ser Thr Pro Lys Tyr Ile Lys Phe Lys His Asp Tyr Asn Ile Leu Glu
65                  70                  75                  80
Phe Asn Asp Gly Thr Phe Glu Tyr Gly Ala Arg Pro Gln Phe Asn Lys
                85                  90                  95
Pro Ala Ala Lys Thr Asp Ala Thr Ile Lys Lys Glu Gln Lys Leu Ile
            100                 105                 110
Gln Ala Gln Asn Leu Val Arg Glu Phe Glu Lys Thr His Thr Val Ser
        115                 120                 125
Ala His Arg Lys Ala Gln Lys Ala Val Asn Leu Val Ser Phe Glu Tyr

    130                 135                 140
Lys Val Lys Lys Met Val Leu Gln Glu Arg Ile Asp Asn Val Leu Lys
145                 150                 155                 160
Gln Gly Leu Val Arg

                165

<210> 25
<211> 319
<212> PRT
<213> Staphylococcus aureus

<400> 25
Met Lys Thr Arg Ile Val Ser Ser Val Thr Thr Thr Leu Leu Leu Gly
1               5                   10                  15
Ser Ile Leu Met Asn Pro Val Ala Asn Ala Ala Asp Ser Asp Ile Asn
            20                  25                  30
Ile Lys Thr Gly Thr Thr Asp Ile Gly Ser Asn Thr Thr Val Lys Thr
        35                  40                  45
Gly Asp Leu Val Thr Tyr Asp Lys Glu Asn Gly Met His Lys Lys Val

    50                  55                  60
Phe Tyr Ser Phe Ile Asp Asp Lys Asn His Asn Lys Lys Leu Leu Val
65                  70                  75                  80
Ile Arg Thr Lys Gly Thr Ile Ala Gly Gln Tyr Arg Val Tyr Ser Glu
                85                  90                  95
Glu Gly Ala Asn Lys Ser Gly Leu Ala Trp Pro Ser Ala Phe Lys Val
            100                 105                 110
Gln Leu Gln Leu Pro Asp Asn Glu Val Ala Gln Ile Ser Asp Tyr Tyr
        115                 120                 125
Pro Arg Asn Ser Ile Asp Thr Lys Glu Tyr Met Ser Thr Leu Thr Tyr
    130                 135                 140
Gly Phe Asn Gly Asn Val Thr Gly Asp Asp Thr Gly Lys Ile Gly Gly
145                 150                 155                 160
Leu Ile Gly Ala Asn Val Ser Ile Gly His Thr Leu Lys Tyr Val Gln
                165                 170                 175
Pro Asp Phe Lys Thr Ile Leu Glu Ser Pro Thr Asp Lys Lys Val Gly
            180                 185                 190
Trp Lys Val Ile Phe Asn Asn Met Val Asn Gln Asn Trp Gly Pro Tyr
        195                 200                 205
Asp Arg Asp Ser Trp Asn Pro Val Tyr Gly Asn Gln Leu Phe Met Lys
    210                 215                 220
Thr Arg Asn Gly Ser Met Lys Ala Ala Glu Asn Phe Leu Asp Pro Asn
225                 230                 235                 240
Lys Ala Ser Ser Leu Leu Ser Ser Gly Phe Ser Pro Asp Phe Ala Thr
                245                 250                 255
Val Ile Thr Met Asp Arg Lys Ala Ser Lys Gln Gln Thr Asn Ile Asp
            260                 265                 270
Val Ile Tyr Glu Arg Val Arg Asp Asp Tyr Gln Leu His Trp Thr Ser
        275                 280                 285
Thr Asn Trp Lys Gly Thr Asn Thr Lys Asp Lys Trp Thr Asp Arg Ser
    290                 295                 300
Ser Glu Arg Tyr Lys Ile Asp Trp Glu Lys Glu Glu Met Thr Asn
305                 310                 315

<210> 26
<211> 428
<212> PRT
<213> Staphylococcus aureus

<400> 26
Met His Met Lys Asn Lys Tyr Ile Ser Lys Leu Leu Val Gly Ala Ala
1               5                   10                  15
Thr Ile Thr Leu Ala Thr Met Ile Ser Asn Gly Glu Ala Lys Ala Ser
            20                  25                  30
Glu Asn Thr Gln Gln Thr Ser Thr Lys His Gln Thr Thr Gln Asn Asn
        35                  40                  45
Tyr Val Thr Asp Gln Gln Lys Ala Phe Tyr Gln Val Leu His Leu Lys

50                  55                  60
Gly Ile Thr Glu Glu Gln Arg Asn Gln Tyr Ile Lys Thr Leu Arg Glu
65                  70                  75                  80
His Pro Glu Arg Ala Gln Glu Val Phe Ser Glu Ser Leu Lys Asp Ser
                85                  90                  95
Lys Asn Pro Asp Arg Arg Val Ala Gln Gln Asn Ala Phe Tyr Asn Val
            100                 105                 110
Leu Lys Asn Asp Asn Leu Thr Glu Gln Glu Lys Asn Asn Tyr Ile Ala
        115                 120                 125
Gln Ile Lys Glu Asn Pro Asp Arg Ser Gln Gln Val Trp Val Glu Ser
    130                 135                 140
Val Gln Ser Ser Lys Ala Lys Glu Arg Gln Asn Ile Glu Asn Ala Asp
145                 150                 155                 160
Lys Ala Ile Lys Asp Phe Gln Asp Asn Lys Ala Pro His Asp Lys Ser
                165                 170                 175
Ala Ala Tyr Glu Ala Asn Ser Lys Leu Pro Lys Asp Leu Arg Asp Lys
            180                 185                 190
Asn Asn Arg Phe Val Glu Lys Val Ser Ile Glu Lys Ala Ile Val Arg
        195                 200                 205
His Asp Glu Arg Val Lys Ser Ala Asn Asp Ala Ile Ser Lys Leu Asn
    210                 215                 220
Glu Lys Asp Ser Ile Glu Asn Arg Arg Leu Ala Gln Arg Glu Val Asn
225                 230                 235                 240
Lys Ala Pro Met Asp Val Lys Glu His Leu Gln Lys Gln Leu Asp Ala
                245                 250                 255
Leu Val Ala Gln Lys Asp Ala Glu Lys Lys Val Ala Pro Lys Val Glu
            260                 265                 270
Ala Pro Gln Ile Gln Ser Pro Gln Ile Glu Lys Pro Lys Ala Glu Ser
        275                 280                 285
Pro Lys Val Glu Val Pro Gln Ser Lys Leu Leu Gly Tyr Tyr Gln Ser
    290                 295                 300
Leu Lys Asp Ser Phe Asn Tyr Gly Tyr Lys Tyr Leu Thr Asp Thr Tyr
305                 310                 315                 320
Lys Ser Tyr Lys Glu Lys Tyr Asp Thr Ala Lys Tyr Tyr Tyr Asn Thr
                325                 330                 335
Tyr Tyr Lys Tyr Lys Gly Ala Ile Asp Gln Thr Val Leu Thr Val Leu
            340                 345                 350
Gly Ser Gly Ser Lys Ser Tyr Ile Gln Pro Leu Lys Val Asp Asp Lys
        355                 360                 365
Asn Gly Tyr Leu Ala Lys Ser Tyr Ala Gln Val Arg Asn Tyr Val Thr
    370                 375                 380
Glu Ser Ile Asn Thr Gly Lys Val Leu Tyr Thr Phe Tyr Gln Asn Pro
385                 390                 395                 400
Thr Leu Val Lys Thr Ala Ile Lys Ala Gln Glu Thr Ala Ser Ser Ile
                405                 410                 415
Lys Asn Thr Leu Ser Asn Leu Leu Ser Phe Trp Lys
            420                 425

<210> 27
<211> 350
<212> PRT
<213> Staphylococcus aureus

<400> 27
Met Thr Lys His Tyr Leu Asn Ser Lys Tyr Gln Ser Glu Gln Arg Ser
1               5                   10                  15
Ser Ala Met Lys Lys Ile Thr Met Gly Thr Ala Ser Ile Ile Leu Gly
            20                  25                  30
Ser Leu Val Tyr Ile Gly Ala Asp Ser Gln Gln Val Asn Ala Ala Thr
        35                  40                  45
Glu Ala Thr Asn Ala Thr Asn Asn Gln Ser Thr Gln Val Ser Gln Ala
    50                  55                  60
Thr Ser Gln Pro Ile Asn Phe Gln Val Gln Lys Asp Gly Ser Ser Glu
65                  70                  75                  80

Lys Ser His Met Asp Asp Tyr Met Gln His Pro Gly Lys Val Ile Lys
                85                  90                  95
Gln Asn Asn Lys Tyr Tyr Phe Gln Thr Val Leu Asn Asn Ala Ser Phe
            100                 105                 110
Trp Lys Glu Tyr Lys Phe Tyr Asn Ala Asn Asn Gln Glu Leu Ala Thr
        115                 120                 125
Thr Val Val Asn Asp Asn Lys Lys Ala Asp Thr Arg Thr Ile Asn Val

    130                 135                 140
Ala Val Glu Pro Gly Tyr Lys Ser Leu Thr Thr Lys Val His Ile Val
145                 150                 155                 160
Val Pro Gln Ile Asn Tyr Asn His Arg Tyr Thr Thr His Leu Glu Phe
                165                 170                 175
Glu Lys Ala Ile Pro Thr Leu Ala Asp Ala Ala Lys Pro Asn Asn Val
            180                 185                 190
Lys Pro Val Gln Pro Lys Pro Ala Gln Pro Lys Thr Pro Thr Glu Gln
        195                 200                 205
Thr Lys Pro Val Gln Pro Lys Val Glu Lys Val Lys Pro Thr Val Thr
    210                 215                 220
Thr Thr Ser Lys Val Glu Asp Asn His Ser Thr Lys Val Val Ser Thr
225                 230                 235                 240
Asp Thr Thr Lys Asp Gln Thr Lys Thr Gln Thr Ala His Thr Val Lys
                245                 250                 255
Thr Ala Gln Thr Ala Gln Glu Gln Asn Lys Val Gln Thr Pro Val Lys
            260                 265                 270
Asp Val Ala Thr Ala Lys Ser Glu Ser Asn Asn Gln Ala Val Ser Asp
        275                 280                 285
Asn Lys Ser Gln Gln Thr Asn Lys Val Thr Lys His Asn Glu Thr Pro
    290                 295                 300
Lys Gln Ala Ser Lys Ala Lys Glu Leu Pro Lys Thr Gly Leu Thr Ser
305                 310                 315                 320
Val Asp Asn Phe Ile Ser Thr Val Ala Phe Ala Thr Leu Ala Leu Leu
                325                 330                 335
Gly Ser Leu Ser Leu Leu Leu Phe Lys Arg Lys Glu Ser Lys
            340                 345                 350


<210> 28
<211> 645
<212> PRT
<213> Staphylococcus aureus

<400> 28
Met Asn Lys Gln Gln Lys Glu Phe Lys Ser Phe Tyr Ser Ile Arg Lys
1               5                   10                  15
Ser Ser Leu Gly Val Ala Ser Val Ala Ile Ser Thr Leu Leu Leu Leu
            20                  25                  30
Met Ser Asn Gly Glu Ala Gln Ala Ala Ala Glu Glu Thr Gly Gly Thr
        35                  40                  45
Asn Thr Glu Ala Gln Pro Lys Thr Glu Ala Val Ala Ser Pro Thr Thr
    50                  55                  60
Thr Ser Glu Lys Ala Pro Glu Thr Lys Pro Val Ala Asn Ala Val Ser
65                  70                  75                  80
Val Ser Asn Lys Glu Val Glu Ala Pro Thr Ser Glu Thr Lys Glu Ala
                85                  90                  95
Lys Glu Val Lys Glu Val Lys Ala Pro Lys Glu Thr Lys Ala Val Lys
            100                 105                 110
Pro Ala Ala Lys Ala Thr Asn Asn Thr Tyr Pro Ile Leu Asn Gln Glu
        115                 120                 125
Leu Arg Glu Ala Ile Lys Asn Pro Ala Ile Lys Asp Lys Asp His Ser
    130                 135                 140
Ala Pro Asn Ser Arg Pro Ile Asp Phe Glu Met Lys Lys Glu Asn Gly
145                 150                 155                 160
Glu Gln Gln Phe Tyr His Tyr Ala Ser Ser Val Lys Pro Ala Arg Val
                165                 170                 175

Ile Phe Thr Asp Ser Lys Pro Glu Ile Glu Leu Gly Leu Gln Ser Gly
            180                 185                 190
Gln Phe Trp Arg Lys Phe Glu Val Tyr Glu Gly Asp Lys Lys Leu Pro
        195                 200                 205
Ile Lys Leu Val Ser Tyr Asp Thr Val Lys Asp Tyr Ala Tyr Ile Arg
    210                 215                 220
Phe Ser Val Ser Asn Gly Thr Lys Ala Val Lys Ile Val Ser Ser Thr
225                 230                 235                 240
His Phe Asn Asn Lys Glu Glu Lys Tyr Asp Tyr Thr Leu Met Glu Phe
                245                 250                 255
Ala Gln Pro Ile Tyr Asn Ser Ala Asp Lys Phe Lys Thr Glu Glu Asp
            260                 265                 270
Tyr Lys Ala Glu Lys Leu Leu Ala Pro Tyr Lys Lys Ala Lys Thr Leu
        275                 280                 285
Glu Arg Gln Val Tyr Glu Leu Asn Lys Ile Gln Asp Lys Leu Pro Glu
    290                 295                 300
Lys Leu Lys Ala Glu Tyr Lys Lys Lys Leu Glu Asp Thr Lys Lys Ala
305                 310                 315                 320
Leu Asp Glu Gln Val Lys Ser Ala Ile Thr Glu Phe Gln Asn Val Gln
                325                 330                 335
Pro Thr Asn Glu Lys Met Thr Asp Leu Gln Asp Thr Lys Tyr Val Val
            340                 345                 350
Tyr Glu Ser Val Glu Asn Asn Glu Ser Met Met Asp Thr Phe Val Lys
        355                 360                 365
His Pro Ile Lys Thr Gly Met Leu Asn Gly Lys Lys Tyr Met Val Met
    370                 375                 380
Glu Thr Thr Asn Asp Asp Tyr Trp Lys Asp Phe Met Val Glu Gly Gln
385                 390                 395                 400
Arg Val Arg Thr Ile Ser Lys Asp Ala Lys Asn Asn Thr Arg Thr Ile
                405                 410                 415
Ile Phe Pro Tyr Val Glu Gly Lys Thr Leu Tyr Asp Ala Ile Val Lys
            420                 425                 430
Val His Val Lys Thr Ile Asp Tyr Asp Gly Gln Tyr His Val Arg Ile
        435                 440                 445
Val Asp Lys Glu Ala Phe Thr Lys Ala Asn Thr Asp Lys Ser Asn Lys
    450                 455                 460
Lys Glu Gln Gln Asp Asn Ser Ala Lys Lys Glu Ala Thr Pro Ala Thr
465                 470                 475                 480
Pro Ser Lys Pro Thr Pro Ser Pro Val Glu Lys Glu Ser Gln Lys Gln
                485                 490                 495
Asp Ser Gln Lys Asp Asp Asn Lys Gln Leu Pro Ser Val Glu Lys Glu
            500                 505                 510
Asn Asp Ala Ser Ser Glu Ser Gly Lys Asp Lys Thr Pro Ala Thr Lys
        515                 520                 525
Pro Thr Lys Gly Glu Val Glu Ser Ser Ser Thr Thr Pro Thr Lys Val
    530                 535                 540
Val Ser Thr Thr Gln Asn Val Ala Lys Pro Thr Thr Ala Ser Ser Lys
545                 550                 555                 560
Thr Thr Lys Asp Val Val Gln Thr Ser Ala Gly Ser Ser Glu Ala Lys
                565                 570                 575
Asp Ser Ala Pro Leu Gln Lys Ala Asn Ile Lys Asn Thr Asn Asp Gly
            580                 585                 590
His Thr Gln Ser Gln Asn Asn Lys Asn Thr Gln Glu Asn Lys Ala Lys
        595                 600                 605
Ser Leu Pro Gln Thr Gly Glu Glu Ser Asn Lys Asp Met Thr Leu Pro
    610                 615                 620
Leu Met Ala Leu Leu Ala Leu Ser Ser Ile Val Ala Phe Val Leu Pro
625                 630                 635                 640
Arg Lys Arg Lys Asn
                645

<210> 29
<211> 953
<212> PRT

<213> Staphylococcus aureus

<400> 29
Met Asn Asn Lys Lys Thr Ala Thr Asn Arg Lys Gly Met Ile Pro Asn
1               5                   10                  15
Arg Leu Asn Lys Phe Ser Ile Arg Lys Tyr Ser Val Gly Thr Ala Ser
            20                  25                  30
Ile Leu Val Gly Thr Thr Leu Ile Phe Gly Leu Ser Gly His Glu Ala
        35                  40                  45
Lys Ala Ala Glu His Thr Asn Gly Glu Leu Asn Gln Ser Lys Asn Glu
    50                  55                  60
Thr Thr Ala Pro Ser Glu Asn Lys Thr Thr Glu Lys Val Asp Ser Arg
65                  70                  75                  80
Gln Leu Lys Asp Asn Thr Gln Thr Ala Thr Ala Asp Gln Pro Lys Val
                85                  90                  95
Thr Met Ser Asp Ser Ala Thr Val Lys Glu Thr Ser Ser Asn Met Gln
            100                 105                 110
Ser Pro Gln Asn Ala Thr Ala Ser Gln Ser Thr Thr Gln Thr Ser Asn
        115                 120                 125
Val Thr Thr Asn Asp Lys Ser Ser Thr Thr Tyr Ser Asn Glu Thr Asp
    130                 135                 140
Lys Ser Asn Leu Thr Gln Ala Lys Asn Val Ser Thr Thr Pro Lys Thr
145                 150                 155                 160
Thr Thr Ile Lys Gln Arg Ala Leu Asn Arg Met Ala Val Asn Thr Val
                165                 170                 175
Ala Ala Pro Gln Gln Gly Thr Asn Val Asn Asp Lys Val His Phe Thr
            180                 185                 190
Asn Ile Asp Ile Ala Ile Asp Lys Gly His Val Asn Lys Thr Thr Gly
        195                 200                 205
Asn Thr Glu Phe Trp Ala Thr Ser Ser Asp Val Leu Lys Leu Lys Ala
    210                 215                 220
Asn Tyr Thr Ile Asp Asp Ser Val Lys Glu Gly Asp Thr Phe Thr Phe
225                 230                 235                 240
Lys Tyr Gly Gln Tyr Phe Arg Pro Gly Ser Val Arg Leu Pro Ser Gln
                245                 250                 255
Thr Gln Asn Leu Tyr Asn Ala Gln Gly Asn Ile Ile Ala Lys Gly Ile
            260                 265                 270
Tyr Asp Ser Lys Thr Asn Thr Thr Thr Tyr Thr Phe Thr Asn Tyr Val
        275                 280                 285
Asp Gln Tyr Thr Asn Val Ser Gly Ser Phe Glu Gln Val Ala Phe Ala
    290                 295                 300
Lys Arg Glu Asn Ala Thr Thr Asp Lys Thr Ala Tyr Lys Met Glu Val
305                 310                 315                 320
Thr Leu Gly Asn Asp Thr Tyr Ser Lys Asp Val Ile Val Asp Tyr Gly
                325                 330                 335
Asn Gln Lys Gly Gln Gln Leu Ile Ser Ser Thr Asn Tyr Ile Asn Asn
            340                 345                 350
Glu Asp Leu Ser Arg Asn Met Thr Val Tyr Val Asn Gln Pro Lys Lys
        355                 360                 365
Thr Tyr Thr Lys Glu Thr Phe Val Thr Asn Leu Thr Gly Tyr Lys Phe
    370                 375                 380
Asn Pro Asp Ala Lys Asn Phe Lys Ile Tyr Glu Val Thr Asp Gln Asn
385                 390                 395                 400
Gln Phe Val Asp Ser Phe Thr Pro Asp Thr Ser Lys Leu Lys Asp Val
                405                 410                 415
Thr Gly Gln Phe Asp Val Ile Tyr Ser Asn Asp Asn Lys Thr Ala Thr
            420                 425                 430
Val Asp Leu Leu Asn Gly Gln Ser Ser Ser Asp Lys Gln Tyr Ile Ile
        435                 440                 445
Gln Gln Val Ala Tyr Pro Asp Asn Ser Ser Thr Asp Asn Gly Lys Ile
    450                 455                 460
Asp Tyr Thr Leu Glu Thr Gln Asn Gly Lys Ser Ser Trp Ser Asn Ser
465                 470                 475                 480
Tyr Ser Asn Val Asn Gly Ser Ser Thr Ala Asn Gly Asp Gln Lys Lys
                485                 490                 495

Tyr Asn Leu Gly Asp Tyr Val Trp Glu Asp Thr Asn Lys Asp Gly Lys
            500                 505                 510
Gln Asp Ala Asn Glu Lys Gly Ile Lys Gly Val Tyr Val Ile Leu Lys
        515                 520                 525
Asp Ser Asn Gly Lys Glu Leu Asp Arg Thr Thr Thr Asp Glu Asn Gly
    530                 535                 540
Lys Tyr Gln Phe Thr Gly Leu Ser Asn Gly Thr Tyr Ser Val Glu Phe
545                 550                 555                 560
Ser Thr Pro Ala Gly Tyr Thr Pro Thr Thr Ala Asn Ala Gly Thr Asp
                565                 570                 575
Asp Ala Val Asp Ser Asp Gly Leu Thr Thr Thr Gly Val Ile Lys Asp
            580                 585                 590
Ala Asp Asn Met Thr Leu Asp Ser Gly Phe Tyr Lys Thr Pro Lys Tyr
        595                 600                 605
Ser Leu Gly Asp Tyr Val Trp Tyr Asp Ser Asn Lys Asp Gly Lys Gln
    610                 615                 620
Asp Ser Thr Glu Lys Gly Ile Lys Gly Val Lys Val Thr Leu Gln Asn
625                 630                 635                 640
Glu Lys Gly Glu Val Ile Gly Thr Thr Glu Thr Asp Glu Asn Gly Lys
                645                 650                 655
Tyr Arg Phe Asp Asn Leu Asp Ser Gly Lys Tyr Lys Val Ile Phe Glu
            660                 665                 670
Lys Pro Ala Gly Leu Thr Gln Thr Gly Thr Asn Thr Thr Glu Asp Asp
        675                 680                 685
Lys Asp Ala Asp Gly Gly Glu Val Asp Val Thr Ile Thr Asp His Asp
    690                 695                 700
Asp Phe Thr Leu Asp Asn Gly Tyr Tyr Glu Glu Glu Thr Ser Asp Ser
705                 710                 715                 720
Asp Ser Asp Ser Asp Ser Asp Ser Asp Ser Asp Ser Asp Ser Asp Ser
                725                 730                 735
Asp Ser Asp Ser Asp Ser Asp Ser Asp Ser Asp Ser Asp Ser Asp Ser
            740                 745                 750
Asp Ser Asp Ser Asp Ser Asp Ser Asp Ser Asp Ser Asp Ser Asp Ser
        755                 760                 765
Asp Ser Asp Ser Glu Ser Asp Ser Asp Ser Asp Ser Asp Ser Asp Ser
    770                 775                 780
Asp Ser Asp Ser Asp Ser Asp Ser Asp Ser Asp Ser Asp Ser Asp Ser
785                 790                 795                 800
Asp Ser Asp Ser Asp Ser Asp Ser Asp Ser Asp Ser Asp Ser Asp Ser
                805                 810                 815
Asp Ser Asp Ser Asp Ser Asp Asn Asp Ser Asp Ser Asp Ser Asp Ser
            820                 825                 830
Asp Ser Asp Ser Asp Ser Asp Ser Asp Ser Asp Ser Asp Ser Asp Ser
        835                 840                 845
Asp Ser Asp Ser Asp Ser Asp Ser Asp Ser Asp Ser Asp Ser Asp Ser
    850                 855                 860
Asp Ser Asp Ser Asp Ser Asp Ser Asp Ser Asp Ser Asp Ser Asp Ser
865                 870                 875                 880
Asp Ser Asp Ser Asp Ser Asp Ser Asp Ser Asp Ser Asp Ala Gly Lys
                885                 890                 895
His Thr Pro Thr Lys Pro Met Ser Thr Val Lys Asp Gln His Lys Thr
            900                 905                 910
Ala Lys Ala Leu Pro Glu Thr Gly Ser Glu Asn Asn Asn Ser Asn Asn
        915                 920                 925
Gly Thr Leu Phe Gly Gly Leu Phe Ala Ala Leu Gly Ser Leu Leu Leu
    930                 935                 940
Phe Gly Arg Arg Lys Lys Gln Asn Lys
945                 950

<210> 30
<211> 935
<212> PRT
<213> Staphylococcus aureus

<400> 30
Met Asn Met Lys Lys Lys Glu Lys His Ala Ile Arg Lys Lys Ser Ile
1               5                   10                  15
Gly Val Ala Ser Val Leu Val Gly Thr Leu Ile Gly Phe Gly Leu Leu
            20                  25                  30
Ser Ser Lys Glu Ala Asp Ala Ser Glu Asn Ser Val Thr Gln Ser Asp
        35                  40                  45
Ser Ala Ser Asn Glu Ser Lys Ser Asn Asp Ser Ser Ser Val Ser Ala
    50                  55                  60
Ala Pro Lys Thr Asp Asp Thr Asn Val Ser Asp Thr Lys Thr Ser Ser
65                  70                  75                  80
Asn Thr Asn Asn Gly Glu Thr Ser Val Ala Gln Asn Pro Ala Gln Gln
                85                  90                  95
Glu Thr Thr Gln Ser Ser Ser Thr Asn Ala Thr Thr Glu Glu Thr Pro
            100                 105                 110
Val Thr Gly Glu Ala Thr Thr Thr Thr Thr Asn Gln Ala Asn Thr Pro
        115                 120                 125
Ala Thr Thr Gln Ser Ser Asn Thr Asn Ala Glu Glu Leu Val Asn Gln
    130                 135                 140
Thr Ser Asn Glu Thr Thr Ser Asn Asp Thr Asn Thr Val Ser Ser Val
145                 150                 155                 160
Asn Ser Pro Gln Asn Ser Thr Asn Ala Glu Asn Val Ser Thr Thr Gln
                165                 170                 175
Asp Thr Ser Thr Glu Ala Thr Pro Ser Asn Asn Glu Ser Ala Pro Gln
            180                 185                 190
Asn Thr Asp Ala Ser Asn Lys Asp Val Val Ser Gln Ala Val Asn Pro
        195                 200                 205
Ser Thr Pro Arg Met Arg Ala Phe Ser Leu Ala Ala Val Ala Ala Asp
    210                 215                 220
Ala Pro Ala Ala Gly Thr Asp Ile Thr Asn Gln Leu Thr Asp Val Lys
225                 230                 235                 240
Val Thr Ile Asp Ser Gly Thr Thr Val Tyr Pro His Gln Ala Gly Tyr
                245                 250                 255
Val Lys Leu Asn Tyr Gly Phe Ser Val Pro Asn Ser Ala Val Lys Gly
            260                 265                 270
Asp Thr Phe Lys Ile Thr Val Pro Lys Glu Leu Asn Leu Asn Gly Val
        275                 280                 285

Thr Ser Thr Ala Lys Val Pro Pro Ile Met Ala Gly Asp Gln Val Leu
    290                 295                 300
Ala Asn Gly Val Ile Asp Ser Asp Gly Asn Val Ile Tyr Thr Phe Thr
305                 310                 315                 320
Asp Tyr Val Asp Asn Lys Glu Asn Val Thr Ala Asn Ile Thr Met Pro
                325                 330                 335
Ala Tyr Ile Asp Pro Glu Asn Val Thr Lys Thr Gly Asn Val Thr Leu
            340                 345                 350
Thr Thr Gly Ile Gly Thr Asn Thr Ala Ser Lys Thr Val Leu Ile Asp
        355                 360                 365
Tyr Glu Lys Tyr Gly Gln Phe His Asn Leu Ser Ile Lys Gly Thr Ile
    370                 375                 380
Asp Gln Ile Asp Lys Thr Asn Asn Thr Tyr Arg Gln Thr Ile Tyr Val
385                 390                 395                 400
Asn Pro Ser Gly Asp Asn Val Val Leu Pro Ala Leu Thr Gly Asn Leu
                405                 410                 415
Ile Pro Asn Thr Lys Ser Asn Ala Leu Ile Asp Ala Lys Asn Thr Asp
            420                 425                 430
Ile Lys Val Tyr Arg Val Asp Asn Ala Asn Asp Leu Ser Glu Ser Tyr
        435                 440                 445
Tyr Val Asn Pro Ser Asp Phe Glu Asp Val Thr Asn Gln Val Arg Ile
    450                 455                 460
Ser Phe Pro Asn Ala Asn Gln Tyr Lys Val Glu Phe Pro Thr Asp Asp
465                 470                 475                 480
Asp Gln Ile Thr Thr Pro Tyr Ile Val Val Val Asn Gly His Ile Asp
                485                 490                 495
Pro Ala Ser Thr Gly Asp Leu Ala Leu Arg Ser Thr Phe Tyr Gly Tyr

500                 505                 510
Asp Ser Asn Phe Ile Trp Arg Ser Met Ser Trp Asp Asn Glu Val Ala
        515                 520                 525
Phe Asn Asn Gly Ser Gly Ser Gly Asp Gly Ile Asp Lys Pro Val Val
    530                 535                 540
Pro Glu Gln Pro Asp Glu Pro Gly Glu Ile Glu Pro Ile Pro Glu Asp
545                 550                 555                 560
Ser Asp Ser Asp Pro Gly Ser Asp Ser Gly Ser Asp Ser Asn Ser Asp
                565                 570                 575
Ser Gly Ser Asp Ser Gly Ser Asp Ser Thr Ser Asp Ser Gly Ser Asp
            580                 585                 590
Ser Ala Ser Asp Ser Asp Ser Ala Ser Asp Ser Asp Ser Ala Ser Asp
        595                 600                 605
Ser Asp Ser Ala Ser Asp Ser Asp Ser Ala Ser Asp Ser Asp Ser Ala
    610                 615                 620
Ser Asp Ser Asp Ser Ala Ser Asp Ser Asp Ser Ala Ser Asp Ser Asp

625                 630                 635                 640
Ser Ala Ser Asp Ser Asp Ser Ala Ser Asp Ser Asp Ser Ala Ser Asp
                645                 650                 655
Ser Asp Ser Ala Ser Asp Ser Asp Ser Ala Ser Asp Ser Asp Ser Asp
            660                 665                 670
Ser Asp Ser Asp Ser Asp Ser Asp Ser Asp Ser Asp Ser Asp Ser Asp
        675                 680                 685
Ser Asp Ser Asp Ser Asp Ser Asp Ser Asp Ser Asp Ser Asp Ser Asp
    690                 695                 700
Ser Asp Ser Asp Ser Asp Ser Asp Ser Asp Ser Asp Ser Asp Ser Asp
705                 710                 715                 720
Ser Asp Ser Asp Ser Asp Ser Asp Ser Asp Ser Asp Ser Asp Ser Asp
                725                 730                 735
Ser Asp Ser Asp Ser Asp Ser Asp Ser Asp Ser Asp Ser Asp Ser Asp
            740                 745                 750
Ser Asp Ser Asp Ser Asp Ser Asp Ser Asp Ser Asp Ser Asp Ser Ala
        755                 760                 765
Ser Asp Ser Asp Ser Asp Ser Asp Ser Glu Ser Asp Ser Asp Ser Asp
    770                 775                 780
Ser Asp Ser Asp Ser Asp Ser Asp Ser Asp Ser Asp Ser Asp Ser Asp
785                 790                 795                 800
Ser Glu Ser Asp Ser Asp Ser Asp Ser Asp Ser Asp Ser Glu Ser Asp
                805                 810                 815
Ser Asp Ser Asp Ser Asp Ser Asp Ser Asp Ser Ala Ser Asp Ser Asp
            820                 825                 830
Ser Gly Ser Asp Ser Asp Ser Ser Ser Asp Ser Asp Ser Asp Ser Thr
        835                 840                 845
Ser Asp Thr Gly Ser Asp Asn Asp Ser Asp Ser Asp Ser Asn Ser Asp
    850                 855                 860
Ser Glu Ser Gly Ser Asn Asn Asn Val Val Pro Pro Asn Ser Pro Lys
865                 870                 875                 880
Asn Gly Thr Asn Ala Ser Asn Lys Asn Glu Ala Lys Asp Ser Lys Glu
                885                 890                 895
Pro Leu Pro Asp Thr Gly Ser Glu Asp Glu Ala Asn Thr Ser Leu Ile
            900                 905                 910
Trp Gly Leu Leu Ala Ser Leu Gly Ser Leu Leu Leu Phe Arg Arg Lys
        915                 920                 925
Lys Glu Asn Lys Asp Lys Lys
    930                 935


<210> 31
<211> 1038
<212> PRT
<213> Staphylococcus aureus

<400> 31
Met Lys Asn Asn Leu Arg Tyr Gly Ile Arg Lys His Lys Leu Gly Ala

1                   5                   10                  15
Ala Ser Val Phe Leu Gly Thr Met Ile Val Val Gly Met Gly Gln Asp
                20                  25                  30

Lys Glu Ala Ala Ala Ser Glu Gln Lys Thr Thr Thr Val Glu Glu Asn
        35                  40                  45
Gly Asn Ser Ala Thr Asp Asn Lys Thr Ser Glu Thr Gln Thr Thr Ala
    50                  55                  60
Thr Asn Val Asn His Ile Glu Glu Thr Gln Ser Tyr Asn Ala Thr Val
65                  70                  75                  80
Thr Glu Gln Pro Ser Asn Ala Thr Gln Val Thr Thr Glu Glu Ala Pro
                85                  90                  95
Lys Ala Val Gln Ala Pro Gln Thr Ala Gln Pro Ala Asn Val Glu Thr
            100                 105                 110
Val Lys Glu Glu Glu Lys Pro Gln Val Lys Glu Thr Thr Gln Pro Gln
        115                 120                 125
Asp Asn Ser Gly Asn Gln Arg Gln Val Asp Leu Thr Pro Lys Lys Val
    130                 135                 140
Thr Gln Asn Gln Gly Thr Glu Thr Gln Val Glu Val Ala Gln Pro Arg
145                 150                 155                 160
Thr Ala Ser Glu Ser Lys Pro Arg Val Thr Arg Ser Ala Asp Val Ala
                165                 170                 175
Glu Ala Lys Glu Ala Ser Asp Val Ser Glu Val Lys Gly Thr Asp Val
            180                 185                 190
Thr Ser Lys Val Thr Val Glu Ser Gly Ser Ile Glu Ala Pro Gln Gly
        195                 200                 205
Asn Lys Val Glu Pro His Ala Gly Gln Arg Val Val Leu Lys Tyr Lys
    210                 215                 220
Leu Lys Phe Ala Asp Gly Leu Lys Arg Gly Asp Tyr Phe Asp Phe Thr
225                 230                 235                 240
Leu Ser Asn Asn Val Asn Thr Tyr Gly Val Ser Thr Ala Arg Lys Val
                245                 250                 255
Pro Glu Ile Lys Asn Gly Ser Val Val Met Ala Thr Gly Glu Ile Leu
            260                 265                 270
Gly Asn Gly Asn Ile Arg Tyr Thr Phe Thr Asn Glu Ile Glu His Lys
        275                 280                 285
Val Glu Val Thr Ala Asn Leu Glu Ile Asn Leu Phe Ile Asp Pro Lys
    290                 295                 300
Thr Val Gln Ser Asn Gly Glu Gln Lys Ile Thr Ser Lys Leu Asn Gly
305                 310                 315                 320
Glu Glu Thr Glu Lys Thr Ile Pro Val Val Tyr Asn Pro Gly Val Ser
                325                 330                 335
Asn Ser Tyr Thr Asn Val Asn Gly Ser Ile Glu Thr Phe Asn Lys Glu
            340                 345                 350
Ser Asn Lys Phe Thr His Ile Ala Tyr Ile Lys Pro Met Asn Gly Asn
        355                 360                 365
Gln Ser Asn Thr Val Ser Val Thr Gly Thr Leu Thr Glu Gly Ser Asn

    370                 375                 380
Leu Ala Gly Gly Gln Pro Thr Val Lys Val Tyr Glu Tyr Leu Gly Lys
385                 390                 395                 400
Lys Asp Glu Leu Pro Gln Ser Val Tyr Ala Asn Thr Ser Asp Thr Asn
                405                 410                 415
Lys Phe Lys Asp Val Thr Lys Glu Met Asn Gly Lys Leu Ser Val Gln
            420                 425                 430
Asp Asn Gly Ser Tyr Ser Leu Asn Leu Asp Lys Leu Asp Lys Thr Tyr
        435                 440                 445
Val Ile His Tyr Thr Gly Glu Tyr Leu Gln Gly Ser Asp Gln Val Asn
    450                 455                 460
Phe Arg Thr Glu Leu Tyr Gly Tyr Pro Glu Arg Ala Tyr Lys Ser Tyr
465                 470                 475                 480
Tyr Val Tyr Gly Gly Tyr Arg Leu Thr Trp Asp Asn Gly Leu Val Leu
                485                 490                 495
Tyr Ser Asn Lys Ala Asp Gly Asn Gly Lys Asn Gly Gln Ile Ile Gln
            500                 505                 510

Asp Asn Asp Phe Glu Tyr Lys Glu Asp Thr Ala Lys Gly Thr Met Ser
        515                 520                 525
Gly Gln Tyr Asp Ala Lys Gln Ile Ile Glu Thr Glu Glu Asn Gln Asp
    530                 535                 540
Asn Thr Pro Leu Asp Ile Asp Tyr His Thr Ala Ile Asp Gly Glu Gly
545                 550                 555                 560
Gly Tyr Val Asp Gly Tyr Ile Glu Thr Ile Glu Glu Thr Asp Ser Ser
                565                 570                 575
Ala Ile Asp Ile Asp Tyr His Thr Ala Val Asp Ser Glu Val Gly His
            580                 585                 590
Val Gly Gly Tyr Thr Glu Ser Ser Glu Glu Ser Asn Pro Ile Asp Phe
        595                 600                 605
Glu Glu Ser Thr His Glu Asn Ser Lys His His Ala Asp Val Val Glu
    610                 615                 620
Tyr Glu Glu Asp Thr Asn Pro Gly Gly Gly Gln Val Thr Thr Glu Ser
625                 630                 635                 640
Asn Leu Val Glu Phe Asp Glu Glu Ser Thr Lys Gly Ile Val Thr Gly
                645                 650                 655
Ala Val Ser Asp His Thr Thr Ile Glu Asp Thr Lys Glu Tyr Thr Thr
            660                 665                 670
Glu Ser Asn Leu Ile Glu Leu Val Asp Glu Leu Pro Glu Glu His Gly
        675                 680                 685
Gln Ala Gln Gly Pro Ile Glu Glu Ile Thr Glu Asn Asn His His Ile
    690                 695                 700
Ser His Ser Gly Leu Gly Thr Glu Asn Gly His Gly Asn Tyr Gly Val
705                 710                 715                 720
Ile Glu Glu Ile Glu Glu Asn Ser His Val Asp Ile Lys Ser Glu Leu
                725                 730                 735
Gly Tyr Glu Gly Gly Gln Asn Ser Gly Asn Gln Ser Phe Glu Glu Asp
            740                 745                 750
Thr Glu Glu Asp Lys Pro Lys Tyr Glu Gln Gly Gly Asn Ile Val Asp
        755                 760                 765
Ile Asp Phe Asp Ser Val Pro Gln Ile His Gly Gln Asn Lys Gly Asp
    770                 775                 780
Gln Ser Phe Glu Glu Asp Thr Glu Lys Asp Lys Pro Lys Tyr Glu His
785                 790                 795                 800
Gly Gly Asn Ile Ile Asp Ile Asp Phe Asp Ser Val Pro Gln Ile His
                805                 810                 815
Gly Phe Asn Lys His Asn Glu Ile Ile Glu Glu Asp Thr Asn Lys Asp
            820                 825                 830
Lys Pro Asn Tyr Gln Phe Gly Gly His Asn Ser Val Asp Phe Glu Glu
        835                 840                 845
Asp Thr Leu Pro Lys Val Ser Gly Gln Asn Glu Gly Gln Gln Thr Ile
    850                 855                 860
Glu Glu Asp Thr Thr Pro Pro Thr Pro Pro Thr Pro Glu Val Pro Ser
865                 870                 875                 880
Glu Pro Glu Thr Pro Met Pro Pro Thr Pro Glu Val Pro Ser Glu Pro
                885                 890                 895
Glu Thr Pro Thr Pro Pro Thr Pro Glu Val Pro Ser Glu Pro Glu Thr
            900                 905                 910
Pro Thr Pro Pro Thr Pro Glu Val Pro Ser Glu Pro Glu Thr Pro Thr
        915                 920                 925
Pro Pro Thr Pro Glu Val Pro Ser Glu Pro Glu Thr Pro Thr Pro Pro
    930                 935                 940
Thr Pro Glu Val Pro Ala Glu Pro Gly Lys Pro Val Pro Pro Ala Lys
945                 950                 955                 960
Glu Glu Pro Lys Lys Pro Ser Lys Pro Val Glu Gln Gly Lys Val Val
                965                 970                 975
Thr Pro Val Ile Glu Ile Asn Glu Lys Val Lys Ala Val Ala Pro Thr
            980                 985                 990
Lys Lys Ala Gln Ser Lys Lys Ser Glu Leu Pro Glu Thr Gly Gly Glu
        995                 1000                1005
Glu Ser Thr Asn Lys Gly Met Leu Phe Gly Gly Leu Phe Ser Ile Leu
    1010                1015                1020
Gly Leu Ala Leu Leu Arg Arg Asn Lys Lys Asn Asn Lys Ala

1025                1030                1035

<210> 32
<211> 877
<212> PRT
<213> Staphylococcus aureus

<400> 32
Met Lys Lys Arg Ile Asp Tyr Leu Ser Asn Lys Gln Asn Lys Tyr Ser
1               5                   10                  15
Ile Arg Arg Phe Thr Val Gly Thr Thr Ser Val Ile Val Gly Ala Thr
            20                  25                  30
Ile Leu Phe Gly Ile Gly Asn His Gln Ala Gln Ala Ser Glu Gln Ser
        35                  40                  45
Asn Asp Thr Thr Gln Ser Ser Lys Asn Asn Ala Ser Ala Asp Ser Glu
    50                  55                  60
Lys Asn Asn Met Ile Glu Thr Pro Gln Leu Asn Thr Thr Ala Asn Asp
65                  70                  75                  80
Thr Ser Asp Ile Ser Ala Asn Thr Asn Ser Ala Asn Val Asp Ser Thr
                85                  90                  95
Thr Lys Pro Met Ser Thr Gln Thr Ser Asn Thr Thr Thr Thr Glu Pro
            100                 105                 110
Ala Ser Thr Asn Glu Thr Pro Gln Pro Thr Ala Ile Lys Asn Gln Ala
        115                 120                 125
Thr Ala Ala Lys Met Gln Asp Gln Thr Val Pro Gln Glu Ala Asn Ser
    130                 135                 140
Gln Val Asp Asn Lys Thr Thr Asn Asp Ala Asn Ser Ile Ala Thr Asn
145                 150                 155                 160
Ser Glu Leu Lys Asn Ser Gln Thr Leu Asp Leu Pro Gln Ser Ser Pro
                165                 170                 175
Gln Thr Ile Ser Asn Ala Gln Gly Thr Ser Lys Pro Ser Val Arg Thr
            180                 185                 190
Arg Ala Val Arg Ser Leu Ala Val Ala Glu Pro Val Val Asn Ala Ala
        195                 200                 205
Asp Ala Lys Gly Thr Asn Val Asn Asp Lys Val Thr Ala Ser Asn Phe
    210                 215                 220
Lys Leu Glu Lys Thr Thr Phe Asp Pro Asn Gln Ser Gly Asn Thr Phe
225                 230                 235                 240
Met Ala Ala Asn Phe Thr Val Thr Asp Lys Val Lys Ser Gly Asp Tyr
                245                 250                 255
Phe Thr Ala Lys Leu Pro Asp Ser Leu Thr Gly Asn Gly Asp Val Asp
            260                 265                 270
Tyr Ser Asn Ser Asn Asn Thr Met Pro Ile Ala Asp Ile Lys Ser Thr
        275                 280                 285
Asn Gly Asp Val Val Ala Lys Ala Thr Tyr Asp Ile Leu Thr Lys Thr
    290                 295                 300
Tyr Thr Phe Val Phe Thr Asp Tyr Val Asn Asn Lys Glu Asn Ile Asn
305                 310                 315                 320
Gly Gln Phe Ser Leu Pro Leu Phe Thr Asp Arg Ala Lys Ala Pro Lys
                325                 330                 335
Ser Gly Thr Tyr Asp Ala Asn Ile Asn Ile Ala Asp Glu Met Phe Asn
            340                 345                 350
Asn Lys Ile Thr Tyr Asn Tyr Ser Ser Pro Ile Ala Gly Ile Asp Lys
        355                 360                 365
Pro Asn Gly Ala Asn Ile Ser Ser Gln Ile Ile Gly Val Asp Thr Ala
    370                 375                 380
Ser Gly Gln Asn Thr Tyr Lys Gln Thr Val Phe Val Asn Pro Lys Gln
385                 390                 395                 400
Arg Val Leu Gly Asn Thr Trp Val Tyr Ile Lys Gly Tyr Gln Asp Lys
                405                 410                 415
Ile Glu Glu Ser Ser Gly Lys Val Ser Ala Thr Asp Thr Lys Leu Arg
            420                 425                 430
Ile Phe Glu Val Asn Asp Thr Ser Lys Leu Ser Asp Ser Tyr Tyr Ala
        435                 440                 445

Asp Pro Asn Asp Ser Asn Leu Lys Glu Val Thr Asp Gln Phe Lys Asn
    450                 455                 460
Arg Ile Tyr Tyr Glu His Pro Asn Val Ala Ser Ile Lys Phe Gly Asp
465                 470                 475                 480
Ile Thr Lys Thr Tyr Val Val Leu Val Glu Gly His Tyr Asp Asn Thr
                485                 490                 495
Gly Lys Asn Leu Lys Thr Gln Val Ile Gln Glu Asn Val Asp Pro Val
            500                 505                 510
Thr Asn Arg Asp Tyr Ser Ile Phe Gly Trp Asn Asn Glu Asn Val Val
        515                 520                 525
Arg Tyr Gly Gly Gly Ser Ala Asp Gly Asp Ser Ala Val Asn Pro Lys
    530                 535                 540
Asp Pro Thr Pro Gly Pro Pro Val Asp Pro Glu Pro Ser Pro Asp Pro
545                 550                 555                 560
Glu Pro Glu Pro Thr Pro Asp Pro Glu Pro Ser Pro Asp Pro Glu Pro
                565                 570                 575
Glu Pro Ser Pro Asp Pro Asp Pro Asp Ser Asp Ser Asp Ser Asp Ser
            580                 585                 590
Gly Ser Asp Ser Asp Ser Gly Ser Asp Ser Asp Ser Glu Ser Asp Ser
        595                 600                 605
Asp Ser Asp Ser Asp Ser Asp Ser Asp Ser Asp Ser Asp Ser Glu Ser
    610                 615                 620
Asp Ser Asp Ser Glu Ser Asp Ser Asp Ser Asp Ser Asp Ser Asp Ser
625                 630                 635                 640
Asp Ser Asp Ser Asp Ser Glu Ser Asp Ser Asp Ser Asp Ser Asp Ser
                645                 650                 655
Asp Ser Asp Ser Asp Ser Asp Ser Glu Ser Asp Ser Asp Ser Glu Ser
            660                 665                 670
Asp Ser Glu Ser Asp Ser Asp Ser Asp Ser Asp Ser Asp Ser Asp Ser
        675                 680                 685
Asp Ser Asp Ser Asp Ser Asp Ser Asp Ser Asp Ser Asp Ser Asp Ser
    690                 695                 700
Asp Ser Asp Ser Asp Ser Asp Ser Glu Ser Asp Ser Asp Ser Asp Ser
705                 710                 715                 720
Asp Ser Asp Ser Asp Ser Asp Ser Asp Ser Asp Ser Asp Ser Asp Ser
                725                 730                 735
Asp Ser Asp Ser Asp Ser Asp Ser Asp Ser Asp Ser Asp Ser Asp Ser
            740                 745                 750
Asp Ser Asp Ser Asp Ser Asp Ser Asp Ser Asp Ser Asp Ser Asp Ser
        755                 760                 765
Asp Ser Asp Ser Asp Ser Asp Ser Asp Ser Asp Ser Asp Ser Asp Ser
    770                 775                 780
Asp Ser Asp Ser Asp Ser Asp Ser Asp Ser Asp Ser Asp Ser Asp Ser
785                 790                 795                 800
Asp Ser Asp Ser Arg Val Thr Pro Pro Asn Asn Glu Gln Lys Ala Pro
                805                 810                 815
Ser Asn Pro Lys Gly Glu Val Asn His Ser Asn Lys Val Ser Lys Gln
            820                 825                 830
His Lys Thr Asp Ala Leu Pro Glu Thr Gly Asp Lys Ser Glu Asn Thr
        835                 840                 845
Asn Ala Thr Leu Phe Gly Ala Met Met Ala Leu Leu Gly Ser Leu Leu
    850                 855                 860
Leu Phe Arg Lys Arg Lys Gln Asp His Lys Glu Lys Ala
865                 870                 875

<210> 33
<211> 658
<212> PRT
<213> Staphylococcus aureus

<400> 33
Met Lys Lys Gln Ile Ile Ser Leu Gly Ala Leu Ala Val Ala Ser Ser
1               5                   10                  15
Leu Phe Thr Trp Asp Asn Lys Ala Asp Ala Ile Val Thr Lys Asp Tyr

            20                  25                  30
Ser Lys Glu Ser Arg Val Asn Glu Lys Ser Lys Lys Gly Ala Thr Val
        35                  40                  45
Ser Asp Tyr Tyr Tyr Trp Lys Ile Ile Asp Ser Leu Glu Ala Gln Phe
    50                  55                  60
Thr Gly Ala Ile Asp Leu Leu Glu Asp Tyr Lys Tyr Gly Asp Pro Ile
65                  70                  75                  80
Tyr Lys Glu Ala Lys Asp Arg Leu Met Thr Arg Val Leu Gly Glu Asp
                85                  90                  95
Gln Tyr Leu Leu Lys Lys Lys Ile Asp Glu Tyr Glu Leu Tyr Lys Lys
            100                 105                 110
Trp Tyr Lys Ser Ser Asn Lys Asn Thr Asn Met Leu Thr Phe His Lys
        115                 120                 125
Tyr Asn Leu Tyr Asn Leu Thr Met Asn Glu Tyr Asn Asp Ile Phe Asn
    130                 135                 140
Ser Leu Lys Asp Ala Val Tyr Gln Phe Asn Lys Glu Val Lys Glu Ile
145                 150                 155                 160
Glu His Lys Asn Val Asp Leu Lys Gln Phe Asp Lys Asp Gly Glu Asp
                165                 170                 175
Lys Ala Thr Lys Glu Val Tyr Asp Leu Val Ser Glu Ile Asp Thr Leu
            180                 185                 190
Val Val Thr Tyr Tyr Ala Asp Lys Asp Tyr Gly Glu His Ala Lys Glu
        195                 200                 205
Leu Arg Ala Lys Leu Asp Leu Ile Leu Gly Asp Thr Asp Asn Pro His
    210                 215                 220
Lys Ile Thr Asn Glu Arg Ile Lys Lys Glu Met Ile Asp Asp Leu Asn
225                 230                 235                 240
Ser Ile Ile Asp Asp Phe Phe Met Glu Thr Lys Gln Asn Arg Pro Asn
                245                 250                 255
Ser Ile Thr Lys Tyr Asp Pro Thr Lys His Asn Phe Lys Glu Lys Ser
            260                 265                 270
Glu Asn Lys Pro Asn Phe Asp Lys Leu Val Glu Glu Thr Lys Lys Ala
        275                 280                 285
Val Lys Glu Ala Asp Glu Ser Trp Lys Asn Lys Thr Val Lys Lys Tyr
    290                 295                 300
Glu Glu Thr Val Thr Lys Ser Pro Val Val Lys Glu Glu Lys Lys Val
305                 310                 315                 320
Glu Glu Pro Gln Leu Pro Lys Val Gly Asn Gln Gln Glu Val Lys Thr
                325                 330                 335
Thr Ala Gly Lys Ala Glu Glu Thr Thr Gln Pro Val Ala Gln Pro Leu
            340                 345                 350
Val Lys Ile Pro Gln Glu Thr Ile Tyr Gly Glu Thr Val Lys Gly Pro
        355                 360                 365
Glu Tyr Pro Thr Met Glu Asn Lys Thr Leu Gln Gly Glu Ile Val Gln
    370                 375                 380
Gly Pro Asp Phe Leu Thr Met Glu Gln Asn Arg Pro Ser Leu Ser Asp
385                 390                 395                 400
Asn Tyr Thr Gln Pro Thr Thr Pro Asn Pro Ile Leu Glu Gly Leu Glu
                405                 410                 415
Gly Ser Ser Ser Lys Leu Glu Ile Lys Pro Gln Gly Thr Glu Ser Thr
            420                 425                 430
Leu Lys Gly Ile Gln Gly Glu Ser Ser Asp Ile Glu Val Lys Pro Gln
        435                 440                 445
Ala Thr Glu Thr Thr Glu Ala Ser Gln Tyr Gly Pro Arg Pro Gln Phe
    450                 455                 460
Asn Lys Thr Pro Lys Tyr Val Lys Tyr Arg Asp Ala Gly Thr Gly Ile
465                 470                 475                 480
Arg Glu Tyr Asn Asp Gly Thr Phe Gly Tyr Glu Ala Arg Pro Arg Phe
                485                 490                 495
Asn Lys Pro Ser Glu Thr Asn Ala Tyr Asn Val Thr Thr Asn Gln Asp
            500                 505                 510
Gly Thr Val Ser Tyr Gly Ala Arg Pro Thr Gln Asn Lys Pro Ser Glu
        515                 520                 525
Thr Asn Ala Tyr Asn Val Thr Thr His Ala Asn Gly Gln Val Ser Tyr
    530                 535                 540

Gly Ala Arg Pro Thr Gln Lys Lys Pro Ser Lys Thr Asn Ala Tyr Asn
545                 550                 555                 560
Val Thr Thr His Ala Asn Gly Gln Val Ser Tyr Gly Ala Arg Pro Thr
                565                 570                 575
Gln Lys Lys Pro Ser Lys Thr Asn Ala Tyr Asn Val Thr Thr His Ala
            580                 585                 590
Asn Gly Gln Val Ser Tyr Gly Ala Arg Pro Thr Tyr Lys Lys Pro Ser
        595                 600                 605
Glu Thr Asn Ala Tyr Asn Val Thr Thr His Ala Asn Gly Gln Val Ser
    610                 615                 620
Tyr Gly Ala Arg Pro Thr Gln Lys Lys Pro Ser Glu Thr Asn Ala Tyr
625                 630                 635                 640
Asn Val Thr Thr His Ala Asp Gly Thr Ala Thr Tyr Gly Pro Arg Val
                645                 650                 655
Thr Lys

<210> 34
<211> 1602
<212> DNA
<213> Staphylococcus aureus

<400> 34
atgttacaag taactgatgt gagtttacgt tttggagatc gtaaactatt tgaagatgta 60
aatattaaat ttacagaagg taattgttat ggattaattg gtgcgaatgg tgcaggtaaa 120
tcaacattct taaaaatatt atctggtgaa ttagattctc aaacaggaca tgtttcatta 180
ggtaaaaatg aacgtctagc tgttttaaaa caggaccact atgcttatga agatgaacgc 240
gtgcttgatg ttgtaattaa aggtcacgaa cgtctttatg aggttatgaa agaaaaagat 300
gaaatctata tgaagccaga tttcagtgat gaagatggta tccgtgctgc tgaacttgaa 360
ggtgaatttg cagaaatgaa tggttggaat gctgaagctg atgctgctaa ccttttatct 420
ggtttaggta tcgatccaac tttacacgat aaaaaaatgg ctgaattaga aaacaaccaa 480
aaaattaaag tattattagc gcaaagttta ttcggtgaac cagacgtact attactggat 540
gagcctacta acggtctcga tattccagca atcagttggt tagaagattt cttaattaac 600
tttgataata ctgttatcgt agtatcgcat gaccgtcatt tcttaaataa tgtatgtact 660
catatcgctg atttagactt cggtaaaatt aaagtttatg ttggtaacta tgatttttgg 720
tatcaatcta gtcagttagc tcaaaagatg gctcaagaac aaaacaagaa aaaagaagaa 780
aaaatgaaag agttacagga ctttattgca cgtttctcag ctaacgcttc taaatctaaa 840
caagcaacaa gtcgtaaaaa acaacttgag aaaattgaat tagatgatat tcaaccatca 900
tcaagaagat atcctttcgt taaattcacg cctgagcgtg agattggtaa cgacttatta 960
atcgttcaaa atctttctaa aacaattgac ggcgaaaaag tattagataa tgtatcattc 1020
acaatgaatc caaatgataa agcgatttta attggagata gtgaaattgc aaaaacaaca 1080
ttacttaaaa tattagctgg cgaaatggaa ccagacgaag gttcatttaa atggggtgtt 1140
actacatcat taagttactt ccctaaagat aactcagagt tctttgaggg tgtaaatatg 1200
aatctcgttg attggttaag acaatatgct cctgaagatg aacaaacaga aacattttta 1260
cgtggtttct taggtcgtat gttatttagt ggtgaagaag ttaagaaaaa agctagtgtg 1320
ctttcaggtg gagaaaaagt acgttgtatg ctaagtaaaa tgatgttatc aagtgcgaat 1380
gtacttttac ttgacgaacc tactaaccac ttagacttag aaagtattac tgctgtcaat 1440
gatggtctta aatcatttaa aggttctatc atctttactt cttatgactt cgaatttatc 1500
aacacgattg caaaccgtgt tatcgattta aataaacaag gcggcgtttc aaaagaaatt 1560
ccatatgaag aatacttgca agaaatcggc gttttaaaat aa                    1602

<210> 35
<211> 1608
<212> DNA
<213> Staphylococcus aureus

<400> 35
atgttacaag taactgatgt aagtttacgt tttggtgatc gtaaactatt tgaagatgta 60
aatataaaat ttacagaggg taattgttat ggattaattg gtgcaaatgg tgctgggaaa 120
tctacattct tgaagatttt atcaggcgaa attgattcac agactggtca tgtatctcta 180
ggtaaagatg agcgtttggc tgtgttaaaa caagatcatt ttgcttatga agatgaacgt 240
gttttagatg ttgtgattaa aggacatgaa cgtttgtatc aagtgatgaa agagaaagat 300
gaaatttata tgaaacctga tttcagcgat gaggacggta ttcgcgctgc agaacttgaa 360
ggagaatttg cagaaatgaa cggttggaat gctgaagctg atgctgctaa cttattatca 420

ggattaggca tagaacctga cttacatgat aaaaatatgt ctgaacttga aaataatcaa 480
aaagttaagg tattgttagc tcaaagttta tttggtgatc ctgacgttct tttactagat 540
gagcctacca atggtttaga tataccagca ataagttggt tagaagactt tttaattaat 600
tttgaaaata ctgtcattgt cgtttcgcat gaccgtcact tcttaaataa tgtttgtact 660
catattgctg atttagactt tggcaaaatt aaactttatg ttggtaacta tgatttttgg 720
tatcaatcaa gtcaattagc acaaaaaatg gcacaagaac aaaataagaa aaaagaagaa 780
aaaatgaaag agttacagga tttcatcgca cgcttctcag caaatgcttc taaatctaaa 840
caggcaacaa gtcgtaagaa acaattagaa aaaattgaat tagatgatat ccagccatca 900
tctcgtagat acccttacgt gaaatttact cctgaacgtg aaattggaaa tgatttactt 960
acagtagaaa atctttctaa aacaattgac ggcgaaaaag tactagacaa tgtttcattc 1020
actatgaatc ctaatgataa agctatttta gttggtgata gcgaaattgc taaaacaaca 1080
ttgttaaaaa ttttagctgg agaaatggaa ccagatgaag gtacatttaa atggggtgta 1140
acgacatctt taagttactt ccctaaagat aactctgagt tctttgatgg tgtcgatatg 1200
aatttagttg aatggttacg tcaatacgct ccagaagatg aacaaactga aacattttta 1260
cgtggtttct taggtcgcat gttatttagt ggtgaggaag ttaagaaaaa agcaagcgtg 1320
ctttcaggtg gagaaaaagt acgttgcatg ttaagtaaaa tgatgttatc aagtgctaac 1380
gtacttttac ttgatgagcc aacaaaccat ttagatttgg aaagtatcac tgctgtaaat 1440
gacggattaa aatcatttaa aggttctatc atcttcactt cttatgattt tgaatttatt 1500
aatacaatcg caaatcgagt gattgacttg aatcaagctg gtgccctttc taaagaagta 1560
ccttatgagg aatacttaca agaaattggt gtattacaaa ataattaa 1608

<210> 36
<211> 1305
<212> DNA
<213> Staphylococcus aureus

<400> 36
atgccaatta ttacagatgt ttacgctcgc gaagtcttag actctcgtgg taacccaact 60
gttgaagtag aagtattaac tgaaagtggc gcatttggtc gtgcattagt accatcaggt 120
gcttcaactg gtgaacacga agctgttgaa ttacgtgatg gagacaaatc acgttattta 180
ggtaaaggtg ttactaaagc agttgaaaac gttaatgaaa tcatcgcacc agaaattatt 240
gaaggtgaat tttcagtatt agatcaagta tctattgata aaatgatgat cgcattagac 300
ggtactccaa acaaaggtaa attaggtgca aatgctattt taggtgtatc tatcgcagta 360
gcacgtgcag cagctgactt attaggtcaa ccactttaca aatatttagg tggatttaat 420
ggtaagcagt taccagtacc aatgatgaac atcgttaatg gtggttctca ctcagatgct 480
ccaattgcat tccaagaatt catgatttta cctgtaggtg ctacaacgtt caaagaatca 540
ttacgttggg gtactgaaat tttccacaac ttaaaatcaa ttttaagcaa acgtggttta 600
gaaactgcag taggtgacga aggtggtttc gctcctaaat ttgaaggtac tgaagatgct 660
gttgaaacaa ttatccaagc aatcgaagca gctggttaca aaccaggtga agaagtattc 720
ttaggatttg actgtgcatc atcagaattc tatgaaaatg gtgtatatga ctacagtaag 780
ttcgaaggcg aacacggtgc aaaacgtaca gctgcagaac aagttgacta cttagaacaa 840
ttagtagaca aatatcctat cattacaatt gaagacggta tggacgaaaa cgactgggat 900
ggttggaaac aacttacaga acgtatcggt gaccgtgtac aattagtagg tgacgattta 960
ttcgtaacaa acactgaaat tttagcaaaa ggtattgaaa acggaattgg taactcaatc 1020
ttaattaaag ttaaccaaat cggtacatta actgaaacat ttgatgcaat cgaaatggct 1080
caaaaagctg gttacacagc agtagtttct caccgttcag gtgaaacaga agatacaaca 1140
attgctgata ttgctgttgc tacaaacgct ggtcaaatta aaactggttc attatcacgt 1200
actgaccgta ttgctaaata caatcaatta ttacgtatcg aagatgaatt atttgaaact 1260
gctaaatatg acggtatcaa atcattctat aacttagata aataa 1305

<210> 37
<211> 1305
<212> DNA
<213> Staphylococcus aureus

<400> 37
atgccaatta ttacagatgt ttacgctcgc gaagtcttag actcacgtgg taacccaaca 60
gttgaagttg aagtattaac tgaaagcggt gctttcggac gtgcattagt accttctggt 120
gcttctactg gtgaacatga agcagttgaa ttacgtgatg gagataaatc acgttattta 180
ggtaaaggtg tgactaaagc ggtagaaaat gttaacgaaa tgatcgcacc agaaatcgtt 240
gaaggtgaat tttcagtttt agatcaagta tctattgata aaatgatgat tcaattagac 300
ggtacacaca acaaaggtaa attaggtgca aatgccattt taggtgtttc tattgccgta 360
gctcgtgcag ctgctgactt attaggtcaa ccattatata aatatttagg tggatttaat 420
ggtaaacaat tgccagtacc tatgatgaat attgttaatg gtggttctca ctcagatgca 480
ccaattgctt tccaagagtt catgatttta cctgtaggtg ctgagtcatt caaagaatca 540

ttacgttggg gtgcagaaat cttccataac cttaaatcaa tcttaagtga acgtggttta 600
gaaactgcag taggtgatga aggtggtttc gctcctagat ttgaaggcac tgaagacgct 660
gtagaaacta ttattaaagc tatcgaaaaa gcaggataca aaccaggtga agatgtattc 720
ttaggatttg actgtgcttc ttctgaattc tatgaaaatg gtgtttatga ttacactaaa 780
ttcgaaggtg aacacggtgc taaacgtagt gcagcagagc aagttgacta cttagaagaa 840
ttaattggta aatatccaat catcactatt gaagatggta tggatgaaaa cgattgggaa 900
ggttggaaac aattaactga tcgtatcggt gataaagttc aattagttgg tgatgattta 960
ttcgtaacta acactgaaat tttatctaaa ggtatcgaac aaggtattgg taactcaatc 1020
ttaatcaaag taaaccaaat cggtacatta actgaaacat tcgatgctat tgaaatggct 1080
caaaaagctg gatatactgc ggttgtatct caccgttctg gtgaaactga agatactaca 1140
attgctgata tcgcagttgc tacaaatgca ggccaaatta aaacaggttc attatctaga 1200
actgaccgta ttgctaaata caatcaatta ttacgtattg aagatgaatt atacgaaaca 1260
gctaaatttg aaggaattaa atctttctac aatttagata aataa 1305

<210> 38
<211> 768
<212> DNA
<213> Staphylococcus aureus

<400> 38
atgaaaaaaa tcgttacagc tacaatcgct acagcaggac ttgccactat cgcatttgca 60
ggacatgatg cacaagccgc agaacaaaat aacaatggat ataattctaa tgacgctcaa 120
tcatacagct atacgtatac aattgatgca caaggtaatt atcattacac ttggacagga 180
aattggaatc caagtcaatt aacgcaaaac aacacatact actacaacaa ctacaatact 240
tatagttata acaatgcatc ttacaataac tactataatc attcatatca atacaataac 300
tatacaaaca atagccaaac agcaacaaat aactattata ctggtggttc aggtgcaagt 360
tatagcacaa caagtaataa tgttcatgtg actacaactg cagcgccatc ttcaaatggt 420
cgttcaattt ctaatggtta tgcatcagga agtaacttat atacttcagg acaatgtact 480
tattatgtat ttgatcgtgt tggtgggaaa attggttcaa catggggtaa cgcaagtaat 540
tgggctaacg cagctgcatc atctggctat acagtgaaca atacaccaaa agttggtgct 600
atcatgcaaa caacacaagg ctattacggt catgttgctt acgttgaagg cgttaacagc 660
aacggttctg ttcgtgtttc agaaatgaac tatggacatg gtgctggtgt ggttacgtct 720
cgtacaattt cagcaaacca agcaggttca tataatttca ttcattaa 768

<210> 39
<211> 804
<212> DNA
<213> Staphylococcus aureus

<400> 39
atgaagaaaa tcgctacagc tactatcgca actgcaggat tcgctacaat cgcaattgca 60
tcaggaaatc aagctcatgc ttctgagcaa gataactacg gttataatcc aaacgaccca 120
acatcatata gctatactta cactattgat gcacaaggta actaccatta cacatggaaa 180
ggtaactggc atccaagtca attaaaccaa gataatggct actacagcta ttactactac 240
aatggctaca ataactacaa caattacaac aatggttata gctacaataa ttacagccgt 300
tacaacaact actcaaataa taatcaatca tataactaca ataactataa tagttacaac 360
acaaacagct accgtactgg tggtttaggt gcaagctaca gcacttcaag caacaatgtt 420
caagtaacta caactatggc tccatcatca aatggccgtt caatctcaag tggttatact 480
tcaggacgta acttatacac ttctggtcaa tgtacatact acgtatttga tcgtgtaggt 540
ggtaaaatcg gttcaacttg gggcaatgca agtaactggg ctaacgcagc tgcaagagct 600
ggttacacag tgaacaatac accaaaagct ggtgcaatta tgcaaacaac tcaaggtgca 660
tacggtcacg ttgcatacgt tgaaagtgtt aacagcaatg gttcagtaag agtttcagaa 720
atgaactatg gttatggccc aggtgttgta acttcacgta caatctcagc tagccaagct 780
gctggttata acttcattca ctaa 804

<210> 40
<211> 774
<212> DNA
<213> Staphylococcus aureus

<400> 40
atgaaaaaaa tcgctacagc tacaattgca actgcaggaa tcgctacttt cgcatttgca 60
caccatgacg cacaagcagc agaacaaaat aatgatgggt acaatccaaa cgacccttat 120
tcatatagct acacttacac aatcgatgct gaaggtaact accactacac ttggaaaggt 180
aactggagtc cagatcgtgt aaatacttca tataactata ataattataa taactacaac 240

tactatggtt acaataacta tagcaactac aataactaca gtaattacaa caattacaac 300
aactatcaat caaacaacac gcaatcacaa agaacaactc aaccgactgg tggtttaggc 360
gcaagctatt caacatcaag tagtaatgtt cacgttacaa caacttctgc gccatcatca 420
aacggtgtat ctttatcaaa cgctcgctca gcatctggta acttatacac ttcaggtcaa 480
tgtacatatt atgtatttga cagagtaggt ggcaaaatcg gttcaacgtg gggtaacgca 540
aacaactggg caaacgctgc agcacgttct ggttacacag taaacaattc gcctgctaaa 600
ggtgcaatct tacaaacgtc acaaggtgca tacggacacg tagcatacgt tgaaggtgta 660
aacagcaatg gttcaatcag agtttcagaa atgaactacg gtcacggtgc aggtgttgtc 720
acttcacgta caatctctgc gagccaagct gcttcatata actatattca ctaa 774

<210> 41
<211> 930
<212> DNA
<213> Staphylococcus aureus

<400> 41
atgaaaaaat tagtaccttt attattagcc ttattacttc tagttgctgc atgtggtact 60
ggtggtaaac aaagcagtga taagtcaaat ggcaaattaa aagtagtaac gacgaattca 120
attttatatg atatggctaa aaatgttggt ggagacaacg tcgatattca tagtattgta 180
cctgttggtc aagatcctca tgaatatgaa gttaaaccta aagatattaa aaagttaact 240
gacgctgacg ttattttata caacggatta aatttagaga ctggtaacgg ttggtttgaa 300
aaagccttag aacaggctgg taaatcatta aaagataaaa aagttatcgc agtatcaaaa 360
gatgttaaac ctatctattt aaacggtgaa gaaggcaaca aagataaaca agatccacac 420
gcatggttaa gtttagataa tggtattaaa tacgtaaaaa caattcaaca aacatttatc 480
gataacgaca aaaaacataa agcagattat gaaaagcaag gtaacaaata cattgctcaa 540
ttggaaaaat taaataatga cagtaaagac aaatttaatg acattccaaa agaacaacgt 600
gccatgatta caagtgaagg tgccttcaag tacttctcaa aacaatacgg tattacacca 660
ggttatattt gggaaattaa cactgaaaaa caaggtacac ctgaacaaat gagacaagct 720
attgagtttg ttaaaaagca caaattaaaa cacttattag tagaaacaag tgttgataag 780
aaagcaatgg aaagtttatc tgaagaaacg aagaaagata tctttggtga agtgtacaca 840
gattcaatcg gtaaagaagg cactaaaggt gactcttact acaaaatgat gaaatcaaat 900
attgaaactg tacacggaag catgaaataa 930

<210> 42
<211> 930
<212> DNA
<213> Staphylococcus aureus

<400> 42
gtgaaaaaaa ttctcgcttt agcaatagca tttttaatta tccttgccgc atgtgggaat 60
cacagtaacc atgaacatca ctcacatgaa ggaaaattaa aagttgtaac tacaaactct 120
attctctatg acatggttaa acgtgtcggt ggaaataagg tcgatgttca tagcatcgtt 180
ccagtaggac aagacccaca tgaatatgag gttaaaccta aagatattaa agcattaaca 240
gatgctgacg ttgtatttta taacggttta aacctagaaa ctggaaatgg ttggtttgaa 300
aaagcacttg accaagcagg aaaatcaaca aaagataaaa atgtgatagc agcatcaaat 360
aatgttaaac caatatactt aaatggtgag gaaggtaaca aaaacaaaca agatccacat 420
gcatggttaa gtttagagaa tggaattaaa tacgtaaaaa caatacaaaa atcactagaa 480
catcatgata aaaaagataa gtctacatat gaaaaacaag ggaatgcata tatatcaaaa 540
ttagaagaac ttaataaaga tagtaaaaat aaatttgatg acatacccaa aaatcaacgt 600
gccatgatga caagtgaagg tgcatttaaa tattttgctc aacaattcga tgttaaacca 660
ggttatattt gggagataaa cacagaaaaa caaggtacac ctggtcaaat gaaacaagcc 720
attaaatttg ttaaagataa tcatttaaaa catttattag tcgaaacaag cgtagataaa 780
aaagctatgc aaagtttatc agaagaaact aagaaagata tttatggtga agtatttacc 840
gactctatag gtaaggaagg tactaaaggt gactcatact ataaaatgat gaaatctaat 900
attgatacaa tacatggtag tatgaaataa 930

<210> 43
<211> 702
<212> DNA
<213> Staphylococcus aureus

<400> 43
atgaaaaaga caattatggc atcatcatta gcagtggcat taggtgtaac aggttacgca 60
gcaggtacag gacatcaagc acacgctgct gaagtaaacg ttgatcaagc acacttagtt 120
gacttagcgc ataatcacca agatcaatta aatgcagctc caatcaaaga tggtgcatat 180

gacatccact ttgtaaaaga tggtttccaa tataacttta cttcaaatgg tactacatgg 240
tcatggagct atgaagcagc taatggtcaa actgctggtt tctcaaacgt tgcaggtgca 300
gactacacta cttcatacaa ccaaggttca gatgtacaat cagtaagcta caatgcacaa 360
tcaagtaact caaacgttga agctgtttca gctccaactt accataacta cagcacttca 420
actacttcaa gttcagtgag attaagcaat ggtaatactg caggtgctac tggttcatca 480
gcagctcaaa tcatggctca acgtactggt gtttcagctt ctacatgggc tgcaatcatc 540
gctcgtgaat caaatggtca agtaaatgct tacaacccat caggtgcttc aggtttattc 600
caaactatgc caggttgggg tccgacaaac actgttgacc aacaaatcaa cgcagctgtt 660
aaagcataca aagcacaagg tttaggtgct tggggattct aa 702

<210> 44
<211> 708
<212> DNA
<213> Staphylococcus aureus

<400> 44
atgaaaaaaa cagttatcgc ttctacatta gcagtatctt taggaattgc aggttacggt 60
ttatcaggac atgaagcaca cgcttcagaa actacaaacg ttgataaagc acacttagta 120
gatttagcac aacataatcc tgaagaatta aatgctaaac cagttcaagc tggtgcttac 180
gatattcatt tcgtagacaa tggataccaa tacaacttca cttcaaatgg ttctgaatgg 240
tcatggagct acgctgtagc tggttcagat gctgattaca cagaatcatc atcaaaccaa 300
gaagtaagtg caaatacaca atctagtaac acaaatgtac aagctgtttc agctccaact 360
tcttcagaaa gtcgtagcta cagcacatca actacttcat actcagcacc aagccataac 420
tacagctctc acagtagttc agtaagatta tcaaatggta atactgctgg ttctgtaggt 480
tcatatgctg ctgctcaaat ggctgcacgt actggtgtat ctgcttcaac atgggaacac 540
atcattgcta gagaatcaaa tggtcaatta catgcacgta atgcttcagg tgctgctgga 600
ttattccaaa ctatgccagg ttggggttca actggttcag taaatgatca aatcaatgcc 660
gcttataaag catataaagc acaaggttta tctgcttggg gtatgtaa 708

<210> 45
<211> 11670
<212> DNA
<213> Staphylococcus aureus

<400> 45
gtgaattatc gtgataaaat tcaaaagttt agtattcgta aatatacagt tggtacattt 60
tcaactgtca ttgcgacatt ggtattttta ggattcaata catcacaagc acatgctgct 120
gaaacaaatc aaccagcaag cgtggttaaa cagaaacaac aaagtaataa tgaacagact 180
gagaatcgag aatctcaagt acaaaattct caaaattcac aaaatagtca atcattatcc 240
gctactcatg aaaatgagca accaaataat agtcaagcta atttagtaaa tcaaaaagta 300
gcgcaatcat ctactactaa tgatgaacaa ccagcatctc aaaatgtaaa tacaaagaaa 360
gattcggcaa cggctgcgac aacacaacca gataaagaag aaagtaagca taaacaaaac 420
gaaagtcaat ctgctaataa aaatggaaac gacaatagag cggctcatgt agaaaatcat 480
gaagcaaatg tagtaacagc ttcagattca tctgataatg gtaacgtaca acatgaccga 540
aatgaattac aagcattttt tgatgcaaat tatcatgatt atcgctttat tgaccgtgaa 600
aatgcagatt ctggcacatt taactatgta aaaggcattt ttgacaagat taatacttta 660
ttaggcagta atgatccaat taacaataaa gacttgcaac ttgcatacaa agaattggaa 720
caagctgttg ctttaattcg tacaatgcct caacgtcaac aaactagccg tcgatcaaac 780
agaattcaaa cgcgttctgt tgagtctaga gctgcagagc ctagatcagt atcagactat 840
caaaatgcaa attcatcata ttatgttgaa aatgctaatg atggttcagg atatcctgta 900
ggtacatata tcaatgcttc tagtaaaggg gcgccatata atttaccaac tacaccatgg 960
aatacattga aggcctctga ctcaaaggaa attgctctta tgacagcgaa acaaactgga 1020
gatggctacc aatgggttat taagtttaat aaaggacatg ctccacatca aaatatgatt 1080
ttctggtttg cattaccagc agaccaagtg ccagtaggaa gaactgactt tgtaacagtt 1140
aattcagatg gaacaaatgt acaatggagt catggagcag gagcaggtgc aaataaacca 1200
cttcaacaaa tgtgggaata tggagtaaat gatcctgatc gttcacatga ctttaaaata 1260
agaaatagaa gtggccaagt aatatatagc tggccaactg tccatgttta ttctttagaa 1320
gatttatcta gagcgagtga ttattttagt gaagctggag cgacacctgc tactaaagca 1380
tttggtagac aaaattttga atatattaat ggtcaaaaac ctgctgaatc accgggtgtt 1440
cctaaagttt atactttcat cggtcaaggt gatgcaagtt atacaatttc atttaaaaca 1500
caaggtccaa ctgttaataa attgtattat gcagcaggtg ggcgtgcttt agagtacaat 1560
caattattta tgtacagtca actatacgtc gaatcaacgc aagaccatca acaacgtctt 1620
aatggtttaa gacaagtggt taatcgtaca tatcgcatag gtacaactaa acgtgtagaa 1680
gtgagtcaag gaaatgtaca aacgaaaaag gtattagaaa gtacaaacct aaatatagat 1740
gattttgttg atgatccttt aagttatgtt aagacgccga gtaataaagt gttaggtttt 1800

tacccaacta atgcaaatac taacgctttt agaccggggg gcgttcaaga attaaatgaa 1860
tatcaattaa gtcaattatt tactgatcaa aaattacaag aagcagcaag aactagaaac 1920
ccaataagat taatgattgg tttcgactat cctgatggtt atggtaatag tgaaacttta 1980
gttcctgtta acttaacggt attacctgaa atccaacata atattaaatt ctttaaaaat 2040
gacgatactc aaaatattgc tgaaaaacca ttttcaaaac aagctgggca tccagttttc 2100
tatgtatatg caggtaacca agggaatgct tccgtgaatt taggtggtag cgtaacatct 2160

attcaaccat tacgtattaa tttaacaagt aatgagaatt ttacagataa agattggcaa 2220
attacaggta ttccgcgtac attacacatt gaaaactcga caaatagaac taataatgct 2280
agagaacgta acattgaact tgttggtaat ttattaccag gggattactt tggtacgata 2340
cgttttggac gtaaagaaca attatttgaa attcgtgtta aaccacatac accaacaatt 2400
acaacgacag ctgagcaatt aagaggtaca gcattacaaa aagtgcctgt taatatttcg 2460
ggaataccgt tggatccatc ggcattggtt tatttagttg caccaacaaa tcaaactacg 2520
aatggtggta gtgaggcaga tcaaatacca tctggttata cgatacttgc gactggtaca 2580
cctgatgggg tgcataatac aattactata cgaccgcaag attatgttgt attcatacca 2640
cctgtaggta aacaaattag agcagtagtt tattataata aagtagttgc atctaatatg 2700
agtaatgctg ttactatttt gccagatgac attccaccaa caatcaataa tcctgttgga 2760
ataaatgcca aatactatcg aggcgacgaa gtcaacttta caatgggagt ctctgataga 2820
cattctggta taaaaaatac aactattact actttgccaa gtggttggac atcaaattta 2880
actaaatccg acaacaaaaa cggctcatta gctattacag gtagagtctc tatgaatcag 2940
gcatttaaca gtgatattac atttaaagta tcagcgacag acaatgtcaa taatacgaca 3000
aatgatagtc aatctaaaca tgtgtcaatt catgtaggta aaattagtga agatgctcat 3060
ccgattgtat taggaaatac tgagaaagtt gtagtagtca atccgactgc tgtatctaat 3120
gatgaaaagc aaagcataat tactgccttt atgaataaaa accaaaatat aagaggatat 3180
ttagcatcaa ctgatccagt aactgtcgat aataatggta acgtcacatt acattaccgt 3240
gatggctcat caacaacgct tgatgctaca aatgtgatga catacgaacc agttgtgaaa 3300
tctgaatatc aaactgccaa tgctgctaaa acagcaacgg taacgattgc taaaggacaa 3360
tcatttaata ttggtgatat taaacaatat tttactttaa gtaatggaca agctattcca 3420
aatggcacat ttacaaatat tacatctgat agaactattc caactgcaca agaagttagt 3480
caaatgaatg caggtacgca gttatatcat atagttgctt caaatgcata tcataaagac 3540
actgaagatt tctatattag tttaaaaatc gttgatgtga aacaacctga aggcgatcaa 3600
cgtgtctatc gtacgtcaac atatgattta accactgatg aaatctcaaa agtaaaacaa 3660
gcttttatta atgcaaatag agatgtaatt acgcttgccg aaggtgatat ttcagttaca 3720
aatacaccta atggtgctaa tgtaagtact attacagtaa atattaataa aggtcgatta 3780
acgaaatcat tcgcgtctaa cctagctaat atgaatttct tgcgttgggt taatttccca 3840
caagattata cagtgacatg gacgaatgca aaaattgcaa acagaccaac agatggtggt 3900
ttatcatggt ccgatgacca taaatcttta atttatcgtt atgatgctac attaggcaca 3960
caaattacaa ctaatgatat tttaacgatg ctaaaagcga ctactacagt gcctggattg 4020
cgtaataata ttactggtaa tgaaaaagca caagcagaag caggtggaag accaaactat 4080

agaacaactg gttattcaca atcaaatgcg acaactgatg gtcaacgtca atttacgttg 4140
aatggtcaag tgattcaaat attagacatc atcaaccctt caaacggtta tggtgggcaa 4200
cctgttacaa attcaaatac tcgtgcaaac catagtaact caactgttgt taacgtaaac 4260
gaaccggcag ctaatggtgc tggcgcattt acaattgacc acgttgtaaa aagtaattct 4320
acacataatg caagtgatgc agtttataaa gcgcagttat acttaacgcc atatggtcca 4380
aaacaatatg ttgaacattt aaatcaaaat acaggaaata ctactgacgc tattaacatt 4440
tattttgtac caagtgactt agtgaatcca acaatttcag taggtaatta cactaatcat 4500
caagtgttct caggtgaaac atttacaaat acgattacag cgaatgataa ctttggtgtg 4560
caatcggtaa ctgtaccaaa tacatcacaa attacaggta ctgttgataa taaccatcaa 4620
catgtttctg caacggcacc aaatgtgaca tcagcaacta gtaagacaat caatttatta 4680
gcaactgata caagtggtaa tacagctaca acttcattca atgtaacagt gaaacctttg 4740
cgtgataaat atcgagttgg tacttcatca acggctgcta atcctgttag aattgccaat 4800
atttcgaata atgcgacagt atcacaagct gatcaaacga caattattaa ttcgttaacg 4860
tttacaagta atgcaccaaa tagaaactat gcaacagcaa gcgcaaatga aatcactagt 4920
aaaacagtta gtaatgtcag tcgtactgga aataatgcca atgtcacagt aactgttact 4980
catcaagatg gaacaacatc aacagtgact gtacctgtaa agcatgtcat tccagaaatc 5040
gttgcacatt cgcattacac tgtacaaggc caagacttcc cagcaggtaa tggttctagt 5100
gcagcagatt actttaagtt atctaatggt agtgccattc cagatgcaac gattacatgg 5160
gtaagtggac aagcgccaaa taaagataat acacgtattg gtgaagatat aacagtaact 5220
gcacatatct taattgatgg cgaaacaacg ccgattacga aaacagcaac atataaagta 5280
gtaagaactg taccgaaaca tgtctttgaa acagccagag gtgttttata cccaggtgtt 5340
tcagatatgt atgatgcgaa acaatatgtt aagccagtaa ataattcttg gtcgacaaat 5400
gcgcaacata tgaattttca atttgttgga acatatggtc ctaacaaaga tgttgtaggt 5460
atatcaacgc gtcttattag agtgacttat gataatagac aaactgaaga tttaactatt 5520
ttatctaaag ttaaacctga cccaccaaga attgacgcaa actctgtgac atataaagca 5580

ggtcttacaa accaagaaat taaagttaat aacgtattaa ataactcgtc agtaaaatta 5640
tttaaagcag ataatacacc attaaatgtc acaaatatta ctcatggtag tggttttagt 5700
tcggttgtga cagtaagtga cgcgttacca aatggcggaa ttaaagcaaa atcttcaatt 5760
tcaatgaaca atgtgacgta tacgacgcaa gacgaacatg gtcaagttgt tacagtaaca 5820
agaaatgaat ctgttgattc aaatgatagt gcttctgtta cagtaacacc acaattacaa 5880
gcaactactg aaggcgctgt atttattaaa ggtggcgacg gttttgattt cggtcatgta 5940
gaacgattta ttcaaaatcc gccacatggg gcaacggtcg catggcatga tagtccagat 6000

acatggaaga atacagtcgg caacacacat aaaactgcgg ttgtaacatt acctagtggt 6060
caaggtacgc gtaatgttga agttccagtc aaagtttatc cagttgctaa tgctaaggcg 6120
ccatcacgtg atgtgaaagg tcaaaatttg acacatggta caaacgctat tgattacatt 6180
acatttgatc caaatactaa tacgaatggt attacagcag catgggcaaa tagacaacaa 6240
ccaaataacc agcaagcagg cgttcaacat ttaaatgtcg atgtcacata tccaggtatt 6300
tcagctgcta aacgagttcc tgtaactgtg aacgtatatc aatttgaatt ccctcaaact 6360
acttatacaa caacagttgg tggcacttta gcaagtggta cgcaagcatc aggatatgca 6420
catatgcaaa acgcttcagg tttaccaaca gatggattta cgtataaatg gaatcgtgat 6480
actacgggta caaacgatgc aaactggcca gcaatgaata aaccaaatac tgcacaagtc 6540
gttaatgcaa aatatgatgt catctataat ggacatacat ttgcaacatc tttaccagcg 6600
aaatttgtag taaaagatgt tcaaccagcg aaaccaactg tcactgaaac agcggcagga 6660
gcgattacaa ttgcacctgg tgcgaaccaa acagtcaata ctcatgctgg taatgttacg 6720
acatatgctg acaaattagt tattaaacgt aatggaaatg ttgtaacgac atttacacgt 6780
cgtaataata cgagcccatg ggtgaaagaa gcatcagcag ataatgtaac aggtattgtt 6840
ggaactaata atggtattac tgtggcagca ggtactttca atcctgctga tacaattcaa 6900
gttgttgcaa cacaaggtag tggcgaaaca atcagtgacg agcaacgtag tgatgatttc 6960
acagttgtcg caccacaacc gaaccaagcg actacgaaaa tttggcaaaa tggtcatatt 7020
gatatcacgc ctaataatcc atcaggacat ttaattaatc caacacaagc aatggatatt 7080
gcttacactg aaaaagtggg taatggtgca gaacatagta agacaattaa tgttgttcgt 7140
ggtcaaaata atcaatggac aattgcgaat aagcctgact atgtaacgtt agatgcacaa 7200
actggtaaag tgacgttcaa tgccaatact ataaaaccaa attcatcaat cacaattact 7260
ccgaaagcag gtacaggtca ctcagtaagt agtaatccaa gtacattaac tgcaccggca 7320
gctcatactg tcaacacaac tgaaattgtg aaagattatg gttcaaatgt aacagcagct 7380
gaaattaaca atgcagttca agttgctaat aaacgtactg caacgattaa aaatggcaca 7440
gcaatgccta ctaatttagc tggtggtagc acaacgacga ttcctgtgac agtaacttac 7500
aatgatggta gtactgaaga agtacaagag tccattttca caaaagcgga taaacgtgag 7560
ttaatcacag ctaaaaatca tttagatgat ccagtaagca ctgaaggtaa aaagccaggt 7620
acaattacgc agtacaataa tgcaatgcat aatgcgcaac aacaaatcaa taccgcgaaa 7680
acagaagcac aacaagtgat taataatgag cgtgcaacac cacaacaagt ttctgacgca 7740
ctaactaaag ttcgtgcagc acaaactaag attgatcaag ctaaagcatt acttcaaaat 7800
aaagaagata atagccaatt agtaacgtct aaaaataact tacaaagttc tgtgaaccaa 7860
gtaccatcaa ctgctggtat gacgcaacaa agtattgata actataatgc gaagaagcgt 7920

gaagcagaaa ctgaaataac tgcagctcaa cgtgttattg acaatggcga tgcaactgca 7980
caacaaattt cagatgaaaa acatcgtgtc gataacgcat taacagcatt aaaccaagcg 8040
aaacatgatt taactgcaga tacacatgcc ttagagcaag cagtgcaaca attgaatcgc 8100
acaggtacaa cgactggtaa gaagccggca agtattactg cttacaataa ttcgattcgt 8160
gcacttcaaa gtgacttaac aagtgctaaa aatagcgcta atgctatcat tcagaagcca 8220
ataagaacag tgcaagaggt acaatctgcg ttaacaaatg taaatcgtgt caatgagcga 8280
ttaacgcaag caattaatca attagtacct ttagctgata atagtgcttt aagaactgct 8340
aagacgaaac ttgatgaaga aatcaataaa tcagtaacta ctgatggtat gacacaatca 8400
tcaatccaag catatgaaaa tgctaaacgt gcaggtcaaa cagaaacaac aaatgcacaa 8460
aatgttatta acaatggtga cgcgacagac caacaaattg ccgcagaaaa aacaaaagta 8520
gaagaaaaat ataatagctt aaaacaagca attgctggat taacaccaga cttggcacca 8580
ttacaaactg caaaaactca gttgcaaaat gatattgatc agccaacgag tacgactggt 8640
atgacaagcg catctgttgc tgcatttaat gacaaacttt cagcagctag aactaaaatt 8700
caagaaattg atcgcgtact agcatctcat ccagatgtag caacgattcg tcaaaacgtg 8760
acagcagcga atgctgctaa aacagcactt gatcaagcgc gcaatggctt aacagtcgat 8820
aaagcacctt tagaaaatgc gaaaaatcaa ctacaacata gtattgatac gcaaacaagt 8880
acaactggta tgacacaaga ctctataaat gcatacaatg cgaagttaac agctgcacgt 8940
aataaggttc aacaaatcaa tcaagtatta gcaggttcac ctactgtaga tcaaattaat 9000
acaaatacgt ctgcagcaaa tcaagcgaaa tctgatttag atcatgcacg tcaagcgtta 9060
acaccagata aagcgccgct tcaaaatgcg aaaacgcaat tagaacaaag cattaatcaa 9120
ccaacagata caacaggtat gacaaccgct tcgttaaatg catacaacca aaaattacaa 9180
gcagcacgtc aaaagttaac tgaaattaat caagtgttga atggcaaccc aactgtccaa 9240
aatatcaatg ataaagtggc agaggcaaac caagctaagg atcaattaaa tacagcacgt 9300
caaggtttaa cattagatag acagccagcg ttaacaacat tacatggtgc atctaactta 9360

aaccaagcac aacaaaataa tttcacgcaa caaattaatg ctgctcaaaa tcatgctgcg 9420
cttgaaacaa ttaagtctaa cattacggct ttaaatactg cgatgacgaa attaaaagac 9480
agtgttgcgg ataataatac aattaaatca ggtcaaaatt acactgacgc aacaccagct 9540
aataaacaag cctatgataa tgcagttaat gcggctaaag gtgtcattgg agaaacgact 9600
aatccaacga tggatgttaa cacagtgaac caaaaagcag catctgttaa atcgacgaaa 9660
gatgctttag atggtcaaca aaacttacaa cgtgcgaaaa cagaagcaac aaatgcgatt 9720
acgcatgcaa gtgatttaaa ccaagcacaa aagaatgcat taacacaaca agtgaatagt 9780
gcacaaaacg tgcaagcagt aaatgatatt aaacaaacga ctcaaagctt aaatactgct 9840

atgacaggtt taaaacgtgg cgttgctaat cataaccaag tcgtacaaag tgataattat 9900
gtcaacgcag atactaataa gaaaaatgat tacaacaatg catacaacca tgcgaatgac 9960
attattaatg gtaatgcaca acatccagtt ataacaccaa gtgatgttaa caatgcttta 10020
tcaaatgtca caagtaaaga acatgcattg aatggtgaag ctaagttaaa tgctgcgaaa 10080
caagaagcga atactgcatt aggtcattta aacaatttaa ataatgtaca acgtcaaaac 10140
ttacaatcgc aaattaatgg tgcgcatcaa attgatgcag ttaatacaat taagcaaaat 10200
gcaacaaact tgaatagtgc aatgggtaac ttaagacaag ctgttgcaga taaagatcaa 10260
gtgaaacgta cagaagatta tgcggatgca gatacagcta aacaaaatgc atataacagt 10320
gcagtttcaa gtgctgaaac aattattaat caaacagcta atccgacaat gtctgttgat 10380
gatgttaatc gtgcaacttc agctgttact actaataaaa atgcattaaa tggtgatgaa 10440
aaattagtac aatctaaaac agatgctgca agagcaattg atgcattacc acatttaaat 10500
aatgcacaaa aagcagatgt taaatctaaa attaatgctg catcaaatat tgctggtgta 10560
aataccgtta aacaacaagg tacagattta aatacagcga tgggtaactt gcagggtgca 10620
atcaatgatg aacaaacgac gcttaatagt caaaattatc aagatgcgac acctagtaag 10680
aaaacagcat acacaaatgc ggtgcaagct gcgaaagata ttttaaataa atcaaatggt 10740
caaaataaaa cgaaagatca agttactgaa gcgatgaatc aagtgaattc ggctaaaaat 10800
aacttagatg gtacgcgttt attagatcaa gcgaagcaaa cagcgaaaca gcagttaaat 10860
aatatgacgc atttaacaac tgcacaaaaa acgaatttaa caaatcaaat taatagtggt 10920
actactgttg ctggtgttca tacggttcaa tcaaatgcca acacattaga tcaagcgatg 10980
aatacgttaa gacaaagtat tgctaacaat gatgcgacta aagcaagtga agattacgta 11040
gatgctaata atgataagca aacagcatat aacaacgcgg tagctgctgc tgaaacgatt 11100
attaatgcga atagtaatcc agaaatgaat ccaagtacga ttacacaaaa agcagagcaa 11160
gtgaatagtt ctaaaacggc acttaacggt gatgaaaact tagctacggc aaaacaaaat 11220
gcgaaaacgt acttaaacac attaacgagt attacagatg ctcaaaagaa caatttgatt 11280
agtcaaatta gtagtgcgac aagagtgagt ggtgttgata ctgtaaaaca aaatgcacaa 11340
catttagatc aagctatggc taacttacaa aatggtatta acaacgaatc tcaagtgaaa 11400
tcatctgaga aatatcgtga tgctgataca aataaacaac aagagtatga taatgctatt 11460
actgcagcga aagcgatttt aaataaatcg acaggtccaa acactgcgca aaatgcagtt 11520
gaagcagcat tgcaacgtgt taatactgcg aaagatgcat tgaatggtga tgcaaaatta 11580
attgcagctc aaaacgcagc gaaacaacat ttaggtactt taacgcatat cactacagca 11640
caacgcaatg atttaacaaa tcaaatttca 11670

<210> 46
<211> 20139
<212> DNA
<213> Staphylococcus aureus

<400> 46
atgggtaact tacaaacggc tatcaacgat aagtcaggaa cattagcgag ccaaaacttc 60
ttggatgctg atgagcaaaa acgtaatgct tacaatcaag ctatatcagc tgccgaaacc 120
attttaaata aacaaactgg accgaataca gcgaaaacag cggttgaaca agcacttaat 180
aatgttaata gtgcgaaaca tgcattaaat ggtacgcaaa acttaaataa tgcgaaacaa 240
gcagcgatta cagcaattaa tggcgcatct gatttaaatc aaaaacaaaa agatgcatta 300
aaagcacaag ctaatggtgc tcaacgcgta tctaatgcaa atgatgtaca acgtaatgcg 360
actgaactga acacggcaat gggtcaatta caacatgcca tcgcagataa gacgaatacg 420
ttagcaagca gtaaatatgt caacgccgat agcactaaac aaaatgctta cacaactaaa 480
gttaccaatg ctgaacatat tattagcggt acgccaacgg ttgttacaac accttcagaa 540
gtaacagctg cagctaatca agtaaacagc gcgaaacaag aattaaatgg tgacgaaaga 600
ttacgtgttg caaaacaaaa cgccaatact gctattgatg cattaacgca attaaatact 660
cctcaaaaag ctaaattaaa agaacaagtg ggacaagcca atagattaga agacgtacaa 720
tctgttcaaa caaatggaca atcattgaac aatgcaatga aaggcttaag agatagtatt 780
gctaacgaaa caacagtcaa agcaagtcaa aactatacag acgcaagtcc gaataaccaa 840
tcaacatata atagcgctgt gtcaaatgcg aaaggtatca ttaatcaaac taacaatcca 900
actatggata ctagtgcgat tacccaagct acaacacaag tgaataatgc taaaaatggt 960

ttaaacggtg ctgaaaactt aagaaatgca caaaacactg ctaagcaaaa cttaaatacg 1020
ttatcacact taacaaataa ccaaaaatct gcaatctcat cacaaattga tcgtgcaggt 1080
catgtgagtg aggtaacagc tgctaaaaat gcagcaactg agttaaacgc gcaaatgggc 1140
aacttggaac aagctatcca tgatcaaaac acagttaaac aaggtgttaa cttcactgat 1200
gcagataaag ctaaacgtga tgcttataca aatgcggtaa gcagagcaga aacaattctg 1260
aataaaacgc aaggtgcaaa tacgtctaaa caagatgttg aagcggctat tcaaaatgtt 1320
acaagtgcta aaaatgcatt gaatggtgat caaaacgtta caaatgcgaa gaatgcagct 1380
aaaaatgcat taaataactt aacgtcaatt aataatgcac aaaaacgtga cttaacaact 1440
aaaattgatc aagcaacaac agtagctggt gttgaagcgg tatctaatac aggtacacaa 1500
ttgaatacag cgatggctaa cttgcaaaat ggtattaatg ataaagcgaa tactttagcg 1560
agcgaaaact atcatgatgc tgattcagat aagaaaactg cttatactca agccgttacg 1620
aacgcagaaa atattttaaa taaaaatagt ggatcaaatt tagataaagc tgccgttgaa 1680
aacgcgttgt cacaagtgac aaatgcgaaa ggtgccctaa atggtaacca taatttagag 1740
caagctaaat caaatgcaaa cactactata aacggccttc aacatttaac aacagcacaa 1800
aaagataaat tgaaacaaca agtgcaacaa gcacaaaatg ttgcaggtgt agatactgtt 1860
aaatcaagtg ccaacacatt aaatggtgct atgggtacgt taagaaatag catacaagat 1920
aacacagcta cgaaaaatgg ccaaaactat cttgatgcta cagaacgtaa caaaacaaac 1980
tataacaatg ctgttgatag tgctaatggt gtcattaatg caacaagcaa tccaaatatg 2040
gatgctaatg caattaacca aatcgctaca caagtgacat caacgaaaaa tgcattagat 2100
ggtacacata atttaacgca agcgaaacaa acagcaacaa atgccatcga tggtgctact 2160
aacttaaata aagcgcaaaa agatgcgtta aaagcacaag ttacaagtgc gcaacgtgtt 2220
gcaaatgtaa caagtatcca acaaactgca aatgaactta atacagctat gggtcaatta 2280
caacatggta ttgatgatga aaatgcaaca aaacaaactc aaaaatatcg tgacgctgaa 2340
caaagtaaga aaactgctta tgatcaagct gtagctgctg cgaaagcaat tttaaataaa 2400
caaacaggtt ccaattcaga taaagcagca gttgaccgtg cattacaaca agtaacaagt 2460
acgaaagatg cattgaatgg ggatgctaaa ctggcagaag cgaaagcggc agctagacaa 2520
aacttaggta ctttaaacca tattacgaat gcacaacgta ctgcgttaga aggtcaaatc 2580
aatcaagcga cgactgttga tggcgttaat actgtaaaaa caaatgccaa tacattagac 2640
ggcgctatga atagcttaca aggtgcaatc aatgataaag atgcgacatt aagaaatcaa 2700
aattatcttg atgcagatga atcaaaacga aatgcatata cgcaagctgt cacagcggct 2760
gaaggcattt taaataaaca aacaggtggt aacacatcta aagcagacgt tgataatgca 2820
ttaaatgcag ttacaagagc gaaagcggct ttaaatggtg ctgaaaactt aagaaatgcg 2880
aaaacttcag caacaaatac gattaatggt ttacctaact taacacaatt acaaaaagac 2940
aacttgaagc atcaagttga acaagcgcaa aatgtagttg gtgtaaatgg tgttaaagat 3000
aaaggtaata cattaaatac tgccatgggt gcattacgta caagtatcca aaatgataat 3060
acgacgaaaa caagtcaaaa ttatcttgat gcatctgata gcaacaaaaa taattacaat 3120
actgctgtaa ataatgcaaa tggtgttatt aatgcaacga acaatccaaa tatggatgct 3180
aatgcgatta atgacatggc aaatcaagtc aatacaacaa aagcagcgtt aaatggtgca 3240
caaaacttag ctcaagctaa aacaaatgcg acgaacacaa ttaacaacgc gcaagactta 3300
aaccaaaaac aaaaagatgc attaaaaaca caagttaaca atgcacaacg tgtatctgat 3360
gcaaataacg ttcaacatac agctactgaa ttgaacggtg cgatgacagc acttaaagca 3420
gctattgcgg ataaagaaag aacaaaagca agcggtaatt atgtcaatgc tgatcaagaa 3480
aaacgtcaag cgtatgattc aaaagtgact aacgctgaaa atatcattaa tggtacacca 3540
aatgcgacat taacagtcaa tgacgtaaat agtgcggcat cacaagtcaa tgcggctaaa 3600
acagcattaa atggtgataa caacttacgt gtagcgaaag agcatgctaa caatacaatt 3660
gacggcttag cacaattgaa taatgtacaa aaagcaaaat taaaagaaca agttcaaagt 3720
gcaactacat tagatggtgt tcaaactgtt aaaaatagtt ctcaaacgtt gaatacagcg 3780
atgaaaggct taagagatag tattgcgaat gaagcaacga ttaaagcagg tcaaaactac 3840
actgacgcaa gtccaaataa tcgtaacgag tacgacagcg cagttactgc agcaaaagca 3900
atcattaatc aaacatcgaa cccaacgatg gaaccaaata ctattacgca agcaacatca 3960
caagtgacaa ctaaagaaca tgcattaaat ggtgcgcaaa acttagctca agctaagaca 4020
acagcgaaaa acaacttgaa taacttaaca tcaattaaca atgcacaaaa agatgcgtta 4080
acgcgtaaca ttgatggtgc aactacagta gctggtgtaa atcaagaaac tgcaaaagca 4140
acagaattaa ataacgcaat gcacagttta caaaatggta tcaatgatga gacacaaaca 4200
aaacaaactc agaaatacct agatgctgag ccaagtaaga aatcagctta tgatcaagca 4260
gtaaatgcag caaaagcaat tttaacaaaa gctagtggtc aaaatgtaga caaagcagca 4320
gttgaacaag cattacaaaa tgtgaacagt acgaagacgg cgttgaacgg tgatgcgaaa 4380
ttaaatgaag ctaaagctgc tgcgaaacaa acgttaggta cattaacaca cattaataat 4440
gcacaacgta atgcgttaga taatgaaatt acacaagcaa caaatgttga aggtgttaat 4500
acagttaaag ccaaagcgca acaattagat ggtgctatgg gtcaattaga aacatcaatt 4560
cgtgataaag acacgacgtt acaaagtcaa aattatcaag atgctgatga tgctaaacga 4620
acggcttatt ctcaagcagt aaatgcagca gcaactattt taaataaaac agctggagga 4680
aatacaccta aagcagatgt cgaaagagca atgcaagctg ttacacaagc caatactgca 4740
ttaaacggta ttcaaaactt agaacgtgcg aaacaggctg cgaacacagc gattacaaat 4800
gcttcggact taaatacaaa acaaaaagaa gcattgaaag cacaagtaac aagtgcagga 4860

cgcgtatctg cagcaaatgg tgttgaacat actgcgactg aattaaatac tgcgatgaca 4920
gctttaaaac gtgccattgc tgataaagct gacacaaaag ctagtggtaa ttatgtcaat 4980
gctgatgcga ataaacgcca agcatatgat gaaaaagtga cagctgcaga acatatcgtt 5040
agtggtacac caacaccaac gttaacacca tcagatgtta caaatgcagc aacgcaagta 5100
acgaatgcga agacgcagtt aaacggtaat cataatttag aagtagcgaa acaaaatgct 5160
aacacagcaa ttgatggttt aacttcttta aatggtccgc aaaaagcaaa acttaaagaa 5220
caagtgggtc aagcgacgac gttgccaaat gttcaaactg ttcgtgataa tgcacaaaca 5280
ttaaacactg caatgaaagg tctacgagat agcattgcga atgaagcaac gattaaagca 5340
ggtcaaaact acacagatgc aagtcaaaac aaacaaaatg actacaacaa tgcagtcact 5400
gcagcaaaag caatcattgg tcaaacaact agtccatcaa tgattgcgca agaaattaat 5460
caagcgaaag accaagtgac agctaaacaa caagcgttaa acggtcaaga aaacttaaga 5520
actgcgcaaa caaatgcgaa gcaacatttg aatggcttaa gtgacttaac taatgcacaa 5580
aaagatgcag cgaaacgcca aatcgaaggt gcaacgcatg ttaatgaagt aacacaagcg 5640
caaaataatg cggacgcatt aaatacagct atgacgaact tgaaaaatgg tattcaagat 5700
caaaatacga ttaagcaagg tgttaacttc actgatgcag atgaagcgaa acgtaatgca 5760
tatacaaatg cagtgacgca agctgaacaa attttaaata aagcacaagg tccaaatact 5820
gcaaaagacg gtgtcgaaac tgcgttacaa aatgtacaac gtgctaaaaa cgaattgaac 5880
ggtaatcaaa atgttgcgaa cgctaagaca actgcgaaaa atgcattgaa taaccttaca 5940
tcaattaata atgcacaaaa agcagcattg aaatcacaaa ttgaaggtgc gacaacagtt 6000
gcaggtgtaa atcaagtgtc tacaatggca tctgaattaa atactgcaat gagcaactta 6060
caacgtggta ttaatgacga agcagctaca aaagcagctc agaaatatac tgaagcagat 6120
agagataaac aaactgcata caatgatgct gtaacagcag ctaaaacgtt attagataaa 6180
acagctggtt caaatgacaa taaagtagcc gttgaacaag cattacaacg tgtgaatact 6240
gctaaaacag cattaaatgg tgacgcgcga ttaaatgaag cgaagaacac agctaaacaa 6300
caattagcga caatgtcaca tttaactaat gctcaaaaag caaacttaac agaacaaatt 6360
gaacgtggta caactgttgc tggtgttcaa ggcatccaag caaatgctgg tactttaaat 6420
caagcaatga atcaattaag acaaagtatt gcttctaaag atgcgactaa atcaagcgaa 6480
gattatcaag acgcgaatgc agatttacaa aatgcataca atgatgcggt aactaatgct 6540
gaaggtatta ttagtgcaac gaataaccct gaaatgaatc ctgatacaat taaccaaaaa 6600
gcgagccaag tgaacagtgc gaagtctgca ttgaacggtg atgaaaaatt agcagcagta 6660
aaacaaactg cgaaatcaga tatcggtcgt ttgacagact tgaacaatgc acaacgaact 6720
gcggcaaatg ctgaagtgga tcaagcacca aatcttgcag ctgtcacagc ggctaaaaat 6780
aaagcaacat cgttaaacac agcgatgggt aatttgaaac atgcacttgc tgaaaaggat 6840
aatacgaaac gtagtgtcaa ttacacagat gcggatcaac caaaacaaca agcgtatgat 6900
actgcagtta cacaagcaga agcaattact aatgcaaatg gcagtaacgc gaatgaaaca 6960
caagttcaag cagcgcttaa ccaattgaat caagctaaaa acgacttgaa tggtgataat 7020
aaagttgctc aagcgaaaga aacagcaaaa cgtgcattag cttcatatag taacttgaat 7080
aacgcgcaat caactgcagc aactagtcaa attgacaatg caacgacagt agcagacgta 7140
actgctgcac aaaatactgc taatgaatta aatacagcaa tgggtcaact tcaaaatggt 7200
attaatgacc aaaacactgt taaacaacaa gtgaacttta cagatgctga ccaaggtaag 7260
aaagatgctt acacaaatgc tgttacgaat gctcaaggta ttttagataa agcaaacggt 7320
caaaatatga caaaagcaca agttgaagct gcattaaatc aagtaacgac tgctaagaat 7380
gctttaaacg gtgatgcaaa tgtaagacaa gcaaaatcag atgcgaaagc aaacttaggt 7440
acattaacac acttaaataa tgcacaaaaa caagatttaa catcacaaat cgaaggtgca 7500
acaacagtca acggtgtaaa tagtgttaaa acgaaagcac aagacttaga tggtgcaatg 7560
caacgattag agtcagcaat cgcaaataaa gatcaaacta aagcgagcga aaactacatt 7620
gacgcagatc caactaagaa aacagcattt gataatgcca tcacacaagc tgaatcttac 7680
ttaaataaag atcatggtac gaataaagat aagcaagctg ttgaacaagc aattcaaagt 7740
gtaacgtcta ctgaaaatgc tttgaacggt gacgcgaact tacaatgcgc taaaactgaa 7800
gctacacaag ctatcgataa cttgacacaa ttgaatacac cgcaaaaaac agcattgaaa 7860
caacaagtga atgctgcaca acgcgtatca ggtgtaactg atctgaaaaa tagtgctaca 7920
tcacttaata atgcgatgga tcaattaaaa caagcaattg gtgatcatga cacaattgta 7980
gctggtggta attacactaa cgcaagtcct gataaacaag gtgcttacac tgatgcatat 8040
aatgctgcga agaatatcgt aaatggttca cctaatgtga ttacaaatgc agcagatgtt 8100
actgcggcaa cacaacgtgt caataatgct gaaacaagtt taaatggtga tacaaactta 8160
gcaactgcga agcaacaagc taaagatgca ttacgtcaaa tgacacattt atctgatgca 8220
caaaaacaaa gtattactgg tcaaattgat agcgcgacac aagtaactgg tgtacaaagt 8280
gtgaaagaca atgcaacaaa tcttgacaat gcaatgaatc aacttcgaaa tagtattgcg 8340
aataaagatg aagtaaaagc gagtcaacca tatgttgatg cagatacaga taaacaaaat 8400
gcatacaata cagcagttac aagtgctgaa aatatcatta atgcaacgag tcagccaaca 8460
cttgatccat ctgcagtaac acaagcagct aatcaagtga acactaacaa aactgcgctt 8520
aatggtgcgc aaaacttagc aaataaaaag caagaaacaa ctgctaacat caaccgatta 8580
agtcatttaa acaatgctca aaagcaagat ttaaatacac aagtgacaaa tgcaccaaat 8640
attagcacag taaatcaagt gaaaactaaa gctgaacaat tagatcaagc aatggaacgt 8700
ttaatcaacg gaatccaaga caaagatcaa gtgaaacaaa gtgttaactt tacagatgca 8760

gatccagaaa aacaaacagc atacaacaat gcggtaactg ctgctgaaaa tattattaat 8820
caagcaaatg gtacaaatgc gaaccaatca caagttgaag cagcactttc aactgtaaca 8880
actactaaac aagcgttgaa tggtgataga aaagtaacag atgctaaaaa caatgcaaac 8940
caaacattat ctacgttaga taacttaaac aatgcacaaa aaggtgctgt tactggaaac 9000
atcaatcaag cgcacactgt agctgaagta acgcaagcca ttcaaaccgc tcaggaactg 9060
aatacagcga tgggtaactt gaaaaatagc ttgaatgata aagacactac acttggcagt 9120
caaaactttg cagatgcaga tccagagaag aaaaatgcat acaatgaagc ggttcgtaat 9180
gctgaaaata ttttaaataa atctacaggt acgaacgtgc ctaaagatca agttgaagca 9240
gctatgaatc aagtgaatac tacaaaagca gcgcttaatg gtactcaaaa ccttgaaaaa 9300
gcgaaacaac acgcaaatac agcaattgac ggtttaagcc atttaacaaa tgcacaaaaa 9360
gaggcattaa aacaattggt acaacaatcg actactgttg cagaagcaca aggtaatgaa 9420
caaaaagcaa acaatgttga tgcagcaatg gacaaattac gtcaaagtat tgcagataat 9480
gcgacaacaa aacaaaacca aaattatact gatgcaagtc cgaataaaaa ggatgcgtac 9540
aataatgctg tcacaactgc acaaggtatt attgatcaaa ctacaaaccc ttcattagat 9600
ccgactgtta tcaatcaagc tgctggacaa gtaagcacgt ctaaaaatgc tttaaatggt 9660
aatgaaaact tagaggcagc gaagcaacaa gcaacgcaat ctttaggttc attagacaac 9720
ttaaataatg cgcaaaaaca agctgttact aatcaaatta atggcgcgca tactgttgat 9780
gaagcaaatc aaattaagca aaatgcgcaa aacttaaata ctgcgatggg taacttgaaa 9840
caagcgatag ctgataaaga tgctacgaaa gcaacagtta acttcactga tgcagatcaa 9900
gcaaaacaac aagcatataa cactgcagtt acaaatgctg aaaatatcat ttcaaaagct 9960
aatggtggta atgcaacaca aactgaagtt gaacaagcaa tccaacaagt aaatgcagca 10020
aaacaagcat taaatggtaa tgccaacgtt caacatgcaa aagacgaagc aacagcatta 10080
attaataact ctaatgatct taaccaagca cagaaagatg cattaaaaca acaagtacaa 10140
aatgcaacta ctgtagctgg tgtaaacaat gttaaacaaa cggcgcaaga gttaaacaat 10200
gcgatgacac aattaaaaca aggcattgca gataaagaac aaacaaaagc tgatggtaac 10260
tttgtcaatg cagattctga caagcaaaat gcatataatc aagcagtagc gaaagctgaa 10320
gcattaatta gtggtacgcc tgatgttgtc gttacaccta gcgaaattac tgcagcgtta 10380
aataaagtta cgcaagctaa aaatgattta aatggtaata caaacttagc aacggcgaaa 10440
caaaatgttc aacatgctat tgatcaattg ccaaacttaa accaagcgca acgtgatgaa 10500
tacagcaaac aaatcacgca agcaacactt gtaccaaacg tcaatgctat tcaacaagcg 10560
gcaacaacgc ttaatgacgc gatgacacaa ttgaaacaag gtattgcgaa taaagcacaa 10620
attaaaggta gcgagaacta tcacgatgct gatactgaca agcaaacagc atatgataat 10680
gcagtaacaa aagcagaaga attgttaaaa caaacaacaa atccaacaat ggatccaaat 10740
acaattcaac aagcattaac taaagtgaat gacacaaatc aagcacttaa cggtaatcaa 10800
aaattagctg atgccaaaca agatgctaag acaacacttg gtacactaga tcatttaaat 10860
gatgctcaaa aacaagcgct aacaactcaa gttgaacaag caccagatat tgcaacagtt 10920
aataatgtta agcaaaatgc tcaaaatctg aataatgcta tgactaactt aaacaatgca 10980
ttacaagata aaactgagac attaaatagc attaacttta ctgatgcaga tcaagctaag 11040
aaagatgatt atactaatgc ggtttcacat gcagaaggta ttttatctaa agcaaatggc 11100
agcaatgcaa gtcaaactga agtggaacaa gcgatgcaac gtgtgaacga agcgaaacaa 11160
gcattgaatg gtaatgacaa tgtacaacgt gcaaaagatg cagcgaaaca agtaattaca 11220
aatgcaaatg atttaaatca agcgcaaaaa gatgcattaa aacaacaagt cgatgctgcg 11280
caaactgttg caaatgtaaa cacgattaag caaacagcac aagatttaaa tcaagcaatg 11340
acacaattga aacaaggtat tgcagataaa gaccaaacta aagcaaatgg taactttgtc 11400
aatgctgata ctgataagca aaatgcatat aacaatgcgg tagcgcatgc tgaacaaatc 11460
attagtggta caccaaatgc aaacgtggat ccacaacaag tggctcaagc gttacaacaa 11520
gtgaatcaag ctaagggtga tttaaacggt aaccacaact tacaagttgc taaagacaat 11580
gcaaatacag ccattgatca gttaccaaac ttaaatcaac cacaaaaaac agcattaaaa 11640
gaccaagtgt cgcatgcaga acttgttaca ggtgttaatg ctattaagca aaatgctgat 11700
gcgttaaata atgcaatggg tacgttgaaa caacaaattc aagcgaatag tcaagtacca 11760
caatcagttg actttacaca agcggatcaa gacaaacaac aagcttataa caatgcagct 11820
aaccaagcgc aacaaatcgc aaatggcaca ccaacacctg tattggcgcc tgatacagta 11880
acaaaagcag ttacaactat gaatcaagcg aaagatgcat taaacggtga tgaaaaatta 11940
gcgcaagcga aacaagatgc tttagcaaat cttgatacgt tacgtgactt aaatcaacca 12000
caacgtgatg cattacgaaa ccaaatcaat caagcacaag ctttagctac agttgaacaa 12060
actaaacaaa atgcacaaaa tgtgaataca gcaatgggta acttgaaaca aggtattgca 12120

aataaagata ctgtgaaagc aagtgagaac taccacgatg ctgatgtcga taagcaaaca 12180
gcatatacaa atgcagtgtc tcaagcggaa ggtattatca atcaaacgac aaatccaacg 12240
cttaacccag atgacattac tcgtgcatta actcaagtga ctgatgctaa aaatagctta 12300
aacggtgaag ctaaattagc cactgaaaag caaaatgcta aagatgccgt aagtggaatg 12360
acgcatttaa acgatgctca aaaacaagca ttaaaaggtc aaatcgatca atcgcctgaa 12420
attgctacag tgaaccaagt taaacaaaca gcaacgagcc tagatcaagc aatggatcaa 12480
ttatcacaag ctattaatga taaagatcaa atattagcgg acggtaatta cttaaatgca 12540
gatcctgaca aacaaaatgc gtataaacag gcagtagcaa aagctgaagc attattgaat 12600

aaacaaagtg gtactaatga agtacaagca caagttgaaa gcatcactaa tgaagtgaac 12660
gcagcgaaac aagcattaaa tggtaatgac aatttggcaa atgcaaaaca acaagcaaaa 12720
caacaattgg cgaacttaac acacttaaat gatgcacaaa aacaatcatt tgaaagtcaa 12780
attacacaag cgccacttgt tacagatgtc actacgatta atcaaaaagc acaaacgtta 12840
gatcatgcga tggaattatt aagaaatagt gttgcggata atcaaacgac attagcgtct 12900
gaagattatc atgatgcaac tgcgcaaaga caaaatgact ataacaaagc tgtaacagct 12960
gctaataata tcattaatca aactacatcg cctacgatga atccagatga tgttaatggt 13020
gcaacgacac aagtgaataa tacgaaagtt gcattagatg gtgatgaaaa ccttgcagca 13080
gctaaacaac aagcaaacaa cagacttgat caattagatc atttgaataa tgcgcaaaag 13140
caacagttac aatcacaaat tacgcaatca tctgatattg ctgcagttaa tggtcacaaa 13200
caaacagcag aatctttaaa tactgcgatg ggtaacttaa ttaatgcgat tgcagatcat 13260
caagccgttg aacaacgtgg taacttcatc aatgctgata ctgataaaca aactgcttat 13320
aatacagcgg taaatgaagc agcagcaatg attaacaaac aaactggtca aaatgcgaac 13380
caaacagaag tagaacaagc tattactaaa gttcaaacaa cacttcaagc gttaaatgga 13440
gatcataatt tacaagttgc taaaacaaat gcgacgcaag caattgatgt tttaacaagc 13500
ttaaatgatc ctcaaaaaac agcattaaaa gaccaagtta cagctgcaac tttagtaact 13560
gcagttcatc aaattgaaca aaatgcgaat acgcttaacc aagcaatgca tggtttaaga 13620
cagagcattc aagataacgc agcaactaaa gcaaatagca aatatatcaa cgaagatcaa 13680
ccagagcaac aaaactatga tcaagctgtt caagccgcaa ataatattat caatgaacaa 13740
actgcaacat tagataataa tgcgattaat caagtagcgg caactgtgaa tacaacgaaa 13800
gcagcattac atggtgatgt gaaattacaa aatgataaag atcatgctaa acaaacggtt 13860
agccaattag cacatctaaa caatgcacaa aaacatatgg aagatacgtt aattgatagt 13920
gaaacaacta gaacagcagt taagcaagat ttgactgaag tacaagcatt agatcaactt 13980
atggatgcat tacaacaaag tattgctgac aaagatgcaa cacgtgcgag cagtgcatat 14040
gtcaatgcag aaccgaataa aaaacaagcc tatgatgaag cagttcaaaa tgctgagtct 14100
atcattgcag gattaaataa tccaactatc aataaaggta atgtatcaag tgcgactcaa 14160
gcagtaatat catctaaaaa tgcattagat ggtgttgaac gattagctca agataagcaa 14220
actgctggaa attctctaaa tcatttagat caattaacac cagctcaaca acaagcgcta 14280
gaaaatcaaa ttaataatgc aacaacttgt gataaagtgg ctgaaatcat tgcacaagcg 14340
caagcattaa atgaagcgat gaaagcatta aaagaaagta ttaaggatca accacaaact 14400
gaagcaagta gtaaatttat taacgaggat caagcgcaaa aagatgcata tacgcaagca 14460
gtacaacacg cgaaagattt gattaacaaa acaactgatc ctacattagc taaatcaatc 14520
attgatcaag cgacacaggc agtgactgat gctaaaaaca atttacatgg tgatcaaaaa 14580
ctagctcaag ataagcaacg tgcaacagaa acgttaaata acttgtctaa cttgaataca 14640
ccacaacgtc aagcacttga aaatcaaatc aataatgcag caactcgtgg tgaagtagca 14700
caaaaattaa ctgaagcaca agcacttaac caagcaatgg aagctttacg taatagcatt 14760
caagatcaac aacaaacaga atctggtagc aagtttatta atgaagataa accgcaaaaa 14820
gatgcttacc aagcagcagt tcaaaatgca aaagatttaa ttaaccaaac aggtaatcca 14880
acgcttgata aagcacaagt tgaacaattg acacatgctt ttaaacaagc taaagataac 14940
ctacacggtg atcaaaaact tgcagacgat aaacaacatg cggttactga tttaaatcaa 15000
ttaaatggtt tgaataatcc gcaacgtcaa gcacttgaaa gccaaataaa caacgcagca 15060
actcgtggcg aagtagcgca aaaattagct gaagcaaaag cgcttgatca agcaatgcaa 15120
gcattacgaa atagtattca agatcaacaa caaacggaag cgggtagcaa gtttatcaat 15180
gaagataaac cgcaaaaaga tgcttaccaa gcagcagttc aaaatgcaaa agatttaatt 15240
aaccaaacag gtaatccaac actcgacaaa tcacaagtag aacaattaac acaagcagta 15300
acaactgcaa aagataatct acatggtgat caaaaacttg ctcgtgatca acaacaagca 15360
gtaacaactg taaatgcatt gccaaactta aatcatgcac aacaacaaac attaactgat 15420
gctataaatg cagcgcctac aagaacagag gttgcacaac atgttcaaac tgctactgaa 15480
cttgatcacg cgatggaaac attgaaaaat aaagttgatc aagtgaatac agataaggct 15540
caaccaaatt acactgaagc gtcaactgat aaaaaagaag cagtagatca agcgttacaa 15600
gctgcacaaa gcattacaga tccaactaat ggttcaaatg cgaataaaga cgctgtagaa 15660
caagcattaa ctaagcttca agaaaaagtg aatgagttaa atggtaatga gagagtcgct 15720
gaagctaaaa cacaagcgaa acaaactatt gaccaattaa cacatttaaa tgctgatcaa 15780
attgcaactg ctaaacaaaa tattgatcaa gcgacgaaac ttcaaccaat cgctgaatta 15840
gtagatcaag caacgcaatt gaaccaatca atggatcaat tacaacaagc agttaatgaa 15900
catgctaacg ttgagcaaac tatagattac acacaagcag attcagataa gcaaaaggct 15960
tataaacaag cgattgctga tgctgaaaat gtattgaaac aaaatgcgaa taagcaacaa 16020
gtggatcaag cacttcaaaa tattttaaat gcaaaacaag cattaaatgg tgatgaacgt 16080
gtagcacttg ctaaaacaaa tggtaaacat gacatcgacc aattgaatgc attaaacaat 16140
gctcaacaag atggatttaa aggtcgcatc gatcaatcaa acgatttaaa tcaaatccaa 16200
caaattgtag atgaggctaa ggcacttaat cgtgcaatgg atcaattgtc acaagaaatc 16260
actggcaatg aaggacgcac gaaaggtagc acgaactatg tcaatgcaga tacacaagtc 16320
aaacaagtat atgatgaagc ggttgataaa gcgaaacaag cacttgataa atcgtctggg 16380
caaaacttaa ctgcagaaca agttatcaaa ttaaatgatg cagtcactgc agctaagaaa 16440
gcattaaatg gtgaagaaag acttaataat cgtaaagctg aagcattaca aagattggat 16500

caattaacac atctaaacaa tgctcaaaga caattagcaa tccaacaaat taataatgct 16560
gaaacgctaa ataaagcatc tcgagcaatt aatagagcaa ctaaattaga taatgcaatg 16620
ggtgcagtac aacaatatat tgacgaacag caccttggtg ttatcagcag cacaaattac 16680
atcaatgcag atgacaattt gaaagcaaat tatgataatg caattgcgaa tgcagcacat 16740
gagttagata aagtgcaagg taatgcaatt gcaaaagctg aagcagagca attgaaacaa 16800
aatattatcg atgctcaaaa tgcattaaat ggagaccaaa accttgcaaa tgccaaagat 16860
aaagcaaatg cgtttgttaa ttcgttaaat ggattaaatc aacagcaaca agatcttgca 16920
cataaagcaa ttaacaatgc cgatactgta tcagatgtaa cagatattgt taataatcaa 16980
attgacttaa atgatgcaat ggaaacattg aaacatttag ttgacaatga aattccaaat 17040
gcagagcaaa ctgtcaatta ccaaaacgct gacgataatg ctaaaacaaa cttcgatgat 17100
gccaaacgtc tagcaaatac attgctaaat agtgataaca caaatgtgaa tgatatcaat 17160
ggcgcaatcc aagcagtcaa tgatgcaatc cataatctta atggtgatca acgactacaa 17220
gatgctaaag acaaggcaat tcaatcaatt aatcaagctt tagctaataa gctaaaagaa 17280
atcgaagctt caaatgcgac ggatcaagac aagcttattg cgaaaaataa agcagaagaa 17340
ttggcaaaca gcatcatcaa caacattaat aaagcaacaa gtaatcaggc tgtatctcaa 17400
gttcaaacag caggcaacca cgcgattgaa caagtgcatg ctaatgaaat accaaaagca 17460
aaaattgatg ccaataaaga cgttgataag caagttcaag cattaattga cgaaattgat 17520
cgaaatccaa atctaacaga taaggaaaaa caagcactta aagatcgtat taatcaaata 17580
cttcaacaag gtcataacga cattaacaat gcgctgacta aagaagaaat tgaacaagct 17640
aaagcacaac ttgcgcaagc attacaagac atcaaagatt tagtgaaagc taaagaagat 17700
gcgaaacaag atgttgataa acaagttcaa gcattaattg acgaaatcga tcaaaatcca 17760
aatctaacag ataaggaaaa acaagcactt aaagatcgta ttaatcaaat acttcaacaa 17820
ggtcataacg gcattaacaa tgcgatgact aaagaagaaa ttgaacaagc caaagcacaa 17880
cttgcacaag cattaaaaga aattaaagat ttagtgaaag ctaaagaaaa tgcgaaacaa 17940
gatgttgata aacaagttca agcattaatt gacgaaatcg atcaaaatcc aaatctaaca 18000
gataaggaaa aacaagcgct taaagatcga atcaatcaaa tactgcaaca aggtcataac 18060
gacattaaca atgcgatgac taaagaagaa attgaacaag ccaaagcaca acttgcacaa 18120
gcattacaag acatcaaaga tttagtgaaa gctaaagaag atgcgaaaaa tgcaataaaa 18180
gccttagcta atgcgaagcg tgatcaaatc aattcaaatc cagatttaac acctgagcaa 18240
aaagcaaaag cgctcaaaga aattgacgaa gctgaaaaac gagcactaca aaacgttgag 18300
aatgctcaaa ctatagatca attaaatcga ggattaaact taggtttaga tgacattaga 18360
aatacacatg tatgggaggt tgatgaacaa cctgctgtaa atgaaatttt tgaagcaaca 18420
cctgagcaaa tcctagttaa tggtgaactc attgtacatc gtgatgacat cattacagaa 18480
caagatattc ttgcacacat aaacttaatt gatcagcttt cagcagaagt tattgataca 18540
ccatcaactg caacgatttc tgatagctta acagcaaaag ttgaagttac attgcttgat 18600
ggatcaaaag tgattgttaa tgttcctgta aaagttgtag aaaaagaatt gtcagtagtc 18660
aaacaacagg caattgaatc aatcgaaaat gcggcacaac aaaagattga tgaaatcaat 18720
aatagtgtga cattaacact ggaacaaaaa gaagctgcaa ttgcagaagt taataagctt 18780
aaacaacaag caattgatca tgttaacaat gcacctgatg ttcattcagt tgaagaaatt 18840
caacaacaag aacaagcgta tattgaacaa tttaatccag aacaatttac gattgaacaa 18900
gcaaaatcaa atgcaattaa atcgattgaa gatgcaattc aacatatgat tgatgaaatc 18960
aaagctcgta ctgatctaac agataaagag aagcaagaag ctattgctaa gttaaatcaa 19020
ttaaaagaac aagcaattca agcgattcaa cgtgcgcaaa gcatcagtga aataactgag 19080
caattggaac aatttaaagc tcaaatgaaa gcagctaatc caacagcaaa agaactagct 19140
aaacgcaagc aagaagctat tagtagaatt aaagactttt caaatgaaaa aataaatagt 19200
attcgaaata gtgaaattgg cacagctgat gaaaaacaag cagcaatgaa tcaaattaac 19260
gaaattgtgc ttgaaacaat tagagatatt aataatgcgc atacattaca gcaagttgag 19320
gctgcattga acaatggtat tgctcgaatt tcagcagtac aaattgtaat atctgatcgt 19380
gctaaacaat cgtcaagtac tggaaatgaa tctaatagcc atttaacaat tggttatgga 19440
actgcaaatc atccatttaa cagttcgact attggacata aaaagaaact tgatgaagat 19500
gatgacattg atccacttca tatgcgtcac tttagtaata atttcggtaa tgttattaaa 19560
aacgctattg gtgtggtggg tatctctggc ttactagcta gtttctggtt cttcattgcc 19620
aaacgtcgtc gtaaagaaga tgaagaggaa gaattagaaa taagagataa taataaagat 19680
tcaataaaag agactttaga cgatacaaaa catttaccac ttttatttgc gaaacgtcgc 19740
agaaaagaag atgaagaaga tgttactgtt gaagaaaaag attcgctaaa taatggcgag 19800
tcactcgata aagttaaaca tacgccgttc ttcttaccaa aacgtcgtcg taaagaagat 19860
gaagaagatg tggaagttac aaatgaaaac acagatgaaa aagtgttgaa agataacgaa 19920
cattcaccac tcttattcgc aaaacgacgc aaagataaag aggaagatgt tgaaacaaca 19980
actagtattg aatctaaaga tgaggacgtt cctttattat tggctaaaaa gaaaaatcaa 20040
aaagataacc aatccaaaga caaaaagtca gcatcaaaaa atacttctaa aaaggtagca 20100
gctaaaaaga agaaaaagaa atctaagaaa aataaaaaa 20139

<210> 47

<211> 2103
<212> DNA
<213> Staphylococcus aureus

<400> 47
ttgaataatc gtgataaatt acaaaaattt agtattcgaa aatacgcaat tggaacattt 60
tctactgtga ttgcaacact tgtgttcatg ggtatcaata caaaccatgc aagtgccgac 120
gagttgaatc aaaatcaaaa gttaattaaa caattaaatc aaacagatga tgatgattcg 180
aatacgcata gtcaagaaat cgaaaataac aaacaaaatt ctagtgggca gactgaatca 240
ttacgttcat caactagtca aaatcaagca aatgcacgac tgtcggatca attcaaagac 300
actaatgaaa catcgcaaca attacctaca aatgtttcgg atgatagtat caatcaatcg 360
catagtgaag caaatatgaa taacgaacca ttgaaagttg ataatagtac tatgcaagca 420
catagtaaaa tagtaagcga tagcgatggg aatgcttctg aaaataaaca tcataaacta 480
acagaaaatg tacttgcaga aagccgagca agtaaaaatg acaaagagaa agagaatcta 540
caagagaaag ataaatcgca gcaagtacat ccaccattag ataaaaatgc attacaagct 600
ttttttgacg catcatatca caattacaga atgattgata gagatcgtgc ggatgcaaca 660
gaatatcaaa aagtcaaatc tacttttgac tacgtcaatg acttactagg taataatcaa 720
aatattcctt cagaacagct tgtttcggca tatcaacaat tagagaaagc attagaactt 780
gcacgtacgt taccacaaca atctactaca gaaaaacgtg gtagaagaag tacgagaagt 840
gttgttgaga atcgttcatc aagaagcgat tacttagatg ctagaactga atattatgtt 900
tcaaaagacg atgatgattc tggtttccct cctggtactt tcttccatgc ttcaaataga 960
agatggcctt ataatttacc aagatctagg aacatcttac gtgcttctga tgtacaaggt 1020
aatgcttata tcactacaaa acgacttaaa gatggatatc aatgggatat tttatttaat 1080
agtaatcata aagggcatga atatatgtac tattggtttg gacttccaag tgatcaaaca 1140
ccaactggtc cagtaacttt cactattatc aaccgtgatg gttcaagtac atctactggt 1200
ggcgttggat ttggatcagg tgcaccacta cctcaatttt ggagatcagc aggtgctatt 1260
aattctagcg tagcgaatga ttttaaacat ggctccgcta caaattatgc attttatgat 1320
ggtgttaata atttttctga ctttgctaga gggggagaat tatacttcga cagagaaggc 1380
gctacacaaa ctaataaata ttatggcgat gaaaacttcg cattgctaaa tagtgagaaa 1440
ccagatcaaa taagaggatt agatacaata tatagtttta aaggtagtgg tgatgtaagt 1500
tatcgtattt catttaaaac tcaaggagct ccaactgcaa gattgtatta tgctgctggc 1560
gcgcgttctg gtgaatataa acaagcaacg aactataacc aactctatgt cgaaccttat 1620
aagaattatc gaaatcgagt acagtcaaat gtccaagtta aaaatcgtac acttcattta 1680
aaaagaacaa tcagacaatt cgatcctaca ttacagagaa ctactgatgt tcctattttg 1740
gatagtgacg gttccggaag tattgattcg gtatacgacc cattaagtta tgtaaagaat 1800
gtgactggta cagtcctagg tatttatcca tcttatcttc cttataatca ggaaagatgg 1860
cagggagcta atgcaatgaa tgcctatcaa attgaagaac ttttttcaca agaaaatctt 1920
caaaatgcag cacgttcagg ccgtccaatt caatttcttg taggttttga tgttgaagat 1980
agccatcata accctgaaac tcttttacca gtaaatttat atgtaaaacc tgagttaaaa 2040
catacaattg agttatatca cgataatgaa aaacaagata gaaaggaatt ttcagtatcg 2100
aaa 2103

<210> 48
<211> 28317
<212> DNA
<213> Staphylococcus aureus

<400> 48
atgagtggaa cgcttcataa cactgtagga tcaggaatat taccttatca acaagagata 60
cgtatcaaac ttactagtaa tgaaccaatt aaagatagtg aatggtctat tacaggatat 120
cctaacacgc ttacattaca aaacgctgtg ggtagaacaa ataatgctac tgaaaaaaac 180
ttagctcttg ttggtcatat tgatccagga aattatttca tcactgttaa gtttggtgat 240
aaagtagaac aatttgaaat tagatcaaaa ccaactccac caagaatcat tacaactgct 300
aatgaattac gtggaaatcc taaccataag cctgaaataa gagtaacaga tataccaaat 360
gatactactg ctaaaatcaa acttgtgatg ggcggaaccg atggcgatca tgatccagaa 420
ataaatccat atactgtccc tgaaaactac acagtagttg cagaagcata ccatgataat 480
gatccaagta aaaatggggt cttaacattc cgttcatcag actaccttaa agatctacca 540
ttaagcggtg aattaaaggc aattgtttat tacaatcaat atgtacaatc aaactttagt 600
aaaagcgttc cgtttagtag cgatacaaca ccacctacaa ttaatgaacc ggcaggacta 660
gttcataagt attacagggg agatcatgta gaaattactc ttccagtcac tgataatact 720
ggcggttcag gtttaagaga tgtaaacgtc aatttacctc aaggttggac aaaaaccttt 780
acaatcaatc ctaataataa tactgagggt acgcttaagt taattggtaa tatacctagt 840
aatgaagcat ataatacgac atatcatttc aatattactg caaccgataa ttctggaaat 900
acaacaaatc cagctaaaac ctttatttta aatgttggta agttggctga tgatttaaat 960
ccagtcggat tatctagaga tcaactacaa ttagtgacag acccttcttc attatctaat 1020

tccgaacgag aagaggtaaa aagaaaaata agtgaagcaa atgctaatat aagatcatat 1080
ttattacaaa ataacccaat actcgctgga gtaaacggcg atgttacatt ttattataga 1140
gatggttctg tagatgttat tgatgctgaa aatgtaatca catatgagcc cgaaagaaaa 1200
tccattttca gtgaaaatgg taatacaaat aaaaaagaag cagtaatcac tattgctaga 1260
ggacaaaact ataccattgg tccaaactta agaaaatatt tctcattaag taatggttcg 1320
gatttaccta atagagattt cacctctata tcagctattg gatctttacc ttcatcgagt 1380
gaaattagtc gactcaatgt tggaaattat aactatagag ttaatgctaa aaatgcttat 1440
cataagactc aacaagaact taatttaaaa cttaaaatag tagaggttaa tgcacctact 1500
ggtaataatc gtgtatatag agttagtact tataatttaa ctaatgatga aatcaataaa 1560
atcaaacaag catttaaagc agctaattct ggacttaatt taaacgataa cgatatcact 1620
gtttcgaata actttgacca tagaaatgtt agtagtgtga cagtaactat acgtaagggc 1680
gatttgataa aagagttttc atcaaatctc aataatatga atttcttacg ttgggttaat 1740
ataagggatg attataccat ttcgtggact tctagtaaga ttcaaggtag aaatacagat 1800
ggtggattag aatggtcacc agatcataaa tcacttattt ataaatatga tgcaacatta 1860
ggtagacaaa taaatactaa tgacgtgtta actttacttc aagcaacagc taaaaactca 1920
aatttacgtt caaatatcaa tagtaatgaa aaacagttag cagaacgagg gtctaatggg 1980
tattctaaat ctataattag agatgatggc gagaaatctt atttacttaa ctcaaatcct 2040
attcaagtat tagacttagt agaaccagat aatggttacg gtggacgtca agtcagtcat 2100
tctaacgtta tatataatga aaaaaattct tctatcgtaa atggtcaagt tccagaagct 2160
aatggggcat ccgcttttaa tattgataaa gttgttaaag ctaatgcggc aaataatggt 2220
attatgggtg ttatctataa ggcacaatta tacttagcac catacagtcc aaaaggttac 2280
attgaaaaat taggccaaaa tttaagcaat accaataacg tgattaatgt ttattttgtg 2340
ccttctgata aagtaaatcc tagtataact gtaggtaatt acgaccatca tacggtatat 2400
tctggtgaaa catttaaaaa tactatcaat gtaaatgata attatggatt aaatacagta 2460
gcttctacaa gtgatagtgc aattactatg accagaaaca acaacgagtt agtaggtcag 2520
gctcctaatg ttactaatag cataaataaa attgtaaaag ttaaagccac agataaaagt 2580
ggaaatgaaa gtattgtttc tttcacagta aatataaaac cattaaacga gaaatataga 2640
ataacaactt catcaagtaa tcaaacacca gtgagaatta gtaatattca aaacaatgct 2700
aacctttcaa ttgaagatca aaatagagta aaatcttcac tcagcatgac taaaatttta 2760
ggtacaagaa attatgtcaa tgagtcaaat aatgacgttc gtagtcaagt tgtaagtaaa 2820
gtaaatagaa gtgggaacaa tgctacagtt aatgttacaa ctacattttc tgatggtaca 2880
actaatacaa taaccgttcc agttaaacat gtgttattag aagttgtacc tactactaga 2940
acaacagtaa gaggacaaca atttccaacc ggcaaaggaa cttccccaaa tgatttcttt 3000
agtttaagaa cgggaggtcc agttgatgcg agaatagttt gggttaataa tcagggaccc 3060
gatataaata gtaatcaaat tggtagagat ttaacattac acgctgaaat attctttgat 3120
ggtgaaacaa caccaattag aaaagatact acttacaaac ttagtcaatc tattccaaag 3180
caaatatatg aaacaactat caatggtcga tttaattcat caggtgatgc atatccagga 3240
aattttgttc aagcagtaaa tcaatattgg ccagaacata tggacttcag atgggcccaa 3300
ggatcaggca caccaagttc tcgtaatgca ggttcattta ctaaaacagt tacggtagtt 3360
tatcaaaacg gccaaactga aaacgttaat gtactattca aagtcaaacc aaataaacct 3420
gttattgata gtaatagtgt gatttcaaaa ggacaattaa atggtcaaca aattttagtt 3480
cgaaatgttc cacaaaatgc acaagtcact ctatatcaat caaatggaac tgttattcct 3540
aatacaaata caactataga ttctaatggt atagctactg taacaattca aggcactcta 3600
ccaaccggaa atattactgc taaaacctca atgacaaata atgtaacgta cactaaacaa 3660
aatagtagtg gaattgcttc aaatacaact gaagatataa gtgttttttc agaaaacagt 3720
gatcaagtaa atgttaccgc tggcatgcaa gctaaaaatg atggtattaa aataattaaa 3780
ggtacaaact ataattttaa tgacttcaat agtttcataa gtaatatacc agcccattct 3840
actcttacat ggaacgagga gcctaatagt tggaaaaaca acatcggtac tacaacaaaa 3900
actgttacag ttactctacc taatcatcaa ggtacgagaa ctgtagatat tccaataaca 3960
atctatccaa cagttacagc taagaatcca gtaagagatc aaaaaggacg aaacttaacc 4020
aatggtactg acgtttataa ttatattatt tttgaaaata ataaccgtct tggaggaaca 4080
gcttcttgga aagacaatcg tcaacctgat aaaaacatag ccggtgtaca aaatttaatt 4140
gcacttgtta attatcctgg catatctaca ccattagaag ttcctgttaa agtgtgggta 4200
tataattttg atttcactca acctatctac aaaattcaag taggagatac attccctaaa 4260
ggaacatggg caggctatta caaacatctt gaaaatggag agggattacc aatagatggt 4320
tggaaatttt attggaacca gcaaagtaca ggaactacta gtgatcaatg gcaatcatta 4380
gcatatacta gaactccttt tgttaaaact ggtacttatg atgtcgttaa tcctagcaac 4440
tggggtgttt ggcaaacatc acaatcagct aaatttatag ttacaaatgc taaacctaat 4500
caaccaacca taactcagtc taaaactggt gatgtaacag taacacctgg tgctgtgcgt 4560
aatatactaa taagtgggac aaatgattat atccaagcat ctgcagataa gattgttatt 4620
aataaaaatg gaaataaatt aactacattt gttaaaaata atgatggtcg ttggactgtt 4680
gaaactgggt cacctgacat aaatggtatc ggaccaacaa ataacggaac tgctatatct 4740
ttaagtcgat tagcagttag acctggggat tcaatagaag caatagcgac tgaaggttcc 4800
ggagaaacta taagtacttc agcaactagt gaaatttata ttgtcaaagc tccacaacct 4860
gaacaagtag caactcatac ttatgataat ggaacattcg atatattacc tgacaattca 4920

cgtaattctt taaatccaac tgaacgtgtc gaaattaatt acactgaaaa attaaatggc 4980
aatgaaacac aaaaatcatt cactattact aaaaataaca acggcaaatg gacgataaat 5040
aataaaccaa attatgtcga gttcaatcag gataatggta aagttgtatt ttcggccaat 5100
acaattaaac ctaattctca aattacaata actcctaaag caggtcaggg taacactgaa 5160
aacacaaatc ctactgtaat tcaagcacct gcgcaacata ctttaacaat caatgaaatt 5220
gttaaagaac agggtcaaaa tgtgactaat gatgatatta ataatgcggt tcaagtgcca 5280
aataaaaata gagttgcgat taaacaagga aacgctcttc caacaaattt agctggtggt 5340
agtacatcac atattccagt agttatttat tacagtgatg gaagttctga agaagctact 5400
gagactgtta gaactaaagt taataaaacc gaattaatca atgctcgtcg tcgactagat 5460
gaagaaatta gtaaagagaa caaaacacca tcaagtatca gaaactttga tcaagctatg 5520
aatcgtgctc aatcacaaat taatacagct aaaagtgatg ctgaccaagt tataggcaca 5580
gaatttgcaa cacctcaaca agtaaattca gctttatcta aagttcaagc ggcacaaaat 5640
aaaataaatg aagctaaagc attattacaa aacaaggctg ataatagtca acttgtgaga 5700
gcaaaagaac aattacaaca atcgattcaa ccagccgctt caactgatgg tatgactcaa 5760
gatagcacaa ggaactacaa caataaacgc caagcagctg aacaagcaat acaacatgca 5820
aatagcgtta taaataatgg agatgcaaca tcccaacaaa ttaatgatgc taaaaacaca 5880
gttgaacagg cacagagaga ttatgttgaa gctaaaagca acttacgtgc tgataagtca 5940
cagttacaaa gcgcttatga tacgttaaat agagatgttt taacaaatga taaaaagcca 6000
gcatctgtaa gacgctataa tgaagccatt tcaaatatta gaaaagaatt agatacagct 6060
aaagcggatg caagtagtac tttgcgaaac accaatcctt ccgttgaaca agttagagac 6120
gctttaaata aaataaatac tgttcaacct aaagtgaatc aagcaattgc tttacttcaa 6180
ccaaaagaaa ataattcaga acttgtacaa gctaaaaaac gtttacaaga cgctgtaaat 6240
gacatacctc aaacacaagg tatgacacaa caaacaatta ataattataa tgacaaacaa 6300
cgtgaagctg aaagagcact tacatctgca caaagagtga ttgataatgg ggatgctaca 6360
actcaagaaa ttacttctga aaaatctaaa gtagagcaag caatgcaagc tttaactaat 6420
gctaaaagta atctgagagc tgataagaat gagttacaga ctgcatataa caaattaatt 6480
gagaacgtat ctaccaatgg taaaaaaccg gcgagtatac gtcaatacga aacagccaaa 6540
gccagaatac aaaatcaaat taatgatgct aaaaatgaag cggagcgaat tttaggtaat 6600
gataatccac aagtatcaca agtaactcaa gcattgaaca aaatcaaagc tattcaacca 6660
aaattaacag aagctatcaa catgcttcaa aacaaagaaa ataatacaga attagtcaat 6720
gctaaaaaca gacttgaaaa tgcagtaaat gatacagatc caacacacgg tatgactcaa 6780
gaaacaatta ataattacaa cgctaaaaag cgagaagctc aaaatgaaat acaaaaagcg 6840
aacatgatta ttaataatgg agatgctact gctcaagata tttcttctga aaaatctaaa 6900
gtagagcaag tattacaagc attacaaaat gctaagaatg acttaagagc tgataaaaga 6960
gaattacaga ctgcatacaa taaacttata caaaatgtta ataccaatgg taaaaaacca 7020
tctagtattc aaaactataa gtctgcaaga cgaaatatcg aaaaccaata taataccgct 7080
aaaaatgaag cacataatgt tcttgaaaat acaaacccta ctgtaaatgc agtagaagat 7140
gctttacgta agataaatgc aattcaacca gaggttacaa aagctattaa tatacttcaa 7200
gataaagaag ataatagcga acttgttaga gcaaaagaaa aattagatca agcgattaat 7260
agtcaaccat cactaaatgg tatgactcaa gaatctatta ataattacac aacaaaacgt 7320
agagaagcac aaaatatagc aagttctgct gacactatta ttaataatgg ggatgcatct 7380
attgaacaaa taacagaaaa taaaattcga gttgaagagg caactaatgc acttaacgaa 7440
gcaaaacaac atttaacggc agatacaact tctttaaaaa ctgaagtacg gaaattaagt 7500
aggagaggcg acacaaacaa caaaaagcct agcagtgtta gtgcttataa caatactatt 7560
cattcgctac aatctgaaat tacacagact gaaaatagag caaatactat catcaataag 7620
cctattcgtt ctgttgaaga agtaaataat gcattgcatg aagtaaacca attgaaccaa 7680
cgcttaacag atacaattaa cttattacaa cctttagcga ataaagaaag cttaaaagaa 7740
gctcgtaatc gacttgaaag taaaattaat gaaaccgttc aaacagacgg tatgactcaa 7800
caatctgttg agaattataa gcaagctaaa ataaaagctc aaaatgaatc tagtattgca 7860
caaactctta ttaataatgg tgatgcatct gatcaagaag tttctacaga aatagaaaaa 7920
ttaaatcaaa agctgtctga attaacaaat tcaatcaatc acttaacagt taataaagaa 7980
cctttagaaa ctgccaaaaa tcagttacaa gcaaatattg accaaaaacc tagcactgat 8040
ggtatgacgc aacaatctgt acaaagctat gaacgtaaac tacaagaagc caaagataaa 8100
ataaactcaa ttaataatgt cttagctaac aatccagatg ttaatgctat cagaacaaac 8160
aaagttgaga cggaacaaat caataatgaa ttaacacagg cgaaacaagg tcttactgtt 8220
gataaacaac cattgattaa tgcaaaaact gctttgcaac aaagtctaga taatcaacca 8280
agtactactg gtatgactga agcaacaatt caaaattata acgctaaacg tcaaaaagca 8340
gagcaagtta tacaaaatgc aaataaaatt attgaaaacg ctcaacctag tgtacaacaa 8400
gtgtctgatg agaaatctaa ggtagagcaa gcactcagtg aattgaacaa cgccaaatca 8460
gcgcttagag ctgataaaca agaattacag caagcatata atcagttgat tcaaccaacg 8520
gatttaaata ataagaaacc agcttctatc actgcgtaca atcaaagata tcaacaattt 8580
agtaacgaat tgaacagcac taaaacaaat acagatcgca ttttaaaaga gcaaaatcca 8640
agtgtagctg atgtcaacaa tgcactaaat aaagtaagag aagtacaaca aaaattaaac 8700
gaagccagag cacttttaca aaataaagaa gataatagtg cactagttcg agccaaagaa 8760
caacttcaac aggcagttga ccaagtccct tcaacagaag gtatgacgca acaaactaaa 8820

gatgattaca attcaaaaca acaagctgct caacaagaaa tatcaaaagc acaacaagtt 8880
atcgataatg gcgatgcgac tacacaacaa atttctaacg ccaaaacaaa tgttgaacgc 8940
gctttagaag cattaaataa tgcaaaaact ggtttaagag cagataaaga ggaacttcaa 9000
aatgcatata atcaattaac tcaaaatatt gatacgagcg gtaaaacgcc tgcaagtatc 9060
aggaaataca atgaagctaa gtcacgtatt caaactcaaa ttgattcagc taaaaatgaa 9120
gcaaacagta ttttaacaaa tgacaatcct caagtatcac aagtgactgc tgcgttaaac 9180
aaaataaaag ctgttcaacc tgaattagat aaagcgatag caatgcttaa aaataaagag 9240
aataataatg cattggttca agcgaaacaa caacttcaac aaattgttaa tgaagtagat 9300
ccaacacaag gcatgacaac agatactgct aataactata aatcaaaaaa acgtgaagct 9360
gaagatgaaa tacaaaaagc tcaacaaatc attaacaatg gcgatgccac tgagcaacaa 9420
attactaacg aaacaaatag agtaaatcaa gcgattaatg caataaacaa agccaaaaac 9480
gatttacgtg ctgataagtc tcaattggaa aatgcttata accaattaat acaaaatgtt 9540
gatacaaatg gtaaaaaacc tgctagtatt caacaatacc aagctgctcg acaagctatt 9600
gagacgcaat acaataacgc taaatcagaa gcacatcaaa ttcttgaaaa tagtaaccct 9660
tcagttaatg aagtagcaca agcattacaa aaagttgaag ctgtacaact taaagttaat 9720
gacgcgattc atatacttca aaataaagag aataatagtg cacttgtcac agctaaaaat 9780
caacttcagc aatcagttaa tgatcaacca ttaacaacag gtatgactca agattctatt 9840
aataactatg aagctaagag aaatgaggct caaagtgcta tcagaaatgc agaagctgtc 9900
atcaacaatg gcgatgcaac tgcaaaacaa atttcagacg agaaatctaa agttgaacaa 9960
gcactagcac atttgaatga tgctaaacag caattaactg cagatactac tgaattacaa 10020
acagcagttc aacaattaaa cagaagaggc gatacaaata ataaaaagcc aagaagtatc 10080
aatgcatata ataaagcaat tcaatcatta gaaacacaaa ttacttctgc taaagataat 10140
gccaacgctg tgatacaaaa acctatacgt actgttcaag aggtaaataa tgcattacaa 10200
caagtaaatc agttgaatca acaattaact gaagcaatta atcaacttca accgctatca 10260
aataatgatg cattaaaagc tgcaagatta aatttagaaa ataaaattaa tcaaactgta 10320
caaactgatg gtatgacaca acaatctata gaggcttatc aaaacgctaa acgcgtagcc 10380
caaaatgaat ctaacactgc tttagcatta attaataacg gcgatgccga tgaacaacaa 10440
attacaactg aaacagaccg agtcaatcag caaactacaa acttaactca agcaattaac 10500
gggttaacag ttaataaaga accattagaa accgctaaaa cagcgttaca aaataacatc 10560
gaccaggtac ctagtacaga tggtatgact cagcaatctg ttgcaaatta taatcaaaaa 10620
ctacaaatag ctaaaaacga aattaacaca attaataacg ttttagcgaa caatccagat 10680
gttaatgcaa tcaaaacgaa taaagcagaa gcggaacgaa tcagtaacga tttaacacaa 10740
gctaagaata acttacaagt tgatactcaa cctttagaaa aaataaaaag acaacttcaa 10800
gatgaaattg atcaaggtac taacacagat ggaatgactc aagattcagt ggataattac 10860
aatgatagct taagtgcagc aattatagaa aaaggcaaag taaataaatt acttaaacgt 10920
aatccgacag tagaacaagt taaagagagc gttgctaatg cacaacaagt catacaagat 10980
ttacaaaatg ctcgaacttc acttgttcca gacaaaactc aacttcaaga agctaaaaat 11040
agattagaaa acagtattaa ccaacaaaca gatactgacg gcatgactca agattcgctt 11100
aacaattata atgataaatt agcaaaagct agacaaaacc ttgaaaaaat atctaaagtt 11160
ttaggtggtc aacctactgt agctgaaatt agacaaaata cagatgaagc aaatgcacat 11220
aaacaagcat tagacactgc acgttctcaa cttacattaa atagagagcc atatatcaat 11280
catattaata atgaaagtca tttaaataac gcgcaaaaag ataattttaa agctcaagtt 11340

aactcagcac ctaatcataa tactttagaa acgattaaaa ataaggctga tactttaaat 11400
caatctatga cagcattaag tgaaagtatt gcagattacg aaaatcaaaa acaacaagaa 11460
aattatttag atgcatctaa caataaacgt caagactatg acaatgcagt caatgcggct 11520
aaaggtattt taaaccaaac tcaaagtccg acaatgagtg ctgatgtgat tgatcaaaaa 11580
gctgaagatg ttaaacgtac gaaaactgcg ttagatggaa atcaaagatt agaagttgct 11640
aaacaacaag cacttaatca tttaaatacc ttaaatgatt taaacgatgc tcagcgacaa 11700
actttaactg atactataaa tcactctcca aacatcaatt cagtgaatca agctaaagaa 11760
aaagctaata ctgttaacac agcaatgact caactgaaac aaactattgc taactatgac 11820
gatgaattgc atgacggcaa ttacattaat gcagataaag acaaaaaaga tgcttataat 11880
aacgctgtta acaatgctaa acaactgatt aatcaatctg atgctaatca agcacaactt 11940
gatccagctg aaattaataa agttacacaa agagtcaata cgactaaaaa tgatctaaat 12000
ggtaatgaca aattggctga agctaaaaga gatgctaata caaccattga tggtttaact 12060
tatctaaatg aagctcaacg taacaaagct aaagaaaatg taggcaaagc ttctacaaaa 12120
acaaatatta cgagtcagtt acaagattac aatcaattga atattgctat gcaagcatta 12180
cgtaacagtg tgaacgacgt taacaatgtt aaagcaaata gcaattatat aaatgaagat 12240
aatggtccaa aagaagctta caatcaagcc gttactcatg ctcaaacatt gataaatgca 12300
caatctaacc ctgaaatgag ccgtgacgta gtaaatcaaa aaacacaagc agtaaatact 12360
gcccatcaga atttacatgg acaacaaaag ttagaacaag cacaaagtag tgctaataca 12420
gaaatcggta acttaccaaa cttaactaat actcaaaaag ctaaagaaaa ggaactggta 12480
aatagtaaac aaactcgtac ggaagtacaa gaacaactta accaagctaa gtcactagat 12540
agttctatgg gcacgttaaa atcattagtt gctaaacaac ctacagtaca aaaaacaagt 12600
gtttatatta acgaagatca acctgagcaa tctgcctaca atgattccat tacaatggga 12660

caaactataa ttaataaaac agctgatcca gtacttgata aaactttagt tgataacgca 12720
atcagtaaca tttcaactaa agagaatgca ctgcatggtg aacaaaaatt aacaactgct 12780
aaaacggaag caattaatgc acttaataca ttagctgatt taaacacacc tcagaaagag 12840
gctattaaaa cagctattaa cactgctcat acaagaactg atgtaactgc agagcaaagt 12900
aaggctaatc aaataaatag tgcaatgcac acgttgagac aaaacatttc tgacaacgaa 12960
tcagtaacaa acgaaagtaa ttatattaac gctgaacccg aaaaacaaca tgcctttact 13020
gaggctctaa ataatgctaa agaaatagtt aatgaacaac aagccactct tgatgccaat 13080
tcaattaacc aaaaagcaca agcgattctt actactaaaa atgctttaga tggtgaagaa 13140
caattacgtc gtgctaaaga aaatgccgat caagaaatca atacgttaaa tcaattgact 13200
gatgcgcaaa gaaatagtga aaaaggttta gtcaacagtt ctcaaactag aacagaagtt 13260
gcttctcaat tagcaaaagc taaagaacta aataaggtga tggaacaact gaatcacctt 13320
atcaatggta aaaaccaaat gataaatagc agtaaattta tcaatgaaga tgcgaaccaa 13380
caacaagcat attcaaatgc gattgcaagt gcagaagcgc ttaaaaacaa atcacaaaac 13440
cctgaattag ataaagtaac aattgaacaa gcaattaata atattaattc tgcaattaac 13500
aatctaaacg gtgaagctaa actgactaaa gctaaagaag atgctgttgc ttcaataaac 13560
aacctaagcg gattaacaaa cgagcaaaaa acaaaagaaa atcaagccgt taatggcgct 13620
caaactagag accaagttgc taataaatta cgtgatgctg aagcattaga tcaatcaatg 13680
caaacattac gtgacttagt taacaatcaa aatgcaatac attcaacaag taattatttt 13740
aacgaggatt caactcaaaa gaatacttat gataatgcaa ttgataatgg ctcgacatat 13800
ataactggtc aacacaatcc agaattaaat aaatctacta ttgatcaaac gattagccga 13860
attaacacag ctaaaaatga tttacatggt gtagaaaagt tacaaagaga taagggaact 13920
gctaatcaag aaattggaca attaggttat ttaaatgacc ctcaaaaatc tggtgaggaa 13980
tccttagtca acggttcaaa tacacgttct gaagtagaag agcatcttaa tgaagctaaa 14040
tcattaaata atgcaatgaa acaattaaga gataaagtag ctgaaaagac taatgtcaaa 14100
caaagtagcg attacattaa tgattcaact gaacatcaac gtgggtatga tcaagcactt 14160
caagaagcag aaaatattat taatgaaatc ggtaatccaa cattaaataa atcggaaatt 14220
gaacaaaagt tacaacaatt gactgacgct caaaatgcgt tacaaggttc acatctatta 14280
gaagaagcta aaaataatgc gattactgga atcaataaac ttacagcatt aaatgatgca 14340
caacgtcaaa aagcaattga aaatgttcaa gcacagcaga caatcccagc agttaatcaa 14400
caattaactt tggatagaga aataaatact gcaatgcaag ctttacgaga taaagtaggc 14460
caacaaaata acgttcacca acaaagtaat tatttcaatg aagatgaaca accaaaacat 14520
aactatgata attctgtaca agccggtcaa actattattg ataaacttca agatccaatc 14580
atgaacaaaa atgaaattga gcaggctatt aatcaaatca atacgactca aacagcgtta 14640
agtggagaaa ataaattaca cactgaccaa gaaagcacaa atagacaaat agaaggttta 14700
tctagtttga acacagctca aatcaacgcc gaaaaagatt tagtcaatca agctaaaaca 14760
agaacagatg ttgctcaaaa gttagctgca gctaaagaaa taaattctgc tatgagtaat 14820
ttaagagatg gcattcaaaa taaagaggac atcaaacgta gcagtgcata tatcaacgca 14880
gatccgacta aagttacagc ttacgatcaa gcactacaga acgcagaaaa tatcatcaat 14940
gccacaccaa acgtagagct taataaagct acaattgaac aagcgctatc acgcgttcaa 15000
caagcacaac aagatcttga tggtgttcaa caattagcta atgctaaaca acaagctaca 15060
caaactgtca atgggttaaa tagcttaaat gacggtcaaa agcgtgaatt aaatctatta 15120
attaattcag ctaatacccg tacaaaagta caagaagaat taaacaaagc aactgaattg 15180
aaccatgcga tggaagcttt aagaaacagt gttcaaaacg ttgatcaagt aaaacaaagt 15240
agcaattatg tcaatgaaga tcaacctgaa cagcacaatt atgataatgc tgtcaatgaa 15300
gctcaagcta caatcaacaa caatgctcaa cctgttctag acaaattagc tatagaacgt 15360
ttaactcaaa ctgttaacac tacaaaagat gcattacatg gtgctcaaaa actgacacaa 15420
gaccaacaag ctgctgaaac tggaatacgt ggtttaacga gtctcaatga acctcagaaa 15480
aatgctgaag tagctaaagt aactgcagca acaacacgtg atgaagtgag aaatattcgt 15540
caagaagcaa caacattaga tactgcaatg cttggtttac gtaaaagcat taaagataaa 15600
aacgatacta aaaatagtag taaatatatt aatgaggatc atgaccaaca acaagcttat 15660
gacaatgctg taaataatgc tcaacaagtt atcgatgaaa ctcaagcaac gttaagctca 15720
gatacaatca atcaattggc aaatgccgta actcaagcta aatctaatct tcatggagat 15780
actaaactac aacacgataa agatagtgct aaacaaacga ttgctcaatt acagaatttg 15840
aattcagctc aaaaacatat ggaagattct ttaattgata atgaatctac acgtacgcaa 15900
gtccaacacg atttaacaga agctcaagct ttagatggtt taatgggtgc cttaaaagaa 15960
agtattaaag attatactaa tattgtttca aacggtaatt acatcaatgc ggaaccatct 16020
aagaaacaag catatgatgc agctgtacaa aatgctcaaa atataataaa tggaacgaat 16080
caaccaacaa ttaataaagg taatgtcact acagcaacac aaaccgtgaa aaatactaaa 16140
gatgccttag acggtgatca tagattagag gaagctaaaa ataatgccaa tcaaacaatc 16200
agaaatctat ctaatttgaa caatgcccaa aaagatgcag agaaaaatct agttaatagc 16260
gcatcaacat tagaacaagt tcaacaaaac ttacaaaccg ctcaacaatt agataatgct 16320
atgggtgagt tacgacaaag tattgctaaa aaagatcaag tgaaagcaga tagtaaatat 16380
ctaaatgaag atcctcaaat taagcaaaac tatgatgatg cagttcaacg tgttgaaact 16440
attattaacg aaactcaaaa ccctgaatta cttaaagcaa acattgacca agcaactcaa 16500
tccgttcaaa atgcagaaca agctttacat ggtgctgaaa aattaaatca agacaaacaa 16560

acgtcttcga cagaactaga tggattaaca gatttaacag atgcacaacg tgaaaaactc 16620
agagaacaaa ttaacacttc taatagtaga gatgatatta agcaaaaaat tgagcaagca 16680
aaagcactaa atgacgcaat gaaaaaactt aaagaacaag ttgcgcaaaa agatggtgtt 16740
catgctaaca gtgattatac aaatgaagat tctgcacaaa aagatgcgta taataatgca 16800
cttaaacaag cggaagacat tattaataac agctcaaatc ctaacttaaa tgcacaagac 16860
attactaatg ctttaaataa tattaaacaa gcacaagata accttcatgg agctcaaaaa 16920
ttacagcaag acaaaaatac aactaatcaa gccattggta acttaaatca tcttaatcaa 16980
cctcaaaaag atgcgcttat acaagctatt aatggagcta catctaggga ccaagttgca 17040
gaaaaactta aagaggccga agcgcttgat gaagctatga aacaacttga agatcaagtg 17100
aatcaagatg atcaaatttc aaatagcagc ccattcataa atgaagactc agacaaacaa 17160
aaaacttata atgataaaat ccaagctgca aaagaaataa ttaatcaaac atctaatcca 17220
accttagata aacaaaaaat tgctgataca cttcaaaata ttaaagatgc agtgaataat 17280
ttacatggtg atcaaaaatt agctcaatct aaacaagatg ctaataatca attaaatcat 17340
ttagatgact taaccgaaga acaaaaaaac cattttaaac cgttaattaa taatgctgat 17400
actcgagatg aggtaaataa acaactagag attgctaaac aattaaatgg tgatatgagt 17460
acacttcata aagtcataaa tgataaagat caaattcaac atttaagcaa ttacattaat 17520
gctgataatg ataaaaaaca aaattatgat aatgctatta aagaagctga ggatttaatt 17580
cataatcatc cagatacatt agatcataaa gcattacaag atttattaaa caagatagac 17640
caagcgcata acgaattaaa tggagaatcc agatttaaac aggctttaga caatgcttta 17700
aacgacatag atagcttaaa cagtctcaat gttccacaac gccaaactgt taaggataac 17760
atcaaccatg tgacaactct agaaagttta gctcaagaat tgcagaaagc aaaagagctt 17820
aatgatgcta tgaaagcaat gagagatagc attatgaatc aagagcaaat tcgtaaaaat 17880
agcaattata ctaatgaaga cttagctcaa caaaatgcct ataatcatgc agtagataaa 17940
ataaataaca ttattggtga agacaatgcg acgatggatc ctcaaataat caaacaagca 18000
actcaagata taaatacagc tataaatgga ttaaatggag atcaaaaact tcaagatgca 18060
aagacagatg ctaaacaaca aattactaac tttactggtt taactgaacc acaaaaacaa 18120
gcattggaaa acatcattaa ccaacaaaca agcagagcaa atgttgctaa acagttaagt 18180
catgctaaat tcttaaatgg aaaaatggaa gaattaaaag ttgcagtagc caaagcgtca 18240
ttagtaagac aaaatagtaa ctatattaat gaagatgtct ctgaaaaaga agcatatgaa 18300
caagctatcg caaaaggtca ggaaataatt aattcagaaa ataatccaac aataagtagt 18360
actgatatca atcgtaccat tcaagaaatt aatgatgctg aacaaaatct tcatggtgat 18420
aataaattaa gacaagcaca ggaaattgca aagaatgaaa tacaaaatct agacggatta 18480
aattcagctc aaataacaaa attaatccaa gatataggca gaacaacaac taaacctgca 18540
gtaactcaga aactagaaga agcaaaagca ataaaccaag ctatgcaaca acttaaacaa 18600
agtatagccg ataaggatgc tactctaaat tctagtaact atctcaatga agattctgag 18660
aaaaagttag cgtacgataa tgctgtaagc caagctgaac aactcataaa tcaacttaac 18720
gacccaacta tggatataag taatattcaa gctattactc aaaaggtcat tcaagcaaaa 18780
gattcattgc acggtgcgaa taaacttgca caaaatcaag cagattcaaa tttaataata 18840
aatcaatcaa caaatttaaa tgataaacaa aagcaagcat taaatgactt aattaatcat 18900
gctcaaacta aacagcaagt ggcagaaata attgcacaag ctaataagtt aaataacgaa 18960
atgggcacac taaaaacact cgtagaagaa cagtcaaacg ttcatcaaca aagtaaatat 19020
attaatgaag atccgcaagt tcaaaatatt tataatgact ccattcaaaa aggtcgagaa 19080
atattaaacg gcactacaga tgatgtttta aacaacaata aaatagcaga tgccattcaa 19140
aacattcatt taactaaaaa cgatttacat ggtgatcaaa aattacaaaa agcacaacaa 19200
gatgcaacca atgaattaaa ctatttaaca aatctaaaca attctcaaag acaaagcgag 19260
catgatgaga ttaactctgc tccttcaaga actgaagttt ctaatgattt aaatcatgct 19320
aaagcactta atgaagctat gcgtcaactt gagaatgaag ttgctcttga aaacagtgtt 19380
aaaaaattaa gcgactttat caatgaagat gaagcggcac aaaatgaata tagtaatgca 19440
cttcaaaaag ctaaagacat tatcaacggc gttccaagta gcactttaga taaagctaca 19500
attgaagatg ctttattaga attgcaaaat gctagagaaa gtttacatgg tgagcaaaaa 19560
cttcaagagg ctaaaaatca agctgttgct gaaattgata atttacaagc attaaatcct 19620
ggacaggttc ttgctgaaaa aacattagtt aaccaagcat caaccaaacc agaagttcaa 19680
gaagccttac aaaaagcaaa agaacttaat gaagctatga aagcactgaa aactgaaata 19740
aataaaaaag aacaaatcaa ggctgatagt agatatgtaa atgctgacag tggtcttcaa 19800
gcaaattaca attctgcgtt aaattatggt tctcaaatta ttgcaactac ccaaccacca 19860
gagcttaata aagatgtaat aaatagagca actcaaacga ttaaaactgc tgaaaataat 19920
ttaaatgggc aatctaaatt agcagaggct aagtcagacg gaaatcaaag catcgaacat 19980
ttgcaaggat taacacaatc acaaaaagat aaacaacatg atttaattaa tcaagctcaa 20040
actaaacaac aggtagatga tatcgtaaat aactctaaac aattagataa ctctatgaat 20100
caactacaac aaattgttaa caatgacaat acagtaaaac aaaatagtga tttcattaat 20160
gaagattcca gccaacagga tgcttataat catgcaattc aagcagcaaa agatttgata 20220
actgctcatc caactatcat ggataaaaat caaatagatc aagctattga aaatatcaaa 20280
caagcactta atgatttaca cggtagtaat aaactatcag aagataaaaa agaagcttca 20340
gaacaactac aaaaccttaa tagcttgacg aacgggcaaa aagatacgat ttttaatcat 20400
attttcagtg caccaacaag aagccaagta ggagaaaaaa ttgcaagtgc taaacaatta 20460

aataatacaa tgaaagcact tagagattct attgctgata ataatgaaat tttacaaagt 20520
agtaagtact tcaatgaaga ttctgaacaa caaaatgctt ataatcaagc cgtaaataaa 20580
gctaaaaata taattaatga tcaaccaaca ccagtaatgg caaatgatga gattcaaagt 20640
gtcctaaatg aagttaaaca aactaaagat aatttacatg gtgatcaaaa acttgctaac 20700
gacaagacag atgctcaagc aacattaaat gcgttaaatt acttaaatca agcgcaaaga 20760
ggtaatcttg aaactaaagt tcaaaactct aattctagac cagaagtaca aaaagtagtt 20820
caattagcaa atcaacttaa tgatgcgatg aaaaaattag atgatgcttt aactggtaat 20880
gacgcaataa aacaaacgag taattatatt aatgaagata cttctcaaca agttaacttt 20940
gatgagtata cagatagagg taaaaacata gttgctgaac aaacaaatcc aaatatgtct 21000
ccaactaata ttaacactat tgctgataaa attactgaag ctaaaaacga tttacatggc 21060
gtacaaaaac taaaacaagc tcaacaacag tccatcaata ctattaatca aatgactggt 21120
ctaaaccaag ctcaaaaaga acaattaaat caagaaattc aacaaactca aacccgttct 21180
gaagtacatc aagtaattaa taaagcacaa gctttaaatg attcaatgaa tactttacgt 21240
caaagtatta ctgatgaaca tgaagttaaa caaacaagta actacatcaa tgaaactgtt 21300
ggtaatcaaa ctgcatataa caatgccgtt gatcgtgtaa aacaaataat caatcaaaca 21360
tctaatccaa ctatgaatcc tttagaggtg gaacgtgcaa catcaaatgt aaaaatttct 21420
aaagatgcac ttcatggtga acgtgaattg aatgacaata aaaattcaaa aacttttgca 21480
gtcaatcact tagataacct caatcaagct caaaaagaag cattaactca tgaaattgaa 21540
caagcaacta tagtttcaca agtaaataat atctataaca aagcgaaagc tttaaataat 21600
gatatgaaaa aacttaaaga tatcgttgct caacaagata atgtgagaca atcaaacaat 21660
tatataaacg aggatagtac acctcaaaat atgtacaacg atacaattaa tcatgcacaa 21720
tcaatcattg atcaagtagc aaaccctacg atgtctcatg acgaaataga gaatgcaatc 21780
aataacataa agcatgccat caatgcactc gatggagaac ataaattaca acaagcaaaa 21840
gaaaatgcaa acttattgat taatagttta aacgatttaa atgcaccaca aagagatgcc 21900
ataaatagat tggttaatga agctcaaaca agagaaaaag tagctgaaca acttcaaagt 21960
gctcaagctt taaatgacgc tatgaagcat ttaagaaaca gcattcaaaa tcaatcatcc 22020
gtaagacaag agagcaaata tattaatgca agtgatgcta aaaaagagca atataatcac 22080
gcagttagag aagtcgaaaa tattatcaat gaacaacatc caacattgga taaagaaata 22140
attaagcaac taacggatgg tgtaaatcaa gcgaataatg acttaaatgg cgttgaatta 22200
ttagatgctg ataagcaaaa cgcacatcaa tcgataccta cattgatgca cttaaatcaa 22260
gcacaacaaa acgcattaaa tgaaaaaatt aataacgcag ttaccagaac tgaagttgcg 22320
gctattattg gccaagcaaa actactcgat catgctatgg agaatttaga agaaagtatc 22380
aaagataaag agcaagtcaa acagtcaagt aactatatta atgaagattc tgatgttcaa 22440
gaaacatacg ataacgccgt tgatcatgtg acagaaatac ttaatcaaac agtaaatcca 22500
actttatcta ttgaagatat agagcatgct atcaacgaag ttaatcaagc gaaaaaacaa 22560
ctcagaggta aacaaaaact ttatcaaact atcgatttag ctgataaaga attaagtaaa 22620
ttggatgatt taacatcaca acaaagcagt tcaatatcta atcaaatata tactgctaaa 22680
acgagaacag aagttgccca agcaattgaa aaagcaaaat cattaaatca tgcaatgaaa 22740
gcacttaaca aagtatataa aaatgcagat aaagtgttag atagtagtcg attcattaac 22800
gaagatcaac ctgaaaaaaa ggcgtatcaa caagctataa atcatgttga ttcaatcatt 22860
catagacaaa caaatcctga aatggatcca acagtaatca atagcataac tcatgaactc 22920
gaaacagctc aaaataactt acatggtgat cagaaacttg ctcatgcaca acaagatgcc 22980
gctaatgtaa ttaatggtct aattcatctt aatgttgctc aacgtgaggt aatgataaat 23040
acgaatacaa atgctacaac acgcgaaaaa gttgcaaaga acttagataa tgctcaagct 23100
cttgataaag ctatggaaac actacaacaa gtagttgctc ataaaaataa tatattgaac 23160
gatagtaaat atttaaatga agattcaaaa tatcaacaac aatacgatcg agttattgct 23220
gatgccgaac aactacttaa tcagacaaca aatccaacat tagaacctta taaagtcgat 23280
attgttaagg ataatgtcct agctaacgaa aaaatactat ttggcgcaga aaaactatca 23340
tatgacaaat caaatgcaaa tgatgaaatt aaacatatga attatcttaa taatgcacaa 23400
aagcaatcta taaaagatat gatttctcac gcagcattaa gaactgaagt taaacaactt 23460
ctgcaacaag ctaaaatcct tgatgaagcc atgaaatcac ttgaagataa aactcaagta 23520
gtgattacag atactacttt gcctaattac actgaagctt cagaggataa aaaggaaaaa 23580
gtagaccaaa ctgtatcaca tgctcaagcg attattgata aaataaatgg ctcaaatgta 23640
agtttagatc aagtacgaca agcactagaa caattaactc aagcatcaga aaacctcgat 23700
ggtgatcagc gagttgaaga agctaaagtt catgctaatc aaacaattga tcaattaaca 23760
catcttaatt cattacaaca acaaactgcg aaagaaagtg ttaaaaacgc aacaaaacta 23820
gaagaaatcg ctactgttag taacaatgct caggcattaa acaaagtaat gggtaaatta 23880
gaacaattca ttaatcatgc tgattctgtt gaaaatagtg ataattatag acaagccgac 23940
gacgacaaaa tcatcgctta tgatgaagca cttgaacatg gacaagatat acaaaaaact 24000
aacgcaaccc aaaatgaaac aaaacaagcg ttacaacaat taatatatgc agaaacatcg 24060
ttaaatggtt tcgaaagatt aaatcatgct agaccacgag ctttagaata tatcaaatca 24120
ctagaaaaaa taaacaatgc tcaaaagtct gctttagagg ataaagtaac gcaatcgcat 24180
gatttattag aattagaaca tattgtcaac gagggcacaa acctcaatga cattatgggt 24240
gaattagcta acgcaatcgt taataactat gctccaacca aagcaagtat aaattatatt 24300
aacgccgata acctacgcaa agataacttt actcaagcta tcaacaatgc acgtgatgca 24360

ctcaacaaaa ctcaaggtca gaacttagat ttcaatgcaa ttgatacatt taaagatgat 24420
atattcaaaa ctaaagatgc acttaacggt attgaacgtt taacagctgc aaaatcaaaa 24480
gcagaaaaac taattgatag tttaaaattt attaataaag ctcaattcac acatgcaaat 24540
gatgaaatta tgaatactaa ttctattgca caattgtcta gaatcgtgaa tcaagcattt 24600
gatttaaatg atgcaatgaa atctttaaga gatgaactta ataatcaagc ttttcctgtc 24660
caagcaagct caaattatat aaattcagat gaagatttaa aacaacaatt tgaccatgct 24720
ttaagtaatg ctcgaaaagt tcttgcaaaa gaaaatggta aaaatttaga tgaaaaacaa 24780
attcagggac tcaaacaagt gattgaggat actaaagatg ctttaaatgg tatccaacgt 24840
ttatcaaaag ctaaagctaa agcaattcaa tacgtacaat ctttatctta tatcaatgat 24900
gcacagcgtc atattgctga aaataatatt cacaactctg atgatttatc atctttagca 24960
aatacattat ctaaagctag tgatttagat aatgcaatga aagacttacg agatactata 25020
gaaagtaatt caacttctgt tccaaatagt gtgaattata ttaatgctga taagaattta 25080
caaattgaat ttgatgaggc gctacaacaa gcaagtgcaa caagttctaa aacttcagaa 25140
aatccagcaa cgattgaaga agtattaggt cttagtcaag ccatttacga tacaaaaaat 25200
gcattaaatg gtgaacaacg acttgcaact gagaagagca aagatctaaa attaataaaa 25260
ggattaaaag atttaaataa agcacaactt gaagatgtca caaacaaggt aaattcagca 25320
aatactttaa cagagttatc tcagctcact caatcaacgt tagaattaaa cgataaaatg 25380
aaattattga gagataagct taaaacttta gtaaatcctg ttaaagcaag tttaaattat 25440
agaaacgctg attataattt aaaacgtcaa tttaacaaag ctttaaaaga agctaaaggc 25500
gtattaaata aaaatagcgg tacaaatgtc aatatcaatg acattcaaca tcttttaaca 25560
caaatagata atgctaaaga ccaattaaat ggtgaacgac gtctaaaaga acatcaacaa 25620
aaatctgaag tatttattat taaagaatta gatatactta ataatgctca aaaagctgca 25680
ataattaatc agattagagc gtctaaagac attaaaataa ttaatcaaat cgttgataat 25740
gcaatagaat taaatgatgc tatgcaaggt ttaaaagaac atgtagctca attaacagca 25800
actacaaaag acaacattga atatttaaat gctgatgaag accataaatt acaatatgat 25860
tacgctatca acttagcgaa taatgttctt gacaaagaaa acggtacaaa taaagacgct 25920
aatatcataa ttggaatgat tcaaaacatg gatgatgcta gagcacttct aaatggaatt 25980
gaaagactta aagatgctca aacaaaagca cataatgaca ttaaagatac gctcaaacgt 26040
caacttgatg aaattgaaca cgctaatgca acatcaaatt ctaaagctca agctaaacaa 26100
atggtaaatg aggaagctag aaaagcgctt tctaatatta atgacgcaac atcaaatgat 26160
ttagttaatc aagcaaaaga tgaagggcaa tctgcaattg aacacataca tgcagatgaa 26220
ttacctaaag caaaactaga tgctaatcaa atgattgacc aaaaagttga agatataaat 26280
cacttaatta gtcaaaatcc aaacttatca aatgaagaaa aaaataaact aatatctcaa 26340
attaataagt tagtaaatgg aattaagaat gaaattcaac aagctataaa caaacaacaa 26400
atagaaaatg ctacaacaaa actagatgaa gtcattgaaa ctactaaaaa attaattatc 26460
gccaaagcag aagctaaaca aatgataaaa gagttatcac aaaagaaacg agatgcaata 26520
aataacaaca ctgatttaac accttctcaa aaggcacatg ctttagcaga tattgataaa 26580
acagaaaaag atgcacttca acatatcgaa aattctaatt caattgatga tatcaataac 26640
aataaagagc atgcatttaa tactttagct catatcatta tttgggatac tgatcagcaa 26700

ccattagttt ttgaactacc tgaattgagc cttcaaaatg ctctagtaac aagtgaggtg 26760
gttgttcaca gagatgaaac tatttcatta gaatctataa ttggagctat gactttaact 26820
gatgaactta aagtcaatat tgtttcatta ccgaacactg ataaagtagc tgatcaccta 26880
accgctaaag ttaaggttat tttagctgat ggctcatatg tcactgtaaa tgttccagtc 26940
aaagttgtag aaaaagaatt acaaatagct aaaaaggatg ctataaaaac aattgatgtt 27000
ctggtaaaac aaaaaatcaa agatatagat tctaataacg aattaacgtc tactcaacgt 27060
gaagatgcaa aagctgaaat tgaaagattg aaaaagcaag ccatcgataa agtgaatcat 27120
tcaaaatcga ttaaagatat tgaaacagta aaacgaactg attttgaaga aatagatcag 27180
tttgatccta aacgctttac gctaaataaa gctaaaaagg atatcattac tgatgttaat 27240
actcaaatcc aaaatggttt caaagaaatt gaaacaataa aaggtttaac ttctaatgaa 27300
aaaactcagt ttgataaaca attaactgca ctacaaaaag aatttttaga aaaagtcgag 27360
catgctcata atttagtaga attaaatcaa ttacaacaag agtttaataa tagatataaa 27420
catattttaa accaagcaca tttactaggt gaaaaacata tagcagaaca taaattagga 27480
tatgttgtag taaacaaaac tcagcaaata ctaaataatc aatctgcttc ttactttata 27540
aaacaatggg cacttgatag aattaaacaa attcaactag aaacgatgaa ttcaattcgt 27600
ggtgcgcata ccgtacaaga tgtacacaaa gcattattac aaggtataga gcaaatcttg 27660
aaagtaaatg taagtattat aaatcaatct ttcaacgatt ccttgcataa ctttaattat 27720
cttcattcaa aatttgatgc tagattaaga gaaaaggatg ttgcaaacca tatcgtacaa 27780
actgaaacat tcaaagaagt tctaaaagga acgggtgttg aaccaggtaa aatcaacaaa 27840
gaaacacagc aaccaaaact tcataagaat gataatgata gcctattcaa acatttagtt 27900
gataatttcg gcaaaactgt aggtgttatt acattaactg gtttactttc tagtttctgg 27960
ttagttttgg ctaaaagacg taaaaaagaa gaagaagaaa aacaatcgat aaaaaatcat 28020
cacaaagata ttcgtctttc agatactgat aaaatagatc caattgtaat aactaagcgt 28080
aaaatagata aagaagaaca aattcaaaac gatgacaaac attcaattcc agttgctaaa 28140
cataagaaat ctaaagaaaa gcaattgagt gaagaggata ttcattcaat ccccgtcgtt 28200

aagcgtaaac aaaacagtga taacaaagat acaaaacaga agaaagttac ttctaaaaag 28260
aagaaaacgc ctcagtcaac taaaaaagtt gtaaaaacca aaaagcgttc taaaaag 28317

<210> 49
<211> 3348
<212> DNA
<213> Staphylococcus aureus

<400> 49
atgagagata agaaaggacc ggtaaataaa agagtagatt ttctatcaaa taaattgaat 60
aaatattcaa taagaaaatt tacagttgga acagcatcta ttttaattgg ctcactaatg 120
tatttgggaa ctcaacaaga agcagaagca gctgaaaaca atattgagaa tccaactaca 180
ttaaaagata atgtccaatc aaaagaagtg aagattgaag aagtaacaaa caaagacact 240
gcaccacaag gtgtagaagc taaatctgaa gtaacttcaa acaaagacac aatcgaacat 300
gaagcatcag taaaagctga agatatatca aaaaaggagg atacaccaaa agaagtagct 360
aatgttgctg aagttcagcc gaaatcgtca gtcactcata acgcagaggc acctaaggtt 420
agaaaagctc gttctgttga tgaaggctct tttgatatta caagagattc taaaaatgta 480
gttgaatcta ccccaattac aattcaaggt aaagaacatt ttgaaggtta cggaagtgtt 540
gatatacaaa aaaacccaac agatttaggg gtatcagagg taaccaggtt taatgttggt 600
aatgaaagta atggtttgat aggagcttta caattaaaaa ataaaataga ttttagtaag 660
gatttcaatt ttaaagttag agtggcaaat aaccatcaat caaataccac aggtgctgat 720
ggttgggggt tcttatttag taaaggaaat gcagaagaat atttaactaa tggtggaatc 780
cttggggata aaggtctggt aaattcaggc ggatttaaaa ttgatactgg atacatttat 840
acaagttcca tggacaaaac tgaaaagcaa gctggacaag gttatagagg atacggagct 900
tttgtgaaaa atgacagttc tggtaattca caaatggttg gagaaaatat tgataaatca 960
aaaactaatt ttttaaacta tgcggacaat tcaactaata catcagatgg aaagtttcat 1020
gggcaacgtt taaatgatgt catcttaact tatgttgctt caactggtaa aatgagagca 1080
gaatatgctg gtaaaacttg ggagacttca ataacagatt taggtttatc taaaaatcag 1140
gcatataatt tcttaattac atctagtcaa agatggggcc ttaatcaagg gataaatgca 1200
aatggctgga tgagaactga cttgaaaggt tcagagttta cttttacacc agaagcgcca 1260
aaaacaataa cagaattaga aaaaaaagtt gaagagattc cattcaagaa agaacgtaaa 1320
tttaatccgg atttagcacc agggacagaa aaagtaacaa gagaaggaca aaaaggtgag 1380
aagacaataa caacaccaac actaaaaaat ccattaactg gagaaattat tagtaaaggt 1440
gaatcgaaag aagagatcac aaaagatccg attaatgaat taacagaata cggaccagaa 1500
acgatagcac caggtcatcg agacgaattt gatccgaagt taccaacagg agagaaagaa 1560
gaagttccag gtaaaccagg aattaagaat ccagaaacag gagacgtagt tagaccaccg 1620
gtcgatagtg taacaaaata tggacctgta aaaggagact cgattgtaga aaaagaagaa 1680
attccattcg agaaagaacg taaatttaat cctgatttag caccaggaac agaaaaagta 1740
acaagagaag gacaaaaagg tgagaagaca ataacgacac caacactaaa aaatccatta 1800
actggagaaa ttattagtaa aggtgaatcg aaagaagaga tcacaaaaga tccgattaat 1860
gaattaacag aatacggacc tgaaacaata gcgccaggtc atcgagacga atttgatccg 1920
aagttaccaa caggagagaa agaagaagtt ccaggtaaac caggaattaa gaatccagaa 1980
acaggagacg tagttagacc gccggtcgat agcgtaacaa aatatggacc tgtaaaagga 2040
gactcgattg tagaaaaaga agaaattcca ttcaagaaag aacgtaaatt taatcctgat 2100
ttagcaccag ggacagaaaa agtaacaaga gaaggacaaa aaggtgagaa gacaataacg 2160
acgccaacac taaaaaatcc attaactgga gaaattatta gtaaaggtga atcgaaagaa 2220
gaaatcacaa aagatccgat taatgaatta acagaatacg gaccagaaac gataacacca 2280
ggtcatcgag acgaatttga tccgaagtta ccaacaggag agaaagagga agttccaggt 2340
aaaccaggaa ttaagaatcc agaaacagga gatgtagtta gaccaccggt cgatagcgta 2400
acaaaatatg gacctgtaaa aggagactcg attgtagaaa aagaagaaat tccattcgag 2460
aaagaacgta aatttaatcc tgatttagca ccagggacag aaaaagtaac aagagaagga 2520
caaaaaggtg agaagacaat aacgacgcca acactaaaaa atccattaac tggagaaatt 2580
attagtaaag gtgaatcgaa agaagaaatc acaaaagatc cagttaatga attaacagaa 2640
ttcggtggcg agaaaatacc gcaaggtcat aaagatatct ttgatccaaa cttaccaaca 2700
gatcaaacgg aaaaagtacc aggtaaacca ggaatcaaga atccagacac aggaaaagtg 2760
atcgaagagc cagtggatga tgtgattaaa cacggaccaa aaacgggtac accagaaaca 2820
aaaacagtag agataccgtt tgaaacaaaa cgtgagttta atccaaaatt acaacctggt 2880
gaagagcgag tgaaacaaga aggacaacca ggaagtaaga caatcacaac accaatcaca 2940
gtgaacccat taacaggtga aaaagttggc gagggtcaac caacagaaga gatcacaaaa 3000
caaccagtag ataagattgt agagttcggt ggagagaaac caaaagatcc aaaaggacct 3060
gaaaacccag agaagccgag cagaccaact catccaagtg gcccagtaaa tcctaacaat 3120
ccaggattat cgaaagacag agcaaaacca aatggcccag ttcattcaat ggataaaaat 3180
gataaagtta aaaaatctaa aattgctaaa gaatcagtag ctaatcaaga gaaaaaacga 3240

gcagaattac caaaaacagg tttagaaagc acgcaaaaag gtttgatctt tagtagtata 3300
attggaattg ctggattaat gttattggct cgtagaagaa agaattaa 3348

<210> 50
<211> 4410
<212> DNA
<213> Staphylococcus aureus

<400> 50
atgggcaaac gtagacaagg tcctattaat aaaaaagtgg attttttacc taacaaatta 60
aacaagtatt ctataagaaa attcactgtt ggtacggcct caatattact tggttcgaca 120
cttatttttg gaagtagtag ccatgaagcg aaagctgcag aagaaaaaca agttgatcca 180
attacacaag ctaatcaaaa tgatagtagt gaaagatcac ttgaaaacac aaatcaacct 240
actgtaaaca atgaagcacc acagatgtct tctacattgc aagcagaaga aggaagcaat 300
gcagaagcac cgaatgttcc aactatcaaa gctaattcag ataatgatac acaaacacaa 360
ttttcagaag cccctacaag aaatgaccta gctagaaaag aagatatccc tgctgtttct 420
aaaaacgagg aattacaatc atcacaacca aacactgaca gtaaaataga acctacaact 480
tcagaacctg tgaatttaaa ttatagttct ccgtttatgt ccttattaag catgcctgct 540
gatagttcat ccaataacac taaaaataca atagatatac cgccaactac ggttaaaggt 600
agagataatt acgattttta cggtagagta gatatccaaa gtaatcctac agatttaaat 660
gcgacaaatt taacgagata taattatgga cagccacctg gtacaacaac agctggtgca 720
gttcaattta aaaatcaagt tagttttgat aaagatttcg actttaacat tagagtagca 780
aacaatcgtc aaagtaatac aactggtgca gatggttggg gctttatgtt cagcaagaaa 840
gatggggatg atttcctaaa aaacggtggt atcttacgtg aaaaaggtac acctagtgca 900
gctggtttca gaattgatac aggatattat aataacgatc cattagataa aatacagaaa 960
caagctggtc aaggctatag agggtatggg acatttgtta aaaatgactc ccaaggtaat 1020
acttctaaag taggatcagg tactccatca acagattttc ttaactacgc agataatact 1080
actaatgatt tagatggtaa attccatggt caaaaattaa ataatgttaa tttgaaatat 1140
aatgcttcaa atcaaacttt tacagctact tatgctggta aaacttggac ggctacgtta 1200
tctgaattag gattgagtcc aactgatagt tacaattttt tagttacatc aagtcaatat 1260
ggaaatggta atagtggtac atacgcagat ggcgttatga gagctgattt agatggtgca 1320
acattgacat atactcctaa agcagtcgat ggagacccaa ttacatcaac taaggaaata 1380
ccatttaata aaaaacgcga atttgatcca aacttagcgc caggtacaga aaaagtcgtt 1440
caaaaaggtg aaccaggaat tgaaacaaca acaacaccaa cttatgtcaa tcctaatact 1500
ggagaaaaag taggtgaagg cacacctaca acaaagatca ctaaacaacc agtggatgaa 1560
atcgttcatt atggtggcga agaaatcaag ccaggacata aagatgaatt tgatccaaat 1620
gcaccgaaag gtagtcaaac aacgcaacca ggtaagccag gagttaaaaa tcctgataca 1680
ggcgaagtag tcacaccacc agtggatgat gtgacaaaat atggtccagt tgatggagat 1740
ccgattacgt caacggaaga aattccattc gacaagaaac gtgaattcaa tcctgattta 1800
aaaccaggtg aagagcgtgt taaacaaaaa ggtgaaccag gaacaaaaac aattacaaca 1860
ccaacaacta agaacccatt aacaggggaa aaagttggcg aaggtgaacc aacagaaaaa 1920
ataacaaaac aaccagtaga tgaaatcaca gaatatggtg gcgaagaaat caagccaggc 1980
cataaggatg aatttgatcc gaacgcaccg aaaggtagcc aagaggacgt tccaggtaaa 2040
ccaggagtta aaaatcctga tacaggcgaa gtagtcacac caccagtgga tgatgtgaca 2100
aaatatggtc cagttgatgg agatccgatt acgtcaacgg aagaaattcc gtttgataaa 2160
aaacgcgaat ttgatccaaa cttagcgcca ggtacagaga aagtcgttca aaaaggtgaa 2220
ccaggaacaa aaacaattac aacaccaaca actaagaacc cattaacagg agaaaaagtt 2280
ggcgaaggtg aaccaacaga aaaaataaca aaacaaccag tggatgaaat cgttcattat 2340
ggtggcgaag aaatcaagcc aggccataag gatgaatttg atccgaacgc accgaaaggt 2400
agccaagagg acgttccagg taagccagga gttaaaaatc ctgatacagg cgaagtagtc 2460
acaccaccag tggatgatgt gacaaaatat ggtccagttg atggagatcc gattacgtca 2520
acggaagaaa ttccattcga caagaaacgt gaattcaatc ctgatttaaa accaggtgaa 2580
gagcgtgtta aacaaaaagg tgaaccagga acaaaaacaa ttacaacacc aacaactaag 2640
aacccattaa caggggaaaa agttggcgaa ggtgaaccaa cagaaaaagt aacaaaacaa 2700
ccagtggatg aaatcgttca ttatggtggc gaagaaatca agccaggcca taaggatgaa 2760
tttgatccaa atgcaccgaa aggtagccaa gaagacgttc caggtaaacc aggagttaaa 2820
aaccctgata caggcgaagt agttactcca ccagtggatg atgtgacaaa atatggtcca 2880
gttgatggag atccgattac gtcaacggaa gaaattccgt ttgataaaaa acgcgaattt 2940
gatccaaact tagcgccagg tacagagaaa gtcgttcaaa aaggtgaacc aggaacaaaa 3000
acaattacaa caccaacaac taagaaccca ttaacaggag aaaaagttgg cgaaggtgaa 3060
ccaacagaaa aaataacaaa acaaccagtg gatgagatcg ttcattatgg tggcgaagaa 3120
atcaagccag gccataagga tgaatttgat ccgaacgcac cgaaaggtag tcaaacaacg 3180
caaccaggta agccaggagt taaaaatcct gatacaggcg aagtagtcac accaccagtg 3240
gatgatgtga caaaatatgg tccagttgat ggagatccga ttacgtcaac ggaagaaatt 3300
ccgtttgata aaaaacgcga atttgatcca aacttagcgc caggtacaga gaaagtcgtt 3360

caaaaaggtg aaccaggaac aaaaacaatt acaacgccaa caactaagaa cccattaaca 3420
ggagaaaaag ttggcgaagg tgaaccaaca gaaaaaataa caaaacaacc agtggatgag 3480
attgttcatt atggtggtga acaaatacca caaggtcata aagatgaatt tgatccaaat 3540
gcacctgtag atagtaaaac tgaagttcca ggtaaaccag gagttaaaaa tcctgataca 3600
ggtgaagttg ttaccccacc agtggatgat gtgacaaaat atggtccgaa agttggtaat 3660
ccaatcacat caacggaaga gattccattt gataagaaac gtgtatttaa tcctgattta 3720
aaaccaggtg aagagcgcgt taaacaaaaa ggtgaaccag gaacaaaaac aattacaaca 3780
ccaatattag ttaatcctat tacaggagaa aaagttggcg aaggtaaatc aacagaaaaa 3840
gtcactaaac aacctgttga cgaaattgtt gagtatggtc caacaaaagc agaaccaggt 3900
aaaccagcgg aaccaggtaa accagcggaa ccaggtaaac cagcggaacc aggtaaacca 3960
gcggaaccag gtacgccagc agaaccaggt aaaccagcgg aaccaggtaa accagcggaa 4020
ccaggtaaac cagcggaacc aggtaaacca gcggaaccag gtaaaccagc ggaaccaggt 4080
acgccagcag aaccaggtaa accagcggaa ccaggtaaac cagcggaacc aggtaaacca 4140
gcggaaccag gtacgccagc agaaccaggt aaaccagcgg aaccaggtac gccagcagaa 4200
ccaggtaaac cagcggaacc aggtacgcca acacaatcag gtgcaccaga acaaccaaat 4260
agatcaatgc attcaacaga taataaaaat caattacctg atacaggtga aaatcgtcaa 4320
gctaatgagg gaactttagt cggatctcta ttagcaattg tcggatcatt gttcatattt 4380
ggtcgtcgta aaaaaggtaa tgaaaaataa 4410

<210> 51
<211> 504
<212> DNA
<213> Staphylococcus aureus

<400> 51
atgaagaaac tatatacatc ttatggcact tatggatttt tacatcaaat aaaaatcaat 60
aacccgaccc atcaactatt ccaattttca gcatcagata cttcagttat ttttgaagaa 120
actgatggtg agactgtttt aaaatcacct tcaatatatg aagttattaa agaaattggt 180
gaattcagtg aacatcattt ctattgtgca atcttcattc cttcaacaga agatcatgca 240
tatcaacttg aaaagaaact gattagtgta gacgataatt tcagaaactt tggtggcttt 300
aaaagctatc gtttgttaag acctgctaaa ggtacaacat ataaaattta tttcggattt 360
gctgatcgac atgcatacga agactttaag caatctgatg cctttaatga ccatttttca 420
aaagacgcat taagtcatta ctttggttca agcggacaac attcaagtta ttttgaaaga 480
tatctatacc caataaaaga atag 504

<210> 52
<211> 504
<212> DNA
<213> Staphylococcus aureus

<400> 52
atgtatttat atacatctta tgggacttac caatttttaa atcaaattaa acttaatcat 60
caagaacgta gtttatttca attttccact aatgattcct caataatctt agaagagtct 120
gagggaaaat caatcttaaa acatcctagt gcatatcaag tgattgatag cacaggtgaa 180
tttaacgaac atcattttta tagtgctatt tttgtcccta catctgaaga tcatcgtcaa 240
cagctagaga aaaaattatt actcgtagac gtacctttaa gaaattttgg tggttttaaa 300
agctatcgtt tattaaaacc cactgagggg tctacctaca aaatttactt tggttttgca 360
aatcgaacag catatgaaga tttcaaagct tctgatatat ttaatgaaaa cttttcaaaa 420
gatgcattga gccaatactt tggtgctagt ggtcaacatt ctagctactt tgaaagatat 480
ttatatccaa tagaagatca ttaa 504

<210> 53
<211> 3426
<212> DNA
<213> Staphylococcus aureus

<400> 53
atgattaaca gggataataa aaaggcaata acaaaaaagg gtatgatttc aaatcgctta 60
aacaaatttt cgattagaaa gtatactgta ggaactgcat cgattttagt aggtacgaca 120
ttgatttttg gtctagggaa ccaagaagct aaagctgctg aaaacactag tacagaaaat 180
gcgaaacaag atgatgcaac gactagtgat aataaagaag tagtgtcgga aactgaaaat 240
aattcgacaa cagaaaatga ttcaacaaat ccaattaaga aagaaacaaa tactgattca 300
caaccagaag ctaaagaaga atcaactaca tcaagtactc aacaacagca aaataacgtt 360
acagctacaa ctgaaactaa gcctcaaaac attgaaaaag aaaatgttaa accttcaact 420
gataaaactg cgacagaaga tacatctgtt attttagaag agaagaaagc accaaattat 480

acaaataacg atgtaactac aaaaccatct acaagtgaaa ttcaaacaaa accaactaca 540
cctcaagaat ctacaaatat tgaaaattca caaccgcaac caacgccttc aaaagtagac 600
aatcaagtta cagatgcaac taatccaaaa gaaccagtaa atgtgtcaaa agaagaactt 660
aaaaataatc ctgagaaatt aaaagaatta gttagaaatg ataacaatac agatcgttca 720
actaaaccag ttgctacagc tccaacaagt gttgcaccaa aacgattaaa tgcgaaaatg 780
cgttttgcag ttgcacaacc agcagcagtt gcttcaaata atgtaaatga cttaattaca 840
gttacgaaac agacgatcaa agttggcgat ggtaaagata atgtggcagc agcgcatgac 900
ggtaaagata ttgaatatga tacagagttt acaattgaca ataaagtcaa aaaaggcgat 960
acaatgacga ttaattatga taagaatgta attccttcgg atttaacaga taaaaatgat 1020
cctatcgata ttactgatcc atcaggagag gtcattgcca aggaacattt tgataaagcg 1080
actaagcaaa tcacatatac atttacagat tatgtagata aatatgaaga tataaaagca 1140
cgtttaactt tatactcata tattgataag caagcagtac ctaatgaaac tagtttgaat 1200
ttaacgtttg caacagcagg taaagaaact agccaaaacg tttctgttga ttatcaagac 1260
ccaatggttc atggtgattc aaacattcaa tctatcttta caaagttaga tgaaaacaaa 1320
caaactattg aacaacaaat ttatgttaat cctttgaaaa aaacagcaac taacactaaa 1380
gttgatatag ctggtagtca agtagatgat tatggaaata ttaaactagg aaatggtagt 1440
accattattg accaaaatac agaaataaaa gtttataaag ttaaccctaa tcaacaattg 1500
cctcaaagta atagaatcta tgattttagt caatacgaag atgtaacaag tcaatttgat 1560
aataaaaaat catttagtaa taatgtagca acattggatt ttggtgatat taattcagcc 1620
tatattatca aagttgttag taaatataca cctacatcag atggcgaact agatattgct 1680
caaggtacta gtatgagaac aactgataaa tatggttatt ataattatgc aggatattca 1740
aacttcatcg taacttctaa tgacactggc ggtggcgacg gtactgttaa acctgaagaa 1800
aagttataca aaattggtga ctatgtatgg gaagacgttg ataaagacgg tgtccaaggt 1860
acagattcga aagaaaagcc aatggcaaac gttttagtta cattaactta cccggacggt 1920
actacaaaat cagtaagaac agatgctaac ggtcattatg aattcggtgg tttgaaagac 1980
ggagaaactt atacagttaa attcgaaacg ccagctggat atcttccaac aaaagtaaat 2040
ggaacaactg atggtgaaaa agactcaaat ggtagttcta taactgttaa aattaatggt 2100
aaagatgata tgtctttaga cactggtttt tataaagaac ctaaatataa tcttggtgac 2160
tatgtatggg aagatacaaa taaagatggt atccaagatg ctaatgaacc tggtatcaaa 2220
gatgttaagg ttacattaaa agatagtact ggaaaagtta ttggtacaac tactactgat 2280
gcctcgggta aatataaatt tacagattta gataatggta actatacagt agaatttgaa 2340
acaccagcag gttacacgcc aacggttaaa aatactacag ctgaagataa agattctaat 2400
ggtttaacaa caacaggtgt cattaaagat gcagataata tgacattaga cagtggtttc 2460
tataaaacac caaaatacag tttaggtgat tatgtttggt acgacagtaa taaagacggt 2520
aaacaagatt caactgaaaa aggtatcaaa gatgttaaag ttactttatt aaatgaaaaa 2580
ggcgaagtaa ttggaacaac taaaacagat gaaaatggta aatatcgttt cgataattta 2640
gatagcggta aatacaaagt tatttttgaa aagcctgctg gcttaacaca aacagttaca 2700
aatacaactg aagatgataa agatgccgat ggtggcgaag ttgacgtaac aattacggat 2760
catgatgatt tcatacttga taacggatac ttcgaagaag atacatcaga cagtgattca 2820
gactcagaca gtgattcaga ctcagacagc gactcagatt cagacagtga ttcagactca 2880
gatagcgatt cagattcaga cagcgactca gactcagata gcgactcaga ctcagacagc 2940
gactcagact cagatagcga ctcagattcg gacagcgatt cagactcaga tagcgactca 3000
gattcagaca gcgattcaga ctcagatagc gactcagatt cagacagtga ctcagactca 3060
gatagcgact cagactcaga cagtgactca gactcagaca gcgattcaga ttcagatagc 3120
gactcagatt cggacagtga ttcagactca gatagcgact cagattcaga cagcgactca 3180
gactcagata gcgactcaga ctcagacagt gattcagact cagatagcga ttcggactcg 3240
gatgcaggaa aacatacacc tgttaaacca atgagtacta ctaaagacca tcacaataaa 3300
gcaaaagcat taccagaaac aggtagtgaa aataacggct caaataacgc aacgttattt 3360
ggtggattat ttgcagcatt aggttcatta ttgttattcg gtcgtcgcaa aaaacaaaac 3420
aaataa 3426

<210> 54
<211> 3171
<212> DNA
<213> Staphylococcus aureus

<400> 54
atgattaata aaaaaaataa tttactaact aaaaagaaac ctatagcaaa taaatccaat 60
aaatatgcaa ttagaaaatt cacagtaggt acagcgtcta ttgtaatagg tgcaacatta 120
ttgtttggtt taggtcataa tgaggccaaa gccgaggaga attcagtaca agacgttaaa 180
gattcgaata cggatgatga attatcagac agcaatgatc agtctagtga tgaagaaaag 240
aatgatgtga tcaataataa tcagtcaata aacaccgacg ataataacca aataattaaa 300
aaagaagaaa cgaataacta cgatggcata gaaaaacgct cagaagatag aacagagtca 360
acaacaaatg tagatgaaaa cgaagcaaca tttttacaaa agacccctca agataatact 420
catcttacag aagaagaggt aaaagaatcc tcatcagtcg aatcctcaaa ttcatcaatt 480

gatactgccc aacaaccatc tcacacaaca ataaatagag aagaatctgt tcaaacaagt 540
gataatgtag aagattcaca cgtatcagat tttgctaact ctaaaataaa agagagtaac 600
actgaatctg gtaaagaaga gaatactata gagcaaccta ataaagtaaa agaagattca 660
acaacaagtc agccgtctgg ctatacaaat atagatgaaa aaatttcaaa tcaagatgag 720
ttattaaatt taccaataaa tgaatatgaa aataaggcta gaccattatc tacaacatct 780
gcccaaccat cgattaaacg tgtaaccgta aatcaattag cggcggaaca aggttcgaat 840
gttaatcatt taattaaagt tactgatcaa agtattactg aaggatatga tgatagtgaa 900
ggtgttatta aagcacatga tgctgaaaac ttaatctatg atgtaacttt tgaagtagat 960
gataaggtga aatctggtga tacgatgaca gtggatatag ataagaatac agttccatca 1020
gatttaaccg atagctttac aataccaaaa ataaaagata attctggaga aatcatcgct 1080
acaggtactt atgataacaa aaataaacaa atcacctata cttttacaga ttatgtagat 1140
aagtatgaaa atattaaagc acaccttaaa ttaacgtcat acattgataa atcaaaggtt 1200
ccaaataata ataccaagtt agatgtagaa tataaaacgg ccctttcatc agtaaataaa 1260
acaattacgg ttgaatatca aagacctaac gaaaatcgga ctgctaacct tcaaagtatg 1320
tttacaaaca tagatacgaa aaatcataca gttgagcaaa cgatttatat taaccctctt 1380
cgttattcag ccaaggaaac aaatgtaaat atttcaggga atggtgatga aggttcaaca 1440
attatagacg atagcacaat aattaaagtt tataaggttg gagataatca aaatttacca 1500
gatagtaaca gaatttatga ttacagtgaa tatgaagatg tcacaaatga tgattatgcc 1560
caattaggaa ataataatga tgtgaatatt aattttggta atatagattc accatatatt 1620
attaaagtta ttagtaaata tgaccctaat aaggatgatt acacgactat acagcaaact 1680
gtgacaatgc agacgactat aaatgagtat actggtgagt ttagaacagc atcctatgat 1740
aatacaattg ctttctctac aagttcaggt caaggacaag gtgacttgcc tcctgaaaaa 1800
acttataaaa tcggagatta cgtatgggaa gatgtagata aagatggtat tcaaaataca 1860
aatgataatg aaaaaccgct tagtaatgta ttggtaactt tgacgtatcc tgatggaact 1920
tcaaaatcag tcagaacaga tgaagatggg aaatatcaat ttgatggatt gaaaaacgga 1980
ttgacttata aaattacatt cgaaacacct gaaggatata cgccgacgct taaacattca 2040
ggaacaaatc ctgcactaga ctcagaaggt aattctgtat gggtaactat taatggacaa 2100
gacgatatga cgattgatag tggattttat caaacaccta aatacagctt agggaactat 2160
gtatggtatg acactaataa agatggtatt caaggtgatg atgaaaaagg aatctctgga 2220
gttaaagtga cgttaaaaga tgaaaacgga aatatcatta gtacaactac aaccgatgaa 2280
aatggaaagt atcaatttga taatttaaat agtggtaatt atattgttca ttttgataaa 2340
ccttcaggta tgactcaaac aacaacagat tctggtgatg atgacgaaca ggatgctgat 2400
ggggaagaag ttcatgtaac aattactgat catgatgact ttagtataga taacggatac 2460
tatgatgacg aatcggattc cgatagtgac tcagacagcg actcagattc cgatagtgat 2520
tcagactccg atagcgactc ggattcagac agcgactcag attcagacag cgactcggat 2580
tctgatagcg actcggattc agacagcgac tcagactcag acagtgattc agattcagac 2640
agcgactcag attccgatag tgattcagac tcagacagcg actcagattc tgatagtgat 2700
tcagactcag acagtgattc agattcagac agcgactcag attccgatag tgattcagac 2760
tcagacagcg actcagattc cgatagtgat tcagactcag acagcgactc agattctgat 2820
agtgattcag actcagacag tgattcagat tccgatagtg attcagactc cgatagcgac 2880
tcagactcgg atagtgactc agattctgat agtgattcag actcagacag tgattcggat 2940
tccgatagtg attcagactc agacagcgac tcagattctg atagtgattc agactcagac 3000
aacgactcag atttaggcaa tagctcagat aagagtacaa aagataaatt acctgataca 3060
ggagctaatg aagattatgg ctctaaaggc acgttacttg gaactctgtt tgcaggttta 3120
ggagcgttat tattagggaa acgtcgcaaa aatagaaaaa ataaaaatta a 3171

<210> 55
<211> 1461
<212> DNA
<213> Staphylococcus aureus

<400> 55
atgtctaata attttaaaga tgactttgaa aaaaatcgtc aatcgataga cacaaattca 60
catcaagacc atacggaaga tgttgaaaaa gaccaatcag aattagaaca tcaggataca 120
atagagaata cggagcaaca gtttccgcca agaaatgccc aaagaagaaa aagacgccgt 180
gatttagcaa cgaatcataa taaacaagtt cacaatgaat cacaaacatc tgaagacaat 240
gttcaaaatg aggctggcac aatagatgat cgtcaagtcg aatcatcaca cagtactgaa 300
agtcaagaac ctagccatca agacagtaca cctcaacatg aagaggaata ttataataag 360
aatgcttttg caatggataa atcacatcca gaaccaatcg aagacaatga taaacacgag 420
actattaaag atgcagaaaa taacactgag cattcaacag tttctgataa gagtatagct 480
gaacaatctc agcaacctaa accatatttt gcaacaggtg ctaaccaagc aaatacatca 540
aaagataaac atgatgatgt aactgttaag caagacaaag atgaatctaa agatcatcat 600
agtggtaaaa aaggcgcagc aattggtgct ggaacagcgg gtgttgcagg tgcagctggt 660
gcaatgggtg tttctaaagc taagaaacat tcaaatgacg ctcaaaacaa aagtaattct 720
gacaagtcga ataactcgac tgaggataaa gcgtctcaag ataagtctaa agatcatcat 780

aatggcaaaa aaggtgcagc gatcggtgct ggaacagcag gtttggctgg aggcgcagca 840
agtaaaagtg cttctgccgc ttcaaaacca catgcctcta ataatgcaag ccaaaaccat 900
gatgaacatg acaatcatga cagagataaa gaacgtaaaa aaggtggcat ggccaaagta 960
ttgttaccat taattgcagc tgtactaatt atcggtgcat tagcgatatt tggaggcatg 1020
gcattaaaca atcataataa tggtacaaaa gaaaataaaa tcgcgaatac aaataaaaat 1080
aatgctgatg aaagtaaaga caaagacaca tctaaagacg cttctaaaga taaatcaaaa 1140
tctacagaca gtgataaatc aaaagaggat caagacaaag cgactaaaga tgaatctgat 1200
aatgatcaaa acaacgctaa tcaagcgaac aatcaagcac aaaataatca aaatcaacaa 1260
caagctaatc aaaatcaaca acagcaacaa caacgtcaag gtggtggcca aagacataca 1320
gtgaatggtc aagaaaactt ataccgtatc gcaattcaat actacggttc aggttcaccg 1380
gaaaatgttg aaaaaattag acgtgccaat ggtttaagtg gtaacaatat tagaaacggt 1440
caacaaatcg ttattccata a 1461

<210> 56
<211> 1419
<212> DNA
<213> Staphylococcus aureus

<400> 56
gtgattgaat taattaaaat ggaagggatg atagttgtgt ctaataataa ttttaaagat 60
gatttcgaaa agaatcgtca atctattaat ccagacgaac agcaaacaga attaaaagaa 120
gatgataaaa caaatgaaaa taaaaaagaa gctgactctc aaaacagttt atctaataac 180
tcaaatcaac aatttcctcc gagaaatgcc caacgacgaa aaagacgtag agagacagca 240
actaatcaaa gcaaacaaca agacgacaaa catcaaaaaa atagtgacgc taaaactaca 300
gaaggttcat tagatgaccg ttatgacgaa gcacagttac agcaacaaca tgataaatcg 360
caacaacaaa ataaaactga aaaacaatca caagataata gaatgaaaga tggaaaagat 420
gcagctattg taaatggaac atctgagtca ccagaacata aatcaaaatc aacacaaaat 480
agacccggcc ctaaagctca acaacaaaag cgtaaatcag aaagtacgca atcaaaaccg 540
tcaacaaaca aagataaaaa agcagctaca ggtgctggaa tagctggtgc agctggtgtt 600
gctggtgcag cagaaacatc caaacgtcat cataataaaa aagataaaca agattctaaa 660
cactcaaacc atgagaatga cgaaaaatct gttaaaaatg atgaccaaaa gcaatctaaa 720
aaaggcaaaa aagcagcagt cggtgctggc gcagctgcag gagttggtgc ggctggtgtt 780
gcgcatcata ataatcaaaa taaacatcat aatgaggaaa aaaattctaa tcaaaacaat 840
cagtacaatg accaatcaga aggtaagaaa aaaggtggtt tcatgaaaat cttgttacca 900
cttatagcag ccattcttat tctaggtgca atagcaatat tcggtggtat ggctctaaat 960
aatcacaacg atagtaaaag tgatgaccaa aaaatagcga atcaaagtaa gaaagactca 1020
gataaaaaag atggtgcgca atccgaagat aacaaagaca aaaaatctga tagtaacaaa 1080
gacaaaaaat ctgattctga taagaacgca gatgatgact ctgataatag ttcctcaaat 1140
cctaacgcta cttcaactaa taataacgat aatgtagcca ataataactc aaattataca 1200
aaccaaaatc aacaagataa tgcaaaccaa aatagcaata atcaacaggc aactcaaggt 1260
caacaatcac atacagtata cggtcaagaa aacttatatc gtatcgccat acaatattat 1320
ggagaaggaa ctcaagctaa cgtagataaa attaaacgtg cgaatggatt aagcagtaat 1380
aatattcata atggtcaaac attagttatt cctcaataa 1419

<210> 57
<211> 498
<212> DNA
<213> Staphylococcus aureus

<400> 57
atgaaaaata aattgatagc aaaatcttta ttaacaatag cggcaattgg tattactaca 60
actacaattg cgtcaacagc agatgcgagc gaaggatacg gtccaagaga aaagaaacca 120
gtgagtatta atcacaatat cgtagagtac aatgatggta cttttaaata tcaatctaga 180
ccaaaattta actcaacacc taaatatatt aaattcaaac atgactataa tattttagaa 240
tttaacgatg gtacattcga atatggtgca cgtccacaat ttaataaacc agcagcgaaa 300
actgatgcaa ctattaaaaa agaacaaaaa ttgattcaag ctcaaaatct tgtgagagaa 360
tttgaaaaaa cacatactgt cagtgcacac agaaaagcac aaaaggcagt caacttagtt 420
tcgtttgaat acaaagtgaa gaaaatggtc ttacaagagc gaattgataa tgtattaaaa 480
caaggattag tgagataa 498

<210> 58
<211> 960
<212> DNA
<213> Staphylococcus aureus

<400> 58
atgaaaacac gtatagtcag ctcagtaaca acaacactat tgctaggttc catattaatg 60
aatcctgtcg ctaatgccgc agattctgat attaatatta aaaccggtac tacagatatt 120
ggaagcaata ctacagtaaa aacaggtgat ttagtcactt atgataaaga aaatggcatg 180
cacaaaaaag tattttatag ttttatcgat gataaaaatc acaataaaaa actgctagtt 240
attagaacga aaggtaccat tgctggtcaa tatagagttt atagcgaaga aggtgctaac 300
aaaagtggtt tagcctggcc ttcagccttt aaggtacagt tgcaactacc tgataatgaa 360
gtagctcaaa tatctgatta ctatccaaga aattcgattg atacaaaaga gtatatgagt 420
actttaactt atggattcaa cggtaatgtt actggtgatg atacaggaaa aattggcggc 480
cttattggtg caaatgtttc gattggtcat acactgaaat atgttcaacc tgatttcaaa 540
acaattttag agagcccaac tgataaaaaa gtaggctgga aagtgatatt taacaatatg 600
gtgaatcaaa attggggacc atatgataga gattcttgga acccggtata tggcaatcaa 660
cttttcatga aaactagaaa tggttctatg aaagcagcag agaacttcct tgatcctaac 720
aaagcaagtt ctctattatc ttcagggttt tcaccagact tcgctacagt tattactatg 780
gatagaaaag catccaaaca acaaacaaat atagatgtaa tatacgaacg agttcgtgat 840
gactaccaat tgcattggac ttcaacaaat tggaaaggta ccaatactaa agataaatgg 900
acagatcgtt cttcagaaag atataaaatc gattgggaaa aagaagaaat gacaaattaa 960

<210> 59
<211> 1287
<212> DNA
<213> Staphylococcus aureus

<400> 59
atacacatga aaaataaata tatctcgaag ttgctagttg gggcagcaac aattacttta 60
gctacaatga tttcaaatgg ggaagcaaaa gcgagtgaaa acacgcaaca aacttcaact 120
aagcaccaaa caactcaaaa caactacgta acagatcaac aaaaagcttt ttatcaagta 180
ttacatctaa aaggtatcac agaagaacaa cgtaaccaat acatcaaaac attacgcgaa 240
cacccagaac gtgcacaaga agtattctct gaatcactta aagacagcaa gaacccagac 300
cgacgtgttg cacaacaaaa cgctttttac aatgttctta aaaatgataa cttaactgaa 360
caagaaaaaa ataattacat tgcacaaatt aaagaaaacc ctgatagaag ccaacaagtt 420
tgggtagaat cagtacaatc ttctaaagct aaagaacgtc aaaatattga aaatgcggat 480
aaagcaatta aagatttcca agataacaaa gcaccacacg ataaatcagc agcatatgaa 540
gctaactcaa aattacctaa agatttacgc gataaaaata accgctttgt agaaaaagtt 600
tcaattgaaa aagcaatcgt tcgtcatgat gagcgtgtga aatcagcaaa tgatgcaatc 660
tcaaaattaa atgaaaaaga ttcaattgaa aacagacgtt tagcacaacg tgaagttaac 720
aaagcaccta tggatgtaaa agagcattta cagaaacaat tagacgcatt agtagctcaa 780
aaagatgctg aaaagaaagt ggcgccaaaa gttgaggctc ctcaaattca atcaccacaa 840
attgaaaaac ctaaagcaga atcaccaaaa gttgaagtcc ctcaatctaa attattaggt 900
tactaccaat cattaaaaga ttcatttaac tatggttaca agtatttaac agatacttat 960
aaaagctata aagaaaaata tgatacagca aagtactact ataatacgta ctataaatac 1020
aaaggtgcga ttgatcaaac agtattaaca gtactaggta gtggttctaa atcttacatc 1080
caaccattga aagttgatga taaaaacggc tacttagcta aatcatatgc acaagtaaga 1140
aactatgtaa ctgagtcaat caatactggt aaagtattat atactttcta ccaaaaccca 1200
acattagtaa aaacagctat taaagctcaa gaaactgcat catcaatcaa aaatacatta 1260
agtaatttat tatcattctg gaaataa 1287

<210> 60
<211> 1053
<212> DNA
<213> Staphylococcus aureus

<400> 60
atgacaaaac attatttaaa cagtaagtat caatcagaac aacgttcatc agctatgaaa 60
aagattacaa tgggtacagc atctatcatt ttaggttccc ttgtatacat aggcgcagac 120
agccaacaag tcaatgcggc aacagaagct acgaacgcaa ctaataatca aagcacacaa 180
gtttctcaag caacatcaca accaattaat ttccaagtgc aaaaagatgg ctcttcagag 240
aagtcacaca tggatgacta tatgcaacac cctggtaaag taattaaaca aaataataaa 300
tattatttcc aaaccgtgtt aaacaatgca tcattctgga aagaatacaa attttacaat 360
gcaaacaatc aagaattagc aacaactgtt gttaacgata ataaaaaagc ggatactaga 420
acaatcaatg ttgcagttga acctggatat aagagcttaa ctactaaagt acatattgtc 480
gtgccacaaa ttaattacaa tcatagatat actacgcatt tggaatttga aaaagcaatt 540
cctacattag ctgacgcagc aaaaccaaac aatgttaaac cggttcaacc aaaaccagct 600
caacctaaaa cacctactga gcaaactaaa ccagttcaac ctaaagttga aaaagttaaa 660

cctactgtaa ctacaacaag caaagttgaa gacaatcact ctactaaagt tgtaagtact 720
gacacaacaa aagatcaaac taaaacacaa actgctcata cagttaaaac agcacaaact 780
gctcaagaac aaaataaagt tcaaacacct gttaaagatg ttgcaacagc gaaatctgaa 840
agcaacaatc aagctgtaag tgataataaa tcacaacaaa ctaacaaagt tacaaaacat 900
aacgaaacgc ctaaacaagc atctaaagct aaagaattac caaaaactgg tttaacttca 960
gttgataact ttattagcac agttgccttc gcaacacttg cccttttagg ttcattatct 1020
ttattacttt tcaaaagaaa agaatctaaa taa 1053

<210> 61
<211> 1938
<212> DNA
<213> Staphylococcus aureus

<400> 61
atgaacaaac agcaaaaaga atttaaatca ttttattcaa ttagaaagtc atcactaggc 60
gttgcatctg tagcgattag tacactttta ttattaatgt caaatggcga agcacaagca 120
gcagctgaag aaacaggtgg tacaaataca gaagcacaac caaaaactga agcagttgca 180
agtccaacaa caacatctga aaaagctcca gaaactaaac cagtagctaa tgctgtctca 240
gtatctaata aagaagttga ggcccctact tctgaaacaa aagaagctaa agaagttaaa 300
gaagttaaag cccctaagga aacaaaagca gttaaaccag cagcaaaagc cactaacaat 360
acatatccta ttttgaatca ggaacttaga gaagcgatta aaaaccctgc aataaaagat 420
aaagatcata gcgcaccaaa ctctcgtcca attgattttg aaatgaaaaa agaaaatggt 480
gagcaacaat tttatcatta tgccagctct gttaaacctg ctagagttat tttcactgat 540
tcaaaaccag aaattgaatt aggattacaa tcaggtcaat tttggagaaa atttgaagtt 600
tatgaaggtg acaaaaagtt gccaattaaa ttagtatcat acgatactgt taaagattac 660
gcttacattc gcttctctgt ttcaaatgga acaaaagccg ttaaaattgt aagttcaact 720
cacttcaata acaaagaaga aaaatacgat tacacattaa tggaattcgc acaaccaatt 780
tataacagtg cagataaatt caaaactgaa gaagattata aagctgaaaa attattagcg 840
ccatataaaa aagcgaaaac actagaaaga caagtttatg aattaaataa aattcaagat 900
aaacttcctg aaaaattaaa ggctgagtac aagaagaaat tagaggatac aaagaaagct 960
ttagatgagc aagtgaaatc agctattact gaattccaaa atgtacaacc aacaaatgaa 1020
aaaatgactg atttacaaga tacaaaatat gttgtttatg aaagtgttga gaataacgaa 1080
tctatgatgg atacttttgt taaacaccct attaaaacag gtatgcttaa cggcaaaaaa 1140
tatatggtca tggaaactac taatgacgat tactggaaag atttcatggt tgaaggtcaa 1200
cgtgttagaa ctataagcaa agatgctaaa aataatacta gaacaattat tttcccatat 1260
gttgaaggta aaactctata tgatgctatc gttaaagttc acgtaaaaac gattgattat 1320
gatggacaat accatgtcag aatcgttgat aaagaagcat ttacaaaagc caataccgat 1380
aaatctaaca aaaaagaaca acaagataac tcagctaaga aggaagctac tccagctacg 1440
cctagcaaac caacaccatc acctgttgaa aaagaatcac aaaaacaaga cagccaaaaa 1500
gatgacaata aacaattacc aagtgttgaa aaagaaaatg acgcatctag tgagtcaggt 1560
aaagacaaaa cgcctgctac aaaaccaact aaaggtgaag tagaatcaag tagtacaact 1620
ccaactaagg tagtatctac gactcaaaat gttgcaaaac caacaactgc ttcatcaaaa 1680
acaacaaaag atgttgttca aacttcagca ggttctagcg aagcaaaaga tagtgctcca 1740
ttacaaaaag caaacattaa aaacacaaat gatggacaca ctcaaagcca aaacaataaa 1800
aatacacaag aaaataaagc aaaatcatta ccacaaactg gtgaagaatc aaataaagat 1860
atgacattac cattaatggc attactagct ttaagtagca tcgttgcatt cgtattacct 1920
agaaaacgta aaaactaa 1938

<210> 62
<211> 2862
<212> DNA
<213> Staphylococcus aureus

<400> 62
atgaataata aaaagacagc aacaaataga aaaggcatga taccaaatcg attaaacaaa 60
ttttcgataa gaaagtattc tgtaggtact gcttcaattt tagtagggac aacattgatt 120
tttgggttaa gtggtcatga agctaaagcg gcagaacata cgaatggaga attaaatcaa 180
tcaaaaaatg aaacgacagc cccaagtgag aataaaacaa ctgaaaaagt tgatagtcgt 240
caactaaaag acaatacgca aactgcaact gcagatcagc ctaaagtgac aatgagtgat 300
agtgcaacag ttaaagaaac tagtagtaac atgcaatcac cacaaaacgc tacagctagt 360
caatctacta cacaaactag caatgtaaca acaaatgata aatcatcaac tacatatagt 420
aatgaaactg ataaaagtaa tttaacacaa gcaaaaaacg tttcaactac acctaaaaca 480
acgactatta aacaaagagc tttaaatcgc atggcagtga atactgttgc agctccacaa 540
caaggaacaa atgttaatga taaagtacat tttacgaaca ttgatattgc gattgataaa 600
ggacatgtta ataaaacaac aggaaatact gaattttggg caacttcaag tgatgtttta 660

aaattaaaag cgaattacac aatcgatgat tctgttaaag agggcgatac atttactttt 720
aaatatggtc aatatttccg tccaggttct gtaagattac cttcacaaac tcaaaattta 780
tataatgccc aaggtaatat tattgcaaaa ggtatttacg atagtaaaac aaatacaaca 840
acgtatactt ttacgaatta tgtagatcaa tacacaaatg ttagcggtag ctttgaacaa 900
gtcgcatttg cgaaacgtga aaatgcaaca actgataaaa ctgcttataa aatggaagta 960
actttaggta atgatacata tagtaaagat gtcattgtcg attatggtaa tcaaaaaggt 1020
caacaactta tttcgagtac aaattatatt aataatgaag atttgtcacg taatatgact 1080
gtttatgtaa atcaacctaa aaagacctat acaaaagaaa catttgtaac aaatttaact 1140
ggttataaat ttaatccaga tgctaaaaac ttcaaaattt acgaagtgac agatcaaaat 1200
caatttgtgg atagtttcac cccagatact tcaaaactta aagatgttac tggtcaattc 1260
gatgttattt atagtaatga taataagacg gcgacagtag atttattgaa tggtcaatct 1320

agtagtgata aacagtacat cattcaacaa gttgcttatc cagataatag ttcaacagat 1380
aatgggaaaa ttgattatac tttagaaaca caaaatggaa aaagtagttg gtcaaacagt 1440
tattcaaatg tgaatggctc atcaactgca aatggcgacc aaaagaaata taatctaggt 1500
gactatgtat gggaagatac aaataaagat ggtaaacaag atgccaatga aaaagggatt 1560
aaaggtgttt atgtcattct taaagatagt aacggtaaag aattagatcg tacgacaaca 1620
gatgaaaatg gtaaatatca gttcactggt ttaagcaatg gaacttatag tgtagagttt 1680
tcaacaccag ccggttatac accgacaact gcaaatgcag gtacagatga tgctgtagat 1740
tctgatggac taactacaac aggtgtcatt aaagacgctg acaacatgac attagatagt 1800
ggattctaca aaacaccaaa atatagttta ggtgattatg tttggtacga cagtaataaa 1860
gatggtaaac aagattcgac tgaaaaagga attaaaggtg ttaaagttac tttgcaaaac 1920
gaaaaaggcg aagtaattgg tacaactgaa acagatgaaa atggtaaata ccgctttgat 1980
aatttagata gtggtaaata caaagttatc tttgaaaagc ctgctggttt aactcaaaca 2040
ggtacaaata caactgaaga tgataaagat gccgatggtg gcgaagttga tgtaacaatt 2100
acggatcatg atgatttcac acttgataat ggctactacg aagaagaaac atcagatagt 2160
gactcagatt cggacagcga ttcagactca gatagcgact cagattcaga tagtgactca 2220
gactcagata gcgactcaga ctcagatagc gactcagaca gcgactcaga ctcagatagt 2280
gattcagatt cggacagcga ctcagattca gacagcgaat cagattcgga tagcgactca 2340
gactcagata gcgactcaga cagcgactca gattcagaca gtgactcaga ctcagacagc 2400
gactcagatt cagacagcga ttcagattcg gatagcgact cagattcaga tagcgattcg 2460
gactcagaca acgactcaga ttctgacagc gattcagact cagatagcga ctcagattca 2520
gacagcgact cagattcaga cagcgattca gattcagata gcgattcaga ttcagacagc 2580
gactcagatt cagatagcga ctcagactca gacagcgatt cagactcaga tagcgactca 2640
gacagcgatt cagattcgga tagcgattca gattcagatg caggtaaaca tactccgact 2700
aaaccaatga gtacggttaa agatcagcat aaaacagcta aagcattacc agaaacaggt 2760
agtgaaaata ataattcaaa taatggcaca ttattcggtg gattattcgc ggcattagga 2820
tcattattgt tattcggtcg tcgtaaaaaa caaaataaat aa 2862

<210> 63
<211> 2808
<212> DNA
<213> Staphylococcus aureus

<400> 63
atgaatatga agaaaaaaga aaaacacgca attcggaaaa aatcgattgg cgtggcttca 60
gtgcttgtag gtacgttaat cggttttgga ctactcagca gtaaagaagc agatgcaagt 120
gaaaatagtg ttacgcaatc tgatagcgca agtaacgaaa gcaaaagtaa tgattcaagt 180
agcgttagtg ctgcacctaa aacagacgac acaaacgtga gtgatactaa aacatcgtca 240
aacactaata atggcgaaac gagtgtggcg caaaatccag cacaacagga aacgacacaa 300
tcatcatcaa caaatgcaac tacggaagaa acgccggtaa ctggtgaagc tactactacg 360
acaacgaatc aagctaatac accggcaaca actcaatcaa gcaatacaaa tgcggaggaa 420
ttagtgaatc aaacaagtaa tgaaacgact tctaatgata ctaatacagt atcatctgta 480
aattcacctc aaaattctac aaatgcggaa aatgtttcaa caacgcaaga tacttcaact 540
gaagcaacac cttcaaacaa tgaatcagct ccacagaata cagatgcaag taataaagat 600
gtagttagtc aagcggttaa tccaagtacg cctagaatga gagcatttag tttagcggca 660
gtagctgcag atgcaccggc agctggcaca gatattacga atcagttgac agatgtgaaa 720
gttactattg actctggtac gactgtgtat ccgcaccaag caggttatgt caaactgaat 780
tatggttttt cagtgcctaa ttctgctgtt aaaggtgaca cattcaaaat aactgtacct 840
aaagaattaa acttaaatgg tgtaacttca actgctaaag tgccaccaat tatggctgga 900
gatcaagtat tggcaaatgg tgtaatcgat agtgatggta atgttattta tacatttaca 960
gactatgttg ataataaaga aaatgtaaca gctaatatta ctatgccagc ttatattgac 1020
cctgaaaatg ttacaaagac aggtaatgtg acattgacaa ctggcatagg aaccaatact 1080
gctagtaaga cagtattaat cgactatgag aaatatggac aattccataa tttatcaatt 1140
aaaggtacga ttgatcaaat cgataaaaca aataatacgt atcgccaaac aatttatgtc 1200

aatccaagcg gagataacgt tgtgttacct gccttaacag gtaatttaat tcctaataca 1260
aagagtaatg cgttaataga tgcaaaaaac actgatatta aagtttatag agtcgataat 1320
gctaatgatt tatctgaaag ttattatgtg aatcctagcg attttgaaga tgtaactaat 1380
caagttagaa tttcatttcc aaatgctaat caatacaaag tagaatttcc tacggacgat 1440
gaccaaatta caacaccgta tattgtagtt gttaatggcc atattgatcc tgctagtaca 1500
ggtgatttag cactacgttc gacattttat ggttatgatt ctaattttat atggagatct 1560
atgtcatggg acaacgaagt agcatttaat aacggatcag gttctggtga cggtatcgat 1620
aaaccagttg ttcctgaaca acctgatgag cctggtgaaa ttgaaccaat tccagaggat 1680
tcagattctg acccaggttc agattctggc agcgattcta attcagatag cggttcagat 1740
tctggcagtg attctacatc agatagtggt tcagattcag cgagtgattc agattcagca 1800
agtgattcag actcagcgag tgattcagat tcagcaagtg attcagattc agcaagtgat 1860
tcagattcag caagtgattc agactcagca agtgattcag attcagcaag tgattcagat 1920
tcagcaagcg attcagattc agcgagcgat tcagattcag cgagcgattc agattcagcg 1980
agtgattccg actcagcgag cgattcagac tcagatagtg actcagattc cgatagcgat 2040
tccgactcag atagcgactc agattcagac agcgattctg actcagacag cgattctgac 2100
tcagacagtg actcagattc cgatagcgat tctgactcag acagtgactc agattccgat 2160
agcgattcag attcagacag tgattcagac tcagatagcg attcagattc cgacagtgac 2220
tcagactcag acagcgattc agattccgat agcgattcag attccgacag tgactcagat 2280
tccgatagtg actcggattc agcgagtgat tcagattcag atagcgattc agaatcagat 2340
agtgactcag actcagacag tgattcagat tcagatagtg actcagactc agacagcgat 2400
tcagaatcag atagtgactc cgattcagac agcgattcag aatcagatag tgactccgat 2460
tcagatagcg attcggattc agcgagtgat tcagactcag gtagtgactc cgattcatca 2520
agtgattcag attccgattc aacgagtgac acaggatcag acaacgactc agacagtgat 2580
tcaaatagcg attccgagtc aggttctaac aataatgtag ttccgcctaa ttcacctaaa 2640
aatggtacta atgcttctaa taaaaatgag gctaaagata gtaaagaacc attaccagat 2700
acaggttctg aagatgaagc gaatacgtca ctaatttggg gattattagc atcattaggt 2760
tcattactac ttttcagaag aaaaaaagaa aataaagata agaaataa 2808

<210> 64
<211> 3117
<212> DNA
<213> Staphylococcus aureus

<400> 64
gtgaaaaaca atcttaggta cggcattaga aaacataaat tgggagcagc atcagtattc 60
ttaggaacaa tgatcgttgt tgggatggga caagataaag aagctgcagc atcagaacaa 120
aagacaacta cagtagaaga aaatgggaat tcagctactg ataataaaac aagtgaaaca 180
caaacaactg ctactaacgt taatcatata gaagaaactc aatcatataa cgcaacagta 240
acagaacaac cgtcaaacgc aacacaagta acaactgaag aagcaccaaa agcagtacaa 300
gcaccacaaa ctgcacaacc agcaaatgta gaaacagtta aagaagaaga gaaacctcaa 360
gttaaggaaa cgacacaacc tcaagacaat agcggaaatc aaagacaagt agatttaaca 420
cctaaaaagg ttacacaaaa tcaaggacca gaaacacaag ttgaagtggc acagccaaga 480
acggcatcag aaagtaagcc acgtgtgaca agatcagcag atgtagcgga agctaaggaa 540
gctagtgacg tttcagaagt taaaggcaca gatgttacaa gtaaagttac agtagaaagt 600
ggttctattg aggcacctca aggaaataaa gtagagccac atgctggtca acgtgtcgta 660
ttgaaataca aattgaaatt cgcagatgga ttaaaaagag gagattattt tgattttaca 720
ttatcaaata atgtaaatac ttatggggtt tcaacagcta gaaaggtacc agagattaaa 780
aatggctcag ttgtaatggc tacaggtgag atcttaggga atggtaacat aagatataca 840
tttactaacg aaattgaaca caaggtagag gtaacagcta atttagaaat caacttattt 900
attgacccta aaactgtaca aagcaatgga gaacaaaaga ttacttctaa attaaatggt 960
gaagaaacag aaaaaacaat accagttgtt tataatccag gtgttagcaa tagttataca 1020
aatgtaaatg gatcaattga aacatttaat aaagaatcta ataaatttac acatatagct 1080

tatattaagc caatgaatgg aaaccagtca aacactgtat cagtaacagg gacgttgact 1140
gaaggtagta atttagctgg tggacaacct actgttaaag tatatgaata tctagggaaa 1200
aaagatgaat tgccacaaag tgtttatgca aatacatcag atactaacaa attcaaagat 1260
gtaacaaagg aaatgaatgg aaaattgagt gtgcaagaca atggtagtta ctcattgaat 1320
ttagataagt tggataaaac gtatgtcatt cattatacag gtgaatattt gcaagggtca 1380
gatcaggtta attttagaac tgaattatat gggtatccag aacgagcata taaatcttac 1440
tatgtttatg ggggatatcg tttaacttgg gataatggtt tagttttata tagcaataaa 1500
gctgacggca atggtaaaaa tggacaaatt attcaagata atgattttga atataaagaa 1560
gatactgcaa aaggaactat gagcgggcag tacgatgcca agcaaattat tgaaacagaa 1620
gaaaatcaag acaatacacc gcttgacatt gattaccaca cagctataga tggtgagggt 1680
ggttatgttg atgggtatat tgaaacaata gaagaaacgg attcatcagc tattgatatc 1740
gattaccata ctgctgtgga tagtgaagtg ggtcacgttg gaggatacac tgagtcctct 1800

gaggaatcaa atccaattga ctttgaagaa tcgacacatg aaaattcaaa acatcacgct 1860
gatgttgttg aatatgaaga ggatacaaat ccaggtggtg gccaagtaac aactgagtct 1920
aacttagttg aatttgacga agagtctaca aaaggtattg taactggcgc agtgagcgac 1980
catacaacaa ttgaagatac gaaagaatat acgactgaaa gtaatctgat tgaactagta 2040
gatgaactac ctgaagaaca tggtcaagca caaggaccaa tcgaggaaat tactgaaaac 2100
aatcatcata tttctcattc tggtttagga actgaaaatg gtcacggtaa ttatggcgtg 2160
attgaagaaa tcgaagaaaa tagccacgtt gatattaaga gtgaattagg ttacgaaggt 2220
ggccaaaata gcggtaacca gtcattcgag gaagacacag aagaagacaa acctaaatat 2280
gaacaaggtg gcaatatcgt agatatcgat ttcgacagtg tacctcaaat tcatggtcaa 2340
aataaaggtg accagtcatt cgaagaagat acagagaaag acaagcctaa atatgaacat 2400
ggcggtaata tcattgatat cgacttcgac agtgtgccac aaattcatgg attcaataag 2460
cataatgaaa ttattgaaga agatacaaac aaagataaac ctaattatca attcggtgga 2520
cacaatagtg ttgactttga agaagataca cttccaaaag taagcggcca aaatgaaggt 2580
caacaaacga ttgaagaaga tacaacgccg ccaacgccac cgacaccaga agtaccgagt 2640
gagccggaaa caccaatgcc accgacacca gaagtaccga gtgagccgga aacaccaacg 2700
ccaccaacac cagaggtacc aagtgagccg gaaacaccaa caccaccgac tccggaagta 2760
ccaagtgagc cggaaacacc aacaccaccg acaccagaag tgccgagtga gccagaaaca 2820
ccaacaccgc caacaccaga ggtaccagct gaacctggta aaccagtacc acccgcaaaa 2880
gaagaaccta aaaagccttc taaaccagtg gaacaaggta aagtagtaac acctgttatt 2940
gaaatcaatg aaaaggttaa agcagtggca ccaactaaaa aagcacaatc taagaaatct 3000

gaactacctg aaacaggtgg agaagaatca acaaacaaag gtatgttgtt cggcggatta 3060
ttcagcattc taggtttagc attattacgc agaaataaaa agaataacaa agcataa 3117

<210> 65
<211> 2634
<212> DNA
<213> Staphylococcus aureus

<400> 65
ttgaaaaaaa gaattgatta tttgtcgaat aagcagaata agtattcgat tagacgtttt 60
acagtaggta ccacatcagt aatagtaggg gcaactatac tatttgggat aggcaatcat 120
caagcacaag cttcagaaca atcgaacgat acaacgcaat cttcgaaaaa taatgcaagt 180
gcagattccg aaaaaaacaa tatgatagaa acacctcaat taaatacaac ggctaatgat 240
acatctgata ttagtgcaaa cacaaacagt gcgaatgtag atagcacaac aaaaccaatg 300
tctacacaaa cgagcaatac cactacaaca gagccagctt caacaaatga aacacctcaa 360
ccgacggcaa ttaaaaatca agcaactgct gcaaaaatgc aagatcaaac tgttcctcaa 420
gaagcaaatt ctcaagtaga taataaaaca acgaatgatg ctaatagcat agcaacaaac 480
agtgagctta aaaattctca aacattagat ttaccacaat catcaccaca aacgatttcc 540
aatgcgcaag gaactagtaa accaagtgtt agaacgagag ctgtacgtag tttagctgtt 600
gctgaaccgg tagtaaatgc tgctgatgct aaaggtacaa atgtaaatga taaagttacg 660
gcaagtaatt tcaagttaga aaagactaca tttgacccta atcaaagtgg taacacattt 720
atggcggcaa attttacagt gacagataaa gtgaaatcag gggattattt tacagcgaag 780
ttaccagata gtttaactgg taatggagac gtggattatt ctaattcaaa taatacgatg 840
ccaattgcag acattaaaag tacgaatggc gatgttgtag ctaaagcaac atatgatatc 900
ttgactaaga cgtatacatt tgtctttaca gattatgtaa ataataaaga aaatattaac 960
ggacaatttt cattaccttt atttacagac cgagcaaagg cacctaaatc aggaacatat 1020
gatgcgaata ttaatattgc ggatgaaatg tttaataata aaattactta taactatagt 1080
tcgccaattg caggaattga taaaccaaat ggcgcgaaca tttcttctca aattattggt 1140
gtagatacag cttcaggtca aaacacatac aagcaaacag tatttgttaa ccctaagcaa 1200
cgagttttag gtaatacgtg ggtgtatatt aaaggctacc aagataaaat cgaagaaagt 1260
agcggtaaag taagtgctac agatacaaaa ctgagaattt ttgaagtgaa tgatacatct 1320
aaattatcag atagctacta tgcagatcca aatgactcta accttaaaga agtaacagac 1380
caatttaaaa atagaatcta ttatgagcat ccaaatgtag ctagtattaa atttggtgat 1440
attactaaaa catatgtagt attagtagaa gggcattacg acaatacagg taagaactta 1500
aaaactcagg ttattcaaga aaatgttgat cctgtaacaa atagagacta cagtattttc 1560
ggttggaata atgagaatgt tgtacgttat ggtggtggaa gtgctgatgg tgattcagca 1620
gtaaatccga aagacccaac tccagggccg ccggttgacc cagaaccaag tccagaccca 1680
gaaccagaac caacgccaga tccagaacca agtccagacc cagaaccgga accaagccca 1740
gacccggatc cggattcgga ttcagacagt gactcaggct cagacagcga ctcaggttca 1800
gatagcgact cagaatcaga tagcgattcg gattcagaca gtgattcaga ttcagacagc 1860
gactcagaat cagatagcga ttcagaatca gatagcgact cagattcaga tagcgattca 1920
gattcagata gcgattcaga atcagatagc gattcggatt cagacagtga ttcagattca 1980
gacagcgact cagaatcaga tagcgactca gaatcagata gtgagtcaga ttcagacagt 2040
gactcggact cagacagtga ttcagactca gatagcgatt cagactcaga tagcgattca 2100

gactcagaca gcgattcaga ttcagacagc gactcagaat cagacagcga ctcagactca 2160
gatagcgact cagactcaga cagcgactca gattcagata gcgattcaga ctcagacagc 2220
gactcagact cagacagcga ctcagactca gatagcgatt cagactcaga cagcgactca 2280
gattcagata gcgattcgga ctcagacagc gattcagatt cagacagcga ctcagactcg 2340
gatagcgatt cagattcaga cagcgactca gactcggata gcgactcgga ttcagatagt 2400
gactccgatt caagagttac accaccaaat aatgaacaga aagcaccatc aaatcctaaa 2460
ggtgaagtaa accattctaa taaggtatca aaacaacaca aaactgatgc tttaccagaa 2520
acaggagata agagcgaaaa cacaaatgca actttatttg gtgcaatgat ggcattatta 2580
ggatcattac tattgtttag aaaacgcaag caagatcata aagaaaaagc gtaa 2634

<210> 66
<211> 1977
<212> DNA
<213> Staphylococcus aureus

<400> 66
atgaaaaagc aaataatttc gctaggcgca ttagcagttg catctagctt atttacatgg 60
gataacaaag cagatgcgat agtaacaaag gattatagta aagaatcaag agtgaatgag 120
aaaagtaaaa agggagctac tgtttcagat tattactatt ggaaaataat tgatagttta 180
gaggcacaat ttactggagc aatagactta ttggaagatt ataaatatgg agatcctatc 240
tataaagaag cgaaagatag attgatgaca agagtattag gagaagacca gtatttatta 300
aagaaaaaga ttgatgaata tgagctttat aaaaagtggt ataaaagttc aaataagaac 360
actaatatgc ttactttcca taaatataat ctttacaatt taacaatgaa tgaatataac 420
gatattttta actctttgaa agatgcagtt tatcaattta ataaagaagt taaagaaata 480
gagcataaaa atgttgactt gaagcagttt gataaagatg gagaagacaa ggcaactaaa 540
gaagtttatg accttgtttc tgaaattgat acattagttg taacttatta tgctgataag 600
gattatgggg agcatgcgaa agagttacga gcaaaactgg acttaatcct tggagataca 660
gacaatccac ataaaattac aaatgagcgt ataaaaaaag aaatgatcga tgacttaaat 720
tcaattatag atgatttctt tatggagact aaacaaaata gaccgaattc tataacaaaa 780
tatgatccaa caaaacacaa ttttaaagag aagagtgaaa ataaacctaa ttttgataaa 840
ttagttgaag aaacaaaaaa agcagttaaa gaagcagacg aatcttggaa aaataaaact 900
gtcaaaaaat acgaggaaac tgtaacaaaa tctcctgttg taaaagaaga gaagaaagtt 960
gaagaacctc aattacctaa agttggaaac cagcaagagg ttaaaactac ggctggtaaa 1020
gctgaagaaa caacacaacc agtggcacag ccattagtaa aaattccaca agaaacaatc 1080
tatggtgaaa ctgtaaaagg tccagaatat ccaacgatgg aaaataaaac gttacaaggt 1140
gaaatcgttc aaggtcccga ttttctaaca atggaacaaa acagaccatc tttaagcgat 1200
aattatactc aaccgacgac accgaaccct attttagaag gtcttgaagg tagctcatct 1260
aaacttgaaa taaaaccaca aggtactgaa tcaacgttga aaggtattca aggagaatca 1320
agtgatattg aagttaaacc tcaagcaact gaaacaacag aagcttctca atatggtccg 1380
agaccgcaat ttaacaaaac acctaagtat gtgaaatata gagatgctgg tacaggtatc 1440
cgtgaataca acgatggaac atttggatat gaagcgagac caagattcaa caagccaagt 1500
gaaacaaatg catacaacgt aacgacaaat caagatggca cagtatcata cggagctcgc 1560
ccaacacaaa acaagccaag tgaaacaaac gcatataacg taacaacaca tgcaaatggt 1620
caagtatcat acggtgctcg cccaacacaa aaaaagccaa gcaaaacaaa tgcatacaac 1680
gtaacaacac atgcaaatgg tcaagtatca tatggcgctc gcccgacaca aaaaaagcca 1740
agcaaaacaa atgcatataa cgtaacaaca catgcaaatg gtcaagtatc atacggagct 1800
cgcccgacat acaagaagcc aagcgaaaca aatgcataca acgtaacaac acatgcaaat 1860
ggtcaagtat catatggcgc tcgcccgaca caaaaaaagc caagcgaaac aaacgcatat 1920
aacgtaacaa cacatgcaga tggtactgcg acatatgggc ctagagtaac aaaataa 1977

<210> 67
<211> 961
<212> PRT
<213> Staphylococcus aureus

<400> 67
Met Lys Ser Asn Leu Arg Tyr Gly Ile Arg Lys His Lys Leu Gly Ala
1               5                   10                  15
Ala Ser Val Phe Leu Gly Thr Met Ile Val Val Gly Met Gly Gln Glu
            20                  25                  30
Lys Glu Ala Ala Ala Ser Glu Gln Asn Asn Thr Thr Val Glu Glu Ser
        35                  40                  45
Gly Ser Ser Ala Thr Glu Ser Lys Ala Ser Glu Thr Gln Thr Thr Thr
    50                  55                  60
Asn Asn Val Asn Thr Ile Asp Glu Thr Gln Ser Tyr Ser Ala Thr Ser

65                  70                  75                  80
Thr Glu Gln Pro Ser Lys Ser Thr Gln Val Thr Thr Glu Glu Ala Pro
                85                  90                  95
Thr Thr Val Gln Ala Pro Lys Val Glu Thr Glu Met Lys Ser Gln Glu
            100                 105                 110
Asp Leu Pro Ser Glu Lys Val Ala Asp Lys Glu Thr Thr Gly Thr Gln
        115                 120                 125
Val Asp Ile Ala Gln Pro Ser Asn Val Ser Glu Ile Lys Pro Arg Met
    130                 135                 140
Lys Arg Ser Ala Asp Val Thr Ala Val Ser Glu Lys Glu Val Ala Glu
145                 150                 155                 160
Glu Ala Lys Ala Thr Gly Thr Asp Val Thr Asn Lys Val Glu Val Thr
                165                 170                 175
Glu Ser Ser Leu Glu Gly His Asn Lys Asp Ser Asn Ile Val Asn Pro
            180                 185                 190
His Asn Ala Gln Arg Val Thr Leu Lys Tyr Lys Trp Lys Phe Gly Glu
        195                 200                 205
Gly Ile Lys Ala Gly Asp Tyr Phe Asp Phe Thr Leu Ser Asp Asn Val
    210                 215                 220
Glu Thr His Gly Ile Ser Thr Leu Arg Lys Val Pro Glu Ile Lys Ser
225                 230                 235                 240
Ser Thr Glu Asp Lys Val Met Ala Asn Gly Gln Val Ile Asn Glu Arg
                245                 250                 255
Thr Ile Arg Tyr Thr Phe Thr Asp Tyr Ile Asn Asn Lys Lys Asp Leu
            260                 265                 270
Thr Ala Glu Leu Asn Leu Asn Leu Phe Ile Asp Pro Thr Thr Val Thr
        275                 280                 285
Lys Gln Gly Ser Gln Lys Val Glu Val Thr Leu Gly Gln Asn Lys Val
    290                 295                 300
Ser Lys Glu Phe Asp Ile Lys Tyr Leu Asp Gly Val Lys Asp Arg Met
305                 310                 315                 320
Gly Val Thr Val Asn Gly Arg Ile Asp Thr Leu Asn Lys Glu Glu Gly
                325                 330                 335
Lys Phe Ser His Phe Ala Tyr Val Lys Pro Asn Asn Gln Ser Leu Thr
            340                 345                 350
Ser Val Thr Val Thr Gly Gln Val Thr Ser Gly Tyr Lys Gln Ser Ala
        355                 360                 365
Asn Asn Pro Thr Val Lys Val Tyr Lys His Ile Gly Ser Asp Glu Leu
    370                 375                 380
Ala Glu Ser Val Tyr Ala Lys Leu Asp Asp Thr Ser Lys Phe Glu Asp
385                 390                 395                 400
Val Thr Glu Lys Val Asn Leu Ser Tyr Thr Ser Asn Gly Gly Tyr Thr
                405                 410                 415
Leu Asn Leu Gly Asp Leu Asp Asn Ser Lys Asp Tyr Val Ile Lys Tyr
            420                 425                 430
Glu Gly Glu Tyr Asp Gln Asn Ala Lys Asp Leu Asn Phe Arg Thr His
        435                 440                 445
Leu Ser Gly Tyr His Lys Tyr Tyr Pro Tyr Tyr Pro Tyr Tyr Pro Tyr
    450                 455                 460
Tyr Pro Val Gln Leu Thr Trp Asn Asn Gly Val Ala Phe Tyr Ser Asn
465                 470                 475                 480
Asn Ala Lys Gly Asp Gly Lys Asp Lys Pro Asn Asp Pro Ile Ile Glu
                485                 490                 495
Lys Ser Glu Pro Ile Asp Leu Asp Ile Lys Ser Glu Pro Pro Val Glu
            500                 505                 510
Lys His Glu Leu Thr Gly Thr Ile Glu Glu Ser Asn Asp Ser Lys Pro
        515                 520                 525
Ile Asp Phe Glu Tyr His Thr Ala Val Glu Gly Ala Glu Gly His Ala
    530                 535                 540
Glu Gly Ile Ile Glu Thr Glu Glu Asp Ser Ile His Val Asp Phe Glu
545                 550                 555                 560
Glu Ser Thr His Glu Asn Ser Lys His His Ala Asp Val Val Glu Tyr
                565                 570                 575
Glu Glu Asp Thr Asn Pro Gly Gly Gly Gln Val Thr Thr Glu Ser Asn
            580                 585                 590

Leu Val Glu Phe Asp Glu Glu Ser Thr Lys Gly Ile Val Thr Gly Ala
        595                 600                 605
Val Ser Asp His Thr Thr Val Glu Asp Thr Lys Glu Tyr Thr Thr Glu
    610                 615                 620
Ser Asn Leu Ile Glu Leu Val Asp Glu Leu Pro Glu Glu His Gly Gln
625                 630                 635                 640
Ala Gln Gly Pro Ile Glu Glu Ile Thr Glu Asn Asn His His Ile Ser
                645                 650                 655
His Ser Gly Leu Gly Thr Glu Asn Gly His Gly Asn Tyr Gly Val Ile
            660                 665                 670
Asp Glu Ile Glu Glu Asn Ser His Val Asp Ile Lys Ser Glu Leu Gly
        675                 680                 685
Tyr Glu Gly Gly Gln Asn Ser Gly Asn Gln Ser Phe Glu Glu Asp Thr
    690                 695                 700
Glu Glu Asp Lys Pro Lys Tyr Glu Gln Gly Gly Asn Ile Val Asp Ile
705                 710                 715                 720
Asp Phe Asp Ser Val Pro Gln Ile His Gly Gln Asn Asn Gly Asn Gln
                725                 730                 735
Ser Phe Glu Glu Asp Thr Glu Glu Asp Lys Pro Lys Tyr Glu Gln Gly
            740                 745                 750
Gly Asn Ile Ile Asp Ile Asp Phe Asp Ser Val Pro Gln Ile His Gly
        755                 760                 765
Phe Asn Lys His Asn Glu Ile Ile Glu Glu Asp Thr Asn Lys Asp Lys
    770                 775                 780
Pro Asn Tyr Gln Phe Gly Gly His Asn Ser Val Asp Phe Glu Glu Asp
785                 790                 795                 800
Thr Leu Pro Lys Val Ser Gly Gln Asn Glu Gly Gln Gln Thr Ile Glu
                805                 810                 815
Glu Asp Thr Thr Pro Pro Thr Pro Pro Thr Pro Glu Val Pro Ser Glu
            820                 825                 830
Pro Glu Thr Pro Thr Pro Pro Thr Pro Glu Val Pro Ser Glu Pro Gly
        835                 840                 845
Glu Pro Thr Pro Pro Lys Pro Glu Val Pro Ser Glu Pro Glu Thr Pro
    850                 855                 860
Val Pro Pro Thr Pro Glu Val Pro Ser Glu Pro Gly Lys Pro Val Pro
865                 870                 875                 880
Pro Ala Lys Glu Glu Pro Lys Lys Pro Ser Lys Pro Val Glu Gln Gly
                885                 890                 895
Lys Val Val Thr Pro Val Ile Glu Ile Asn Glu Lys Val Lys Ala Val
            900                 905                 910
Ala Pro Thr Lys Gln Lys Gln Ser Lys Lys Ser Glu Leu Pro Glu Thr
        915                 920                 925
Gly Gly Glu Glu Ser Thr Asn Lys Gly Met Leu Phe Gly Gly Leu Phe
    930                 935                 940
Ser Ile Leu Gly Leu Val Leu Leu Arg Arg Asn Lys Lys Asn Asn Lys
945                 950                 955                 960
Ala

<210> 68
<211> 578
<212> PRT
<213> Staphylococcus aureus

<400> 68
Met Lys Phe Lys Ser Leu Ile Thr Thr Thr Leu Ala Leu Gly Val Ile
1               5                   10                  15
Ala Ser Thr Gly Ala Asn Phe Asn Thr Asn Glu Ala Ser Ala Ala Ala
            20                  25                  30
Lys Pro Leu Asp Lys Ser Ser Ser Thr Leu His His Gly His Ser Asn
        35                  40                  45
Ile Gln Ile Pro Tyr Thr Ile Thr Val Asn Gly Thr Ser Gln Asn Ile
    50                  55                  60
Leu Ser Ser Leu Thr Phe Asn Lys Asn Gln Asn Ile Ser Tyr Lys Asp

65                  70                  75                  80
Ile Glu Asn Lys Val Lys Ser Val Leu Tyr Phe Asn Arg Gly Ile Ser
                85                  90                  95
Asp Ile Asp Leu Arg Leu Ser Lys Gln Ala Glu Tyr Thr Val His Phe
            100                 105                 110
Lys Asn Gly Thr Lys Arg Val Ile Asp Leu Lys Ser Gly Ile Tyr Thr
        115                 120                 125
Ala Asp Leu Ile Asn Thr Ser Asp Ile Lys Ala Ile Ser Val Asn Val
    130                 135                 140
Asp Thr Lys Lys Gln Pro Lys Asp Lys Ala Lys Ala Asn Val Gln Val
145                 150                 155                 160
Pro Tyr Thr Ile Thr Val Asn Gly Thr Ser Gln Asn Ile Leu Ser Asn
                165                 170                 175
Leu Thr Phe Asn Lys Asn Gln Asn Ile Ser Tyr Lys Asp Leu Glu Gly
            180                 185                 190
Lys Val Lys Ser Val Leu Glu Ser Asn Arg Gly Ile Thr Asp Val Asp
        195                 200                 205
Leu Arg Leu Ser Lys Gln Ala Lys Tyr Thr Val Asn Phe Lys Asn Gly
    210                 215                 220
Thr Lys Lys Val Ile Asp Leu Lys Ser Gly Ile Tyr Thr Ala Asn Leu
225                 230                 235                 240
Ile Asn Ser Ser Asp Ile Lys Ser Ile Asn Ile Asn Val Asp Thr Lys
                245                 250                 255
Lys His Ile Glu Asn Lys Ala Lys Arg Asn Tyr Gln Val Pro Tyr Ser
            260                 265                 270
Ile Asn Leu Asn Gly Thr Ser Thr Asn Ile Leu Ser Asn Leu Ser Phe
        275                 280                 285
Ser Asn Lys Pro Trp Thr Asn Tyr Lys Asn Leu Thr Ser Gln Ile Lys
    290                 295                 300
Ser Val Leu Lys His Asp Arg Gly Ile Ser Glu Gln Asp Leu Lys Tyr
305                 310                 315                 320
Ala Lys Lys Ala Tyr Tyr Thr Val Tyr Phe Lys Asn Gly Gly Lys Arg
                325                 330                 335
Ile Leu Gln Leu Asn Ser Lys Asn Tyr Thr Ala Asn Leu Val His Ala
            340                 345                 350
Lys Asp Val Lys Arg Ile Glu Ile Thr Val Lys Thr Gly Thr Lys Ala
        355                 360                 365
Lys Ala Asp Arg Tyr Val Pro Tyr Thr Ile Ala Val Asn Gly Thr Ser
    370                 375                 380
Thr Pro Ile Leu Ser Lys Leu Lys Ile Ser Asn Lys Gln Leu Ile Ser
385                 390                 395                 400
Tyr Lys Tyr Leu Asn Asp Lys Val Lys Ser Val Leu Lys Ser Glu Arg
                405                 410                 415
Gly Ile Ser Asp Leu Asp Leu Lys Phe Ala Lys Gln Ala Lys Tyr Thr
            420                 425                 430
Val Tyr Phe Lys Asn Gly Lys Lys Gln Val Val Asn Leu Lys Ser Asp
        435                 440                 445
Ile Phe Thr Pro Asn Leu Phe Ser Ala Lys Asp Ile Lys Lys Ile Asp
    450                 455                 460
Ile Asp Val Lys Gln Tyr Thr Lys Ser Lys Lys Lys Ile Asn Lys Ser
465                 470                 475                 480
Asn Asn Val Lys Phe Pro Val Thr Ile Asn Lys Phe Glu Asn Ile Val
                485                 490                 495
Ser Asn Glu Phe Val Phe Tyr Asn Ala Ser Lys Ile Thr Ile Asn Asp
            500                 505                 510
Leu Ser Ile Lys Leu Lys Ser Ala Met Ala Asn Asp Gln Gly Ile Thr
        515                 520                 525
Lys His Asp Ile Gly Leu Ala Glu Arg Ala Val Tyr Lys Val Tyr Phe
    530                 535                 540
Lys Asn Gly Ser Ser Lys Tyr Val Asp Leu Lys Thr Glu Tyr Lys Asp
545                 550                 555                 560
Glu Arg Val Phe Lys Ala Thr Asp Ile Lys Lys Val Asp Ile Glu Leu
                565                 570                 575
Lys Phe

<210> 69
<211> 1642
<212> PRT
<213> Staphylococcus aureus

<400> 69
Met Asn Lys His His Pro Lys Leu Arg Ser Phe Tyr Ser Ile Arg Lys
1               5                   10                  15
Ser Thr Leu Gly Val Ala Ser Val Ile Val Ser Thr Leu Phe Leu Ile
            20                  25                  30
Thr Ser Gln His Gln Ala Gln Ala Ala Glu Asn Thr Asn Thr Ser Asp
        35                  40                  45
Lys Ile Ser Glu Asn Gln Asn Asn Asn Ala Thr Thr Thr Gln Pro Pro
    50                  55                  60
Lys Asp Thr Asn Gln Thr Gln Pro Ala Thr Gln Pro Ala Asn Thr Ala
65                  70                  75                  80
Lys Asn Tyr Pro Ala Ala Asp Glu Ser Leu Lys Asp Ala Ile Lys Asp
                85                  90                  95
Pro Ala Leu Glu Asn Lys Glu His Asp Ile Gly Pro Arg Glu Gln Val
            100                 105                 110
Asn Phe Gln Leu Leu Asp Lys Asn Asn Glu Thr Gln Tyr Tyr His Phe
        115                 120                 125
Phe Ser Ile Lys Asp Pro Ala Asp Val Tyr Tyr Thr Lys Lys Lys Ala
    130                 135                 140
Glu Val Glu Leu Asp Ile Asn Thr Ala Ser Thr Trp Lys Lys Phe Glu
145                 150                 155                 160
Val Tyr Glu Asn Asn Gln Lys Leu Pro Val Arg Leu Val Ser Tyr Ser
                165                 170                 175
Pro Val Pro Glu Asp His Ala Tyr Ile Arg Phe Pro Val Ser Asp Gly
            180                 185                 190
Thr Gln Glu Leu Lys Ile Val Ser Ser Thr Gln Ile Asp Asp Gly Glu
        195                 200                 205
Glu Thr Asn Tyr Asp Tyr Thr Lys Leu Val Phe Ala Lys Pro Ile Tyr
    210                 215                 220
Asn Asp Pro Ser Leu Val Lys Ser Asp Thr Asn Asp Ala Val Val Thr
225                 230                 235                 240
Asn Asp Gln Ser Ser Ser Val Ala Ser Asn Gln Thr Asn Thr Asn Thr
                245                 250                 255
Ser Asn Gln Asn Ile Ser Thr Ile Asn Asn Ala Asn Asn Gln Pro Gln
            260                 265                 270
Ala Thr Thr Asn Met Ser Gln Pro Ala Gln Pro Lys Ser Ser Thr Asn
        275                 280                 285
Ala Asp Gln Ala Ser Ser Gln Pro Ala His Glu Thr Asn Ser Asn Gly
    290                 295                 300
Asn Thr Asn Asp Lys Thr Asn Glu Ser Ser Asn Gln Ser Asp Val Asn
305                 310                 315                 320
Gln Gln Tyr Pro Pro Ala Asp Glu Ser Leu Gln Asp Ala Ile Lys Asn
                325                 330                 335
Pro Ala Ile Ile Asp Lys Glu His Thr Ala Asp Asn Trp Arg Pro Ile
            340                 345                 350
Asp Phe Gln Met Lys Asn Asp Lys Gly Glu Arg Gln Phe Tyr His Tyr
        355                 360                 365
Ala Ser Thr Val Glu Pro Ala Thr Val Ile Phe Thr Lys Thr Gly Pro
    370                 375                 380
Ile Ile Glu Leu Gly Leu Lys Thr Ala Ser Thr Trp Lys Lys Phe Glu
385                 390                 395                 400
Val Tyr Glu Gly Asp Lys Lys Leu Pro Val Glu Leu Val Ser Tyr Asp
                405                 410                 415
Ser Asp Lys Asp Tyr Ala Tyr Ile Arg Phe Pro Val Ser Asn Gly Thr
            420                 425                 430
Arg Glu Val Lys Ile Val Ser Ser Ile Glu Tyr Gly Glu Asn Ile His
        435                 440                 445
Glu Asp Tyr Asp Tyr Thr Leu Met Val Phe Ala Gln Pro Ile Thr Asn

450                 455                 460
Asn Pro Asp Asp Tyr Val Asp Glu Glu Thr Tyr Asn Leu Gln Lys Leu
465                 470                 475                 480
Leu Ala Pro Tyr His Lys Ala Lys Thr Leu Glu Arg Gln Val Tyr Glu
                485                 490                 495
Leu Glu Lys Leu Gln Glu Lys Leu Pro Glu Lys Tyr Lys Ala Glu Tyr
            500                 505                 510
Lys Lys Lys Leu Asp Gln Thr Arg Val Glu Leu Ala Asp Gln Val Lys
        515                 520                 525
Ser Ala Val Thr Glu Phe Glu Asn Val Thr Pro Thr Asn Asp Gln Leu
    530                 535                 540
Thr Asp Leu Gln Glu Ala His Phe Val Val Phe Glu Ser Glu Glu Asn
545                 550                 555                 560
Ser Glu Ser Val Met Asp Gly Phe Val Glu His Pro Phe Tyr Thr Ala
                565                 570                 575
Thr Leu Asn Gly Gln Lys Tyr Val Val Met Lys Thr Lys Asp Asp Ser
            580                 585                 590
Tyr Trp Lys Asp Leu Ile Val Glu Gly Lys Arg Val Thr Thr Val Ser
        595                 600                 605
Lys Asp Pro Lys Asn Asn Ser Arg Thr Leu Ile Phe Pro Tyr Ile Pro
    610                 615                 620
Asp Lys Ala Val Tyr Asn Ala Ile Val Lys Val Val Val Ala Asn Ile
625                 630                 635                 640
Gly Tyr Glu Gly Gln Tyr His Val Arg Ile Ile Asn Gln Asp Ile Asn
                645                 650                 655
Thr Lys Asp Asp Asp Thr Ser Gln Asn Asn Thr Ser Glu Pro Leu Asn
            660                 665                 670
Val Gln Thr Gly Gln Glu Gly Lys Val Ala Asp Thr Asp Val Ala Glu
        675                 680                 685
Asn Ser Ser Thr Ala Thr Asn Pro Lys Asp Ala Ser Asp Lys Ala Asp
    690                 695                 700
Val Ile Glu Pro Glu Ser Asp Val Val Lys Asp Ala Asp Asn Asn Ile
705                 710                 715                 720
Asp Lys Asp Val Gln His Asp Val Asp His Leu Ser Asp Met Ser Asp
                725                 730                 735
Asn Asn His Phe Asp Lys Tyr Asp Leu Lys Glu Met Asp Thr Gln Ile
            740                 745                 750
Ala Lys Asp Thr Asp Arg Asn Val Asp Lys Asp Ala Asp Asn Ser Val
        755                 760                 765
Gly Met Ser Ser Asn Val Asp Thr Asp Lys Asp Ser Asn Lys Asn Lys
    770                 775                 780
Asp Lys Val Ile Gln Leu Asn His Ile Ala Asp Lys Asn Asn His Thr
785                 790                 795                 800
Gly Lys Ala Ala Lys Leu Asp Val Val Lys Gln Asn Tyr Asn Asn Thr
                805                 810                 815
Asp Lys Val Thr Asp Lys Lys Thr Thr Glu His Leu Pro Ser Asp Ile
            820                 825                 830
His Lys Thr Val Asp Lys Thr Val Lys Thr Lys Glu Lys Ala Gly Thr
        835                 840                 845
Pro Ser Lys Glu Asn Lys Leu Ser Gln Ser Lys Met Leu Pro Lys Thr
    850                 855                 860
Gly Glu Thr Thr Ser Ser Gln Ser Trp Trp Gly Leu Tyr Ala Leu Leu
865                 870                 875                 880
Gly Met Leu Ala Leu Phe Ile Pro Lys Phe Arg Lys Glu Ser Lys Met
                885                 890                 895
Ala Glu Thr Thr Gln Asp Gln Thr Thr Asn Lys Asn Val Leu Asp Ser
            900                 905                 910
Asn Lys Val Lys Ala Thr Thr Glu Gln Ala Lys Ala Glu Val Lys Asn
        915                 920                 925
Pro Thr Gln Asn Ile Ser Gly Thr Gln Val Tyr Gln Asp Pro Ala Ile
    930                 935                 940
Val Gln Pro Lys Thr Ala Asn Asn Lys Thr Gly Asn Ala Gln Val Ser
945                 950                 955                 960
Gln Lys Val Asp Thr Ala Gln Val Asn Gly Asp Thr Arg Ala Asn Gln
                965                 970                 975

Ser Ala Thr Thr Asn Asn Thr Gln Pro Val Ala Lys Ser Thr Ser Thr
                980                 985                 990
Thr Ala Pro Lys Thr Asn Thr Asn Val Thr Asn Ala Gly Tyr Ser Leu
        995                 1000                1005
Val Asp Asp Glu Asp Asp Asn Ser Glu Asn Gln Ile Asn Pro Glu Leu
    1010                1015                1020
Ile Lys Ser Ala Ala Lys Pro Ala Ala Leu Glu Thr Gln Tyr Lys Thr
1025                1030                1035                1040
Ala Ala Pro Lys Ala Ala Thr Thr Ser Ala Pro Lys Ala Lys Thr Glu
                1045                1050                1055
Ala Thr Pro Lys Val Thr Thr Phe Ser Ala Ser Ala Gln Pro Arg Ser
            1060                1065                1070
Val Ala Ala Thr Pro Lys Thr Ser Leu Pro Lys Tyr Lys Pro Gln Val
        1075                1080                1085
Asn Ser Ser Ile Asn Asp Tyr Ile Cys Lys Asn Asn Leu Lys Ala Pro
    1090                1095                1100
Lys Ile Glu Glu Asp Tyr Thr Ser Tyr Phe Pro Lys Tyr Ala Tyr Arg
1105                1110                1115                1120
Asn Gly Val Gly Arg Pro Glu Gly Ile Val Val His Asp Thr Ala Asn
                1125                1130                1135
Asp Arg Ser Thr Ile Asn Gly Glu Ile Ser Tyr Met Lys Asn Asn Tyr
            1140                1145                1150
Gln Asn Ala Phe Val His Ala Phe Val Asp Gly Asp Arg Ile Ile Glu
        1155                1160                1165
Thr Ala Pro Thr Asp Tyr Leu Ser Trp Gly Val Gly Ala Val Gly Asn
    1170                1175                1180
Pro Arg Phe Ile Asn Val Glu Ile Val His Thr His Asp Tyr Ala Ser
1185                1190                1195                1200
Phe Ala Arg Ser Met Asn Asn Tyr Ala Asp Tyr Ala Ala Thr Gln Leu
                1205                1210                1215
Gln Tyr Tyr Gly Leu Lys Pro Asp Ser Ala Glu Tyr Asp Gly Asn Gly
            1220                1225                1230
Thr Val Trp Thr His Tyr Ala Val Ser Lys Tyr Leu Gly Gly Thr Asp
        1235                1240                1245
His Ala Asp Pro His Gly Tyr Leu Arg Ser His Asn Tyr Ser Tyr Asp
    1250                1255                1260
Gln Leu Tyr Asp Leu Ile Asn Glu Lys Tyr Leu Ile Lys Met Gly Lys
1265                1270                1275                1280
Val Ala Pro Trp Gly Thr Gln Ser Thr Thr Thr Pro Thr Thr Pro Ser
                1285                1290                1295
Lys Pro Thr Thr Pro Ser Lys Pro Ser Thr Gly Lys Leu Thr Val Ala
            1300                1305                1310
Ala Asn Asn Gly Val Ala Gln Ile Lys Pro Thr Asn Ser Gly Leu Tyr
        1315                1320                1325
Thr Thr Val Tyr Asp Lys Thr Gly Lys Ala Thr Asn Glu Val Gln Lys
    1330                1335                1340
Thr Phe Ala Val Ser Lys Thr Ala Thr Leu Gly Asn Gln Lys Phe Tyr
1345                1350                1355                1360
Leu Val Gln Asp Tyr Asn Ser Gly Asn Lys Phe Gly Trp Val Lys Glu
                1365                1370                1375
Gly Asp Val Val Tyr Asn Thr Ala Lys Ser Pro Val Asn Val Asn Gln
            1380                1385                1390
Ser Tyr Ser Ile Lys Pro Gly Thr Lys Leu Tyr Thr Val Pro Trp Gly
        1395                1400                1405
Thr Ser Lys Gln Val Ala Gly Ser Val Ser Gly Ser Gly Asn Gln Thr
    1410                1415                1420
Phe Lys Ala Ser Lys Gln Gln Gln Ile Asp Lys Ser Ile Tyr Leu Tyr
1425                1430                1435                1440
Gly Ser Val Asn Gly Lys Ser Gly Trp Val Ser Lys Ala Tyr Leu Val
                1445                1450                1455
Asp Thr Ala Lys Pro Thr Pro Thr Pro Thr Pro Lys Pro Ser Thr Pro
            1460                1465                1470
Thr Thr Asn Asn Lys Leu Thr Val Ser Ser Leu Asn Gly Val Ala Gln
        1475                1480                1485
Ile Asn Ala Lys Asn Asn Gly Leu Phe Thr Thr Val Tyr Asp Lys Thr

1490                1495                1500
Gly Lys Pro Thr Lys Glu Val Gln Lys Thr Phe Ala Val Thr Lys Glu
1505                1510                1515                1520
Ala Ser Leu Gly Gly Asn Lys Phe Tyr Leu Val Lys Asp Tyr Asn Ser
                1525                1530                1535
Pro Thr Leu Ile Gly Trp Val Lys Gln Gly Asp Val Ile Tyr Asn Asn
            1540                1545                1550
Ala Lys Ser Pro Val Asn Val Met Gln Thr Tyr Thr Val Lys Pro Gly
        1555                1560                1565
Thr Lys Leu Tyr Ser Val Pro Trp Gly Thr Tyr Lys Gln Glu Ala Gly
    1570                1575                1580
Ala Val Ser Gly Thr Gly Asn Gln Thr Phe Lys Ala Thr Lys Gln Gln
1585                1590                1595                1600
Gln Ile Asp Lys Ser Ile Tyr Leu Phe Gly Thr Val Asn Gly Lys Ser
                1605                1610                1615
Gly Trp Val Ser Lys Ala Tyr Leu Ala Val Pro Ala Ala Pro Lys Lys
            1620                1625                1630
Ala Val Ala Gln Pro Lys Thr Ala Val Lys
        1635                1640

<210> 70
<211> 482
<212> PRT
<213> Staphylococcus aureus

<400> 70
Met Ala Tyr Thr Val Thr Lys Pro Gln Thr Thr Gln Thr Val Ser Lys
1               5                   10                  15
Ile Ala Gln Val Lys Pro Asn Asn Thr Gly Ile Arg Ala Ser Val Tyr
            20                  25                  30
Glu Lys Thr Ala Lys Asn Gly Ala Lys Tyr Ala Asp Arg Thr Phe Tyr
        35                  40                  45
Val Thr Lys Glu Arg Ala His Gly Asn Glu Thr Tyr Val Leu Leu Asn
    50                  55                  60
Asn Thr Ser His Asn Ile Pro Leu Gly Trp Phe Asn Val Lys Asp Leu
65                  70                  75                  80
Asn Val Gln Asn Leu Gly Lys Glu Val Lys Thr Thr Gln Lys Tyr Thr
                85                  90                  95
Val Asn Lys Ser Asn Asn Gly Leu Ser Met Val Pro Trp Gly Thr Lys
            100                 105                 110
Asn Gln Val Ile Leu Thr Gly Asn Asn Ile Ala Gln Gly Thr Phe Asn
        115                 120                 125
Ala Thr Lys Gln Val Ser Val Gly Lys Asp Val Tyr Leu Tyr Gly Thr
    130                 135                 140
Ile Asn Asn Arg Thr Gly Trp Val Asn Ala Lys Asp Leu Thr Ala Pro
145                 150                 155                 160
Thr Ala Val Lys Pro Thr Thr Ser Ala Ala Lys Asp Tyr Asn Tyr Thr
                165                 170                 175
Tyr Val Ile Lys Asn Gly Asn Gly Tyr Tyr Tyr Val Thr Pro Asn Ser
            180                 185                 190
Asp Thr Ala Lys Tyr Ser Leu Lys Ala Phe Asn Glu Gln Pro Phe Ala
        195                 200                 205
Val Val Lys Glu Gln Val Ile Asn Gly Gln Thr Trp Tyr Tyr Gly Lys
    210                 215                 220
Leu Ser Asn Gly Lys Leu Ala Trp Ile Lys Ser Thr Asp Leu Ala Lys
225                 230                 235                 240
Glu Leu Ile Lys Tyr Asn Gln Thr Gly Met Thr Leu Asn Gln Val Ala
                245                 250                 255
Gln Ile Gln Ala Gly Leu Gln Tyr Lys Pro Gln Val Gln Arg Val Pro
            260                 265                 270
Gly Lys Trp Thr Asp Ala Lys Phe Asn Asp Val Lys His Ala Met Asp
        275                 280                 285
Thr Lys Arg Leu Ala Gln Asp Pro Ala Leu Lys Tyr Gln Phe Leu Arg
    290                 295                 300

Leu Asp Gln Pro Gln Asn Ile Ser Ile Asp Lys Ile Asn Gln Phe Leu
305                 310                 315                 320
Lys Gly Lys Gly Val Leu Glu Asn Gln Gly Ala Ala Phe Asn Lys Ala
                325                 330                 335
Ala Gln Met Tyr Gly Ile Asn Glu Val Tyr Leu Ile Ser His Ala Leu
            340                 345                 350
Leu Glu Thr Gly Asn Gly Thr Ser Gln Leu Ala Lys Gly Ala Asp Val
        355                 360                 365
Val Asn Asn Lys Val Val Thr Asn Ser Asn Thr Lys Tyr His Asn Val
    370                 375                 380
Phe Gly Ile Ala Ala Tyr Asp Asn Asp Pro Leu Arg Glu Gly Ile Lys
385                 390                 395                 400
Tyr Ala Lys Gln Ala Gly Trp Asp Thr Val Ser Lys Ala Ile Val Gly
                405                 410                 415
Gly Ala Lys Phe Ile Gly Asn Ser Tyr Val Lys Ala Gly Gln Asn Thr
            420                 425                 430
Leu Tyr Lys Met Arg Trp Asn Pro Ala His Pro Gly Thr His Gln Tyr
        435                 440                 445
Ala Thr Asp Val Asp Trp Ala Asn Ile Asn Ala Lys Ile Ile Lys Gly
    450                 455                 460
Tyr Tyr Asp Lys Ile Gly Glu Val Gly Lys Tyr Phe Asp Ile Pro Gln
465                 470                 475                 480
Tyr Lys

<210> 71
<211> 706

<212> PRT
<213> Staphylococcus aureus

<400> 71
Asp Arg Val Leu Ala Ser His Pro Asp Val Ala Thr Ile Arg Gln Asn
1               5                   10                  15
Val Thr Ala Ala Asn Ala Ala Lys Ser Ala Leu Asp Gln Ala Arg Asn
            20                  25                  30
Gly Leu Thr Val Asp Lys Ala Pro Leu Glu Asn Ala Lys Asn Gln Leu
        35                  40                  45
Gln His Ser Ile Asp Thr Gln Thr Ser Thr Thr Gly Met Thr Gln Asp
    50                  55                  60
Ser Ile Asn Ala Tyr Asn Ala Lys Leu Thr Ala Ala Arg Asn Lys Ile
65                  70                  75                  80
Gln Gln Ile Asn Gln Val Leu Ala Gly Ser Pro Thr Val Glu Gln Ile
                85                  90                  95
Asn Thr Asn Thr Ser Thr Ala Asn Gln Ala Lys Ser Asp Leu Asp His
            100                 105                 110
Ala Arg Gln Ala Leu Thr Pro Asp Lys Ala Pro Leu Gln Thr Ala Lys
        115                 120                 125
Thr Gln Leu Glu Gln Ser Ile Asn Gln Pro Thr Asp Thr Thr Gly Met
    130                 135                 140
Thr Thr Ala Ser Leu Asn Ala Tyr Asn Gln Lys Leu Gln Ala Ala Arg
145                 150                 155                 160
Gln Lys Leu Thr Glu Ile Asn Gln Val Leu Asn Gly Asn Pro Thr Val
                165                 170                 175
Gln Asn Ile Asn Asp Lys Val Thr Glu Ala Asn Gln Ala Lys Asp Gln
            180                 185                 190
Leu Asn Thr Ala Arg Gln Gly Leu Thr Leu Asp Arg Gln Pro Ala Leu
        195                 200                 205
Thr Thr Leu His Gly Ala Ser Asn Leu Asn Gln Ala Gln Gln Asn Asn
    210                 215                 220
Phe Thr Gln Gln Ile Asn Ala Ala Gln Asn His Ala Ala Leu Glu Thr
225                 230                 235                 240
Ile Lys Ser Asn Ile Thr Ala Leu Asn Thr Ala Met Thr Lys Leu Lys
                245                 250                 255

Asp Ser Val Ala Asp Asn Asn Thr Ile Lys Ser Asp Gln Asn Tyr Thr
            260                 265                 270
Asp Ala Thr Pro Ala Asn Lys Gln Ala Tyr Asp Asn Ala Val Asn Ala
        275                 280                 285
Ala Lys Gly Val Ile Gly Glu Thr Thr Asn Pro Thr Met Asp Val Asn
    290                 295                 300
Thr Val Asn Gln Lys Ala Ala Ser Val Lys Ser Thr Lys Asp Ala Leu
305                 310                 315                 320
Asp Gly Gln Gln Asn Leu Gln Arg Ala Lys Thr Glu Ala Thr Asn Ala
                325                 330                 335
Ile Thr His Ala Ser Asp Leu Asn Gln Ala Gln Lys Asn Ala Leu Thr
            340                 345                 350
Gln Gln Val Asn Ser Ala Gln Asn Val Gln Ala Val Asn Asp Ile Lys
        355                 360                 365
Gln Thr Thr Gln Ser Leu Asn Thr Ala Met Thr Gly Leu Lys Arg Gly
    370                 375                 380
Val Ala Asn His Asn Gln Val Val Gln Ser Asp Asn Tyr Val Asn Ala
385                 390                 395                 400
Asp Thr Asn Lys Lys Asn Asp Tyr Asn Asn Ala Tyr Asn His Ala Asn
                405                 410                 415
Asp Ile Ile Asn Gly Asn Ala Gln His Pro Val Ile Thr Pro Ser Asp
            420                 425                 430
Val Asn Asn Ala Leu Ser Asn Val Thr Ser Lys Glu His Ala Leu Asn
        435                 440                 445
Gly Glu Ala Lys Leu Asn Ala Ala Lys Gln Glu Ala Asn Thr Ala Leu
    450                 455                 460
Gly His Leu Asn Asn Leu Asn Asn Ala Gln Arg Gln Asn Leu Gln Ser
465                 470                 475                 480
Gln Ile Asn Gly Ala His Gln Ile Asp Ala Val Asn Thr Ile Lys Gln
                485                 490                 495
Asn Ala Thr Asn Leu Asn Ser Ala Met Gly Asn Leu Arg Gln Ala Val
            500                 505                 510
Ala Asp Lys Asp Gln Val Lys Arg Thr Glu Asp Tyr Ala Asp Ala Asp
        515                 520                 525
Thr Ala Lys Gln Asn Ala Tyr Asn Ser Ala Val Ser Ser Ala Glu Thr
    530                 535                 540
Ile Ile Asn Gln Thr Thr Asn Pro Thr Met Ser Val Asp Asp Val Asn
545                 550                 555                 560
Arg Ala Thr Ser Ala Val Thr Ser Asn Lys Asn Ala Leu Asn Gly Tyr
                565                 570                 575
Glu Lys Leu Ala Gln Ser Lys Thr Asp Ala Ala Arg Ala Ile Asp Ala
            580                 585                 590
Leu Pro His Leu Asn Asn Ala Gln Lys Ala Asp Val Lys Ser Lys Ile
        595                 600                 605
Asn Ala Ala Ser Asn Ile Ala Gly Val Asn Thr Val Lys Gln Gln Gly
    610                 615                 620
Thr Asp Leu Asn Thr Ala Met Gly Asn Leu Gln Gly Ala Ile Asn Asp
625                 630                 635                 640
Glu Gln Thr Thr Leu Asn Ser Gln Asn Tyr Gln Asp Ala Thr Pro Ser
                645                 650                 655
Lys Lys Thr Ala Tyr Thr Asn Ala Val Gln Ala Ala Lys Asp Ile Leu
            660                 665                 670
Asn Lys Ser Asn Gly Gln Asn Lys Thr Lys Asp Gln Val Thr Glu Ala
        675                 680                 685
Met Asn Gln Val Asn Ser Ala Lys Asn Asn Leu Asp Gly Thr Arg Leu
    690                 695                 700
Leu Asp
705

<210> 72
<211> 241
<212> PRT
<213> Staphylococcus aureus

<400> 72
Ala Ser Thr Gln His Thr Val Gln Ser Gly Glu Ser Leu Trp Ser Ile
1               5                   10                  15
Ala Gln Lys Tyr Asn Thr Ser Val Glu Ser Ile Lys Gln Asn Asn Gln
            20                  25                  30
Leu Asp Asn Asn Leu Val Phe Pro Gly Gln Val Ile Ser Val Gly Gly
        35                  40                  45
Ser Asp Ala Gln Asn Thr Ser Asn Thr Ser Pro Gln Ala Gly Ser Ala
    50                  55                  60
Ser Ser His Thr Val Gln Ala Gly Glu Ser Leu Asn Ile Ile Ala Ser
65                  70                  75                  80
Arg Tyr Gly Val Ser Val Asp Gln Leu Met Ala Ala Asn Asn Leu Arg
                85                  90                  95
Gly Tyr Leu Ile Met Pro Asn Gln Thr Leu Gln Ile Pro Asn Gly Gly
            100                 105                 110
Ser Gly Gly Thr Thr Pro Thr Ala Thr Thr Gly Ser Asn Gly Asn Ala
        115                 120                 125
Ser Ser Phe Asn His Gln Asn Leu Tyr Thr Ala Gly Gln Cys Thr Trp
    130                 135                 140
Tyr Val Phe Asp Arg Arg Ala Gln Ala Gly Ser Pro Ile Ser Thr Tyr
145                 150                 155                 160
Trp Ser Asp Ala Lys Tyr Trp Ala Gly Asn Ala Ala Asn Asp Gly Tyr
                165                 170                 175
Gln Val Asn Asn Thr Pro Ser Val Gly Ser Ile Met Gln Ser Thr Pro
            180                 185                 190
Gly Pro Tyr Gly His Val Ala Tyr Val Glu Arg Val Asn Gly Asp Gly
        195                 200                 205
Ser Ile Leu Ile Ser Glu Met Asn Tyr Thr Tyr Gly Pro Tyr Asn Met
    210                 215                 220
Asn Tyr Arg Thr Ile Pro Ala Ser Glu Val Ser Ser Tyr Ala Phe Ile
225                 230                 235                 240
His

<210> 73
<211> 995
<212> PRT
<213> Staphylococcus aureus

<400> 73
   Met Asn Asn Lys Lys Thr Ala Thr Asn Arg Lys Gly Met Ile Pro Asn
   1               5                   10                  15
   Arg Leu Asn Lys Phe Ser Ile Arg Lys Tyr Ser Val Gly Thr Ala Ser
               20                  25                  30
   Ile Leu Val Gly Thr Thr Leu Ile Phe Gly Leu Ser Gly His Glu Ala
           35                  40                  45
   Lys Ala Ala Glu His Thr Asn Gly Glu Leu Asn Gln Ser Lys Asn Glu
       50                  55                  60
   Thr Thr Ala Pro Ser Glu Asn Lys Thr Thr Lys Lys Val Asp Ser Arg
   65                  70                  75                  80
   Gln Leu Lys Asp Asn Thr Gln Thr Ala Thr Ala Asp Gln Pro Lys Val
                   85                  90                  95
   Thr Met Ser Asp Ser Ala Thr Val Lys Glu Thr Ser Ser Asn Met Gln
               100                 105                 110
   Ser Pro Gln Asn Ala Thr Ala Asn Gln Ser Thr Thr Lys Thr Ser Asn
           115                 120                 125
   Val Thr Thr Asn Asp Lys Ser Ser Thr Thr Tyr Ser Asn Glu Thr Asp
       130                 135                 140
   Lys Ser Asn Leu Thr Gln Ala Lys Asp Val Ser Thr Thr Pro Lys Thr
   145                 150                 155                 160
   Thr Thr Ile Lys Pro Arg Thr Leu Asn Arg Met Ala Val Asn Thr Val
                   165                 170                 175
   Ala Ala Pro Gln Gln Gly Thr Asn Val Asn Asp Lys Val His Phe Ser
   180 185 190
   Asn Ile Asp Ile Ala Ile Asp Lys Gly His Val Asn Gln Thr Thr Gly
   195 200 205
   Lys Thr Glu Phe Trp Ala Thr Ser Ser Asp Val Leu Lys Leu Lys Ala
   210 215 220
   Asn Tyr Thr Ile Asp Asp Ser Val Lys Glu Gly Asp Thr Phe Thr Phe
   225 230 235 240
   Lys Tyr Gly Gln Tyr Phe Arg Pro Gly Ser Val Arg Leu Pro Ser Gln
   245 250 255
   Thr Gln Asn Leu Tyr Asn Ala Gln Gly Asn Ile Ile Ala Lys Gly Ile
   260 265 270
   Tyr Asp Ser Thr Thr Asn Thr Thr Thr Tyr Thr Phe Thr Asn Tyr Val
   275 280 285
   Asp Gln Tyr Thr Asn Val Arg Gly Ser Phe Glu Gln Val Ala Phe Ala
   290 295 300
   Lys Arg Lys Asn Ala Thr Thr Asp Lys Thr Ala Tyr Lys Met Glu Val
   305 310 315 320
   Thr Leu Gly Asn Asp Thr Tyr Ser Glu Glu Ile Ile Val Asp Tyr Gly
   325 330 335
   Asn Lys Lys Ala Gln Pro Leu Ile Ser Ser Thr Asn Tyr Ile Asn Asn
   340 345 350
   Glu Asp Leu Ser Arg Asn Met Thr Ala Tyr Val Asn Gln Pro Lys Asn
   355 360 365
   Thr Tyr Thr Lys Gln Thr Phe Val Thr Asn Leu Thr Gly Tyr Lys Phe
   370 375 380
   Asn Pro Asn Ala Lys Asn Phe Lys Ile Tyr Glu Val Thr Asp Gln Asn
   385 390 395 400
   Gln Phe Val Asp Ser Phe Thr Pro Asp Thr Ser Lys Leu Lys Asp Val
   405 410 415
   Thr Asp Gln Phe Asp Val Ile Tyr Ser Asn Asp Asn Lys Thr Ala Thr
   420 425 430
   Val Asp Leu Met Lys Gly Gln Thr Ser Ser Asn Lys Gln Tyr Ile Ile
   435 440 445
   Gln Gln Val Ala Tyr Pro Asp Asn Ser Ser Thr Asp Asn Gly Lys Ile
   450 455 460
   Asp Tyr Thr Leu Asp Thr Asp Lys Thr Lys Tyr Ser Trp Ser Asn Ser
   465 470 475 480
   Tyr Ser Asn Val Asn Gly Ser Ser Thr Ala Asn Gly Asp Gln Lys Lys
   485 490 495
   Tyr Asn Leu Gly Asp Tyr Val Trp Glu Asp Thr Asn Lys Asp Gly Lys
   500 505 510
   Gln Asp Ala Asn Glu Lys Gly Ile Lys Gly Val Tyr Val Ile Leu Lys
   515 520 525
   Asp Ser Asn Gly Lys Glu Leu Asp Arg Thr Thr Thr Asp Glu Asn Gly
   530 535 540
   Lys Tyr Gln Phe Thr Gly Leu Ser Asn Gly Thr Tyr Ser Val Glu Phe
   545 550 555 560
   Ser Thr Pro Ala Gly Tyr Thr Pro Thr Thr Ala Asn Val Gly Thr Asp
   565 570 575
   Asp Ala Val Asp Ser Asp Gly Leu Thr Thr Thr Gly Val Ile Lys Asp
   580 585 590
   Ala Asp Asn Met Thr Leu Asp Ser Gly Phe Tyr Lys Thr Pro Lys Tyr
   595 600 605
   Ser Leu Gly Asp Tyr Val Trp Tyr Asp Ser Asn Lys Asp Gly Lys Gln
   610 615 620
   Asp Ser Thr Glu Lys Gly Ile Lys Gly Val Lys Val Thr Leu Gln Asn
   625 630 635 640
   Glu Lys Gly Glu Val Ile Gly Thr Thr Glu Thr Asp Glu Asn Gly Lys
   645 650 655
   Tyr Arg Phe Asp Asn Leu Asp Ser Gly Lys Tyr Lys Val Ile Phe Glu
   660 665 670
   Lys Pro Ala Gly Leu Thr Gln Thr Gly Thr Asn Thr Thr Glu Asp Asp
   675 680 685
   Lys Asp Ala Asp Gly Gly Glu Val Asp Val Thr Ile Thr Asp His Asp
   690 695 700
   Asp Phe Thr Leu Asp Asn Gly Tyr Tyr Glu Glu Glu Thr Ser Asp Ser
   705                 710                 715                 720
   Asp Ser Asp Ser Asp Ser Asp Ser Asp Ser Asp Ser Asp Ser Asp Ser
                   725                 730                 735
   Asp Ser Asp Ser Asp Ser Asp Ser Asp Ser Asp Ser Asp Ser Asp Ser
               740                 745                 750
   Asp Ser Asp Ser Asp Ser Asp Ser Asp Ser Asp Ser Asp Ser Asp Ser
           755                 760                 765
   Asp Ser Asp Ser Asp Ser Asp Ser Asp Ser Asp Ser Asp Ser Asp Ser
       770                 775                 780
   Asp Ser Asp Ser Asp Ser Asp Ser Asp Ser Asp Ser Asp Ser Asp Ser
   785                 790                 795                 800
   Asp Ser Asp Ser Asp Ser Asp Ser Asp Ser Asp Ser Asp Ser Asp Ser
                   805                 810                 815
   Asp Ser Asp Ser Asp Ser Asp Ser Asp Ser Asp Ser Asp Ser Asp Ser
               820                 825                 830
   Asp Ser Asp Ser Asp Ser Asp Ser Asp Ser Asp Ser Asp Ser Asp Ser
           835                 840                 845
   Asp Ser Asp Ser Asp Ser Asp Ser Asp Asn Asp Ser Asp Ser Asp Ser
       850                 855                 860
   Asp Ser Asp Ser Asp Ser Asp Ser Asp Ser Asp Ser Asp Ser Asp Ser
   865                 870                 875                 880
   Asp Ser Asp Ser Asp Ser Asp Ser Asp Ser Asp Ser Asp Ser Asp Ser
                   885                 890                 895
   Asp Ser Asp Ser Asp Ser Asp Ser Asp Ser Asp Ser Asp Ser Asp Ser
               900                 905                 910
   Asp Ser Asp Ser Asp Ser Asp Ser Asp Ser Asp Asn Asp Ser Asp Ser
           915                 920                 925
   Asp Ser Asp Ser Asp Ser Asp Ala Gly Lys His Thr Pro Ala Lys Pro
       930                 935                 940
   Met Ser Thr Val Lys Asp Gln His Lys Thr Ala Lys Ala Leu Pro Glu
   945                 950                 955                 960
   Thr Gly Ser Glu Asn Asn Asn Ser Asn Asn Gly Thr Leu Phe Gly Gly
                   965                 970                 975
   Leu Phe Ala Ala Leu Gly Ser Leu Leu Leu Phe Gly Arg Arg Lys Lys
               980                 985                 990
   Gln Asn Lys
           995

<210> 74
<211> 2186
<212> PRT
<213> Staphylococcus aureus

<400> 74
Met Asn Leu Leu Lys Lys Asn Lys Tyr Ser Ile Arg Lys Tyr Lys Val
1               5                   10                  15
Gly Ile Phe Ser Thr Leu Ile Gly Thr Val Leu Leu Leu Ser Asn Pro
            20                  25                  30
Asn Gly Ala Gln Ala Leu Thr Thr Asp Asn Asn Val Gln Ser Asp Thr
        35                  40                  45
Asn Gln Ala Thr Pro Val Asn Ser Gln Asp Lys Asp Val Ala Asn Asn
    50                  55                  60
Arg Gly Leu Ala Asn Ser Ala Gln Asn Thr Pro Asn Gln Ser Ala Thr
65                  70                  75                  80
Thr Asn Gln Ala Thr Asn Gln Ala Leu Val Asn His Asn Asn Gly Ser
                85                  90                  95
Ile Val Asn Gln Ala Thr Pro Thr Ser Val Gln Ser Ser Thr Pro Ser
            100                 105                 110
Ala Gln Asn Asn Asn His Thr Asp Gly Asn Thr Thr Ala Thr Glu Thr
        115                 120                 125
Val Ser Asn Ala Asn Asn Asn Asp Val Val Ser Asn Asn Thr Ala Leu
    130                 135                 140
Asn Val Pro Thr Lys Thr Asn Glu Asn Gly Ser Gly Gly His Leu Thr

145                 150                 155                 160
Leu Lys Glu Ile Gln Glu Asp Val Arg His Ser Ser Asn Lys Pro Glu
                165                 170                 175
Leu Val Ala Ile Ala Glu Pro Ala Ser Asn Arg Pro Lys Lys Arg Ser
            180                 185                 190
Arg Arg Ala Ala Pro Ala Asp Pro Asn Ala Thr Pro Ala Asp Pro Ala
        195                 200                 205
Ala Ala Ala Val Gly Asn Gly Gly Ala Pro Val Ala Ile Thr Ala Pro
    210                 215                 220
Tyr Thr Pro Thr Thr Asp Pro Asn Ala Asn Asn Ala Gly Gln Asn Ala
225                 230                 235                 240
Pro Asn Glu Val Leu Ser Phe Asp Asp Asn Gly Ile Arg Pro Ser Thr
                245                 250                 255
Asn Arg Ser Val Pro Thr Val Asn Val Val Asn Asn Leu Pro Gly Phe
            260                 265                 270
Thr Leu Ile Asn Gly Gly Lys Val Gly Val Phe Ser His Ala Met Val
        275                 280                 285
Arg Thr Ser Met Phe Asp Ser Gly Asp Asn Lys Asn Tyr Gln Ala Gln
    290                 295                 300
Gly Asn Val Ile Ala Leu Gly Arg Ile His Gly Thr Asp Thr Asn Asp
305                 310                 315                 320
His Gly Asp Phe Asn Gly Ile Glu Lys Ala Leu Thr Val Asn Pro Asn
                325                 330                 335
Ser Glu Leu Ile Phe Glu Phe Asn Thr Met Thr Thr Lys Asn Gly Gln
            340                 345                 350
Gly Ala Thr Asn Val Ile Ile Lys Asn Ala Asp Thr Asn Asp Thr Ile
        355                 360                 365
Ala Glu Lys Thr Val Glu Gly Gly Pro Thr Leu Arg Leu Phe Lys Val
    370                 375                 380
Pro Asp Asn Val Arg Asn Leu Lys Ile Gln Phe Val Pro Lys Asn Asp
385                 390                 395                 400
Ala Ile Thr Asp Ala Arg Gly Ile Tyr Gln Leu Lys Asp Gly Tyr Lys
                405                 410                 415
Tyr Tyr Ser Phe Val Asp Ser Ile Gly Leu His Ser Gly Ser His Val
            420                 425                 430
Phe Val Glu Arg Arg Thr Met Asp Pro Thr Ala Thr Asn Asn Lys Glu
        435                 440                 445
Phe Thr Val Thr Thr Ser Leu Lys Asn Asn Gly Asn Ser Gly Ala Ser
    450                 455                 460
Leu Asp Thr Asn Asp Phe Val Tyr Gln Val Gln Leu Pro Glu Gly Val
465                 470                 475                 480
Glu Tyr Val Asn Asn Ser Leu Thr Lys Asp Phe Pro Ser Asn Asn Ser
                485                 490                 495
Gly Val Asp Val Asn Asp Met Asn Val Thr Tyr Asp Ala Ala Asn Arg
            500                 505                 510
Val Ile Thr Ile Lys Ser Thr Gly Gly Gly Thr Ala Asn Ser Pro Ala
        515                 520                 525
Arg Leu Met Pro Asp Lys Ile Leu Asp Leu Arg Tyr Lys Leu Arg Val
    530                 535                 540
Asn Asn Val Pro Thr Pro Arg Thr Val Thr Phe Asn Glu Thr Leu Thr
545                 550                 555                 560
Tyr Lys Thr Tyr Thr Gln Asp Phe Ile Asn Ser Ala Ala Glu Ser His
                565                 570                 575
Thr Val Ser Thr Asn Pro Tyr Thr Ile Asp Ile Ile Met Asn Lys Asp
            580                 585                 590
Ala Leu Gln Ala Glu Val Asp Arg Arg Ile Gln Gln Ala Asp Tyr Thr
        595                 600                 605
Phe Ala Ser Leu Asp Ile Phe Asn Gly Leu Lys Arg Arg Ala Gln Thr
    610                 615                 620
Ile Leu Asp Glu Asn Arg Asn Asn Val Pro Leu Asn Lys Arg Val Ser
625                 630                 635                 640
Gln Ala Tyr Ile Asp Ser Leu Thr Asn Gln Met Gln His Thr Leu Ile
                645                 650                 655
Arg Ser Val Asp Ala Glu Asn Ala Val Asn Lys Lys Val Asp Gln Met

660                 665                 670
Glu Asp Leu Val Asn Gln Asn Asp Glu Leu Thr Asp Glu Glu Lys Gln
        675                 680                 685
Ala Ala Ile Gln Val Ile Glu Glu His Lys Asn Glu Ile Ile Gly Asn
    690                 695                 700
Ile Gly Asp Gln Thr Thr Asp Asp Gly Val Thr Arg Ile Lys Asp Gln
705                 710                 715                 720
Gly Ile Gln Thr Leu Ser Gly Asp Thr Ala Thr Pro Val Val Lys Pro
                725                 730                 735
Asn Ala Lys Lys Ala Ile Arg Asp Lys Ala Thr Lys Gln Arg Glu Ile
            740                 745                 750
Ile Asn Ala Thr Pro Asp Ala Thr Glu Asp Glu Ile Gln Asp Ala Leu
        755                 760                 765
Asn Gln Leu Ala Thr Asp Glu Thr Asp Ala Ile Asp Asn Val Thr Asn
    770                 775                 780
Ala Thr Thr Asn Ala Asp Val Glu Thr Ala Lys Asn Asn Gly Ile Asn
785                 790                 795                 800
Thr Ile Gly Ala Val Val Pro Gln Val Thr His Lys Lys Ala Ala Arg
                805                 810                 815
Asp Ala Ile Asn Gln Ala Thr Ala Thr Lys Arg Gln Gln Ile Asn Ser
            820                 825                 830
Asn Arg Glu Ala Thr Gln Glu Glu Lys Asn Ala Ala Leu Asn Glu Leu
        835                 840                 845
Thr Gln Ala Thr Asn His Ala Leu Glu Gln Ile Asn Gln Ala Thr Thr
    850                 855                 860
Asn Ala Asn Val Asp Asn Ala Lys Gly Asp Gly Leu Asn Ala Ile Asn
865                 870                 875                 880
Pro Ile Ala Pro Val Thr Val Val Lys Gln Ala Ala Arg Asp Ala Val
                885                 890                 895
Ser His Asp Ala Gln Gln His Ile Ala Glu Ile Asn Ala Asn Pro Asp
            900                 905                 910
Ala Thr Gln Glu Glu Arg Gln Ala Ala Ile Asp Lys Val Asn Ala Ala
        915                 920                 925
Val Thr Ala Ala Asn Thr Asn Ile Leu Asn Ala Asn Thr Asn Ala Asp
    930                 935                 940
Val Glu Gln Val Lys Thr Asn Ala Ile Gln Gly Ile Gln Ala Ile Thr
945                 950                 955                 960
Pro Ala Thr Lys Val Lys Thr Asp Ala Lys Asn Ala Ile Asp Lys Ser
                965                 970                 975
Ala Glu Thr Gln His Asn Thr Ile Phe Asn Asn Asn Asp Ala Thr Leu
            980                 985                 990
Glu Glu Gln Gln Ala Ala Gln Gln Leu Leu Asp Gln Ala Val Ala Thr
        995                 1000                1005
Ala Lys Gln Asn Ile Asn Ala Ala Asp Thr Asn Gln Glu Val Ala Gln
    1010                1015                1020
Ala Lys Asp Gln Gly Thr Gln Asn Ile Val Val Ile Gln Pro Ala Thr
1025                1030                1035                1040
Gln Val Lys Thr Asp Thr Arg Asn Val Val Asn Asp Lys Ala Arg Glu
                1045                1050                1055
Ala Ile Thr Asn Ile Asn Ala Thr Thr Gly Ala Thr Arg Glu Glu Lys
            1060                1065                1070
Gln Glu Ala Ile Asn Arg Val Asn Thr Leu Lys Asn Arg Ala Leu Thr
        1075                1080                1085
Asp Ile Gly Val Thr Ser Thr Thr Ala Met Val Asn Ser Ile Arg Asp
    1090                1095                1100
Asp Ala Val Asn Gln Ile Gly Ala Val Gln Pro His Val Thr Lys Lys
1105                1110                1115                1120
Gln Thr Ala Thr Gly Val Leu Asn Asp Leu Ala Thr Ala Lys Lys Gln
                1125                1130                1135
Glu Ile Asn Gln Asn Thr Asn Ala Thr Thr Glu Glu Lys Gln Val Ala
            1140                1145                1150
Leu Asn Gln Val Asp Gln Glu Leu Ala Thr Ala Ile Asn Asn Ile Asn
        1155                1160                1165
Gln Ala Asp Thr Asn Ala Glu Val Asp Gln Ala Gln Gln Leu Gly Thr
    1170                1175                1180

Lys Ala Ile Asn Ala Ile Gln Pro Asn Ile Val Lys Lys Pro Ala Ala
1185                1190                1195                1200
Leu Ala Gln Ile Asn Gln His Tyr Asn Ala Lys Leu Ala Glu Ile Asn
                1205                1210                1215
Ala Thr Pro Asp Ala Thr Asn Asp Glu Lys Asn Ala Ala Ile Asn Thr
            1220                1225                1230
Leu Asn Gln Asp Arg Gln Gln Ala Ile Glu Ser Ile Lys Gln Ala Asn
        1235                1240                1245
Thr Asn Ala Glu Val Asp Gln Ala Ala Thr Val Ala Glu Asn Asn Ile
    1250                1255                1260
Asp Ala Val Gln Val Asp Val Val Lys Lys Gln Ala Ala Arg Asp Lys
1265                1270                1275                1280
Ile Thr Ala Glu Val Ala Lys Arg Ile Glu Ala Val Lys Gln Thr Pro
                1285                1290                1295
Asn Ala Thr Asp Glu Glu Lys Gln Ala Ala Val Asn Gln Ile Asn Gln
            1300                1305                1310
Leu Lys Asp Gln Ala Ile Asn Gln Ile Asn Gln Asn Gln Thr Asn Asp
        1315                1320                1325
Gln Val Asp Thr Thr Thr Asn Gln Ala Val Asn Ala Ile Asp Asn Val
    1330                1335                1340
Glu Ala Glu Val Val Ile Lys Thr Lys Ala Ile Ala Asp Ile Glu Lys
1345                1350                1355                1360
Ala Val Lys Glu Lys Gln Gln Gln Ile Asp Asn Ser Leu Asp Ser Thr
                1365                1370                1375
Asp Asn Glu Lys Glu Val Ala Ser Gln Ala Leu Ala Lys Glu Lys Glu
            1380                1385                1390
Lys Ala Leu Ala Ala Ile Asp Gln Ala Gln Thr Asn Ser Gln Val Asn
        1395                1400                1405
Gln Ala Ala Thr Asn Gly Val Ser Ala Ile Lys Ile Ile Gln Pro Glu
    1410                1415                1420
Thr Lys Val Lys Pro Ala Ala Arg Glu Lys Ile Asn Gln Lys Ala Asn
1425                1430                1435                1440
Glu Leu Arg Ala Lys Ile Asn Gln Asp Lys Glu Ala Thr Ala Glu Glu
                1445                1450                1455
Arg Gln Val Ala Leu Asp Lys Ile Asn Glu Phe Val Asn Gln Ala Met
            1460                1465                1470
Thr Asp Ile Thr Asn Asn Arg Thr Asn Gln Gln Val Asp Asp Thr Thr
        1475                1480                1485
Ser Gln Ala Leu Asp Ser Ile Ala Leu Val Thr Pro Asp His Ile Val
    1490                1495                1500
Arg Ala Ala Ala Arg Asp Ala Val Lys Gln Gln Tyr Glu Ala Lys Lys
1505                1510                1515                1520
Arg Glu Ile Glu Gln Ala Glu His Ala Thr Asp Glu Glu Lys Gln Val
                1525                1530                1535
Ala Leu Asn Gln Leu Ala Asn Asn Glu Lys Arg Ala Leu Gln Asn Ile
            1540                1545                1550
Asp Gln Ala Ile Ala Asn Asn Asp Val Lys Arg Val Glu Thr Asn Gly
        1555                1560                1565
Ile Ala Thr Leu Lys Gly Val Gln Pro His Ile Val Ile Lys Pro Glu
    1570                1575                1580
Ala Gln Gln Ala Ile Lys Ala Ser Ala Glu Asn Gln Val Glu Ser Ile
1585                1590                1595                1600
Lys Asp Thr Pro His Ala Thr Val Asp Glu Leu Asp Glu Ala Asn Gln
                1605                1610                1615
Leu Ile Ser Asp Thr Leu Lys Gln Ala Gln Gln Glu Ile Glu Asn Thr
            1620                1625                1630
Asn Gln Asp Ala Ala Val Thr Asp Val Arg Asn Gln Thr Ile Lys Ala
        1635                1640                1645
Ile Glu Gln Ile Lys Pro Lys Val Arg Arg Lys Arg Ala Ala Leu Asp
    1650                1655                1660
Ser Ile Glu Glu Asn Asn Lys Asn Gln Leu Asp Ala Ile Arg Asn Thr
1665                1670                1675                1680
Leu Asp Thr Thr Gln Asp Glu Arg Asp Val Ala Ile Asp Thr Leu Asn
                1685                1690                1695
Lys Ile Val Asn Thr Ile Lys Asn Asp Ile Ala Gln Asn Lys Thr Asn

                1700                1705                1710
Ala Glu Val Asp Arg Thr Glu Thr Asp Gly Asn Asp Asn Ile Lys Val
        1715                1720                1725
Ile Leu Pro Lys Val Gln Val Lys Pro Ala Ala Arg Gln Ser Val Gly
    1730                1735                1740
Val Lys Ala Glu Ala Gln Asn Ala Leu Ile Asp Gln Ser Asp Leu Ser
1745                1750                1755                1760
Thr Glu Glu Glu Arg Leu Ala Ala Lys His Leu Val Glu Gln Ala Leu
                1765                1770                1775
Asn Gln Ala Ile Asp Gln Ile Asn His Ala Asp Lys Thr Ala Gln Val
            1780                1785                1790
Asn Gln Asp Ser Ile Asn Ala Gln Asn Ile Ile Ser Lys Ile Lys Pro
        1795                1800                1805
Ala Thr Thr Val Lys Ala Thr Ala Leu Gln Gln Ile Gln Asn Ile Ala
    1810                1815                1820
Thr Asn Lys Ile Asn Leu Ile Lys Ala Asn Asn Glu Ala Thr Asp Glu
1825                1830                1835                1840
Glu Gln Asn Ile Ala Ile Ala Gln Val Glu Lys Glu Leu Ile Lys Ala
                1845                1850                1855
Lys Gln Gln Ile Ala Ser Ala Val Thr Asn Ala Asp Val Ala Tyr Leu
            1860                1865                1870
Leu His Asp Glu Lys Asn Glu Ile Arg Glu Ile Glu Pro Val Ile Asn
        1875                1880                1885
Arg Lys Ala Ser Ala Arg Glu Gln Leu Thr Thr Leu Phe Asn Asp Lys
    1890                1895                1900
Lys Gln Ala Ile Glu Ala Asn Ile Gln Ala Thr Val Glu Glu Arg Asn
1905                1910                1915                1920
Ser Ile Leu Ala Gln Leu Gln Asn Ile Tyr Asp Thr Ala Ile Gly Gln
                1925                1930                1935
Ile Asp Gln Asp Arg Ser Asn Ala Gln Val Asp Lys Thr Ala Ser Leu
            1940                1945                1950
Asn Leu Gln Thr Ile His Asp Leu Asp Val His Pro Ile Lys Lys Pro
        1955                1960                1965
Asp Ala Glu Lys Thr Ile Asn Asp Asp Leu Ala Arg Val Thr Ala Leu
    1970                1975                1980
Val Gln Asn Tyr Arg Lys Val Ser Asn Arg Asn Lys Ala Asp Ala Leu
1985                1990                1995                2000
Lys Ala Ile Thr Ala Leu Lys Leu Gln Met Asp Glu Glu Leu Lys Thr
                2005                2010                2015
Ala Arg Thr Asn Ala Asp Val Asp Ala Val Leu Lys Arg Phe Asn Val
            2020                2025                2030
Ala Leu Ser Asp Ile Glu Ala Val Ile Thr Glu Lys Glu Asn Ser Leu
        2035                2040                2045
Leu Arg Ile Asp Asn Ile Ala Gln Gln Thr Tyr Ala Lys Phe Lys Ala
    2050                2055                2060
Ile Ala Thr Pro Glu Gln Leu Ala Lys Val Lys Val Leu Ile Asp Gln
2065                2070                2075                2080
Tyr Val Ala Asp Gly Asn Arg Met Ile Asp Glu Asp Ala Thr Leu Asn
                2085                2090                2095
Asp Ile Lys Gln His Thr Gln Phe Ile Val Asp Glu Ile Leu Ala Ile
            2100                2105                2110
Lys Leu Pro Ala Glu Ala Thr Lys Val Ser Pro Lys Glu Ile Gln Pro
        2115                2120                2125
Ala Pro Lys Val Cys Thr Pro Ile Lys Lys Glu Glu Thr His Glu Ser
    2130                2135                2140
Arg Lys Val Glu Lys Glu Leu Pro Asn Thr Gly Ser Glu Gly Met Asp
2145                2150                2155                2160
Leu Pro Leu Lys Glu Phe Ala Leu Ile Thr Gly Ala Ala Leu Leu Ala
                2165                2170                2175
Arg Arg Arg Thr Lys Asn Glu Lys Glu Ser
            2180                2185

<210> 75
<211> 773

<212> PRT
<213> Staphylococcus aureus

<400> 75
Glu Glu Asn Ser Val Gln Asp Val Lys Asp Ser Asn Thr Asp Asp Glu
1               5                   10                  15
Leu Ser Asp Ser Asn Asp Gln Ser Ser Asp Glu Glu Lys Asn Asp Val
            20                  25                  30
Ile Asn Asn Asn Gln Ser Ile Asn Thr Asp Asp Asn Asn Gln Ile Ile
        35                  40                  45
Lys Lys Glu Glu Thr Asn Asn Tyr Asp Gly Ile Glu Lys Arg Ser Glu
    50                  55                  60
Asp Arg Thr Glu Ser Thr Thr Asn Val Asp Glu Asn Glu Ala Thr Phe
65                  70                  75                  80
Leu Gln Lys Thr Pro Gln Asp Asn Thr His Leu Thr Glu Glu Glu Val
                85                  90                  95
Lys Glu Ser Ser Ser Val Glu Ser Ser Asn Ser Ser Ile Asp Thr Ala
            100                 105                 110
Gln Gln Pro Ser His Thr Thr Ile Asn Arg Glu Glu Ser Val Gln Thr
        115                 120                 125
Ser Asp Asn Val Glu Asp Ser His Val Ser Asp Phe Ala Asn Ser Lys
    130                 135                 140
Ile Lys Glu Ser Asn Thr Glu Ser Gly Lys Glu Glu Asn Thr Ile Glu
145                 150                 155                 160
Gln Pro Asn Lys Val Lys Glu Asp Ser Thr Thr Ser Gln Pro Ser Gly
                165                 170                 175
Tyr Thr Asn Ile Asp Glu Lys Ile Ser Asn Gln Asp Glu Leu Leu Asn
            180                 185                 190
Leu Pro Ile Asn Glu Tyr Glu Asn Lys Ala Arg Pro Leu Ser Thr Thr
        195                 200                 205
Ser Ala Gln Pro Ser Ile Lys Arg Val Thr Val Asn Gln Leu Ala Ala
    210                 215                 220
Glu Gln Gly Ser Asn Val Asn His Leu Ile Lys Val Thr Asp Gln Ser
225                 230                 235                 240
Ile Thr Glu Gly Tyr Asp Asp Ser Glu Gly Val Ile Lys Ala His Asp
                245                 250                 255
Ala Glu Asn Leu Ile Tyr Asp Val Thr Phe Glu Val Asp Asp Lys Val
            260                 265                 270
Lys Ser Gly Asp Thr Met Thr Val Asp Ile Asp Lys Asn Thr Val Pro
        275                 280                 285
Ser Asp Leu Thr Asp Ser Phe Thr Ile Pro Lys Ile Lys Asp Asn Ser
    290                 295                 300
Gly Glu Ile Ile Ala Thr Gly Thr Tyr Asp Asn Lys Asn Lys Gln Ile
305                 310                 315                 320
Thr Tyr Thr Phe Thr Asp Tyr Val Asp Lys Tyr Glu Asn Ile Lys Ala
                325                 330                 335
His Leu Lys Leu Thr Ser Tyr Ile Asp Lys Ser Lys Val Pro Asn Asn
            340                 345                 350
Asn Thr Lys Leu Asp Val Glu Tyr Lys Thr Ala Leu Ser Ser Val Asn
        355                 360                 365
Lys Thr Ile Thr Val Glu Tyr Gln Arg Pro Asn Glu Asn Arg Thr Ala
    370                 375                 380
Asn Leu Gln Ser Met Phe Thr Asn Ile Asp Thr Lys Asn His Thr Val
385                 390                 395                 400
Glu Gln Thr Ile Tyr Ile Asn Pro Leu Arg Tyr Ser Ala Lys Glu Thr
                405                 410                 415
Asn Val Asn Ile Ser Gly Asn Gly Asp Glu Gly Ser Thr Ile Ile Asp
            420                 425                 430
Asp Ser Thr Ile Ile Lys Val Tyr Lys Val Gly Asp Asn Gln Asn Leu
        435                 440                 445
Pro Asp Ser Asn Arg Ile Tyr Asp Tyr Ser Glu Tyr Glu Asp Val Thr
    450                 455                 460
Asn Asp Asp Tyr Ala Gln Leu Gly Asn Asn Asn Asp Val Asn Ile Asn
465                 470                 475                 480
Phe Gly Asn Ile Asp Ser Pro Tyr Ile Ile Lys Val Ile Ser Lys Tyr

485                 490                 495
Asp Pro Asn Lys Asp Asp Tyr Thr Thr Ile Gln Gln Thr Val Thr Met
            500                 505                 510
Gln Thr Thr Ile Asn Glu Tyr Thr Gly Glu Phe Arg Thr Ala Ser Tyr
        515                 520                 525
Asp Asn Thr Ile Ala Phe Ser Thr Ser Ser Gly Gln Gly Gln Gly Asp
    530                 535                 540
Leu Pro Pro Glu Lys Thr Tyr Lys Ile Gly Asp Tyr Val Trp Glu Asp
545                 550                 555                 560
Val Asp Lys Asp Gly Ile Gln Asn Thr Asn Asp Asn Glu Lys Pro Leu
                565                 570                 575
Ser Asn Val Leu Val Thr Leu Thr Tyr Pro Asp Gly Thr Ser Lys Ser
            580                 585                 590
Val Arg Thr Asp Glu Asp Gly Lys Tyr Gln Phe Asp Gly Leu Lys Asn
        595                 600                 605
Gly Leu Thr Tyr Lys Ile Thr Phe Glu Thr Pro Glu Gly Tyr Thr Pro
    610                 615                 620
Thr Leu Lys His Ser Gly Thr Asn Pro Ala Leu Asp Ser Glu Gly Asn
625                 630                 635                 640
Ser Val Trp Val Thr Ile Asn Gly Gln Asp Asp Met Thr Ile Asp Ser
                645                 650                 655
Gly Phe Tyr Gln Thr Pro Lys Tyr Ser Leu Gly Asn Tyr Val Trp Tyr
            660                 665                 670
Asp Thr Asn Lys Asp Gly Ile Gln Gly Asp Asp Glu Lys Gly Ile Ser
        675                 680                 685
Gly Val Lys Val Thr Leu Lys Asp Glu Asn Gly Asn Ile Ile Ser Thr
    690                 695                 700
Thr Thr Thr Asp Glu Asn Gly Lys Tyr Gln Phe Asp Asn Leu Asn Ser
705                 710                 715                 720
Gly Asn Tyr Ile Val His Phe Asp Lys Pro Ser Gly Met Thr Gln Thr
                725                 730                 735
Thr Thr Asp Ser Gly Asp Asp Asp Glu Gln Asp Ala Asp Gly Glu Glu
            740                 745                 750
Val His Val Thr Ile Thr Asp His Asp Asp Phe Ser Ile Asp Asn Gly
        755                 760                 765
Tyr Tyr Asp Asp Glu
    770

<210> 76
<211> 2886
<212> DNA
<213> Staphylococcus aureus

<400> 76
gtgaaaagca atcttagata cggcataaga aaacacaaat tgggagcggc ctcagtattc 60
ttaggaacaa tgatcgttgt tggaatggga caagaaaaag aagctgcagc atcggaacaa 120
aacaatacta cagtagagga aagtgggagt tcagctactg aaagtaaagc aagcgaaaca 180
caaacaacta caaataacgt taatacaata gatgaaacac aatcatacag cgcgacatca 240
actgagcaac catcaaaatc aactcaagta acaacagaag aagcaccaac aactgtgcaa 300
gcaccaaaag tagaaaccga aatgaaatca caagaagatt taccatcaga aaaagttgct 360

gataaggaaa ctacaggaac tcaagttgac atagctcaac caagtaacgt ctcagaaatt 420
aaaccaagaa tgaaaagatc agctgacgtt acagcagttt cagagaaaga agtagcggaa 480
gaagctaaag cgacaggtac agatgtaaca aataaagtgg aagttactga aagctcttta 540
gaaggacata ataaagattc gaatattgtt aatccgcata atgctcaaag agtaacttta 600
aaatacaaat ggaaatttgg agaaggaatt aaggcaggag attattttga tttcacatta 660
agtgataatg ttgaaacaca tggtatatca acactgcgta aagttccgga gataaaaagt 720
tcaacagaag ataaagttat ggcaaatggt caagttataa atgaacgtac aattcgctat 780
acatttactg attatataaa taacaaaaaa gatttaactg ctgaattaaa cttaaaccta 840
ttcattgacc caacaacagt gacaaagcaa gggagtcaaa aagttgaagt aacactaggt 900
caaaataaag tctcaaaaga atttgatatc aaatatttag acggcgttaa agatagaatg 960
ggtgttactg ttaatggtcg tattgatact ttgaataaag aagagggtaa atttagccat 1020
tttgcatatg tgaagcctaa caaccagtcg ttaacttctg tcacagtaac tggtcaagta 1080
acatctggat ataaacaaag tgctaataat ccaacagtca aagtatataa acacattggt 1140

tcagatgaat tagctgaaag tgtttatgca aagcttgatg ataccagtaa atttgaagat 1200
gtgactgaaa aagtaaatct atcttacaca agtaatggtg ggtacacatt gaaccttggc 1260
gatttagata attcgaaaga ctatgtaatt aaatatgaag gtgaatatga tcaaaatgct 1320
aaggatctaa atttccgaac acatctttca ggatatcata aatactaccc atactatcct 1380
tattacccgt attatccagt tcaattaact tggaacaacg gtgttgcatt ttactctaat 1440
aatgctaaag gcgatggtaa agataaacca aatgatccta tcattgagaa gagtgaacca 1500
attgatttag acattaaatc agagccacca gtggagaagc atgaattgac tggtacaatc 1560
gaagaaagta acgattctaa gccaattgat tttgaatatc atacagctgt tgaaggtgca 1620
gaaggtcatg cagaaggtat tattgaaact gaagaagatt ctattcatgt ggattttgaa 1680
gaatctacac atgaaaattc aaaacatcac gctgatgttg ttgaatatga agaggataca 1740
aacccaggtg gtggccaagt aacaactgag tctaacttag ttgaatttga cgaagagtct 1800
acaaaaggta ttgtaactgg cgcagtgagc gaccatacaa cagttgaaga tacgaaagaa 1860
tatacaactg aaagtaatct gattgaatta gtggatgaat tacctgaaga acatggtcaa 1920
gcacaagggc caatcgagga aattactgaa aacaatcatc atatttctca ttctggttta 1980
ggaactgaaa atggtcacgg taattatggc gtgattgatg aaatcgaaga aaatagccac 2040
gttgatatta agagtgaatt aggttatgaa ggtggccaaa atagcggtaa tcagtcattc 2100
gaggaagaca cagaagaaga taaacctaaa tatgaacaag gtggtaatat cgtagatatc 2160
gatttcgaca gtgtacctca aattcatggt caaaataatg gtaaccagtc attcgaggaa 2220
gacacagaag aagacaagcc taagtatgaa caaggtggta acatcattga tatcgacttc 2280
gacagtgtgc cacaaattca tggattcaat aagcataatg aaattattga agaagataca 2340
aacaaagata aacctaatta tcaatttggt ggacacaaca gtgttgattt tgaagaagat 2400
acacttccaa aagtaagtgg tcaaaatgaa ggtcaacaaa cgattgaaga agatacaacg 2460
ccgccaacac cgccaacacc agaggtacca agtgagccgg aaacaccaac accaccaaca 2520
ccagaagtac cgagtgagcc aggcgaacca acgccaccaa aaccggaagt accaagtgag 2580
ccggaaacac cagtaccacc aacaccagag gtaccatctg aacctggtaa accagtacca 2640
cctgctaaag aagaacctaa aaaaccttct aaaccagtgg aacaaggtaa ggtagtaaca 2700
cctgttattg aaatcaatga aaaggttaaa gcagtggcac caactaaaca aaaacaatct 2760
aagaaatctg aactacctga aacaggtgga gaagaatcaa caaacaaagg tatgttgttc 2820
ggcggattat tcagcattct aggtttagta ttattacgca gaaataaaaa gaataacaaa 2880
gcataa 2886

<210> 77
<211> 1737
<212> DNA
<213> Staphylococcus aureus

<400> 77
atgaaattta agtcattgat tacaacaaca ttagcattag gcgttatagc atcaacagga 60
gcaaacttta atactaacga agcatctgcc gcagctaagc cattagataa atcatcaagt 120
acattacacc atggacattc taacatccag attccatata caattactgt gaacggtaca 180
agccaaaaca ttttatcaag cttaacattt aataagaatc aaaatattag ttataaagat 240
atagagaata aagttaaatc agttttatac tttaatagag gtattagtga tatcgattta 300
agactttcaa agcaagcgga atatacggtt cattttaaaa atggaacaaa aagagttatc 360
gatttgaaat caggtatcta cacagctgac ttaatcaata caagtgacat taaagctatc 420
agtgttaacg tagatactaa aaagcaacct aaagataaag ctaaagcaaa tgttcaagtg 480
ccatatacaa tcacagtgaa cggcacaagc caaaacattt tatcaaacct aacatttaat 540
aaaaatcaaa atattagtta caaagattta gagggtaaag ttaaatcagt tttagaatca 600
aatagaggta ttactgatgt tgatttaaga ctttcgaagc aagcgaaata tacagttaat 660
tttaaaaatg gaacgaagaa agttatcgat ttgaaatcag gtatttacac agcgaattta 720
atcaattcaa gtgatattaa aagtatcaat attaacgtag atacaaaaaa acatatcgaa 780
aataaagcta aaagaaacta tcaagttcca tattcaatta atctaaatgg tacatctaca 840
aacattttat cgaatctttc attttcaaat aaaccttgga caaattacaa aaatttaact 900
agtcaaataa aatcagtact gaagcatgat agaggtatta gtgaacaaga tttaaaatat 960
gctaagaaag cttattatac tgtttatttt aaaaatggtg gtaaaagaat cttacagtta 1020
aattcaaaaa attacacagc aaacttagtt catgcgaaag atgttaagag aattgaaatt 1080
actgttaaaa caggaactaa agcgaaagca gacagatatg taccatacac aattgcagta 1140
aatggcacat caacaccaat tttatcaaaa ctaaaaattt cgaataaaca attaattagt 1200
tacaaatatt taaacgacaa agtgaaatct gtattaaaaa gtgaaagagg tatcagtgat 1260
cttgacttaa aatttgcgaa acaagcaaaa tatacagtat atttcaaaaa tggaaagaaa 1320
caagtagtga atttaaaatc agacatcttt acacctaatt tatttagtgc caaagatatt 1380
aaaaagattg atattgatgt aaaacaatac actaaatcaa aaaaaaaaat aaataaatct 1440
aataatgtga aattcccagt aacaataaat aaatttgaaa acatagtttc aaatgaattt 1500
gtgttctata atgcaagcaa aattacaatt aatgatttaa gtataaaact taaatcagca 1560
atggcaaatg atcaagggat aactaaacat gacataggac ttgctgaacg cgcagtgtat 1620
aaagtgtatt ttaaaaatgg ttcgtcaaaa tatgtagact taaaaactga gtataaagat 1680

gaaagagtat ttaaagcaac tgacattaaa aaggtagata ttgaacttaa attctaa 1737

<210> 78
<211> 2688
<212> DNA
<213> Staphylococcus aureus

<400> 78
atgaacaaac atcacccaaa attaaggtct ttctattcta ttagaaaatc aactctaggc 60
gttgcatcgg tcattgtcag tacactattt ttaattactt ctcaacatca agcacaagca 120
gcagaaaata caaatacttc agataaaatc tcggaaaatc aaaataataa tgcaactaca 180
actcagccac ctaaggatac aaatcaaaca caacctgcta cgcaaccagc aaacactgcg 240
aaaaactatc ctgcagcgga tgaatcactt aaagatgcaa ttaaagatcc tgcattagaa 300
aataaagaac atgatatagg tccaagagaa caagtcaatt tccagttatt agataaaaac 360
aatgaaacgc agtactatca ctttttcagc atcaaagatc cagcagatgt gtattacact 420
aaaaagaaag cagaagttga attagacatc aatactgctt caacatggaa gaagtttgaa 480
gtctatgaaa acaatcaaaa attgccagtg agacttgtat catatagtcc tgtaccagaa 540
gaccatgcct atattcgatt cccagtttca gatggcacac aagaattgaa aattgtttct 600
tcgactcaaa ttgatgatgg agaagaaaca aattatgatt atactaaatt agtatttgct 660
aaacctattt ataacgatcc ttcacttgta aaatcagata caaatgatgc agtagtaacg 720
aatgatcaat caagttcagt cgcaagtaat caaacaaaca cgaatacatc taatcaaaat 780
atatcaacga tcaacaatgc taataatcaa ccgcaggcaa cgaccaatat gagtcaacct 840
gcacaaccaa aatcgtcaac gaatgcagat caagcgtcaa gccaaccagc tcatgaaaca 900
aattctaatg gtaatactaa cgataaaacg aatgagtcaa gtaatcagtc ggatgttaat 960
caacagtatc caccagcaga tgaatcacta caagatgcaa ttaaaaaccc ggctatcatc 1020
gataaagaac atacagctga taattggcga ccaattgatt ttcaaatgaa aaatgataaa 1080
ggtgaaagac agttctatca ttatgctagt actgttgaac cagcaactgt catttttaca 1140
aaaacaggac caataattga attaggttta aagacagctt caacatggaa gaaatttgaa 1200
gtttatgaag gtgacaaaaa gttaccagtc gaattagtat catatgattc tgataaagat 1260
tatgcctata ttcgtttccc agtatctaat ggtacgagag aagttaaaat tgtgtcatct 1320
attgaatatg gtgagaacat ccatgaagac tatgattata cgctaatggt ctttgcacag 1380
cctattacta ataacccaga cgactatgtg gatgaagaaa catacaattt acaaaaatta 1440
ttagctccgt atcacaaagc taaaacgtta gaaagacaag tttatgaatt agaaaaatta 1500
caagagaaat tgccagaaaa atataaggcg gaatataaaa agaaattaga tcaaactaga 1560
gtagagttag ctgatcaagt taaatcagca gtgacggaat ttgaaaatgt tacacctaca 1620
aatgatcaat taacagattt acaagaagcg cattttgttg tttttgaaag tgaagaaaat 1680
agtgagtcag ttatggacgg ctttgttgaa catccattct atacagcaac tttaaatggt 1740
caaaaatatg tagtgatgaa aacaaaggat gacagttact ggaaagattt aattgtagaa 1800
ggtaaacgtg tcactactgt ttctaaagat cctaaaaata attctagaac gctgattttc 1860
ccatatatac ctgacaaagc agtttacaat gcgattgtta aagtcgttgt ggcaaacatt 1920
ggttatgaag gtcaatatca tgtcagaatt ataaatcagg atatcaatac aaaagatgat 1980
gatacatcac aaaataacac gagtgaaccg ctaaatgtac aaacaggaca agaaggtaag 2040
gttgctgata cagatgtagc tgaaaatagc agcactgcaa caaatcctaa agatgcgtct 2100
gataaagcag atgtgataga accagagtct gacgtggtta aagatgctga taataatatt 2160
gataaagatg tgcaacatga tgttgatcat ttatccgata tgtcggataa taatcacttc 2220
gataaatatg atttaaaaga aatggatact caaattgcca aagatactga tagaaatgtg 2280
gataaagatg ccgataatag cgttggtatg tcatctaatg tcgatactga taaagactct 2340
aataaaaata aagacaaagt catacagctg aatcatattg ccgataaaaa taatcatact 2400
ggaaaagcag caaagcttga cgtagtgaaa caaaattata ataatacaga caaagttact 2460
gacaaaaaaa caactgaaca tctgccgagt gatattcata aaactgtaga taaaacagtg 2520
aaaacaaaag aaaaagccgg cacaccatcg aaagaaaaca aacttagtca atctaaaatg 2580
ctaccaaaaa ctggagaaac aacttcaagc caatcatggt ggggcttata tgcgttatta 2640
ggtatgttag ctttattcat tcctaaattc agaaaagaat ctaaataa 2688

<210> 79
<211> 2238
<212> DNA
<213> Staphylococcus aureus

<400> 79
gctgagacga cacaagatca aactactaat aaaaacgttt tagatagtaa taaagttaaa 60
gcaactactg aacaagcaaa agctgaggta aaaaatccaa cgcaaaacat ttctggcact 120
caagtatatc aagaccctgc tattgtccaa ccaaaaacag caaataacaa aacaggcaat 180
gctcaagtaa gtcaaaaagt tgatactgca caagtaaatg gtgacactcg tgctaatcaa 240
tcagcgacta caaataatac gcagcctgtt gcaaagtcaa caagcactac agcacctaaa 300

actaacacta atgttacaaa tgctggttat agtttagttg atgatgaaga tgataattca 360
gaaaatcaaa ttaatccaga attaattaaa tcagctgcta aacctgcagc tcttgaaacg 420
caatataaaa ccgcagcacc taaagctgca actacatcag cacctaaagc taaaactgaa 480
gcgacaccta aagtaactac ttttagcgct tcagcacaac caagatcagt tgctgcaaca 540
ccaaaaacga gtttgccaaa atataaacca caagtaaact cttcaattaa cgattacatt 600
tgtaaaaata acttaaaagc acctaaaatt gaagaagatt atacatctta cttccctaaa 660
tacgcatacc gtaacggcgt aggtcgtcct gaaggtatcg tagttcatga tacagctaat 720
gatcgttcga cgataaatgg tgaaattagt tatatgaaaa ataactatca aaacgcattc 780
gtacatgcat ttgttgatgg ggatcgtata atcgaaacag caccaacgga ttacttatct 840
tggggtgtcg gtgcagtcgg taaccctaga ttcatcaatg ttgaaatcgt acacacacac 900
gactatgctt catttgcacg ttcaatgaat aactatgctg actatgcagc tacacaatta 960
caatattatg gtttaaaacc agacagtgct gagtatgatg gaaatggtac agtatggact 1020
cactacgctg taagtaaata tttaggtggt actgaccatg ccgatccaca tggatattta 1080
agaagtcata attatagtta tgatcaatta tatgacttaa ttaatgaaaa atatttaata 1140
aaaatgggta aagtggcgcc atggggtacg caatctacaa ctacccctac tacaccatca 1200
aaaccaacaa caccgtcgaa accatcaact ggtaaattaa cagttgctgc aaacaatggt 1260
gtcgcacaaa tcaaaccaac aaatagtggt ttatatacta ctgtatacga caaaactggt 1320
aaagcaacta atgaagttca aaaaacattt gctgtatcta aaacagctac attaggtaat 1380
caaaaattct atcttgttca agattacaat tctggtaata aatttggttg ggttaaagaa 1440
ggcgatgtgg tttacaacac agctaaatca cctgtaaatg taaatcaatc atattcaatc 1500
aaacctggta cgaaacttta tacagtacct tggggtacat ctaaacaagt tgctggtagt 1560
gtgtctggct ctggaaacca aacatttaag gcttcaaagc aacaacaaat tgataaatca 1620
atttatttat atggctctgt gaatggtaaa tctggttggg taagtaaagc atatttagtt 1680
gatactgcta aacctacgcc tacaccaaca cctaagccat caacacctac aacaaataat 1740
aaattaacag tttcatcatt aaacggtgtt gctcaaatta atgctaaaaa caatggctta 1800
ttcactacag tttatgacaa aactggtaag ccaacgaaag aagttcaaaa aacatttgct 1860
gtaacaaaag aagcaagttt aggtggaaac aaattctact tagttaaaga ttacaatagt 1920
ccaactttaa ttggttgggt taaacaaggt gacgttattt ataacaatgc aaaatcacct 1980
gtaaatgtaa tgcaaacata tacagtaaaa ccaggcacta aattatattc agtaccttgg 2040
ggcacttata aacaagaagc tggtgcagtt tctggtacag gtaaccaaac ttttaaagcg 2100
actaagcaac aacaaattga taaatctatc tatttatttg gaactgtaaa tggtaaatct 2160
ggttgggtaa gtaaagcata tttagctgta cctgctgcac ctaaaaaagc agtagcacaa 2220
ccaaaaacag ctgtaaaa 2238

<210> 80
<211> 1443
<212> DNA
<213> Staphylococcus aureus

<400> 80
gcttatactg ttactaaacc acaaacgact caaacagtta gcaagattgc tcaagttaaa 60
ccaaacaaca ctggtattcg tgcttctgtt tatgaaaaaa cagcgaaaaa cggtgcgaaa 120
tatgcagacc gtacgttcta tgtaacaaaa gagcgtgctc atggtaatga aacgtatgta 180
ttattaaaca atacaagcca taacatccca ttaggttggt tcaatgtaaa agacttaaat 240
gttcaaaact taggcaaaga agttaaaacg actcaaaaat atactgttaa taaatcaaat 300
aacggcttat caatggttcc ttggggtact aaaaaccaag tcattttaac aggcaataac 360
attgctcaag gtacatttaa tgcaacgaaa caagtatctg taggcaaaga tgtttattta 420
tacggtacta ttaataaccg cactggttgg gtaaatgcaa aagatttaac tgcaccaact 480
gctgtgaaac caactacatc agctgccaaa gattataact acacttatgt aattaaaaat 540
ggtaatggtt attactatgt aacaccaaat tctgatacag ctaaatactc attaaaagca 600
tttaatgaac aaccattcgc agttgttaaa gaacaagtca ttaatggaca aacttggtac 660
tatggtaaat tatctaacgg taaattagca tggattaaat caactgattt agctaaagaa 720
ttaattaagt ataatcaaac aggtatgaca ttaaaccaag ttgctcaaat acaagctggt 780
ttacaatata aaccacaagt acaacgtgta ccaggtaagt ggacagatgc taaatttaat 840
gatgttaagc atgcaatgga tacgaagcgt ttagctcaag atccagcatt aaaatatcaa 900
ttcttacgct tagaccaacc acaaaatatt tctattgata aaattaatca attcttaaaa 960
ggtaaaggtg tattagaaaa ccaaggtgct gcatttaaca aagctgctca aatgtatggc 1020
attaatgaag tttatcttat ctcacatgcc ctattagaaa caggtaacgg tacttctcaa 1080
ttagcgaaag gtgcagatgt agtgaacaac aaagttgtaa ctaactcaaa cacgaaatac 1140
cataacgtat ttggtattgc tgcatatgat aacgatcctt tacgtgaagg tattaaatat 1200
gctaaacaag ctggttggga cacagtatca aaagcaatcg ttggtggtgc taaattcatc 1260
ggcaactcat atgtaaaagc tggtcaaaat acactttaca aaatgagatg gaatcctgca 1320
catccaggaa cacaccaata tgctacagat gtagattggg ctaacatcaa tgctaaaatc 1380
atcaaaggct actatgataa aattggcgaa gtcggcaaat acttcgacat cccacaatat 1440
aaa 1443

<210> 81
<211> 2118
<212> DNA
<213> Staphylococcus aureus

<400> 81
gatcgtgtat tagcctcaca tccagatgtt gcgacaatac gtcaaaacgt gacagcagcg 60
aatgccgcta aatcagcact tgatcaagca cgtaatggct taacagtcga taaagcgcct 120
ttagaaaatg cgaaaaatca actacaacat agtattgaca cgcaaacaag tacaactggt 180
atgacacaag actctataaa tgcatacaat gcgaagttaa cagctgcacg taataagatt 240
caacaaatca atcaagtatt agcaggttca ccgactgtag aacaaattaa tacaaatacg 300
tctacagcaa atcaagctaa atctgattta gatcatgcac gtcaagcttt aacaccagat 360
aaagcgccgc ttcaaactgc gaaaacgcaa ttagaacaaa gcattaatca accaacggat 420
acaacaggta tgacgaccgc ttcgttaaat gcgtacaacc aaaaattaca agcagcgcgt 480
caaaagttaa ctgaaattaa tcaagtgttg aatggcaacc caactgtcca aaatatcaat 540
gataaagtga cagaggcaaa ccaagctaag gatcaattaa atacagcacg tcaaggttta 600
acattagata gacagccagc gttaacaaca ttacatggtg catctaactt aaaccaagca 660
caacaaaata atttcacgca acaaattaat gctgctcaaa atcatgctgc gcttgaaaca 720
attaagtcta acattacggc tttaaatact gcgatgacga aattaaaaga cagtgttgcg 780
gataataata caattaaatc agatcaaaat tacactgacg caacaccagc taataaacaa 840
gcgtatgata atgcagttaa tgcggctaaa ggtgtcattg gagaaacgac taatccaacg 900
atggatgtta acacagtgaa ccaaaaagca gcatctgtta aatcgacgaa agatgcttta 960
gatggtcaac aaaacttaca acgtgcgaaa acagaagcaa caaatgcgat tacgcatgca 1020
agtgatttaa accaagcaca aaagaatgca ttaacacaac aagtgaatag tgcacaaaac 1080
gtgcaagcag taaatgatat taaacaaacg actcaaagct taaatactgc tatgacaggt 1140
ttaaaacgtg gcgttgctaa tcataaccaa gtcgtacaaa gtgataatta tgtcaacgca 1200
gatactaata agaaaaatga ttacaacaat gcatacaacc atgcgaatga cattattaat 1260
ggtaatgcac aacatccagt tataacacca agtgatgtta acaatgcttt atcaaatgtc 1320
acaagtaaag aacatgcatt gaatggtgaa gctaagttaa atgctgcgaa acaagaagcg 1380
aatactgcat taggtcattt aaacaattta aataatgcac aacgtcaaaa cttacaatcg 1440
caaattaatg gtgcgcatca aattgatgca gttaatacaa ttaagcaaaa tgcaacaaac 1500
ttgaatagtg caatgggtaa cttaagacaa gctgttgcag ataaagatca agtgaaacgt 1560
acagaagatt atgcggatgc agatacagct aaacaaaatg catataacag tgcagtttca 1620
agtgccgaaa caatcattaa tcaaacaaca aatccaacga tgtctgttga tgatgttaat 1680
cgtgcaactt cagctgttac ttctaataaa aatgcattaa atggttatga aaaattagca 1740
caatctaaaa cagatgctgc aagagcaatt gatgcattac cacatttaaa taatgcacaa 1800
aaagcagatg ttaaatctaa aattaatgct gcatcaaata ttgctggcgt aaatactgtt 1860
aaacaacaag gtacagattt aaatacagcg atgggtaact tgcaaggtgc aatcaatgat 1920
gaacaaacga cgcttaatag tcaaaactat caagatgcga cacctagtaa gaaaacagca 1980
tacacaaatg cggtacaagc tgcgaaagat attttaaata aatcaaatgg tcaaaataaa 2040
acgaaagatc aagttactga agcgatgaat caagtgaatt ctgctaaaaa taacttagat 2100
ggtacgcgtt tattagat 2118

<210> 82
<211> 726
<212> DNA
<213> Staphylococcus aureus

<400> 82
gcttctacac aacatacagt acaatctggt gaatcattat ggagtattgc tcaaaaatac 60
aacacttcag tagagagtat taaacaaaat aaccaattag ataacaactt ggtattccct 120
ggtcaagtta tctcagtagg tggaagtgat gcacaaaata cgtcaaacac ttctccacaa 180
gctggttcag catcatctca tactgtacaa gctggtgaat cattaaatat cattgctagc 240
agatatggtg tttcagttga tcaattaatg gcagccaata acttacgtgg ttatttaatt 300
atgcctaacc aaacattaca aattcctaat ggtggatcag gtggtacaac accaacagct 360
acaacaggta gcaatggcaa tgcatcatct tttaatcacc aaaatttata cactgctggt 420
caatgtacat ggtacgtatt tgaccgtcgt gctcaagctg gtagtccaat tagcacatat 480
tggtcagacg ctaagtattg ggctggtaac gcagctaatg atggttacca agtaaacaac 540
acaccatcag ttggttcaat tatgcaaagc acacctggtc catatggtca tgttgcttat 600
gttgaacgtg tcaatggtga tggtagtatc ttgatttctg aaatgaatta cacatatggt 660
ccatacaata tgaactaccg tacaattcca gcttcagaag tttctagcta tgcattcatc 720
cattaa 726

<210> 83

<211> 2988
<212> DNA
<213> Staphylococcus aureus

<400> 83
atgaataata aaaagacagc aacaaataga aaaggcatga taccaaatcg attaaacaaa 60
ttttcgataa gaaagtattc tgtaggtact gcttcaattt tagtagggac aacattgatt 120
tttgggttaa gtggtcatga agctaaagcg gcagaacata cgaatggaga attaaatcaa 180
tcaaaaaatg aaacgacagc cccaagtgag aataaaacaa ctaaaaaagt tgatagtcgt 240
caactaaaag acaatacgca aactgcaact gcagatcagc ctaaagtgac aatgagtgat 300
agtgcaacag ttaaagaaac tagtagtaac atgcaatcac cacaaaacgc tacagctaat 360
caatctacta caaaaactag caatgtaaca acaaatgata aatcatcaac tacatatagt 420
aatgaaactg ataaaagtaa tttaacacaa gcaaaagatg tttcaactac acctaaaaca 480
acgactatta aaccaagaac tttaaatcgc atggcagtga atactgttgc agctccacaa 540
caaggaacaa atgttaatga taaagtacat ttttcaaata ttgacattgc gattgataaa 600
ggacatgtta atcagactac tggtaaaact gaattttggg caacttcaag tgatgtttta 660
aaattaaaag caaattacac aatcgatgat tctgttaaag agggcgatac atttactttt 720
aaatatggtc aatatttccg tccaggatca gtaagattac cttcacaaac tcaaaattta 780
tataatgccc aaggtaatat tattgcaaaa ggtatttatg atagtacaac aaacacaaca 840
acatatactt ttacgaacta tgtagatcaa tatacaaatg ttagaggtag ctttgaacaa 900
gttgcatttg cgaaacgtaa aaatgcaaca actgataaaa cagcttataa aatggaagta 960
actttaggta atgatacata tagcgaagaa atcattgtcg attatggtaa taaaaaagca 1020
caaccgctta tttcaagtac aaactatatt aacaatgaag atttatcgcg taatatgact 1080
gcatatgtaa atcaacctaa aaatacatat actaaacaaa cgtttgttac taatttaact 1140
ggatataaat ttaatccaaa tgcaaaaaac ttcaaaattt acgaagtgac agatcaaaat 1200
caatttgtgg atagtttcac ccctgatact tcaaaactta aagatgttac tgatcaattc 1260
gatgttattt atagtaatga taataaaaca gctacagtcg atttaatgaa aggccaaaca 1320
agcagcaata aacaatacat cattcaacaa gttgcttatc cagataatag ttcaacagat 1380
aatggaaaaa ttgattatac tttagacact gacaaaacta aatatagttg gtcaaatagt 1440
tattcaaatg tgaatggctc atcaactgct aatggcgacc aaaagaaata taatctaggt 1500
gactatgtat gggaagatac aaataaagat ggtaaacaag atgccaatga aaaagggatt 1560
aaaggtgttt atgtcattct taaagatagt aacggtaaag aattagatcg tacgacaaca 1620
gatgaaaatg gtaaatatca gttcactggt ttaagcaatg gaacttatag tgtagagttt 1680
tcaacaccag ccggttatac accgacaact gcaaatgtag gtacagatga tgctgtagat 1740
tctgatggac taactacaac aggtgtcatt aaagacgctg acaacatgac attagatagt 1800
ggattctaca aaacaccaaa atatagttta ggtgattatg tttggtacga cagtaataaa 1860
gatggtaaac aagattcgac tgaaaaagga attaaaggtg ttaaagttac tttgcaaaac 1920
gaaaaaggcg aagtaattgg tacaactgaa acagatgaaa atggtaaata ccgctttgat 1980
aatttagata gtggtaaata caaagttatc tttgaaaaac ctgctggctt aactcaaaca 2040
ggtacaaata caactgaaga tgataaagat gccgatggtg gcgaagttga tgtaacaatt 2100
acggatcatg atgatttcac acttgataat ggctactacg aagaagaaac atcagatagc 2160
gactcagatt ctgacagcga ttcagactca gatagcgact cagattcaga tagcgactca 2220
gattcagaca gcgattcaga cagcgactca gactcagata gcgattcaga ttcagacagc 2280
gactcagact cagacagcga ttcagactcg gatagcgact cagactcaga tagcgactca 2340
gattcggata gcgactcaga ctcagatagc gattcagatt cagatagcga ttcggactca 2400
gacagtgatt cagattcaga ctcagatagc gactcagatt ctgacagcga ttcagactca 2460
gacagcgact cagactcaga cagtgattca gattcagaca gcgactcaga ttcagatagc 2520
gactcagact cagatagcga ctcagattca gatagcgatt cggactcaga caacgactca 2580
gattcagata gcgattcaga ttcagatagc gactcagatt cggacagcga ttcagactca 2640
gatagcgatt cagactcaga cagcgattca gattcagata gcgactcaga ctcagatagc 2700
gactcagact cggatagcga ttcagattca gacagcgact cagattcaga tagcgattcg 2760
gactcagaca acgactcaga ttcagatagc gattcagatt cagatgcagg taaacatact 2820
ccggctaaac caatgagtac ggttaaagat cagcataaaa cagctaaagc attaccagaa 2880
acaggtagtg aaaataataa ttcaaataat ggcacattat tcggtggatt attcgcggca 2940
ttaggatcat tattgttatt cggtcgtcgt aaaaaacaaa ataaataa 2988

<210> 84
<211> 6561
<212> DNA
<213> Staphylococcus aureus

<400> 84
atgaatttgt taaagaaaaa taaatatagt attaggaagt ataaagtagg catattctct 60
actttaatcg gaacagtttt attactttca aacccaaatg gtgcacaagc cttaactacg 120
gataataatg tacaaagcga tactaatcaa gcaacacctg taaattcaca agataaagat 180

gttgctaata atagaggttt agcaaatagt gcgcagaata cacctaatca atctgcaaca 240
accaatcaag caacgaatca agcattggtt aatcataata atggtagtat agtaaatcaa 300
gctacgccaa catcagtgca atcaagtacg ccttcagcac aaaacaataa tcatacagat 360
ggcaatacaa cagcaactga gacagtgtca aacgctaata ataatgatgt agtgtcgaat 420
aataccgcat taaatgtacc aactaaaaca aatgaaaatg gttcaggagg acatctaact 480
ttaaaggaaa ttcaagaaga tgttcgtcat tcttcaaata aaccagagct agttgcaatt 540
gctgaaccag catctaatag accgaaaaag agaagtagac gtgcggcacc ggcagatcct 600
aatgcaactc cagcagatcc agcggctgca gcggtaggaa acggtggtgc accagttgca 660
attacagcgc catatacgcc aacaactgat cctaatgcca ataatgcagg acaaaatgca 720
cctaacgaag tgctgtcatt tgatgacaat ggtattagac caagtaccaa ccgttctgtg 780
ccaacagtaa acgttgttaa taacttgccg ggcttcacac taatcaatgg tggcaaagta 840
ggggtgttta gtcatgcaat ggtaagaacg agcatgtttg attcaggaga taataagaac 900
tatcaagcac aaggaaatgt aattgcatta ggtcgtatac atggaactga tacgaatgac 960
catggcgatt ttaatggtat cgagaaagca ttaacagtaa atccgaattc tgaattaatc 1020
tttgaattta atacaatgac tactaaaaac ggtcaaggcg caacaaatgt tattatcaaa 1080
aatgctgata ctaatgatac gattgctgaa aagactgttg aaggcggtcc aactttgcgt 1140
ttatttaaag tacctgataa tgtgagaaat ctcaaaattc aatttgtacc taaaaatgac 1200
gcaataacag atgcgcgtgg catttatcaa ctaaaagatg gttacaaata ctatagcttt 1260
gttgactcta tcggacttca ttctgggtca catgtttttg ttgaaagacg aacaatggat 1320
ccaacagcaa caaataataa agagtttact gtaacaacat cattaaagaa taatggtaat 1380
tctggtgctt ctctagatac aaatgacttt gtatatcaag ttcaattacc tgaaggtgtt 1440
gaatatgtga acaattcatt gactaaagat tttccaagta acaattcagg cgttgatgtt 1500
aatgatatga atgttacata tgatgcagca aatcgtgtga taacaattaa aagtactgga 1560
ggaggtacag caaactctcc ggcacgactt atgcctgata aaatactcga tttaagatat 1620
aaattacgtg taaataatgt gccgacacca agaacagtaa catttaacga gacattaacg 1680
tataaaacat atacacaaga tttcattaat tcagctgcag aaagtcatac tgtaagtaca 1740
aatccatata ctatcgatat catcatgaat aaagatgcat tacaagccga agttgacaga 1800
cgtattcaac aagctgatta tacatttgcg tcattagata tctttaatgg tctgaaacga 1860
cgcgcacaaa cgattttaga tgaaaatcgt aacaatgtac cattaaataa aagagtttct 1920
caagcatata ttgattcatt aactaatcaa atgcaacata cgttaattcg aagtgttgat 1980
gctgaaaatg cagttaataa aaaagttgac caaatggaag atttagttaa tcaaaatgat 2040
gaattgacag atgaagaaaa acaagcagca atacaagtta tcgaggaaca taaaaatgaa 2100
ataattggta atattggtga ccaaacgact gatgatggcg ttactagaat caaagatcaa 2160
ggtatacaga ccttaagtgg ggatactgca acaccggttg ttaaaccaaa tgctaaaaaa 2220
gcaatacgtg ataaagcaac gaaacaaagg gaaattatca atgcaacacc agatgctact 2280
gaagacgaga ttcaagatgc actaaatcaa ttagctacgg atgaaacaga tgctattgat 2340
aatgttacga atgctactac aaatgctgac gttgaaacag ctaaaaataa tggcatcaat 2400
actattggag cagttgttcc tcaagtaact cataaaaaag ctgcaagaga tgcaattaac 2460
caagcaacag caacgaaaag acaacaaata aatagtaata gagaagcaac tcaggaagag 2520
aaaaatgcag cattgaacga attaactcaa gcaaccaacc atgctttaga acaaatcaat 2580
caagcaacaa caaatgctaa tgttgataac gccaaaggag atggtctaaa tgccattaat 2640
ccaattgctc ctgtaactgt tgttaagcaa gctgcaaggg atgccgtatc acatgatgca 2700
caacaacata tcgcagagat caatgctaat cctgatgcga ctcaagaaga aagacaagca 2760

gcaattgaca aagtgaatgc tgctgtaact gcagcaaaca caaacatttt aaacgctaat 2820
accaatgctg atgttgaaca agtaaagaca aatgcgattc aaggaataca agcaattaca 2880
ccagctacaa aagtaaaaac agatgcaaaa aatgccatcg ataaaagtgc ggaaacgcaa 2940
cataatacga tatttaataa taatgatgcg acgctcgaag aacaacaagc agcacaacaa 3000
ttacttgatc aagctgtagc cacagcgaag caaaatatta atgcagcaga tacgaatcaa 3060
gaagttgcac aagcaaaaga tcagggcaca caaaatatag tagtgattca accggcaaca 3120
caagttaaaa cggatactcg caatgttgta aatgataaag cgcgagaggc gataacaaat 3180
atcaatgcta caactggcgc gactcgagaa gagaaacaag aagcgataaa tcgtgtcaat 3240
acacttaaaa atagagcatt aactgatatt ggtgtgacgt ctactactgc gatggtcaat 3300
agtattagag acgatgcagt caatcaaatc ggcgcagttc aaccgcatgt aacgaagaaa 3360
caaactgcta caggtgtatt aaatgattta gcaactgcta aaaagcaaga aattaatcaa 3420
aacacaaatg caacaactga agaaaagcaa gtggctttaa atcaagtgga tcaagagtta 3480
gcaacggcaa ttaataatat aaatcaagct gatacaaatg cggaagtaga tcaagcgcaa 3540
caattaggta caaaagcaat taatgcgatt cagccaaata ttgttaaaaa acctgcagca 3600
ttagcacaaa tcaatcagca ttataatgct aaattagctg aaatcaatgc tacaccagat 3660
gcaacgaatg atgagaaaaa tgctgcgatc aatactttaa atcaagacag acaacaagct 3720
attgaaagta ttaaacaagc taacacaaat gcagaagtag accaagctgc gacagtagca 3780
gagaataata tcgatgctgt tcaagttgat gtagtaaaaa aacaagcagc gcgagataaa 3840
atcactgctg aagtggcgaa gcgtattgaa gcggttaaac aaacacctaa tgcaactgac 3900
gaagaaaagc aggctgctgt taatcaaatc aatcaactta aagatcaagc aattaatcaa 3960
attaatcaaa accaaacaaa tgatcaggta gacacaacta caaatcaagc ggtaaatgct 4020

atagataatg ttgaagctga agtagtaatt aaaacaaagg caattgcaga tattgaaaaa 4080
gctgttaaag aaaagcaaca gcaaattgat aatagtcttg attcaacaga taatgagaaa 4140
gaagttgctt cacaagcatt agctaaagaa aaagaaaaag cacttgcagc tattgaccaa 4200
gctcaaacga atagtcaggt gaatcaagca gcaacaaatg gtgtatcagc gattaaaatt 4260
attcaacctg aaacaaaagt taaaccagct gcacgtgaaa aaatcaatca aaaagcgaat 4320
gaattacgtg ctaagattaa tcaggataaa gaagcaacag cagaagaaag acaagtagca 4380
ctagataaaa tcaatgaatt tgtaaatcaa gccatgacag atattacgaa taatagaaca 4440
aatcaacaag ttgatgatac aacaagtcaa gcgcttgata gcattgcttt agtgacgcct 4500
gaccatattg ttagagcagc tgctagagat gcagttaagc aacaatatga agctaaaaag 4560
cgcgaaattg agcaagcgga acatgcgact gatgaagaaa aacaagttgc tttaaatcaa 4620
ttagcgaata atgaaaaacg tgcattacaa aacatcgatc aagcaatagc gaataatgat 4680

gtgaaacgtg ttgaaacaaa tggcattgct acactaaaag gtgtacaacc tcatattgta 4740
attaagcctg aagcacaaca agcaataaaa gcaagtgcag aaaatcaagt agaatcaata 4800
aaagatacac cacatgcaac agttgatgaa ttagatgaag cgaatcaatt aattagcgac 4860
acactcaaac aagcgcaaca agaaatagaa aatacaaatc aagatgctgc tgttactgat 4920
gttagaaatc aaacaatcaa ggcaatagag caaataaaac ctaaagtaag acgtaaacga 4980
gctgcgcttg atagcattga agaaaataat aaaaatcaac tcgatgcaat ccgaaatacg 5040
ttggatacta ctcaagatga aagagatgtt gctattgata ctttaaataa aattgtaaat 5100
acaattaaaa atgacattgc acaaaacaaa acgaatgcag aagtggatcg aactgagact 5160
gatggcaacg acaacatcaa agtgatttta cctaaagttc aagttaaacc agcagcgcgt 5220
caatctgttg gtgtaaaagc cgaagctcaa aatgcactaa tcgatcaaag cgatttatca 5280
actgaagaag aaagactagc tgctaaacat ttagtagaac aagcacttaa tcaggctatt 5340
gatcagatca atcatgcaga taagactgcc caagttaatc aagatagtat aaatgctcaa 5400
aatattattt caaaaattaa accagcgaca acagttaaag caacagcatt acaacaaatt 5460
caaaatatcg ctacaaataa aattaattta attaaagcaa ataacgaagc gacagatgaa 5520
gaacaaaata ttgcaatagc acaagttgaa aaagagttaa ttaaagctaa acaacaaatt 5580
gctagtgcag tgactaatgc agatgtggca tatttattgc atgatgagaa aaacgaaatt 5640
cgtgaaatcg aacctgttat taacagaaag gcgtctgctc gagaacaatt gacaacatta 5700
ttcaacgata aaaaacaagc aattgaagcg aatattcaag caacggtaga agaaagaaat 5760
agtatattag cacagttaca aaatatttat gacactgcta ttggacaaat tgatcaagat 5820
cgtagcaatg cacaagttga taaaacagca tcattaaatc tacaaacaat acatgattta 5880
gatgtacatc ctattaaaaa gccagatgct gaaaaaacga ttaatgatga tcttgcacgc 5940
gtcactgctt tagtgcaaaa ttatcgaaaa gtaagtaatc gtaataaggc tgatgcatta 6000
aaagctataa ctgctttaaa attacaaatg gatgaagaat taaaaacagc acgcactaat 6060
gctgatgttg atgcagtttt aaaacgattt aatgttgcat taagcgatat agaagcagta 6120
attactgaaa aagaaaatag cttactgcga attgataaca ttgctcaaca aacatatgcg 6180
aaattcaaag cgatcgcaac accagaacaa ttagctaaag taaaagtatt aattgatcaa 6240
tatgttgcag atggcaatag aatgattgat gaagatgcga cattaaatga catcaaacaa 6300
cacacgcaat tcattgttga tgaaatttta gcaattaaat taccagctga agcgacgaaa 6360
gtatcaccaa aagaaattca gccagctcca aaagtttgta cgcctattaa aaaagaagag 6420
acacatgaat cgcgcaaagt tgaaaaagaa cttccaaata caggttctga aggaatggat 6480
ttaccattga aagaatttgc actgattaca ggtgcggctt tgttagctag aagacgtact 6540
aaaaacgaaa aagaatcata a 6561

<210> 85
<211> 2319
<212> DNA
<213> Staphylococcus aureus

<400> 85
gaggagaatt cagtacaaga cgttaaagat tcgaatacgg atgatgaatt atcagacagc 60
aatgatcagt ctagtgatga agaaaagaat gatgtgatca ataataatca gtcaataaac 120
accgacgata ataaccaaat aattaaaaaa gaagaaacga ataactacga tggcatagaa 180
aaacgctcag aagatagaac agagtcaaca acaaatgtag atgaaaacga agcaacattt 240
ttacaaaaga cccctcaaga taatactcat cttacagaag aagaggtaaa agaatcctca 300
tcagtcgaat cctcaaattc atcaattgat actgcccaac aaccatctca cacaacaata 360
aatagagaag aatctgttca aacaagtgat aatgtagaag attcacacgt atcagatttt 420
gctaactcta aaataaaaga gagtaacact gaatctggta aagaagagaa tactatagag 480
caacctaata aagtaaaaga agattcaaca acaagtcagc cgtctggcta tacaaatata 540
gatgaaaaaa tttcaaatca agatgagtta ttaaatttac caataaatga atatgaaaat 600
aaggctagac cattatctac aacatctgcc caaccatcga ttaaacgtgt aaccgtaaat 720
caattagcgg cggaacaagg ttcgaatgtt aatcatttaa ttaaagttac tgatcaaagt 720

attactgaag gatatgatga tagtgaaggt gttattaaag cacatgatgc tgaaaactta 780
atctatgatg taacttttga agtagatgat aaggtgaaat ctggtgatac gatgacagtg 840
gatatagata agaatacagt tccatcagat ttaaccgata gctttacaat accaaaaata 900
aaagataatt ctggagaaat catcgctaca ggtacttatg ataacaaaaa taaacaaatc 960
acctatactt ttacagatta tgtagataag tatgaaaata ttaaagcaca ccttaaatta 1020
acgtcataca ttgataaatc aaaggttcca aataataata ccaagttaga tgtagaatat 1080
aaaacggccc tttcatcagt aaataaaaca attacggttg aatatcaaag acctaacgaa 1140
aatcggactg ctaaccttca aagtatgttt acaaacatag atacgaaaaa tcatacagtt 1200
gagcaaacga tttatattaa ccctcttcgt tattcagcca aggaaacaaa tgtaaatatt 1260
tcagggaatg gtgatgaagg ttcaacaatt atagacgata gcacaataat taaagtttat 1320
aaggttggag ataatcaaaa tttaccagat agtaacagaa tttatgatta cagtgaatat 1380
gaagatgtca caaatgatga ttatgcccaa ttaggaaata ataatgatgt gaatattaat 1440
tttggtaata tagattcacc atatattatt aaagttatta gtaaatatga ccctaataag 1500
gatgattaca cgactataca gcaaactgtg acaatgcaga cgactataaa tgagtatact 1560
ggtgagttta gaacagcatc ctatgataat acaattgctt tctctacaag ttcaggtcaa 1620
ggacaaggtg acttgcctcc tgaaaaaact tataaaatcg gagattacgt atgggaagat 1680
gtagataaag atggtattca aaatacaaat gataatgaaa aaccgcttag taatgtattg 1740
gtaactttga cgtatcctga tggaacttca aaatcagtca gaacagatga agatgggaaa 1800
tatcaatttg atggattgaa aaacggattg acttataaaa ttacattcga aacacctgaa 1860
ggatatacgc cgacgcttaa acattcagga acaaatcctg cactagactc agaaggtaat 1920
tctgtatggg taactattaa tggacaagac gatatgacga ttgatagtgg attttatcaa 1980
acacctaaat acagcttagg gaactatgta tggtatgaca ctaataaaga tggtattcaa 2040
ggtgatgatg aaaaaggaat ctctggagtt aaagtgacgt taaaagatga aaacggaaat 2100
atcattagta caactacaac cgatgaaaat ggaaagtatc aatttgataa tttaaatagt 2160
ggtaattata ttgttcattt tgataaacct tcaggtatga ctcaaacaac aacagattct 2220
ggtgatgatg acgaacagga tgctgatggg gaagaagttc atgtaacaat tactgatcat 2280
gatgacttta gtatagataa cggatactat gatgacgaa 2319

<210> 86
<211> 1141
<212> PRT
<213> Staphylococcus aureus

<400> 86
Met Ile Asn Arg Asp Asn Lys Lys Ala Ile Thr Lys Lys Gly Met Ile
1               5                   10                  15
Ser Asn Arg Leu Asn Lys Phe Ser Ile Arg Lys Tyr Thr Val Gly Thr
            20                  25                  30
Ala Ser Ile Leu Val Gly Thr Thr Leu Ile Phe Gly Leu Gly Asn Gln
        35                  40                  45
Glu Ala Lys Ala Ala Glu Asn Thr Ser Thr Glu Asn Ala Lys Gln Asp
    50                  55                  60
Asp Ala Thr Thr Ser Asp Asn Lys Glu Val Val Ser Glu Thr Glu Asn
65                  70                  75                  80
Asn Ser Thr Thr Glu Asn Asp Ser Thr Asn Pro Ile Lys Lys Glu Thr
                85                  90                  95
Asn Thr Asp Ser Gln Pro Glu Ala Lys Glu Glu Ser Thr Thr Ser Ser
            100                 105                 110
Thr Gln Gln Gln Gln Asn Asn Val Thr Ala Thr Thr Glu Thr Lys Pro
        115                 120                 125
Gln Asn Ile Glu Lys Glu Asn Val Lys Pro Ser Thr Asp Lys Thr Ala
    130                 135                 140
Thr Glu Asp Thr Ser Val Ile Leu Glu Glu Lys Lys Ala Pro Asn Tyr
145                 150                 155                 160
Thr Asn Asn Asp Val Thr Thr Lys Pro Ser Thr Ser Glu Ile Gln Thr
                165                 170                 175
Lys Pro Thr Thr Pro Gln Glu Ser Thr Asn Ile Glu Asn Ser Gln Pro
            180                 185                 190
Gln Pro Thr Pro Ser Lys Val Asp Asn Gln Val Thr Asp Ala Thr Asn
        195                 200                 205
Pro Lys Glu Pro Val Asn Val Ser Lys Glu Glu Leu Lys Asn Asn Pro
    210                 215                 220
Glu Lys Leu Lys Glu Leu Val Arg Asn Asp Asn Asn Thr Asp Arg Ser
225                 230                 235                 240
Thr Lys Pro Val Ala Thr Ala Pro Thr Ser Val Ala Pro Lys Arg Leu

245                 250                 255
Asn Ala Lys Met Arg Phe Ala Val Ala Gln Pro Ala Ala Val Ala Ser
            260                 265                 270
Asn Asn Val Asn Asp Leu Ile Thr Val Thr Lys Gln Thr Ile Lys Val
        275                 280                 285
Gly Asp Gly Lys Asp Asn Val Ala Ala Ala His Asp Gly Lys Asp Ile
    290                 295                 300
Glu Tyr Asp Thr Glu Phe Thr Ile Asp Asn Lys Val Lys Lys Gly Asp
305                 310                 315                 320
Thr Met Thr Ile Asn Tyr Asp Lys Asn Val Ile Pro Ser Asp Leu Thr
                325                 330                 335
Asp Lys Asn Asp Pro Ile Asp Ile Thr Asp Pro Ser Gly Glu Val Ile
            340                 345                 350
Ala Lys Gly Thr Phe Asp Lys Ala Thr Lys Gln Ile Thr Tyr Thr Phe
        355                 360                 365
Thr Asp Tyr Val Asp Lys Tyr Glu Asp Ile Lys Ala Arg Leu Thr Leu
    370                 375                 380
Tyr Ser Tyr Ile Asp Lys Gln Ala Val Pro Asn Glu Thr Ser Leu Asn
385                 390                 395                 400
Leu Thr Phe Ala Thr Ala Gly Lys Glu Thr Ser Gln Asn Val Ser Val
                405                 410                 415
Asp Tyr Gln Asp Pro Met Val His Gly Asp Ser Asn Ile Gln Ser Ile
            420                 425                 430
Phe Thr Lys Leu Asp Glu Asn Lys Gln Thr Ile Glu Gln Gln Ile Tyr
        435                 440                 445
Val Asn Pro Leu Lys Lys Thr Ala Thr Asn Thr Lys Val Asp Ile Ala
    450                 455                 460
Gly Ser Gln Val Asp Asp Tyr Gly Asn Ile Lys Leu Gly Asn Gly Ser
465                 470                 475                 480
Thr Ile Ile Asp Gln Asn Thr Glu Ile Lys Val Tyr Lys Val Asn Pro
                485                 490                 495
Asn Gln Gln Leu Pro Gln Ser Asn Arg Ile Tyr Asp Phe Ser Gln Tyr
            500                 505                 510
Glu Asp Val Thr Ser Gln Phe Asp Asn Lys Lys Ser Phe Ser Asn Asn
        515                 520                 525
Val Ala Thr Leu Asp Phe Gly Asp Ile Asn Ser Ala Tyr Ile Ile Lys
    530                 535                 540
Val Val Ser Lys Tyr Thr Pro Thr Ser Asp Gly Glu Leu Asp Ile Ala
545                 550                 555                 560
Gln Gly Thr Ser Met Arg Thr Thr Asp Lys Tyr Gly Tyr Tyr Asn Tyr
                565                 570                 575
Ala Gly Tyr Ser Asn Phe Ile Val Thr Ser Asn Asp Thr Gly Gly Gly
            580                 585                 590
Asp Gly Thr Val Lys Pro Glu Glu Lys Leu Tyr Lys Ile Gly Asp Tyr
        595                 600                 605
Val Trp Glu Asp Val Asp Lys Asp Gly Val Gln Gly Thr Asp Ser Lys
    610                 615                 620
Glu Lys Pro Met Ala Asn Val Leu Val Thr Leu Thr Tyr Pro Asp Gly
625                 630                 635                 640
Thr Thr Lys Ser Val Arg Thr Asp Ala Asn Gly His Tyr Glu Phe Gly
                645                 650                 655
Gly Leu Lys Asp Gly Glu Thr Tyr Thr Val Lys Phe Glu Thr Pro Ala
            660                 665                 670
Gly Tyr Leu Pro Thr Lys Val Asn Gly Thr Thr Asp Gly Glu Lys Asp
        675                 680                 685
Ser Asn Gly Ser Ser Ile Thr Val Lys Ile Asn Gly Lys Asp Asp Met
    690                 695                 700
Ser Leu Asp Thr Gly Phe Tyr Lys Glu Pro Lys Tyr Asn Leu Gly Asp
705                 710                 715                 720
Tyr Val Trp Glu Asp Thr Asn Lys Asp Gly Ile Gln Asp Ala Asn Glu
                725                 730                 735
Pro Gly Ile Lys Asp Val Lys Val Thr Leu Lys Asp Ser Thr Gly Lys
            740                 745                 750
Val Ile Gly Thr Thr Thr Thr Asp Ala Ser Gly Lys Tyr Lys Phe Thr
        755                 760                 765

Asp Leu Asp Asn Gly Asn Tyr Thr Val Glu Phe Glu Thr Pro Ala Gly
    770                 775                 780
Tyr Thr Pro Thr Val Lys Asn Thr Thr Ala Glu Asp Lys Asp Ser Asn
785                 790                 795                 800
Gly Leu Thr Thr Thr Gly Val Ile Lys Asp Ala Asp Asn Met Thr Leu
                805                 810                 815
Asp Ser Gly Phe Tyr Lys Thr Pro Lys Tyr Ser Leu Gly Asp Tyr Val
            820                 825                 830
Trp Tyr Asp Ser Asn Lys Asp Gly Lys Gln Asp Ser Thr Glu Lys Gly
        835                 840                 845
Ile Lys Asp Val Lys Val Thr Leu Leu Asn Glu Lys Gly Glu Val Ile
    850                 855                 860
Gly Thr Thr Lys Thr Asp Glu Asn Gly Lys Tyr Arg Phe Asp Asn Leu
865                 870                 875                 880
Asp Ser Gly Lys Tyr Lys Val Ile Phe Glu Lys Pro Ala Gly Leu Thr
                885                 890                 895
Gln Thr Val Thr Asn Thr Thr Glu Asp Asp Lys Asp Ala Asp Gly Gly
            900                 905                 910
Glu Val Asp Val Thr Ile Thr Asp His Asp Asp Phe Ile Leu Asp Asn
        915                 920                 925
Gly Tyr Phe Glu Glu Asp Thr Ser Asp Ser Asp Ser Asp Ser Asp Ser
    930                 935                 940
Asp Ser Asp Ser Asp Ser Asp Ser Asp Ser Asp Ser Asp Ser Asp Ser
945                 950                 955                 960
Asp Ser Asp Ser Asp Ser Asp Ser Asp Ser Asp Ser Asp Ser Asp Ser
                965                 970                 975
Asp Ser Asp Ser Asp Ser Asp Ser Asp Ser Asp Ser Asp Ser Asp Ser
            980                 985                 990
Asp Ser Asp Ser Asp Ser Asp Ser Asp Ser Asp Ser Asp Ser Asp Ser
        995                 1000                1005
Asp Ser Asp Ser Asp Ser Asp Ser Asp Ser Asp Ser Asp Ser Asp Ser
    1010                1015                1020
Asp Ser Asp Ser Asp Ser Asp Ser Asp Ser Asp Ser Asp Ser Asp Ser
1025                1030                1035                1040
Asp Ser Asp Ser Asp Ser Asp Ser Asp Ser Asp Ser Asp Ser Asp Ser
                1045                1050                1055
Asp Ser Asp Ser Asp Ser Asp Ser Asp Ser Asp Ser Asp Ser Asp Ser
            1060                1065                1070
Asp Ser Asp Ser Asp Ser Asp Ser Asp Ala Gly Lys His Thr Pro Val
        1075                1080                1085
Lys Pro Met Ser Thr Thr Lys Asp His His Asn Lys Ala Lys Ala Leu
    1090                1095                1100
Pro Glu Thr Gly Ser Glu Asn Asn Gly Ser Asn Asn Ala Thr Leu Phe
1105                1110                1115                1120
Gly Gly Leu Phe Ala Ala Leu Gly Ser Leu Leu Leu Phe Gly Arg Arg
                1125                1130                1135
Lys Lys Gln Asn Lys
            1140

<210> 87
<211> 3426
<212> DNA
<213> Staphylococcus aureus

<400> 87
atgattaaca gggataataa aaaggcaata acaaaaaagg gtatgatttc aaatcgctta 60
aacaaatttt cgattagaaa gtatactgta ggaactgcat cgattttagt aggtacgaca 120
ttgatttttg gtctagggaa ccaagaagct aaagctgctg aaaacactag tacagaaaat 180
gcgaaacaag atgatgcaac gactagtgat aataaagaag tagtgtcgga aactgaaaat 240
aattcgacaa cagaaaatga ttcaacaaat ccaattaaga aagaaacaaa tactgattca 300
caaccagaag ctaaagaaga atcaactaca tcaagtactc aacaacagca aaataacgtt 360
acagctacaa ctgaaactaa gcctcaaaac attgaaaaag aaaatgttaa accttcaact 420
gataaaactg cgacagaaga tacatctgtt attttagaag agaagaaagc accaaattat 480
acaaataacg atgtaactac aaaaccatct acaagtgaaa ttcaaacaaa accaactaca 540

cctcaagaat ctacaaatat tgaaaattca caaccgcaac caacgccttc aaaagtagac 600
aatcaagtta cagatgcaac taatccaaaa gaaccagtaa atgtgtcaaa agaagaactt 660
aaaaataatc ctgagaaatt aaaagaatta gttagaaatg ataacaatac agatcgttca 720
actaaaccag ttgctacagc tccaacaagt gttgcaccaa aacgattaaa tgcgaaaatg 780
cgttttgcag ttgcacaacc agcagcagtt gcttcaaata atgtaaatga cttaattaca 840
gttacgaaac agacgatcaa agttggcgat ggtaaagata atgtggcagc agcgcatgac 900
ggtaaagata ttgaatatga tacagagttt acaattgaca ataaagtcaa aaaaggcgat 960
acaatgacga ttaattatga taagaatgta attccttcgg atttaacaga taaaaatgat 1020
cctatcgata ttactgatcc atcaggagag gtcattgcca aaggaacatt tgataaagcg 1080
actaagcaaa tcacatatac atttacagat tatgtagata aatatgaaga tataaaagca 1140
cgtttaactt tatactcata tattgataag caagcagtac ctaatgaaac tagtttgaat 1200
ttaacgtttg caacagcagg taaagaaact agccaaaacg tttctgttga ttatcaagac 1260
ccaatggttc atggtgattc aaacattcaa tctatcttta caaagttaga tgaaaacaaa 1320
caaactattg aacaacaaat ttatgttaat cctttgaaaa aaacagcaac taacactaaa 1380
gttgatatag ctggtagtca agtagatgat tatggaaata ttaaactagg aaatggtagt 1440
accattattg accaaaatac agaaataaaa gtttataaag ttaaccctaa tcaacaattg 1500
cctcaaagta atagaatcta tgattttagt caatacgaag atgtaacaag tcaatttgat 1560
aataaaaaat catttagtaa taatgtagca acattggatt ttggtgatat taattcagcc 1620
tatattatca aagttgttag taaatataca cctacatcag atggcgaact agatattgct 1680
caaggtacta gtatgagaac aactgataaa tatggttatt ataattatgc aggatattca 1740
aacttcatcg taacttctaa tgacactggc ggtggcgacg gtactgttaa acctgaagaa 1800
aagttataca aaattggtga ctatgtatgg gaagacgttg ataaagacgg tgtccaaggt 1860
acagattcga aagaaaagcc aatggcaaac gttttagtta cattaactta cccggacggt 1920
actacaaaat cagtaagaac agatgctaac ggtcattatg aattcggtgg tttgaaagac 1980
ggagaaactt atacagttaa attcgaaacg ccagctggat atcttccaac aaaagtaaat 2040
ggaacaactg atggtgaaaa agactcaaat ggtagttcta taactgttaa aattaatggt 2100
aaagatgata tgtctttaga cactggtttt tataaagaac ctaaatataa tcttggtgac 2160
tatgtatggg aagatacaaa taaagatggt atccaagatg ctaatgaacc tggtatcaaa 2220
gatgttaagg ttacattaaa agatagtact ggaaaagtta ttggtacaac tactactgat 2280
gcctcgggta aatataaatt tacagattta gataatggta actatacagt agaatttgaa 2340
acaccagcag gttacacgcc aacggttaaa aatactacag ctgaagataa agattctaat 2400
ggtttaacaa caacaggtgt cattaaagat gcagataata tgacattaga cagtggtttc 2460
tataaaacac caaaatacag tttaggtgat tatgtttggt acgacagtaa taaagacggt 2520
aaacaagatt caactgaaaa aggtatcaaa gatgttaaag ttactttatt aaatgaaaaa 2580
ggcgaagtaa ttggaacaac taaaacagat gaaaatggta aatatcgttt cgataattta 2640
gatagcggta aatacaaagt tatttttgaa aagcctgctg gcttaacaca aacagttaca 2700
aatacaactg aagatgataa agatgccgat ggtggcgaag ttgacgtaac aattacggat 2760
catgatgatt tcatacttga taacggatac ttcgaagaag atacatcaga cagtgattca 2820
gactcagaca gtgattcaga ctcagacagc gactcagatt cagacagtga ttcagactca 2880
gatagcgatt cagattcaga cagcgactca gactcagata gcgactcaga ctcagacagc 2940
gactcagact cagatagcga ctcagattcg gacagcgatt cagactcaga tagcgactca 3000
gattcagaca gcgattcaga ctcagatagc gactcagatt cagacagtga ctcagactca 3060
gatagcgact cagactcaga cagtgactca gactcagaca gcgattcaga ttcagatagc 3120
gactcagatt cggacagtga ttcagactca gatagcgact cagattcaga cagcgactca 3180
gactcagata gcgactcaga ctcagacagt gattcagact cagatagcga ttcggactcg 3240
gatgcaggaa aacatacacc tgttaaacca atgagtacta ctaaagacca tcacaataaa 3300
gcaaaagcat taccagaaac aggtagtgaa aataacggct caaataacgc aacgttattt 3360
ggtggattat ttgcagcatt aggttcatta ttgttattcg gtcgtcgcaa aaaacaaaac 3420
aaataa 3426

<210> 88
<211> 1349
<212> PRT
<213> Staphylococcus aureus

<400> 88
Met Leu Asn Arg Glu Asn Lys Thr Ala Ile Thr Arg Lys Gly Met Val
1               5                   10                  15
Ser Asn Arg Leu Asn Lys Phe Ser Ile Arg Lys Tyr Thr Val Gly Thr
            20                  25                  30
Ala Ser Ile Leu Val Gly Thr Thr Leu Ile Phe Gly Leu Gly Asn Gln
        35                  40                  45
Glu Ala Lys Ala Ala Glu Ser Thr Asn Lys Glu Leu Asn Glu Ala Thr
    50                  55                  60
Thr Ser Ala Ser Asp Asn Gln Ser Ser Asp Lys Val Asp Met Gln Gln

65                  70                  75                  80
Leu Asn Gln Glu Asp Asn Thr Lys Asn Asp Asn Gln Lys Glu Met Val
                85                  90                  95
Ser Ser Gln Gly Asn Glu Thr Thr Ser Asn Gly Asn Lys Leu Ile Glu
            100                 105                 110
Lys Glu Ser Val Gln Ser Thr Thr Gly Asn Lys Val Glu Val Ser Thr
        115                 120                 125
Ala Lys Ser Asp Glu Gln Ala Ser Pro Lys Ser Thr Asn Glu Asp Leu
    130                 135                 140
Asn Thr Lys Gln Thr Ile Ser Asn Gln Glu Ala Leu Gln Pro Asp Leu
145                 150                 155                 160
Gln Glu Asn Lys Ser Val Val Asn Val Gln Pro Thr Asn Glu Glu Asn
                165                 170                 175
Lys Lys Val Asp Ala Lys Thr Glu Ser Thr Thr Leu Asn Val Lys Ser
            180                 185                 190
Asp Ala Ile Lys Ser Asn Asp Glu Thr Leu Val Asp Asn Asn Ser Asn
        195                 200                 205
Ser Asn Asn Glu Asn Asn Ala Asp Ile Ile Leu Pro Lys Ser Thr Ala
    210                 215                 220
Pro Lys Arg Leu Asn Thr Arg Met Arg Ile Ala Ala Val Gln Pro Ser
225                 230                 235                 240
Ser Thr Glu Ala Lys Asn Val Asn Asp Leu Ile Thr Ser Asn Thr Thr
                245                 250                 255
Leu Thr Val Val Asp Ala Asp Lys Asn Asn Lys Ile Val Pro Ala Gln
            260                 265                 270
Asp Tyr Leu Ser Leu Lys Ser Gln Ile Thr Val Asp Asp Lys Val Lys
        275                 280                 285
Ser Gly Asp Tyr Phe Thr Ile Lys Tyr Ser Asp Thr Val Gln Val Tyr
    290                 295                 300
Gly Leu Asn Pro Glu Asp Ile Lys Asn Ile Gly Asp Ile Lys Asp Pro
305                 310                 315                 320
Asn Asn Gly Glu Thr Ile Ala Thr Ala Lys His Asp Thr Ala Asn Asn
                325                 330                 335
Leu Ile Thr Tyr Thr Phe Thr Asp Tyr Val Asp Arg Phe Asn Ser Val
            340                 345                 350

Gln Met Gly Ile Asn Tyr Ser Ile Tyr Met Asp Ala Asp Thr Ile Pro
        355                 360                 365
Val Ser Lys Asn Asp Val Glu Phe Asn Val Thr Ile Gly Asn Thr Thr
    370                 375                 380
Thr Lys Thr Thr Ala Asn Ile Gln Tyr Pro Asp Tyr Val Val Asn Glu
385                 390                 395                 400
Lys Asn Ser Ile Gly Ser Ala Phe Thr Glu Thr Val Ser His Val Gly
                405                 410                 415
Asn Lys Glu Asn Pro Gly Tyr Tyr Lys Gln Thr Ile Tyr Val Asn Pro
            420                 425                 430
Ser Glu Asn Ser Leu Thr Asn Ala Lys Leu Lys Val Gln Ala Tyr His
        435                 440                 445
Ser Ser Tyr Pro Asn Asn Ile Gly Gln Ile Asn Lys Asp Val Thr Asp
    450                 455                 460
Ile Lys Ile Tyr Gln Val Pro Lys Gly Tyr Thr Leu Asn Lys Gly Tyr
465                 470                 475                 480
Asp Val Asn Thr Lys Glu Leu Thr Asp Val Thr Asn Gln Tyr Leu Gln
                485                 490                 495
Lys Ile Thr Tyr Gly Asp Asn Asn Ser Ala Val Ile Asp Phe Gly Asn
            500                 505                 510
Ala Asp Ser Ala Tyr Val Val Met Val Asn Thr Lys Phe Gln Tyr Thr
        515                 520                 525
Asn Ser Glu Ser Pro Thr Leu Val Gln Met Ala Thr Leu Ser Ser Thr
    530                 535                 540
Gly Asn Lys Ser Val Ser Thr Gly Asn Ala Leu Gly Phe Thr Asn Asn
545                 550                 555                 560
Gln Ser Gly Gly Ala Gly Gln Glu Val Tyr Lys Ile Gly Asn Tyr Val
                565                 570                 575
Trp Glu Asp Thr Asn Lys Asn Gly Val Gln Glu Leu Gly Glu Lys Gly

            580                 585                 590
Val Gly Asn Val Thr Val Thr Val Phe Asp Asn Asn Thr Asn Thr Lys
        595                 600                 605
Val Gly Glu Ala Val Thr Lys Glu Asp Gly Ser Tyr Leu Ile Pro Asn
    610                 615                 620
Leu Pro Asn Gly Asp Tyr Arg Val Glu Phe Ser Asn Leu Pro Lys Gly
625                 630                 635                 640
Tyr Glu Val Thr Pro Ser Lys Gln Gly Asn Asn Glu Glu Leu Asp Ser
                645                 650                 655
Asn Gly Leu Ser Ser Val Ile Thr Val Asn Gly Lys Asp Asn Leu Ser
            660                 665                 670
Ala Asp Leu Gly Ile Tyr Lys Pro Lys Tyr Asn Leu Gly Asp Tyr Val
        675                 680                 685
Trp Glu Asp Thr Asn Lys Asn Gly Ile Gln Asp Gln Asp Glu Lys Gly
    690                 695                 700
Ile Ser Gly Val Thr Val Thr Leu Lys Asp Glu Asn Gly Asn Val Leu
705                 710                 715                 720
Lys Thr Val Thr Thr Asp Ala Asp Gly Lys Tyr Lys Phe Thr Asp Leu
                725                 730                 735
Asp Asn Gly Asn Tyr Lys Val Glu Phe Thr Thr Pro Glu Gly Tyr Thr
            740                 745                 750
Pro Thr Thr Val Thr Ser Gly Ser Asp Ile Glu Lys Asp Ser Asn Gly
        755                 760                 765
Leu Thr Thr Thr Gly Val Ile Asn Gly Ala Asp Asn Met Thr Leu Asp
    770                 775                 780
Ser Gly Phe Tyr Lys Thr Pro Lys Tyr Asn Leu Gly Asn Tyr Val Trp
785                 790                 795                 800
Glu Asp Thr Asn Lys Asp Gly Lys Gln Asp Ser Thr Glu Lys Gly Ile
                805                 810                 815
Ser Gly Val Thr Val Thr Leu Lys Asn Glu Asn Gly Glu Val Leu Gln
            820                 825                 830
Thr Thr Lys Thr Asp Lys Asp Gly Lys Tyr Gln Phe Thr Gly Leu Glu
        835                 840                 845
Asn Gly Thr Tyr Lys Val Glu Phe Glu Thr Pro Ser Gly Tyr Thr Pro
    850                 855                 860
Thr Gln Val Gly Ser Gly Thr Asp Glu Gly Ile Asp Ser Asn Gly Thr
865                 870                 875                 880
Ser Thr Thr Gly Val Ile Lys Asp Lys Asp Asn Asp Thr Ile Asp Ser
                885                 890                 895
Gly Phe Tyr Lys Pro Thr Tyr Asn Leu Gly Asp Tyr Val Trp Glu Asp
            900                 905                 910
Thr Asn Lys Asn Gly Val Gln Asp Lys Asp Glu Lys Gly Ile Ser Gly
        915                 920                 925
Val Thr Val Thr Leu Lys Asp Glu Asn Asp Lys Val Leu Lys Thr Val
    930                 935                 940
Thr Thr Asp Glu Asn Gly Lys Tyr Gln Phe Thr Asp Leu Asn Asn Gly
945                 950                 955                 960
Thr Tyr Lys Val Glu Phe Glu Thr Pro Ser Gly Tyr Thr Pro Thr Ser
                965                 970                 975
Val Thr Ser Gly Asn Asp Thr Glu Lys Asp Ser Asn Gly Leu Thr Thr
            980                 985                 990
Thr Gly Val Ile Lys Asp Ala Asp Asn Met Thr Leu Asp Ser Gly Phe
        995                 1000                1005
Tyr Lys Thr Pro Lys Tyr Ser Leu Gly Asp Tyr Val Trp Tyr Asp Ser
    1010                1015                1020
Asn Lys Asp Gly Lys Gln Asp Ser Thr Glu Lys Gly Ile Lys Asp Val
1025                1030                1035                1040
Lys Val Thr Leu Leu Asn Glu Lys Gly Glu Val Ile Gly Thr Thr Lys
                1045                1050                1055
Thr Asp Glu Asn Gly Lys Tyr Cys Phe Asp Asn Leu Asp Ser Gly Lys
            1060                1065                1070
Tyr Lys Val Ile Phe Glu Lys Pro Ala Gly Leu Thr Gln Thr Val Thr
        1075                1080                1085
Asn Thr Thr Glu Asp Asp Lys Asp Ala Asp Gly Gly Glu Val Asp Val

1090                1095                1100
Thr Ile Thr Asp His Asp Asp Phe Thr Leu Asp Asn Gly Tyr Phe Glu
1105                1110                1115                1120
Glu Asp Thr Ser Asp Ser Asp Ser Asp Ser Asp Ser Asp Ser Asp Ser
                1125                1130                1135
Asp Ser Asp Ser Asp Ser Asp Ser Asp Ser Asp Ser Asp Ser Asp Ser
            1140                1145                1150
Asp Ser Asp Ser Asp Ser Asp Ser Asp Ser Asp Ser Asp Ser Asp Ser
        1155                1160                1165
Asp Ser Asp Ser Asp Ser Asp Ser Asp Ser Asp Ser Asp Ser Asp Ser
    1170                1175                1180
Asp Ser Asp Ser Asp Ser Asp Ser Asp Ser Asp Ser Asp Ser Asp Ser
1185                1190                1195                1200
Asp Ser Asp Ser Asp Ser Asp Ser Asp Ser Asp Ser Asp Ser Asp Ser
                1205                1210                1215
Asp Ser Asp Ser Asp Ser Asp Ser Asp Ser Asp Ser Asp Ser Asp Ser
            1220                1225                1230
Asp Ser Asp Ser Asp Ser Asp Ser Asp Ser Asp Ser Asp Ser Asp Ser
        1235                1240                1245
Asp Ser Asp Ser Asp Ser Asp Ser Asp Ser Asp Ser Asp Ser Asp Ser
    1250                1255                1260
Asp Ser Asp Ser Asp Ser Asp Ser Asp Ser Asp Ser Asp Ser Asp Ser
1265                1270                1275                1280
Asp Ser Asp Ser Asp Ser Asp Ser Asp Ala Gly Lys His Thr Pro Val
                1285                1290                1295
Lys Pro Met Ser Thr Thr Lys Asp His His Asn Lys Ala Lys Ala Leu
            1300                1305                1310
Pro Glu Thr Gly Ser Glu Asn Asn Gly Ser Asn Asn Ala Thr Leu Phe
        1315                1320                1325
Gly Gly Leu Phe Ala Ala Leu Gly Ser Leu Leu Leu Phe Gly Arg Arg
    1330                1335                1340
Lys Lys Gln Asn Lys
1345

<210> 89
<211> 4050
<212> DNA
<213> Staphylococcus aureus

<400> 89
atgctaaaca gagaaaataa aacggcaata acaaggaaag gcatggtatc caatcgatta 60
aataaatttt cgattagaaa gtacacagtg ggaacagcat caattttagt aggtacaaca 120
ttaatttttg gtctggggaa ccaagaagca aaggctgcag aaagtactaa taaagaattg 180

aacgaagcga caacttcagc aagtgataat caatcgagtg ataaagttga tatgcagcaa 240
ctaaatcaag aagacaatac taaaaatgat aatcaaaaag aaatggtatc atctcaaggt 300
aatgaaacga cttcaaatgg gaataaatta atagaaaaag aaagtgtaca atctaccact 360
ggaaataaag ttgaagtttc aactgccaaa tcagatgagc aagcttcacc aaaatctacg 420
aatgaagatt taaacactaa acaaactata agtaatcaag aagcgttaca acctgatttg 480
caagagaata aatcagtggt aaatgttcaa ccaactaatg aggaaaacaa aaaggtagat 540
gccaaaactg aatcaactac attaaatgtt aaaagtgatg ctatcaagag taatgatgaa 600
actcttgttg ataacaatag taattcaaat aatgaaaata atgcagatat cattttgcca 660
aaaagtacag cacctaaacg tttgaataca agaatgcgta tagcagcagt acagccatca 720
tcaacagagg ctaaaaatgt taatgattta atcacatcaa atacaacatt aactgtcgtt 780
gatgcagata aaaacaataa aatcgtacca gcccaagatt atttatcatt aaaatcacaa 840
attacagttg atgacaaagt taaatcaggt gattatttca caattaaata ctcagataca 900
gtacaagtat atggattgaa tccggaagat attaaaaata ttggtgatat taaagatcca 960
aataatggtg aaacaattgc gactgcaaaa catgatactg caaataattt aattacatat 1020
acatttacag attatgttga tcgatttaat tctgtacaaa tgggaattaa ttattcaatt 1080
tatatggatg ctgatacaat tcctgttagt aaaaacgatg ttgagtttaa tgttacgata 1140
ggtaatacta caacaaaaac aactgctaac attcaatatc cagattatgt tgtaaatgag 1200
aaaaattcaa ttggatcagc gttcactgaa acagtttcac atgttggaaa taaagaaaat 1260
ccagggtact ataaacaaac gatttatgta aatccatcgg aaaattcttt aacaaatgcc 1320
aaactaaaag ttcaagctta ccactcaagt tatcctaata atatcgggca aataaataaa 1380

gatgtaacag atataaaaat atatcaagtt cctaaaggtt atacattaaa taaaggatac 1440
gatgtgaata ctaaagagct tacagatgta acaaatcaat acttgcagaa aattacatat 1500
ggcgacaaca atagcgctgt tattgatttt ggaaatgcag attctgctta tgttgtaatg 1560
gttaatacaa aattccaata tacaaatagc gaaagcccaa cacttgttca aatggctact 1620
ttatcttcaa caggtaataa atccgtttct actggcaatg ctttaggatt tactaataac 1680
caaagtggcg gagctggtca agaagtatat aaaattggta actacgtatg ggaagatact 1740
aataaaaacg gtgttcaaga attaggagaa aaaggcgttg gcaatgtaac tgtaactgta 1800
tttgataata atacaaatac aaaagtagga gaagcagtta ctaaagaaga tgggtcatac 1860
ttgattccaa acttacctaa tggagattac cgtgtagaat tttcaaactt accaaaaggt 1920
tatgaagtaa ccccttcaaa acaaggtaat aacgaagaat tagattcaaa cggcttatct 1980
tcagttatta cagttaatgg caaagataac ttatctgcag acttaggtat ttacaaacct 2040
aaatacaact taggtgacta tgtctgggaa gatacaaata aaaatggtat ccaagaccaa 2100
gatgaaaaag gtatatctgg cgtaacggta acattaaaag atgaaaacgg taacgtgtta 2160
aaaacagtta caacagacgc tgatggcaaa tataaattta ctgatttaga taatggtaat 2220
tataaagttg aatttactac accagaaggc tatacaccga ctacagtaac atctggtagc 2280
gacattgaaa aagactctaa tggtttaaca acaacaggtg ttattaatgg tgctgataac 2340
atgacattag atagtggatt ctacaaaaca ccaaaatata atttaggtaa ttatgtatgg 2400
gaagatacaa ataaagatgg taagcaggat tcaactgaaa aaggtatttc aggcgtaaca 2460
gttacattga aaaatgaaaa cggtgaagtt ttacaaacaa ctaaaacaga taaagatggt 2520
aaatatcaat ttactggatt agaaaatgga acttataaag ttgaattcga aacaccatca 2580
ggttacacac caacacaagt aggttcagga actgatgaag gtatagattc aaatggtaca 2640
tcaacaacag gtgtcattaa agataaagat aacgatacta ttgactctgg tttctacaaa 2700
ccgacttaca acttaggtga ctatgtatgg gaagatacaa ataaaaacgg tgttcaagat 2760
aaagatgaaa agggcatttc aggtgtaaca gttacgttaa aagatgaaaa cgacaaagtt 2820
ttaaaaacag ttacaacaga tgaaaatggt aaatatcaat tcactgattt aaacaatgga 2880
acttataaag ttgaattcga gacaccatca ggttatacac caacttcagt aacttctgga 2940
aatgatactg aaaaagattc taatggttta acaacaacag gtgtcattaa agatgcagat 3000
aacatgacat tagacagtgg tttctataaa acaccaaaat atagtttagg tgattatgtt 3060
tggtacgaca gtaataaaga cggcaaacaa gattcaactg aaaaaggtat caaagatgtt 3120
aaagttactt tattaaatga aaaaggcgaa gtaattggaa caactaaaac agatgaaaat 3180
ggtaaatact gctttgataa tttagatagc ggtaaataca aagttatttt tgaaaagcct 3240
gctggcttaa cacaaacagt tacaaataca actgaagatg ataaagatgc agatggtggc 3300
gaagttgacg taacaattac ggatcatgat gatttcacac ttgataacgg atacttcgaa 3360
gaagatacat cagacagcga ttcagactca gatagtgact cagacagcga ctcagactca 3420
gacagcgact cagactcaga cagtgattca gattcagaca gcgactcaga ttcagatagc 3480
gactcagatt cggacagcga ttcagactca gatagcgact cagattcaga tagcgattca 3540
gactcagaca gcgactcaga ttcagatagc gattcggact cagacagcga ttcagactca 3600
gatagcgact cagactcaga cagcgactca gattcagata gcgattcgga ctcagatagc 3660
gactcagatt cagacagcga ttcagactca gatagcgact cagattcaga cagcgattca 3720
gactcagata gcgactcaga ctcagacagt gattcagatt cagacagcga ctcagactca 3780
gatagcgact cagattcgga cagcgactca gactctgata gcgactcaga ctcagacagt 3840
gattcagaca gcgattcaga ctcggatgca ggaaaacata cacctgttaa accaatgagt 3900
actactaaag accatcacaa taaagcaaaa gcattaccag aaacaggtag tgaaaataac 3960
ggctcaaata acgcaacgtt atttggtgga ttatttgcag cattaggttc attattgtta 4020
ttcggtcgtc gcaaaaaaca aaacaaataa 4050

<210> 90
<211> 635
<212> PRT
<213> Staphylococcus aureus

<400> 90
Met Ala Lys Tyr Arg Gly Lys Pro Phe Gln Leu Tyr Val Lys Leu Ser
1               5                   10                  15
Cys Ser Thr Met Met Ala Thr Ser Ile Ile Leu Thr Asn Ile Leu Pro
            20                  25                  30
Tyr Asp Ala Gln Ala Ala Ser Glu Lys Asp Thr Glu Ile Thr Lys Glu
        35                  40                  45
Ile Leu Ser Lys Gln Asp Leu Leu Asp Lys Val Asp Lys Ala Ile Arg
    50                  55                  60
Gln Ile Glu Gln Leu Lys Gln Leu Ser Ala Ser Ser Lys Glu His Tyr
65                  70                  75                  80
Lys Ala Gln Leu Asn Glu Ala Lys Thr Ala Ser Gln Ile Asp Glu Ile
                85                  90                  95
Ile Lys Arg Ala Asn Glu Leu Asp Ser Lys Asp Asn Lys Ser Ser His

            100                 105                 110
Thr Glu Met Asn Gly Gln Ser Asp Ile Asp Ser Lys Leu Asp Gln Leu
        115                 120                 125
Leu Lys Asp Leu Asn Glu Val Ser Ser Asn Val Asp Arg Gly Gln Gln
    130                 135                 140
Ser Gly Glu Asp Asp Leu Asn Ala Met Lys Asn Asp Met Ser Gln Thr
145                 150                 155                 160
Ala Thr Thr Lys His Gly Glu Lys Asp Asp Lys Asn Asp Glu Ala Met
                165                 170                 175
Val Asn Lys Ala Leu Glu Asp Leu Asp His Leu Asn Gln Gln Ile His
            180                 185                 190
Lys Ser Lys Asp Ala Ser Lys Asp Thr Ser Glu Asp Pro Ala Val Ser
        195                 200                 205
Thr Thr Asp Asn Asn His Glu Val Ala Lys Thr Pro Asn Asn Asp Gly
    210                 215                 220
Ser Gly His Val Val Leu Asn Lys Phe Leu Ser Asn Glu Glu Asn Gln
225                 230                 235                 240
Ser His Ser Asn Arg Leu Thr Asp Lys Leu Gln Gly Ser Asp Lys Ile
                245                 250                 255
Asn His Ala Met Ile Glu Lys Leu Ala Lys Ser Asn Ala Ser Thr Gln
            260                 265                 270
His Tyr Thr Tyr His Lys Leu Asn Thr Leu Gln Ser Leu Asp Gln Arg
        275                 280                 285
Ile Ala Asn Thr Gln Leu Pro Lys Asn Gln Lys Ser Asp Leu Met Ser
    290                 295                 300
Glu Val Asn Lys Thr Lys Glu Arg Ile Lys Ser Gln Arg Asn Ile Ile
305                 310                 315                 320
Leu Glu Glu Leu Ala Arg Thr Asp Asp Lys Lys Tyr Ala Thr Gln Ser
                325                 330                 335
Ile Leu Glu Ser Ile Phe Asn Lys Asp Glu Ala Val Lys Ile Leu Lys
            340                 345                 350
Asp Ile Arg Val Asp Gly Lys Thr Asp Gln Gln Ile Ala Asp Gln Ile
        355                 360                 365
Thr Arg His Ile Asp Gln Leu Ser Leu Thr Thr Ser Asp Asp Leu Leu
    370                 375                 380
Thr Ser Leu Ile Asp Gln Ser Gln Asp Lys Ser Leu Leu Ile Ser Gln
385                 390                 395                 400
Ile Leu Gln Thr Lys Leu Gly Lys Ala Glu Ala Asp Lys Leu Ala Lys
                405                 410                 415
Asp Trp Thr Asn Lys Gly Leu Ser Asn Arg Gln Ile Val Asp Gln Leu
            420                 425                 430
Lys Lys His Phe Ala Ser Thr Gly Asp Thr Ser Ser Asp Asp Ile Leu
        435                 440                 445
Lys Ala Ile Leu Asn Asn Ala Lys Asp Lys Lys Gln Ala Ile Glu Thr
    450                 455                 460
Ile Leu Ala Thr Arg Ile Glu Arg Gln Lys Ala Lys Leu Leu Ala Asp
465                 470                 475                 480
Leu Ile Thr Lys Ile Glu Thr Asp Gln Asn Lys Ile Phe Asn Leu Val
                485                 490                 495
Lys Ser Ala Leu Asn Gly Lys Ala Asp Asp Leu Leu Asn Leu Gln Lys
            500                 505                 510
Arg Leu Asn Gln Thr Lys Lys Asp Ile Asp Tyr Ile Leu Ser Pro Ile
        515                 520                 525
Val Asn Arg Pro Ser Leu Leu Asp Arg Leu Asn Lys Asn Gly Lys Thr
    530                 535                 540
Thr Asp Leu Asn Lys Leu Ala Asn Leu Met Asn Gln Gly Ser Asp Leu
545                 550                 555                 560
Leu Asp Ser Ile Pro Asp Ile Pro Thr Pro Lys Pro Glu Lys Thr Leu
                565                 570                 575
Thr Leu Gly Lys Gly Asn Gly Leu Leu Ser Gly Leu Leu Asn Ala Asp
            580                 585                 590
Gly Asn Val Ser Leu Pro Lys Ala Gly Glu Thr Ile Lys Glu His Trp
        595                 600                 605
Leu Pro Ile Ser Val Ile Val Gly Ala Met Gly Val Leu Met Ile Trp
    610                 615                 620

Leu Ser Arg Arg Asn Lys Leu Lys Asn Lys Ala
625                 630                 635

<210> 91
<211> 1908
<212> DNA
<213> Staphylococcus aureus

<400> 91
atggctaaat atcgagggaa accgtttcaa ttatatgtaa agttatcgtg ttcgacaatg 60
atggcgacaa gtatcatttt aacgaatatc ttgccgtacg atgcccaagc tgcatctgaa 120
aaggatactg aaattacaaa agagatatta tctaagcaag atttattaga caaagttgac 180
aaggcaattc gtcaaattga gcaattaaaa cagttatcgg cttcatctaa agaacattat 240
aaagcacaac taaatgaagc gaaaacagca tcgcaaatag atgaaatcat aaaacgagct 300
aatgagttgg atagcaaaga caataaaagt tctcacactg aaatgaacgg tcaaagtgat 360
atagacagta aattagatca attgcttaaa gatttaaatg aggtttcttc aaatgttgat 420
aggggtcaac aaagtggcga ggacgatctt aatgcaatga aaaatgatat gtcacaaacg 480
gctacaacaa aacatggaga aaaagatgat aaaaatgatg aagcaatggt aaataaggcg 540
ttagaagacc tagaccattt gaatcagcaa atacacaaat cgaaagatgc atcgaaagat 600
acatcggaag atccagcagt gtctacaaca gataataatc atgaagtagc taaaacgcca 660
aataatgatg gttctggaca tgttgtgtta aataaattcc tttcaaatga agagaatcaa 720
agccatagta atcgactcac tgataaatta caaggaagcg ataaaattaa tcatgctatg 780
attgaaaaat tagctaaaag taatgcctca acgcaacatt acacatatca taaactgaat 840
acgttacaat ctttagatca acgtattgca aatacgcaac ttcctaaaaa tcaaaaatca 900
gacttaatga gcgaagtaaa taagacgaaa gagcgtataa aaagtcaacg aaatattatt 960
ttggaagaac ttgcacgtac tgatgataaa aagtatgcta cacaaagcat tttagaaagt 1020
atatttaata aagacgaggc agttaaaatt ctaaaagata tacgtgttga tggtaaaaca 1080
gatcaacaaa ttgcagatca aattactcgt catattgatc aattatctct gacaacgagt 1140
gatgatttat taacgtcatt gattgatcaa tcacaagata agtcgctatt gatttctcaa 1200
attttacaaa cgaaattagg aaaagctgaa gcagataaat tggctaaaga ttggacgaat 1260
aaaggattat caaatcgcca aatcgttgac caattgaaga aacattttgc atcaactggc 1320
gacacgtctt cagatgatat attaaaagca attttgaata atgccaaaga taaaaaacaa 1380
gcaattgaaa cgattttagc aacacgtata gaaagacaaa aggcaaaatt actggcagat 1440
ttaattacta aaatagaaac agatcaaaat aaaattttta atttagttaa atcggcattg 1500
aatggtaaag cggatgattt attgaattta caaaagagac tcaatcaaac gaaaaaagat 1560
atagattata ttttatcacc aatagtaaat cgtccaagtt tactagatcg attgaataaa 1620
aatgggaaaa cgacagattt aaataagtta gcaaatttaa tgaatcaagg atcagattta 1680
ttagacagta ttccagatat acccacacca aagccagaaa agacgttaac acttggtaaa 1740
ggtaatggat tgttaagtgg attattaaat gctgatggta atgtatcttt gcctaaagcg 1800
ggggaaacga taaaagaaca ttggttgccg atatctgtaa ttgttggtgc aatgggtgta 1860
ctaatgattt ggttatcacg acgcaataag ttgaaaaata aagcataa 1908

<210> 92
<211> 241
<212> PRT
<213> Staphylococcus aureus

<400> 92
Met Lys Lys Leu Ala Thr Val Gly Ser Leu Ile Val Thr Ser Thr Leu
1               5                   10                  15
Val Phe Ser Ser Met Pro Phe Gln Asn Ala His Ala Asp Thr Thr Ser
            20                  25                  30
Met Asn Val Ser Asn Lys Gln Ser Gln Asn Val Gln Asn His Arg Pro
        35                  40                  45
Tyr Gly Gly Val Val Pro Gln Gly Met Thr Gln Ala Gln Tyr Thr Glu
    50                  55                  60
Leu Glu Lys Ala Leu Pro Gln Leu Ser Ala Gly Ser Asn Met Gln Asp
65                  70                  75                  80
Tyr Asn Met Lys Leu Tyr Asp Ala Thr Gln Asn Ile Ala Asp Lys Tyr
                85                  90                  95
Asn Val Ile Ile Thr Thr Asn Val Gly Val Phe Lys Pro His Ala Val
            100                 105                 110
Arg Asp Met Asn Gly His Ala Leu Pro Leu Thr Lys Asp Gly Asn Phe
        115                 120                 125

Tyr Gln Thr Asn Val Asp Ala Asn Gly Val Asn His Gly Gly Ser Glu
    130                 135                 140
Met Val Gln Asn Lys Thr Gly His Met Ser Gln Gln Gly His Met Asn
145                 150                 155                 160
Gln Asn Thr His Met Asn Gln Gln Pro His Met Gln Gln Gly His Met
                165                 170                 175
Gln Ser Ser Asn His Gln Met Met Ser Pro Lys Ala Asn Met His Ser
            180                 185                 190
Ser Asn His Gln Met Asn Gln Ser Asn Lys Lys Val Leu Pro Ala Ala
        195                 200                 205
Gly Glu Ser Met Thr Ser Ser Ile Leu Thr Ala Ser Ile Ala Ala Leu
    210                 215                 220
Leu Leu Val Ser Gly Leu Phe Leu Ala Phe Arg Arg Arg Ser Thr Asn
225                 230                 235                 240
Lys

<210> 93
<211> 726
<212> DNA
<213> Staphylococcus aureus

<400> 93
atgaaaaaat tagcaacagt aggttcttta attgtaacaa gcactttagt attctcaagt 60
atgccttttc aaaatgcgca tgccgacaca acttcaatga atgtgtcgaa taaacaaagc 120
caaaatgtac aaaatcatcg tccttatggc ggagtagtac cacaaggaat gacgcaagca 180
caatatactg aattagagaa agctttaccc caattaagcg ctggcagtaa tatgcaagac 240
tataatatga aattgtatga tgcgacgcaa aatattgctg ataaatacaa tgtgataatt 300
acaactaatg taggggtatt taaaccacat gctgttagag atatgaatgg ccatgcgtta 360
cctttaacaa aagatggcaa tttttatcaa acgaatgtag atgcaaatgg tgttaatcat 420
ggtggtagtg aaatggtgca aaataaaaca ggtcatatga gtcaacaagg ccatatgaat 480
cagaacacac acatgaacca acagccacac atgcaacaag gtcatatgca atcatcaaac 540
catcaaatga tgagtccaaa agcaaatatg cattcatcaa atcatcaaat gaaccaaagt 600
aacaaaaaag ttttaccagc tgctggtgaa agtatgacat caagtattct tactgcaagt 660
attgccgcac tactattagt atctgggtta ttcttagcat ttagacgacg ttcaacaaat 720
aaataa                                                            726

<210> 94
<211> 774
<212> PRT
<213> Staphylococcus aureus

<400> 94
Met Glu Glu Asn Ser Val Gln Asp Val Lys Asp Ser Asn Thr Asp Asp
1               5                   10                  15
Glu Leu Ser Asp Ser Asn Asp Gln Ser Ser Asp Glu Glu Lys Asn Asp
            20                  25                  30
Val Ile Asn Asn Asn Gln Ser Ile Asn Thr Asp Asp Asn Asn Gln Ile
        35                  40                  45
Ile Lys Lys Glu Glu Thr Asn Asn Tyr Asp Gly Ile Glu Lys Arg Ser
    50                  55                  60
Glu Asp Arg Thr Glu Ser Thr Thr Asn Val Asp Glu Asn Glu Ala Thr
65                  70                  75                  80
Phe Leu Gln Lys Thr Pro Gln Asp Asn Thr His Leu Thr Glu Glu Glu
                85                  90                  95
Val Lys Glu Ser Ser Ser Val Glu Ser Ser Asn Ser Ser Ile Asp Thr
            100                 105                 110
Ala Gln Gln Pro Ser His Thr Thr Ile Asn Arg Glu Glu Ser Val Gln
        115                 120                 125
Thr Ser Asp Asn Val Glu Asp Ser His Val Ser Asp Phe Ala Asn Ser
    130                 135                 140
Lys Ile Lys Glu Ser Asn Thr Glu Ser Gly Lys Glu Glu Asn Thr Ile
145                 150                 155                 160
Glu Gln Pro Asn Lys Val Lys Glu Asp Ser Thr Thr Ser Gln Pro Ser

                165                 170                 175
Gly Tyr Thr Asn Ile Asp Glu Lys Ile Ser Asn Gln Asp Glu Leu Leu
            180                 185                 190
Asn Leu Pro Ile Asn Glu Tyr Glu Asn Lys Ala Arg Pro Leu Ser Thr
        195                 200                 205
Thr Ser Ala Gln Pro Ser Ile Lys Arg Val Thr Val Asn Gln Leu Ala
    210                 215                 220
Ala Glu Gln Gly Ser Asn Val Asn His Leu Ile Lys Val Thr Asp Gln
225                 230                 235                 240
Ser Ile Thr Glu Gly Tyr Asp Asp Ser Glu Gly Val Ile Lys Ala His
                245                 250                 255
Asp Ala Glu Asn Leu Ile Tyr Asp Val Thr Phe Glu Val Asp Asp Lys
            260                 265                 270
Val Lys Ser Gly Asp Thr Met Thr Val Asp Ile Asp Lys Asn Thr Val
        275                 280                 285
Pro Ser Asp Leu Thr Asp Ser Phe Thr Ile Pro Lys Ile Lys Asp Asn
    290                 295                 300
Ser Gly Glu Ile Ile Ala Thr Gly Thr Tyr Asp Asn Lys Asn Lys Gln
305                 310                 315                 320
Ile Thr Tyr Thr Phe Thr Asp Tyr Val Asp Lys Tyr Glu Asn Ile Lys
                325                 330                 335
Ala His Leu Lys Leu Thr Ser Tyr Ile Asp Lys Ser Lys Val Pro Asn
            340                 345                 350
Asn Asn Thr Lys Leu Asp Val Glu Tyr Lys Thr Ala Leu Ser Ser Val
        355                 360                 365
Asn Lys Thr Ile Thr Val Glu Tyr Gln Arg Pro Asn Glu Asn Arg Thr
    370                 375                 380
Ala Asn Leu Gln Ser Met Phe Thr Asn Ile Asp Thr Lys Asn His Thr
385                 390                 395                 400
Val Glu Gln Thr Ile Tyr Ile Asn Pro Leu Arg Tyr Ser Ala Lys Glu
                405                 410                 415
Thr Asn Val Asn Ile Ser Gly Asn Gly Asp Glu Gly Ser Thr Ile Ile
            420                 425                 430
Asp Asp Ser Thr Ile Ile Lys Val Tyr Lys Val Gly Asp Asn Gln Asn
        435                 440                 445
Leu Pro Asp Ser Asn Arg Ile Tyr Asp Tyr Ser Glu Tyr Glu Asp Val
    450                 455                 460
Thr Asn Asp Asp Tyr Ala Gln Leu Gly Asn Asn Asn Asp Val Asn Ile
465                 470                 475                 480
Asn Phe Gly Asn Ile Asp Ser Pro Tyr Ile Ile Lys Val Ile Ser Lys
                485                 490                 495
Tyr Asp Pro Asn Lys Asp Asp Tyr Thr Thr Ile Gln Gln Thr Val Thr
            500                 505                 510
Met Gln Thr Thr Ile Asn Glu Tyr Thr Gly Glu Phe Arg Thr Ala Ser
        515                 520                 525
Tyr Asp Asn Thr Ile Ala Phe Ser Thr Ser Ser Gly Gln Gly Gln Gly
    530                 535                 540
Asp Leu Pro Pro Glu Lys Thr Tyr Lys Ile Gly Asp Tyr Val Trp Glu
545                 550                 555                 560
Asp Val Asp Lys Asp Gly Ile Gln Asn Thr Asn Asp Asn Glu Lys Pro
                565                 570                 575
Leu Ser Asn Val Leu Val Thr Leu Thr Tyr Pro Asp Gly Thr Ser Lys
            580                 585                 590
Ser Val Arg Thr Asp Glu Asp Gly Lys Tyr Gln Phe Asp Gly Leu Lys
        595                 600                 605
Asn Gly Leu Thr Tyr Lys Ile Thr Phe Glu Thr Pro Glu Gly Tyr Thr
    610                 615                 620
Pro Thr Leu Lys His Ser Gly Thr Asn Pro Ala Leu Asp Ser Glu Gly
625                 630                 635                 640
Asn Ser Val Trp Val Thr Ile Asn Gly Gln Asp Asp Met Thr Ile Asp
                645                 650                 655
Ser Gly Phe Tyr Gln Thr Pro Lys Tyr Ser Leu Gly Asn Tyr Val Trp
            660                 665                 670
Tyr Asp Thr Asn Lys Asp Gly Ile Gln Gly Asp Asp Glu Lys Gly Ile
        675                 680                 685

Ser Gly Val Lys Val Thr Leu Lys Asp Glu Asn Gly Asn Ile Ile Ser
    690                 695                 700
Thr Thr Thr Thr Asp Glu Asn Gly Lys Tyr Gln Phe Asp Asn Leu Asn
705                 710                 715                 720
Ser Gly Asn Tyr Ile Val His Phe Asp Lys Pro Ser Gly Met Thr Gln
                725                 730                 735
Thr Thr Thr Asp Ser Gly Asp Asp Asp Glu Gln Asp Ala Asp Gly Glu
            740                 745                 750
Glu Val His Val Thr Ile Thr Asp His Asp Asp Phe Ser Ile Asp Asn
        755                 760                 765
Gly Tyr Tyr Asp Asp Glu
    770

<210> 95
<211> 128
<212> PRT
<213> Staphylococcus aureus

<400> 95
Met Asp Val Asn Thr Val Asn Gln Lys Ala Ala Ser Val Lys Ser Thr
 1               5                  10                  15
Lys Asp Ala Leu Asp Gly Gln Gln Asn Leu Gln Arg Ala Lys Thr Glu
            20                  25                  30
Ala Thr Asn Ala Ile Thr His Ala Ser Asp Leu Asn Gln Ala Gln Lys
        35                  40                  45
Asn Ala Leu Thr Gln Gln Val Asn Ser Ala Gln Asn Val His Ala Val
    50                  55                  60
Asn Asp Ile Lys Gln Thr Thr Gln Ser Leu Asn Thr Ala Met Thr Gly
65                  70                  75                  80
Leu Lys Arg Gly Val Ala Asn His Asn Gln Val Val Gln Ser Asp Asn
                85                  90                  95
Tyr Val Asn Ala Asp Thr Asn Lys Lys Asn Asp Tyr Asn Asn Ala Tyr
            100                 105                 110
Asn His Ala Asn Asp Ile Ile Asn Gly Asn Ala Gln His Pro Val Ile
        115                 120                 125

<210> 96
<211> 6
<212> PRT
<213> Staphylococcus aureus

<400> 96
Thr Val Asn Gln Lys Ala
 1               5

<210> 97
<211> 6
<212> PRT
<213> Staphylococcus aureus

<400> 97
Lys Ser Thr Lys Asp Ala
 1               5

<210> 98
<211> 13
<212> PRT
<213> Staphylococcus aureus

<400> 98
Asp Gly Gln Gln Asn Leu Gln Arg Ala Lys Thr Glu Ala

1                 5                   10


<210> 99
<211> 10
<212> PRT
<213> Staphylococcus aureus

<400> 99
Asp Leu Asn Gln Ala Gln Lys Asn Ala Leu
1               5                   10


<210> 100
<211> 9
<212> PRT
<213> Staphylococcus aureus

<400> 100
Asp Ile Lys Gln Thr Thr Gln Ser Leu
1               5


<210> 101
<211> 13
<212> PRT
<213> Staphylococcus aureus

<400> 101
Ala Asp Thr Asn Lys Lys Asn Asp Tyr Asn Asn Ala Tyr
1               5                   10


<210> 102
<211> 7
<212> PRT
<213> Staphylococcus aureus

<400> 102
Asp Ile Ile Asn Gly Asn Ala
1               5


<210> 103
<211> 128
<212> PRT
<213> Staphylococcus aureus

<400> 103
Met Asp Val Asn Thr Val Asn Gln Lys Ala Ala Ser Val Lys Ser Thr
1               5                   10                  15
Lys Asp Ala Leu Asp Gly Gln Gln Asn Leu Gln Arg Ala Lys Thr Glu
            20                  25                  30
Ala Thr Asn Ala Ile Thr His Ala Ser Asp Leu Asn Gln Ala Gln Lys
        35                  40                  45
Asn Ala Leu Thr Gln Gln Val Asn Ser Ala Gln Asn Val His Ala Val
    50                  55                  60
Asn Asp Ile Lys Gln Thr Thr Gln Ser Leu Asn Thr Ala Met Thr Gly
65                  70                  75                  80
Leu Lys Arg Gly Val Ala Asn His Asn Gln Val Val Gln Ser Asp Asn
                85                  90                  95
Tyr Val Asn Ala Asp Thr Asn Lys Lys Asn Asp Tyr Asn Asn Ala Tyr
            100                 105                 110
Asn His Ala Asn Asp Ile Ile Asn Gly Asn Ala Gln His Pro Val Ile
        115                 120                 125

<210> 104
<211> 9
<212> PRT
<213> Staphylococcus aureus

<400> 104
Met Asp Val Asn Thr Val Asn Gln Lys
1               5

<210> 105
<211> 9
<212> PRT
<213> Staphylococcus aureus

<400> 105
Val Asn Thr Val Asn Gln Lys Ala Ala
1               5

<210> 106
<211> 9
<212> PRT
<213> Staphylococcus aureus

<400> 106
Val Asn Gln Lys Ala Ala Ser Val Lys
1               5

<210> 107
<211> 9
<212> PRT
<213> Staphylococcus aureus

<400> 107
Leu Gln Arg Ala Lys Thr Glu Ala Thr
1               5

<210> 108
<211> 9
<212> PRT
<213> Staphylococcus aureus

<400> 108
Ile Thr His Ala Ser Asp Leu Asn Gln
1               5

<210> 109
<211> 9
<212> PRT
<213> Staphylococcus aureus

<400> 109
Leu Asn Gln Ala Gln Lys Asn Ala Leu
1               5

<210> 110
<211> 9
<212> PRT

<213> Staphylococcus aureus

<400> 110
Leu Thr Gln Gln Val Asn Ser Ala Gln
1               5

<210> 111
<211> 9
<212> PRT
<213> Staphylococcus aureus

<400> 111
Val Asn Ser Ala Gln Asn Val His Ala
1               5

<210> 112
<211> 9
<212> PRT
<213> Staphylococcus aureus

<400> 112
Val His Ala Val Asn Asp Ile Lys Gln
1               5

<210> 113
<211> 9
<212> PRT
<213> Staphylococcus aureus

<400> 113
Val Asn Asp Ile Lys Gln Thr Thr Gln
1               5

<210> 114
<211> 9
<212> PRT
<213> Staphylococcus aureus

<400> 114
Ile Lys Gln Thr Thr Gln Ser Leu Asn
1               5

<210> 115
<211> 9
<212> PRT
<213> Staphylococcus aureus

<400> 115
Leu Asn Thr Ala Met Thr Gly Leu Lys
1               5

<210> 116
<211> 9
<212> PRT
<213> Staphylococcus aureus

<400> 116
Met Thr Gly Leu Lys Arg Gly Val Ala
1               5

<210> 117
<211> 9
<212> PRT
<213> Staphylococcus aureus

<400> 117
Leu Lys Arg Gly Val Ala Asn His Asn
1               5

<210> 118
<211> 9
<212> PRT
<213> Staphylococcus aureus

<400> 118
Val Ala Asn His Asn Gln Val Val Gln
1               5

<210> 119
<211> 9
<212> PRT
<213> Staphylococcus aureus

<400> 119
Val Val Gln Ser Asp Asn Tyr Val Asn
1               5

<210> 120
<211> 9
<212> PRT
<213> Staphylococcus aureus

<400> 120
Val Gln Ser Asp Asn Tyr Val Asn Ala
1               5

<210> 121
<211> 9
<212> PRT
<213> Staphylococcus aureus

<400> 121
Tyr Val Asn Ala Asp Thr Asn Lys Lys
1               5

<210> 122
<211> 9
<212> PRT
<213> Staphylococcus aureus

<400> 122
Val Asn Ala Asp Thr Asn Lys Lys Asn
1               5

<210> 123
<211> 9
<212> PRT

<213> Staphylococcus aureus

<400> 123
Tyr Asn Asn Ala Tyr Asn His Ala Asn
1               5

<210> 124
<211> 9
<212> PRT
<213> Staphylococcus aureus

<400> 124
Tyr Asn His Ala Asn Asp Ile Ile Asn
1               5

<210> 125
<211> 9
<212> PRT
<213> Staphylococcus aureus

<400> 125
Ile Ile Asn Gly Asn Ala Gln His Pro
1               5

<210> 126
<211> 9
<212> PRT
<213> Staphylococcus aureus

<400> 126
Ile Asn Gly Asn Ala Gln His Pro Val
1               5

<210> 127
<211> 52
<212> PRT
<213> Staphylococcus aureus

<400> 127
Gln Thr Thr Gln Asn Asn Tyr Val Thr Asp Gln Gln Lys Ala Phe Tyr
1               5                   10                  15
Gln Val Leu His Leu Lys Gly Ile Thr Glu Glu Gln Arg Asn Gln Tyr
            20                  25                  30
Ile Lys Thr Leu Arg Glu His Pro Glu Arg Ala Gln Glu Val Phe Ser
        35                  40                  45
Glu Ser Leu Lys
    50

<210> 128
<211> 36
<212> PRT
<213> Staphylococcus aureus

<400> 128
Val Lys Asn Asn Leu Arg Tyr Gly Ile Arg Lys His Lys Leu Gly Ala
1               5                   10                  15
Ala Ser Val Phe Leu Gly Thr Met Ile Val Val Gly Met Gly Gln Asp
            20                  25                  30
Lys Glu Ala Ala
        35

<210> 129
<211> 6
<212> PRT
<213> Staphylococcus aureus

<400> 129
Asp Arg His Phe Leu Asn
 1               5

<210> 130
<211> 4
<212> PRT
<213> Staphylococcus aureus

<400> 130
Gly Asn Tyr Asp
1

<210> 131
<211> 5
<212> PRT
<213> Staphylococcus aureus

<400> 131
Arg Arg Tyr Pro Phe
 1               5

<210> 132
<211> 6
<212> PRT
<213> Staphylococcus aureus

<400> 132
Lys Thr Thr Leu Leu Lys
 1               5

<210> 133
<211> 7
<212> PRT
<213> Staphylococcus aureus

<400> 133
Gly Val Thr Thr Ser Leu Ser
 1               5

<210> 134
<211> 5
<212> PRT
<213> Staphylococcus aureus

<400> 134
Val Asp Trp Leu Arg
 1               5

<210> 135
<211> 4
<212> PRT

<213> Staphylococcus aureus

<400> 135
Arg Gly Phe Leu
1

<210> 136
<211> 15
<212> PRT
<213> Staphylococcus aureus

<400> 136
Lys Ile Lys Val Tyr Val Gly Asn Tyr Asp Phe Trp Tyr Gln Ser
1               5                   10                  15

<210> 137
<211> 16
<212> PRT
<213> Staphylococcus aureus

<400> 137
Thr Val Ile Val Val Ser His Asp Arg His Phe Leu Tyr Asn Asn Val
1               5                   10                  15

<210> 138
<211> 15
<212> PRT
<213> Staphylococcus aureus

<400> 138
Thr Glu Thr Phe Leu Arg Gly Phe Leu Gly Arg Met Leu Phe Ser
1               5                   10                  15

<210> 139
<211> 35
<212> DNA
<213> Staphylococcus aureus

<400> 139
cgcggatccg cagattctga tattaatatt aaaac                                35

<210> 140
<211> 33
<212> DNA
<213> Staphylococcus aureus

<400> 140
cccaagcttt taatttgtca tttcttcttt ttc                                  33

<210> 141
<211> 34
<212> DNA
<213> Staphylococcus aureus

<400> 141
cgcggatccg ctgggtctaa taattttaaa gatg                                 34

<210> 142
<211> 30
<212> DNA
<213> Staphylococcus aureus

<400> 142
cccaagcttt tatggaataa cgatttgttg                                        30

<210> 143
<211> 32
<212> DNA
<213> Staphylococcus aureus

<400> 143
cgcggatcca gtgaaaatag tgttacgcaa tc                                     32

<210> 144
<211> 33
<212> DNA
<213> Staphylococcus aureus

<400> 144
cccaagcttt tactctggaa ttggttcaat ttc                                    33

<210> 145
<211> 31
<212> DNA
<213> Staphylococcus aureus

<400> 145
cgcggatcca cacaaacaac tgcaactaac g                                      31

<210> 146
<211> 35
<212> DNA
<213> Staphylococcus aureus

<400> 146
cccaagcttt tatgctttgt gattcttttt caaac                                  35

<210> 147
<211> 26
<212> DNA
<213> Staphylococcus aureus

<400> 147
cgcggatcca acacgcaaca aacttc                                            26

<210> 148
<211> 36
<212> DNA
<213> Staphylococcus aureus

<400> 148
ggaactgcag ttatttccag aatgataata aattac                                 36

<210> 149
<211> 28
<212> DNA
<213> Staphylococcus aureus

<400> 149
cgcggatccg cagaacatac gaatggag                                          28

<210> 150
<211> 32
<212> DNA
<213> Staphylococcus aureus

<400> 150
cccaagcttt tatgtttctt cttcgtagta gc                                    32

<210> 151
<211> 28
<212> DNA
<213> Staphylococcus aureus

<400> 151
cgcggatccg aggagaattc agtacaag                                         28

<210> 152
<211> 31
<212> DNA
<213> Staphylococcus aureus

<400> 152
cccaagcttt tattcgtcat catagtatcc g                                     31

<210> 153
<211> 25
<212> DNA
<213> Staphylococcus aureus

<400> 153
aaaagtactc accaccacca ccacc                                            25

<210> 154
<211> 29
<212> DNA
<213> Staphylococcus aureus

<400> 154
aaaagtactc acttgattca tcgcttcag                                        29

<210> 155
<211> 33
<212> DNA
<213> Staphylococcus aureus

<400> 155
gcgcgccatg gcacaagctt ctacacaaca tac                                   33

<210> 156
<211> 32
<212> DNA
<213> Staphylococcus aureus

<400> 156
gcgcgctcga gatggatgaa tgcatagcta ga                                    32

<210> 157
<211> 48
<212> DNA
<213> Staphylococcus aureus

<400> 157
gcatccatgg caccatcacc atcaccacga agtaaacgtt gatcaagc                   48

<210> 158
<211> 34
<212> DNA
<213> Staphylococcus aureus

<400> 158

agcactcgag ttagaatccc caagcaccta aacc 34

<210> 159
<211> 29
<212> DNA
<213> Staphylococcus aureus

<400> 159
gcacccatgg cagaaaatac aaatacttc 29

<210> 160
<211> 31
<212> DNA
<213> Staphylococcus aureus

<400> 160
ttttctcgag cattttagat tgactaagtt g 31

<210> 161
<211> 33
<212> DNA
<213> Staphylococcus aureus

<400> 161
caagtcccat ggctgagacg acacaagatc aac 33

<210> 162
<211> 31
<212> DNA
<213> Staphylococcus aureus

<400> 162
cagtctcgag ttttacagct gtttttggtt g 31

<210> 163
<211> 30
<212> DNA
<213> Staphylococcus aureus

<400> 163
agctcatatg gcttatactg ttactaaacc 30

<210> 164
<211> 29
<212> DNA
<213> Staphylococcus aureus

<400> 164
gcgcctcgag tttatattgt gggatgtcg 29

<210> 165
<211> 34
<212> DNA
<213> Staphylococcus aureus

<400> 165
caagtcccat ggcaacagaa gctacgaacg caac 34

<210> 166
<211> 35
<212> DNA
<213> Staphylococcus aureus

<400> 166

accagtctcg agtaattctt tagctttaga gcttg 35

<210> 167
<211> 33
<212> DNA
<213> Staphylococcus aureus

<400> 167
tattctcgag gctttgagtg tgtccatcat ttg 33

<210> 168
<211> 32
<212> DNA
<213> Staphylococcus aureus

<400> 168
gaagccatgg cagcagctga agaaacaggt gg 32

<210> 169
<211> 30
<212> DNA
<213> Staphylococcus aureus

<400> 169
gattacacca tggttaaacc tcaagcgaaa 30

<210> 170
<211> 30
<212> DNA
<213> Staphylococcus aureus

<400> 170
aggtgtctcg agtgcgattg tagcttcatt 30